(19) Europäisches Patentamt
European Patent Office
Office européen des brevets

(11) EP 1 671 941 A1

(12) **EUROPEAN PATENT APPLICATION**
published in accordance with Art. 158(3) EPC

(43) Date of publication:
**21.06.2006 Bulletin 2006/25**

(21) Application number: **04773671.5**

(22) Date of filing: **30.09.2004**

(51) Int Cl.:
*C07C 215/46* (1990.01) *C07C 233/75* (1990.01)
*C07C 309/66* (1990.01) *C07C 317/28* (1990.01)
*C07C 323/42* (1990.01) *C07D 213/38* (1985.01)
*C07D 213/40* (1985.01) *C07D 213/64* (1985.01)
*C07D 231/14* (1985.01) *C07D 249/08* (1985.01)
*C07D 307/83* (1985.01) *C07F 7/18* (1968.09)
*A01N 37/22* (1985.01) *C07C 43/12* (1968.09)

(86) International application number:
**PCT/JP2004/014810**

(87) International publication number:
**WO 2005/030699 (07.04.2005 Gazette 2005/14)**

(84) Designated Contracting States:
**AT BE BG CH CY CZ DE DK EE ES FI FR GB GR HU IE IT LI LU MC NL PL PT RO SE SI SK TR**

(30) Priority: **30.09.2003 JP 2003339358**

(71) Applicant: **Nissan Chemical Industries, Ltd.**
**Tokyo 101-0054 (JP)**

(72) Inventors:
- **IKEDA, Eitatsu,c/o Chemi.Research.Laboratories Funabashi-shi, Chiba 274-8507 (JP)**
- **MAEDA, Kazushige,c/o Chemi.Research.Laboratories Funabashi-shi, Chiba 274-8507 (JP)**
- **FURUKAWA, Yuuki,c/o Chemi.Research.Laboratories Funabashi-shi, Chiba 274-8507 (JP)**
- **TAKII, Shinji,c/o Res.Sta.Biological Sciences Minamisaitama-gun.saitama 349-0294 (JP)**

(74) Representative: **Albrecht, Thomas**
**Kraus & Weisert**
**Patent- und Rechtsanwälte**
**Thomas-Wimmer-Ring 15**
**80539 München (DE)**

(54) **SUBSTITUTED BENZANILIDE COMPOUND AND PEST CONTROL AGENT**

(57) The present invention is to provide a novel agrigultural chemical, particularly an insecticide or acaricide, and discloses a substituted benzanilide compound represented by the formula (1):

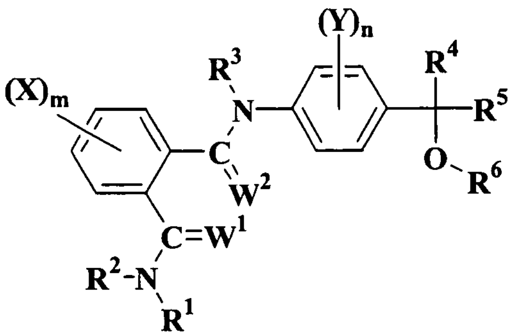

(1)

wherein $W^1$ and $W^2$ each independently represent an oxygen atom or a sulfur atom, X represents a halogen atom, etc., Y represents $C_1$ to $C_6$ alkyl, etc., $R^1$ represents $C_1$ to $C_{12}$ alkyl, a $C_1$ to $C_6$ alkylthio($C_1$ to $C_6$) alkyl, a $C_1$ to $C_6$ alkylsulfinyl ($C_1$ to $C_6$) alkyl or a $C_1$ to $C_6$ alkylsulfonyl($C_1$ to $C_6$) alkyl, etc., $R^2$ and $R^3$ each independently represent a hydrogen atom, etc., $R^4$ represents a $C_1$ to $C_6$ alkyl or a $C_1$ to $C_6$ haloalkyl, etc., $R^5$ represents a phenoxy($C_1$ to $C_6$) haloalkyl

EP 1 671 941 A1

substituted by $(Z)_{p1}$, a phenyl($C_2$ to $C_6$) alkenyl, substituted by $(Z)_{p1}$ phenyl, a phenyl substituted by $(Z)_{p1}$ or L, etc., $R^6$ represents a hydrogen atom, a $C_1$ to $C_{12}$ alkyl, a $C_1$ to $C_6$ alkoxy($C_1$ to $C_6$) alkyl or a $C_1$ to $C_6$ alkylcarbonyl, etc., L represents an aromatic heterocyclic ring such as L-1 to L-4, L-8 to L-13, L-15 to L-23, L-45 to L-52 or L-53, etc., Z represents a halogen atom, a $C_1$ to $C_6$ haloalkyl, a $C_1$ to $C_6$ haloalkoxy or a $C_1$ to $C_6$ haloalkoxy-($C_1$ to $C_6$) haloalkoxy, etc., m and n each independently is an integer of 0 to 4, p1 is an integer or 1 to 5,
or a salt thereof, and a noxious organism controlling agent containing these.

## Description

Technical field

The present invention relates to a novel substituted benzanilide compound and a salt thereof, and a noxious organism controlling agent which comprises the compound as an effective ingredient. The noxious organism controlling agent in the present invention means a noxious insect controlling agent objective to noxious arthropods in the agricultural and horticultural field or stock-raising and hygiene field (a medicine for animals or an insecticide for domestic or business purpose). Also, as the agricultural chemical in the present invention means an insecticide, an acaricide, a nematocide, a herbicide and a fungicide in the agricultural and horticultural field.

Background art

Heretofore, it has been known that specific substituted benzanilide derivatives have cytokine production inhibiting activity, vasopressin antagonistic activity, etc., and have been used as a medicine (for example, see WO 98/024771 A brochure, WO 99/051580A brochure and JP-A-2002-249473.). Also, it has been known that specific substituted benzanilide derivatives have insecticidal activity (for example, see EP-A-0919542, EP-A-1006107, WO 01/021576A brochure, WO 01/046124A brochure, JP-A-2001-335559, WO 02/062807A brochure. WO 02/094765A brochure, WO 2004/000796A brochure and WO 2004/018415A brochure.). However, there is no disclosure therein about the substituted benzanilide compounds according to the present invention.

DISCLOSURE OF THE INVENTION

Problems to be solved by the invention

Due to use of noxious organism controlling agents such as an insecticide and a fungicide for a long period of time, noxious insects have obtained resistivity thereto in recent years, so that prevention thereof by the conventionally used insecticides or fungicides becomes difficult. Also, a part of the known noxious organism controlling agents has high toxicity, or some of them are putting an ecological system in confusion due to their long residual activity. Under such a circumstance, it has been usually expected to develop a novel noxious organism controlling agent which has low toxicity and low remaining property.

Means to solve the problems

The present inventors have conducted earnest studies to solve the above-mentioned problems, and as a result, they have found that the novel substituted benzanilide compound represented by the following formula (1) according to the present invention is an extremely useful compound which has an excellent noxious organism controlling activity, in particular, an insecticidal and acaricidal activity, and causing substantially no bad effect against non-target organisms such as mammals, fishes and useful insects, whereby they have accomplished the present invention.

That is, the present invention relates to the following [1] to [8].

[1] A substituted benzanilide compound represented by the formula (1):

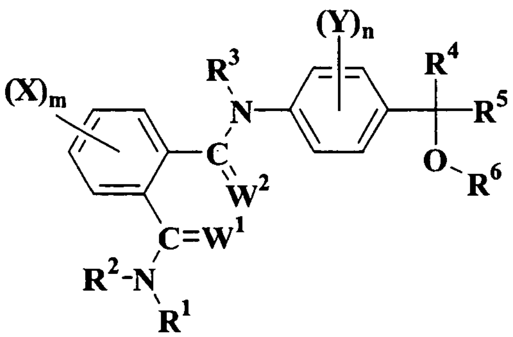

(1)

wherein $W^1$ and $W^2$ each independently represent an oxygen atom or a sulfur atom,
X represents a halogen atom, cyano, nitro, azide, -SCN, $-SF_5$, a $C_1$ to $C_6$ alkyl, a ($C_1$ to $C_6$) alkyl optionally substituted

by $R^7$, a $C_3$ to $C_8$ cycloalkyl, a ($C_3$ to $C_8$) cycloalkyl optionally substituted by $R^7$, a $C_2$ to $C_6$ alkenyl, a ($C_2$ to $C_6$) alkenyl optionally substituted by $R^7$, a $C_3$ to $C_8$ cycloalkenyl, a $C_3$ to $C_8$ halocycloalkenyl, a $C_2$ to $C_6$ alkynyl, a ($C_2$ to $C_6$) alkynyl optionally substituted by $R^7$, -OH, -$OR^8$, -$OS(O)_2R^8$, -SH, -$S(O)_rR^8$, -CHO, -$C(O)R^9$, -$C(O)OR^9$, -C (O)$SR^9$, -$C(O)NHR^{10}$, -$C(O)N(R^{10})R^9$, -$C(S)OR^9$, -$C(S)SR^9$, -$C(S)NHR^{10}$, -$C(S)N(R^{10})R^9$, -$CH{=}NOR^{11}$, -$C(R^9)$ $=NOR^{11}$, -$S(O)_2OR^9$, -$S(O)_2NHR^{10}$, -$S(O)_2N(R^{10})R^9$, -$Si(R^{13})(R^{14})R^{12}$, phenyl, a phenyl substituted by $(Z)_{p1}$, L or M, when m is 2, 3 or 4, each X may be the same or different from each other,

and when two Xs are adjacent to each other, the adjacent two Xs may form a 5-membered ring or 6-membered ring with the carbon atoms to which two Xs are bonded by forming -$CH_2CH_2CH_2$-, -$CH_2CH_2O$-, -$CH_2OCH_2$-, -$OCH_2O$-, -$CH_2CH_2S$-, -$CH_2SCH_2$-, -$CH_2CH_2N(R^{15})$-, -$CH_2N(R^{15})CH_2$-, -$CH_2CH_2CH_2CH_2$-, -$CH_2CH_2CH_2O$-, -$CH_2CH_2OCH_2$-, -$CH_2OCH_2O$-, -$OCH_2CH_2O$-, -$OCH_2CH_2S$-, -$CH_2CH{=}CH$-, -$OCH{=}CH$-, -$SCH{=}CH$-, -$N(R^{15})CH{=}CH$-, -$OCH{=}N$-, -$SCH{=}N$-, -$N(R^{15})CH{=}N$-, -$N(R^{15})N{=}CH$-, -$CH{=}CHCH{=}CH$-, -$OCH_2CH{=}CH$-, -$N{=}CHCH{=}CH$-, -$N{=}CHCH{=}N$- or -$N{=}CHN{=}CH$-, and at this time, each hydrogen atom bonded to the respective carbon atoms which form the ring may be optionally substituted by Z, and further when it is substituted by two or more Zs at the same time, each Z may be the same or different from each other,

Y represents a halogen atom, cyano, nitro, a $C_1$ to $C_6$ alkyl, a ($C_1$ to $C_6$) alkyl optionally substituted by $R^7$, a $C_3$ to $C_8$ cycloalkyl, -$OR^8$, -$S(O)_rR^8$, -$NH_2$, a $C_1$ to $C_6$ alkylamino, a di($C_1$ to $C_6$ alkyl)amino or -$Si(R^{13})(R^{14})R^{12}$, when n is 2, 3 or 4, each Y may be the same or different from each other, and when two Ys are adjacent to each other, the adjacent two Ys may form a 5-membered ring or 6-membered ring with the carbon atoms to which two Ys are bonded by forming -$CH_2CH_2CH_2$-, -$CH_2CH_2O$- -$CH_2OCH_2$-, -$OCH_2O$-, -$CH_2CH_2S$-, -$CH_2SCH_2$-, -$SCH_2S$-, -$CH_2CH_2CH_2CH_2$-, -$CH_2CH_2CH_2O$-, -$CH_2CH_2OCH_2$-,- $CH_2OCH_2O$-, -$OCH_2CH_2O$-, -$OCH_2CH_2S$-, -$SCH_2CH_2S$-, -$OCH{=}N$- or -$SCH{=}N$-, and at this time, each hydrogen atom bonded to the respective carbon atoms which form the ring may be optionally substituted by Z, and further when it is substituted by two or more Zs at the same time, each Z may be the same or different from each other,

$R^1$ represents a hydrogen atom, cyano, a $C_1$ to $C_{12}$ alkyl, a ($C_1$ to $C_{12}$) alkyl optionally substituted by $R^{16}$, a $C_3$ to $C_{12}$ cycloalkyl, a ($C_3$ to $C_{12}$) cycloalkyl optionally substituted by $R^{16}$, a $C_3$ to $C_{12}$ alkenyl, a ($C_3$ to $C_{12}$) alkenyl optionally substituted by $R^{16}$, a $C_3$ to $C_{12}$ cycloalkenyl, a $C_3$ to $C_{12}$ halocycloalkenyl, a $C_3$ to $C_{12}$ alkynyl, a ($C_3$ to $C_{12}$) alkynyl optionally substituted by $R^{16}$, -OH, a $C_1$ to $C_8$ alkoxy, a $C_3$ to $C_8$ alkenyloxy, a $C_3$ to $C_8$ haloalkenyloxy, phenoxy, a phenoxy substituted by $(Z)_{p1}$, a phenyl($C_1$ to $C_4$) alkoxy, a phenyl($C_1$ to $C_4$) alkoxy substituted by $(Z)_{p1}$, -$N(R^{20})R^{19}$, phenyl, a phenyl substituted by $(Z)_{p1}$, L or M,

$R^2$ and $R^3$ each independently represent a hydrogen atom, cyano, a $C_1$ to $C_{12}$ alkyl, a ($C_1$ to $C_{12}$) alkyl optionally substituted by $R^{16}$, a $C_3$ to $C_{12}$ alkenyl, a $C_3$ to $C_{12}$ haloalkenyl, a $C_3$ to $C_{12}$ alkynyl, a $C_3$ to $C_{12}$ haloalkynyl, -OH, a $C_1$ to $C_8$ alkoxy, a $C_1$ to $C_6$ alkylthio, a $C_1$ to $C_6$ haloalkylthio, phenylthio, a phenylthio substituted by $(Z)_{P1}$, -S $(O)_2R^9$, -$SN(R^{18})R^{17}$, -$S(O)_2N(R^{10})R^9$, -$N(R^{20})R^{19}$, -$C(O)R^9$, -$C(O)OR^9$, -$C(O)SR^9$, -$C(O)N(R^{10})R^9$, -$C(S)OR^9$, -C (S)$SR^9$, -$C(S)N(R^{10})R^9$, phenyl or a phenyl substituted by $(Z)_{p1}$, or $R^2$ is combined with $R^1$ to form a $C_2$ to $C_6$ alkylene chain whereby it may form a 3 to 7-membered ring with the nitrogen atom to which they are bonded, and the alkylene chain at this time may contain one oxygen atom, sulfur atom or nitrogen atom, and may be optionally substituted by a halogen atom, a $C_1$ to $C_6$ alkyl group, a $C_1$ to $C_6$ haloalkyl group, a $C_1$ to $C_6$ alkoxy group, a $C_1$ to $C_6$ alkylcarbonyl group or a $C_1$ to $C_6$ alkoxycarbonyl group,

$R^4$ represents a $C_1$ to $C_6$ alkyl, a $C_1$ to $C_6$ haloalkyl, a ($C_1$ to $C_6$) alkyl optionally substituted by $R^{21}$, a ($C_1$ to $C_6$) haloalkyl optionally substituted by $R^{21}$, a $C_3$ to $C_8$ cycloalkyl, a $C_3$ to $C_8$ halocycloalkyl, a ($C_3$ to $C_8$) cycloalkyl optionally substituted by $R^{21}$, a ($C_3$ to $C_8$) halocycloalkyl optionally substituted by $R^{21}$, a $C_3$ to $C_6$ alkenyl, a $C_3$ to $C_6$ haloalkenyl, a $C_3$ to $C_6$ alkynyl, a $C_3$ to $C_6$ haloalkynyl, phenyl, a phenyl substituted by $(Z)_{p1}$, L or M,

$R^5$ represents cyano, a ($C_1$ to $C_6$) alkyl optionally substituted by $R^{21}$, a ($C_1$ to $C_6$) haloalkyl optionally substituted by $R^{21}$, a ($C_3$ to $C_8$) cycloalkyl optionally substituted by $R^{21}$, a ($C_3$ to $C_8$) halocycloalkyl optionally substituted by $R^{21}$, a ($C_2$ to $C_6$) alkenyl optionally substituted by $R^{21}$, a $C_3$ to $C_8$ cycloalkenyl, a $C_3$ to $C_8$ halocycloalkenyl, a ($C_2$ to $C_6$) alkynyl optionally substituted by $R^{21}$, -$OR^8$, -$S(O)_rR^8$, -$N(R^{10})R^9$, -CHO, -$C(O)R^9$, -$CH{=}NOR^{11}$, -$C(R^9){=}NOR^{11}$, -$C(O)OR^9$, -$C(O)SR^9$, -$C(O)NHR^{10}$, -$C(O)N(R^{10})R^9$, -$C(S)OR^9$, -$C(S)SR^9$, -$C(S)NHR^{10}$, -$C(S)N(R^{10})R^9$, phenyl, a phenyl substituted by $(Z)_{p1}$, biphenyl, a biphenyl substituted by $(Z)_{p1}$, phenoxyphenyl, a phenoxyphenyl substituted by $(Z)_{p1}$, pyridyloxyphenyl, a pyridyloxyphenyl substituted by $(Z)_{p1}$, phenylthiophenyl, a phenylthiophenyl substituted by $(Z)_{P1}$, phenylsulfinylphenyl, a phenylsulfinylphenyl substituted by $(Z)_{p1}$, phenylsulfonylphenyl, a phenylsulfonylphenyl substituted by $(Z)_{P1}$, L or M, or it forms a $C_2$ to $C_3$ alkylene chain with Y present at the adjacent position in combination whereby it may form a 5 to 6-membered ring which fuses with a benzene ring, and the alkylene chain at this time may contain one oxygen atom, sulfur atom or nitrogen atom, and may be optionally substituted by a halogen atom or a $C_1$ to $C_6$ haloalkyl group,

$R^6$ represents a hydrogen atom, a $C_1$ to $C_6$ alkyl, a $C_1$ to $C_6$ haloalkyl, a ($C_1$ to $C_6$) alkyl optionally substituted by $R^{21}$, a ($C_1$ to $C_6$) haloalkyl optionally substituted by $R^{21}$, a $C_3$ to $C_8$ cycloalkyl, a $C_3$ to $C_6$ alkenyl, a $C_3$ to $C_6$ haloalkenyl, a phenyl($C_3$ to $C_6$) alkenyl, a phenyl($C_3$ to $C_6$) alkenyl substituted by $(Z)_{p1}$, a $C_3$ to $C_8$ cycloalkenyl, a $C_3$ to $C_6$ alkynyl, a $C_3$ to $C_6$ haloalkynyl, a phenyl($C_3$ to $C_6$) alkynyl, a phenyl($C_3$ to $C_6$) alkynyl substituted by $(Z)_{p1}$,

$-S(O)_2R^9$, $-C(O)R^9$, $-C(O)OR^9$, $-C(O)SR^9$, $-C(S)OR^9$, $-C(S)SR^9$, $-C(O)NHR^{10}$, $-C(O)N(R^{10})R^9$, $-C(S)NHR^{10}$, $-C(S)N(R^{10})R^9$, $-Si(R^{13})(R^{14})R^{12}$, $-P(O)(OR^{22})_2$, $-P(S)(OR^{22})_2$ or M,

L represents an aromatic heterocyclic ring represented by any one of the formula L-1 to the formula L-58,

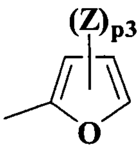
L-1

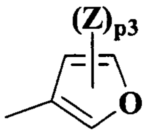
L-2

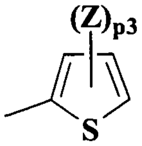
L-3

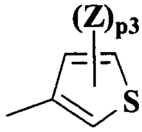
L-4

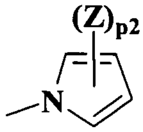
L-5

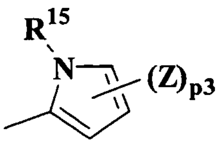
L-6

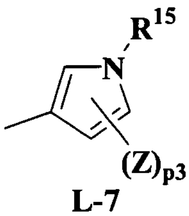
L-7

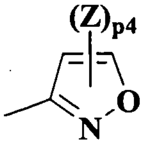
L-8

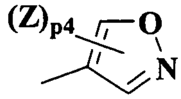
L-9

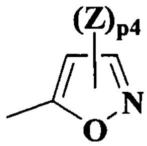
L-10

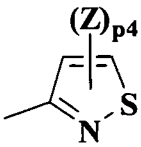
L-11

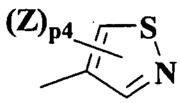
L-12

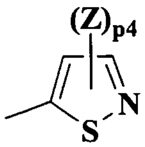
L-13

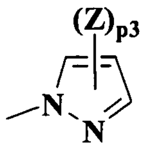
L-14

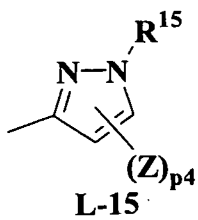
L-15

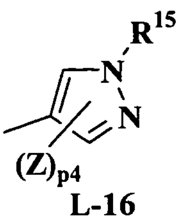
L-16

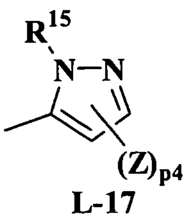
L-17

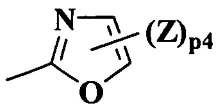
L-18

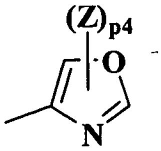
L-19

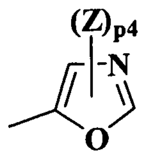
L-20

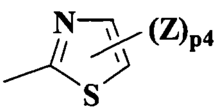
L-21

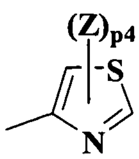
L-22

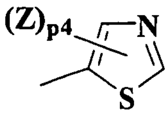
L-23

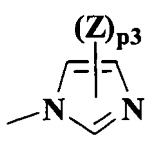
L-24

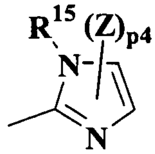
L-25

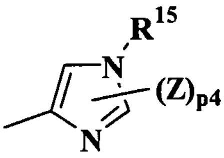
L-26

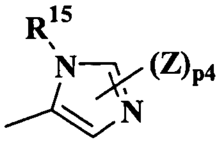
L-27

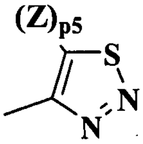
L-28

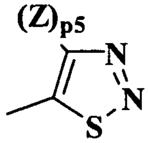
L-29

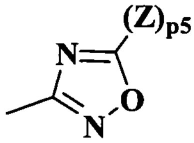
L-30

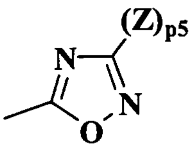
L-31

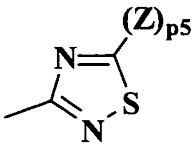
L-32

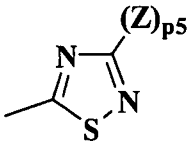
L-33

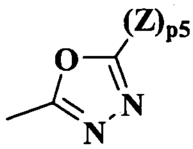
L-34

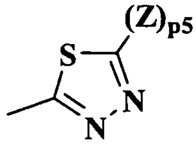
L-35

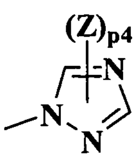
L-36

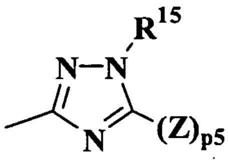
L-37

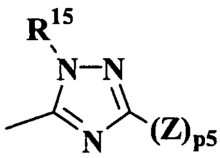
L-38

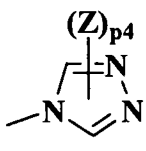
L-39

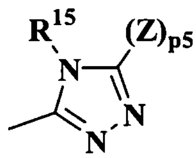
L-40

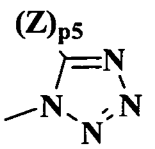
L-41

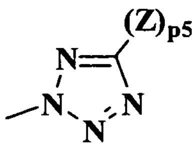
L-42

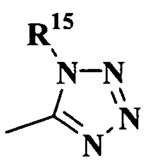
L-43

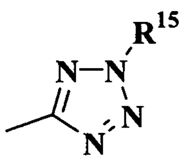
L-44

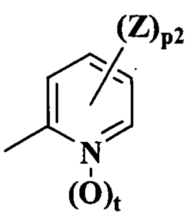
L-45

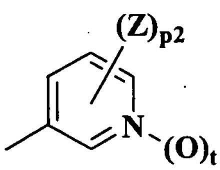
L-46

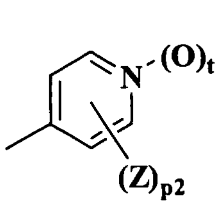
L-47

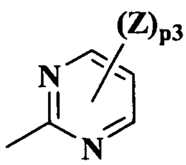
L-48

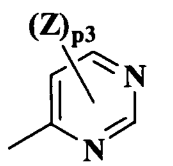
L-49

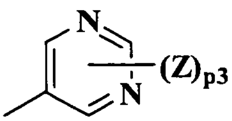 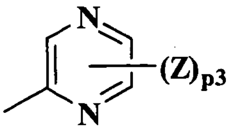 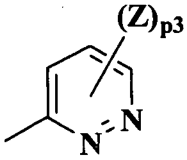 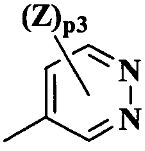 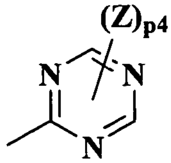

L-50 L-51 L-52 L-53 L-54

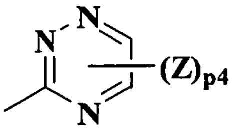 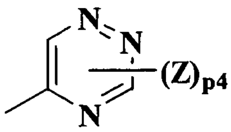 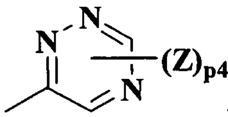 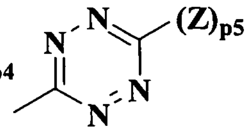

L-55 L-56 L-57 L-58

M represents an aromatic heterocyclic ring represented by any one of the formula M-1 to the formula M-28,

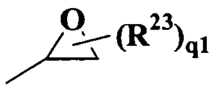 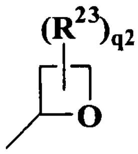 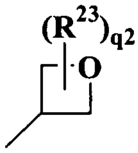

M-1 M-2 M-3

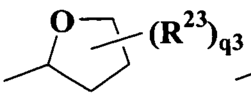 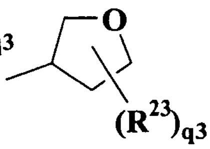 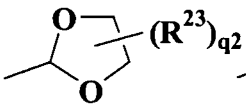 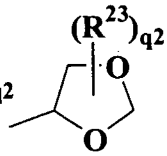 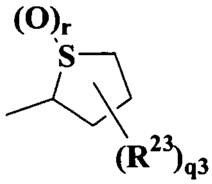

M-4 M-5 M-6 M-7 M-8

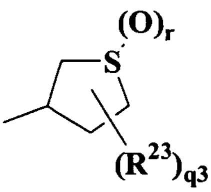 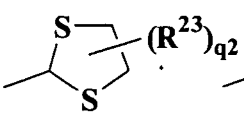 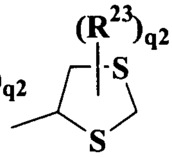 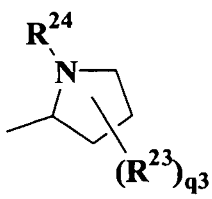 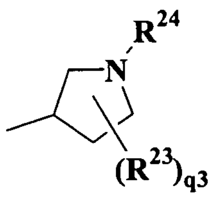

M-9 M-10 M-11 M-12 M-13

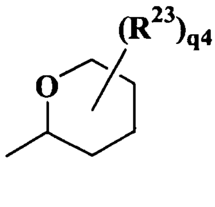
M-14

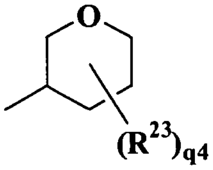
M-15

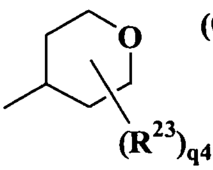
M-16

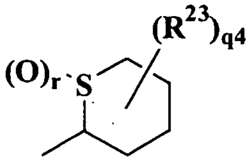
M-17

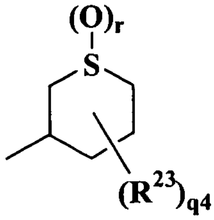
M-18

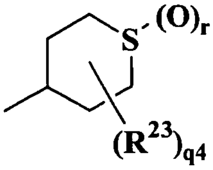
M-19

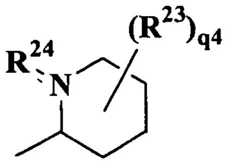
M-20

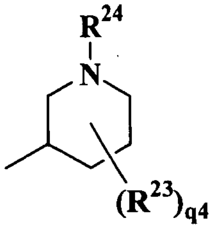
M-21

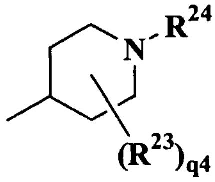
M-22

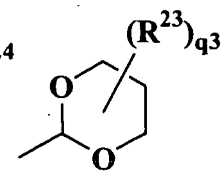
M-23

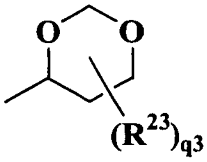
M-24

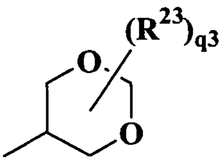
M-25

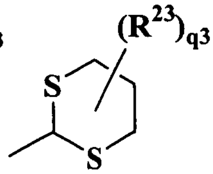
M-26

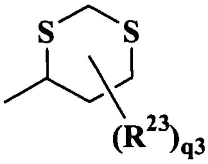
M-27

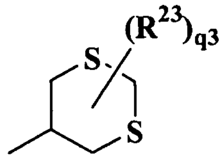
M-28

Z represents a halogen atom, cyano, nitro, azide, -SCN, $-SF_5$, a $C_1$ to $C_6$ alkyl, a $C_1$ to $C_6$ haloalkyl, a $C_1$ to $C_3$ alkoxy($C_1$ to $C_3$)alkyl, a $C_1$ to $C_3$ haloalkoxy($C_1$ to $C_3$)alkyl, cyano($C_1$ to $C_6$)alkyl, hydroxy($C_1$ to $C_3$) haloalkyl, a $C_1$ to $C_3$ alkoxy($C_1$ to $C_3$) haloalkyl, a $C_1$ to $C_3$ haloalkoxy($C_1$ to $C_3$) haloalkyl, a $C_1$ to $C_3$ alkylthio ($C_1$ to $C_3$)alkyl, a $C_1$ to $C_3$ haloalkylthio ($C_1$ to $C_3$)alkyl, a $C_1$ to $C_3$ alkylsulfinyl($C_1$ to $C_3$)alkyl, a $C_1$ to $C_3$ haloalkylsulfinyl($C_1$ to $C_3$)alkyl, a $C_1$ to $C_3$ alkylsulfonyl($C_1$ to $C_3$)alkyl, a $C_1$ to $C_3$ haloalkylsulfonyl($C_1$ to $C_3$)alkyl, a $C_3$ to $C_8$ cycloalkyl, a $C_3$ to $C_8$ halocycloalkyl, a $C_2$ to $C_6$ alkenyl, a $C_2$ to $C_6$ haloalkenyl, a $C_3$ to $C_8$ cycloalkenyl, a $C_3$ to $C_8$ halocycloalkenyl, a $C_2$ to $C_6$ alkynyl, a $C_2$ to $C_6$ haloalkynyl, -OH, a $C_1$ to $C_6$ alkoxy, a $C_1$ to $C_6$ haloalkoxy, a $C_1$ to $C_3$ haloalkoxy($C_1$ to $C_3$) haloalkoxy, a $C_2$ to $C_6$ alkenyloxy, a $C_2$ to $C_6$ haloalkenyloxy, a $C_3$ to $C_6$ alkynyloxy, a $C_3$ to $C_6$ haloalkynyloxy, a $C_1$ to $C_6$ alkylsulfonyloxy, a $C_1$ to $C_6$ haloalkylsulfonyloxy, -SH, a $C_1$ to $C_6$ alkylthio, a $C_1$ to $C_6$ haloalkylthio, a $C_1$ to $C_6$ alkylsulfinyl, a $C_1$ to $C_6$ haloalkylsulfinyl, a $C_1$ to $C_6$ alkylsulfonyl, a $C_1$ to $C_6$ haloalkylsulfonyl, $-NH_2$, a $C_1$ to $C_6$ alkylamino, a di($C_1$ to $C_6$ alkyl)amino, a $C_1$ to $C_6$ alkylsulfonylamino, a $C_1$ to $C_6$ haloalkylsulfonylamino, a $C_1$ to $C_6$ alkoxycarbonyl, a $C_1$ to $C_6$ haloalkoxycarbonyl, $-C(O)NH_2$, a $C_1$ to $C_6$ alkylaminocarbonyl, a di($C_1$ to $C_6$ alkyl) aminocarbonyl, $-C(S)NH_2$, a $C_1$ to $C_6$ alkylaminosulfonyl, a di($C_1$ to $C_6$ alkyl)aminosulfonyl or tri($C_1$ to $C_6$ alkyl)silyl,

when p1, p2, p3 or p4 is an integer of 2 or more, each Z may be the same or different from each other,

further, when two Zs are adjacent to each other, the adjacent two Zs may form a 5-membered ring or 6-membered ring with the carbon atoms to which two Zs are bonded by forming $-CH_2CH_2CH_2-$, $-CH_2CH_2O-$ $-CH_2OCH_2-$ $-OCH_2O-$ $-CH_2CH_2S-$, $-CH_2SCH_2-$, $-CH_2CH_2CH_2CH_2-$, $-CH_2CH_2CH_2O-$, $-CH_2CH_2OCH_2-$, $-CH_2OCH_2O-$, $-OCH_2CH_2O-$, $-OCH_2CH_2S-$ or -CH=CHCH=CH-, and at this time, each hydrogen atom bonded to the respective carbon atoms which form the ring may be optionally substituted by a halogen atom or a $C_1$ to $C_6$ alkyl group,

$R^7$ represents a halogen atom, cyano, a $C_3$ to $C_8$ cycloalkyl, a $C_3$ to $C_8$ halocycloalkyl, -OH, $-OR^8$, -SH, $-S(O)_rR^8$, $-N(R^{10})R^9$, $-N(R^{10})CHO$, $-N(R^{10})C(O)R^9$, $-N(R^{10})C(O)OR^9$, $-N(R^{10})C(O)SR^9$, $-N(R^{10})C(S)OR^9$, $-N(R^{10})C(S)SR^9$, $-N(R^{10})S(O)_2R^9$,- $C(O)OR^9$, $-C(O)N(R^{10})R^9$, $-Si(R^{13})(R^{14})R^{12}$, phenyl, a phenyl substituted by $(Z)_{p1}$, L or M,

$R^8$ represents a $C_1$ to $C_6$ alkyl, a ($C_1$ to $C_6$) alkyl optionally substituted by $R^{25}$, a $C_3$ to $C_8$ cycloalkyl, a ($C_3$ to $C_8$) cycloalkyl optionally substituted by $R^{25}$, a $C_2$ to $C_6$ alkenyl, a ($C_2$ to $C_6$) alkenyl optionally substituted by $R^{25}$, a $C_3$ to $C_8$ cycloalkenyl, a $C_3$ to $C_8$ halocycloalkenyl, a $C_3$ to $C_6$ alkynyl, a ($C_3$ to $C_6$) alkynyl optionally substituted by $R^{25}$,

phenyl, a phenyl substituted by $(Z)_{p1}$, L or M,

$R^9$ represents a $C_1$ to $C_6$ alkyl, a $C_1$ to $C_6$ haloalkyl, a $C_3$ to $C_6$ cycloalkyl ($C_1$ to $C_4$)alkyl, a $C_1$ to $C_6$ alkoxy($C_1$ to $C_4$)alkyl, a $C_1$ to $C_6$ alkylthio ($C_1$ to $C_4$)alkyl, a cyano($C_1$ to $C_6$)alkyl, a phenyl($C_1$ to $C_4$)alkyl, a phenyl($C_1$ to $C_4$) alkyl substituted by $(Z)_{p1}$, an L-($C_1$ to $C_4$)alkyl, an M-($C_1$ to $C_4$)alkyl, a $C_3$ to $C_8$ cycloalkyl, a $C_3$ to $C_8$ halocycloalkyl, a $C_3$ to $C_6$ alkenyl, a $C_3$ to $C_6$ haloalkenyl, a $C_3$ to $C_6$ alkynyl, phenyl or a phenyl substituted by $(Z)_{p1}$,

$R^{10}$ represents a hydrogen atom or a $C_1$ to $C_6$ alkyl, or $R^9$ and $R^{10}$ are combined in combination to form a $C_2$ to $C_6$ alkylene chain whereby they may form a 3 to 7-membered ring with an atom(s) to which they are bonded, and at this time, the alkylene chain may contain one oxygen atom, sulfur atom or nitrogen atom, and may be optionally substituted by a halogen atom, a $C_1$ to $C_6$ alkyl group, a $C_1$ to $C_6$ alkoxy group, a formyl group, a $C_1$ to $C_6$ alkylcarbonyl group or a $C_1$ to $C_6$ alkoxycarbonyl group,

$R^{11}$ represents a hydrogen atom, a $C_1$ to $C_6$ alkyl, a $C_1$ to $C_6$ haloalkyl, a phenyl($C_1$ to $C_4$)alkyl, a phenyl($C_1$ to $C_4$) alkyl substituted by $(Z)_{p1}$, a $C_3$ to $C_6$ alkenyl, a $C_3$ to $C_6$ haloalkenyl, a $C_3$ to $C_6$ alkynyl or a $C_3$ to $C_6$ haloalkynyl, or $R^{11}$ is combined with $R^9$ to form a $C_2$ to $C_4$ alkylene chain whereby it may form a 5 to 7-membered ring with an atom(s) to which they are bonded, and at this time, the alkylene chain may contain one oxygen atom, sulfur atom or nitrogen atom, and may be optionally substituted by a halogen atom or a $C_1$ to $C_6$ alkyl group,

$R^{12}$ represents a $C_1$ to $C_6$ alkyl, a $C_1$ to $C_6$ haloalkyl, a $C_3$ to $C_6$ alkenyl, phenyl or a phenyl substituted by $(Z)_{p1}$,

$R^{13}$ and $R^{14}$ each independently represent a $C_1$ to $C_6$ alkyl or a $C_1$ to $C_6$ haloalkyl,

$R^{15}$ represents a hydrogen atom, a $C_1$ to $C_6$ alkyl, a $C_1$ to $C_6$ haloalkyl, a $C_1$ to $C_6$ alkoxycarbonyl($C_1$ to $C_4$)alkyl, a $C_1$ to $C_6$ haloalkoxycarbonyl($C_1$ to $C_4$)alkyl, a phenyl($C_1$ to $C_4$)alkyl, a phenyl($C_1$ to $C_4$) alkyl substituted by $(Z)_{p1}$, a $C_3$ to $C_6$ alkenyl, a $C_3$ to $C_6$ haloalkenyl, a $C_3$ to $C_6$ alkynyl, a $C_3$ to $C_6$ haloalkynyl, a $C_1$ to $C_6$ alkoxy, a $C_1$ to $C_6$ alkoxycarbonyl, a $C_1$ to $C_6$ haloalkoxycarbonyl, phenyl or a phenyl substituted by $(Z)_{p1}$,

$R^{16}$ represents a halogen atom, cyano, nitro, a $C_3$ to $C_8$ cycloalkyl, a $C_3$ to $C_8$ halocycloalkyl, $-OR^{26}$, $-N(R^{27})R^{26}$, -SH, $-S(O)_rR^{28}$, $-SO_2NHR^{30}$, $-SO_2N(R^{30})R^{29}$, -CHO, $-C(O)R^{29}$, -C(O)OH, $-C(O)OR^{29}$, $-C(O)SR^{29}$, $-C(O)NHR^{30}$, $-C(O)N(R^{30})R^{29}$, $-C(O)C(O)OR^{29}$, $-C(R^{32})=NOH$, $-C(R^{32})=NOR^{31}$, $-Si(R^{13})(R^{14})R^{12}$, $-P(O)(OR^{22})_2$, $-P(S)(OR^{22})_2$, $-P(phenyl)_2$, $P(O)(phenyl)_2$, phenyl, a phenyl substituted by $(Z)_{p1}$, L or M,

$R^{17}$ represents a $C_1$ to $C_{12}$ alkyl, a $C_1$ to $C_{12}$ haloalkyl, a $C_1$ to $C_{12}$ alkoxy($C_1$ to $C_{12}$)alkyl, a cyano($C_1$ to $C_{12}$)alkyl, a $C_1$ to $C_{12}$ alkoxycarbonyl($C_1$ to $C_{12}$)alkyl, a phenyl($C_1$ to $C_4$)alkyl, a phenyl($C_1$ to $C_4$) alkyl substituted by $(Z)_{p1}$, a $C_3$ to $C_{12}$ alkenyl, a $C_3$ to $C_{12}$ haloalkenyl, a $C_3$ to $C_{12}$ alkynyl, a $C_3$ to $C_{12}$ haloalkynyl, a $C_1$ to $C_{12}$ alkylcarbonyl, a $C_1$ to $C_{12}$ alkoxycarbonyl, phenyl or a phenyl substituted by $(Z)_{p1}$,

$R^{18}$ represents a $C_1$ to $C_{12}$ alkyl, a $C_1$ to $C_{12}$ haloalkyl, a $C_1$ to $C_{12}$ alkoxy($C_1$ to $C_{12}$)alkyl, a cyano($C_1$ to $C_{12}$)alkyl, a $C_1$ to $C_{12}$ alkoxycarbonyl($C_1$ to $C_{12}$)alkyl, a phenyl($C_1$ to $C_4$)alkyl, a phenyl($C_1$ to $C_4$) alkyl substituted by $(Z)_{p1}$, a $C_3$ to $C_{12}$ alkenyl, a $C_3$ to $C_{12}$ haloalkenyl, a $C_3$ to $C_{12}$ alkynyl, a $C_3$ to $C_{12}$ haloalkynyl, phenyl or a phenyl substituted by $(Z)_{p1}$, or $R^{17}$ and $R^{18}$ are combined in combination to form a $C_4$ to $C_7$ alkylene chain whereby it may form a 5 to 8-membered ring with the nitrogen atom to which they are bonded, and at this time, the alkylene chain may contain one oxygen atom or sulfur atom, and may be optionally substituted by a $C_1$ to $C_4$ alkyl group or a $C_1$ to $C_4$ alkoxy group,

$R^{19}$ represents a hydrogen atom, a $C_1$ to $C_6$ alkyl, a $C_1$ to $C_6$ haloalkyl, a phenyl($C_1$ to $C_4$)alkyl, a phenyl($C_1$ to $C_4$) alkyl substituted by $(Z)_{p1}$, a $C_3$ to $C_6$ alkenyl, a $C_3$ to $C_6$ haloalkenyl, a $C_3$ to $C_6$ alkynyl, -CHO, a $C_1$ to $C_6$ alkylcarbonyl, a $C_1$ to $C_6$ haloalkylcarbonyl, a $C_1$ to $C_6$ alkoxycarbonyl, a $C_1$ to $C_6$ haloalkoxycarbonyl, a phenyl($C_1$ to $C_4$) alkoxycarbonyl, a phenyl($C_1$ to $C_4$) alkoxycarbonyl substituted by $(Z)_{p1}$, phenoxycarbonyl, a phenoxycarbonyl substituted by $(Z)_{p1}$, phenylcarbonyl, a phenylcarbonyl substituted by $(Z)_{p1}$, phenyl or a phenyl substituted by $(Z)_{p1}$,

$R^{20}$ represents a hydrogen atom, a $C_1$ to $C_6$ alkyl, -CHO, a $C_1$ to $C_6$ alkylcarbonyl, a $C_1$ to $C_6$ haloalkylcarbonyl or a $C_1$ to $C_6$ alkoxycarbonyl,

$R^{21}$ represents cyano, a $C_3$ to $C_8$ cycloalkyl, a $C_3$ to $C_8$ halocycloalkyl, -OH, $-OR^8$ , -SH, $-S(O)_rR^8$, $-N(R^{10})R^9$, $-N(R^{10})CHO$, $-N(R^{10})C(O)R^9$, $-N(R^{10})C(O)OR^9$, $-N(R^{10})C(O)SR^9$, $-N(R^{10})C(S)OR^9$, $-N(R^{10})C(S)SR^9$, $-N(R^{10})S(O)_2R^9$, $-C(O)OR^9$, $-C(O)N(R^{10})R^9$, $-Si(R^{13})(R^{14})R^{12}$, phenyl, a phenyl substituted by $(Z)_{p1}$, L or M,

$R^{22}$ represents a $C_1$ to $C_6$ alkyl or a $C_1$ to $C_6$ haloalkyl,

$R^{23}$ represents a halogen atom, cyano, a $C_1$ to $C_6$ alkyl, a $C_1$ to $C_6$ haloalkyl, a hydroxy($C_1$ to $C_6$)alkyl, a $C_1$ to $C_4$ alkoxy($C_1$ to $C_4$)alkyl, a $C_1$ to $C_4$ alkoxycarbonyl($C_1$ to $C_4$)alkyl, a $C_1$ to $C_6$ alkoxy, a $C_1$ to $C_6$ alkoxycarbonyl, phenyl or a phenyl substituted by $(Z)_{p1}$, when q1, q2, q3 or q4 is an integer of 2 or more, each $R^{23}$ may be the same or different from each other,

$R^{24}$ represents a hydrogen atom, a $C_1$ to $C_6$ alkyl, a $C_1$ to $C_6$ haloalkyl, -CHO, a $C_1$ to $C_6$ alkylcarbonyl, a $C_1$ to $C_6$ haloalkylcarbonyl, a phenyl($C_1$ to $C_4$)alkylcarbonyl, a phenyl($C_1$ to $C_4$)alkylcarbonyl substituted by $(Z)_{p1}$, a $C_1$ to $C_6$ alkoxycarbonyl, a $C_1$ to $C_6$ haloalkoxycarbonyl, a phenyl($C_1$ to $C_4$) alkoxycarbonyl, a phenyl($C_1$ to $C_4$)alkoxycarbonyl substituted by $(Z)_{p1}$, a $C_1$ to $C_6$ alkylthio carbonyl, a $C_1$ to $C_6$ alkoxythiocarbonyl, a $C_1$ to $C_6$ alkylaminocarbonyl, a di($C_1$ to $C_6$ alkyl)aminocarbonyl, a $C_1$ to $C_6$ alkylaminothiocarbonyl, a di($C_1$ to $C_6$ alkyl)aminothiocarbonyl, phenylcarbonyl, a phenylcarbonyl substituted by $(Z)_{p1}$, a $C_1$ to $C_6$ alkylsulfonyl, a $C_1$ to $C_6$ haloalkylsulfonyl, phenylsulfonyl, a phenylsulfonyl substituted by $(Z)_{p1}$, $-P(O)(OR^{22})_2$ or $-P(S)(OR^{22})_2$,

$R^{25}$ represents a halogen atom, cyano, a $C_3$ to $C_8$ cycloalkyl, a $C_3$ to $C_8$ halocycloalkyl, a $C_1$ to $C_6$ alkoxy, a $C_1$ to

$C_6$ haloalkoxy, a $C_1$ to $C_6$ alkylthio, a $C_1$ to $C_6$ haloalkylthio, a $C_1$ to $C_6$ alkylsulfonyl, a $C_1$ to $C_6$ haloalkylsulfonyl, a $C_1$ to $C_6$ alkylamino, a di($C_1$ to $C_6$ alkyl)amino, -CHO, a $C_1$ to $C_6$ alkylcarbonyl, a $C_1$ to $C_6$ haloalkylcarbonyl, a $C_1$ to $C_6$ alkoxycarbonyl, a $C_1$ to $C_6$ haloalkoxycarbonyl, -CH=NOR$^{11}$, -C(R$^9$)=NOR$^{11}$, phenyl, a phenyl substituted by $(Z)_{p1}$, L or M,

R$^{26}$ represents a hydrogen atom, a $C_1$ to $C_8$ alkyl, a ($C_1$ to $C_8$) alkyl optionally substituted by R$^{33}$, a $C_3$ to $C_8$ cycloalkyl, a ($C_3$ to $C_8$) cycloalkyl optionally substituted by R$^{33}$, a $C_3$ to $C_8$ alkenyl, a ($C_3$ to $C_8$) alkenyl optionally substituted by R$^{33}$, a $C_3$ to $C_8$ alkynyl, a ($C_3$ to $C_8$) alkynyl optionally substituted by R$^{33}$, -CHO, -C(O)R$^{29}$, -C(O)OR$^{29}$, -C(O)SR$^{29}$, -C(O)NHR$^{30}$, -C(O)N(R$^{30}$)R$^{29}$, -C(O)C(O)R$^{29}$, -C(O)C(O)OR$^{29}$, -C(S)R$^{29}$, -C(S)OR$^{29}$, C(S)SR$^{29}$, -C(S)NHR$^{30}$, -C(S)N(R$^{30}$)R$^{29}$, -S(O)$_2$R$^{29}$, -S(O)$_2$N(R$^{30}$)R$^{29}$, -Si(R$^{13}$)(R$^{14}$)R$^{12}$, P(O)(OR$^{22}$h, -P(S)(OR$^{22}$h, phenyl, a phenyl substituted by $(Z)_{p1}$, L or M,

R$^{27}$ represents a hydrogen atom, a $C_1$ to $C_6$ alkyl, a $C_1$ to $C_6$ haloalkyl, a $C_3$ to $C_6$ cycloalkyl or a $C_1$ to $C_6$ alkoxy, or R$^{26}$ and R$^{27}$ are combined in combination to form a $C_2$ to $C_5$ alkylene chain whereby it forms a 3 to 6-membered ring with the nitrogen atom to which they are bonded, and at this time, the alkylene chain may contain one oxygen atom or sulfur atom, and may be substituted by a halogen atom, a $C_1$ to $C_6$ alkyl group, a $C_1$ to $C_6$ alkoxy group, a phenyl group or a phenyl group substituted by $(Z)_{p1}$,

R$^{28}$ represents a $C_1$ to $C_8$ alkyl, a ($C_1$ to $C_8$) alkyl optionally substituted by R$^{33}$, a $C_3$ to $C_8$ cycloalkyl, a ($C_3$ to $C_8$) cycloalkyl optionally substituted by R$^{33}$, a $C_3$ to $C_8$ alkenyl, a ($C_3$ to $C_8$) alkenyl optionally substituted by R$^{33}$, a $C_3$ to $C_8$ alkynyl, a ($C_3$ to $C_8$) alkynyl optionally substituted by R$^{33}$, -SH, a $C_1$ to $C_6$ alkylthio, a $C_1$ to $C_6$ haloalkylthio, phenylthio, a phenylthio substituted by $(Z)_{p1}$, -CHO, -C(O)R$^{29}$, -C(O)OR$^{29}$, -C(O)SR$^{29}$, -C(O)NHR$^{30}$, -C(O)N(R$^{30}$)R$^{29}$, -C(O)C(O)R$^{29}$, -C(O)C(O)OR$^{29}$, -C(S)R$^{29}$, -C(S)OR$^{29}$, -C(S)SR$^{29}$, -C(S)NHR$^{30}$, -C(S)N(R$^{30}$)R$^{29}$, -P(O)(OR$^{22}$)$_2$, -P(S)(OR$^{22}$)$_2$, phenyl, a phenyl substituted by $(Z)_{p1}$, L-18, L-21, L-25, L-30 to L-35, L-45, L-48, L-49 or M,

R$^{29}$ represents a $C_1$ to $C_6$ alkyl, a $C_1$ to $C_6$ haloalkyl, a $C_3$ to $C_8$ cycloalkyl ($C_1$ to $C_4$)alkyl, a $C_1$ to $C_6$ alkoxy($C_1$ to $C_4$)alkyl, a $C_1$ to $C_6$ haloalkoxy($C_1$ to $C_4$)alkyl, a $C_1$ to $C_6$ alkylthio ($C_1$ to $C_4$)alkyl, a $C_1$ to $C_6$ haloalkylthio ($C_1$ to $C_4$)alkyl, a $C_1$ to $C_6$ alkylsulfonyl($C_1$ to $C_4$)alkyl, a $C_1$ to $C_6$ haloalkylsulfonyl($C_1$ to $C_4$)alkyl, a cyano($C_1$ to $C_6$)alkyl, a $C_1$ to $C_6$ alkylcarbonyl($C_1$ to $C_4$)alkyl, a $C_1$ to $C_6$ haloalkylcarbonyl($C_1$ to $C_4$)alkyl, a $C_1$ to $C_6$ alkoxycarbonyl($C_1$ to $C_4$)alkyl, a di($C_1$ to $C_6$ alkyl)aminocarbonyl($C_1$ to $C_4$)alkyl, a tri($C_1$ to $C_4$ alkyl)silyl ($C_1$ to $C_4$)alkyl, a phenyl($C_1$ to $C_4$)alkyl, a phenyl($C_1$ to $C_4$) alkyl substituted by $(Z)_{p1}$, an L-($C_1$ to $C_4$)alkyl, an M-($C_1$ to $C_4$)alkyl, a $C_3$ to $C_8$ cycloalkyl, a $C_3$ to $C_8$ halocycloalkyl, a $C_2$ to $C_6$ alkenyl, a $C_2$ to $C_6$ haloalkenyl, a $C_2$ to $C_6$ alkynyl, a $C_2$ to $C_6$ haloalkynyl, phenyl, a phenyl substituted by $(Z)_{p1}$, L or M,

R$^{30}$ represents a hydrogen atom, a $C_1$ to $C_6$ alkyl, a $C_1$ to $C_6$ haloalkyl, phenyl or a phenyl substituted by $(Z)_{p1}$, or R$^{29}$ and R$^{30}$ are combined to form a $C_2$ to $C_5$ alkylene chain whereby it may form a 3 to 6-membered ring with the nitrogen atom to which they are bonded, and at this time, the alkylene chain may contain one oxygen atom, sulfur atom or nitrogen atom, and may be optionally substituted by a halogen atom, a $C_1$ to $C_6$ alkyl group, a $C_1$ to $C_6$ alkoxy group, a formyl group, a $C_1$ to $C_6$ alkylcarbonyl group, a $C_1$ to $C_6$ alkoxycarbonyl group, a phenyl group or a phenyl group substituted by $(Z)_{p1}$,

R$^{31}$ represents a hydrogen atom, a $C_1$ to $C_8$ alkyl, a ($C_1$ to $C_8$) alkyl optionally substituted by R$^{33}$, a $C_3$ to $C_8$ cycloalkyl, a $C_3$ to $C_8$ alkenyl, a ($C_3$ to $C_8$) alkenyl optionally substituted by R$^{33}$, a $C_3$ to $C_8$ alkynyl or a ($C_3$ to $C_8$) alkynyl optionally substituted by R$^{33}$,

R$^{32}$ represents a hydrogen atom, a $C_1$ to $C_6$ alkyl, a $C_1$ to $C_6$ haloalkyl, a $C_3$ to $C_8$ cycloalkyl ($C_1$ to $C_4$)alkyl, a $C_1$ to $C_6$ alkoxy($C_1$ to $C_4$)alkyl, a $C_1$ to $C_6$ haloalkoxy($C_1$ to $C_4$)alkyl, a $C_1$ to $C_6$ alkylthio ($C_1$ to $C_4$)alkyl, a $C_1$ to $C_6$ haloalkylthio ($C_1$ to $C_4$)alkyl, a $C_1$ to $C_6$ alkylsulfonyl($C_1$ to $C_4$)alkyl, a $C_1$ to $C_6$ haloalkylsulfonyl($C_1$ to $C_4$)alkyl, a phenyl($C_1$ to $C_4$)alkyl, a phenyl($C_1$ to $C_4$) alkyl substituted by $(Z)_{p1}$, phenyl or a phenyl substituted by $(Z)_{p1}$,

R$^{33}$ represents a halogen atom, cyano, nitro, a $C_3$ to $C_8$ cycloalkyl, a $C_3$ to $C_8$ halocycloalkyl, -OH, -OR$^{34}$, -SH, -S(O)$_r$R$^{34}$, -NHR$^{35}$, -N(R$^{35}$)R$^{34}$, -CHO, -C(O)R$^{29}$, -C(O)OR$^{29}$, -C(O)SR$^{29}$, -C(O)NHR$^{30}$, -C(O)N(R$^{30}$)R$^{29}$, -C(O)C(O)OR$^{29}$, -CH=NOR$^{11}$, -C(R$^9$)=NOR$^{11}$, -Si(R$^{13}$)(R$^{14}$)R$^{12}$, -P(O)(OR$^{22}$)$_2$, -P(S)(OR$^{22}$)$_2$, -P(phenyl)$_2$, -P(O)(phenyl)$_2$, phenyl, a phenyl substituted by $(Z)_{p1}$, L or M,

R$^{34}$ represents a $C_1$ to $C_6$ alkyl, a $C_1$ to $C_6$ haloalkyl, a $C_1$ to $C_6$ alkoxy($C_1$ to $C_4$)alkyl, a $C_1$ to $C_6$ alkylthio($C_1$ to $C_4$) alkyl, a phenyl($C_1$ to $C_4$)alkyl, a phenyl($C_1$ to $C_4$) alkyl substituted by $(Z)_{p1}$, a $C_3$ to $C_8$ cycloalkyl, a $C_3$ to $C_8$ halocycloalkyl, a $C_3$ to $C_6$ alkenyl, a $C_3$ to $C_6$ haloalkenyl, a $C_3$ to $C_8$ cycloalkenyl, a $C_3$ to $C_8$ halocycloalkenyl, a $C_3$ to $C_6$ alkynyl, a $C_3$ to $C_6$ haloalkynyl, -CHO, a $C_1$ to $C_6$ alkylcarbonyl, a $C_1$ to $C_6$ haloalkylcarbonyl, a $C_1$ to $C_6$ alkoxycarbonyl, a $C_1$ to $C_6$ haloalkoxycarbonyl, a $C_1$ to $C_6$ alkylaminocarbonyl, a di($C_1$ to $C_6$ alkyl) aminocarbonyl, phenylcarbonyl, a phenylcarbonyl substituted by $(Z)_{p1}$, a $C_1$ to $C_6$ alkylaminothiocarbonyl, a di($C_1$ to $C_6$ alkyl)aminothiocarbonyl, phenyl, a phenyl substituted by $(Z)_{p1}$, L or M,

R$^{35}$ represents a hydrogen atom, a $C_1$ to $C_6$ alkyl, a $C_1$ to $C_6$ haloalkyl, a $C_3$ to $C_8$ cycloalkyl, a $C_3$ to $C_6$ alkenyl, a $C_3$ to $C_6$ alkynyl, a $C_1$ to $C_6$ alkylcarbonyl, a $C_1$ to $C_6$ haloalkylcarbonyl, a $C_1$ to $C_6$ alkoxycarbonyl, a $C_1$ to $C_6$ haloalkoxycarbonyl, phenoxycarbonyl, a phenoxycarbonyl substituted by $(Z)p_1$, phenylcarbonyl, a phenylcarbonyl substituted by $(Z)p_1$, a $C_1$ to $C_6$ alkylsulfonyl, a $C_1$ to $C_6$ haloalkylsulfonyl, phenyl, a phenyl substituted by $(Z)p_1$, L or M, or R$^{34}$ and R$^{35}$ are combined to form a $C_2$ to $C_5$ alkylene chain, whereby it may form a 3 to 6-membered ring

with the nitrogen atom to which they are bonded, and at this time, the alkylene chain may contain one oxygen atom or sulfur atom, and may be substituted by a halogen atom or a methyl group,
m is an integer of 0 to 4,
n is an integer of 0 to 4,
p1 is an integer of 1 to 5,
p2 is an integer of 0 to 4,
p3 is an integer of 0 to 3,
p4 is an integer of 0 to 2,
p5 is an integer of 0 or 1,
q1 is an integer of 0 to 3,
q2 is an integer of 0 to 5,
q3 is an integer of 0 to 7,
q4 is an integer of 0 to 9,
r is an integer of 0 to 2,
t is an integer of 0 or 1,
or a salt thereof.

[2] The substituted benzanilide compound according to the above [1], wherein $W^1$ and $W^2$ each respresent an oxygen atom,
X represents a halogen atom, cyano, nitro, a $C_1$ to $C_6$ alkyl, a $C_1$ to $C_6$ haloalkyl, a $C_1$ to $C_6$ alkoxy, a $C_1$ to $C_6$ haloalkoxy, a $C_1$ to $C_6$ alkylsulfonyloxy, a $C_1$ to $C_6$ haloalkylsulfonyloxy, a $C_1$ to $C_6$ alkylthio, a $C_1$ to $C_6$ haloalkylthio, a $C_1$ to $C_6$ alkylsulfinyl, a $C_1$ to $C_6$ haloalkylsulfinyl, a $C_1$ to $C_6$ alkylsulfonyl or a $C_1$ to $C_6$ haloalkylsulfonyl, and when m is 2 or 3, each X may be the same or different from each other, and when two Xs are adjacent to each other, the adjacent two Xs may form a 5-membered ring or 6-membered ring with the carbon atoms to which two Xs are bonded by forming $-OCH_2O$ or $-OCH_2CH_2O-$, and at this time, the hydrogen atom(s) bonded to the respective carbon atoms which form a ring may be optionally replaced with a halogen atom, a $C_1$ to $C_4$ alkyl group or a $C_1$ to $C_4$ haloalkyl group,
Y represents a halogen atom, a $C_1$ to $C_6$ alkyl, a $C_1$ to $C_6$ haloalkyl, a hydroxy($C_1$ to $C_6$)alkyl, a $C_1$ to $C_3$ alkoxy($C_1$ to $C_3$)alkyl, a $C_1$ to $C_6$ alkoxy, a $C_1$ to $C_6$ haloalkoxy, a $C_1$ to $C_6$ alkylthio, a $C_1$ to $C_6$ haloalkylthio, a $C_1$ to $C_6$ alkylamino or a di($C_1$ to $C_6$ alkyl)amino, when n is 2 or 3, each Y may be the same or different from each other,
$R^1$ represents a $C_1$ to $C_8$ alkyl, a ($C_1$ to $C_8$) alkyl optionally substituted by $R^{16}$, a $C_3$ to $C_8$ cycloalkyl, a $C_3$ to $C_8$ alkenyl, a $C_3$ to $C_8$ alkynyl, a $C_1$ to $C_8$ alkoxy, M-4, M-5, M-8, M-9, M-13 to M-19, M-21 or M-22,
$R^2$ and $R^3$ each independently represent a hydrogen atom, a $C_1$ to $C_6$ alkyl, a $C_1$ to $C_4$ alkoxy($C_1$ to $C_4$)alkyl, a $C_1$ to $C_4$ alkylthio ($C_1$ to $C_4$)alkyl, a $C_1$ to $C_4$ alkylsulfonyl($C_1$ to $C_4$)alkyl, a $C_3$ to $C_6$ alkenyl, a $C_3$ to $C_6$ alkynyl, a $C_1$ to $C_6$ alkylthio, a $C_1$ to $C_6$ haloalkylthio, phenylthio, a phenylthio substituted by $(Z)_{p1}$ or $-SN(R^{18})R^{17}$, or $R^2$ and $R^1$ may be combined to form a $C_2$ to $C_6$ alkylene chain whereby they may form a 3 to 7-membered ring with the nitrogen atom to which they are bonded, and at this time, the alkylene chain may contain one oxygen atom or sulfur atom,
$R^4$ represents a $C_1$ to $C_6$ alkyl, a $C_1$ to $C_6$ haloalkyl, a ($C_1$ to $C_6$) alkyl optionally substituted by $R^{21}$, a ($C_1$ to $C_6$) haloalkyl optionally substituted by $R^{21}$, a $C_3$ to $C_8$ cycloalkyl, a $C_3$ to $C_8$ halocycloalkyl, a $C_3$ to $C_6$ haloalkenyl, a $C_3$ to $C_6$ haloalkynyl, phenyl or a phenyl substituted by $(Z)_{p1}$,
$R^5$ represents cyano, a ($C_1$ to $C_6$) alkyl optionally substituted by $R^{21}$, a ($C_1$ to $C_6$) haloalkyl optionally substituted by $R^{21}$, a $C_3$ to $C_8$ cycloalkyl, a $C_3$ to $C_8$ halocycloalkyl, a ($C_2$ to $C_6$) alkenyl optionally substituted by $R^{21}$, a ($C_2$ to $C_6$) alkynyl optionally substituted by $R^{21}$, $-C(O)OR^9$, $-C(O)SR^9$, $-C(O)NHR^{10}$, $-C(O)N(R^{10})R^9$, $-C(S)OR^9$, $-C(S)SR^9$, $-C(S)NHR^{10}$, $-C(S)N(R^{10})R^9$, phenyl, a phenyl substituted by $(Z)_{p1}$, a phenoxyphenyl substituted by $(Z)_{p1}$, a pyridyloxyphenyl substituted by $(Z)_{p1}$, L-1 to L-4, L-8 to L-13, L-15 to L-23, L-25 to L-35, L-37, L-38, L-40, L-43 to L-58, M-4, M-5, M-8, M-9, M-14 to M-18 or M-19, or may be combined with Y existing at the adjacent position to form a $C_2$ to $C_3$ alkylene chain, whereby it may form a 5 to 6-membered ring which fuses with a benzene ring, and at this time, the alkylene chain may contain one oxygen atom, sulfur atom or nitrogen atom, and may be optionally substituted by a halogen atom or a $C_1$ to $C_6$ haloalkyl group,
$R^6$ represents a hydrogen atom, a $C_1$ to $C_6$ alkyl, a $C_1$ to $C_6$ haloalkyl, a $C_1$ to $C_4$ alkoxy($C_1$ to $C_4$)alkyl, a $C_1$ to $C_4$ alkylthio($C_1$ to $C_4$)alkyl, a cyano($C_1$ to $C_6$)alkyl, a phenyl-($C_1$ to $C_4$)alkyl, a phenyl($C_1$ to $C_4$) alkyl substituted by $(Z)_{p1}$, a $C_3$ to $C_6$ alkenyl, a $C_3$ to $C_6$ haloalkenyl, a phenyl($C_3$ to $C_6$) alkenyl, a phenyl($C_3$ to $C_6$) alkenyl substituted by $(Z)p_1$, a $C_3$ to $C_6$ alkynyl, a $C_3$ to $C_6$ haloalkynyl, a phenyl($C_3$ to $C_6$) alkynyl, a phenyl($C_3$ to $C_6$) alkynyl substituted by $(Z)_{p1}$, $-S(O)_2R^9$, $-C(O)R^9$, $-C(O)NHR^{10}$, $-C(O)N(R^{10})R^9$, $-C(S)NHR^{10}$, $-C(S)N(R^{10})R^9$, $-Si(R^{13})(R^{14})R^{12}$, $-P(O)(OR^{22})_2$ or $-P(S)(OR^{22})_2$,
Z represents a halogen atom, cyano, nitro, a $C_1$ to $C_6$ alkyl, a $C_1$ to $C_6$ haloalkyl, a $C_1$ to $C_3$ alkylthio($C_1$ to $C_3$)alkyl, a $C_1$ to $C_3$ haloalkylthio($C_1$ to $C_3$)alkyl, a $C_1$ to $C_3$ alkylsulfinyl($C_1$ to $C_3$)alkyl, a $C_1$ to $C_3$ haloalkylsulfinyl($C_1$ to $C_3$) alkyl, a $C_1$ to $C_3$ alkylsulfonyl($C_1$ to $C_3$)alkyl, a $C_1$ to $C_3$ haloalkylsulfonyl($C_1$ to $C_3$)alkyl, a $C_1$ to $C_6$ alkoxy, a $C_1$ to $C_6$ haloalkoxy, a $C_1$ to $C_3$ haloalkoxy($C_1$ to $C_3$) haloalkoxy, a $C_1$ to $C_6$ alkylsulfonyloxy, a $C_1$ to $C_6$ haloalkylsulfonyloxy, a $C_1$ to $C_6$ alkylthio, a $C_1$ to $C_6$ haloalkylthio, a $C_1$ to $C_6$ alkylsulfinyl, a $C_1$ to $C_6$ haloalkylsulfinyl, a $C_1$ to $C_6$ alkylsulfonyl,

a $C_1$ to $C_6$ haloalkylsulfonyl, $-C(O)NH_2$ or $-C(S)NH_2$, and when p1, p2, p3 or p4 is an integer of 2 or more, each Z may be the same or different from each other,

further, when two Zs are adjacent to each other, the adjacent two Zs may form a 5-membered ring or 6-membered ring with the carbon atoms to which two Zs are bonded by forming $-CF_2CF_2O-$, $-CF_2OCF_2-$, $-OCF_2O-$, $-OCF_2CHFO-$, $-OCF_2CF_2O-$ or -CH=CHCH=CH-,

$R^9$ represents a $C_1$ to $C_6$ alkyl, a $C_1$ to $C_6$ haloalkyl, a $C_3$ to $C_6$ cycloalkyl($C_1$ to $C_4$)alkyl, a phenyl($C_1$ to $C_4$)alkyl, a phenyl($C_1$ to $C_4$) alkyl substituted by $(Z)_{p1}$, a $C_3$ to $C_8$ cycloalkyl, a $C_3$ to $C_8$ halocycloalkyl, phenyl or a phenyl substituted by $(Z)_{p1}$,

$R^{10}$ represents a hydrogen atom or a $C_1$ to $C_6$ alkyl, or $R^9$ and $R^{10}$ are combined to form a $C_4$ to $C_5$ alkylene chain, whereby it may form a 5-membered ring or 6-membered ring with the nitrogen atom to which they are bonded, and at this time, the alkylene chain may contain one oxygen atom or sulfur atom,

$R^{12}$ represents a $C_1$ to $C_6$ alkyl, phenyl or a phenyl substituted by $(Z)_{p1}$,

$R^{13}$ and $R^{14}$ each independently represent a $C_1$ to $C_6$ alkyl,

$R^{15}$ represents a $C_1$ to $C_6$ alkyl, a $C_1$ to $C_6$ haloalkyl, a phenyl($C_1$ to $C_4$)alkyl, a phenyl($C_1$ to $C_4$) alkyl substituted by $(Z)_{p1}$, phenyl or a phenyl substituted by $(Z)_{p1}$,

$R^{16}$ represents a halogen atom, cyano, a $C_3$ to $C_6$ cycloalkyl, $-OR^{26}$, $-N(R^{27})R^{26}$, $-S(O)_rR^{28}$, $-SO_2N(R^{30})R^{29}$, a $C_1$ to $C_6$ alkoxycarbonyl, $-C(O)N(R^{30})R^{29}$, $-C(R^{32})=NOH$, $-C(R^{32})=NOR^{31}$, $-Si(R^{13})(R^{14})R^{12}$, phenyl, a phenyl substituted by $(Z)_{p1}$, L-1, L-2, L-3, L-4, L-45, L-46, L-47 or M,

$R^{17}$ represents a $C_1$ to $C_6$ alkyl, a $C_1$ to $C_6$ alkoxycarbonyl($C_1$ to $C_4$) alkyl or a $C_1$ to $C_6$ alkoxycarbonyl,

$R^{18}$ represents a $C_1$ to $C_6$ alkyl, or $R^{17}$ and $R^{18}$ are combined to form a $C_4$ to $C_5$ alkylene chain whereby it may form a 5-membered ring or 6-membered ring with the nitrogen atom to which they are bonded, and at this time, the alkylene chain may contain one oxygen atom or sulfur atom, and may be optionally substituted by a methyl group or a methoxy group,

$R^{21}$ represents cyano, a $C_3$ to $C_6$ cycloalkyl, a $C_3$ to $C_6$ halocycloalkyl, a $C_1$ to $C_6$ alkoxy, a $C_1$ to $C_6$ haloalkoxy, phenoxy, a phenoxy substituted by $(Z)_{p1}$, a $C_1$ to $C_6$ alkylthio, a $C_1$ to $C_6$ haloalkylthio, phenylthio, a phenylthio substituted by $(Z)_{p1}$, a $C_1$ to $C_6$ alkylsulfinyl, a $C_1$ to $C_6$ haloalkylsulfinyl, a $C_1$ to $C_6$ alkylsulfonyl, a $C_1$ to $C_6$ haloalkylsulfonyl, phenylsulfonyl, a phenylsulfonyl substituted by $(Z)p_1$, a $C_1$ to $C_6$ alkylamino, a di($C_1$ to $C_6$ alkyl)amino, phenylamino, a phenylamino substituted by $(Z)_{p1}$, a $C_1$ to $C_6$ alkoxycarbonyl, phenyl, a phenyl substituted by $(Z)p_1$, L-1 to L-5, L-8 to L-24, L-36, L-39, L-45 to L-52 or L-53,

$R^{22}$ represents a $C_1$ to $C_6$ alkyl,

$R^{23}$ represents a $C_1$ to $C_4$ alkyl, when q1, q2, q3 or q4 is an integer of 2 or more, each $R^{23}$ may be the same or different from each other,

$R^{24}$ represents -CHO, a $C_1$ to $C_6$ alkylcarbonyl, a $C_1$ to $C_6$ alkoxycarbonyl or a $C_1$ to $C_6$ alkylsulfonyl,

$R^{26}$ represents a hydrogen atom, a $C_1$ to $C_6$ alkyl, a $C_1$ to $C_6$ haloalkyl, a $C_1$ to $C_4$ alkoxy($C_1$ to $C_4$)alkyl, a $C_1$ to $C_4$ alkylthio($C_1$ to $C_4$)alkyl, a phenyl($C_1$ to $C_4$)alkyl, a phenyl-($C_1$ to $C_4$) alkyl substituted by $(Z)_{p1}$, a $C_1$ to $C_6$ alkylcarbonyl, a $C_1$ to $C_6$ haloalkylcarbonyl, a $C_3$ to $C_6$ cycloalkylcarbonyl, a $C_1$ to $C_6$ alkoxycarbonyl, $-C(O)N(R^{30})R^{29}$, a $C_1$ to $C_6$ alkylsulfonyl, a di($C_1$ to $C_6$ alkyl)aminosulfonyl, phenylsulfonyl, a phenylsulfonyl substituted by $(Z)_{p1}$, a di($C_1$ to $C_6$ alkyl)phosphoryl, a di($C_1$ to $C_6$ alkyl)thiophosphoryl, a tri($C_1$ to $C_4$ alkyl)-silyl, phenyl or a phenyl substituted by $(Z)_{p1}$,

$R^{27}$ represents a hydrogen atom or a $C_1$ to $C_6$ alkyl,

$R^{28}$ represents a $C_1$ to $C_6$ alkyl, a $C_1$ to $C_6$ haloalkyl, a hydroxy($C_1$ to $C_4$)alkyl, a $C_1$ to $C_4$ alkoxy($C_1$ to $C_4$)alkyl, a $C_1$ to $C_4$ alkylthio($C_1$ to $C_4$)alkyl, a $C_1$ to $C_4$ alkylcarbonyl($C_1$ to $C_4$)alkyl, a $C_1$ to $C_4$ alkoxycarbonyl($C_1$ to $C_4$)alkyl, a $C_1$ to $C_4$ alkylaminocarbonyl($C_1$ to $C_4$)alkyl, a di($C_1$ to $C_4$ alkyl)aminocarbonyl($C_1$ to $C_4$)alkyl, a tri($C_1$ to $C_4$ alkyl) silyl ($C_1$ to $C_4$)alkyl, a phenyl($C_1$ to $C_4$)alkyl, a phenyl($C_1$ to $C_4$) alkyl substituted by $(Z)_{p1}$, a $C_3$ to $C_6$ alkenyl, a $C_3$ to $C_6$ alkynyl, a $C_1$ to $C_6$ alkylthio, phenyl, a phenyl substituted by $(Z)_{p1}$, L-21, L-35, L-45 or L-48,

$R^{29}$ represents a $C_1$ to $C_6$ alkyl, a $C_1$ to $C_4$ alkoxy($C_1$ to $C_4$)alkyl, a $C_1$ to $C_4$ alkylthio($C_1$ to $C_4$)alkyl, a phenyl($C_1$ to $C_4$)alkyl, a phenyl($C_1$ to $C_4$) alkyl substituted by $(Z)_{p1}$, a $C_3$ to $C_6$ cycloalkyl, a $C_3$ to $C_6$ alkenyl, a $C_2$ to $C_6$ alkynyl, phenyl or a phenyl substituted by $(Z)_{p1}$,

$R^{30}$ represents a hydrogen atom or a $C_1$ to $C_6$ alkyl, or $R^{29}$ and $R^{30}$ are combined to form a $C_2$ to $C_5$ alkylene chain, whereby it may form a 3 to 6-membered ring with the nitrogen atom to which they are bonded, and at this time, the alkylene chain may contain one oxygen atom or sulfur atom,

$R^{31}$ represents a $C_1$ to $C_6$ alkyl, a phenyl($C_1$ to $C_4$) alkyl or a phenyl($C_1$ to $C_4$) alkyl substituted by $(Z)_{p1}$,

$R^{32}$ represents a hydrogen atom or a $C_1$ to $C_6$ alkyl,

m is an integer of 0 to 3,

n is an integer of 0 to 3,

q2, q3 and q4 are each independently an integer of 0 to 2

or a salt thereof.

[3] The substituted benzanilide compound according to the above [2], wherein X represents a halogen atom, nitro, a $C_1$ to $C_6$ alkyl, a $C_1$ to $C_6$ haloalkyl, a $C_1$ to $C_6$ alkoxy, a $C_1$ to $C_6$ haloalkoxy, a $C_1$ to $C_6$ alkylsulfonyloxy, a $C_1$ to

$C_6$ alkylthio, a $C_1$ to $C_6$ haloalkylthio, a $C_1$ to $C_6$ alkylsulfinyl, a $C_1$ to $C_6$ haloalkylsulfinyl, a $C_1$ to $C_6$ alkylsulfonyl or a $C_1$ to $C_6$ haloalkylsulfonyl, and when m is 2, each X may be the same or different from each other,

Y represents a halogen atom, a $C_1$ to $C_6$ alkyl, a $C_1$ to $C_6$ haloalkyl, a $C_1$ to $C_6$ alkoxy or a $C_1$ to $C_6$ alkylthio, and when n is 2, each Y may be the same or different from each other,

$R^1$ represents a $C_1$ to $C_8$ alkyl, a ($C_1$ to $C_8$) alkyl optionally substituted by $R^{16}$, a $C_3$ to $C_8$ alkenyl or a $C_3$ to $C_8$ alkynyl,

$R^2$ represents a hydrogen atom or a $C_1$ to $C_6$ alkyl,

$R^3$ represents a hydrogen atom,

$R^4$ represents a $C_1$ to $C_6$ alkyl, a $C_1$ to $C_6$ haloalkyl, a $C_1$ to $C_3$ alkoxy($C_1$ to $C_3$)-haloalkyl, a $C_1$ to $C_3$ alkylthio($C_1$ to $C_3$)haloalkyl, a $C_3$ to $C_6$ cycloalkyl or a $C_3$ to $C_6$ halocycloalkyl,

$R^5$ represents a ($C_1$ to $C_6$) alkyl optionally substituted by $R^{21}$, a ($C_1$ to $C_6$) haloalkyl optionally substituted by $R^{21}$, a ($C_2$ to $C_6$) alkenyl optionally substituted by $R^{21}$, a ($C_2$ to $C_6$) alkynyl optionally substituted by $R^{21}$, a $C_1$ to $C_6$ alkoxycarbonyl, phenyl, a phenyl substituted by $(Z)_{p1}$, a phenoxyphenyl substituted by $(Z)_{p1}$, a pyridyloxyphenyl substituted by $(Z)_{p1}$, L-1 to L-4, L-8 to L-13, L-15 to L-23, L-45 to L-52 or L-53, or may be combined with Y existing at the adjacent position to form a $C_2$ to $C_3$ alkylene chain, whereby it may form a 5 to 6-membered ring which fuses with a benzene ring, and at this time, the alkylene chain may contain one oxygen atom, sulfur atom or nitrogen atom, and may be optionally substituted by a halogen atom,

$R^6$ represents a hydrogen atom, a $C_1$ to $C_6$ alkyl, a $C_1$ to $C_4$ alkoxy($C_1$ to $C_4$)alkyl, a $C_1$ to $C_6$ alkylcarbonyl or a tri ($C_1$ to $C_4$ alkyl)silyl,

$R^{16}$ represents $-OR^{26}$, $-N(R^{27})R^{26}$, $-S(O)_rR^{28}$, $-SO_2N(R^{30})R^{29}$, $-C(R^{32})=NOH$ or $-C(R^{32})=NOR^{31}$,

$R^{21}$ represents a $C_1$ to $C_6$ alkoxy, a $C_1$ to $C_6$ haloalkoxy, phenoxy, a phenoxy substituted by $(Z)_{p1}$, phenylthio, a phenylthio substituted by $(Z)_{p1}$, phenylsulfonyl, a phenylsulfonyl substituted by $(Z)_{p1}$, a $C_1$ to $C_6$ alkylamino, a di($C_1$ to $C_6$ alkyl)amino, phenylamino, a phenylamino substituted by $(Z)_{p1}$, a $C_1$ to $C_6$ alkoxycarbonyl, phenyl, a phenyl substituted by $(Z)_{p1}$, L-1 to L-5, L-8 to L-24, L-36, L-39, L-45 to L-52 or L-53,

$R^{26}$ represents a hydrogen atom, a $C_1$ to $C_6$ alkyl, a $C_1$ to $C_6$ alkylcarbonyl, a $C_1$ to $C_6$ alkoxycarbonyl, a $C_1$ to $C_6$ alkylaminocarbonyl, a di($C_1$ to $C_6$ alkyl)aminocarbonyl or a $C_1$ to $C_6$ alkylsulfonyl,

$R^{28}$ represents a $C_1$ to $C_6$ alkyl,

$R^{29}$ represents a $C_1$ to $C_6$ alkyl,

$R^{30}$ represents a hydrogen atom or a $C_1$ to $C_6$ alkyl,

$R^{31}$ represents a $C_1$ to $C_6$ alkyl,

$R^{32}$ represents a hydrogen atom,

m is an integer of 0 to 2,

n is an integer of 0 to 2

or a salt thereof.

[4] The substituted benzanilide compound according to the above [3], wherein X represents a halogen atom, nitro, a $C_1$ to $C_4$ alkyl, a $C_1$ to $C_4$ haloalkyl, a $C_1$ to $C_4$ alkylthio, a $C_1$ to $C_4$ alkylsulfinyl or a $C_1$ to $C_4$ alkylsulfonyl, and when m is 2, each X may be the same or different from each other,

Y represents a halogen atom or a $C_1$ to $C_4$ alkyl, when n is 2, each Y may be the same or different from each other,

$R^1$ represents a $C_1$ to $C_8$ alkyl, a $C_1$ to $C_4$ alkylthio($C_1$ to $C_4$)alkyl, a $C_1$ to $C_4$ alkylsulfinyl($C_1$ to $C_4$) alkyl or a $C_1$ to $C_4$ alkylsulfonyl($C_1$ to $C_4$)alkyl,

$R^2$ represents a hydrogen atom,

$R^4$ represents a $C_1$ to $C_6$ alkyl or a $C_1$ to $C_6$ haloalkyl,

$R^5$ represents phenyl, a phenyl substituted by $(Z)_{p1}$, a phenoxyphenyl substituted by $(Z)p_1$, a pyridyloxyphenyl substituted by $(Z)_{p1}$, L-1 to L-4, L-8 to L-13, L-15 to L-23, L-45 to L-52 or L-53,

$R^6$ represents a hydrogen atom

or a salt thereof.

[5] An N-substituted phenyl-3-nitrophthalimide or substituted aniline represented by the formula (2) or the formula (3):

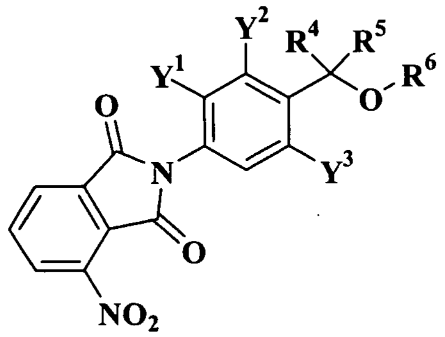

(2)

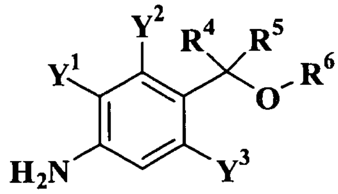

(3)

wherein $Y^1$ represents a hydrogen atom, a halogen atom, a $C_1$ to $C_6$ alkyl, a $C_1$ to $C_6$ haloalkyl, a $C_1$ to $C_6$ alkoxy or a $C_1$ to $C_6$ alkylthio,

$Y^2$ and $Y^3$ each independently represent a hydrogen atom, or may form a $C_2$ to $C_3$ alkylene chain in combination with $R^5$, whereby it may form a 5 to 6-membered ring which fuses with a benzene ring, at this time, the alkylene chain may contain one oxygen atom, sulfur atom or nitrogen atom, and may be optionally substituted by a halogen atom,

$R^4$ represents a $C_1$ to $C_6$ alkyl, a $C_1$ to $C_6$ haloalkyl, a $C_1$ to $C_3$ alkoxy($C_1$ to $C_3$) haloalkyl, a $C_1$ to $C_3$ alkylthio($C_1$ to $C_3$) haloalkyl, a $C_3$ to $C_6$ cycloalkyl or a $C_3$ to $C_6$ halo-cycloalkyl,

$R^5$ represents a ($C_1$ to $C_6$) alkyl optionally substituted by $R^{21}$, a ($C_1$ to $C_6$) haloalkyl optionally substituted by $R^{21}$, a ($C_2$ to $C_6$) alkenyl optionally substituted by $R^{21}$, a ($C_2$ to $C_6$) alkynyl optionally substituted by $R^{21}$, a $C_1$ to $C_6$ alkoxycarbonyl, phenyl, a phenyl substituted by $(Z)_{p1}$, a phenoxyphenyl substituted by $(Z)_{p1}$, a pyridyloxyphenyl substituted by $(Z)_{p1}$, L-1 to L-4, L-8 to L-13, L-15 to L-23, L-45 to L-52 or L-53,

$R^6$ represents a hydrogen atom, a $C_1$ to $C_6$ alkyl, a $C_1$ to $C_4$ alkoxy($C_1$ to $C_4$)alkyl, a $C_1$ to $C_6$ alkylcarbonyl or a tri ($C_1$ to $C_4$ alkyl)silyl,

$R^{21}$ represents a $C_1$ to $C_6$ alkoxy, a $C_1$ to $C_6$ haloalkoxy, phenoxy, a phenoxy substituted by $(Z)_{p1}$, phenylthio, a phenylthio substituted by $(Z)_{p1}$, phenylsulfonyl, a phenylsulfonyl substituted by $(Z)_{p1}$, a $C_1$ to $C_6$ alkylamino, a di($C_1$ to $C_6$ alkyl)amino, phenylamino, a phenylamino substituted by $(Z)_{p1}$, a $C_1$ to $C_6$ alkoxycarbonyl, phenyl, a phenyl substituted by $(Z)_{p1}$, L-1 to L-5, L-8 to L-24, L-36, L-39, L-45 to L-52 or L-53,

L-1 to L-5, L-8 to L-24, L-36, L-39, L-45 to L-52 or L-53 each represent the following aromatic heterocyclic ring,

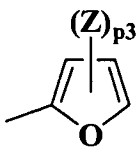

L-1

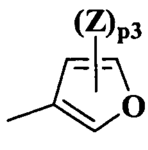

L-2

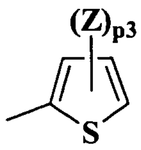

L-3

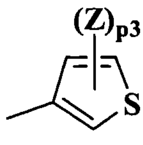

L-4

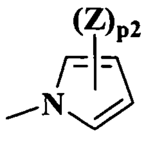

L-5

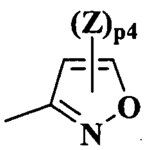

L-8

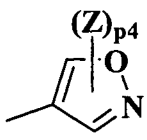

L-9

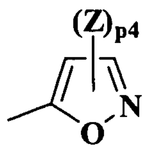

L-10

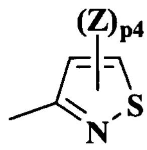

L-11

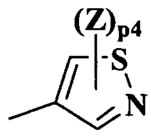

L-12

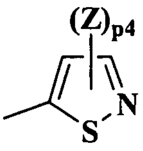 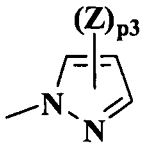 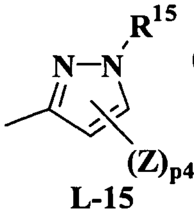 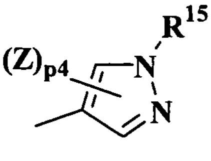 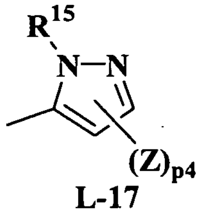

L-13 L-14 L-15 L-16 L-17

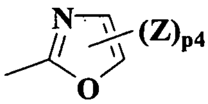 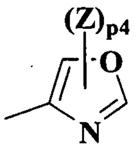 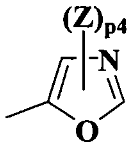 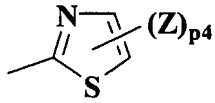 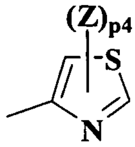

L-18 L-19 L-20 L-21 L-22

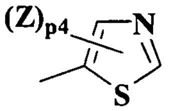 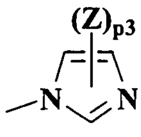 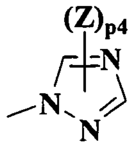 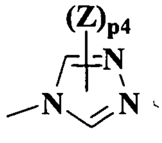

L-23 L-24 L-36 L-39

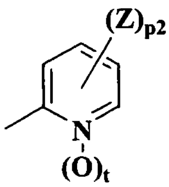 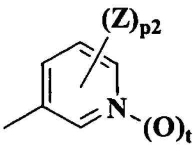 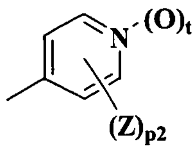 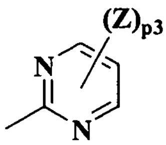 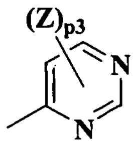

L-45 L-46 L-47 L-48 L-49

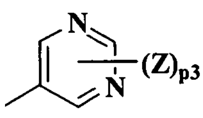 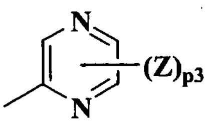 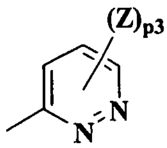 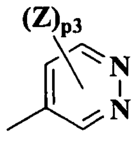

L-50 L-51 L-52 L-53

Z represents a halogen atom, cyano, nitro, a $C_1$ to $C_6$ alkyl, a $C_1$ to $C_6$ haloalkyl, a $C_1$ to $C_3$ alkylthio($C_1$ to $C_3$)alkyl, a $C_1$ to $C_3$ haloalkylthio($C_1$ to $C_3$)alkyl, a $C_1$ to $C_3$ alkylsulfinyl($C_1$ to $C_3$)alkyl, a $C_1$ to $C_3$ haloalkylsulfinyl($C_1$ to $C_3$) alkyl, a $C_1$ to $C_3$ alkylsulfonyl($C_1$ to $C_3$)alkyl, a $C_1$ to $C_3$ haloalkylsulfonyl($C_1$ to $C_3$)alkyl, a $C_1$ to $C_6$ alkoxy, a $C_1$ to $C_6$ haloalkoxy, a $C_1$ to $C_3$ haloalkoxy($C_1$ to $C_3$) haloalkoxy, a $C_1$ to $C_6$ alkylsulfonyloxy, a $C_1$ to $C_6$ haloalkylsulfonyloxy, a $C_1$ to $C_6$ alkylthio, a $C_1$ to $C_6$ haloalkylthio, a $C_1$ to $C_6$ alkylsulfinyl, a $C_1$ to $C_6$ haloalkylsulfinyl, a $C_1$ to $C_6$ alkylsulfonyl, a $C_1$ to $C_6$ haloalkylsulfonyl, $-C(O)NH_2$ or $-C(S)NH_2$, when p1, p2, p3 or p4 is an integer of 2 or more, each Z may be the same or different from each other,

further, when two Zs are adjacent to each other, the adjacent two Zs may form a 5-membered ring or 6-membered ring with the carbon atoms to which two Zs are bonded by forming $-CF_2CF_2O-$ $-CF_2OCF_2-$ $-OCF_2O-$, $-OCF_2CHFO-$, $-OCF_2CF_2O-$or $-CH=CHCH=CH-$,

$R^{15}$ represents a $C_1$ to $C_6$ alkyl, phenyl or a phenyl substituted by $(Z)_{p1}$,
p1 is an integer of 1 to 5,
p2 is an integer of 0 to 4,
p3 is an integer of 0 to 3,
p4 is an integer of 0 to 2,
p5 is an integer of 0 or 1,
r is an integer of 0 to 2,
t is an integer of 0 or 1.]
or a salt thereof.
[6] A noxious organism controlling agent which comprises one or more kinds selected from the substituted benzanilide compound and a salt thereof according to the above [1] to
[4] as an effective ingredient.
[7] An agricultural chemical which comprises one or more kinds selected from the substituted benzanilide compound and a salt thereof according to the above [1] to [4] as an effective ingredient.
[8] An insecticide or araricide which comprises one or more kinds selected from the substituted benzanilide compound and a salt thereof according to the above [1] to [4] as an effective ingredient.

Effects of the invention

Due to use of an insecticide or a fungicide for a long period of time, noxious insects have obtained resistivity thereto in recent years, so that it becomes difficult to prevent them by the conventional insecticides or fungicides. Also, in a part of the insecticides, there exist those having high toxicity or having long residual activity in an environment, so that there is a problem that they disturb an ecological system. On the other hand, the compounds of the present invention have excellent insecticidal and acaricidal activities against many agricultural noxious insects and spider mites, and show sufficient preventing effects against noxious insects which obtained resistivity to the conventional insecticides. Moreover, the compounds do not substantially show bad influence against mammals, fishes and useful insects, and are low residual activity so that load against the environment is low.
Accordingly, the present invention can provide a useful and novel noxious organism controlling agent.

Best mode for carrying out the invention

In the compounds included in the present invention, there are some cases in which geometric isomers of E-isomer and Z-isomer depending on the kind of the substituent(s), and the present invention includes these E-isomer, Z-isomer or a mixture of E-isomer and Z-isomer in an optional ratio. Also, in the compounds contained in the present invention, there exist optical isomers depending on the presence of one or more asymmetric carbon atoms, the present invention includes all the optical isomers or racemic mixtures. Moreover, in the compounds of the present invention represented by the formula (1), when $R^1$ or $R^2$ is a hydrogen atom, it can conceive the presence of tautomeric isomers represented by the following formula, and the present invention also includes these structures.

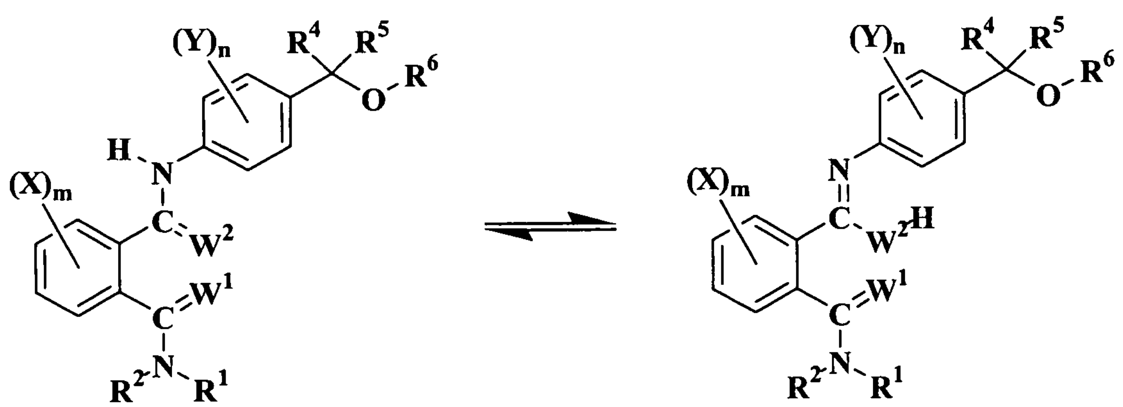

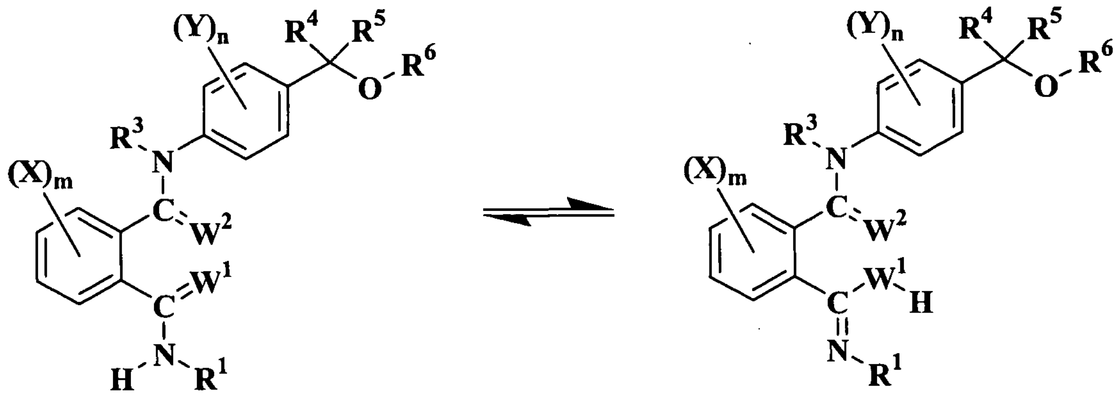

Among the compounds included in the present invention, those which can be an acid addition salt according to the conventional method may include, for example, a salt of a hydrohalogenic acid such as hydrofluoric acid, hydrochloric acid, hydrobromic acid, hydroiodic acid, etc., a salt of an inorganic acid such as nitric acid, sulfuric acid, phosphoric acid, chloric acid, perchloric acid, etc., a salt of a sulfonic acid such as methanesulfonic acid, ethanesulfonic acid, trifluoromethanesulfonic acid, benzenesulfonic acid, p-toluenesulfonic acid, etc., a salt of a carbonic acid such as formic acid, acetic acid, propionic acid, trifluoroacetic acid, fumaric acid, tartaric acid, oxalic acid, maleic acid, malic acid, succinic acid, benzoic acid, mandelic acid, ascorbic acid, lactic acid, gluconic acid, citric acid, etc., or a salt of an amino acid such as glutamic acid, aspartic acid, etc.

Or else, among the compounds included in the present invention, those which can be made a metal salt according to the conventional manner may include, for example, a salt of an alkali metal such as lithium, sodium and potassium, a salt of an alkaline earth metal such as calcium, barium and magnesium or a salt of aluminum.

Next, specific examples of the respective substituent(s) shown in the present specification are shown below. Here, n- means normal, i- means iso, s- means secondary and t- means tertiary, respectively, and Ph means phenyl.

As the halogen atom in the compounds of the present invention, there may bementioned a fluorine atom, a chlorine atom, a bromine atom and an iodine atom. Incidentally, the expression "halo" also means these halogen atoms in the present specification.

The expression $C_a$ to $C_b$ alkyl in the present specification represents a linear or branched hydrocarbon group having a number of a to b carbon atoms, and, for example, specific examples may include a methyl group, ethyl group, n-propyl group, i-propyl group, n-butyl group, s-butyl group, i-butyl group, t-butyl group, n-pentyl group, 1-methylbutyl group, 2-methylbutyl group, 3-methylbutyl group, 1-ethylpropyl group, 1,1-dimethylpropyl group, 1,2-dimethylpropyl group, neopentyl group, n-hexyl group, 1-methylpentyl group, 2-methylpentyl group, 3-methylpentyl group, 4-methylpentyl group, 1-ethylbutyl group, 2-ethylbutyl group, 1,1-dimethylbutyl group, 1,2-dimethylbutyl group, 1,3-dimethylbutyl group, 2,2-dimethylbutyl group, 2,3-dimethylbutyl group, 3,3-dimethylbutyl group, 1,1,2-trimethylpropyl group, 1,2,2-trimethylpropyl group, 1-ethyl-1-methylpropyl group, 1-ethyl-2-methylpropyl group, heptyl group, 1-methylhexyl group, 1,1-dimethylpentyl group, octyl group, 1-methylheptyl group, 1,1-dimethylhexyl group, nonyl group, 1-methyloctyl group, 1,1-dimethylheptyl group, decyl group, 1-methylnonyl group, undecyl group, 1-methyldecyl group, dodecyl group, 1-methylundecyl group, etc., and it can be selected from a designated range of carbon atoms.

The expression $C_a$ to $C_b$ haloalkyl in the present specification represents a linear or branched hydrocarbon group having a to b carbon atoms in which the hydrogen atom bonded to the carbon atom is optionally substituted by a halogen atom, and when it is substituted by 2 or more halogen atoms, these halogen atoms may be the same with each other or may be different from each other. Specific examples may include, for example, a fluoromethyl group, chloromethyl group, bromomethyl group, difluoromethyl group, dichloromethylgroup, trifluoromethyl group, chlorodifluoromethyl group, trichloromethyl group, bromodifluoromethyl group, 2-fluoroethyl group, 1-chloroethyl group, 2-chloroethyl group, 1-bromoethyl group, 2-bromoethyl group, 2,2-difluoroethyl group, 1,2-dichloroethyl group, 2,2-dichloroethyl group, 2-bromo-2-chloroethyl group, 2,2,2-trifluoroethyl group, 2,2,2-trichloroethyl group, 1,1,2,2-tetrafluoroethyl group, 2-chloro-1,1,2-trifluoroethyl group, 2-bromo-1,1,2-trifluoroethyl group, pentafluoroethyl group, 2-chloro-1,1,2,2-tetrafluoroethyl group, 1-chloro-1,2,2,2-tetrafluoroethyl group, 2-bromo-1,1,2,2-tetrafluoroethyl group, 2,2-dichloro-1,1,2-trifluoroethyl group, 2,2,2-trichloro-1,1-difluoroethyl group, 1-chloropropyl group, 2-chloropropyl group, 3-chloropropyl group, 3-bromopropyl group, 2-fluoro-1-methylethyl group, 2-chloro-1-methylethyl group, 2-bromo-1-methylethyl group, 2,2,3,3,3-pentafluoropropyl group, 1,1,2,3,3,3-hexafluoropropyl group, 2,2,2-trifluoro-1-trifluoromethylethyl group, heptafluoropropyl group, 1,2,2,2-tetrafluoro-1-trifluoromethylethyl group, 2-bromo-1,1,2,3,3,3-hexafluoropropyl group, 4-chlorobutyl group, 2-chloro-1,1-dimethylethyl group, 2-bromo-1,1-dimethylethyl group, 3,3,3-trifluoro-1-methylpropyl group, nonafluorobutyl group, 5-chloropentyl group, 2,3-dibromo-1,1-dimethylpropyl group, 6-chlorohexyl group, tridecafluorohexyl group, 7-bromoheptyl group, 8-chlorooctyl group, 9-bromononyl group, 10-chlorodecyl group, 11-bromoundecyl group, 12-bromododecyl group, etc., and each may be selected from the range of the carbon numbers as designated, respectively.

The expression hydroxy($C_a$ to $C_b$) alkyl in the present specification represents a linear or branched alkyl group having a to b carbon atoms in which the hydrogen atom bonded to the carbon atom is optionally substituted by a hydroxyl group, and there may be specifically mentioned, for example, hydroxymethyl group, 1-hydroxyethyl group, 2-hydroxyethyl group, 3-hydroxypropyl group, 2-hydroxy-1-methylethyl group, 4-hydroxybutyl group, 2-hydroxy-1,1-dimethylethyl group, 3-hydroxy-1-methylpropyl group, 6-hydroxyhexyl group, etc., and each may be selected from the range of the carbon numbers as designated, respectively.

The expression cyano($C_a$ to $C_b$) alkyl in the present specification represents a linear or branched alkyl group having a to b carbon atoms in which the hydrogen atom bonded to the carbon atom is optionally substituted by a cyano group, and there may be specifically mentioned, for example, cyanomethyl group, 1-cyanoethyl group, 2-cyanoethyl group, 3-cyanopropyl group, 1-cyano-1-methylethyl group, 4-cyanobutyl group, 2-cyano-1,1-dimethylethyl group, 1-cyano-1-methylpropyl group, 6-cyanohexyl group, etc., and each may be selected from the range of the carbon numbers as designated, respectively.

The expression $C_a$ to $C_b$ cycloalkyl in the present specification represents a cyclic hydrocarbon group having a to b carbon atoms, and may form a monocyclic or heterocyclic structure from a 3-membered ring to a 6-membered ring. Also, respective rings may be optionally substituted by an alkyl group(s) in the range of the designated number of the carbon atoms. There may be specifically mentioned, for example, cyclopropyl group, 1-methylcyclopropyl group, 2-methylcyclopropyl group, 2,2-dimethylcyclopropyl group, 2,2,3,3-tetramethylcyclopropyl group, cyclobutyl group, cyclopentyl group, 1-methylcyclopentyl group, 2-methylcyclopentyl group, 3-methylcyclopentyl group, cyclohexyl group, 1-methylcyclohexyl group, 2-methylcyclohexyl group, 3-methylcyclohexyl group, 4-methylcyclohexyl group, bicyclo [2.2.1]heptan-2-yl group, etc., and each may be selected from the range of the carbon numbers as designated, respectively.

The expression $C_a$ to $C_b$ halocycloalkyl in the present specification represents a cyclic hydrocarbon group having a to b carbon atoms in which the hydrogen atom bonded to the carbon atom is optionally substituted by a halogen atom, and may form a monocyclic or heterocyclic structure from a 3-membered ring to a 6-membered ring. Also, respective rings may be optionally substituted by an alkyl group(s) in the range of the designated number of the carbon atoms, substitution by the halogen atom may be at the ring structure portion, a side chain portion, or may be both of the portions, and further, when it is substituted by 2 or more halogen atoms, these halogen atoms may be the same with each other or may be different from each other. There may be specifically mentioned, for example, 1-bromocyclopropyl group, 2,2-dichlorocyclopropyl group, 2,2-dibromocyclopropyl group, 2,2-difluoro-1-methylcyclopropyl group, 2,2-dichloro-1-methylcyclopropyl group, 2,2-dibromo-1-methylcyclopropyl group, 2,2-dichloro-3,3-dimethylcyclopropyl group, 2,2,3,3-tetrafluorocyclobutyl group, 2-fluoro-cyclohexyl group, 2-chlorocyclohexyl group, 3-chlorocyclohexyl group, 4-chlorocyclohexyl group, 2-trifluoromethylcyclohexyl group, 3-trifluoromethylcyclohexyl group, 4-trifluoromethylcyclohexyl group, etc., and each may be selected from the range of the carbon numbers as designated, respectively.

The expression $C_a$ to $C_b$ alkenyl in the present specification represents an unsaturated hydrocarbon group which is linear or branched having a to b carbon atoms, and having one or more double bonds in the molecule, and there may be specifically mentioned, for example, vinyl group, 1-propenyl group, 1-methylethenyl group, 2-propenyl group, 1-butenyl group, 1-methyl-1-propenyl group, 2-methyl-1-propenyl group, 2-butenyl group, 1-methyl-2-propenyl group, 2-methyl-2-propenyl group, 3-butenyl group, 1,3-butadienyl, group, 1-methyl-2-butenyl group, 2-methyl-2-butenyl group, 3-methyl-2-butenyl group, 1,1-dimethyl-2-propenyl group, 2-hexenyl group, 2-methyl-2-pentenyl group, 1,3-dimethyl-2-butenyl group, 1,1,2-trimethyl-2-propenyl group, 1,1-dimethyl-3-butenyl group, 2,4-hexadienyl group, 2-heptenyl group, 1,1-dimethyl-4-pentenyl group, 2-octenyl group, 1-methyl-2-heptenyl group, 2-undecenyl group, 10-undecenyl group, 2-dodecenyl group, 11-dodecenyl group, etc., and each may be selected from the range of the carbon numbers as designated, respectively.

The expression $C_a$ to $C_b$ haloalkenyl in the present specification represents an unsaturated hydrocarbon group which is linear or branched having a to b carbon atoms, and having one or more double bonds in the molecule in which the hydrogen atom bonded to the carbon atom is optionally substituted by a halogen atom. At this time, when it is substituted by 2 or more halogen atoms, these halogen atoms may be the same with each other or may be different from each other. There may be specifically mentioned, for example, 2-chlorovinyl group, 2-bromovinyl group, 2,2-dichlorovinyl group, 2,2-dibromovinyl group, 3-bromo-2-propenyl group, 1-chloromethylvinyl group, 2-bromo-1-methylvinyl group, 1-trifluoromethylvinyl group, 2-chloro-3,3,3-trifluoro-1-propenyl group, 1-trifluoromethyl-2,2-difluorovinyl group, 2-chloro-2-propenyl group, 3,3-difluoro-2-propenyl group, 3,3-dichloro-2-propenyl group, 2,3,3-trifluoro-2-propenyl group, 2,3,3-trichloro-2-propenyl group, 4,4-difluoro-3-butenyl group, 3,4,4-trifluoro-3-butenyl group, 3-chloro-4,4,4-trifluoro-2-butenyl group, 3,3,3-trifluoro-1-methyl-1-propenyl group, 3,3,3-trifluoro-2-trifluoromethyl-1-propenyl group, 1,3,3,3-tetrafluoro-2-trifluoromethyl-1-propenyl group, 3,3,4,4,5,5,5-heptafluoro-1-pentenyl group, 5,5-difluoro-4-pentenyl group, 4,5,5-trifluoro-4-pentenyl group, 3,4,4,4-tetrafluoro-3-trifluoromethyl-1-butenyl group, 4,4,5,5,6,6,6-heptafluoro-2-hexenyl group, 3,4,4,5,5,5-hexafluoro-3-trifluoromethyl-1-pentenyl group, 2-perfluorohexylethenyl group, etc.,

and each may be selected from the range of the carbon numbers as designated, respectively.

The expression $C_a$ to $C_b$ cycloalkenyl in the present specification represents a cyclic unsaturated hydrocarbon group having a to b carbon atoms and having 1 or more double bonds, and may form a monocyclic or heterocyclic structure from a 3-membered ring to a 6-membered ring. Also, respective rings may be optionally substituted by an alkyl group(s) in the range of the designated number of the carbon atoms, and the double bond may be either of the endo- or exo-form. There may be specifically mentioned, for example, cyclopenten-1-yl group, 2-cyclopenten-1-yl group, 3-cyclopenten-1-yl group, cyclohexen-1-yl group, 2-cyclohexen-1-yl group, 3-cyclohexen-1-yl group, 2-methyl-2-cyclohexen-1-yl group, 3-methyl-2-cyclohexen-1-yl group, bicyclo-[2.2.1]-5-hepten-2-yl group, etc., and each may be selected from the range of the carbon numbers as designated, respectively.

The expression $C_a$ to $C_b$ halocycloalkenyl in the present specification represents a cyclic unsaturated hydrocarbon group having a to b carbon atoms and having 1 or more double bonds in which the hydrogen atom bonded to the carbon atom is optionally substituted by a halogen atom, and may form a monocyclic or heterocyclic structure from a 3-membered ring to a 6-membered ring. Also, the respective rings may be optionally substituted by an alkyl group(s) in the range of the designated number of the carbon atoms, and the double bond may be either of the endo- or exo-form. Also, substitution by the halogen atom may be at the ring structure portion, a side chain portion, or may be both of the portions, and further, when it is substituted by 2 or more halogen atoms, these halogen atoms may be the same with each other or may be different from each other. There may be specifically mentioned, for example, 2-chlorobicyclo [2.2.1]-5-hepten-2-yl group, etc., and each may be selected from the range of the carbon numbers as designated, respectively.

The expression $C_a$ to $C_b$ alkynyl in the present specification represents a linear or branched unsaturated hydrocarbon group having one or more triple bonds in the molecule with a to b carbon atoms, and there may be specifically mentioned, for example, ethynyl group, 1-propynyl group, 2-propynyl group, 1-methyl-2-propynyl group, 2-butynyl group, 3-butynyl group, 2-pentynyl group, 1-methyl-2-butynyl group, 1-methyl-3-butynyl group, 1,1-dimethyl-2-propynyl group, 1-hexynyl group, 3,3-dimethyl-1-butynyl group, 2-hexynyl group, 1-methyl-2-pentynyl group, 1,1-dimethyl-2-butynyl group, 2-heptynyl group, 1,1-dimethyl-2-pentynyl group, 2-octynyl group, 2-nonynyl group, 2-decynyl group, 2-undecynyl group, etc., and each may be selected from the range of the carbon numbers as designated, respectively.

The expression $C_a$ to $C_b$ haloalkynyl in the present specification represents a linear or branched unsaturated hydrocarbon group having one or more triple bonds in the molecule with a to b carbon atoms in which the hydrogen atom bonded to the carbon atom is optionally substituted by a halogen atom. At this time, when it is substituted by two or more halogen atoms, these halogen atoms may be the same with each other or may be different from each other. There may be specifically mentioned, for example, 2-chloroethynyl group, 2-bromoethynyl group, 2-iodoethynyl group, 3-chloro-2-propynyl group, 3-bromo-2-propynyl group, 3-iodo-2-propynyl group, 3,3,3-trifluoro-1-propynyl group, 3-chloro-1-methyl-2-propynyl group, 3-bromo-1-methyl-2-propynyl group, 3-iodo-1-methyl-2-propynyl group, 3-chloro-1,1-dimethyl-2-propynyl group, 3-bromo-1,1-dimethyl-2-propynyl group, 3-iodo-1,1-dimethyl-2-propynyl group, etc., and each may be selected from the range of the carbon numbers as designated, respectively.

The expression $C_a$ to $C_b$ alkoxy in the present specification represents an alkyl-O- group having the above-mentioned meaning with a to b carbon atoms, and there may be specifically mentioned, for example, methoxy group, ethoxy group, n-propyloxy group, i-propyloxy group, n-butyloxy group, s-butyloxy group, i-butyloxy group, t-butyloxy group, n-pentyloxy group, 1-methylbutyloxy group, 2-methylbutyloxy group, 3-methylbutyloxy group, 1-ethylpropyloxy group, 1,1-dimethylpropyloxy group, 1,2-dimethylpropyloxy group, neopentyloxy group, n-hexyloxy group, 1,1-dimethylbutyloxy group, etc., and each may be selected from the range of the carbon numbers as designated, respectively.

The expression $C_a$ to $C_b$ haloalkoxy in the present specification represents a haloalkyl-O- group having the above-mentioned meaning with a to b carbon atoms, and there may be specifically mentioned, for example, difluoromethoxy group, trifluoromethoxy group, chlorodifluoromethoxy group, bromodifluoromethoxy group, 2-fluoroethoxy group, 2-chloroethoxy group, 2,2,2-trifluoroethoxy group, 1,1,2,2,-tetrafluoroethoxy group, 2-chloro-1,1,2-trifluoroethoxy group, 2-bromo-1,1,2-trifluoroethoxy group, pentafluoroethoxy group, 2-bromo-1,1,2,2-tetrafluoroethoxy group, 2,2-dichloro-1,1,2-trifluoroethoxy group, 2,2,2-trichloro-1,1-difluoroethoxy group, 2-chloropropyloxy group, 3-chloropropyloxy group, heptafluoropropyloxy group, 2,2,2-trifluoro-1-trifluoromethylethoxy group, 2,2,3,3-tetrafluoropropyloxy group, 1,1,2,3,3,3-hexafluoropropyloxy group, 2-bromo-1,1,2,3,3,3-hexafluoropropyloxy group, etc., and each may be selected from the range of the carbon numbers as designated, respectively.

The expression $C_a$ to $C_b$ alkenyloxy in the present specification represents an alkenyl-O- group having the above-mentioned meaning with a to b carbon atoms, and there may be specifically mentioned, for example, 2-propenyloxy group, 2-butenyloxy group, 2-methyl-2-propenyloxy group, 3-methyl-2-butenyloxy group, etc., and each may be selected from the range of the carbon numbers as designated, respectively.

The expression $C_a$ to $C_b$ haloalkenyloxy in the present specification represents a haloalkenyl-O- group having the above-mentioned meaning with a to b carbon atoms, and there may be specifically mentioned, for example, 2-chloro-2-propenyl group, 3-chloro-2-propenyl group, 3,3-difluoro-2-propenyl group, 3,3-dichloro-2-propenyl group, 2,3,3-trifluoro-2-propenyl group, etc., and each may be selected from the range of the carbon numbers as designated, respectively.

The expression $C_a$ to $C_b$ alkylthio in the present specification represents an alkyl-S- group having the above-mentioned meaning with a to b carbon atoms, and there may be specifically mentioned, for example, methylthio group, ethylthio group, n-propylthio group, i-propylthio group, n-butylthio group, s-butylthio group, i-butylthio group, t-butylthio group, n-pentylthio group, 1-methylbutylthio group, 2-methylbutylthio group, 3-methylbutylthio group, 1-ethylpropylthio group, 1,1-dimethylpropylthio group, 1,2-dimethylpropylthio group, neopentylthio group, n-hexylthio group, etc., and each may be selected from the range of the carbon numbers as designated, respectively.

The expression $C_a$ to $C_b$ haloalkylthio in the present specification represents a haloalkyl-S- group having the above-mentioned meaning with a to b carbon atoms, and there may be specifically mentioned, for example, difluoromethylthio group, trifluoromethylthio group, bromodifluoromethylthio group, 2,2,2-trifluoroethylthio group, 1,1,2,2-tetrafluoroethylthio group, 1,1,2-trifluoro-2-chloroethylthio group, pentafluoroethylthio group, 2-bromo-1,1,2,2-tetrafluoroethylthio group, heptafluoropropylthio group, 1,2,2,2-tetrafluoro-1-trifluoromethylethylthio group, nonafluorobutylthio group, etc., and each may be selected from the range of the carbon numbers as designated, respectively.

The expression $C_a$ to $C_b$ alkylsulfinyl in the present specification represents an alkyl-S(O)- group having the above-mentioned meaning with a to b carbon atoms, and there may be specifically mentioned, for example, methylsulfinyl group, ethylsulfinyl group, n-propylsulfinyl group, i-propylsulfinyl group, n-butylsulfinyl group, s-butylsulfinyl group, i-butylsulfinyl group, t-butylsulfinyl group, etc., and each may be selected from the range of the carbon numbers as designated, respectively.

The expression $C_a$ to $C_b$ haloalkylsulfinyl in the present specification represents a haloalkyl-S(O)- group having the above-mentioned meaning with a to b carbon atoms, and there may be specifically mentioned, for example, difluoromethylsulfinyl group, trifluoromethylsulfinyl group, bromodifluoromethylsulfinyl group, 2,2,2-trifluoroethylsulfinyl group, 2-bromo-1,1,2,2-tetrafluoroethylsulfinyl group, 1,2,2,2-tetrafluoro-1-trifluoromethylethylsulfinyl group, nonafluorobutylsulfinyl group, etc., and each may be selected from the range of the carbon numbers as designated, respectively.

The expression $C_a$ to $C_b$ alkylsulfonyl in the present specification represents an alkyl-$SO_2$- group having the above-mentioned meaning with a to b carbon atoms, and there may be specifically mentioned, for example, methanesulfonyl group, ethanesulfonyl group, n-propylsulfonyl group, i-propylsulfonyl group, n-butylsulfonyl group, s-butylsulfonyl group, i-butylsulfonyl group, t-butylsulfonyl group, n-pentylsulfonyl group, n-hexylsulfonyl group, etc., and each may be selected from the range of the carbon numbers as designated, respectively.

The expression $C_a$ to $C_b$ haloalkylsulfonyl in the present specification represents a haloalkyl-$SO_2$- group having the above-mentioned meaning with a to b carbon atoms, and there may be specifically mentioned, for example, difluoromethanesulfonyl group, trifluoromethanesulfonyl group, chlorodifluoromethanesulfonyl group, bromodifluoromethanesulfonyl group, 2,2,2-trifluoroethanesulfonyl group, 1,1,2,2-tetrafluoroethanesulfonyl group, 1,1,2-trifluoro-2-chloroethanesulfonyl group, etc., and each may be selected from the range of the carbon numbers as designated, respectively.

The expression $C_a$ to $C_b$ alkylamino in the present specification represents an amino group in which either one of the hydrogen atoms is substituted by the alkyl group having the above-mentioned meaning with a to b carbon atoms, and there may be specifically mentioned, for example, methylamino group, ethylamino group, n-propylamino group, i-propylamino group, n-butylamino group, i-butylamino group, t-butylamino group, etc., and each may be selected from the range of the carbon numbers as designated, respectively.

The expression di($C_a$ to $C_b$ alkyl)amino in the present specification represents an amino group in which both of the hydrogen atoms are substituted by the alkyl group having the above-mentioned meaning with a to b carbon atoms, which may be the same with each other or may be different from each other, and there may be specifically mentioned, for example, dimethylamino group, ethyl(methyl)amino group, diethylamino group, n-propyl(methyl)amino group, i-propyl (methyl)amino group, a di(n-propyl)amino group, n-butyl(methyl)amino group, i-butyl(methyl)amino group, t-butyl(methyl) amino group, etc., and each may be selected from the range of the carbon numbers as designated, respectively.

The expression $C_a$ to $C_b$ alkylcarbonyl in the present specification represents an alkyl-C(O)- group having the above-mentioned meaning with a to b carbon atoms, and there may be specifically mentioned, for example, $CH_3C(O)$- group, $CH_3CH_2C(O)$-group, a $CH_3CH_2CH_2C(O)$- group, $(CH_3)_2CHC(O)$- group, $CH_3(CH_2)_3C(O)$- group, $(CH_3)_2CHCH_2C(O)$- group, $CH_3CH_2CH(CH_3)C(O)$- group, $(CH_3)_3CC(O)$- group, $CH_3(CH_2)_4C(O)$- group, $CH_3(CH_2)_5C(O)$- group, etc., and each may be selected from the range of the carbon numbers as designated, respectively.

The expression $C_a$ to $C_b$ haloalkylcarbonyl in the present specification represents a haloalkyl-C(O)- group having the above-mentioned meaning with a to b carbon atoms, and there may be specifically mentioned, for example, $FCH_2C(O)$- group, $ClCH_2C(O)$- group, $F_2CHC(O)$- group, $Cl_2CHC(O)$- group, $CF_3C(O)$- group, $ClCF_2C(O)$- group, $BrCF_2C(O)$- group, $CCl_3C(O)$- group, $CF_3CF_2C(O)$- group, $ClCH_2CH_2CH_2C(O)$-group, $CF_3CF_2CF_2C(O)$- group, $ClCH_2C(CH_3)_2C(O)$- group, etc., and each may be selected from the range of the carbon numbers as designated, respectively.

The expression $C_a$ to $C_b$ cycloalkylcarbonyl in the present specification represents a cycloalkyl-C(O)- group having the above-mentioned meaning with a to b carbon atoms, and there may be specifically mentioned, for example, cyclopropyl-C(O)-group, 1-methylcyclopropyl-C(O)- group, 2-methylcyclopropyl-C(O)- group, 2,2-dimethylcyclopropyl-C(O)- group, cyclobutyl-C(O)- group, cyclopentyl-C(O)- group, cyclohexyl-C(O)- group, etc., and each may be selected

from the range of the carbon numbers as designated, respectively.

The expression $C_a$ to $C_b$ alkoxycarbonyl in the present specification represents an alkyl-O-C(O)- group having the above-mentioned meaning with a to b carbon atoms, and there may be specifically mentioned, for example, $CH_3OC(O)$- group, $CH_3CH_2OC(O)$- group, $CH_3CH_2CH_2OC(O)$- group, $(CH_3)_2CHOC(O)$- group, $CH_3(CH_2)_3OC(O)$- group, $(CH_3)_2CHCH_2OC(O)$- group, $(CH_3)_3COC(O)$- group, etc., and each may be selected from the range of the carbon numbers as designated, respectively.

The expression $C_a$ to $C_b$ haloalkoxycarbonyl in the present specification represents a haloalkyl-O-C(O)- group having the above-mentioned meaning with a to b carbon atoms, and there may be specifically mentioned, for example, $ClCH_2CH_2OC(O)$- group, $CF_3CH_2OC(O)$- group, etc., and each may be selected from the range of the carbon numbers as designated, respectively.

The expression $C_a$ to $C_b$ alkylthiocarbonyl in the present specification represents an alkyl-S-C(O)- group having the above-mentioned meaning with a to b carbon atoms, and there may be specifically mentioned, for example, $CH_3SC(O)$- group, $CH_3CH_2SC(O)$- group, $CH_3CH_2CH_2SC(O)$- group, $(CH_3)_2CHSC(O)$- group, $CH_3(CH_2)_3SC(O)$- group, $(CH_3)_2CHCH_2SC(O)$- group, $(CH_3)_3CSC(O)$- group, etc., and each may be selected from the range of the carbon numbers as designated, respectively.

The expression $C_a$ to $C_b$ alkylthiocarbonyl in the present specification represents a alkyl-O-C(S)- group having the above-mentioned meaning with a to b carbon atoms, and there may be specifically mentioned, for example, $CH_3OC(S)$- group, $CH_3CH_2OC(S)$ group, $CH_3CH_2CH_2OC(S)$- group, $(CH_3)_2CHOC(S)$- group, $CH_3(CH_2)_3OC(S)$- group, $(CH_3)_2CHCH_2OC(S$ group, $(CH_3)_3COC(S)$- group, etc., and each may be selected from the range of the carbon numbers as designated, respectively.

The expression $C_a$ to $C_b$ alkylaminocarbonyl in the present specification represents a carbamoyl group in which either one of the hydrogen atoms is substituted by the alkyl group having the above-mentioned meaning with a to b carbon atoms, and there may be specifically mentioned, for example, $CH_3NHC(O)$- group, $CH_3CH_2NHC(O)$-group, $CH_3CH_2CH_2NHC(O)$- group, $(CH_3)_2CHNHC(O)$- group, $CH_3(CH_2)_3NHC(O)$- group, $(CH_3)_2CHCH_2NHC(O)$- group, $CH_3CH_2CH(CH_3)NHC(O)$- group, $(CH_3)_3CNHC(O)$- group, etc., and each may be selected from the range of the carbon numbers as designated, respectively.

The expression di($C_a$ to $C_b$ alkyl)aminocarbonyl in the present specification represents a carbamoyl group in which both of the hydrogen atoms are substituted by the alkyl group having the above-mentioned meaning with a to b carbon atoms, which may be the same with each other or may be different from each other, and there may be specifically mentioned, for example, $(CH_3)_2NC(O)$- group, $CH_3CH_2N(CH_3)C(O)$- group, $(CH_3CH_2)_2NC(O)$- group, $(CH_3CH_2CH_2)_2NC(O)$- group, $(CH_3CH_2CH_2CH_2)_2NC(O)$- group, etc., and each may be selected from the range of the carbon numbers as designated, respectively.

The expression $C_a$ to $C_b$ alkylaminothiocarbonyl in the present specification represents a thiocarbamoyl group in which either one of the hydrogen atoms is substituted by the alkyl group having the above-mentioned meaning with a to b carbon atoms, and there may be specifically mentioned, for example, $CH_3NHC(S)$- group, $CH_3CH_2NHC(S)$- group, $CH_3CH_2CH_2NHC(S)$- group, $(CH_3)_2CHNHC(S)$- group, $CH_3(CH_2)_3NHC(S)$- group, $(CH_3)_2CHCH_2NHC(S)$- group, $CH_3CH_2CH(CH_3)NHC(S)$-group, $(CH_3)_3CNHC(S)$- group, etc., and each may be selected from the range of the carbon numbers as designated, respectively.

The expression di($C_a$ to $C_b$ alkyl)aminothiocarbonyl in the present specification represents a thiocarbamoyl group in which both of the hydrogen atoms are substituted by the alkyl group having the above-mentioned meaning with a to b carbon atoms, which may be the same with each other or may be different from each other, and there may be specifically mentioned, for example, $(CH_3)_2NC(S)$- group, $CH_3CH_2N(CH_3)C(S)$-group, $(CH_3CH_2)_2NC(S)$- group, $(CH_3CH_2CH_2)_2NC(S)$- group, $(CH_3CH_2CH_2CH_2)_2NC(S)$-group, etc., and each may be selected from the range of the carbon numbers as designated, respectively.

The expression $C_a$ to $C_b$ alkylaminosulfonyl in the present specification represents a sulfamoyl group in which either one of the hydrogen atoms is substituted by the alkyl group having the above-mentioned meaning with a to b carbon atoms, and there may be specifically mentioned, for example, $CH_3NHSO_2$- group, $CH_3CH_2NHSO_2$-group, $CH_3CH_2CH_2NHSO_2$- group, $(CH_3)_2CHNHSO_2$- group, $CH_3(CH_2)_3NHSO_2$- group, $(CH_3)_2CHCH_2NHSO_2$- group, $CH_3CH_2CH(CH_3)NHSO_2$- group, $(CH_3)_3CNHSO_2$- group, etc., and each may be selected from the range of the carbon numbers as designated, respectively.

The expression di($C_a$ to $C_b$ alkyl)aminosulfonyl in the present specification represents a sulfamoyl group in which both of the hydrogen atoms are substituted by the alkyl group having the above-mentioned meaning with a to b carbon atoms, which may be the same with each other or may be different from each other, and there may be specifically mentioned, for example, $(CH_3)_2NSO_2$- group, $CH_3CH_2N(CH_3)SO_2$- group, $(CH_3CH_2)_2NSO_2$- group, $(CH_3CH_2CH_2)_2NSO_2$- group, $(CH_3CH_2CH_2CH_2)_2NSO_2$- group, etc., and each may be selected from the range of the carbon numbers as designated, respectively.

The expression di($C_a$ to $C_b$ alkyl)phosphoryl in the present specification represents a phosphoryl group in which both of the hydrogen atoms are substituted by the alkyl group having the above-mentioned meaning with a to b carbon

atoms, which may be the same with each other or may be different from each other, and there may be specifically mentioned, for example, $(CH_3O)_2P(O)$- group, $(CH_3CH_2O)_2P(O)$- group, etc., and each may be selected from the range of the carbon numbers as designated, respectively.

The expression di($C_a$ to $C_b$ alkyl)thiophosphoryl in the present specification represents a thiophosphoryl group in which both of the hydrogen atoms are substituted by the alkyl group having the above-mentioned meaning with a to b carbon atoms, which may be the same with each other or may be different from each other, and there may be specifically mentioned, for example, $(CH_3O)_2P(S)$- group, $(CH_3CH_2O)_2P(S)$-group, etc., and each may be selected from the range of the carbon numbers as designated, respectively.

The expression tri($C_a$ to $C_b$ alkyl)silyl in the present specification represents a silyl group which is substituted by the alkyl group(s) having the above-mentioned meaning with a to b carbon atoms, which may be the same with each other or may be different from each other, and there may be specifically mentioned, for example, trimethylsilyl group, triethylsilyl group, tri(n-propyl)silyl group, ethyldimethylsilyl group, n-propyldimethylsilyl group, n-butyldimethylsilyl group, i-butyldimethylsilyl group, t-butyldimethylsilyl group, etc., and each may be selected from the range of the carbon numbers as designated, respectively.

The expression $C_a$ to $C_b$ alkylsulfonyloxy in the present specification represents an alkyl-$SO_2$-O- group having the above-mentioned meaning with a to b carbon atoms, and there may be specifically mentioned, for example, $CH_3SO_2$-O- group, $CH_3CH_2SO_2$-O- group, $CH_3CH_2CH_2SO_2$-O- group, $(CH_3)_2CHSO_2$-O- group, $CH_3(CH_2)_3SO_2$-O- group, $(CH_3)_2CHCH_2SO_2$-O- group, $CH_3CH_2CH(CH_3)SO_2$-O- group, $(CH_3)_3CSO_2$-O- group, etc., and each may be selected from the range of the carbon numbers as designated, respectively.

The expression $C_a$ to $C_b$ haloalkylsulfonyloxy in the present specification represents a haloalkylsulfonyl-O-group having the above-mentioned meaning with a to b carbon atoms, and there may be specifically mentioned, for example, $CF_3SO_2$-O-group, $CF_3CF_2SO_2$-O- group, etc., and each may be selected from the range of the carbon numbers as designated, respectively.

The expression $C_a$ to $C_b$ alkylsulfonylamino in the present specification represents a alkylsulfonyl-NH- group having the above-mentioned meaning with a to b carbon atoms, and there may be specifically mentioned, for example, $CH_3SO_2$-NH- group, a $CH_3CH_2SO_2$-NH- group, a $CH_3CH_2CH_2SO_2$-NH- group, $(CH_3)_2CHSO_2$-NH- group, a $CH_3(CH_2)_3SO_2$-NH- group, $(CH_3)_2CHCH_2SO_2$-NH- group, a $CH_3CH_2CH(CH_3)SO_2$-NH group, $(CH_3)_3CSO_2$-NH- group, etc., and each may be selected from the range of the carbon numbers as designated, respectively.

The expression $C_a$ to $C_b$ haloalkylsulfonylamino in the present specification represents a haloalkylsulfonyl-NH-group having the above-mentioned meaning with a to b carbon atoms, and there may be specifically mentioned, for example, $CF_3SO_2$-NH- group, a $CF_3CF_2SO_2$-NH- group, etc., and each may be selected from the range of the carbon numbers as designated, respectively.

The expression $C_a$ to $C_b$ cycloalkyl ($C_d$ to $C_e$)alkyl, a $C_a$ to $C_b$ alkoxy($C_d$ to $C_e$)alkyl, a $C_a$ to $C_b$ haloalkoxy($C_d$ to $C_e$)alkyl, a $C_a$ to $C_b$ alkylthio ($C_d$ to $C_e$)alkyl, a $C_a$ to $C_b$ haloalkylthio ($C_d$ to $C_e$)alkyl, a $C_a$ to $C_b$ alkylsulfinyl($C_d$ to $C_e$) alkyl, a $C_a$ to $C_b$ haloalkylsulfinyl-($C_d$ to $C_e$)alkyl, a $C_a$ to $C_b$ alkylsulfonyl($C_d$ to $C_e$)alkyl, a $C_a$ to $C_b$ haloalkylsulfonyl($C_d$ to $C_e$)alkyl, a $C_a$ to $C_b$ alkylcarbonyl($C_d$ to $C_e$)alkyl, a $C_a$ to $C_b$ haloalkylcarbonyl($C_d$ to $C_e$)alkyl, a $C_a$ to $C_b$ alkoxycarbonyl ($C_d$ to $C_e$)alkyl, a $C_a$ to $C_b$ haloalkoxycarbonyl($C_d$ to $C_e$)alkyl, a $C_a$ to $C_b$ alkylaminocarbonyl($C_d$ to $C_e$)alkyl, a di($C_a$ to $C_b$ alkyl)aminocarbonyl($C_d$ to $C_e$)alkyl, tri($C_a$ to $C_b$ alkyl)silyl ($C_d$ to $C_e$)alkyl, a phenyl($C_d$ to $C_e$)alkyl, a phenyl($C_d$ to $C_e$) alkyl substituted by $(Z)_{p1}$, L-($C_d$ to $C_e$) alkyl or M-($C_d$ to $C_e$)alkyl, etc., in the present specification each represent a linear or branched hydrocarbon group having d to e carbon atoms in which the hydrogen atom(s) bonded to the carbon atom (s) is/are optionally substituted by the optional $C_a$ to $C_b$cyclo alkyl group, a $C_a$ to $C_b$ alkoxy group, a $C_a$ to $C_b$ haloalkoxy group, a $C_a$ to $C_b$ alkylthio group, a $C_a$ to $C_b$ haloalkylthio group, a $C_a$ to $C_b$ alkylsulfinyl group, a $C_a$ to $C_b$ haloalkylsulfinyl group, a $C_a$ to $C_b$ alkylsulfonyl group, a $C_a$ to $C_b$ haloalkylsulfonyl group, a $C_a$ to $C_b$ alkylcarbonyl group, a $C_a$ to $C_b$ haloalkylcarbonyl group, a $C_a$ to $C_b$ alkoxycarbonyl group, a $C_a$ to $C_b$ haloalkoxycarbonyl group, a $C_a$ to $C_b$ alkylamino-carbonyl group, a di($C_a$ to $C_b$ alkyl)aminocarbonyl group, tri($C_a$ to $C_b$ alkyl)silyl group, a phenyl group, a phenyl group substituted by $(Z)_{p1}$, L group or M group, each of which have the above-mentioned meanings, and each may be selected from the range of the carbon numbers as designated, respectively.

The expression ($C_a$ to $C_b$) alkyl optionally substituted by $R^7$, ($C_a$ to $C_b$) alkyl optionally substituted by $R^{16}$, ($C_a$ to $C_b$) alkyl optionally substituted by $R^{21}$, ($C_a$ to $C_b$) alkyl optionally substituted by $R^{25}$ or ($C_a$ to $C_b$) alkyl optionally substituted by $R^{33}$ in the present specification represent a linear or branched hydrocarbon group having a to b carbon atoms in which the hydrogen atom(s) bonded to the carbon atom(s) is/are optionally substituted by an optional $R^7$, $R^{16}$, $R^{21}$, $R^{25}$ or $R^{33}$, and each may be selected from the range of the carbon numbers as designated, respectively. At this time, when two or more substituent(s) $R^7$, $R^{16}$, $R^{21}$, $R^{25}$ or $R^{33}$ exist on the respective ($C_a$ to $C_b$) alkyl group, the respective $R^7$, $R^{16}$, $R^{21}$, $R^{25}$ or $R^{33}$ may be the same with each other or may be different from each other.

The expression hydroxy($C_d$ to $C_e$)haloalkyl, $C_a$ to $C_b$ alkoxy($C_d$ to $C_e$)haloalkyl or $C_a$ to $C_b$ haloalkoxy($C_d$ to $C_e$)haloalkyl in the present specification represents a haloalkyl group having the above-mentioned meaning with d to e carbon atoms, optionally substituted by a hydroxyl group, a $C_a$ to $C_b$ alkoxy group having the above-mentioned meaning or a $C_a$ to $C_b$ haloalkoxy group having the above-mentioned meaning, and there may be specifically mentioned, for

example, 2,2,2-trifluoro-1-hydroxy-1-(trifluoromethyl)-ethyl group, 2,2,2-trifluoro-1-methoxy-1-(trifluoromethyl)ethyl group, 2,2,2-trifluoro-1-(2,2,2-trifluoroethoxy)-1-(trifluoromethyl)ethyl group, 3-(1,2-dichloro-1,2,2-trifluoroethoxy)-1,1,2,2,3,3-hexafluoropropyl group, etc., and each may be selected from the range of the carbon numbers as designated, respectively.

The expression ($C_a$ to $C_b$) haloalkyl optionally substituted by $R^{21}$ in the present specification represents a haloalkyl group optionally substituted by $R^{21}$ having the above-mentioned meaning with a to b carbon atoms optionally substituted by $R^{21}$, and each may be selected from the range of the carbon numbers as designated, respectively. At this time, when two or more substituent $R^{21}$s exist on the respective ($C_a$ to $C_b$)haloalkyl group, the respective $R^{21}$s may be the same with each other or may be different from each other.

The expression ($C_a$ to $C_b$) cycloalkyl optionally substituted by $R^7$, ($C_a$ to $C_b$) cycloalkyl optionally substituted by $R^{16}$, ($C_a$ to $C_b$) cycloalkyl optionally substituted by $R^{21}$, ($C_a$ to $C_b$) cycloalkyl optionally substituted by $R^{25}$ or ($C_a$ to $C_b$) cycloalkyl optionally substituted by $R^{33}$, etc., in the present specification represents a cycloalkyl group optionally substituted by $R^7$, $R^{16}$, $R^{21}$, $R^{25}$ or $R^{33}$ having the above-mentioned meaning with a to b carbon atoms optionally substituted by $R^7$, $R^{16}$, $R^{21}$, $R^{25}$ or $R^{33}$. At this time, substitution by $R^7$, $R^{16}$, $R^{21}$, $R^{25}$ or $R^{33}$ may be at the ring structure portion or at the side chain portion, or may be at the both of them, and further, when two or more substituents $R^7$s, $R^{16}$s, $R^{21}$s, $R^{25}$s or $R^{33}$s exist on the ($C_a$ to $C_b$) cycloalkyl group, the respective $R^7$s, $R^{16}$s, $R^{21}$s, $R^{25}$s or $R^{33}$s may be the same with each other or may be different from each other.

The expression ($C_a$ to $C_b$) halocycloalkyl optionally substituted by $R^{21}$ in the present specification represents a halocycloalkyl group optionally substituted by $R^{21}$ having the above-mentioned meaning with a to b carbon atoms optionally substituted by $R^{21}$. At this time, substitution by $R^{21}$ may be at the ring structure portion or at the side chain portion, or may be at the both of them, and further, when two or more substituents $R^{21}$ s exist on the respective ($C_a$ to $C_b$) halocycloalkyl group, the respective $R^{21}$s may be the same with each other or may be different from each other.

The expression phenyl($C_d$ to $C_e$)alkenyl or a phenyl($C_d$ to $C_e$)alkenyl substituted by $(Z)_{p1}$, etc., in the present specification represents an alkenyl group having the above-mentioned meaning with d to e carbon atoms optionally substituted by a phenyl group or a phenyl group substituted by $(Z)_{p1}$ each having the above-mentioned meanings, and each may be selected from the range of the carbon numbers as designated, respectively.

The expression ($C_a$ to $C_b$) alkenyl optionally substituted by $R^7$, a ($C_a$ to $C_b$) alkenyl optionally substituted by $R^{16}$, the ($C_a$ to $C_b$) alkenyl optionally substituted by $R^{21}$, the ($C_a$ to $C_b$) alkenyl optionally substituted by $R^{25}$ or the ($C_a$ to $C_b$) alkenyl optionally substituted by $R^{33}$ in the present specification represents an alkenyl group optionally substituted by $R^7$, $R^{16}$, $R^{21}$, $R^{25}$ or $R^{33}$ having the above-mentioned meaning with a to b carbon atoms optionally substituted by $R^7$, $R^{16}$, $R^{21}$, $R^{25}$ or $R^{33}$, and each may be selected from the range of the carbon numbers as designated, respectively. At this time, when two or more substituents $R^7$s, $R^{16}$s, $R^{21}$s, $R^{25}$s or $R^{33}$s exist on the respective ($C_a$ to $C_b$) alkenyl group, the respective $R^7$s, $R^{16}$s, $R^{21}$s, $R^{25}$s or $R^{33}$s may be the same with each other or may be different from each other.

The expression phenyl($C_d$ to $C_e$) alkynyl or a phenyl($C_d$ to $C_e$) alkynyl substituted by $(Z)_{p1}$ in the present specification represents an alkynyl group having the above-mentioned meaning with d to e carbon atoms optionally substituted by a phenyl group or a phenyl group substituted by $(Z)_{p1}$, and each may be selected from the range of the carbon numbers as designated, respectively.

The expression ($C_a$ to $C_b$) alkynyl optionally substituted by $R^7$, the ($C_a$ to $C_b$) alkynyl optionally substituted by $R^{16}$, the ($C_a$ to $C_b$) alkynyl optionally substituted by $R^{21}$, the ($C_a$ to $C_b$) alkynyl optionally substituted by $R^{25}$ or the ($C_a$ to $C_b$) alkynyl optionally substituted by $R^{33}$ in the present specification represents an alkynyl group optionally substituted by $R^7$, $R^{16}$, $R^{21}$, $R^{25}$ or $R^{33}$ having the above-mentioned meaning with a to b carbon atoms optionally substituted by $R^7$, $R^{16}$, $R^{21}$, $R^{25}$ or $R^{33}$, and each may be selected from the range of the carbon numbers as designated, respectively. At this time, when two or more substituents $R^7$s, $R^{16}$s, $R^{21}$ s, $R^{25}$s or $R^{33}$s exist on the respective ($C_a$ to $C_b$) alkynyl group, the respective $R^7$s, $R^{16}$s, $R^{21}$s, $R^{25}$s or $R^{33}$s may be the same with each other or may be different from each other.

The expression phenyl($C_a$ to $C_b$) alkoxy or a phenyl($C_a$ to $C_b$) alkoxy substituted by $(Z)_{p1}$ in the present specification represents a ($C_a$ to $C_b$) alkoxy group having the above-mentioned meaning optionally substituted by a phenyl group or phenyl group substituted by $(Z)_{p1}$, and as the ($C_a$ to $C_b$) alkoxy group, there may be mentioned, for example, $-CH_2O$-group, $-CH(CH_3)O$ group, $-C(CH_3)_2O$ group, $-CH_2CH_2O-$ group, $-CH(CH_3)CH_2O-$ group, $-C(CH_3)_2CH_2O-$ group, etc., and each may be selected from the range of the carbon numbers as designated, respectively.

The expression $C_a$ to $C_b$ haloalkoxy($C_d$ to $C_e$) haloalkoxy in the present specification represents a haloalkoxy group having the above-mentioned meaning with d to e carbon atoms optionally substituted by a $C_a$ to $C_b$ haloalkoxy group having the above-mentioned meaning, and there may be mentioned, for example, 1,1,2-trifluoro-2-trifluoromethoxyethoxy group, 1,1,2-trifluoro-2-heptafluoropropyloxyethoxy group, etc., and each may be selected from the range of the carbon numbers as designated, respectively.

The expression phenyl($C_a$ to $C_b$)alkylcarbonyl or a phenyl($C_a$ to $C_b$)alkylcarbonyl substituted by $(Z)_{p1}$ in the present specification represents a ($C_a$ to $C_b$)alkylcarbonyl group having the above-mentioned meaning and optionally substituted by a phenyl group or a phenyl group substituted by $(Z)_{p1}$, and as the ($C_a$ to $C_b$)alkylcarbonyl group, there may be mentioned, for example, $-CH_2C(O)-$ group, $-CH(CH_3)C(O)-$ group, $-C(CH_3)_2C(O)-$ group, $-CH_2CH_2C(O)-$ group,

$-CH(CH_3)CH_2C(O)-$ group, $-C(CH_3)_2CH_2C(O)-$ group, $-CH_2CH(CH_3)C(O)-$ group, $-CH_2C(CH_3)_2C(O)-$ group, $-CH_2CH_2CH_2C(O)-$ group, etc., and each may be selected from the range of the carbon numbers as designated, respectively.

The expression a phenyl($C_a$ to $C_b$) alkoxycarbonyl or a phenyl($C_a$ to $C_b$) alkoxycarbonyl substituted by $(Z)_{p1}$ in the present specification represents a ($C_a$ to $C_b$) alkoxycarbonyl group having the above-mentioned meaning and optionally substituted by a phenyl group or a phenyl group substituted by $(Z)_{p1}$, and as the ($C_a$ to $C_b$) alkoxycarbonyl group, there may be mentioned, for example, $-CH_2O-C(O)-$ group, $-CH(CH_3)O-C(O)-$group, $-C(CH_3)_2O-C(O)-$ group, $-CH_2CH_2O-C(O)-$ group, $-CH(CH_3)CH_2O-C(O)-$ group, $-C(CH_3)_2CH_2O-C(O)-$ group, etc., and each may be selected from the range of the carbon numbers as designated, respectively.

In the present specification, as specific examples of the expressions
"$R^2$ is combined with $R^1$ to form a $C_2$ to $C_6$ alkylene chain whereby it may form a 3 to 7-membered ring with the nitrogen atom to which they are bonded, and at this time, the alkylene chain may contain one oxygen atom, sulfur atom or nitrogen atom,",
"$R^{17}$ and $R^{18}$ are combined in combination to form a $C_4$ to $C_7$ alkylene chain whereby it may form a 5 to 8-membered ring with the nitrogen atom to which they are bonded, and at this time, the alkylene chain may contain one oxygen atom or sulfur atom,",
"$R^{26}$ and $R^{27}$ are combined in combination to form a $C_2$ to $C_5$ alkylene chain whereby it may form a 3 to 6-membered ring with the nitrogen atom to which they are bonded, and at this time, the alkylene chain may contain one oxygen atom or sulfur atom,",
"$R^{29}$ and $R^{30}$ are combined in combination to form a $C_2$ to $C_5$ alkylene chain whereby it may form a 3 to 6-membered ring with the nitrogen atom to which they are bonded, and at this time, the alkylene chain may contain one oxygen atom, sulfur atom or nitrogen atom,",
and
"$R^{34}$ and $R^{35}$ are combined in combination to form a $C_2$ to $C_5$ alkylene chain whereby it may form a 3 to 6-membered ring with the nitrogen atom to which they are bonded, and at this time, the alkylene chain may contain one oxygen atom or sulfur atom," there may be mentioned, for example, azilidine, azetidine, pyrrolidine, oxazolidine, thiazolidine, imidazolidine, piperidine, morpholine, thiomorpholine, piperazine, homopiperidine, heptamethyleneimine, etc., and each may be selected from the range of the carbon numbers as designated, respectively.

In the present specification, as specific examples of the expression
"$R^9$ and $R^{10}$ are combined in combination to form a $C_2$ to $C_6$ alkylene chain whereby it may form a 3 to 7-membered ring with the nitrogen atom to which they are bonded, and at this time, the alkylene chain may contain one oxygen atom, sulfur atom or nitrogen atom,"
there may be mentioned, for example, azilidine, azetidine, azetidin-2-one, pyrrolidine, pyrrolidin-2-one, oxazolidine, oxazolidin-2-one, thiazolidine, thiazolidin-2-one, imidazolidine, imidazolidin-2-one, piperidine, piperidin-2-one, morpholine, tetrahydro-1,3-oxazin-2-one, thiomorpholine, tetrahydro-1,3-thiazin-2-one, piperazine, tetrahydropyrimidin-2-one, homopiperidine, homopiperidin-2-one, etc., and each may be selected from the range of the carbon numbers as designated, respectively.

In the present specification, as specific examples of the expression
"$R^{11}$ is combined with $R^9$ to form a $C_2$ to $C_4$ alkylene chain whereby it may form a 5 to 7-membered ring with the atoms to which they are bonded, and at this time, the alkylene chain may contain one oxygen atom, sulfur atom or nitrogen atom," there may be mentioned, for example, isoxazoline, 1,4,2-dioxazoline, 1,4,2-oxathiazoline, 1,2,4-oxadiazoline, dihydro-1,2-oxadine, dihydro-1,4,2-dioxadine, dihydro-1,4,2-oxathizine, dihydro-4H-1,2,4-oxadiazine, tetrahydro-1,2-oxazepine, etc., and each may be selected from the range of the carbon numbers as designated, respectively.

In the compounds included in the present invention, as the substituent represented by $W^1$ or $W^2$, there may be mentioned, for example, oxygen atom or sulfur atom, and of these, the oxygen atom is preferred.

In the compounds included in the present invention, as a scope of the substituent represented by X, there may be mentioned, for example, the following respective groups. At this time, in the respective cases mentioned below, when m is an integer of 2 or more, the respective Xs may be the same with each other or different from each other.

That is, X-I: a halogen atom.

X-II: cyano and nitro.

X-III: a $C_1$ to $C_6$ alkyl and a $C_1$ to $C_6$ haloalkyl.

X-IV: a $C_1$ to $C_6$ alkoxy, a $C_1$ to $C_6$ haloalkoxy and a $C_1$ to $C_6$ alkylsulfonyloxy.

X-V: a $C_1$ to $C_6$ alkylthio, a $C_1$ to $C_6$ haloalkylthio, a $C_1$ to $C_6$ alkylsulfinyl, a $C_1$ to $C_6$ haloalkylsulfinyl, a $C_1$ to $C_6$ alkylsulfonyl and a $C_1$ to $C_6$ haloalkylsulfonyl.

X-VI: m is an integer of 2 or more, and, two Xs are adjacent to each other, and further adjacent two Xs form $-OCH_2O-$ or $-OCH_2CH_2O-$, whereby the form a 5-membered ring or 6-membered ring with carbon atoms to which they are bonded to. At this time, a hydrogen atom bonded to the respective carbon atoms which form the ring may be optionally substituted by a halogen atom, a $C_1$ to $C_4$ alkyl group or a $C_1$ to $C_4$ haloalkyl group.

In the compounds included in the present invention, as m which represents a number of the substituent represented by X, an integer of 0 to 4 is mentioned, and of these, m is preferably 0, 1 and 2.

In the compounds included in the present invention, as the scope of the substituent represented by Y, there may be mentioned, for example, the following respective groups. At this time, in the respective cases mentioned below, when n is an integer of 2 or more, the respective Ys may be the same with each other or different from each other.

That is, Y-I: a halogen atom.

Y-II: a $C_1$ to $C_6$ alkyl.

Y-III: a $C_1$ to $C_6$ haloalkyl, a hydroxy($C_1$ to $C_6$) alkyl and a $C_1$ to $C_4$ alkoxy($C_1$ to $C_4$)alkyl.

Y-IV: a $C_1$ to $C_6$ alkoxy and a $C_1$ to $C_6$ haloalkoxy.

Y-V: a $C_1$ to $C_6$ alkylthio and a $C_1$ to $C_6$ haloalkylthio .

Y-VI: a $C_1$ to $C_6$ alkylamino and a di($C_1$ to $C_6$ alkyl)amino.

In the compounds included in the present invention, as n which represents a number of the substituent represented by Y, an integer of 0 to 4 may be mentioned, and of these, n is preferably 0, 1 and 2.

In the compounds included in the present invention, as the scope of the substituent represented by $R^1$, there may be mentioned, for example, the following respective groups.

That is, $R^1$-I: a $C_1$ to $C_8$ alkyl and a $C_3$ to $C_8$ cycloalkyl.

$R^1$-II: a $C_1$ to $C_8$ alkyl optionally substituted by $-OR^{26}$ [here, $R^{26}$ represents a $C_1$ to $C_6$ alkyl, a $C_1$ to $C_6$ alkylaminocarbonyl or a di($C_1$ to $C_6$ alkyl)aminocarbonyl.].

$R^1$-III: a $C_1$ to $C_8$ alkyl optionally substituted by -CH=NOH or $-CH=NOR^{31}$ [here, $R^{31}$ represents a $C_1$ to $C_6$ alkyl.].

$R^1$-IV: a $C_1$ to $C_8$ alkyl optionally substituted by $R^{16}$ [here, $R^{16}$ represents $-OR^{26}$, $R^{26}$ represents a hydrogen atom, a $C_1$ to $C_6$ alkyl, a $C_1$ to $C_6$ haloalkyl, a $C_1$ to $C_4$ alkoxy($C_1$ to $C_4$)alkyl, a $C_1$ to $C_4$ alkylthio($C_1$ to $C_4$)alkyl, a phenyl ($C_1$ to $C_4$)alkyl, a phenyl($C_1$ to $C_4$) alkyl substituted by $(Z)_{p1}$, a $C_1$ to $C_6$ alkylcarbonyl, a $C_1$ to $C_6$ haloalkylcarbonyl, a $C_3$ to $C_6$ cycloalkylcarbonyl, $-C(O)N(R^{3°})R^{29}$, a di($C_1$ to $C_6$ alkyl)phosphoryl, a di($C_1$ to $C_6$ alkyl)-thiophosphoryl, a tri($C_1$ to $C_4$ alkyl)silyl, phenyl or a phenyl substituted by $(Z)_{P1}$, $R^{29}$ represents a $C_1$ to $C_6$ alkyl, a $C_1$ to $C_4$ alkoxy($C_1$ to $C_4$) alkyl, a $C_1$ to $C_4$ alkylthio($C_1$ to $C_4$)alkyl, a phenyl($C_1$ to $C_4$)alkyl, a phenyl($C_1$ to $C_4$) alkyl substituted by $(Z)_{P1}$, a $C_3$ to $C_6$ cycloalkyl, a $C_3$ to $C_6$ alkenyl, a $C_3$ to $C_6$ alkynyl, phenyl or a phenyl substituted by $(Z)_{p1}$, $R^{30}$ represents a hydrogen atom or a $C_1$ to $C_6$ alkyl, or $R^{29}$ and $R^{30}$ are combined to form a $C_2$ to $C_5$ alkylene chain whereby it may form a 3 to 6-membered ring with the nitrogen atom to which they are bonded, and at this time, the alkylene chain may contain one oxygen atom or sulfur atom.], M-4, M-5, M-14, M-15 and M-16.

$R^1$-V: a $C_1$ to $C_8$ alkyl optionally substituted by $R^{16}$ [here, $R^{16}$ represents cyano, a $C_1$ to $C_6$ alkoxycarbonyl, a $C_1$ to $C_6$ alkylaminocarbonyl, a di($C_1$ to $C_6$ alkyl)aminocarbonyl, $-C(R^{32})=NOH$ or $-C(R^{32})=NOR^{31}$, $R^{31}$ represents a $C_1$ to $C_6$ alkyl, a phenyl($C_1$ to $C_4$) alkyl or a phenyl($C_1$ to $C_4$) alkyl substituted by $(Z)_{p1}$, $R^{32}$ represents a hydrogen atom or a $C_1$ to $C_6$ alkyl.].

$R^1$-VI: a $C_1$ to $C_8$ alkyl optionally substituted by $R^{16}$ [here, $R^{16}$ represents a halogen atom, a $C_3$ to $C_6$ cycloalkyl, a tri($C_1$ to $C_6$ alkyl)silyl , phenyl, a phenyl substituted by $(Z)_{p1}$, L-1, L-2, L-3, L-4, L-45, L-46, L-47 or M.].

$R^1$-VII: a $C_1$ to $C_6$ alkyl, a $C_1$ to $C_4$ alkylthio($C_1$ to $C_4$)alkyl, a $C_1$ to $C_4$ alkylsulfinyl-($C_1$ to $C_4$) alkyl and a $C_1$ to $C_4$ alkylsulfonyl($C_1$ to $C_4$)alkyl.

$R^1$-VIII: a $C_3$ to $C_8$ alkenyl and a $C_3$ to $C_8$alkynyl.

$R^1$-IX: a $C_1$ to $C_4$ alkylthio($C_1$ to $C_4$)alkyl, a $C_1$ to $C_4$ alkylsulfinyl($C_1$ to $C_4$) alkyl and a $C_1$ to $C_4$ alkylsulfonyl ($C_1$ to $C_4$)alkyl.

$R^1$-X: a ($C_1$ to $C_8$) alkyl optionally substituted by $-N(R^{27})R^{26}$ [here, $R^{26}$ represents a $C_1$ to $C_6$ alkoxycarbonyl, a $C_1$ to $C_6$ alkylsulfonyl or a di($C_1$ to $C_6$ alkyl)thiophosphoryl, $R^{27}$ represents a hydrogen atom or a $C_1$ to $C_6$ alkyl.].

$R^1$-XI: a $C_1$ to $C_8$ alkyl optionally substituted by $R^{16}$ [here, $R^{16}$ represents $-S(O)_rR^{28}$ or $-SO_2N(R^{30})R^{29}$, $R^{28}$ represents a $C_1$ to $C_6$ alkyl, a $C_1$ to $C_6$ haloalkyl, a hydroxy($C_1$ to $C_4$)alkyl, a $C_1$ to $C_4$ alkoxy($C_1$ to $C_4$)alkyl, a $C_1$ to $C_4$ alkylthio($C_1$ to $C_4$)alkyl, a $C_1$ to $C_4$ alkylcarbonyl($C_1$ to $C_4$)alkyl, a $C_1$ to $C_4$ alkoxycarbonyl($C_1$ to $C_4$)alkyl, a $C_1$ to $C_4$ alkylaminocarbonyl($C_1$ to $C_4$)alkyl, a di($C_1$ to $C_4$ alkyl)aminocarbonyl($C_1$ to $C_4$)alkyl, a tri($C_1$ to $C_4$ alkyl)silyl ($C_1$ to $C_4$) alkyl, a phenyl($C_1$ to $C_4$)alkyl, a phenyl($C_1$ to $C_4$) alkyl substituted by $(Z)_{p1}$, a $C_3$ to $C_6$ alkenyl, a $C_3$ to $C_6$ alkynyl, a $C_1$ to $C_6$ alkylthio, phenyl, a phenyl substituted by $(Z)_{p1}$, L-21, L-35, L-45 or L-48, r is an integer of 0 to 2, $R^{29}$ represents a $C_1$ to $C_6$ alkyl, $R^{30}$ represents a hydrogen atom or a $C_1$ to $C_6$ alkyl.], M-8, M-9, M-17, M-18 and M-19.

$R^1$-XII: a $C_1$ to $C_8$ alkyl optionally substituted by $R^{16}$ [here, $R^{16}$ represents $-N(R^{27})R^{26}$, $R^{26}$ represents a $C_1$ to $C_6$ alkylcarbonyl, a $C_3$ to $C_6$cyclo alkylcarbonyl, a $C_1$ to $C_6$alkoxycarbonyl, a di($C_1$ to $C_6$ alkyl)aminocarbonyl, a $C_1$ to $C_6$ alkylsulfonyl, a di($C_1$ to $C_6$ alkyl)aminosulfonyl, phenylsulfonyl, a phenylsulfonyl substituted by $(Z)_{p1}$ or a di($C_1$ to $C_6$ alkyl)thiophosphoryl, $R^{27}$ represents a hydrogen atom or a $C_1$ to $C_6$ alkyl.], M-13, M-21 and M-22.

$R^1$-XIII: a 3 to 7-membered ring formed by $R^1$ and $R^2$ in combination is azilidine, azetidine, pyrrolidine, oxazolidine, thiazolidine, piperidine, morpholine, thiomorpholine and homopiperidine.

R'-XIV: a $C_1$ to $C_6$ alkyl, a $C_1$ to $C_4$ alkyl optionally substituted by $-OR^{26}$ [here, $R^{26}$ represents a $C_1$ to $C_4$ alkyl, a $C_1$ to $C_4$ alkylaminocarbonyl or a di($C_1$ to $C_4$ alkyl)aminocarbonyl.], a $C_1$ to $C_4$ alkylthio($C_1$ to $C_4$)alkyl, a $C_1$ to $C_4$ alkylsulfinyl($C_1$ to $C_4$) alkyl and a $C_1$ to $C_4$ alkylsulfonyl($C_1$ to $C_4$)alkyl, a ($C_1$ to $C_4$) alkyl optionally substituted by $-N(R^{27})$

$R^{26}$ [here, $R^{26}$ represents a $C_1$ to $C_4$ alkoxycarbonyl, a $C_1$ to $C_4$ alkylsulfonyl or a di($C_1$ to $C_4$ alkyl)thiophosphoryl, and $R^{27}$ represents a hydrogen atom or a $C_1$ to $C_4$ alkyl.] and a $C_1$ to $C_4$ alkyl optionally substituted by -CH=NOH or -CH=NOR$^{31}$ [here, $R^{31}$ represents a $C_1$ to $C_4$ alkyl.].

In the compounds included in the present invention, as scopes of the substituents represented by $R^2$ and $R^3$, there may be mentioned, for example, the following respective groups.

That is, $R^2$-I or $R^3$-I: a hydrogen atom.

$R^2$-II or $R^3$-II: a hydrogen atom and a $C_1$ to $C_6$ alkyl.

$R^2$-III or $R^3$-III: a hydrogen atom, a $C_1$ to $C_4$ alkoxy($C_1$ to $C_4$)alkyl, a $C_1$ to $C_4$ alkylthio($C_1$ to $C_4$) alkyl and a $C_1$ to $C_4$ alkylsulfonyl($C_1$ to $C_4$)alkyl.

$R^2$-IV or $R^3$-IV: a hydrogen atom, a $C_3$ to $C_6$ alkenyl and $C_3$ to $C_6$alkynyl.

$R^2$-V or $R^3$-V: a hydrogen atom, a $C_1$ to $C_6$ alkylthio, a $C_1$ to $C_6$ haloalkylthio, phenylthio, a phenylthio substituted by $(Z)_{p1}$ and -SN($R^{18}$)$R^{17}$ [here, $R^{17}$ represents a $C_1$ to $C_6$ alkyl, a $C_1$ to $C_6$ alkoxycarbonyl($C_1$ to $C_4$) alkyl or a $C_1$ to $C_6$ alkoxycarbonyl, $R^{18}$ represents a $C_1$ to $C_6$ alkyl, or $R^{17}$ and $R^{18}$ are combined in combination to form a $C_4$ to $C_5$ alkylene chain, whereby it may form a 5-membered ring or 6-membered ring with the nitrogen atom to which they are bonded, and at this time, the alkylene chain may contain one oxygen atom or sulfur atom, and may be optionally substituted by methyl group or methoxy group.].

In the compounds included in the present invention, as a scope of the substituent represented by $R^4$, there may be mentioned, for example, the following respective groups.

That is, $R^4$-I: a $C_1$ to $C_6$ alkyl and a $C_1$ to $C_6$ haloalkyl.

$R^4$-II: a $C_1$ to $C_6$ alkyl, a $C_1$ to $C_6$ haloalkyl, a $C_3$ to $C_8$ cycloalkyl and a $C_3$ to $C_8$ halocycloalkyl.

$R^4$-III: a ($C_1$ to $C_6$) alkyl optionally substituted by $R^{21}$ and a ($C_1$ to $C_6$) haloalkyl optionally substituted by $R^{21}$ [here, $R^{21}$ represents cyano, a $C_3$ to $C_6$ cycloalkyl, a $C_3$ to $C_6$ halocycloalkyl, a $C_1$ to $C_6$ alkoxy, a $C_1$ to $C_6$ haloalkoxy, phenoxy, a phenoxy substituted by $(Z)_{p1}$, a $C_1$ to $C_6$ alkylthio, a $C_1$ to $C_6$ haloalkylthio, phenylthio, a phenylthio substituted by $(Z)_{p1}$, a $C_1$ to $C_6$ alkylsulfinyl, a $C_1$ to $C_6$ haloalkylsulfinyl, a $C_1$ to $C_6$ alkylsulfonyl, a $C_1$ to $C_6$ haloalkylsulfonyl, phenylsulfonyl, a phenylsulfonyl substituted by $(Z)_{p1}$, a $C_1$ to $C_6$ alkylamino, a di($C_1$ to $C_6$ alkyl)amino, phenylamino, a phenylamino substituted by $(Z)_{p1}$, a $C_1$ to $C_6$ alkoxycarbonyl, phenyl, a phenyl substituted by $(Z)_{p1}$, L-1 to L-5, L-8 to L-24, L-36, L-39, L-45 to L-52 or L-53.].

$R^4$-IV: a $C_3$ to $C_6$ haloalkenyl, a $C_3$ to $C_6$ haloalkynyl, phenyl and a phenyl substituted by $(Z)_{p1}$.

In the compounds included in the present invention, as a scope of the substituent represented by $R^5$, there may be mentioned, for example, the following respective groups.

That is, $R^5$-I: a ($C_1$ to $C_6$) alkyl optionally substituted by $R^{21}$, a ($C_1$ to $C_6$) haloalkyl optionally substituted by $R^{21}$, a ($C_2$ to $C_6$) alkenyl optionally substituted by $R^{21}$ and a($C_2$ to $C_6$) alkynyl optionally substituted by $R^{21}$ [here, $R^{21}$ represents a $C_1$ to $C_6$ alkoxy, a $C_1$ to $C_6$ haloalkoxy, phenoxy, a phenoxy substituted by $(Z)_{p1}$, phenylthio, a phenylthio substituted by $(Z)_{p1}$, phenylsulfonyl, a phenylsulfonyl substituted by $(Z)_{p1}$, a $C_1$ to $C_6$ alkylamino, a di($C_1$ to $C_6$ alkyl) amino, phenylamino, a phenylamino substituted by $(Z)_{p1}$, a $C_1$ to $C_6$ alkoxycarbonyl, phenyl, a phenyl substituted by $(Z)_{p1}$, L-1 to L-5, L-8 to L-24, L-36, L-39, L-45 to L-52 or L-53.].

$R^5$-11: a ($C_1$ to $C_6$) alkyl optionally substituted by $R^{21}$, a ($C_1$ to $C_6$) haloalkyl optionally substituted by $R^{21}$, a ($C_2$ to $C_6$) alkenyl optionally substituted by $R^{21}$ and a ($C_2$ to $C_6$) alkynyl optionally substituted by $R^{21}$ [here, $R^{21}$ represents cyano, a $C_3$ to $C_6$ cycloalkyl, a $C_3$ to $C_6$ halocycloalkyl, a $C_1$ to $C_6$ alkoxy, a $C_1$ to $C_6$ haloalkoxy, phenoxy, a phenoxy substituted by $(Z)_{p1}$, a $C_1$ to $C_6$ alkylthio, a $C_1$ to $C_6$ haloalkylthio, phenylthio, a phenylthio substituted by $(Z)_{p1}$, a $C_1$ to $C_6$ alkylsulfinyl, a $C_1$ to $C_6$ haloalkylsulfinyl, a $C_1$ to $C_6$ alkylsulfonyl, a $C_1$ to $C_6$ haloalkylsulfonyl, phenylsulfonyl, a phenylsulfonyl substituted by $(Z)_{p1}$, a $C_1$ to $C_6$ alkylamino, a di($C_1$ to $C_6$ alkyl)amino, phenylamino, a phenylamino substituted by $(Z)_{p1}$, a $C_1$ to $C_6$ alkoxycarbonyl, phenyl, a phenyl substituted by $(Z)_{p1}$, L-1 to L-5, L-8 to L-24, L-36, L-39, L-45 to L-52 or L-53.].

$R^5$-III: phenyl, a phenyl substituted by $(Z)_{p1}$, a phenoxyphenyl substituted by $(Z)_{p1}$, a pyridyloxyphenyl substituted by $(Z)_{p1,}$ L-1 to L-4, L-8 to L-13, L-15 to L-23, L-45 to L-52 and L-53.

$R^5$-IV: a $C_3$ to $C_8$ cycloalkyl, a $C_3$ to $C_8$ halocycloalkyl, phenyl, a phenyl substituted by $(Z)_{P1}$, a phenoxyphenyl substituted by $(Z)_{p1}$, a pyridyloxyphenyl substituted by $(Z)_{p1}$, L-1 to L-4, L-8 to L-13, L-15 to L-23, L-25 to L-35, L-37, L-38, L-40, L-43 to L-58, M-4, M-5, M-8, M-9, M-14 to M-18 and M-19.

$R^5$-V: cyano, -C(O)OR$^9$, -C(O)SR$^9$, -C(O)NHR$^{10}$, -C(O)N($R^{10}$)$R^9$, -C(S)OR$^9$, -C(S)SR$^9$, -C(S)NHR$^{10}$ and -C(S)N($R^{10}$)$R^9$ [here, $R^9$ represents a $C_1$ to $C_6$ alkyl, a $C_1$ to $C_6$ haloalkyl, a $C_3$ to $C_6$ cycloalkyl($C_1$ to $C_4$)alkyl, a phenyl($C_1$ to $C_4$)alkyl, a phenyl($C_1$ to $C_4$) alkyl substituted by $(Z)_{p1}$, a $C_3$ to $C_8$ cycloalkyl, a $C_3$ to $C_8$ halocycloalkyl, phenyl or a phenyl substituted by $(Z)_{p1}$, $R^{10}$ represents a hydrogen atom or a $C_1$ to $C_6$ alkyl, or $R^9$ and $R^{10}$ are combined to form a $C_4$ to $C_5$ alkylene chain whereby it may form a 5-membered ring or 6-membered ring with the nitrogen atom to which they are bonded, and at this time, the alkylene chain may contain one oxygen atom or sulfur atom.].

$R^5$-VI: it forms a $C_2$ to $C_3$ alkylene chain with Y existing at the adjavent portion whereby it forms a 5 to 6-membered ring which fuses with a benzene ring, and at this time, the alkylene chain may contain one oxygen atom, sulfur atom or nitrogen atom, and may be optionally substituted by a halogen atom or a $C_1$ to $C_6$ haloalkyl group.

In the compounds included in the present invention, as a scope of the substituent represented by $R^6$, there may be mentioned, for example, the following respective groups.

That is, $R^6$-I: a hydrogen atom.

$R^6$-II: a hydrogen atom, a $C_1$ to $C_6$ alkyl, a $C_1$ to $C_4$ alkoxy($C_1$ to $C_4$)alkyl, a $C_1$ to $C_6$ alkylcarbonyl and a tri($C_1$ to $C_4$ alkyl)silyl .

$R^6$-III: a hydrogen atom, a $C_1$ to $C_6$ alkyl, a $C_1$ to $C_6$ haloalkyl, a $C_1$ to $C_4$ alkoxy($C_1$ to $C_4$)alkyl, a $C_1$ to $C_4$ alkylthio($C_1$ to $C_4$)alkyl, cyano($C_1$ to $C_6$)alkyl, a phenyl($C_1$ to $C_4$)alkyl, a phenyl($C_1$ to $C_4$) alkyl substituted by $(Z)_{p1}$, a $C_3$ to $C_6$ alkenyl, a $C_3$ to $C_6$ haloalkenyl, a phenyl($C_3$ to $C_6$) alkenyl, a phenyl($C_3$ to $C_6$) alkenyl substituted by $(Z)_{p1}$, a $C_3$ to $C_6$ alkynyl, a $C_3$ to $C_6$ haloalkynyl, a phenyl($C_3$ to $C_6$) alkynyl, a phenyl($C_3$ to $C_6$) alkynyl substituted by $(Z)_{p1}$, -S(O)$_2$R$^9$, -C(O)R$^9$, -C(O)NHR$^{10}$, -C(O)N(R$^{10}$)R$^9$, -C(S)NHR$^{10}$, -C(S)N(R$^{10}$)R$^9$ [here, $R^9$ represents a $C_1$ to $C_6$ alkyl, a $C_1$ to $C_6$ haloalkyl, a $C_3$ to $C_6$ cycloalkyl($C_1$ to $C_4$)alkyl, a phenyl($C_1$ to $C_4$)alkyl, a phenyl($C_1$ to $C_4$) alkyl substituted by $(Z)_{p1}$, a $C_3$ to $C_8$ cycloalkyl, a $C_3$ to $C_8$ halocycloalkyl, phenyl or a phenyl substituted by $(Z)_{p1}$, $R^{10}$ represents a hydrogen atom or a $C_1$ to $C_6$ alkyl, or $R^9$ and $R^{10}$ are combined to form a $C_4$ to $C_5$ alkylene chain, whereby it may form a 5-membered ring or 6-membered ring with a nitrogen atom to which they are bonded to, and the alkylene chain at this time may contain one oxygen atom or sulfur atom.], -Si(R$^{13}$)(R$^{14}$)R$^{12}$ [here, $R^{12}$ represents a $C_1$ to $C_6$ alkyl, phenyl or a phenyl substituted by $(Z)_{p1}$, $R^{13}$ and $R^{14}$ each independently represent a $C_1$ to $C_6$ alkyl.], -P(O)(OR$^{22}$)$_2$ and -P(S)(OR$^{22}$)$_2$ [here, $R^{22}$ represents a $C_1$ to $C_6$ alkyl.].

The respective groups showing the scopes of the respective substituents in the compounds included in the present invention can be optionally combined with each other and represent the respective scopes of the compounds of the present invention. Examples of the combination of the scopes with regard to $R^1$, $R^4$, $R^5$ and $R^6$ may be mentioned, for example, the combinations shown in the following Table 1. Provided that the combinations shown in Table 1 are only for exemplary purpose, and the present invention is not limited by these alone.

Table 1

| $R^1$ | $R^4$ | $R^5$ | $R^6$ |
|---|---|---|---|
| $R^1$-I | $R^4$-I | $R^5$-I | $R^6$-I |
| $R^1$-I | $R^4$-I | $R^5$-I | $R^6$-II |
| $R^1$-I | $R^4$-I | $R^5$-III | $R^6$-I |
| $R^1$-I | $R^4$-I | $R^5$-III | $R^6$-II |
| $R^1$-I | $R^4$-I | $R^5$-VI | $R^6$-I |
| $R^1$-I | $R^4$-I | $R^5$-VI | $R^6$-II |
| $R^1$-II | $R^4$-I | $R^5$-I | $R^6$-I |
| $R^1$-II | $R^4$-I | $R^5$-I | $R^6$-II |
| $R^1$-II | $R^4$-I | $R^5$-III | $R^6$-I |
| $R^1$-II | $R^4$-I | $R^5$-III | $R^6$-II |
| $R^1$-II | $R^4$-I | $R^5$-III | $R^6$-III |
| $R^1$-II | $R^4$-I | $R^5$-IV | $R^6$-I |
| $R^1$-II | $R^4$-I | $R^5$-IV | $R^6$-II |
| $R^1$-II | $R^4$-I | $R^5$-V | $R^6$-I |
| $R^1$-II | $R^4$-I | $R^5$-V | $R^6$-II |
| $R^1$-II | $R^4$-I | $R^5$-VI | $R^6$-I |
| $R^1$-II | $R^4$-I | $R^5$-VI | $R^6$-II |
| $R^1$-II | $R^4$-II | $R^5$-I | $R^6$-I |
| $R^1$-II | $R^4$-II | $R^5$-I | $R^6$-II |
| $R^1$-II | $R^4$-II | $R^5$-III | $R^6$-I |
| $R^1$-II | $R^4$-II | $R^5$-III | $R^6$-II |
| $R^1$-II | $R^4$-II | $R^5$-III | $R^6$-III |
| $R^1$-II | $R^4$-II | $R^5$-IV | $R^6$-I |
| $R^1$-II | $R^4$-II | $R^5$-IV | $R^6$-II |
| $R^1$-II | $R^4$-II | $R^5$-V | $R^6$-I |
| $R^1$-II | $R^4$-II | $R^5$-V | $R^6$-II |
| $R^1$-II | $R^4$-II | $R^5$-VI | $R^6$-I |
| $R^1$-II | $R^4$-II | $R^5$-VI | $R^6$-II |
| $R^1$-II | $R^4$-III | $R^5$-III | $R^6$-I |
| $R^1$-II | $R^4$-III | $R^5$-III | $R^6$-II |
| $R^1$-II | $R^4$-IV | $R^5$-III | $R^6$-I |
| $R^1$-III | $R^4$-I | $R^5$-I | $R^6$-I |
| $R^1$-III | $R^4$-I | $R^5$-I | $R^6$-II |
| $R^1$-III | $R^4$-I | $R^5$-III | $R^6$-I |
| $R^1$-III | $R^4$-I | $R^5$-III | $R^6$-II |
| $R^1$-III | $R^4$-I | $R^5$-III | $R^6$-III |
| $R^1$-III | $R^4$-I | $R^5$-IV | $R^6$-I |

Table 1 (contd.)

| $R^1$ | $R^4$ | $R^5$ | $R^6$ |
|---|---|---|---|
| $R^1$-VIII | $R^4$-I | $R^5$-I | $R^6$-I |
| $R^1$-VIII | $R^4$-I | $R^5$-I | $R^6$-II |
| $R^1$-VIII | $R^4$-I | $R^5$-III | $R^6$-I |
| $R^1$-VIII | $R^4$-I | $R^5$-III | $R^6$-II |
| $R^1$-VIII | $R^4$-I | $R^5$-VI | $R^6$-I |
| $R^1$-VIII | $R^4$-I | $R^5$-VI | $R^6$-II |
| $R^1$-IX | $R^4$-I | $R^5$-I | $R^6$-I |
| $R^1$-IX | $R^4$-I | $R^5$-I | $R^6$-II |
| $R^1$-IX | $R^4$-I | $R^5$-III | $R^6$-I |
| $R^1$-IX | $R^4$-I | $R^5$-III | $R^6$-II |
| $R^1$-IX | $R^4$-I | $R^5$-III | $R^6$-III |
| $R^1$-IX | $R^4$-I | $R^5$-IV | $R^6$-I |
| $R^1$-IX | $R^4$-I | $R^5$-IV | $R^6$-II |
| $R^1$-IX | $R^4$-I | $R^5$-V | $R^6$-I |
| $R^1$-IX | $R^4$-I | $R^5$-V | $R^6$-II |
| $R^1$-IX | $R^4$-I | $R^5$-VI | $R^6$-I |
| $R^1$-IX | $R^4$-I | $R^5$-VI | $R^6$-II |
| $R^1$-IX | $R^4$-II | $R^5$-I | $R^6$-I |
| $R^1$-IX | $R^4$-II | $R^5$-I | $R^6$-II |
| $R^1$-IX | $R^4$-II | $R^5$-III | $R^6$-I |
| $R^1$-IX | $R^4$-II | $R^5$-III | $R^6$-II |
| $R^1$-IX | $R^4$-II | $R^5$-III | $R^6$-III |
| $R^1$-IX | $R^4$-II | $R^5$-IV | $R^6$-I |
| $R^1$-IX | $R^4$-II | $R^5$-IV | $R^6$-II |
| $R^1$-IX | $R^4$-II | $R^5$-V | $R^6$-I |
| $R^1$-IX | $R^4$-II | $R^5$-V | $R^6$-II |
| $R^1$-IX | $R^4$-II | $R^5$-VI | $R^6$-I |
| $R^1$-IX | $R^4$-II | $R^5$-VI | $R^6$-II |
| $R^1$-IX | $R^4$-III | $R^5$-III | $R^6$-I |
| $R^1$-IX | $R^4$-III | $R^5$-III | $R^6$-II |
| $R^1$-IX | $R^4$-IV | $R^5$-III | $R^6$-I |
| $R^1$-X | $R^4$-I | $R^5$-I | $R^6$-I |
| $R^1$-X | $R^4$-I | $R^5$-I | $R^6$-II |
| $R^1$-X | $R^4$-I | $R^5$-III | $R^6$-I |
| $R^1$-X | $R^4$-I | $R^5$-III | $R^6$-II |
| $R^1$-X | $R^4$-I | $R^5$-III | $R^6$-III |
| $R^1$-X | $R^4$-I | $R^5$-IV | $R^6$-I |

| | | | | | | | |
|---|---|---|---|---|---|---|---|
| $R^1$-III | $R^4$-I | $R^5$-IV | $R^6$-II | $R^1$-X | $R^4$-I | $R^5$-IV | $R^6$-II |
| $R^1$-III | $R^4$-I | $R^5$-V | $R^6$-I | $R^1$-X | $R^4$-I | $R^5$-V | $R^6$-I |
| $R^1$-III | $R^4$-I | $R^5$-V | $R^6$-II | $R^1$-X | $R^4$-I | $R^5$-V | $R^6$-II |
| $R^1$-III | $R^4$-I | $R^5$-VI | $R^6$-I | $R^1$-X | $R^4$-I | $R^5$-VI | $R^6$-I |
| $R^1$-III | $R^4$-I | $R^5$-VI | $R^6$-II | $R^1$-X | $R^4$-I | $R^5$-VI | $R^6$-II |
| $R^1$-III | $R^4$-II | $R^5$-I | $R^6$-I | $R^1$-X | $R^4$-II | $R^5$-I | $R^6$-I |
| $R^1$-III | $R^4$-II | $R^5$-I | $R^6$-II | $R^1$-X | $R^4$-II | $R^5$-I | $R^6$-II |
| $R^1$-III | $R^4$-II | $R^5$-III | $R^6$-I | $R^1$-X | $R^4$-II | $R^5$-III | $R^6$-I |
| $R^1$-III | $R^4$-II | $R^5$-III | $R^6$-II | $R^1$-X | $R^4$-II | $R^5$-III | $R^6$-II |
| $R^1$-III | $R^4$-II | $R^5$-III | $R^6$-III | $R^1$-X | $R^4$-II | $R^5$-III | $R^6$-III |
| $R^1$-III | $R^4$-II | $R^5$-IV | $R^6$-I | $R^1$-X | $R^4$-II | $R^5$-IV | $R^6$-I |
| $R^1$-III | $R^4$-II | $R^5$-IV | $R^6$-II | $R^1$-X | $R^4$-II | $R^5$-IV | $R^6$-II |
| $R^1$-III | $R^4$-II | $R^5$-V | $R^6$-I | $R^1$-X | $R^4$-II | $R^5$-V | $R^6$-I |
| $R^1$-III | $R^4$-II | $R^5$-V | $R^6$-II | $R^1$-X | $R^4$-II | $R^5$-V | $R^6$-II |
| $R^1$-III | $R^4$-II | $R^5$-VI | $R^6$-I | $R^1$-X | $R^4$-II | $R^5$-VI | $R^6$-I |
| $R^1$-III | $R^4$-II | $R^5$-VI | $R^6$-II | $R^1$-X | $R^4$-II | $R^5$-VI | $R^6$-II |
| $R^1$-III | $R^4$-III | $R^5$-III | $R^6$-I | $R^1$-X | $R^4$-III | $R^5$-III | $R^6$-I |
| $R^1$-III | $R^4$-III | $R^5$-III | $R^6$-II | $R^1$-X | $R^4$-III | $R^5$-III | $R^6$-II |
| $R^1$-III | $R^4$-IV | $R^5$-III | $R^6$-I | $R^1$-X | $R^4$-IV | $R^5$-III | $R^6$-I |
| $R^1$-IV | $R^4$-I | $R^5$-I | $R^6$-I | $R^1$-XI | $R^4$-I | $R^5$-I | $R^6$-I |
| $R^1$-IV | $R^4$-I | $R^5$-III | $R^6$-I | $R^1$-XI | $R^4$-I | $R^5$-III | $R^6$-I |
| $R^1$-IV | $R^4$-I | $R^5$-III | $R^6$-II | $R^1$-XI | $R^4$-I | $R^5$-III | $R^6$-II |
| $R^1$-IV | $R^4$-I | $R^5$-VI | $R^6$-I | $R^1$-XI | $R^4$-I | $R^5$-VI | $R^6$-I |
| $R^1$-V | $R^4$-I | $R^5$-I | $R^6$-I | $R^1$-XII | $R^4$-I | $R^5$-I | $R^6$-I |
| $R^1$-V | $R^4$-I | $R^5$-III | $R^6$-I | $R^1$-XII | $R^4$-I | $R^5$-III | $R^6$-I |
| $R^1$-V | $R^4$-I | $R^5$-III | $R^6$-II | $R^1$-XII | $R^4$-I | $R^5$-III | $R^6$-II |
| $R^1$-V | $R^4$-I | $R^5$-VI | $R^6$-I | $R^1$-XII | $R^4$-I | $R^5$-VI | $R^6$-I |
| $R^1$-VI | $R^4$-I | $R^5$-I | $R^6$-I | $R^1$-XIII | $R^4$-I | $R^5$-I | $R^6$-I |
| $R^1$-VI | $R^4$-I | $R^5$-III | $R^6$-I | $R^1$-XIII | $R^4$-I | $R^5$-III | $R^6$-I |
| $R^1$-VI | $R^4$-I | $R^5$-III | $R^6$-II | $R^1$-XIII | $R^4$-I | $R^5$-III | $R^6$-II |
| $R^1$-VI | $R^4$-I | $R^5$-VI | $R^6$-I | $R^1$-XIII | $R^4$-I | $R^5$-VI | $R^6$-I |
| $R^1$-VII | $R^4$-I | $R^5$-I | $R^6$-I | $R^1$-XIV | $R^4$-I | $R^5$-I | $R^6$-I |
| $R^1$-VII | $R^4$-I | $R^5$-I | $R^6$-II | $R^1$-XIV | $R^4$-I | $R^5$-I | $R^6$-II |
| $R^1$-VII | $R^4$-I | $R^5$-I | $R^6$-III | $R^1$-XIV | $R^4$-I | $R^5$-I | $R^6$-III |
| $R^1$-VII | $R^4$-I | $R^5$-II | $R^6$-I | $R^1$-XIV | $R^4$-I | $R^5$-II | $R^6$-I |
| $R^1$-VII | $R^4$-I | $R^5$-II | $R^6$-II | $R^1$-XIV | $R^4$-I | $R^5$-II | $R^6$-II |
| $R^1$-VII | $R^4$-I | $R^5$-III | $R^6$-I | $R^1$-XIV | $R^4$-I | $R^5$-III | $R^6$-I |
| $R^1$-VII | $R^4$-I | $R^5$-III | $R^6$-II | $R^1$-XIV | $R^4$-I | $R^5$-III | $R^6$-II |
| $R^1$-VII | $R^4$-I | $R^5$-III | $R^6$-III | $R^1$-XIV | $R^4$-I | $R^5$-III | $R^6$-III |
| $R^1$-VII | $R^4$-I | $R^5$-IV | $R^6$-I | $R^1$-XIV | $R^4$-I | $R^5$-IV | $R^6$-I |
| $R^1$-VII | $R^4$-I | $R^5$-IV | $R^6$-II | $R^1$-XIV | $R^4$-I | $R^5$-IV | $R^6$-II |

| | | | | | | | |
|---|---|---|---|---|---|---|---|
| $R^1$-VII | $R^4$-I | $R^5$-IV | $R^6$-III | $R^1$-XIV | $R^4$-I | $R^5$-IV | $R^6$-III |
| $R^1$-VII | $R^4$-I | $R^5$-V | $R^6$-I | $R^1$-XIV | $R^4$-I | $R^5$-V | $R^6$-I |
| $R^1$-VII | $R^4$-I | $R^5$-V | $R^6$-II | $R^1$-XIV | $R^4$-I | $R^5$-V | $R^6$-II |
| $R^1$-VII | $R^4$-I | $R^5$-VI | $R^6$-I | $R^1$-XIV | $R^4$-I | $R^5$-VI | $R^6$-I |
| $R^1$-VII | $R^4$-I | $R^5$-VI | $R^6$-II | $R^1$-XIV | $R^4$-I | $R^5$-VI | $R^6$-II |
| $R^1$-VII | $R^4$-I | $R^5$-VI | $R^6$-III | $R^1$-XIV | $R^4$-I | $R^5$-VI | $R^6$-III |
| $R^1$-VII | $R^4$-II | $R^5$-I | $R^6$-I | $R^1$-XIV | $R^4$-II | $R^5$-I | $R^6$-I |
| $R^1$-VII | $R^4$-II | $R^5$-I | $R^6$-II | $R^1$-XIV | $R^4$-II | $R^5$-I | $R^6$-II |
| $R^1$-VII | $R^4$-II | $R^5$-II | $R^6$-I | $R^1$-XIV | $R^4$-II | $R^5$-II | $R^6$-I |
| $R^1$-VII | $R^4$-II | $R^5$-III | $R^6$-I | $R^1$-XIV | $R^4$-II | $R^5$-III | $R^6$-I |
| $R^1$-VII | $R^4$-II | $R^5$-III | $R^6$-II | $R^1$-XIV | $R^4$-II | $R^5$-III | $R^6$-II |
| $R^1$-VII | $R^4$-II | $R^5$-IV | $R^6$-I | $R^1$-XIV | $R^4$-II | $R^5$-IV | $R^6$-I |
| $R^1$-VII | $R^4$-II | $R^5$-IV | $R^6$-II | $R^1$-XIV | $R^4$-II | $R^5$-IV | $R^6$-II |
| $R^1$-VII | $R^4$-II | $R^5$-V | $R^6$-I | $R^1$-XIV | $R^4$-II | $R^5$-V | $R^6$-I |
| $R^1$-VII | $R^4$-II | $R^5$-VI | $R^6$-I | $R^1$-XIV | $R^4$-II | $R^5$-VI | $R^6$-I |
| $R^1$-VII | $R^4$-II | $R^5$-VI | $R^6$-II | $R^1$-XIV | $R^4$-II | $R^5$-VI | $R^6$-II |
| $R^1$-VII | $R^4$-III | $R^5$-I | $R^6$-I | $R^1$-XIV | $R^4$-III | $R^5$-I | $R^6$-I |
| $R^1$-VII | $R^4$-III | $R^5$-I | $R^6$-II | $R^1$-XIV | $R^4$-III | $R^5$-I | $R^6$-II |
| $R^1$-VII | $R^4$-III | $R^5$-III | $R^6$-I | $R^1$-XIV | $R^4$-III | $R^5$-III | $R^6$-I |
| $R^1$-VII | $R^4$-III | $R^5$-III | $R^6$-II | $R^1$-XIV | $R^4$-III | $R^5$-III | $R^6$-II |
| $R^1$-VII | $R^4$-III | $R^5$-IV | $R^6$-I | $R^1$-XIV | $R^4$-III | $R^5$-IV | $R^6$-I |
| $R^1$-VII | $R^4$-III | $R^5$-V | $R^6$-I | $R^1$-XIV | $R^4$-III | $R^5$-V | $R^6$-I |
| $R^1$-VII | $R^4$-III | $R^5$-VI | $R^6$-I | $R^1$-XIV | $R^4$-III | $R^5$-VI | $R^6$-I |
| $R^1$-VII | $R^4$-IV | $R^5$-I | $R^6$-I | $R^1$-XIV | $R^4$-IV | $R^5$-I | $R^6$-I |
| $R^1$-VII | $R^4$-IV | $R^5$-I | $R^6$-II | $R^1$-XIV | $R^4$-IV | $R^5$-I | $R^6$-II |
| $R^1$-VII | $R^4$-IV | $R^5$-III | $R^6$-I | $R^1$-XIV | $R^4$-IV | $R^5$-III | $R^6$-I |
| $R^1$-VII | $R^4$-IV | $R^5$-III | $R^6$-II | $R^1$-XIV | $R^4$-IV | $R^5$-III | $R^6$-II |
| $R^1$-VII | $R^4$-IV | $R^5$-IV | $R^6$-I | $R^1$-XIV | $R^4$-IV | $R^5$-IV | $R^6$-I |
| $R^1$-VII | $R^4$-IV | $R^5$-V | $R^6$-I | $R^1$-XIV | $R^4$-IV | $R^5$-V | $R^6$-I |
| $R^1$-VII | $R^4$-IV | $R^5$-VI | $R^6$-I | $R^1$-XIV | $R^4$-IV | $R^5$-VI | $R^6$-I |

The compounds of the present invention can be prepared, for example, by the following methods.

Preparation method A

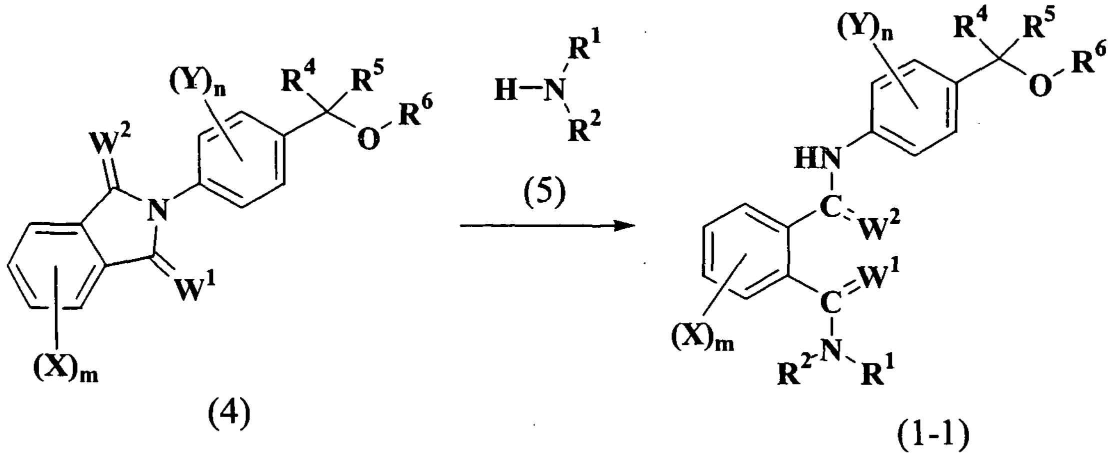

The compound represented by Formula (4) [wherein $W^1$, $W^2$, X, Y, $R^4$, $R^5$, $R^6$, m and n have the same meanings as defined above.] and the compound represented by Formula (5) [wherein $R^1$ and $R^2$ have the same meanings as defined above.] are reacted in a solvent which is inactive to the reaction or in the absence of a solvent, and in the presence of a catalyst if necessary, to obtain the compound of the present invention represented by Formula (1-1) [wherein $W^1$, $W^2$, X, Y, $R^1$, $R^2$, $R^4$, $R^5$, $R^6$, m and n have the same meanings as defined above.] wherein $R^3$ in the formula (1) is a hydrogen atom.

As amounts of the reaction substrates, 1 to 50 equivalents of the compound represented by Formula (5) can be used based on 1 equivalent of the compound represented by Formula (4).

When a solvent is used, the solvent to be used is not limited so long as it does not interfere the progress of the reaction, and there may be mentioned, for example, an aromatic hydrocarbon such as benzene, toluene, xylene, etc., an aliphatic hydrocarbon such as hexane, heptane, etc., an alicyclic hydrocarbon such as cyclohexane, etc., an aromatic halogenated hydrocarbon such as chlorobenzene, dichlorobenzene, etc., an aliphatic halogenated hydrocarbon such as dichloromethane, chloroform, carbon tetrachloride, 1,2-dichloroethane, 1,1,1-trichloroethane, trichloroethylene, tetrachloroethylene, etc., an ether such as diethyl ether, 1,2-dimethoxyethane, tetrahydrofuran, 1,4-dioxane, etc., an ester such as ethyl acetate, ethyl propionate, etc., an amide such as dimethylformamide, dimethylacetamide, N-methyl-2-pyrrolidorle, etc., a carboxylic acid such as formic acid, acetic acid, propionic acid, etc., an amine such as triethylamine, tributyl amine, N,N-dimethylaniline, etc., a pyridine such as pyridine, picoline, etc., an alcohol such as methanol, ethanol, ethylene glycol, etc., acetonitrile, dimethylsulfoxide, sulfolane, 1,3-dimethyl-2-imidazolidinone and water, etc. These solvents may be used alone, or may be used in combination of two or more kinds.

When a catalyst is used, the catalyst for the reaction may be mentioned, for example, mineral acids such as hydrochloric acid, sulfuric acid, nitric acid, etc., organic acids such as formic acid, acetic acid, propionic acid, trifluoroacetic acid, methanesulfonic acid, benzenesulfonic acid, p-toluenesulfonic acid, etc., acid addition salts of an amine such as triethylamine hydrochloride, pyridine hydrochloride, etc., Lewis acids such as zinc chloride, zinc iodide, titanium tetrachloride, cerium chloride, ytterbium trifrate, boron trifluoride-ether complex, etc., in an amount of 0.001 to 1 equivalent based on the compound represented by Formula (4).

The reaction temperature may be set at an optional temperature from -60°C to a reflux temperature of the reaction mixture, and the reaction time may vary depending on the concentrations of the reaction substrates, and the reaction temperature, and may be optionally set usually in the range of 5 minutes to 100 hours.

In general, the reaction is preferably carried out by using, for example, 1 to 10 equivalents of the compound represented by Formula (5) based on 1 equivalent of the compound represented by Formula (4), in the absence of a solvent, or using a solvent such as tetrahydrofuran or 1,4-dioxane, etc., in the temperature range of from 50°C to a reflux temperature of the reaction mixture for 30 minutes to 24 hours.

Preparation method B

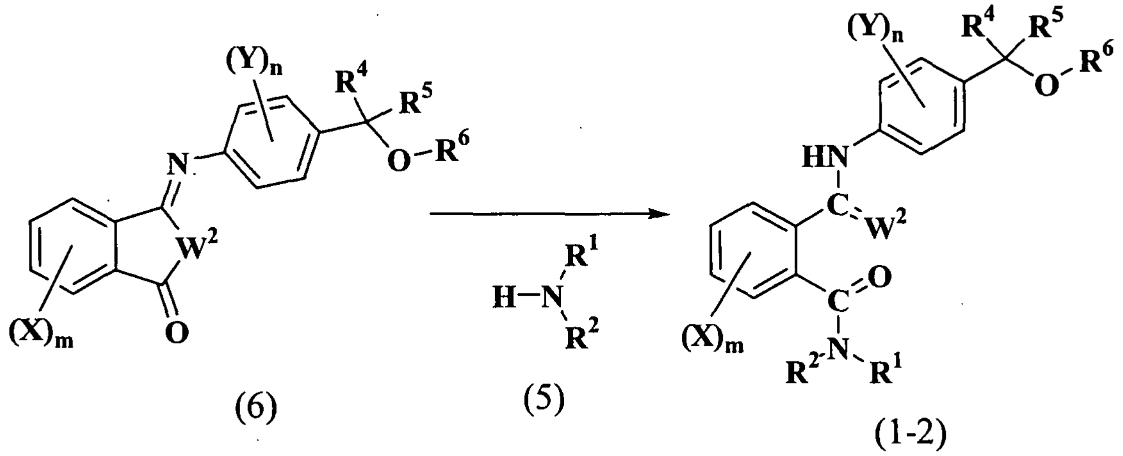

(6) (5) (1-2)

The compound represented by Formula (6) [wherein $W^2$, X, Y, $R^4$, $R^5$, $R^6$, m and n have the same meanings as defined above.] and the compound represented by Formula (5) [wherein $R^1$ and $R^2$ have the same meanings as defined above.] are reacted under the similar conditions as in Preparation method A to obtain the compound of the present invention represented by Formula (1-2) [wherein $W^2$, X, Y, $R^1$, $R^2$, $R^4$, $R^5$, $R^6$, m and n have the same meanings as defined above.] where $W^1$ is an oxygen atom and $R^3$ is a hydrogen atom in Formula (1).

Preparation method C

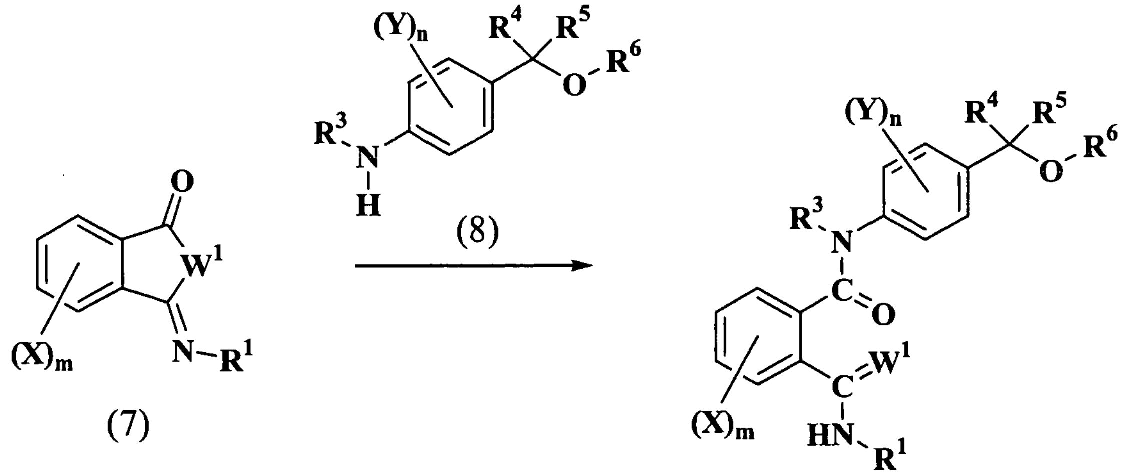

(8) (7) (1-3)

The compound represented by Formula (7) [wherein $W^1$, X, $R^1$ and m have the same meanings as defined above.] and the compound represented by Formula (8) [wherein Y, $R^3$, $R^4$, $R^5$, $R^6$ and n have the same meanings as defined above.] are reacted under the similar conditions as in Preparation method A to obtain the compound of the present invention represented by Formula (1-3) [wherein $W^1$, X, Y, $R^1$, $R^3$, $R^4$, $R^5$, $R^6$, m and n have the same meanings as defined above.] where $W^2$ is an oxygen atom and $R^2$ is a hydrogen atom in Formula (1).

Preparation method D

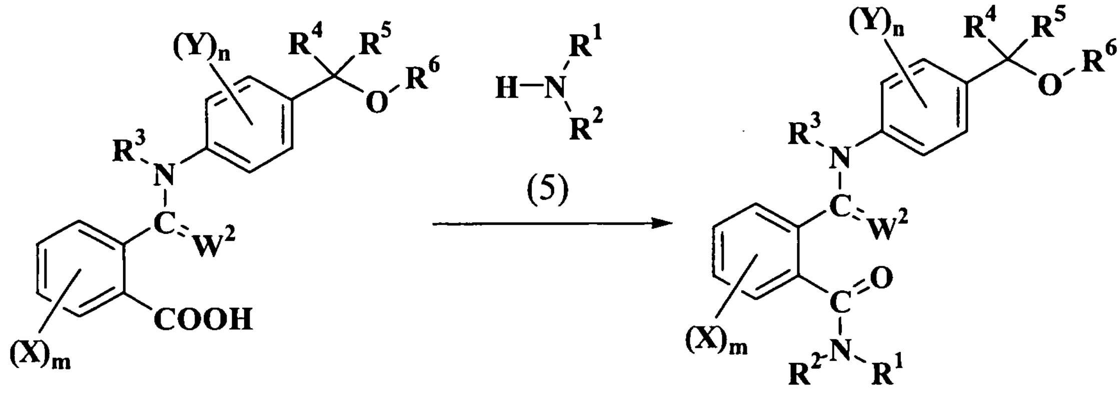

(9) (1-4)

The compound represented by Formula (9) [wherein $W^2$, X, Y, $R^3$, $R^4$, $R^5$, $R^6$, m and n have the same meanings as defined above.] and the compound represented by Formula (5) [wherein $R^1$ and $R^2$ have the same meanings as defined above.] are reacted in a solvent which is inactive to the reaction or in the absence of a solvent, and in the presence of a base if necessary, and using a condensing agent to obtain the compound of the present invention represented by Formula (1-4) [wherein $W^2$, X, Y, $R^1$, $R^2$, $R^3$, $R^4$, $R^5$, $R^6$, m and n have the same meanings as defined above.] where $W^1$ is an oxygen atom in Formula (1).

As amounts of the reaction substrates, 1 to 100 equivalents of the compound represented by Formula (5) can be used based on 1 equivalent of the compound represented by Formula (9).

The condensing agent is not particularly limited so long as it can be used for usual amide synthesis, and there may be mentioned, for example, Mukaiyama reagent (2-chloro-N-methylpyridinium iodide), DCC (1,3-dicyclohexylcarbodiimide), WSC(1-ethyl-3-(3-dimethylaminopropyl)-carbodiimide hydrochloride), a CDI (carbonyldiimidazole), dimethylpropynylsulfonium bromide, propargyltriphenylphosphonium bromide, DEPC (diethyl cyanophosphate), etc., and it can be used in an amount of 1 to 4 equivalents based on the amount of the compound represented by Formula (9).

When a solvent is used, the solvent to be used is not limited so long as it does not interfere the progress of the reaction, and there may be mentioned, for example, aromatic hydrocarbons such as benzene, toluene, xylene, etc., aliphatic hydrocarbons such as hexane, heptane, etc., alicyclic hydrocarbons such as cyclohexane, etc., aromatic halogenated hydrocarbons such as chlorobenzene, dichlorobenzene, etc., aliphatic halogenated hydrocarbons such as dichloromethane, chloroform, carbon tetrachloride, 1,2-dichloroethane, 1,1,1-trichloroethane, trichloroethylene, tetrachloroethylene, etc., ethers such as diethyl ether, 1,2-dimethoxyethane, tetrahydrofuran, 1,4-dioxane, etc., esters such as ethyl acetate, ethyl propionate, etc., amides such as dimethylformamide, dimethylacetamide, N-methyl-2-pyrrolidone, etc., amines such as triethylamine, tributyl amine, N,N-dimethylaniline, etc., pyridines such as pyridine, picoline, etc., acetonitrile and dimethylsulfoxide, etc. These solvents may be used alone, or may be used in combination of two or more kinds

Addition of a base is not necessarily required, and when the base is used, the base to be used may be mentioned, for example, alkali metal hydroxides such as sodium hydroxide, potassium hydroxide, etc., alkali metal carbonates such as sodium carbonate, potassium carbonate, sodium hydrogen carbonate, etc., organic bases such as triethylamine, tributylamine, N,N-dimethylaniline, pyridine, 4-(dimethylamino)pyridine, imidazole, 1,8-diazabicyclo[5,4,0]-7-undecene, etc., and it can be used in an amount of 1 to 4 equivalents based on the amount of the compound represented by Formula (9).

The reaction temperature can be set an optional temperature from -60°C to the reflux temperature of the reaction mixture, and the reaction time may vary depending on the concentrations of the reaction substrates and the reaction temperature, but it can be optionally set usually within the range of from 5 minutes to 100 hours.

In general, the reaction is preferably carried out by using, for example, 1 to 20 equivalents of the compound represented by Formula (5) and 1 to 4 equivalents of a condensing agent such as WSC (1-ethyl-3-(3-dimethylaminopropyl)-carbodiimide hydrochloride), CDI (carbonyldiimidazole), etc., based on 1 equivalent of the compound represented by Formula (9), and if necessary, in the presence of 1 to 4 equivalents of a base such as potassium carbonate, triethylamine, pyridine, 4-(dimethylamino)pyridine, etc., in the absence of a solvent or in the presence of a solvent such as dichloromethane, chloroform, diethyl ether, tetrahydrofuran, 1,4-dioxane, etc., in the range of 0°C to a reflux temperature of these solvents, for 10 minutes to 24 hours. Preparation method E

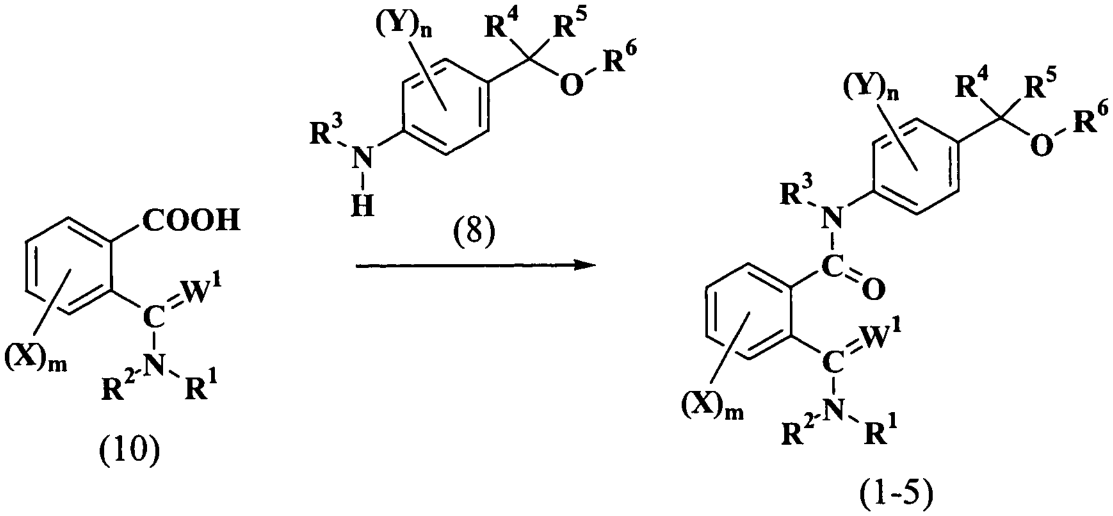

(10)

(8)

(1-5)

The compound represented by Formula (10) [wherein $W^1$, X, $R^1$, $R^2$ and m have the same meanings as defined above.] and the compound represented by Formula (8) [wherein Y, $R^3$, $R^4$, $R^5$, $R^6$ and n have the same meanings as defined above.] are reacted under the similar conditions as in Preparation method D to obtain the compound of the present invention represented by Formula (1-5) [wherein $W^1$, X, Y, $R^1$, $R^2$, $R^3$, $R^4$, $R^5$, $R^6$, m and n have the same meanings as defined above.] where $W^2$ is an oxygen atom in Formula (1).

Preparation method F

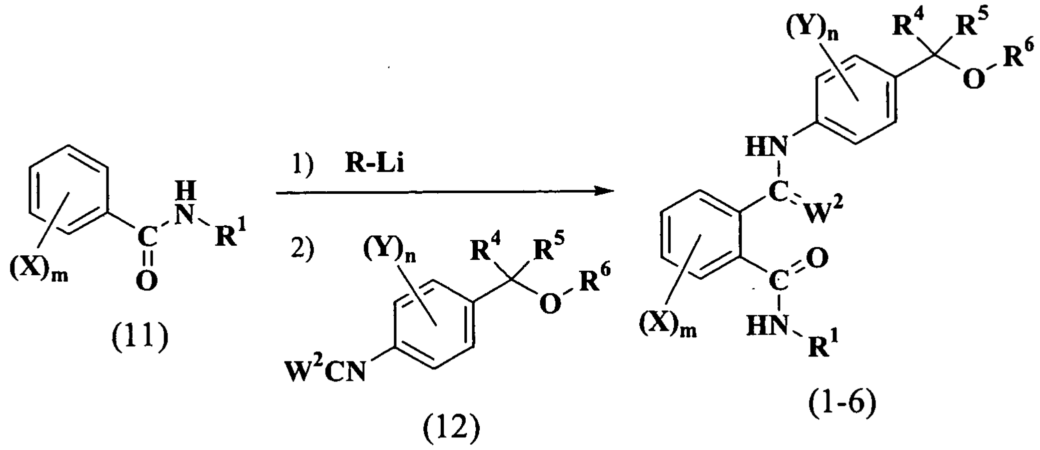


(11)

(12)

(1-6)

The compound represented by Formula (11) [wherein X, $R^1$ and m have the same meanings as defined above.] is subjected to selective lithiation according to the conventionally known method described in a literature, for example, the method described in Chemical Reviews [Chem. Rev.] 1990, vol. 90, p. 879, etc., and then, reacting with the compound represented by Formula (12) [wherein $W^2$, Y, $R^4$, $R^5$, $R^6$ and n have the same meanings as defined above.] to obtain the compound of the present invention represented by Formula (1-6) [wherein $W^2$, X, Y, $R^1$, $R^4$, $R^5$, $R^6$, m and n have the same meanings as defined above.] where $W^1$ is an oxygen atom, and $R^2$ and $R^3$ are hydrogen atoms in Formula (1). Incidentally, R-Li represents an alkyl lithium reagent such as butyl lithium, etc. Preparation method G

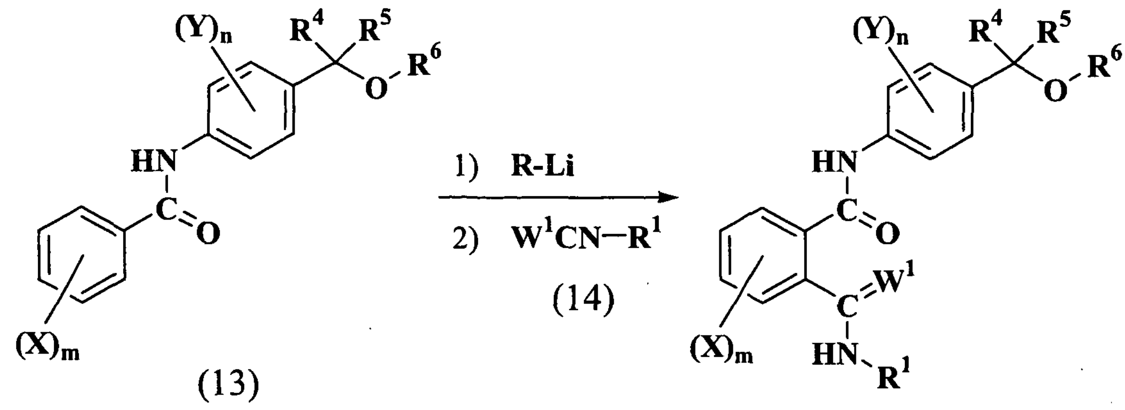


(13) (1-7)

The compound represented by Formula (13) [wherein X, Y, $R^4$, $R^5$, $R^6$, m and n have the same meanings as defined above.] and the compound represented by Formula (14) [wherein $W^1$ and $R^1$ have the same meanings as defined above.] are reacted under similar conditions as in Preparation method F to obtain the compound of the present invention represented by Formula (1-7) [wherein $W^1$, X, Y, $R^1$, $R^4$, $R^5$, $R^6$, m and n have the same meanings as defined above.] where $W^2$ is an oxygen atom, and $R^2$ and $R^3$ are hydrogen atoms in Formula (1). Incidentally, R-Li represents an alkyl lithium reagent such as butyl lithium, etc. Preparation method H

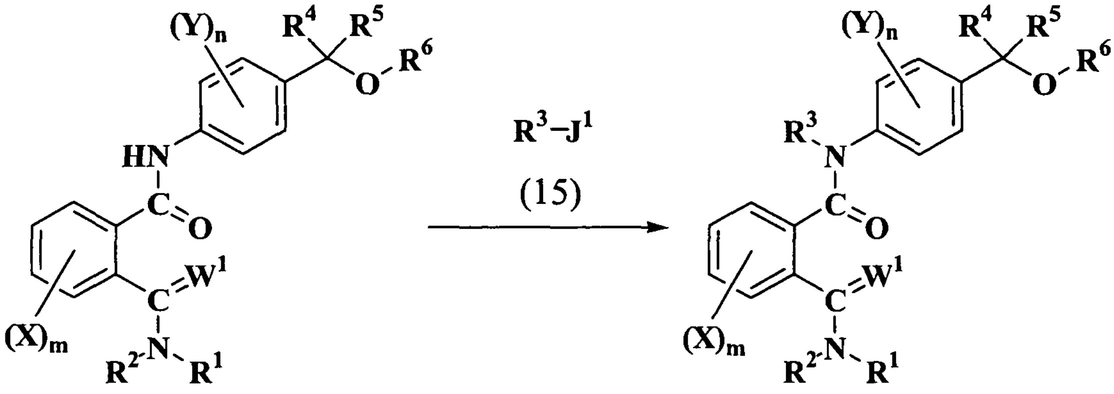


(1-8) (1-5)

The compound of the present invention represented by Formula (1-8) [wherein $W^1$, X, Y, $R^1$, $R^2$, $R^4$, $R^5$, $R^6$, m and n have the same meanings as defined above.] where $W^2$ is an oxygen atom, and $R^3$ is a hydrogen atom in the compound represented by Formula (1) and the compound represented by Formula (15) [wherein $R^3$ has the same meaning as defined above, and $J^1$ represents a good eliminating group such as a chlorine atom, a bromine atom, an iodine atom, a $C_1$ to $C_4$ alkylcarbonyloxy group (for example, a pivaloyloxy group), a $C_1$ to $C_4$ alkylsulfonate group (for example, a methane-sulfonyloxy group), a $C_1$ to $C_4$ haloalkylsulfonate group (for example, a trifluoromethanesulfonyloxy group), an arylsulfonate group (for example, a benzenesulfonyloxy group, p-toluenesulfonyloxy group) or an azolyl group (for example, an imidazol-1-yl group), etc.] are reacted, if necessary, in the presence of a base, and if necessary, by using a solvent which is inactive to said reaction to obtain the compound of the present invention represented by Formula (1-5) [wherein $W^1$, X, Y, $R^1$, $R^2$, $R^3$, $R^4$, $R^5$, $R^6$, m and n have the same meanings as defined above.] where $W^1$ is an oxygen atom in Formula (1).

As amounts of the reaction substrates, 1 to 50 equivalents of the compound represented by Formula (15) can be used based on 1 equivalent of the compound represented by Formula (1-8).

When a solvent is used, the solvent to be used is not limited so long as it does not interfere the progress of the reaction, and there may be mentioned, for example, aromatic hydrocarbons such as benzene, toluene, xylene, etc., aliphatic hydrocarbons such as hexane, heptane, etc., alicyclic hydrocarbons such as cyclohexane, etc., aromatic halogenated hydrocarbons such as chlorobenzene, dichlorobenzene, etc., aliphatic halogenated hydrocarbons such as

dichloromethane, chloroform, carbon tetrachloride, 1,2-dichloroethane, 1,1,1-trichloroethane, trichloroethylene, tetrachloroethylene, etc., ethers such as diethyl ether, 1,2-dimethoxyethane, tetrahydrofuran, 1,4-dioxane, etc., esters such as ethyl acetate, ethyl propionate, etc., amides such as dimethylformamide, dimethylacetamide, N-methyl-2-pyrrolidone, etc., amines such as triethylamine, tributyl amine, N,N-dimethylaniline, etc., pyridines such as pyridine, picoline, etc., alcohols such as methanol, ethanol, ethylene glycol, etc., acetonitrile, dimethylsulfoxide, sulfolane, 1,3-dimethyl-2-imidazolidinone and water, etc. These solvents may be used alone, or may be used in combination of two or more kinds

When a base is used, as the base to be used, there may be mentioned, for example, alkali metal hydrides such as sodium hydride, potassium hydrides, etc., alkali metal hydroxides such as sodium hydroxide, potassium hydroxide, etc., alkali metal alkoxides such as sodium ethoxide, potassium tert-butoxide, etc., alkali metal amides such as lithium diisopropylamide, lithium hexamethyldisilazane, sodium amide, etc., organic metal compounds such as tertiary butyl lithium, etc., alkali metal carbonates such as sodium carbonate, potassium carbonate, sodium hydrogen carbonate, etc., organic bases such as triethylamine, tributylamine, N,N-dimethylaniline, pyridine, 4-(dimethylamino)pyridine, imidazole, 1,8-diazabicyclo[5,4,0]-7-undecene, etc., and it can be used in an amount of 1 to 4 equivalents based on the amount of the compound represented by Formula (1-8).

The reaction temperature can be set an optional temperature from -60°C to the reflux temperature of the reaction mixture, and the reaction time may vary depending on the concentrations of the reaction substrates, and the reaction temperature, but it can be optionally set usually within the range of from 5 minutes to 100 hours.

In general, the reaction is preferable carried out by using, for example, 1 to 10 equivalents of the compound represented by Formula (15) based on 1 equivalent of the compound represented by Formula (1-8), in tetrahydrofuran, 1,4-dioxane, acetonitrile or a polar solvent such as N,N-dimethylformamide, etc., and if necessary, by using 1 to 3 equivalents of a base such as sodium hydride, potassium tert-butoxide, potassium hydroxide, potassium carbonate, triethylamine or pyridine, etc. based on 1 equivalent of the compound represented by Formula (1-8) in a temperature range of 0 to 90°C for 10 minutes to 24 hours. Preparation method I

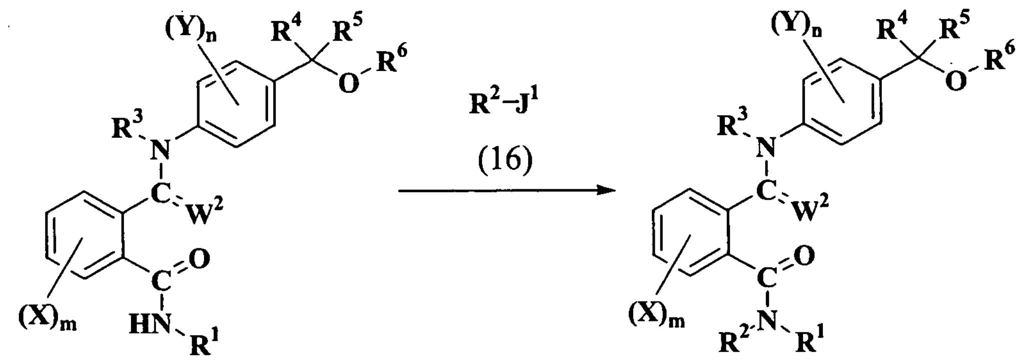


(1-9) (1-4)

The compound of the present invention represented by Formula (1-9) [wherein $W^2$, X, Y, $R^1$, $R^3$, $R^4$, $R^5$, $R^6$, m and n have the same meanings as defined above.] where $W^2$ is an oxygen atom and $R^2$ is a hydrogen atom in Formula (1) and the compound represented by Formula (16) [wherein $R^2$ and $J^1$ have the same meanings as defined above.] are reacted under the similar conditions as in Preparation method H to obtain the compound of the present invention represented by Formula (1-4) [wherein $W^2$, X, Y, $R^1$, $R^2$, $R^3$, $R^4$, $R^5$, $R^6$, m and n have the same meanings as defined above.] where $W^1$ is an oxygen atom in Formula (1).

In Preparation method A to Preparation method I, the reaction mixture after completion of the reaction can be subjected to usual post-treatment such as direct concentration, or dissolution in an organic solvent, and after washing with water, subjecting to concentration, or pouring in ice-water, extraction with an organic solvent and then concentration, and the like, to obtain the objective compound of the present invention. Also, when purification is required to be carried out, it can be separated and purified by optional purification methods such as recrystallization, column chromatography, thin layer chromatography, fractionation by liquid chromatography, and the like.

In Preparation method A, the compound represented by Formula (4-1) which is a compound where $W^1$ and $W^2$ are oxygen atoms in the compound represented by Formula (4), which are starting compound for preparing the compound of the present invention, can be synthesized as mentioned below.

Reaction formula 1

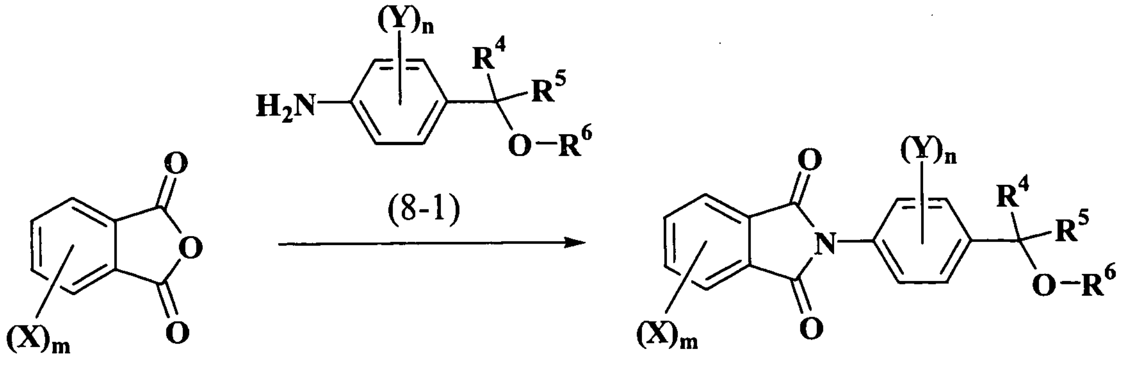

(8-1)

(17) (4-1)

That is, the compound represented by Formula (17) [wherein X and m have the same meanings as defined above.] and the compound represented by Formula (8-1) [wherein Y, $R^4$, $R^5$, $R^6$ and n have the same meanings as defined above.] where $R^3$ is a hydrogen atom in the compound represented by Formula (8) are reacted in accordance with the conventionally known method described in literatures, for example, the methods as described in Berichte der Deutschen Chemischen Gesellschaft [Ber. Dtsch. Chem. Ges.] 1907, vol. 40, p. 3177, Journal of the Chemical Society [J. Chem. Soc.] 1954, p. 2023, Journal of the Chemical Society Perkin Transactions, 1 [J. Chem. Soc. Perkin Trans. 1] 1994, p. 2975, etc., the compound represented by Formula (4-1) [wherein X, Y, $R^4$, $R^5$, $R^6$, m and n have the same meanings as defined above.] where $W^1$ and $W^2$ are oxygen atoms in the compound represented by Formula (4) can be easily synthesized.

Some of the compounds represented by Formula (5) used in Preparation method A, Preparation method B and Preparation method D are conventionally known compounds, and some of which can be obtained as commercially available products. Also, those other than the above can be synthesized according to the methods described in, for example, the methods as described in Chemical and Pharmaceutical Bulletin [Chem. Pharm. Bull.] 1982, vol. 30, p. 1921, Journal of the American Chemical Society [J. Am. Chem. Soc.] 1986, vol. 108, p. 3811, International Patent Publication (WO 01/23350), etc., and the respective synthetic methods of the other primary or secondary alkylamines described in literatures.

In Preparation method B, the compound represented by Formula (6) which is a starting compound for preparing the compound of the present invention can be synthesized as mentioned below.

Reaction formula 2

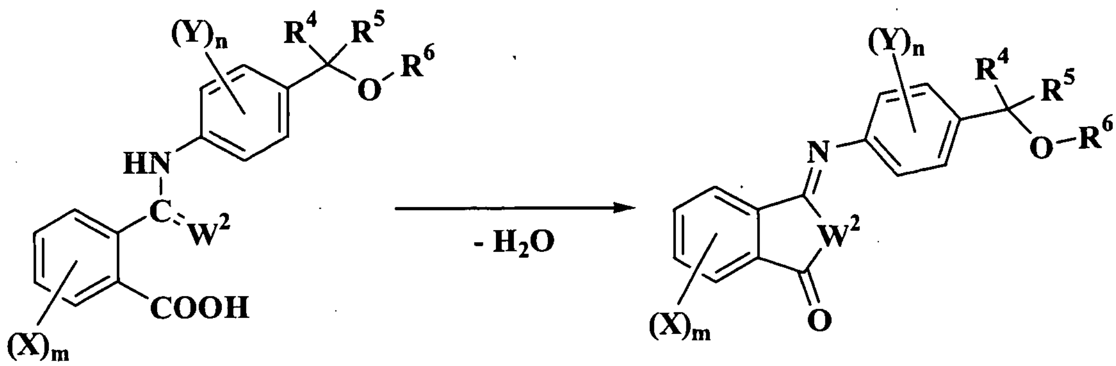


(9-1) (6)

That is, the compound represented by Formula (9-1) [wherein $W^2$, X, Y, $R^4$, $R^5$, $R^6$, m and n have the same meanings as defined above.] where $R^3$ is a hydrogen atom in the compound represented by Formula (9) is subjected to cyclization according to the methods such as a synthetic method of isoimide by general dehydration and cyclization

described in literatures, for example, the methods described in Journal of the American Chemical Society [J. Am. Chem. Soc.] 1975, vol. 97, p. 5582, Journal of Medicinal Chemistry [J. Med. Chem.] 1967, vol. 10, p. 982, The Journal of Organic Chemistry [J. Org. Chem.] 1963, vol. 28, p. 2018, etc., the compound represented by Formula (6) [wherein $W^2$, X, Y, $R^4$, $R^5$, $R^6$, m and n have the same meanings as defined above.] can be easily synthesized.

In Preparation method C, the compound represented by Formula (7) which is a starting compound for preparing the compound of the present invention can be synthesized as mentioned below.

Reaction formula 3

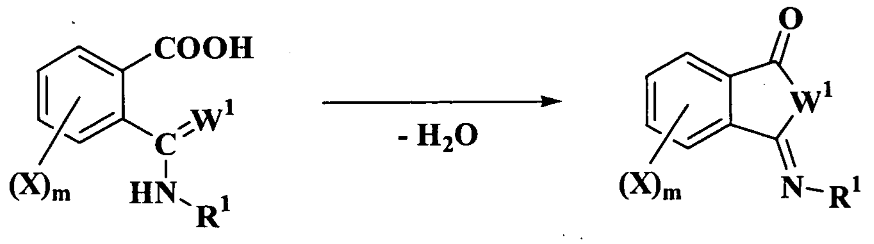


(10-1) (7)

That is, the compound represented by Formula (10-1) [wherein $W^1$, X, $R^1$ and m have the same meanings as defined above.] wherein $R^2$ is a hydrogen atom in Formula (10) is reacted in the same manner as in Reaction formula 2, the compound represented by Formula (7) [wherein $W^1$, X, $R^1$ and m have the same meanings as defined above.] can be easily synthesized.

The compound represented by Formula (8) used in Preparation method C and Preparation method E can be synthesized by, for example, using the method shown by the following Reaction formula 4 to Reaction formula 7 and the like.

Reaction formula 4

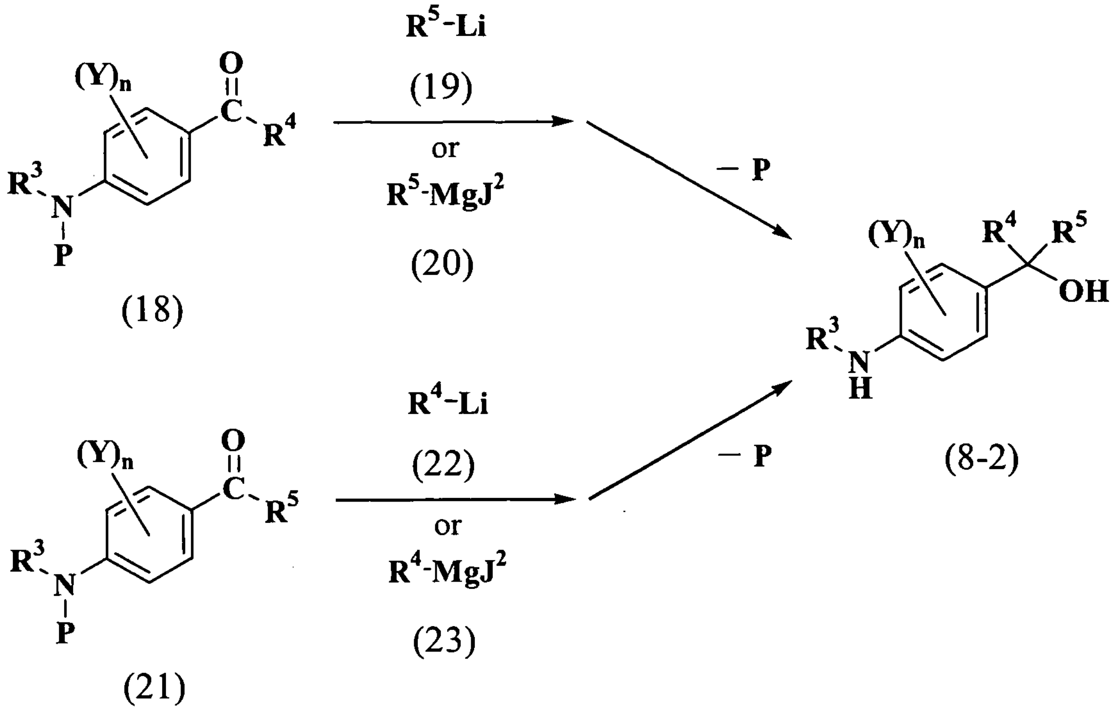


(18) (21) (8-2)

The compound represented by Formula (18) [wherein Y, $R^3$, $R^4$ and n have the same meanings as defined

above, and P represents a protective group for an amino group generally employed such as acetyl group, pivaloyl group, benzoyl group, tert-butoxycarbonyl group, benzyloxycarbonyl group, etc.] and a lithium reagent of the compound represented by Formula (19) [wherein $R^5$ has the same meanings as defined above.] or a Grignard reagent of the compound represented by Formula (20) [wherein $R^5$ has the same meaning as mentioned above, and $J^2$ represents a halogen atom such as a bromine atom, an iodine atom, etc.] are reacted according to the conventionally knowm method described in literatures, for example, Heterocycles [Heterocycles] 1994, vol. 39, p. 801, Journal of the American Chemical Society [J. Am. Chem. Soc.] 1988, vol. 110, p. 1862, Tetrahedron [Tetrahedron] 1960, vol. 11, p. 252, Tetrahedron Letters [Tetrahedron Lett.] 1995, vol. 36, p. 9117, etc., and subjecting to deprotection by the method generally employed for the respective protective groups, so that the compound represented by Formula (8-2) [wherein Y, $R^3$, $R^4$, $R^5$ and n have the same meanings as defined above.] wherein $R^6$ is a hydrogen atom in Formula (8) can be obtained.

Also, the compound represented by Formula (21) [wherein Y, $R^3$, $R^5$, n and P have the same meanings as defined above.] and a lithium reagent of the compound represented by Formula (22) [wherein $R^4$ has the same meaning as defined above.] or a Grignard reagent of the compound represented by Formula (23) [wherein $R^4$ and $J^2$ have the same meanings as defined above.] are reacted under the similar conditions as mentioned above, the compound represented by Formula (8-2) can be obtained.

Some of the lithium reagents represented by Formula (19) and by Formula (22) herein used are known compounds, and part thereof can be obtained as a commercially available product. Also, other compounds than the above can be easily synthesized from the conventionally known halogenated compounds according to the general halogen-metal exchange reaction as described in the literatures, for example, Bulletin of the Chemical Society of Japan [Bull. Chem. Soc. Jpn.] 1990, vol. 63, p. 3719, The Journal of Organic Chemistry [J. Org. Chem.] 1983, vol. 48, p. 1449, etc.

Also, some of the Grignard reagents represented by Formula (20) and by Formula (23) herein used are known compounds, and part thereof can be obtained as a commercially available product. Also, other compounds than the above can be easily synthesized from the conventionally known halogenated compounds according to the general preparation methods of Grignard reagents as described in the literatures, for example, Angewante Chemie international Edition in English [Angew. Chem. Int. Ed. Engl.] 1969, vol. 8, p. 279, The Journal of Organic Chemistry [J. Org. Chem.] 1989, vol. 54, p. 4413, etc. from a conventionally known halogenated compound.

Reaction formula 5

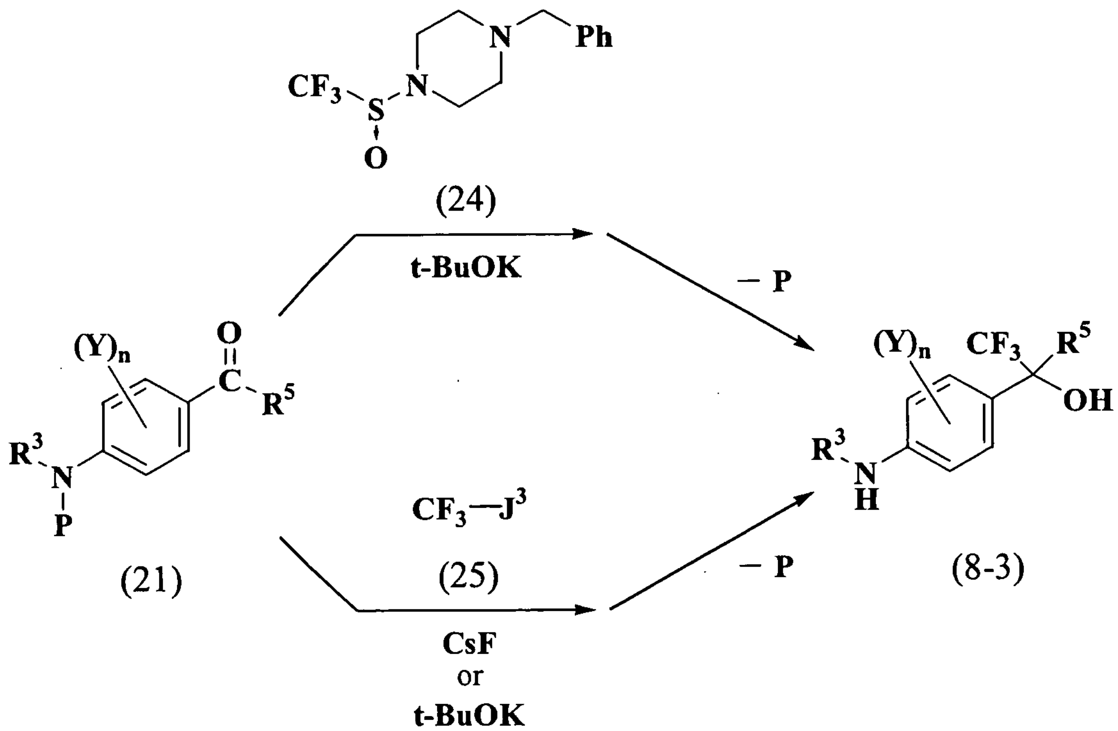


The compound represented by Formula (21) [wherein Y, $R^3$, $R^5$, n and P have the same meanings as defined above.] is reacted with the compound represented by Formula (24) by the conventionally known method described in literatures, for example, according to the method described in Synlett [Synlett] 2003, p. 233, or reacted with the conventionally known compound represented by Formula (25) [wherein $J^3$ represents a hydrogen atom, a bromine atom or a trimethylsilyl group.] by the conventionally known method described in literatures, for example, according to the method

described in The Journal of Organic Chemistry [J. Org. Chem.] 2000, vol. 65, p. 8848 and 2001, vol. 66, p. 1436, Tetrahedron [Tetrahedron] 1989, vol. 45, p. 1423, etc., and then subjecting to deprotection by the method generally employed for the respective protective groups, so that the compound represented by Formula (8-3) [wherein Y, $R^3$, $R^5$ and n have the same meanings as defined above.] wherein $R^4$ is a trifluoromethyl group, and $R^6$ is a hydrogen atom in Formula (8) can be obtained.

Reaction formula 6

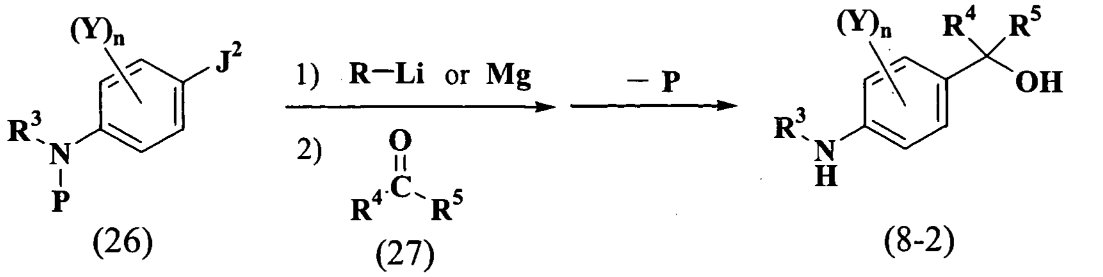


(26) (27) (8-2)

The compound represented by Formula (26) [wherein Y, $R^3$, n, $J^2$ and P have the same meanings as defined above.] is subjected according to the general method described in literatures, for example, lithiation and then, reacting with the compound represented by Formula (27) [wherein $R^4$ and $R^5$ have the same meanings as defined above.] as dsiclosed in Tetrahedron Letters [Tetrahedron Lett.] 1995, vol. 36, p. 9117, etc., or form a Grignard reagent, and then reacting the resulting material with the compound represented by Formula (27) according to the method as disclosed in Tetrahedron [Tetrahedron] 1960, vol. 11, p. 252, The Journal of Organic Chemistry [J. Org. Chem.] 1988, vol. 53, p. 754. etc., and then subjecting to deprotection by the method generally employed for the respective protective groups, so that the compound represented by Formula (8-2) [wherein Y, $R^3$, $R^4$, $R^5$ and n have the same meanings as defined above.] wherein $R^6$ is a hydrogen atom in Formula (8) can be obtained. Incidentally, R-Li represents an alkyl lithium reagent such as butyl lithium, etc.

Reaction formula 7

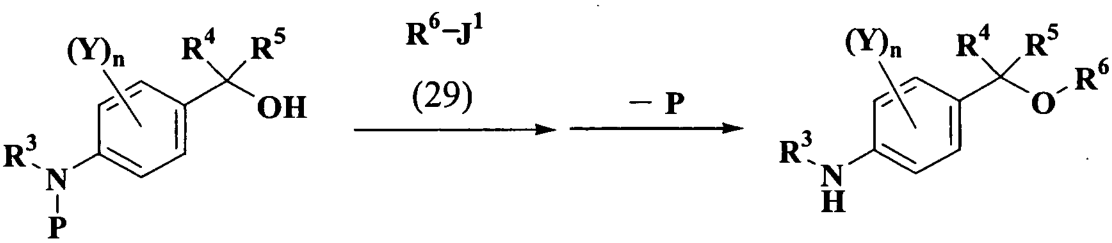


(28) (8)

The compound represented by Formula (28) [wherein Y, $R^3$, $R^4$, $R^5$, n and P have the same meanings as defined above.] which is used as an intermediate in Reaction formula 4, Reaction formula 5 and Reaction formula 6 and the compound represented by Formula (29) [wherein $R^6$ and $J^1$ have the same meanings as defined above.] are reacted under the same conditions as in Preparation method H, and then subjecting to deprotection by the method generally employed for the respective protective groups, so that the compound represented by Formula (8) [wherein Y, $R^3$, $R^4$, $R^5$ and n have the same meanings as mentioned above, and $R^6$ represents the above-mentioned meaning except for the hydrogen atom.] can be obtained.

In Preparation method D, the compound represented by Formula (9) which is a starting material for preparing the compounds of the present invention can be synthesized by the method as disclosed in, for example, the method represented by the following Reaction formula 8 or Reaction formula 9, etc.

Reaction formula 8

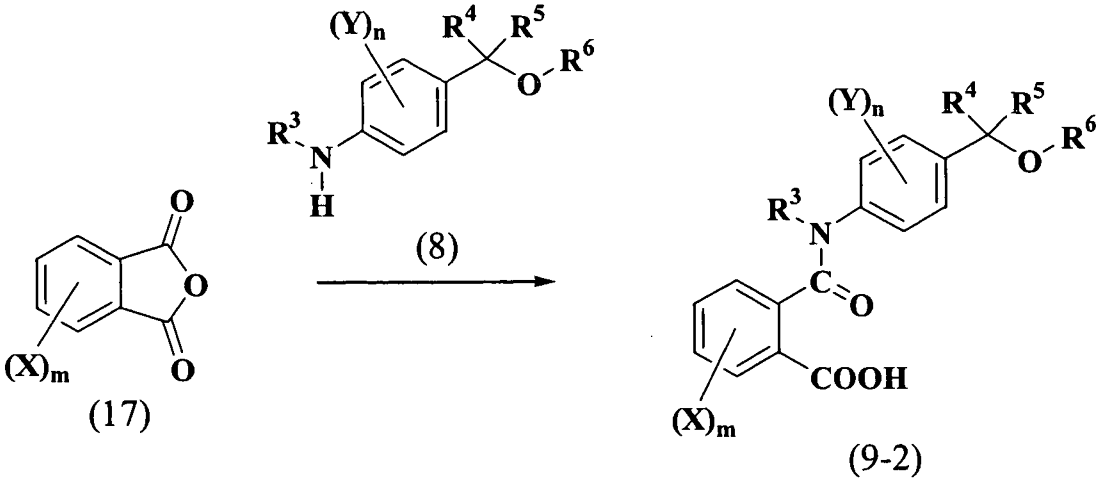


(17) (9-2)

The compound represented by Formula (17) [wherein X and m have the same meanings as defined above.] and the compound represented by Formula (8) [wherein Y, $R^3$, $R^4$, $R^5$, $R^6$ and n have the same meanings as defined above.] are reacted under the similar conditions as in Preparation method A, the compound represented by Formula (9-2) [wherein X, Y, $R^3$, $R^4$, $R^5$, $R^6$, m and n have the same meanings as defined above.] wherein $W^2$ is an oxygen atom in the compound represented by Formula (9) can be obtained.

Reaction formula 9

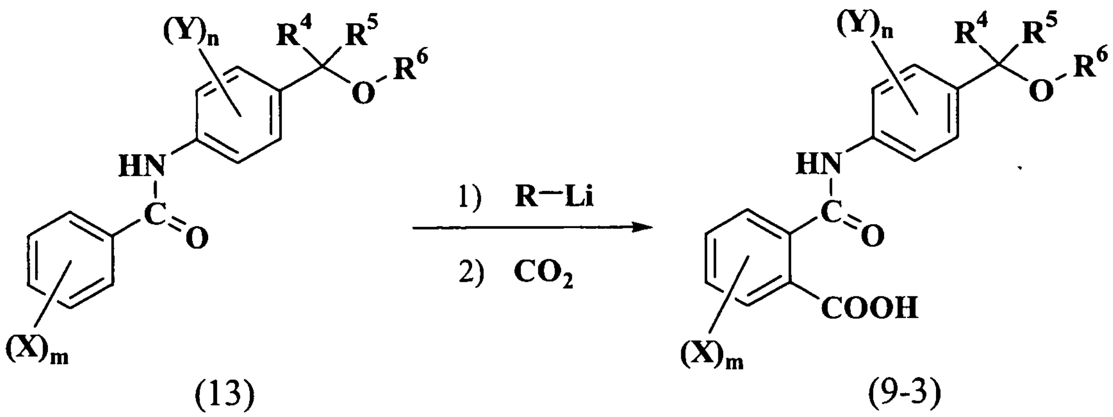


(13) (9-3)

The compound represented by Formula (13) [wherein X, Y, $R^4$, $R^5$, $R^6$, m and n have the same meanings as defined above.] is subjected to selective lithiation according to the conventionally known method described in a literature, for example, the method described in Chemical Reviews [Chem. Rev.] 1990, vol. 90, p. 879, etc., and then, reacting with carbon dioxide to obtain the compound of the present invention represented by Formula (9-3) [wherein X, Y, $R^4$, $R^5$, $R^6$, m and n have the same meanings as defined above.] where $W^2$ is an oxygen atom, and $R^3$ is a hydrogen atom in the compound represented by Formula (9). Incidentally, R-Li represents an alkyl lithium reagent such as butyl lithium, etc.

In Preparation method E, the compound represented by Formula (10) which is a starting compound for preparing the compound of the present invention can be synthesized, for example, by the method represented by the following Reaction formula 10 or Reaction formula 11.

Reaction formula 10

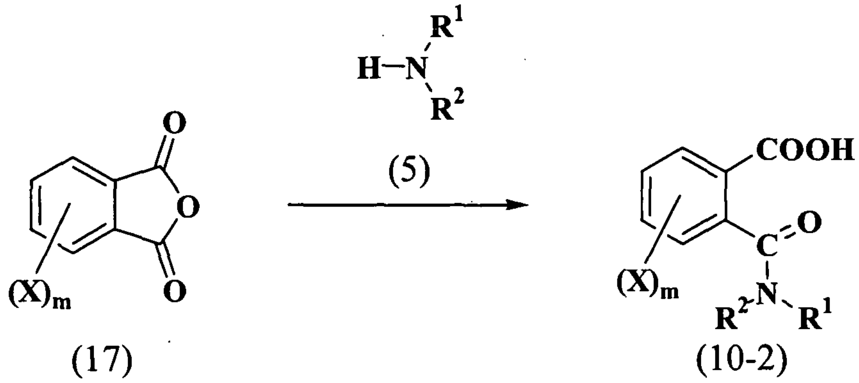


The compound represented by Formula (17) [wherein X and m have the same meanings as defined above.] and the compound represented by Formula (5) [wherein $R^1$ and $R^2$ have the same meanings as defined above.] are reacted under the same conditions as in Preparation method A to prepare the compound represented by Formula (10-2) [wherein X, $R^1$, $R^2$ and m have the same meanings as defined above.] wherein $W^1$ is an oxygen atom in the compound represented by Formula (10) can be obtained.

Reaction formula 11

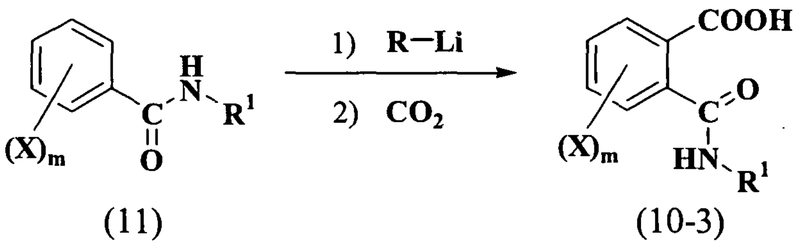


The compound represented by Formula (11) [wherein X, $R^1$ and m have the same meanings as defined above.] is reacted in the same manner as in Reaction formula 12, the compound represented by Formula (10-3) [wherein X, $R^1$ and m have the same meanings as defined above.] wherein $W^1$ is an oxygen atom, and $R^2$ is a hydrogen atom in Formula (10) can be obtained. Incidentally, R-Li represents an alkyl lithium reagent such as butyl lithium, etc.

In Preparation method F, some of the compounds represented by Formula (11) which are starting compounds for preparing the compounds of the present invention are known compounds and part thereof can be obtained as commercially available product. Also, those other than the above can be easily synthesized according to the conventionally known methods described in literatures, for example, the methods as described in Bulletin of the Chemical Society of Japan [Bull. Chem. Soc. Jpn.] 1985, vol. 58, p. 3291, The Journal of Organic Chemistry [J. Org. Chem.] 1991, vol. 56, p. 2395, Tetrahedron Letters [Tetrahedron Lett.] 1994, vol. 35, p. 2113, International Patent Publication (WO 98/23581), etc.

The compound represented by Formula (12) to be used in Preparation method F can be synthesized as follows.

Reaction formula 12

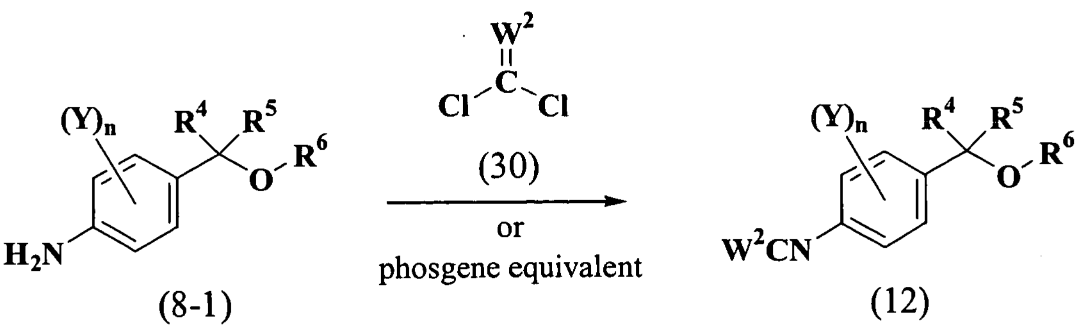

That is, the compound represented by Formula (8-1) [wherein Y, $R^4$, $R^5$, $R^6$ and n have the same meanings as defined above.] wherein $R^3$ is a hydrogen atom in Formula (8) and commercially available phosgene, thiophosgene represented by Formula (30) [wherein $W^2$ represents an oxygen atom or sulfur atom.], or equivalents thereof are reacted according to the conventionally known method described in literatures, for example, the method described in Angewante Chemie international Edition in English [Angew. Chem. Int. Ed. Engl.] 1987, vol. 26, p. 894 and 1995, vol. 34, p. 2497, The Journal of Organic Chemistry [J. Org. Chem.] 1976, vol. 41, p. 2070, Synthesis [Synthesis] 1988, p. 990, Tetrahedron Letters [Tetrahedron Lett.] 1997, vol. 38, p. 919, etc., the compound represented by Formula (12) [wherein $W^2$, Y, $R^4$, $R^5$, $R^6$ and n have the same meanings as defined above.] can be easily synthesized.

In Preparation method G, the compound represented by Formula (13) which is a starting compound for preparing the compound of the present invention can be synthesized as follows.

Reaction formula 13

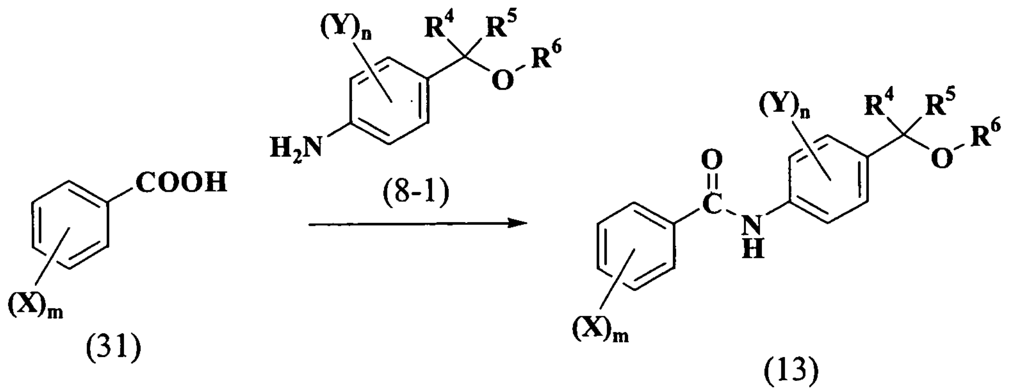

That is, the compound represented by Formula (31) [wherein X and m have the same meanings as defined above.] and the compound represented by Formula (8-1) [wherein Y, $R^4$, $R^5$, $R^6$ and n have the same meanings as defined above.] wherein $R^3$ is a hydrogen atom in the compound represented by Formula (8) are reacted under the same conditions as in Preparation method D, or the compound represented by Formula (31) is converted into a corresponding carboxylic acid chloride using the conventionally knowm methods (for example, a chlorinating agent such as thionyl chloride, phosphorus pentachloride or oxalyl chloride, etc.), and then, reacting with the compound represented by Formula (8-1), whereby the compound represented by Formula (13) [wherein X, Y, $R^4$, $R^5$, $R^6$, m and n have the same meanings as defined above.] can be easily synthesized.

The compound represented by Formula (31) herein used is a conventionally known compound, and some of which can be obtained as a commercially available product.

Some of the compounds represented by Formula (14) used in Preparation method G are conventionally known compounds, and some of which can be obtained as commercially available products. Also, those other than the above can be easily synthesized according to the methods described in, for example, The Journal of Organic Chemistry [J. Org. Chem.] 1996, vol. 61, p. 3883, p. 3929 and p. 6575, Tetrahedron Letters [Tetrahedron Lett.] 1999, vol. 40, p. 363 and p. 6121, etc.

Some of the compounds represented by Formula (15) used in Preparation method H, some of the compounds represented by Formula (16) used in Preparation method I and some of the compound represented by Formula (29) used in Reaction formula 7 are conventionally known compounds, and some of which can be obtained as commercially available products. Also, those other than the above can be easily synthesized according to the methods described in, for example, Chemistry Letters [Chem. Lett.] 1976, p. 373, Journal of The American Chemical Society [J. Am. Chem. Soc.] 1964, vol. 86, p. 4383, The Journal of Organic Chemistry [J. Org. Chem.] 1976, vol. 41, p. 4028 and 1978, vol. 43, p. 3244, Organic Synthesis [Org. Synth.] 1988, Collective Volume 6, p. 101, Tetrahedron Letters [Tetrahedron Lett.] 1972, p. 4339, British Patent (GB 2,161,802 B), European Patent (EP 0,051,273 B), etc.

Some of the compounds represented by Formula (17) are conventionally known compounds, and some of which can be obtained as commercially available products. Also, those other than the above can be easily synthesized, for example, as follows.

Reaction formula 14

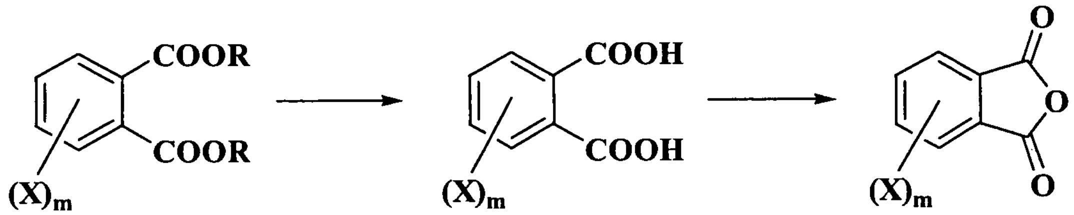

(32) (33) (17)

That is, the compound represented by Formula (32) [wherein X and m have the same meanings as defined above, and R represents a lower alkyl group such as methyl group, ethyl group, etc.] is subjected to a general hydrolysis reaction described in literatures, for example, the methods as described in Angewante Chemie [Angew. Chem.] 1951, vol. 63, p. 329, Journal of the American Chemical Society [J. Am. Chem. Soc.] 1929, vol. 51, p. 1865, etc. to form a phthalic acid derivative represented by Formula (33) [wherein X and m have the same meanings as defined above.], and then, subjecting to a general dehydration and cyclization reaction under the conditions described in, for example, the methods as described in The Journal of Organic Chemistry [J. Org. Chem.] 1987, vol. 52, p. 129, etc., so that the compound represented by Formula (17) [wherein X and m have the same meanings as defined above.] can be obtained.

The compound represented by Formula (32) herein used is a conventionally known compound, and some of which can be obtained as a commercially available product.

The compound represented by Formula (18) can be synthesized, for example, as follows.

Reaction formula 15

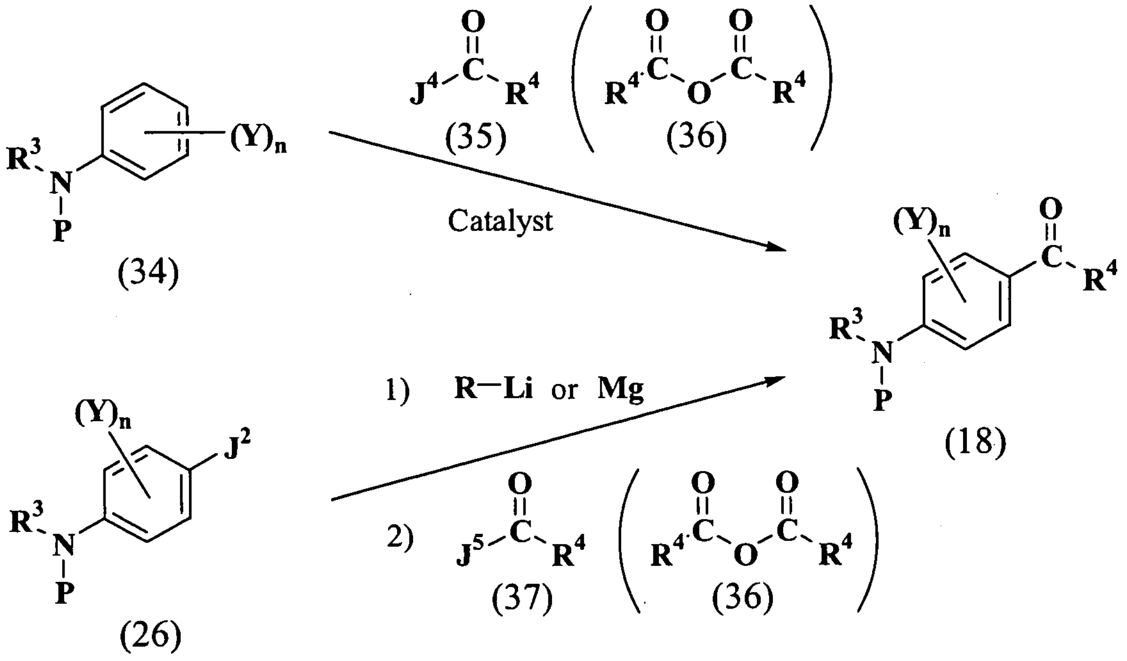


(35) (36) (34) (18) (37) (36) (26)

That is, the conventionally known compound represented by Formula (34) [wherein Y, $R^3$, n and P have the same meanings as defined above.] and the conventionally known compound represented by Formula (35) [wherein $R^4$ has the same meanings as defined above, $J^4$ represents an eliminating group such as a halogen atom, trifluoromethanesulfonyloxy group, 2-pyridyloxy group, etc.] or the conventionally known compound represented by Formula (36) [wherein $R^4$ have the same meanings as defined above.] are reacted by a general acylation reaction of the aromatic ring according to the methods described in literatures, for example, the methods as described in Chemistry Letters [Chem. Lett.] 1990, p. 783, The Journal of Organic Chemistry [J. Org. Chem.] 1991, vol. 56, p. 1963, etc., so that the compound represented by Formula (18) [wherein Y, $R^3$, $R^4$, n and P have the same meanings as defined above.] can be obtained.

Or else, the compound represented by Formula (26) [wherein Y, $R^3$, n, $J^2$ and P have the same meanings as

defined above.] is reacted according to the general methods described in literatures, for example, subjecting to lithiation, and then, reacting with the conventionally known compound represented by Formula (37) [wherein $R^4$ has the same meanings as defined above, $J^5$ represents a halogen atom, a hydroxyl group, a metal salt (e.g., -OLi, -ONa), a $C_1$ to $C_4$ alkoxy group (e.g., methoxy group, ethoxy group), a di($C_1$ to $C_4$ alkyl)amino group (e.g., diethylamino group), a $C_1$ to $C_4$ alkoxy($C_1$ to $C_4$ alkyl)amino group (e.g., O,N-dimethylhydroxyamino group) or a cyclic amino group (e.g., piperidin-1-yl group, morpholin-4-yl group, 4-methylpiperazin-1-yl group).] or reacting with the conventionally known compound represented by Formula (36) according to the methods as described in Journal of the American Chemical Society [J. Am. Chem. Soc.] 1955, vol. 77, p. 3657, Tetrahedron Letters [Tetrahedron Lett.] 1980, vol. 21, p. 2129 and 1991, vol. 32, p. 2003, U.S. Patent Publication (US Patent No. 5,514,816), etc., or forming a Grignard reagent, and then, reacting with the compound represented by Formula (37) or the compound represented by Formula (36) according to the methods as described in Heterocycles, 1987, vol. 25, p. 221, Synthetic Communications [Synth. Commun.] 1985, vol. 15, p. 1291 and 1990, vol. 20, p. 1469, German Patent Publication (DE 19727042 A), etc., so that the compound represented by Formula (18) can be obtained.

The compound represented by Formula (21) can be synthesized in the same manner as in the compound represented by Formula (18).

The compound represented by Formula (26) can be synthesized, for example, as follows.

Reaction formula 16

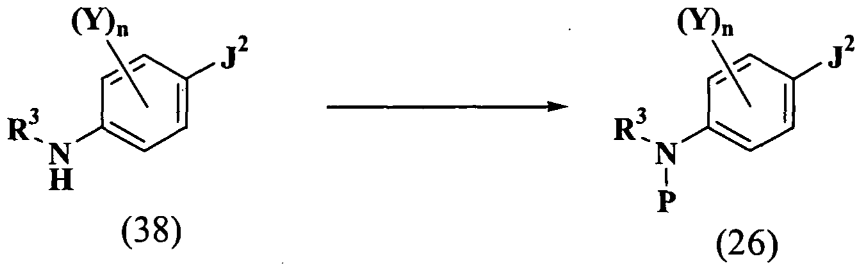

(38) (26)

That is, an amino group of the conventionally known substituted aniline represented by Formula (38) [wherein Y, $R^3$, n and $J^2$ have the same meanings as defined above.] is protected according to the general method described in literatures, for example, the methods as described in Journal of Medicinal Chemistry [J. Med. Chem.] 1996, vol. 39, p. 673 and 1997, vol. 40, p. 3542, etc., so that the compound represented by Formula (26) [wherein Y, $R^3$, n, $J^2$ and P have the same meanings as defined above.] can be obtained.

Some of the compounds represented by Formula (27) are conventionally known compounds, and some of which can be obtained as commercially available products. Also, those other than the above can be synthesized in the same manner as in the compound represented by Formula (18).

In these respective reactions, after completion of the reaction, by carrying out the usual post-treatments, respective preparation intermediates which are starting compounds in Preparation method A to Preparation method I can be obtained.

Also, the respective preparation intermediates prepared by these methods may be used as such in the reaction of the next Step without isolation and purification.

As the compounds included in the present invention, there may be specifically mentioned, for example, those of the compounds shown in Table 2 to Table 5. Provided that the compounds shown in Table 2 to Table 5 are only for the exemplary purpose, and the present invention is not limited only by these.

Incidentally, in the tables, the description Et means an ethyl group, and in the same manner, n-Pr and Pr-n are normal propyl group, i-Pr and Pr-I are isopropyl group, c-Pr and Pr-c are cyclopropyl group, n-Bu and Bu-n are normal butyl group, s-Bu and Bu-s are secondary butyl group, i-Bu and Bu-I are isobutyl group, t-Bu and Bu-t are tertiary butyl group, c-Bu and Bu-c are cyclobutyl group, n-Pen and Pen-n are normal pentyl group, c-Pen and Pen-c are cyclopentyl group, n-Hex and Hex-n are normal hexyl group, c-Hex and Hex-c are cyclohexyl group, Oct are octyl group, Ph is a phenyl group, 1-Naph is 1-naphthyl group, 2-Naph is 2-naphthyl group, respectively,

In the tables, the aromatic heterocycles represented by L-1 a to L-55a each represent the following structures, respectively,

L-1a : 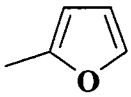 L-1b : 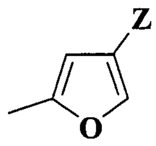 L-1c : 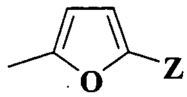

L-2a : 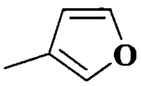 L-2b : 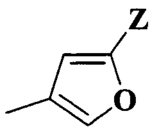 L-3a : 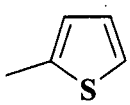

L-3b : 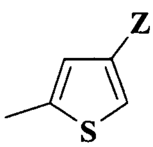 L-3c : 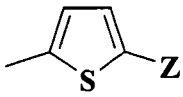 L-3d : 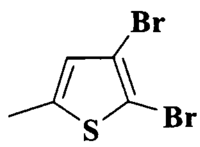

L-4a : 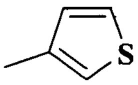 L-4b : 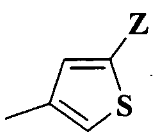 L-5a : 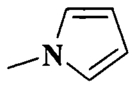

L-5b : 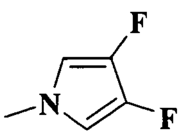 L-6a : 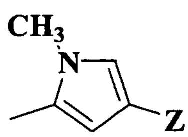 L-6b : 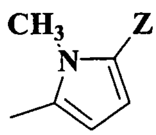

L-8a : 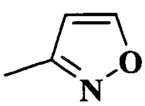 L-10a : 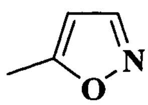 L-10b : 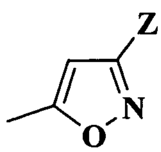

L-11a : 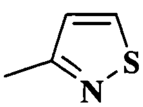 L-14a : 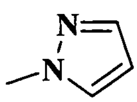 L-14b : 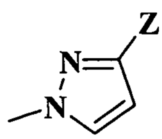

L-14c : 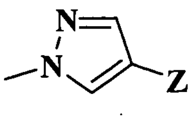 L-15a : 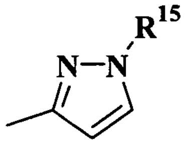 L-15b : 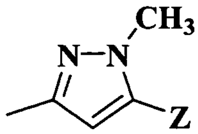

L-16a : 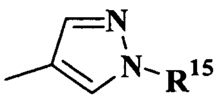 L-17a : 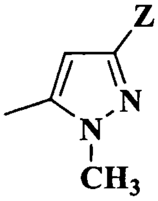 L-19a : 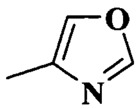

L-20a : 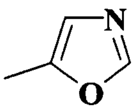 L-21a : 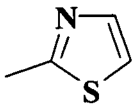 L-21b : 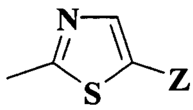

L-22a : 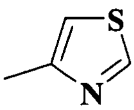 L-22b : 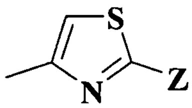 L-23a : 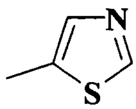

L-23b : 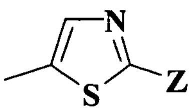 L-23c : 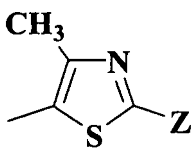 L-24a : 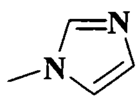

L-25a : 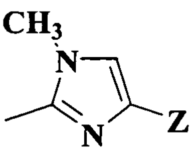 L-25b : 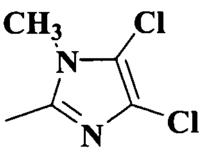 L-30a : 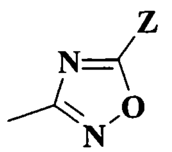

L-31a : 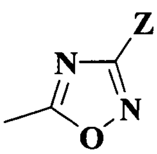 L-34a : 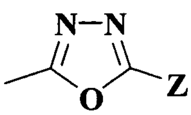 L-35a : 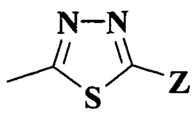

L-36a : 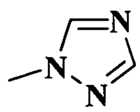

L-38a : 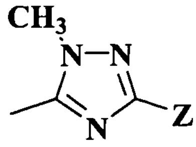

L-45a : 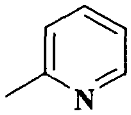

L-45b : 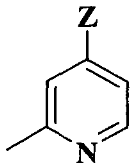

L-45c : 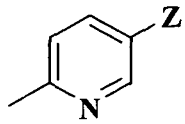

L-45d : 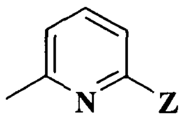

L-45e : 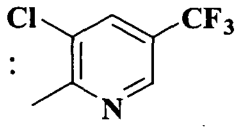

L-45f : 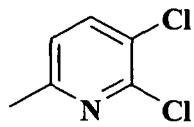

L-46a : 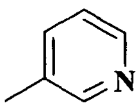

L-46b : 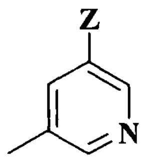

L-46c : 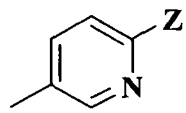

L-46d : 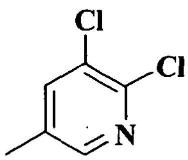

L-47a : 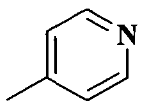

L-47b : 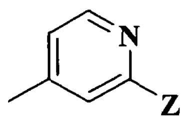

L-47c : 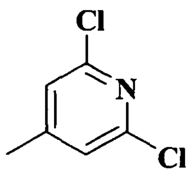

L-47d : 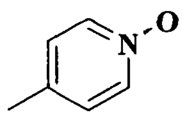

L-48a : 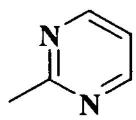

L-48b : 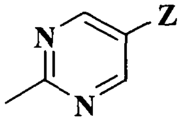

L-50a : 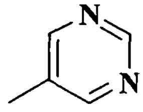

L-50b : 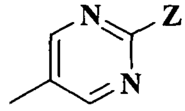

L-51a : 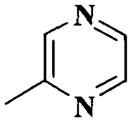

L-51b : 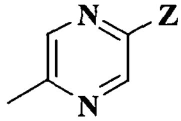

L-53a : 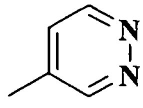

L-55a : 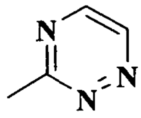

For example, the expression (L-16a)$CHF_2$ repersents a 1-difluoromethylpyrazol-4-yl group, and the expression (L-45c)$CF_3$ repersents a 5-trifluoromethylpyridin-2-yl group.

In the tables, the aliphatic heterocycles represented by M-4a to M-22a each represent the following structures, respectively,

M-4a : 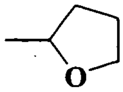 M-5a : 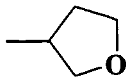 M-7a : 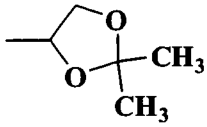

M-8a : 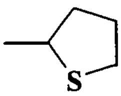 M-9a : 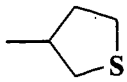 M-9b : 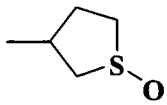

M-9c : 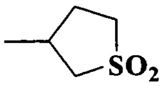 M-16a : 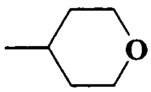 M-19a : 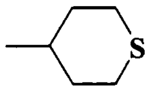

M-22a : 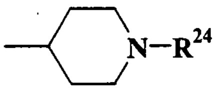

For example, the expression (M-22a)$C(O)OCH_3$ repersents a 1-methoxycarbonylpiperidin-4-yl group.

Moreover, in the tables, T-1 to T-24 each represent the following structures, respectively.

T-1 : 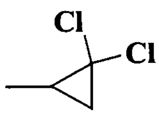 T-2 : 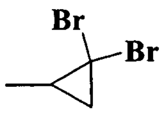 T-3 : 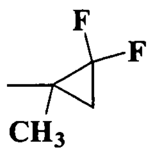

T-4 : 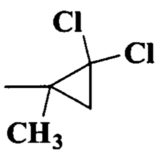 T-5 : 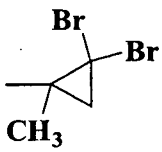 T-6 : 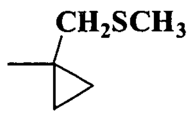

T-7 : 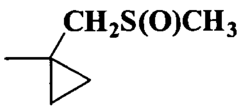 T-8 : 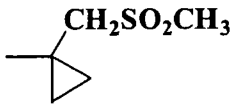 T-9 : 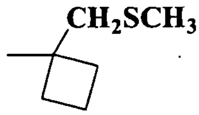

T-10 : 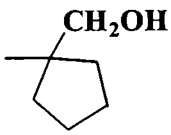 T-11 : 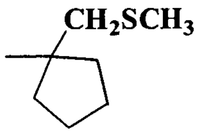 T-12 : 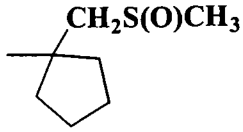

T-13 : 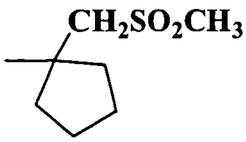 T-14 : 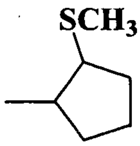 T-15 : 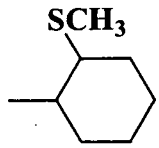

T-16 : 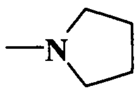 T-17 : 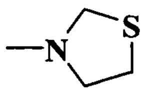 T-18 : 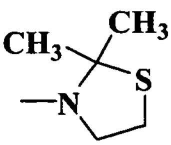

T-19 : 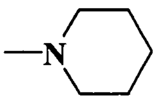 T-20 : 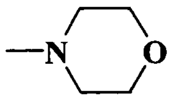 T-21 : 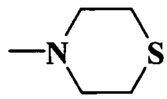

T-22 : 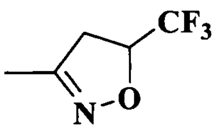 T-23 : 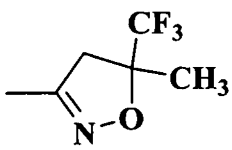 T-24 : 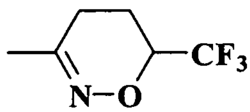

Table 2

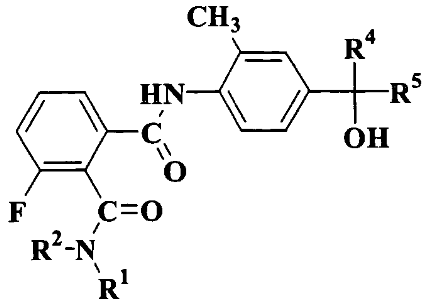

[1] - 1 ,

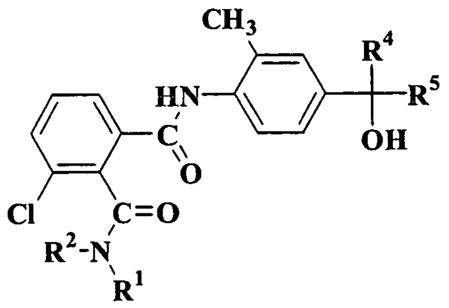

[1] - 2 ,

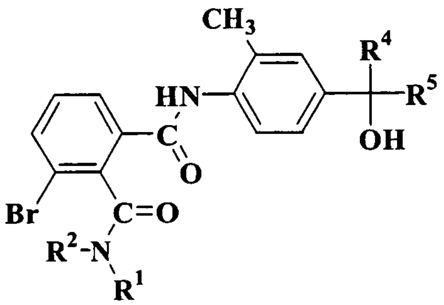

[1] - 3 ,

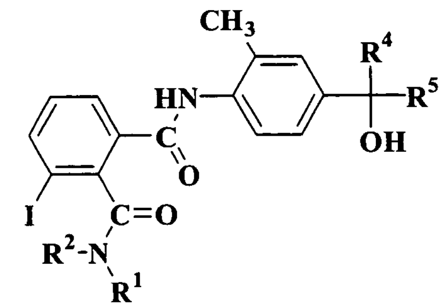

[1] - 4 ,

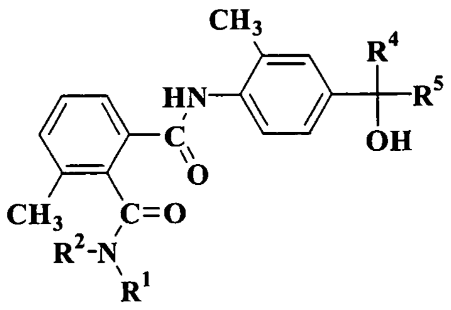

[1] - 5 ,

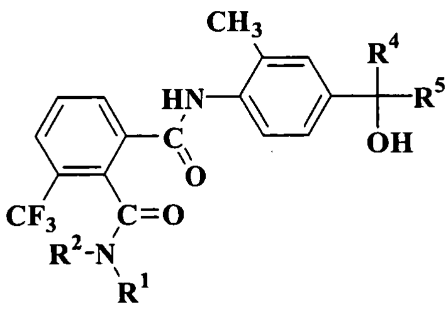

[1] - 6 ,

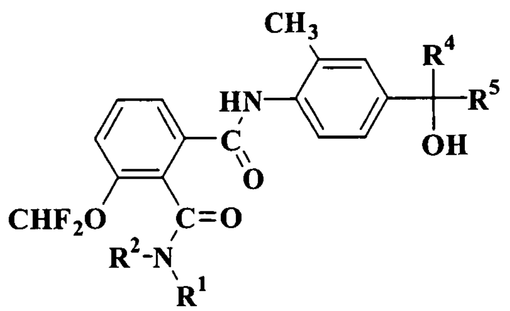

[1] - 7 ,

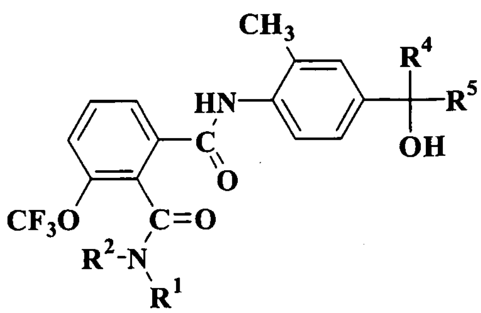

[1] - 8 ,

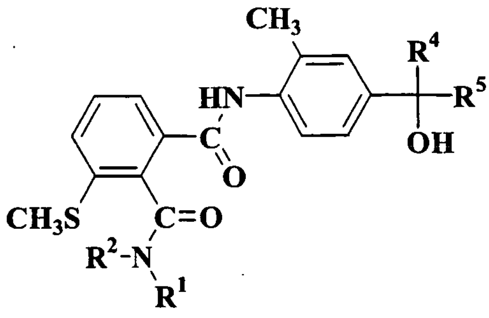

[1] - 9 ,

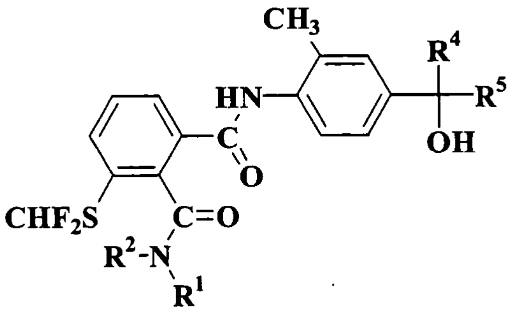

[1] - 10 ,

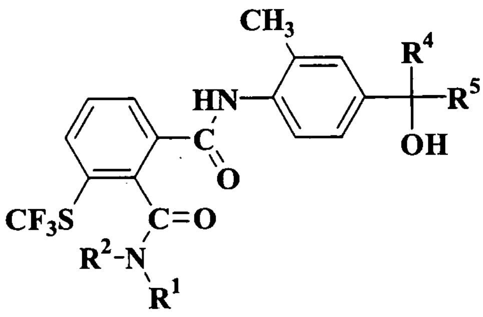

[1] - 11 ,

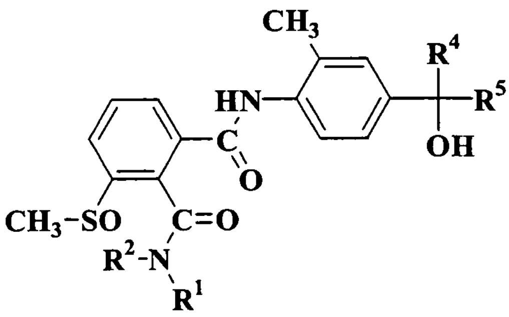

[1] - 12 ,

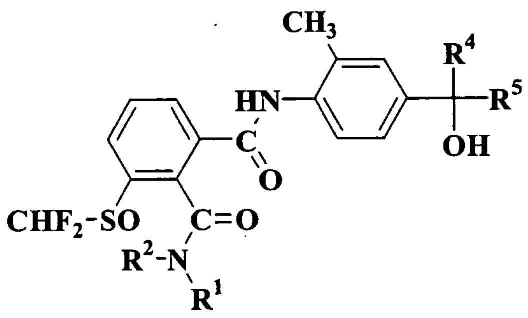

[1] - 13 ,

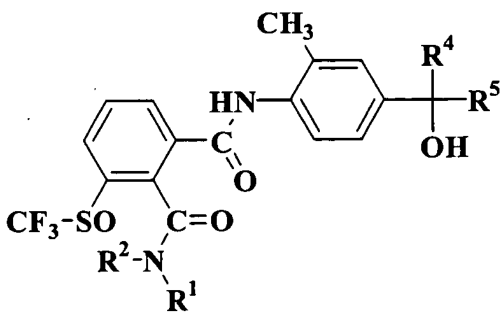

[1] - 14 ,

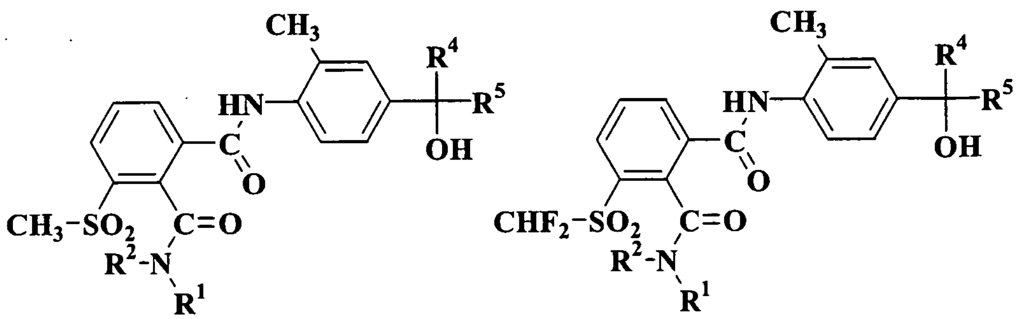

[1] - 15 , [1] - 16 ,

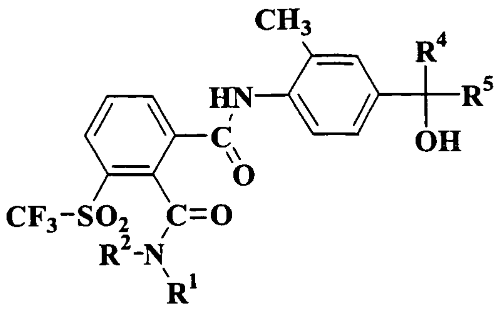

[1] - 17 ,

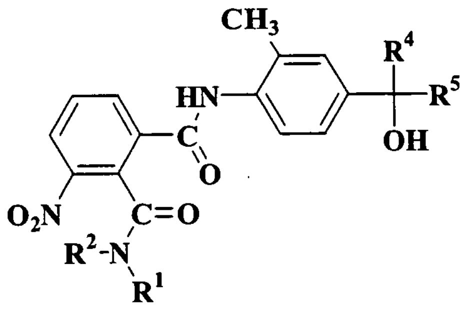

[1] - 18 ,

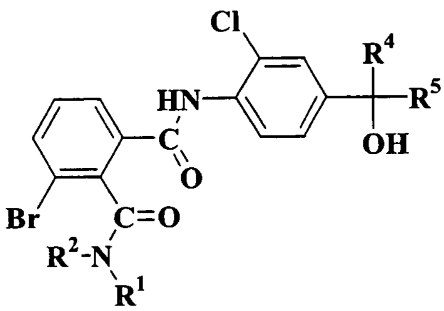

[1] - 19 ,

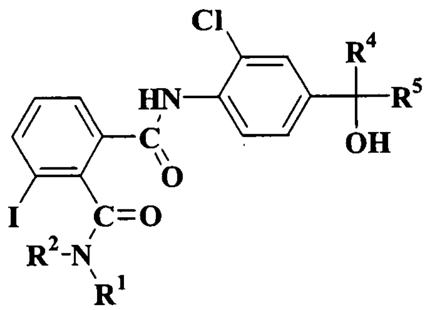

[1] - 20 ,

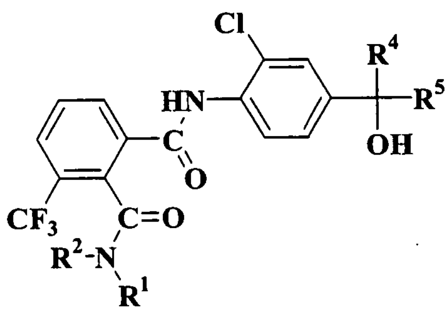

[1] - 21 ,

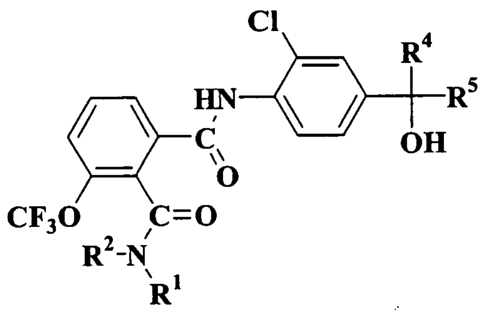

[1] - 22 ,

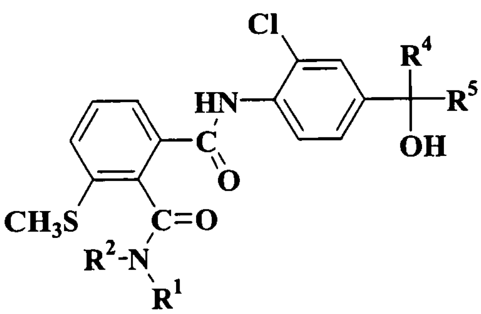

[1] - 23 ,

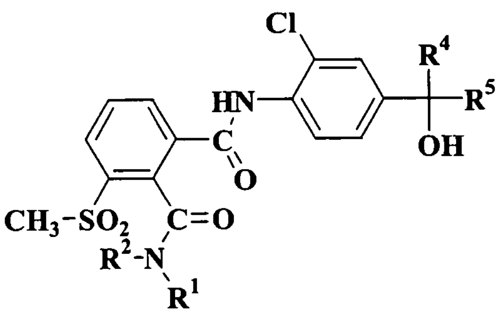

[1] - 24 ,

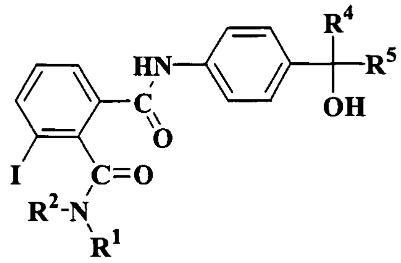

[1] - 25 ,

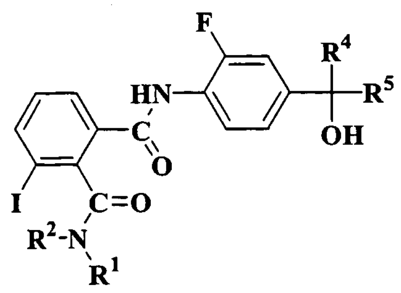

[1] - 26 ,

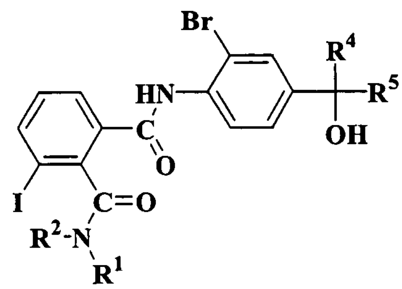

[1] - 27 or

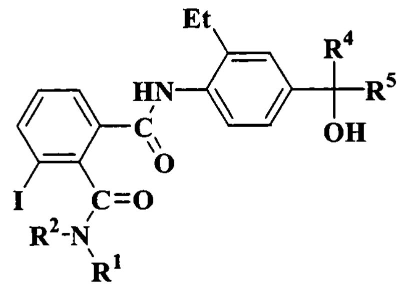

[1] - 28

| $R^2$ | $R^1$ | $R^4$ | $R^5$ |
|---|---|---|---|
| H | $CH_3$ | $CF_3$ | $CF_2O$ (Ph-4-Cl) |
| H | $CH_3$ | $CF_3$ | CH=CH (Ph-4-Cl) |
| H | $CH_3$ | $CF_3$ | CH=CH (Ph-4-$OCF_3$) |
| H | $CH_3$ | $CF_3$ | CH=CH (Ph-3, 4-$Cl_2$) |
| H | $CH_3$ | $CF_3$ | Ph-4-F |
| H | $CH_3$ | $CF_3$ | Ph-4-Cl |
| H | $CH_3$ | $CF_3$ | Ph-4-Br |
| H | $CH_3$ | $CF_3$ | Ph-4-I |
| H | $CH_3$ | $CF_3$ | Ph-4-$CF_3$ |
| H | $CH_3$ | $CF_3$ | Ph-4-$OCHF_2$ |
| H | $CH_3$ | $CF_3$ | Ph-4-$OCF_3$ |
| H | $CH_3$ | $CF_3$ | Ph-4-$OCF_2Br$ |
| H | $CH_3$ | $CF_3$ | Ph-4-$OCF_2CHF_2$ |
| H | $CH_3$ | $CF_3$ | Ph-4-$OCF_2CHFCl$ |
| H | $CH_3$ | $CF_3$ | Ph-4-$OCF_2CHFCF_3$ |
| H | $CH_3$ | $CF_3$ | Ph-4-$OCF_2CHFOCF_3$ |
| H | $CH_3$ | $CF_3$ | Ph-4-$OCF_2CHFOCF_2CF_2CF_3$ |
| H | $CH_3$ | $CF_3$ | Ph-4-O (L-45e) |
| H | $CH_3$ | $CF_3$ | Ph-3, 4-$Cl_2$ |
| H | $CH_3$ | $CF_3$ | Ph (-3-$OCF_2O$-4-) |

| | | | |
|---|---|---|---|
| H | $CH_3$ | $CF_3$ | Ph (-3-$OCF_2CF_2O$-4-) |
| H | $CH_3$ | $CF_3$ | (L-45c) Cl |
| H | $CH_3$ | $CF_3$ | (L-45c) Br |
| H | $CH_3$ | $CF_3$ | (L-45c) $CF_3$ |
| H | $CH_3$ | $CF_3$ | L-45e |
| H | $CH_3$ | $CF_3$ | L-45f |
| H | $CH_3$ | $CF_3$ | (L-46c) Cl |
| H | $CH_3$ | $CF_3$ | (L-46c) Br |
| H | $CH_3$ | $CF_3$ | (L-46c) $CF_3$ |
| H | $CH_3$ | $CF_3$ | L-46d |
| $CH_3$ | $CH_3$ | $CF_3$ | Ph-4-Br |
| $CH_3$ | $CH_3$ | $CF_3$ | Ph-4-$CF_3$ |
| $CH_3$ | $CH_3$ | $CF_3$ | Ph-4-$OCF_3$ |
| $CH_3$ | $CH_3$ | $CF_3$ | (L-45c) $CF_3$ |
| H | Et | $CH_3$ | Ph-4-Cl |
| H | Et | $CH_3$ | Ph-4-$CF_3$ |
| H | Et | $CH_3$ | Ph-4-$OCF_3$ |
| H | Et | $CH_3$ | (L-45c) Cl |
| H | Et | $CH_3$ | (L-45c) Br |
| H | Et | $CH_3$ | (L-45c) $CF_3$ |
| H | Et | Et | Ph-4-Br |
| H | Et | n-Pr | Ph-4-$CF_3$ |
| H | Et | i-Pr | Ph-4-$OCF_3$ |
| H | Et | c-Pr | (L-45c) $CF_3$ |
| H | Et | $CHF_2$ | Ph-4-Br |
| H | Et | $CF_3$ | $CF_2O$ (Ph-4-Cl) |
| H | Et | $CF_3$ | CH=CH (Ph-4-Cl) |
| H | Et | $CF_3$ | CH=CH (Ph-4-$OCF_3$) |
| H | Et | $CF_3$ | CH=CH (Ph-3, 4-$Cl_2$) |
| H | Et | $CF_3$ | Ph-4-F |
| H | Et | $CF_3$ | Ph-4-Cl |
| H | Et | $CF_3$ | Ph-4-Br |
| H | Et | $CF_3$ | Ph-4-I |
| H | Et | $CF_3$ | Ph-4-$CF_3$ |
| H | Et | $CF_3$ | Ph-4-$OCHF_2$ |
| H | Et | $CF_3$ | Ph-4-$OCF_3$ |
| H | Et | $CF_3$ | Ph-4-$OCF_2Br$ |
| H | Et | $CF_3$ | Ph-4-$OCF_2CHF_2$ |
| H | Et | $CF_3$ | Ph-4-$OCF_2CHFCl$ |
| H | Et | $CF_3$ | Ph-4-$OCF_2CHFCF_3$ |
| H | Et | $CF_3$ | Ph-4-$OCF_2CHFOCF_3$ |

| | | | |
|---|---|---|---|
| H | Et | $CF_3$ | Ph-4-$OCF_2CHFOCF_2CF_2CF_3$ |
| H | Et | $CF_3$ | Ph-4-O (L-45g) |
| H | Et | $CF_3$ | Ph-3, 4-$Cl_2$ |
| H | Et | $CF_3$ | Ph (-3-$OCF_2O$-4-) |
| H | Et | $CF_3$ | Ph (-3-$OCF_2CF_2O$-4-) |
| H | Et | $CF_3$ | (L-45c) Cl |
| H | Et | $CF_3$ | (L-45c) Br |
| H | Et | $CF_3$ | (L-45c) $CF_3$ |
| H | Et | $CF_3$ | L-45e |
| H | Et | $CF_3$ | L-45f |
| H | Et | $CF_3$ | (L-46c) Cl |
| H | Et | $CF_3$ | (L-46c) Br |
| H | Et | $CF_3$ | (L-46c) $CF_3$ |
| H | Et | $CF_3$ | L-46d |
| H | Et | $CF_2Cl$ | Ph-4-$CF_3$ |
| H | Et | $CF_2Br$ | Ph-4-$OCF_3$ |
| H | Et | $CF_2CF_3$ | (L-45c) $CF_3$ |
| H | Et | $CF_2CF_2CF_3$ | Ph-4-Br |
| H | Et | $CF_2OCH_3$ | Ph-4-$CF_3$ |
| H | Et | $CF_2SCH_3$ | Ph-4-$OCF_3$ |
| Et | Et | $CH_3$ | Ph-4-Br |
| Et | Et | $CH_3$ | Ph-4-$CF_3$ |
| Et | Et | $CH_3$ | Ph-4-$OCF_3$ |
| Et | Et | $CH_3$ | (L-45c) Cl |
| Et | Et | $CH_3$ | (L-45c) Br |
| Et | Et | $CH_3$ | (L-45c) $CF_3$ |
| Et | Et | $CF_3$ | $CF_2O$ (Ph-4-Cl) |
| Et | Et | $CF_3$ | CH=CH (Ph-4-Cl) |
| Et | Et | $CF_3$ | CH=CH (Ph-4-$OCF_3$) |
| Et | Et | $CF_3$ | CH=CH (Ph-3, 4-$Cl_2$) |
| Et | Et | $CF_3$ | Ph-4-F |
| Et | Et | $CF_3$ | Ph-4-Cl |
| Et | Et | $CF_3$ | Ph-4-Br |
| Et | Et | $CF_3$ | Ph-4-I |
| Et | Et | $CF_3$ | Ph-4-$CF_3$ |
| Et | Et | $CF_3$ | Ph-4-$OCHF_2$ |
| Et | Et | $CF_3$ | Ph-4-$OCF_3$ |
| Et | Et | $CF_3$ | Ph-4-$OCF_2Br$ |
| Et | Et | $CF_3$ | Ph-4-$OCF_2CHF_2$ |
| Et | Et | $CF_3$ | Ph-4-$OCF_2CHFCl$ |
| Et | Et | $CF_3$ | Ph-4-$OCF_2CHFCF_3$ |

| | | | |
|---|---|---|---|
| Et | Et | $CF_3$ | Ph-4-$OCF_2CHFOCF_3$ |
| Et | Et | $CF_3$ | Ph-4-$OCF_2CHFOCF_2CF_2CF_3$ |
| Et | Et | $CF_3$ | Ph-4-O (L-45g) |
| Et | Et | $CF_3$ | Ph-3, 4-$Cl_2$ |
| Et | Et | $CF_3$ | Ph (-3-$OCF_2O$-4-) |
| Et | Et | $CF_3$ | Ph (-3-$OCF_2CF_2O$-4-) |
| Et | Et | $CF_3$ | (L-45c) Cl |
| Et | Et | $CF_3$ | (L-45c) Br |
| Et | Et | $CF_3$ | (L-45c) $CF_3$ |
| Et | Et | $CF_3$ | L-45e |
| Et | Et | $CF_3$ | L-45f |
| Et | Et | $CF_3$ | (L-46c) Cl |
| Et | Et | $CF_3$ | (L-46c) Br |
| Et | Et | $CF_3$ | (L-46c) $CF_3$ |
| Et | Et | $CF_3$ | L-46d |
| H | n-Pr | $CH_3$ | Ph-4-Br |
| H | n-Pr | $CH_3$ | Ph-4-$CF_3$ |
| H | n-Pr | $CH_3$ | Ph-4-$OCF_3$ |
| H | n-Pr | $CH_3$ | (L-45c) Cl |
| H | n-Pr | $CH_3$ | (L-45c) Br |
| H | n-Pr | $CH_3$ | (L-45c) $CF_3$ |
| H | n-Pr | Et | (L-45c) $CF_3$ |
| H | n-Pr | n-Pr | Ph-4-Br |
| H | n-Pr | i-Pr | Ph-4-$CF_3$ |
| H | n-Pr | c-Pr | Ph-4-$OCF_3$ |
| H | n-Pr | $CHF_2$ | (L-45c) $CF_3$ |
| H | n-Pr | $CF_3$ | $CF_2O$ (Ph-4-Cl) |
| H | n-Pr | $CF_3$ | CH=CH (Ph-4-Cl) |
| H | n-Pr | $CF_3$ | CH=CH (Ph-4-$OCF_3$) |
| H | n-Pr | $CF_3$ | CH=CH (Ph-3, 4-$Cl_2$) |
| H | n-Pr | $CF_3$ | Ph-4-F |
| H | n-Pr | $CF_3$ | Ph-4-Cl |
| H | n-Pr | $CF_3$ | Ph-4-Br |
| H | n-Pr | $CF_3$ | Ph-4-I |
| H | n-Pr | $CF_3$ | Ph-4-$CF_3$ |
| H | n-Pr | $CF_3$ | Ph-4-$OCHF_2$ |
| H | n-Pr | $CF_3$ | Ph-4-$OCF_3$ |
| H | n-Pr | $CF_3$ | Ph-4-$OCF_2Br$ |
| H | n-Pr | $CF_3$ | Ph-4-$OCF_2CHF_2$ |
| H | n-Pr | $CF_3$ | Ph-4-$OCF_2CHFCl$ |
| H | n-Pr | $CF_3$ | Ph-4-$OCF_2CHFCF_3$ |

| | | | |
|---|---|---|---|
| H | n-Pr | $CF_3$ | Ph-4-$OCF_2CHFOCF_3$ |
| H | n-Pr | $CF_3$ | Ph-4-$OCF_2CHFOCF_2CF_2CF_3$ |
| H | n-Pr | $CF_3$ | Ph-4-O (L-45g) |
| H | n-Pr | $CF_3$ | Ph-3, 4-$Cl_2$ |
| H | n-Pr | $CF_3$ | Ph (-3-$OCF_2O$-4-) |
| H | n-Pr | $CF_3$ | Ph (-3-$OCF_2CF_2O$-4-) |
| H | n-Pr | $CF_3$ | (L-45c) Cl |
| H | n-Pr | $CF_3$ | (L-45c) Br |
| H | n-Pr | $CF_3$ | (L-45c) $CF_3$ |
| H | n-Pr | $CF_3$ | L-45e |
| H | n-Pr | $CF_3$ | L-45f |
| H | n-Pr | $CF_3$ | (L-46c) Cl |
| H | n-Pr | $CF_3$ | (L-46c) Br |
| H | n-Pr | $CF_3$ | (L-46c) $CF_3$ |
| H | n-Pr | $CF_3$ | L-46d |
| H | n-Pr | $CF_2Cl$ | Ph-4-Br |
| H | n-Pr | $CF_2Br$ | Ph-4-$CF_3$ |
| H | n-Pr | $CF_2CF_3$ | Ph-4-$OCF_3$ |
| H | n-Pr | $CF_2CF_2CF_3$ | (L-45c) $CF_3$ |
| H | n-Pr | $CF_2OCH_3$ | Ph-4-Br |
| H | n-Pr | $CF_2SCH_3$ | Ph-4-$CF_3$ |
| $CH_3$ | n-Pr | $CF_3$ | Ph-4-Br |
| $CH_3$ | n-Pr | $CF_3$ | Ph-4-$CF_3$ |
| $CH_3$ | n-Pr | $CF_3$ | Ph-4-$OCF_3$ |
| $CH_3$ | n-Pr | $CF_3$ | (L-45c) $CF_3$ |
| Et | n-Pr | $CF_3$ | Ph-4-$OCF_3$ |
| n-Pr | n-Pr | $CF_3$ | (L-45c) $CF_3$ |
| H | i-Pr | $CH_3$ | $CF_2O$ (Ph-4-Cl) |
| H | i-Pr | $CH_3$ | CH=CH (Ph-4-Cl) |
| H | i-Pr | $CH_3$ | CH=CH (Ph-4-$OCF_3$) |
| H | i-Pr | $CH_3$ | CH=CH (Ph-3, 4-$Cl_2$) |
| H | i-Pr | $CH_3$ | Ph-4-F |
| H | i-Pr | $CH_3$ | Ph-4-Cl |
| H | i-Pr | $CH_3$ | Ph-4-Br |
| H | i-Pr | $CH_3$ | Ph-4-I |
| H | i-Pr | $CH_3$ | Ph-4-$CF_3$ |
| H | i-Pr | $CH_3$ | Ph-4-$OCHF_2$ |
| H | i-Pr | $CH_3$ | Ph-4-$OCF_3$ |
| H | i-Pr | $CH_3$ | Ph-4-$OCF_2Br$ |
| H | i-Pr | $CH_3$ | Ph-4-$OCF_2CHF_2$ |
| H | i-Pr | $CH_3$ | Ph-4-$OCF_2CHFCl$ |

| | | | |
|---|---|---|---|
| H | i-Pr | $CH_3$ | Ph-4-$OCF_2CHFCF_3$ |
| H | i-Pr | $CH_3$ | Ph-4-$OCF_2CHFOCF_3$ |
| H | i-Pr | $CH_3$ | Ph-4-$OCF_2CHFOCF_2CF_2CF_3$ |
| H | i-Pr | $CH_3$ | Ph-4-O (L-45g) |
| H | i-Pr | $CH_3$ | Ph-3, 4-$Cl_2$ |
| H | i-Pr | $CH_3$ | Ph (-3-$OCF_2O$-4-) |
| H | i-Pr | $CH_3$ | Ph (-3-$OCF_2CF_2O$-4-) |
| H | i-Pr | $CH_3$ | (L-45c) Cl |
| H | i-Pr | $CH_3$ | (L-45c) Br |
| H | i-Pr | $CH_3$ | (L-45c) $CF_3$ |
| H | i-Pr | $CH_3$ | L-45e |
| H | i-Pr | $CH_3$ | L-45f |
| H | i-Pr | $CH_3$ | (L-46c) Cl |
| H | i-Pr | $CH_3$ | (L-46c) Br |
| H | i-Pr | $CH_3$ | (L-46c) $CF_3$ |
| H | i-Pr | $CH_3$ | L-46d |
| H | i-Pr | Et | $CF_2O$ (Ph-4-Cl) |
| H | i-Pr | Et | CH=CH (Ph-4-Cl) |
| H | i-Pr | Et | CH=CH (Ph-4-$OCF_3$) |
| H | i-Pr | Et | CH=CH (Ph-3, 4-$Cl_2$) |
| H | i-Pr | Et | Ph-4-Cl |
| H | i-Pr | Et | Ph-4-Br |
| H | i-Pr | Et | Ph-4-$CF_3$ |
| H | i-Pr | Et | Ph-4-$OCF_3$ |
| H | i-Pr | Et | Ph-4-$OCF_2Br$ |
| H | i-Pr | Et | Ph-4-$OCF_2CHFCF_3$ |
| H | i-Pr | Et | Ph-4-$OCF_2CHFOCF_3$ |
| H | i-Pr | Et | Ph-4-$OCF_2CHFOCF_2CF_2CF_3$ |
| H | i-Pr | Et | Ph-4-O (L-45g) |
| H | i-Pr | Et | Ph-3, 4-$Cl_2$ |
| H | i-Pr | Et | Ph (-3-$OCF_2O$-4-) |
| H | i-Pr | Et | Ph (-3-$OCF_2CF_2O$-4-) |
| H | i-Pr | Et | (L-45c) Br |
| H | i-Pr | Et | (L-45c) $CF_3$ |
| H | i-Pr | Et | L-45e |
| H | i-Pr | Et | L-45f |
| H | i-Pr | Et | (L-46c) Br |
| H | i-Pr | Et | (L-46c) $CF_3$ |
| H | i-Pr | Et | L-46d |
| H | i-Pr | n-Pr | $CF_2O$ (Ph-4-Cl) |
| H | i-Pr | n-Pr | CH=CH (Ph-4-Cl) |

| | | | |
|---|---|---|---|
| H | i-Pr | n-Pr | CH=CH (Ph-4-$OCF_3$) |
| H | i-Pr | n-Pr | CH=CH (Ph-3, 4-$Cl_2$) |
| H | i-Pr | n-Pr | Ph-4-Cl |
| H | i-Pr | n-Pr | Ph-4-Br |
| H | i-Pr | n-Pr | Ph-4-$CF_3$ |
| H | i-Pr | n-Pr | Ph-4-$OCF_3$ |
| H | i-Pr | n-Pr | Ph-4-$OCF_2Br$ |
| H | i-Pr | n-Pr | Ph-4-$OCF_2CHFCF_3$ |
| H | i-Pr | n-Pr | Ph-4-$OCF_2CHFOCF_3$ |
| H | i-Pr | n-Pr | Ph-4-$OCF_2CHFOCF_2CF_2CF_3$ |
| H | i-Pr | n-Pr | Ph-4-O (L-45g) |
| H | i-Pr | n-Pr | Ph-3, 4-$Cl_2$ |
| H | i-Pr | n-Pr | Ph (-3-$OCF_2O$-4-) |
| H | i-Pr | n-Pr | Ph (-3-$OCF_2CF_2O$-4-) |
| H | i-Pr | n-Pr | (L-45c) Br |
| H | i-Pr | n-Pr | (L-45c) $CF_3$ |
| H | i-Pr | n-Pr | L-45e |
| H | i-Pr | n-Pr | L-45f |
| H | i-Pr | n-Pr | (L-46c) Br |
| H | i-Pr | n-Pr | (L-46c) $CF_3$ |
| H | i-Pr | n-Pr | L-46d |
| H | i-Pr | i-Pr | $CF_2O$ (Ph-4-Cl) |
| H | i-Pr | i-Pr | CH=CH (Ph-4-Cl) |
| H | i-Pr | i-Pr | CH=CH (Ph-4-$OCF_3$) |
| H | i-Pr | i-Pr | CH=CH (Ph-3, 4-$Cl_2$) |
| H | i-Pr | i-Pr | Ph-4-Cl |
| H | i-Pr | i-Pr | Ph-4-Br |
| H | i-Pr | i-Pr | Ph-4-$CF_3$ |
| H | i-Pr | i-Pr | Ph-4-$OCF_3$ |
| H | i-Pr | i-Pr | Ph-4-$OCF_2Br$ |
| H | i-Pr | i-Pr | Ph-4-$OCF_2CHFCF_3$ |
| H | i-Pr | i-Pr | Ph-4-$OCF_2CHFOCF_3$ |
| H | i-Pr | i-Pr | Ph-4-$OCF_2CHFOCF_2CF_2CF_3$ |
| H | i-Pr | i-Pr | Ph-4-O (L-45g) |
| H | i-Pr | i-Pr | Ph-3, 4-$Cl_2$ |
| H | i-Pr | i-Pr | Ph (-3-$OCF_2O$-4-) |
| H | i-Pr | i-Pr | Ph (-3-$OCF_2CF_2O$-4-) |
| H | i-Pr | i-Pr | (L-45c) Br |
| H | i-Pr | i-Pr | (L-45c) $CF_3$ |
| H | i-Pr | i-Pr | L-45e |
| H | i-Pr | i-Pr | L-45f |

| | | | |
|---|---|---|---|
| H | i-Pr | i-Pr | (L-46c) Br |
| H | i-Pr | i-Pr | (L-46c) $CF_3$ |
| H | i-Pr | i-Pr | L-46d |
| H | i-Pr | c-Pr | $CF_2O$ (Ph-4-Cl) |
| H | i-Pr | c-Pr | CH=CH (Ph-4-Cl) |
| H | i-Pr | c-Pr | CH=CH (Ph-4-$OCF_3$) |
| H | i-Pr | c-Pr | CH=CH (Ph-3, 4-$Cl_2$) |
| H | i-Pr | c-Pr | Ph-4-Cl |
| H | i-Pr | c-Pr | Ph-4-Br |
| H | i-Pr | c-Pr | Ph-4-$CF_3$ |
| H | i-Pr | c-Pr | Ph-4-$OCF_3$ |
| H | i-Pr | c-Pr | Ph-4-$OCF_2Br$ |
| H | i-Pr | c-Pr | Ph-4-$OCF_2CHFCF_3$ |
| H | i-Pr | c-Pr | Ph-4-$OCF_2CHFOCF_3$ |
| H | i-Pr | c-Pr | Ph-4-$OCF_2CHFOCF_2CF_2CF_3$ |
| H | i-Pr | c-Pr | Ph-4-O (L-45g) |
| H | i-Pr | c-Pr | Ph-3, 4-$Cl_2$ |
| H | i-Pr | c-Pr | Ph (-3-$OCF_2O$-4-) |
| H | i-Pr | c-Pr | Ph (-3-$OCF_2CF_2O$-4-) |
| H | i-Pr | c-Pr | (L-45c) Br |
| H | i-Pr | c-Pr | (L-45c) $CF_3$ |
| H | i-Pr | c-Pr | L-45e |
| H | i-Pr | c-Pr | L-45f |
| H | i-Pr | c-Pr | (L-46c) Br |
| H | i-Pr | c-Pr | (L-46c) $CF_3$ |
| H | i-Pr | c-Pr | L-46d |
| H | i-Pr | n-Bu | Ph-4-Br |
| H | i-Pr | s-Bu | Ph-4-$CF_3$ |
| H | i-Pr | i-Bu | Ph-4-$OCF_3$ |
| H | i-Pr | c-Bu | (L-45c) $CF_3$ |
| H | i-Pr | n-Pen | Ph-4-Br |
| H | i-Pr | c-Pen | Ph-4-$CF_3$ |
| H | i-Pr | n-Hex | Ph-4-$OCF_3$ |
| H | i-Pr | c-Hex | (L-45c) $CF_3$ |
| H | i-Pr | $CH_2F$ | Ph-4-Br |
| H | i-Pr | $CH_2Cl$ | Ph-4-$CF_3$ |
| H | i-Pr | $CH_2Br$ | Ph-4-$OCF_3$ |
| H | i-Pr | $CHF_2$ | $CF_2O$ (Ph-4-Cl) |
| H | i-Pr | $CHF_2$ | CH=CH (Ph-4-Cl) |
| H | i-Pr | $CHF_2$ | CH=CH (Ph-4-$OCF_3$) |
| H | i-Pr | $CHF_2$ | CH=CH (Ph-3, 4-$Cl_2$) |

| | | | |
|---|---|---|---|
| H | i-Pr | $CHF_2$ | Ph-4-F |
| H | i-Pr | $CHF_2$ | Ph-4-Cl |
| H | i-Pr | $CHF_2$ | Ph-4-Br |
| H | i-Pr | $CHF_2$ | Ph-4-I |
| H | i-Pr | $CHF_2$ | Ph-4-$CF_3$ |
| H | i-Pr | $CHF_2$ | Ph-4-$OCHF_2$ |
| H | i-Pr | $CHF_2$ | Ph-4-$OCF_3$ |
| H | i-Pr | $CHF_2$ | Ph-4-$OCF_2Br$ |
| H | i-Pr | $CHF_2$ | Ph-4-$OCF_2CHF_2$ |
| H | i-Pr | $CHF_2$ | Ph-4-$OCF_2CHFCl$ |
| H | i-Pr | $CHF_2$ | Ph-4-$OCF_2CHFCF_3$ |
| H | i-Pr | $CHF_2$ | Ph-4-$OCF_2CHFOCF_3$ |
| H | i-Pr | $CHF_2$ | Ph-4-$OCF_2CHFOCF_2CF_2CF_3$ |
| H | i-Pr | $CHF_2$ | Ph-4-O (L-45g) |
| H | i-Pr | $CHF_2$ | Ph-3, 4-$Cl_2$ |
| H | i-Pr | $CHF_2$ | Ph (-3-$OCF_2O$-4-) |
| H | i-Pr | $CHF_2$ | Ph (-3-$OCF_2CF_2O$-4-) |
| H | i-Pr | $CHF_2$ | (L-45c) Cl |
| H | i-Pr | $CHF_2$ | (L-45c) Br |
| H | i-Pr | $CHF_2$ | (L-45c) $CF_3$ |
| H | i-Pr | $CHF_2$ | L-45e |
| H | i-Pr | $CHF_2$ | L-45f |
| H | i-Pr | $CHF_2$ | (L-46c) Cl |
| H | i-Pr | $CHF_2$ | (L-46c) Br |
| H | i-Pr | $CHF_2$ | (L-46c) $CF_3$ |
| H | i-Pr | $CHF_2$ | L-46d |
| H | i-Pr | CHFCl | (L-45c) $CF_3$ |
| H | i-Pr | CHFBr | Ph-4-Br |
| H | i-Pr | $CF_3$ | T-1 |
| H | i-Pr | $CF_3$ | T-2 |
| H | i-Pr | $CF_3$ | T-3 |
| H | i-Pr | $CF_3$ | T-4 |
| H | i-Pr | $CF_3$ | T-5 |
| H | i-Pr | $CF_3$ | $CH_2OCH_3$ |
| H | i-Pr | $CF_3$ | $CH_2OEt$ |
| H | i-Pr | $CF_3$ | $CH_2OPr$-n |
| H | i-Pr | $CF_3$ | $CH_2OPr$-i |
| H | i-Pr | $CF_3$ | $CH_2OBu$-n |
| H | i-Pr | $CF_3$ | $CH_2OCH_2CF_3$ |
| H | i-Pr | $CF_3$ | $CH_2OCH_2CF_2CF_3$ |
| H | i-Pr | $CF_3$ | $CH_2OCH(CF_3)_2$ |

| | | | |
|---|---|---|---|
| H | i-Pr | $CF_3$ | $CH_2OC(O)(Ph$-2-Cl) |
| H | i-Pr | $CF_3$ | $CH_2OC(O)(Ph$-3-Cl) |
| H | i-Pr | $CF_3$ | $CH_2OC(O)(Ph$-4-Cl) |
| H | i-Pr | $CF_3$ | $CH_2OPh$ |
| H | i-Pr | $CF_3$ | $CH_2O$(Ph-4-F) |
| H | i-Pr | $CF_3$ | $CH_2O$(Ph-2-Cl) |
| H | i-Pr | $CF_3$ | $CH_2O$(Ph-3-Cl) |
| H | i-Pr | $CF_3$ | $CH_2O$(Ph-4-Cl) |
| H | i-Pr | $CF_3$ | $CH_2O$(Ph-4-Br) |
| H | i-Pr | $CF_3$ | $CH_2O$(Ph-4-$CF_3$) |
| H | i-Pr | $CF_3$ | $CH_2O$(Ph-4-$OCF_3$) |
| H | i-Pr | $CF_3$ | $CH_2CH_2OCH_3$ |
| H | i-Pr | $CF_3$ | $CF_2OPh$ |
| H | i-Pr | $CF_3$ | $CF_2O$(Ph-3-F) |
| H | i-Pr | $CF_3$ | $CF_2O$(Ph-4-F) |
| H | i-Pr | $CF_3$ | $CF_2O$(Ph-2-Cl) |
| H | i-Pr | $CF_3$ | $CF_2O$(Ph-3-Cl) |
| H | i-Pr | $CF_3$ | $CF_2O$(Ph-4-Cl) |
| H | i-Pr | $CF_3$ | $CF_2O$(Ph-3-Br) |
| H | i-Pr | $CF_3$ | $CF_2O$(Ph-4-Br) |
| H | i-Pr | $CF_3$ | $CF_2O$(Ph-3-$CF_3$) |
| H | i-Pr | $CF_3$ | $CF_2O$(Ph-4-$CF_3$) |
| H | i-Pr | $CF_3$ | $CF_2O$(Ph-3-$OCF_3$) |
| H | i-Pr | $CF_3$ | $CF_2O$(Ph-4-$OCF_3$) |
| H | i-Pr | $CF_3$ | M-4a |
| H | i-Pr | $CF_3$ | M-5a |
| H | i-Pr | $CF_3$ | $CH_2SCH_3$ |
| H | i-Pr | $CF_3$ | $CH_2SO_2CH_3$ |
| H | i-Pr | $CF_3$ | $CH_2SCF_3$ |
| H | i-Pr | $CF_3$ | $CH_2SO_2CF_3$ |
| H | i-Pr | $CF_3$ | $CH_2SPh$ |
| H | i-Pr | $CF_3$ | $CH_2S$(Ph-3-Cl) |
| H | i-Pr | $CF_3$ | $CH_2S$(Ph-4-Cl) |
| H | i-Pr | $CF_3$ | $CH_2SO_2$(Ph-3-Cl) |
| H | i-Pr | $CF_3$ | $CH_2SO_2$(Ph-4-Cl) |
| H | i-Pr | $CF_3$ | $CH_2CH_2SCH_3$ |
| H | i-Pr | $CF_3$ | $CH_2CH_2SO_2CH_3$ |
| H | i-Pr | $CF_3$ | $CH_2CH(CH_3)SCH_3$ |
| H | i-Pr | $CF_3$ | $CH_2CH(CH_3)SEt$ |
| H | i-Pr | $CF_3$ | $CH_2CH_2SCF_3$ |
| H | i-Pr | $CF_3$ | $CH_2N(CH_3)_2$ |

| | | | |
|---|---|---|---|
| H | i-Pr | $CF_3$ | $CH_2NHPh$ |
| H | i-Pr | $CF_3$ | $CH_2NH$ (Ph-3-Cl) |
| H | i-Pr | $CF_3$ | $CH_2NH$ (Ph-4-Cl) |
| H | i-Pr | $CF_3$ | $CF_2C$ (O) OEt |
| H | i-Pr | $CF_3$ | $CH_2CH_2Ph$ |
| H | i-Pr | $CF_3$ | $CH_2CH_2$ (Ph-3-Cl) |
| H | i-Pr | $CF_3$ | $CH_2CH_2$ (Ph-4-Cl) |
| H | i-Pr | $CF_3$ | CH ($CH_3$) $CH_2Ph$ |
| H | i-Pr | $CF_3$ | $CH_2$ (L-5a) |
| H | i-Pr | $CF_3$ | $CH_2$ (L-14a) |
| H | i-Pr | $CF_3$ | $CH_2$ (L-24a) |
| H | i-Pr | $CF_3$ | $CH_2$ (L-36a) |
| H | i-Pr | $CF_3$ | T-22 |
| H | i-Pr | $CF_3$ | T-23 |
| H | i-Pr | $CF_3$ | T-24 |
| H | i-Pr | $CF_3$ | C (O) $OCH_3$ |
| H | i-Pr | $CF_3$ | C (O) OEt |
| H | i-Pr | $CF_3$ | C (O) OPr-n |
| H | i-Pr | $CF_3$ | C (O) OPr-i |
| H | i-Pr | $CF_3$ | C (O) OBu-n |
| H | i-Pr | $CF_3$ | C (O) OBu-t |
| H | i-Pr | $CF_3$ | C (O) $OCH_2CF_3$ |
| H | i-Pr | $CF_3$ | C (O) SEt |
| H | i-Pr | $CF_3$ | C (O) N $(CH_3)_2$ |
| H | i-Pr | $CF_3$ | CH=CHPh |
| H | i-Pr | $CF_3$ | CH=CH (Ph-3-F) |
| H | i-Pr | $CF_3$ | CH=CH (Ph-4-F) |
| H | i-Pr | $CF_3$ | CH=CH (Ph-2-Cl) |
| H | i-Pr | $CF_3$ | CH=CH (Ph-3-Cl) |
| H | i-Pr | $CF_3$ | CH=CH (Ph-4-Cl) |
| H | i-Pr | $CF_3$ | CH=CH (Ph-3-Br) |
| H | i-Pr | $CF_3$ | CH=CH (Ph-4-Br) |
| H | i-Pr | $CF_3$ | CH=CH (Ph-3-$CF_3$) |
| H | i-Pr | $CF_3$ | CH=CH (Ph-4-$CF_3$) |
| H | i-Pr | $CF_3$ | CH=CH (Ph-3-$OCF_3$) |
| H | i-Pr | $CF_3$ | CH=CH (Ph-4-$OCF_3$) |
| H | i-Pr | $CF_3$ | CH=CH (Ph-3-$SCH_3$) |
| H | i-Pr | $CF_3$ | CH=CH (Ph-4-$SCH_3$) |
| H | i-Pr | $CF_3$ | CH=CH (Ph-3-$SO_2CH_3$) |
| H | i-Pr | $CF_3$ | CH=CH (Ph-4-$SO_2CH_3$) |
| H | i-Pr | $CF_3$ | CH=CH (Ph-3, 4-$F_2$) |

| | | | |
|---|---|---|---|
| H | i-Pr | $CF_3$ | CH=CH (Ph-3-F-4-Cl) |
| H | i-Pr | $CF_3$ | CH=CH (Ph-3, 4-$Cl_2$) |
| H | i-Pr | $CF_3$ | CH=CH (Ph-3, 4-$Br_2$) |
| H | i-Pr | $CF_3$ | CH=CH (Ph-3-F-4-$CF_3$) |
| H | i-Pr | $CF_3$ | CH=CH (Ph-3-Cl-4-$OCF_3$) |
| H | i-Pr | $CF_3$ | CH=CH [Ph (-3-$OCF_2O$-4-) ] |
| H | i-Pr | $CF_3$ | Ph |
| H | i-Pr | $CF_3$ | Ph-3-F |
| H | i-Pr | $CF_3$ | Ph-4-F |
| H | i-Pr | $CF_3$ | Ph-2-Cl |
| H | i-Pr | $CF_3$ | Ph-3-Cl |
| H | i-Pr | $CF_3$ | Ph-4-Cl |
| H | i-Pr | $CF_3$ | Ph-3-Br |
| H | i-Pr | $CF_3$ | Ph-4-Br |
| H | i-Pr | $CF_3$ | Ph-3-I |
| H | i-Pr | $CF_3$ | Ph-4-I |
| H | i-Pr | $CF_3$ | Ph-4-$CH_3$ |
| H | i-Pr | $CF_3$ | Ph-4-Et |
| H | i-Pr | $CF_3$ | Ph-4-Pr-n |
| H | i-Pr | $CF_3$ | Ph-4-Pr-i |
| H | i-Pr | $CF_3$ | Ph-4-Bu-n |
| H | i-Pr | $CF_3$ | Ph-4-Bu-i |
| H | i-Pr | $CF_3$ | Ph-4-Bu-t |
| H | i-Pr | $CF_3$ | Ph-3-$CF_3$ |
| H | i-Pr | $CF_3$ | Ph-4-$CF_3$ |
| H | i-Pr | $CF_3$ | Ph-4-C $(CF_3)_2OH$ |
| H | i-Pr | $CF_3$ | Ph-4-C $(CF_3)_2OCH_3$ |
| H | i-Pr | $CF_3$ | Ph-4-$CH_2OCH_3$ |
| H | i-Pr | $CF_3$ | Ph-4-$CH_2OCH_2CF_3$ |
| H | i-Pr | $CF_3$ | Ph-4-$CH_2SCH_3$ |
| H | i-Pr | $CF_3$ | Ph-4-$CH_2S(O)CH_3$ |
| H | i-Pr | $CF_3$ | Ph-4-$CH_2SO_2CH_3$ |
| H | i-Pr | $CF_3$ | Ph-4-$CH_2SEt$ |
| H | i-Pr | $CF_3$ | Ph-4-$CH_2S(O)Et$ |
| H | i-Pr | $CF_3$ | Ph-4-$CH_2SO_2Et$ |
| H | i-Pr | $CF_3$ | Ph-4-$CH_2SPr$-n |
| H | i-Pr | $CF_3$ | Ph-4-$CH_2SO_2Pr$-n |
| H | i-Pr | $CF_3$ | Ph-4-$CH_2SPr$-i |
| H | i-Pr | $CF_3$ | Ph-4-$CH_2SO_2Pr$-i |
| H | i-Pr | $CF_3$ | Ph-4-$CH_2SPr$-c |
| H | i-Pr | $CF_3$ | Ph-4-$CH_2SO_2Pr$-c |

| | | | |
|---|---|---|---|
| H | i-Pr | $CF_3$ | Ph-4-$CH_2$SBu-n |
| H | i-Pr | $CF_3$ | Ph-4-$CH_2SO_2$Bu-n |
| H | i-Pr | $CF_3$ | Ph-4-$CH_2SCF_3$ |
| H | i-Pr | $CF_3$ | Ph-4-$CH_2$S (O) $CF_3$ |
| H | i-Pr | $CF_3$ | Ph-4-$CH_2SO_2CF_3$ |
| H | i-Pr | $CF_3$ | Ph-4-$CH_2SCH_2CF_3$ |
| H | i-Pr | $CF_3$ | Ph-4-OH |
| H | i-Pr | $CF_3$ | Ph-4-$OCH_3$ |
| H | i-Pr | $CF_3$ | Ph-4-OEt |
| H | i-Pr | $CF_3$ | Ph-4-OPr-n |
| H | i-Pr | $CF_3$ | Ph-4-OPr-i |
| H | i-Pr | $CF_3$ | Ph-4-OBu-n |
| H | i-Pr | $CF_3$ | Ph-4-OBu-t |
| H | i-Pr | $CF_3$ | Ph-4-$OCHF_2$ |
| H | i-Pr | $CF_3$ | Ph-3-$OCF_3$ |
| H | i-Pr | $CF_3$ | Ph-4-$OCF_3$ |
| H | i-Pr | $CF_3$ | Ph-4-$OCF_2$Br |
| H | i-Pr | $CF_3$ | Ph-4-$OCH_2CF_3$ |
| H | i-Pr | $CF_3$ | Ph-4-$OCF_2CHF_2$ |
| H | i-Pr | $CF_3$ | Ph-4-$OCF_2$CHFCl |
| H | i-Pr | $CF_3$ | Ph-4-$OCF_2$CHFBr |
| H | i-Pr | $CF_3$ | Ph-4-$OCF_2CF_2$Br |
| H | i-Pr | $CF_3$ | Ph-4-$OCF_2CFCl_2$ |
| H | i-Pr | $CF_3$ | Ph-4-$OCF_2CCl_3$ |
| H | i-Pr | $CF_3$ | Ph-4-$OCH_2CF_2CHF_2$ |
| H | i-Pr | $CF_3$ | Ph-3-$OCF_2CHFCF_3$ |
| H | i-Pr | $CF_3$ | Ph-4-$OCF_2CHFCF_3$ |
| H | i-Pr | $CF_3$ | Ph-4-OCH $(CF_3)_2$ |
| H | i-Pr | $CF_3$ | Ph-4-$OCF_2CFBrCF_3$ |
| H | i-Pr | $CF_3$ | Ph-3-$OCF_2CHFOCF_3$ |
| H | i-Pr | $CF_3$ | Ph-4-$OCF_2CHFOCF_3$ |
| H | i-Pr | $CF_3$ | Ph-4-$OCF_2CHFOCF_2CF_2CF_3$ |
| H | i-Pr | $CF_3$ | Ph-4-$OCH_2CH=CH_2$ |
| H | i-Pr | $CF_3$ | Ph-4-$OCH_2CH=CF_2$ |
| H | i-Pr | $CF_3$ | Ph-4-$OCH_2CF=CF_2$ |
| H | i-Pr | $CF_3$ | Ph-4-$OCH_2CH=CCl_2$ |
| H | i-Pr | $CF_3$ | Ph-4-$OCH_2CCl=CCl_2$ |
| H | i-Pr | $CF_3$ | Ph-4-$OCH_2$Ph |
| H | i-Pr | $CF_3$ | Ph-4-$OSO_2CH_3$ |
| H | i-Pr | $CF_3$ | Ph-4-$OSO_2$Et |
| H | i-Pr | $CF_3$ | Ph-4-$OSO_2$Pr-n |

| | | | |
|---|---|---|---|
| H | i-Pr | $CF_3$ | Ph-4-$OSO_2$Pr-i |
| H | i-Pr | $CF_3$ | Ph-4-$OSO_2$Bu-n |
| H | i-Pr | $CF_3$ | Ph-4-$OSO_2CHCl_2$ |
| H | i-Pr | $CF_3$ | Ph-4-$OSO_2CF_3$ |
| H | i-Pr | $CF_3$ | Ph-4-$OSO_2CH_2CF_3$ |
| H | i-Pr | $CF_3$ | Ph-3-O (Ph-4-Cl) |
| H | i-Pr | $CF_3$ | Ph-4-O (Ph-4-Cl) |
| H | i-Pr | $CF_3$ | Ph-4-O (Ph-4-Br) |
| H | i-Pr | $CF_3$ | Ph-4-O (Ph-4-$CF_3$) |
| H | i-Pr | $CF_3$ | Ph-4-O (L-21b) Br |
| H | i-Pr | $CF_3$ | Ph-4-O (L-21b) $CF_3$ |
| H | i-Pr | $CF_3$ | Ph-3-O (L-45c) Br |
| H | i-Pr | $CF_3$ | Ph-4-O (L-45c) Br |
| H | i-Pr | $CF_3$ | Ph-3-O (L-45c) $CF_3$ |
| H | i-Pr | $CF_3$ | Ph-4-O (L-45c) $CF_3$ |
| H | i-Pr | $CF_3$ | Ph-3-O (L-45e) |
| H | i-Pr | $CF_3$ | Ph-4-O (L-45e) |
| H | i-Pr | $CF_3$ | Ph-3-O (L-48b) Br |
| H | i-Pr | $CF_3$ | Ph-4-O (L-48b) Br |
| H | i-Pr | $CF_3$ | Ph-4-$SCH_3$ |
| H | i-Pr | $CF_3$ | Ph-4-S (O) $CH_3$ |
| H | i-Pr | $CF_3$ | Ph-4-$SO_2CH_3$ |
| H | i-Pr | $CF_3$ | Ph-4-SEt |
| H | i-Pr | $CF_3$ | Ph-4-S (O) Et |
| H | i-Pr | $CF_3$ | Ph-4-$SO_2$Et |
| H | i-Pr | $CF_3$ | Ph-4-SPr-n |
| H | i-Pr | $CF_3$ | Ph-4-S (O) Pr-n |
| H | i-Pr | $CF_3$ | Ph-4-$SO_2$Pr-n |
| H | i-Pr | $CF_3$ | Ph-4-SPr-i |
| H | i-Pr | $CF_3$ | Ph-4-S (O) Pr-i |
| H | i-Pr | $CF_3$ | Ph-4-$SO_2$Pr-i |
| H | i-Pr | $CF_3$ | Ph-4-SBu-n |
| H | i-Pr | $CF_3$ | Ph-4-S (O) Bu-n |
| H | i-Pr | $CF_3$ | Ph-4-$SO_2$Bu-n |
| H | i-Pr | $CF_3$ | Ph-4-SBu-t |
| H | i-Pr | $CF_3$ | Ph-4-S (O) Bu-t |
| H | i-Pr | $CF_3$ | Ph-4-$SO_2$Bu-t |
| H | i-Pr | $CF_3$ | Ph-4-$SCH_2F$ |
| H | i-Pr | $CF_3$ | Ph-4-S (O) $CH_2F$ |
| H | i-Pr | $CF_3$ | Ph-4-$SO_2CH_2F$ |
| H | i-Pr | $CF_3$ | Ph-4-$SCHF_2$ |

| | | | |
|---|---|---|---|
| H | i-Pr | $CF_3$ | Ph-4-S(O)$CHF_2$ |
| H | i-Pr | $CF_3$ | Ph-4-$SO_2CHF_2$ |
| H | i-Pr | $CF_3$ | Ph-3-$SCF_3$ |
| H | i-Pr | $CF_3$ | Ph-4-$SCF_3$ |
| H | i-Pr | $CF_3$ | Ph-4-S(O)$CF_3$ |
| H | i-Pr | $CF_3$ | Ph-4-$SO_2CF_3$ |
| H | i-Pr | $CF_3$ | Ph-4-$SCF_2Cl$ |
| H | i-Pr | $CF_3$ | Ph-4-S(O)$CF_2Cl$ |
| H | i-Pr | $CF_3$ | Ph-4-$SO_2CF_2Cl$ |
| H | i-Pr | $CF_3$ | Ph-4-$SCF_2Br$ |
| H | i-Pr | $CF_3$ | Ph-4-S(O)$CF_2Br$ |
| H | i-Pr | $CF_3$ | Ph-4-$SO_2CF_2Br$ |
| H | i-Pr | $CF_3$ | Ph-3-S(Ph-4-Cl) |
| H | i-Pr | $CF_3$ | Ph-4-S(Ph-4-Cl) |
| H | i-Pr | $CF_3$ | Ph-4-S(Ph-4-Br) |
| H | i-Pr | $CF_3$ | Ph-4-S(Ph-4-$CF_3$) |
| H | i-Pr | $CF_3$ | Ph-4-S(L-45c)Br |
| H | i-Pr | $CF_3$ | Ph-4-S(L-45c)$CF_3$ |
| H | i-Pr | $CF_3$ | Ph-4-S(L-45e) |
| H | i-Pr | $CF_3$ | Ph-4-S(L-48b)Br |
| H | i-Pr | $CF_3$ | Ph-4-$NO_2$ |
| H | i-Pr | $CF_3$ | Ph-4-N$(CH_3)_2$ |
| H | i-Pr | $CF_3$ | Ph-4-N$(Et)_2$ |
| H | i-Pr | $CF_3$ | Ph-4-(T-16) |
| H | i-Pr | $CF_3$ | Ph-4-CN |
| H | i-Pr | $CF_3$ | Ph-4-C(O)$OCH_3$ |
| H | i-Pr | $CF_3$ | Ph-4-C(O)$NH_2$ |
| H | i-Pr | $CF_3$ | Ph-4-C(O)$NHCH_3$ |
| H | i-Pr | $CF_3$ | Ph-4-C(O)NHEt |
| H | i-Pr | $CF_3$ | Ph-4-C(O)N$(CH_3)_2$ |
| H | i-Pr | $CF_3$ | Ph-4-C(S)$NH_2$ |
| H | i-Pr | $CF_3$ | Ph-3-Ph |
| H | i-Pr | $CF_3$ | Ph-4-Ph |
| H | i-Pr | $CF_3$ | Ph-4-(L-5a) |
| H | i-Pr | $CF_3$ | Ph-4-(L-14a) |
| H | i-Pr | $CF_3$ | Ph-4-(L-24a) |
| H | i-Pr | $CF_3$ | Ph-4-(L-36a) |
| H | i-Pr | $CF_3$ | Ph-2,4-$F_2$ |
| H | i-Pr | $CF_3$ | Ph-3,4-$F_2$ |
| H | i-Pr | $CF_3$ | Ph-2-Cl-4-F |
| H | i-Pr | $CF_3$ | Ph-3-Cl-4-F |

| | | | |
|---|---|---|---|
| H | i-Pr | $CF_3$ | Ph-2-F-4-Cl |
| H | i-Pr | $CF_3$ | Ph-3-F-4-Cl |
| H | i-Pr | $CF_3$ | Ph-2, 4-$Cl_2$ |
| H | i-Pr | $CF_3$ | Ph-3, 4-$Cl_2$ |
| H | i-Pr | $CF_3$ | Ph-3, 5-$Cl_2$ |
| H | i-Pr | $CF_3$ | Ph-3-Br-4-F |
| H | i-Pr | $CF_3$ | Ph-2-F-4-Br |
| H | i-Pr | $CF_3$ | Ph-3, 4-$Br_2$ |
| H | i-Pr | $CF_3$ | Ph-3-$CH_3$-4-F |
| H | i-Pr | $CF_3$ | Ph-3-F-4-$CH_3$ |
| H | i-Pr | $CF_3$ | Ph-3, 4-$(CH_3)_2$ |
| H | i-Pr | $CF_3$ | Ph-3-$CF_3$-4-F |
| H | i-Pr | $CF_3$ | Ph-3-$CF_3$-4-Cl |
| H | i-Pr | $CF_3$ | Ph-2-F-4-$CF_3$ |
| H | i-Pr | $CF_3$ | Ph-3-F-4-$CF_3$ |
| H | i-Pr | $CF_3$ | Ph-2-Cl-4-$CF_3$ |
| H | i-Pr | $CF_3$ | Ph-3-Br-4-$OCH_3$ |
| H | i-Pr | $CF_3$ | Ph-3-F-4-$OCHF_2$ |
| H | i-Pr | $CF_3$ | Ph-3-Cl-4-$OCHF_2$ |
| H | i-Pr | $CF_3$ | Ph-3-Br-4-$OCHF_2$ |
| H | i-Pr | $CF_3$ | Ph-3-F-4-$OCF_3$ |
| H | i-Pr | $CF_3$ | Ph-3-Cl-4-$OCF_3$ |
| H | i-Pr | $CF_3$ | Ph-3-Br-4-$OCF_3$ |
| H | i-Pr | $CF_3$ | Ph-3-F-4-$OCF_2Br$ |
| H | i-Pr | $CF_3$ | Ph-3-Cl-4-$OCF_2Br$ |
| H | i-Pr | $CF_3$ | Ph-3-Br-4-$OCF_2Br$ |
| H | i-Pr | $CF_3$ | Ph-3-F-4-$OCF_2CHF_2$ |
| H | i-Pr | $CF_3$ | Ph-3-Cl-4-$OCF_2CHF_2$ |
| H | i-Pr | $CF_3$ | Ph-3-Br-4-$OCF_2CHF_2$ |
| H | i-Pr | $CF_3$ | Ph-3-F-4-$OCF_2CHFCl$ |
| H | i-Pr | $CF_3$ | Ph-3-Cl-4-$OCF_2CHFCl$ |
| H | i-Pr | $CF_3$ | Ph-3-Br-4-$OCF_2CHFCl$ |
| H | i-Pr | $CF_3$ | Ph-3-F-4-$OCF_2CHFCF_3$ |
| H | i-Pr | $CF_3$ | Ph-3-Cl-4-$OCF_2CHFCF_3$ |
| H | i-Pr | $CF_3$ | Ph-3-Br-4-$OCF_2CHFCF_3$ |
| H | i-Pr | $CF_3$ | Ph-3-F-4-$OCF_2CHFOCF_3$ |
| H | i-Pr | $CF_3$ | Ph-3-Cl-4-$OCF_2CHFOCF_3$ |
| H | i-Pr | $CF_3$ | Ph-3-Br-4-$OCF_2CHFOCF_3$ |
| H | i-Pr | $CF_3$ | Ph-3-$CH_3$-4-$OCF_2CHFOCF_3$ |
| H | i-Pr | $CF_3$ | Ph-3-F-4-$OCF_2CHFOCF_2CF_2CF_3$ |
| H | i-Pr | $CF_3$ | Ph-3-Cl-4-$OCF_2CHFOCF_2CF_2CF_3$ |

| | | | |
|---|---|---|---|
| H | i-Pr | $CF_3$ | Ph-3-Br-4-$OCF_2CHFOCF_2CF_2CF_3$ |
| H | i-Pr | $CF_3$ | Ph(-3-$OCF_2O$-4-) |
| H | i-Pr | $CF_3$ | Ph(-3-$OCF_2CF_2O$-4-) |
| H | i-Pr | $CF_3$ | Ph-3-OPh-4-F |
| H | i-Pr | $CF_3$ | Ph-3-$NO_2$-4-F |
| H | i-Pr | $CF_3$ | Ph-3-$NO_2$-4-Cl |
| H | i-Pr | $CF_3$ | Ph-3-CN-4-F |
| H | i-Pr | $CF_3$ | Ph-2, 3, 4-$F_3$ |
| H | i-Pr | $CF_3$ | Ph-2, 4, 5-$F_3$ |
| H | i-Pr | $CF_3$ | Ph-3, 4, 5-$F_3$ |
| H | i-Pr | $CF_3$ | Ph-2, 3-$F_2$-4-$CH_3$ |
| H | i-Pr | $CF_3$ | Ph-2, 3-$F_2$-4-$CF_3$ |
| H | i-Pr | $CF_3$ | Ph-3, 4-$F_2$-5-$CF_3$ |
| H | i-Pr | $CF_3$ | Ph-2-F-3-Cl-5-$CF_3$ |
| H | i-Pr | $CF_3$ | Ph-3, 5-$Cl_2$-4-$OCH_3$ |
| H | i-Pr | $CF_3$ | 1-Naph |
| H | i-Pr | $CF_3$ | 2-Naph |
| H | i-Pr | $CF_3$ | L-1a |
| H | i-Pr | $CF_3$ | (L-1b) Br |
| H | i-Pr | $CF_3$ | (L-1c) Cl |
| H | i-Pr | $CF_3$ | (L-1c) Br |
| H | i-Pr | $CF_3$ | (L-1c) I |
| H | i-Pr | $CF_3$ | (L-1c) $CF_3$ |
| H | i-Pr | $CF_3$ | (L-1c) $SCH_3$ |
| H | i-Pr | $CF_3$ | (L-1c) $SO_2CH_3$ |
| H | i-Pr | $CF_3$ | (L-1c) $NO_2$ |
| H | i-Pr | $CF_3$ | L-2a |
| H | i-Pr | $CF_3$ | (L-2b) Br |
| H | i-Pr | $CF_3$ | L-3a |
| H | i-Pr | $CF_3$ | (L-3b) Cl |
| H | i-Pr | $CF_3$ | (L-3b) Br |
| H | i-Pr | $CF_3$ | (L-3b) $SCH_3$ |
| H | i-Pr | $CF_3$ | (L-3b) $SO_2CH_3$ |
| H | i-Pr | $CF_3$ | (L-3c) F |
| H | i-Pr | $CF_3$ | (L-3c) Cl |
| H | i-Pr | $CF_3$ | (L-3c) Br |
| H | i-Pr | $CF_3$ | (L-3c) I |
| H | i-Pr | $CF_3$ | (L-3c) $CF_3$ |
| H | i-Pr | $CF_3$ | (L-3c) $SCH_3$ |
| H | i-Pr | $CF_3$ | (L-3c) $SO_2CH_3$ |
| H | i-Pr | $CF_3$ | (L-3c) $NO_2$ |

| | | | |
|---|---|---|---|
| H | i-Pr | $CF_3$ | (L-3c) CN |
| H | i-Pr | $CF_3$ | L-3d |
| H | i-Pr | $CF_3$ | L-4a |
| H | i-Pr | $CF_3$ | (L-4b) Cl |
| H | i-Pr | $CF_3$ | (L-4b) Br |
| H | i-Pr | $CF_3$ | (L-4b) $NO_2$ |
| H | i-Pr | $CF_3$ | (L-4b) CN |
| H | i-Pr | $CF_3$ | (L-6a) Cl |
| H | i-Pr | $CF_3$ | (L-6a) Br |
| H | i-Pr | $CF_3$ | (L-6b) Cl |
| H | i-Pr | $CF_3$ | (L-6b) Br |
| H | i-Pr | $CF_3$ | (L-6b) $NO_2$ |
| H | i-Pr | $CF_3$ | L-8a |
| H | i-Pr | $CF_3$ | L-10a |
| H | i-Pr | $CF_3$ | (L-10b) Cl |
| H | i-Pr | $CF_3$ | (L-10b) Br |
| H | i-Pr | $CF_3$ | (L-10b) $SCH_3$ |
| H | i-Pr | $CF_3$ | L-11a |
| H | i-Pr | $CF_3$ | (L-15a) $CHF_2$ |
| H | i-Pr | $CF_3$ | (L-15b) $CF_3$ |
| H | i-Pr | $CF_3$ | (L-16a) $CHF_2$ |
| H | i-Pr | $CF_3$ | (L-16a) $CF_2Br$ |
| H | i-Pr | $CF_3$ | (L-17a) Cl |
| H | i-Pr | $CF_3$ | L-19a |
| H | i-Pr | $CF_3$ | L-20a |
| H | i-Pr | $CF_3$ | L-21a |
| H | i-Pr | $CF_3$ | (L-21b) Cl |
| H | i-Pr | $CF_3$ | (L-21b) Br |
| H | i-Pr | $CF_3$ | (L-21b) I |
| H | i-Pr | $CF_3$ | (L-21b) $CF_3$ |
| H | i-Pr | $CF_3$ | (L-21b) $NO_2$ |
| H | i-Pr | $CF_3$ | L-22a |
| H | i-Pr | $CF_3$ | (L-22b) Cl |
| H | i-Pr | $CF_3$ | (L-22b) $CF_3$ |
| H | i-Pr | $CF_3$ | (L-22b) $SCH_3$ |
| H | i-Pr | $CF_3$ | L-23a |
| H | i-Pr | $CF_3$ | (L-23b) Cl |
| H | i-Pr | $CF_3$ | (L-23b) Br |
| H | i-Pr | $CF_3$ | (L-23b) $SCH_3$ |
| H | i-Pr | $CF_3$ | (L-23b) $NO_2$ |
| H | i-Pr | $CF_3$ | (L-23c) Cl |

| | | | |
|---|---|---|---|
| H | i-Pr | $CF_3$ | (L-23c) Br |
| H | i-Pr | $CF_3$ | (L-23c) $SCH_3$ |
| H | i-Pr | $CF_3$ | (L-25a) Cl |
| H | i-Pr | $CF_3$ | (L-25a) Br |
| H | i-Pr | $CF_3$ | (L-25a) I |
| H | i-Pr | $CF_3$ | (L-25a) $CF_3$ |
| H | i-Pr | $CF_3$ | L-25b |
| H | i-Pr | $CF_3$ | (L-30a) $SCH_3$ |
| H | i-Pr | $CF_3$ | (L-31a) Cl |
| H | i-Pr | $CF_3$ | (L-31a) Br |
| H | i-Pr | $CF_3$ | (L-31a) $SCH_3$ |
| H | i-Pr | $CF_3$ | (L-34a) Ph |
| H | i-Pr | $CF_3$ | (L-35a) Ph |
| H | i-Pr | $CF_3$ | (L-38a) Cl |
| H | i-Pr | $CF_3$ | (L-38a) Br |
| H | i-Pr | $CF_3$ | (L-38a) $NO_2$ |
| H | i-Pr | $CF_3$ | L-45a |
| H | i-Pr | $CF_3$ | (L-45b) Cl |
| H | i-Pr | $CF_3$ | (L-45c) F |
| H | i-Pr | $CF_3$ | (L-45c) Cl |
| H | i-Pr | $CF_3$ | (L-45c) Br |
| H | i-Pr | $CF_3$ | (L-45c) I |
| H | i-Pr | $CF_3$ | (L-45c) $CF_3$ |
| H | i-Pr | $CF_3$ | (L-45c) $NO_2$ |
| H | i-Pr | $CF_3$ | (L-45d) F |
| H | i-Pr | $CF_3$ | (L-45d) Cl |
| H | i-Pr | $CF_3$ | (L-45d) Br |
| H | i-Pr | $CF_3$ | L-45e |
| H | i-Pr | $CF_3$ | L-45f |
| H | i-Pr | $CF_3$ | L-46a |
| H | i-Pr | $CF_3$ | (L-46b) Br |
| H | i-Pr | $CF_3$ | (L-46c) F |
| H | i-Pr | $CF_3$ | (L-46c) Cl |
| H | i-Pr | $CF_3$ | (L-46c) Br |
| H | i-Pr | $CF_3$ | (L-46c) I |
| H | i-Pr | $CF_3$ | (L-46c) $CF_3$ |
| H | i-Pr | $CF_3$ | (L-46c) $CH_2SCH_3$ |
| H | i-Pr | $CF_3$ | (L-46c) $CH_2SO_2CH_3$ |
| H | i-Pr | $CF_3$ | (L-46c) $OCH_2CF_3$ |
| H | i-Pr | $CF_3$ | (L-46c) $OCH(CF_3)_2$ |
| H | i-Pr | $CF_3$ | (L-46c) $OSO_2CH_3$ |

| | | | |
|---|---|---|---|
| H | i-Pr | $CF_3$ | (L-46c) $SCH_3$ |
| H | i-Pr | $CF_3$ | (L-46c) $SO_2CH_3$ |
| H | i-Pr | $CF_3$ | (L-46c) SEt |
| H | i-Pr | $CF_3$ | (L-46c) SPr-i |
| H | i-Pr | $CF_3$ | (L-46c) CN |
| H | i-Pr | $CF_3$ | L-46d |
| H | i-Pr | $CF_3$ | L-47a |
| H | i-Pr | $CF_3$ | (L-47b) F |
| H | i-Pr | $CF_3$ | (L-47b) Cl |
| H | i-Pr | $CF_3$ | L-47c |
| H | i-Pr | $CF_3$ | L-47d |
| H | i-Pr | $CF_3$ | L-48a |
| H | i-Pr | $CF_3$ | (L-48b) Br |
| H | i-Pr | $CF_3$ | L-50a |
| H | i-Pr | $CF_3$ | (L-50b) Cl |
| H | i-Pr | $CF_3$ | (L-50b) Br |
| H | i-Pr | $CF_3$ | (L-50b) I |
| H | i-Pr | $CF_3$ | (L-50b) $SCH_3$ |
| H | i-Pr | $CF_3$ | (L-50b) $SO_2CH_3$ |
| H | i-Pr | $CF_3$ | L-51a |
| H | i-Pr | $CF_3$ | (L-51b) Cl |
| H | i-Pr | $CF_3$ | (L-51b) $SCH_3$ |
| H | i-Pr | $CF_3$ | L-53a |
| H | i-Pr | $CF_3$ | L-55a |
| H | i-Pr | $CF_2Cl$ | $CF_2O$(Ph-4-Cl) |
| H | i-Pr | $CF_2Cl$ | CH=CH(Ph-4-Cl) |
| H | i-Pr | $CF_2Cl$ | CH=CH(Ph-4-$OCF_3$) |
| H | i-Pr | $CF_2Cl$ | CH=CH(Ph-3, 4-$Cl_2$) |
| H | i-Pr | $CF_2Cl$ | Ph-4-F |
| H | i-Pr | $CF_2Cl$ | Ph-4-Cl |
| H | i-Pr | $CF_2Cl$ | Ph-4-Br |
| H | i-Pr | $CF_2Cl$ | Ph-4-I |
| H | i-Pr | $CF_2Cl$ | Ph-4-$CF_3$ |
| H | i-Pr | $CF_2Cl$ | Ph-4-$OCHF_2$ |
| H | i-Pr | $CF_2Cl$ | Ph-4-$OCF_3$ |
| H | i-Pr | $CF_2Cl$ | Ph-4-$OCF_2Br$ |
| H | i-Pr | $CF_2Cl$ | Ph-4-$OCF_2CHF_2$ |
| H | i-Pr | $CF_2Cl$ | Ph-4-$OCF_2CHFCl$ |
| H | i-Pr | $CF_2Cl$ | Ph-4-$OCF_2CHFCF_3$ |
| H | i-Pr | $CF_2Cl$ | Ph-4-$OCF_2CHFOCF_3$ |
| H | i-Pr | $CF_2Cl$ | Ph-4-$OCF_2CHFOCF_2CF_2CF_3$ |

| | | | |
|---|---|---|---|
| H | i-Pr | $CF_2Cl$ | Ph-4-O (L-45g) |
| H | i-Pr | $CF_2Cl$ | Ph-3, 4-$Cl_2$ |
| H | i-Pr | $CF_2Cl$ | Ph (-3-O$CF_2$O-4-) |
| H | i-Pr | $CF_2Cl$ | Ph (-3-O$CF_2CF_2$O-4-) |
| H | i-Pr | $CF_2Cl$ | (L-45c) Cl |
| H | i-Pr | $CF_2Cl$ | (L-45c) Br |
| H | i-Pr | $CF_2Cl$ | (L-45c) $CF_3$ |
| H | i-Pr | $CF_2Cl$ | L-45e |
| H | i-Pr | $CF_2Cl$ | L-45f |
| H | i-Pr | $CF_2Cl$ | (L-46c) Cl |
| H | i-Pr | $CF_2Cl$ | (L-46c) Br |
| H | i-Pr | $CF_2Cl$ | (L-46c) $CF_3$ |
| H | i-Pr | $CF_2Cl$ | L-46d |
| H | i-Pr | $CFCl_2$ | Ph-4-$CF_3$ |
| H | i-Pr | $CF_2Br$ | $CF_2$O (Ph-4-Cl) |
| H | i-Pr | $CF_2Br$ | CH=CH (Ph-4-Cl) |
| H | i-Pr | $CF_2Br$ | CH=CH (Ph-4-O$CF_3$) |
| H | i-Pr | $CF_2Br$ | CH=CH (Ph-3, 4-$Cl_2$) |
| H | i-Pr | $CF_2Br$ | Ph-4-F |
| H | i-Pr | $CF_2Br$ | Ph-4-Cl |
| H | i-Pr | $CF_2Br$ | Ph-4-Br |
| H | i-Pr | $CF_2Br$ | Ph-4-I |
| H | i-Pr | $CF_2Br$ | Ph-4-$CF_3$ |
| H | i-Pr | $CF_2Br$ | Ph-4-O$CHF_2$ |
| H | i-Pr | $CF_2Br$ | Ph-4-O$CF_3$ |
| H | i-Pr | $CF_2Br$ | Ph-4-O$CF_2$Br |
| H | i-Pr | $CF_2Br$ | Ph-4-O$CF_2CHF_2$ |
| H | i-Pr | $CF_2Br$ | Ph-4-O$CF_2$CHFCl |
| H | i-Pr | $CF_2Br$ | Ph-4-O$CF_2$CHF$CF_3$ |
| H | i-Pr | $CF_2Br$ | Ph-4-O$CF_2$CHFO$CF_3$ |
| H | i-Pr | $CF_2Br$ | Ph-4-O$CF_2$CHFO$CF_2CF_2CF_3$ |
| H | i-Pr | $CF_2Br$ | Ph-4-O (L-45g) |
| H | i-Pr | $CF_2Br$ | Ph-3, 4-$Cl_2$ |
| H | i-Pr | $CF_2Br$ | Ph (-3-O$CF_2$O-4-) |
| H | i-Pr | $CF_2Br$ | Ph (-3-O$CF_2CF_2$O-4-) |
| H | i-Pr | $CF_2Br$ | (L-45c) Cl |
| H | i-Pr | $CF_2Br$ | (L-45c) Br |
| H | i-Pr | $CF_2Br$ | (L-45c) $CF_3$ |
| H | i-Pr | $CF_2Br$ | L-45e |
| H | i-Pr | $CF_2Br$ | L-45f |
| H | i-Pr | $CF_2Br$ | (L-46c) Cl |

| | | | |
|---|---|---|---|
| H | i-Pr | $CF_2Br$ | (L-46c) Br |
| H | i-Pr | $CF_2Br$ | (L-46c) $CF_3$ |
| H | i-Pr | $CF_2Br$ | L-46d |
| H | i-Pr | CFClBr | Ph-4-$OCF_3$ |
| H | i-Pr | $CFBr_2$ | (L-45c) $CF_3$ |
| H | i-Pr | $CF_2CHF_2$ | Ph-4-Br |
| H | i-Pr | $CF_2CF_3$ | $CF_2O$ (Ph-4-Cl) |
| H | i-Pr | $CF_2CF_3$ | CH=CH (Ph-4-Cl) |
| H | i-Pr | $CF_2CF_3$ | CH=CH (Ph-4-$OCF_3$) |
| H | i-Pr | $CF_2CF_3$ | CH=CH (Ph-3, 4-$Cl_2$) |
| H | i-Pr | $CF_2CF_3$ | Ph-4-F |
| H | i-Pr | $CF_2CF_3$ | Ph-4-Cl |
| H | i-Pr | $CF_2CF_3$ | Ph-4-Br |
| H | i-Pr | $CF_2CF_3$ | Ph-4-I |
| H | i-Pr | $CF_2CF_3$ | Ph-4-$CF_3$ |
| H | i-Pr | $CF_2CF_3$ | Ph-4-$OCHF_2$ |
| H | i-Pr | $CF_2CF_3$ | Ph-4-$OCF_3$ |
| H | i-Pr | $CF_2CF_3$ | Ph-4-$OCF_2Br$ |
| H | i-Pr | $CF_2CF_3$ | Ph-4-$OCF_2CHF_2$ |
| H | i-Pr | $CF_2CF_3$ | Ph-4-$OCF_2CHFCl$ |
| H | i-Pr | $CF_2CF_3$ | Ph-4-$OCF_2CHFCF_3$ |
| H | i-Pr | $CF_2CF_3$ | Ph-4-$OCF_2CHFOCF_3$ |
| H | i-Pr | $CF_2CF_3$ | Ph-4-$OCF_2CHFOCF_2CF_2CF_3$ |
| H | i-Pr | $CF_2CF_3$ | Ph-4-O (L-45g) |
| H | i-Pr | $CF_2CF_3$ | Ph-3, 4-$Cl_2$ |
| H | i-Pr | $CF_2CF_3$ | Ph (-3-$OCF_2O$-4-) |
| H | i-Pr | $CF_2CF_3$ | Ph (-3-$OCF_2CF_2O$-4-) |
| H | i-Pr | $CF_2CF_3$ | (L-45c) Cl |
| H | i-Pr | $CF_2CF_3$ | (L-45c) Br |
| H | i-Pr | $CF_2CF_3$ | (L-45c) $CF_3$ |
| H | i-Pr | $CF_2CF_3$ | L-45e |
| H | i-Pr | $CF_2CF_3$ | L-45f |
| H | i-Pr | $CF_2CF_3$ | (L-46c) Cl |
| H | i-Pr | $CF_2CF_3$ | (L-46c) Br |
| H | i-Pr | $CF_2CF_3$ | (L-46c) $CF_3$ |
| H | i-Pr | $CF_2CF_3$ | L-46d |
| H | i-Pr | $CF_2CF_2Cl$ | Ph-4-$CF_3$ |
| H | i-Pr | $CFClCF_3$ | Ph-4-$OCF_3$ |
| H | i-Pr | $CFClCF_2Cl$ | (L-45c) $CF_3$ |
| H | i-Pr | $CF_2CF_2Br$ | Ph-4-Br |
| H | i-Pr | $CFBrCF_3$ | Ph-4-$CF_3$ |

| | | | |
|---|---|---|---|
| H | i-Pr | $CF_2CHFCF_3$ | Ph-4-$OCF_3$ |
| H | i-Pr | $CF_2CF_2CF_3$ | $CF_2O$ (Ph-4-Cl) |
| H | i-Pr | $CF_2CF_2CF_3$ | CH=CH (Ph-4-Cl) |
| H | i-Pr | $CF_2CF_2CF_3$ | CH=CH (Ph-4-$OCF_3$) |
| H | i-Pr | $CF_2CF_2CF_3$ | CH=CH (Ph-3, 4-$Cl_2$) |
| H | i-Pr | $CF_2CF_2CF_3$ | Ph-4-F |
| H | i-Pr | $CF_2CF_2CF_3$ | Ph-4-Cl |
| H | i-Pr | $CF_2CF_2CF_3$ | Ph-4-Br |
| H | i-Pr | $CF_2CF_2CF_3$ | Ph-4-I |
| H | i-Pr | $CF_2CF_2CF_3$ | Ph-4-$CF_3$ |
| H | i-Pr | $CF_2CF_2CF_3$ | Ph-4-$OCHF_2$ |
| H | i-Pr | $CF_2CF_2CF_3$ | Ph-4-$OCF_3$ |
| H | i-Pr | $CF_2CF_2CF_3$ | Ph-4-$OCF_2Br$ |
| H | i-Pr | $CF_2CF_2CF_3$ | Ph-4-$OCF_2CHF_2$ |
| H | i-Pr | $CF_2CF_2CF_3$ | Ph-4-$OCF_2CHFCl$ |
| H | i-Pr | $CF_2CF_2CF_3$ | Ph-4-$OCF_2CHFCF_3$ |
| H | i-Pr | $CF_2CF_2CF_3$ | Ph-4-$OCF_2CHFOCF_3$ |
| H | i-Pr | $CF_2CF_2CF_3$ | Ph-4-$OCF_2CHFOCF_2CF_2CF_3$ |
| H | i-Pr | $CF_2CF_2CF_3$ | Ph-4-O (L-45g) |
| H | i-Pr | $CF_2CF_2CF_3$ | Ph-3, 4-$Cl_2$ |
| H | i-Pr | $CF_2CF_2CF_3$ | Ph (-3-$OCF_2O$-4-) |
| H | i-Pr | $CF_2CF_2CF_3$ | Ph (-3-$OCF_2CF_2O$-4-) |
| H | i-Pr | $CF_2CF_2CF_3$ | (L-45c) Cl |
| H | i-Pr | $CF_2CF_2CF_3$ | (L-45c) Br |
| H | i-Pr | $CF_2CF_2CF_3$ | (L-45c) $CF_3$ |
| H | i-Pr | $CF_2CF_2CF_3$ | L-45e |
| H | i-Pr | $CF_2CF_2CF_3$ | L-45f |
| H | i-Pr | $CF_2CF_2CF_3$ | (L-46c) Cl |
| H | i-Pr | $CF_2CF_2CF_3$ | (L-46c) Br |
| H | i-Pr | $CF_2CF_2CF_3$ | (L-46c) $CF_3$ |
| H | i-Pr | $CF_2CF_2CF_3$ | L-46d |
| H | i-Pr | $CF(CF_3)_2$ | (L-45c) $CF_3$ |
| H | i-Pr | $CF_2CFClCF_2Cl$ | Ph-4-Br |
| H | i-Pr | $CF_2CFBrCF_2Cl$ | Ph-4-$CF_3$ |
| H | i-Pr | $CF_2CF_2CF_2CHF_2$ | Ph-4-$OCF_3$ |
| H | i-Pr | $CF_2CF_2CF_2CF_3$ | (L-45c) $CF_3$ |
| H | i-Pr | $CF(CF_3)CF_2CF_3$ | Ph-4-Br |
| H | i-Pr | $CF_2CF_2CF_2CF_2Cl$ | Ph-4-$CF_3$ |
| H | i-Pr | $CF_2CF_2CF_2CF_2CF_2CF_3$ | Ph-4-$OCF_3$ |
| H | i-Pr | T-1 | (L-45c) $CF_3$ |
| H | i-Pr | T-2 | Ph-4-Br |

| | | | |
|---|---|---|---|
| H | i-Pr | $CH_2OCH_3$ | Ph-4-$CF_3$ |
| H | i-Pr | $CH_2OEt$ | Ph-4-$OCF_3$ |
| H | i-Pr | $CH_2OCH_2CF_3$ | (L-45c) $CF_3$ |
| H | i-Pr | $CH_2OCH(CF_3)_2$ | Ph-4-Br |
| H | i-Pr | $CF_2OCH_3$ | $CF_2O$ (Ph-4-Cl) |
| H | i-Pr | $CF_2OCH_3$ | CH=CH (Ph-4-Cl) |
| H | i-Pr | $CF_2OCH_3$ | CH=CH (Ph-4-$OCF_3$) |
| H | i-Pr | $CF_2OCH_3$ | CH=CH (Ph-3, 4-$Cl_2$) |
| H | i-Pr | $CF_2OCH_3$ | Ph-4-F |
| H | i-Pr | $CF_2OCH_3$ | Ph-4-Cl |
| H | i-Pr | $CF_2OCH_3$ | Ph-4-Br |
| H | i-Pr | $CF_2OCH_3$ | Ph-4-I |
| H | i-Pr | $CF_2OCH_3$ | Ph-4-$CF_3$ |
| H | i-Pr | $CF_2OCH_3$ | Ph-4-$OCHF_2$ |
| H | i-Pr | $CF_2OCH_3$ | Ph-4-$OCF_3$ |
| H | i-Pr | $CF_2OCH_3$ | Ph-4-$OCF_2Br$ |
| H | i-Pr | $CF_2OCH_3$ | Ph-4-$OCF_2CHF_2$ |
| H | i-Pr | $CF_2OCH_3$ | Ph-4-$OCF_2CHFCl$ |
| H | i-Pr | $CF_2OCH_3$ | Ph-4-$OCF_2CHFCF_3$ |
| H | i-Pr | $CF_2OCH_3$ | Ph-4-$OCF_2CHFOCF_3$ |
| H | i-Pr | $CF_2OCH_3$ | Ph-4-$OCF_2CHFOCF_2CF_2CF_3$ |
| H | i-Pr | $CF_2OCH_3$ | Ph-4-O (L-45g) |
| H | i-Pr | $CF_2OCH_3$ | Ph-3, 4-$Cl_2$ |
| H | i-Pr | $CF_2OCH_3$ | Ph (-3-$OCF_2O$-4-) |
| H | i-Pr | $CF_2OCH_3$ | Ph (-3-$OCF_2CF_2O$-4-) |
| H | i-Pr | $CF_2OCH_3$ | (L-45c) Cl |
| H | i-Pr | $CF_2OCH_3$ | (L-45c) Br |
| H | i-Pr | $CF_2OCH_3$ | (L-45c) $CF_3$ |
| H | i-Pr | $CF_2OCH_3$ | L-45e |
| H | i-Pr | $CF_2OCH_3$ | L-45f |
| H | i-Pr | $CF_2OCH_3$ | (L-46c) Cl |
| H | i-Pr | $CF_2OCH_3$ | (L-46c) Br |
| H | i-Pr | $CF_2OCH_3$ | (L-46c) $CF_3$ |
| H | i-Pr | $CF_2OCH_3$ | L-46d |
| H | i-Pr | $CF_2OCF_2CF_2CF_3$ | Ph-4-$CF_3$ |
| H | i-Pr | $CF_2OCF_2CF_2OCF_3$ | Ph-4-$OCF_3$ |
| H | i-Pr | $CF(CF_3)OCH_3$ | (L-45c) $CF_3$ |
| H | i-Pr | $CF(CF_3)OCF_2CF_2CF_3$ | Ph-4-Br |
| H | i-Pr | $CH_2SCH_3$ | Ph-4-$CF_3$ |
| H | i-Pr | $CH_2SO_2CH_3$ | Ph-4-$OCF_3$ |
| H | i-Pr | $CH_2SEt$ | (L-45c) $CF_3$ |

| | | | |
|---|---|---|---|
| H | i-Pr | $CH_2SCF_3$ | Ph-4-Br |
| H | i-Pr | $CH_2SPh$ | Ph-4-$CF_3$ |
| H | i-Pr | $CH_2CH_2SCH_3$ | Ph-4-$OCF_3$ |
| H | i-Pr | $CH_2CH_2SCF_3$ | (L-45c) $CF_3$ |
| H | i-Pr | $CF_2SCH_3$ | $CF_2O$ (Ph-4-Cl) |
| H | i-Pr | $CF_2SCH_3$ | CH=CH (Ph-4-Cl) |
| H | i-Pr | $CF_2SCH_3$ | CH=CH (Ph-4-$OCF_3$) |
| H | i-Pr | $CF_2SCH_3$ | CH=CH (Ph-3, 4-$Cl_2$) |
| H | i-Pr | $CF_2SCH_3$ | Ph-4-F |
| H | i-Pr | $CF_2SCH_3$ | Ph-4-Cl |
| H | i-Pr | $CF_2SCH_3$ | Ph-4-Br |
| H | i-Pr | $CF_2SCH_3$ | Ph-4-I |
| H | i-Pr | $CF_2SCH_3$ | Ph-4-$CF_3$ |
| H | i-Pr | $CF_2SCH_3$ | Ph-4-$OCHF_2$ |
| H | i-Pr | $CF_2SCH_3$ | Ph-4-$OCF_3$ |
| H | i-Pr | $CF_2SCH_3$ | Ph-4-$OCF_2Br$ |
| H | i-Pr | $CF_2SCH_3$ | Ph-4-$OCF_2CHF_2$ |
| H | i-Pr | $CF_2SCH_3$ | Ph-4-$OCF_2CHFCl$ |
| H | i-Pr | $CF_2SCH_3$ | Ph-4-$OCF_2CHFCF_3$ |
| H | i-Pr | $CF_2SCH_3$ | Ph-4-$OCF_2CHFOCF_3$ |
| H | i-Pr | $CF_2SCH_3$ | Ph-4-$OCF_2CHFOCF_2CF_2CF_3$ |
| H | i-Pr | $CF_2SCH_3$ | Ph-4-O (L-45g) |
| H | i-Pr | $CF_2SCH_3$ | Ph-3, 4-$Cl_2$ |
| H | i-Pr | $CF_2SCH_3$ | Ph (-3-$OCF_2O$-4-) |
| H | i-Pr | $CF_2SCH_3$ | Ph (-3-$OCF_2CF_2O$-4-) |
| H | i-Pr | $CF_2SCH_3$ | (L-45c) Cl |
| H | i-Pr | $CF_2SCH_3$ | (L-45c) Br |
| H | i-Pr | $CF_2SCH_3$ | (L-45c) $CF_3$ |
| H | i-Pr | $CF_2SCH_3$ | L-45e |
| H | i-Pr | $CF_2SCH_3$ | L-45f |
| H | i-Pr | $CF_2SCH_3$ | (L-46c) Cl |
| H | i-Pr | $CF_2SCH_3$ | (L-46c) Br |
| H | i-Pr | $CF_2SCH_3$ | (L-46c) $CF_3$ |
| H | i-Pr | $CF_2SCH_3$ | L-46d |
| H | i-Pr | $CF_2SEt$ | Ph-4-Br |
| H | i-Pr | $CF_2SPr$-n | Ph-4-$CF_3$ |
| H | i-Pr | $CF_2SPr$-i | Ph-4-$OCF_3$ |
| H | i-Pr | $CF_2SCH_2Ph$ | (L-45c) $CF_3$ |
| H | i-Pr | $CF_2SPh$ | Ph-4-Br |
| H | i-Pr | $CF_2C$ (O) OEt | Ph-4-$CF_3$ |
| H | i-Pr | $CF_2SO_2N$ $(CH_3)_2$ | Ph-4-$OCF_3$ |

| | | | |
|---|---|---|---|
| H | i-Pr | CN | (L-45c) $CF_3$ |
| H | i-Pr | C (O) $OCH_3$ | Ph-4-Br |
| H | i-Pr | C (O) OEt | Ph-4-$CF_3$ |
| H | i-Pr | C (O) OPr-n | Ph-4-$OCF_3$ |
| H | i-Pr | C (O) OPr-i | (L-45c) $CF_3$ |
| H | i-Pr | C (O) OBu-n | Ph-4-Br |
| H | i-Pr | C (O) OBu-t | Ph-4-$CF_3$ |
| H | i-Pr | C (O) SEt | Ph-4-$OCF_3$ |
| H | i-Pr | C (O) $NH_2$ | (L-45c) $CF_3$ |
| H | i-Pr | C (S) $NH_2$ | Ph-4-Br |
| H | i-Pr | C ($CH_3$) =$NOCH_3$ | Ph-4-$CF_3$ |
| H | i-Pr | Ph | Ph-4-$OCF_3$ |
| H | i-Pr | Ph-4-F | (L-45c) $CF_3$ |
| H | i-Pr | Ph-4-Cl | Ph-4-Br |
| H | i-Pr | Ph-4-$CF_3$ | Ph-4-$CF_3$ |
| H | i-Pr | Ph-4-$OCF_3$ | Ph-4-$OCF_3$ |
| $CH_3$ | i-Pr | $CF_3$ | Ph-4-Br |
| $CH_3$ | i-Pr | $CF_3$ | Ph-4-$CF_3$ |
| $CH_3$ | i-Pr | $CF_3$ | Ph-4-$OCF_3$ |
| $CH_3$ | i-Pr | $CF_3$ | (L-45c) $CF_3$ |
| Et | i-Pr | $CF_3$ | (L-45c) $CF_3$ |
| i-Pr | i-Pr | $CF_3$ | Ph-4-Br |
| $CH_2OCH_3$ | i-Pr | $CF_3$ | Ph-4-$CF_3$ |
| $CH_2OEt$ | i-Pr | $CF_3$ | Ph-4-$OCF_3$ |
| H | c-Pr | $CF_3$ | $CF_2O$ (Ph-4-Cl) |
| H | c-Pr | $CF_3$ | CH=CH (Ph-4-Cl) |
| H | c-Pr | $CF_3$ | CH=CH (Ph-4-$OCF_3$) |
| H | c-Pr | $CF_3$ | CH=CH (Ph-3, 4-$Cl_2$) |
| H | c-Pr | $CF_3$ | Ph-4-F |
| H | c-Pr | $CF_3$ | Ph-4-Cl |
| H | c-Pr | $CF_3$ | Ph-4-Br |
| H | c-Pr | $CF_3$ | Ph-4-I |
| H | c-Pr | $CF_3$ | Ph-4-$CF_3$ |
| H | c-Pr | $CF_3$ | Ph-4-$OCHF_2$ |
| H | c-Pr | $CF_3$ | Ph-4-$OCF_3$ |
| H | c-Pr | $CF_3$ | Ph-4-$OCF_2Br$ |
| H | c-Pr | $CF_3$ | Ph-4-$OCF_2CHF_2$ |
| H | c-Pr | $CF_3$ | Ph-4-$OCF_2CHFCl$ |
| H | c-Pr | $CF_3$ | Ph-4-$OCF_2CHFCF_3$ |
| H | c-Pr | $CF_3$ | Ph-4-$OCF_2CHFOCF_3$ |
| H | c-Pr | $CF_3$ | Ph-4-$OCF_2CHFOCF_2CF_2CF_3$ |

| | | | |
|---|---|---|---|
| H | c-Pr | $CF_3$ | Ph-4-O (L-45g) |
| H | c-Pr | $CF_3$ | Ph-3, 4-$Cl_2$ |
| H | c-Pr | $CF_3$ | Ph (-3-$OCF_2O$-4-) |
| H | c-Pr | $CF_3$ | Ph (-3-$OCF_2CF_2O$-4-) |
| H | c-Pr | $CF_3$ | (L-45c) Cl |
| H | c-Pr | $CF_3$ | (L-45c) Br |
| H | c-Pr | $CF_3$ | (L-45c) $CF_3$ |
| H | c-Pr | $CF_3$ | L-45e |
| H | c-Pr | $CF_3$ | L-45f |
| H | c-Pr | $CF_3$ | (L-46c) Cl |
| H | c-Pr | $CF_3$ | (L-46c) Br |
| H | c-Pr | $CF_3$ | (L-46c) $CF_3$ |
| H | c-Pr | $CF_3$ | L-46d |
| H | n-Bu | $CF_3$ | CH=CH (Ph-4-Cl) |
| H | n-Bu | $CF_3$ | Ph-4-Cl |
| H | n-Bu | $CF_3$ | Ph-4-Br |
| H | n-Bu | $CF_3$ | Ph-4-$CF_3$ |
| H | n-Bu | $CF_3$ | Ph-4-$OCF_3$ |
| H | n-Bu | $CF_3$ | Ph-4-$OCF_2Br$ |
| H | n-Bu | $CF_3$ | Ph-4-$OCF_2CHFCF_3$ |
| H | n-Bu | $CF_3$ | Ph-4-$OCF_2CHFOCF_3$ |
| H | n-Bu | $CF_3$ | Ph-4-$OCF_2CHFOCF_2CF_2CF_3$ |
| H | n-Bu | $CF_3$ | Ph-4-O (L-45g) |
| H | n-Bu | $CF_3$ | Ph-3, 4-$Cl_2$ |
| H | n-Bu | $CF_3$ | Ph (-3-$OCF_2O$-4-) |
| H | n-Bu | $CF_3$ | Ph (-3-$OCF_2CF_2O$-4-) |
| H | n-Bu | $CF_3$ | (L-45c) $CF_3$ |
| H | n-Bu | $CF_3$ | L-45f |
| H | n-Bu | $CF_3$ | (L-46c) $CF_3$ |
| H | n-Bu | $CF_3$ | L-46d |
| H | i-Bu | $CF_3$ | (L-45c) $CF_3$ |
| $CH_3$ | i-Bu | $CF_3$ | Ph-4-Br |
| H | $CH_2$Pr-c | $CF_3$ | Ph-4-$CF_3$ |
| H | s-Bu | $CH_3$ | Ph-4-Br |
| H | s-Bu | $CH_3$ | Ph-4-$CF_3$ |
| H | s-Bu | $CH_3$ | Ph-4-$OCF_3$ |
| H | s-Bu | $CH_3$ | (L-45c) Cl |
| H | s-Bu | $CH_3$ | (L-45c) Br |
| H | s-Bu | $CH_3$ | (L-45c) $CF_3$ |
| H | s-Bu | Et | Ph-4-$OCF_3$ |
| H | s-Bu | n-Pr | (L-45c) $CF_3$ |

| | | | |
|---|---|---|---|
| H | s-Bu | i-Pr | Ph-4-Br |
| H | s-Bu | c-Pr | Ph-4-$CF_3$ |
| H | s-Bu | $CHF_2$ | Ph-4-$OCF_3$ |
| H | s-Bu | $CF_3$ | $CF_2O$(Ph-4-Cl) |
| H | s-Bu | $CF_3$ | CH=CH(Ph-4-Cl) |
| H | s-Bu | $CF_3$ | CH=CH(Ph-4-$OCF_3$) |
| H | s-Bu | $CF_3$ | CH=CH(Ph-3, 4-$Cl_2$) |
| H | s-Bu | $CF_3$ | Ph-4-F |
| H | s-Bu | $CF_3$ | Ph-4-Cl |
| H | s-Bu | $CF_3$ | Ph-4-Br |
| H | s-Bu | $CF_3$ | Ph-4-I |
| H | s-Bu | $CF_3$ | Ph-4-$CF_3$ |
| H | s-Bu | $CF_3$ | Ph-4-$OCHF_2$ |
| H | s-Bu | $CF_3$ | Ph-4-$OCF_3$ |
| H | s-Bu | $CF_3$ | Ph-4-$OCF_2Br$ |
| H | s-Bu | $CF_3$ | Ph-4-$OCF_2CHF_2$ |
| H | s-Bu | $CF_3$ | Ph-4-$OCF_2CHFCl$ |
| H | s-Bu | $CF_3$ | Ph-4-$OCF_2CHFCF_3$ |
| H | s-Bu | $CF_3$ | Ph-4-$OCF_2CHFOCF_3$ |
| H | s-Bu | $CF_3$ | Ph-4-$OCF_2CHFOCF_2CF_2CF_3$ |
| H | s-Bu | $CF_3$ | Ph-4-O(L-45g) |
| H | s-Bu | $CF_3$ | Ph-3, 4-$Cl_2$ |
| H | s-Bu | $CF_3$ | Ph(-3-$OCF_2O$-4-) |
| H | s-Bu | $CF_3$ | Ph(-3-$OCF_2CF_2O$-4-) |
| H | s-Bu | $CF_3$ | (L-45c)Cl |
| H | s-Bu | $CF_3$ | (L-45c)Br |
| H | s-Bu | $CF_3$ | (L-45c)$CF_3$ |
| H | s-Bu | $CF_3$ | L-45e |
| H | s-Bu | $CF_3$ | L-45f |
| H | s-Bu | $CF_3$ | (L-46c)Cl |
| H | s-Bu | $CF_3$ | (L-46c)Br |
| H | s-Bu | $CF_3$ | (L-46c)$CF_3$ |
| H | s-Bu | $CF_3$ | L-46d |
| H | s-Bu | $CF_2Cl$ | (L-45c)$CF_3$ |
| H | s-Bu | $CF_2Br$ | Ph-4-Br |
| H | s-Bu | $CF_2CF_3$ | Ph-4-$CF_3$ |
| H | s-Bu | $CF_2CF_2CF_3$ | Ph-4-$OCF_3$ |
| H | s-Bu | $CF_2OCH_3$ | (L-45c)$CF_3$ |
| H | s-Bu | $CF_2SCH_3$ | Ph-4-Br |
| H | t-Bu | $CH_3$ | Ph-4-Br |
| H | t-Bu | $CH_3$ | Ph-4-$CF_3$ |

| | | | |
|---|---|---|---|
| H | t-Bu | $CH_3$ | Ph-4-$OCF_3$ |
| H | t-Bu | $CH_3$ | (L-45c) Cl |
| H | t-Bu | $CH_3$ | (L-45c) Br |
| H | t-Bu | $CH_3$ | (L-45c) $CF_3$ |
| H | t-Bu | Et | Ph-4-$CF_3$ |
| H | t-Bu | n-Pr | Ph-4-$OCF_3$ |
| H | t-Bu | i-Pr | (L-45c) $CF_3$ |
| H | t-Bu | c-Pr | Ph-4-Br |
| H | t-Bu | $CHF_2$ | Ph-4-$CF_3$ |
| H | t-Bu | $CF_3$ | $CF_2O$ (Ph-4-Cl) |
| H | t-Bu | $CF_3$ | CH=CH (Ph-4-Cl) |
| H | t-Bu | $CF_3$ | CH=CH (Ph-4-$OCF_3$) |
| H | t-Bu | $CF_3$ | CH=CH (Ph-3, 4-$Cl_2$) |
| H | t-Bu | $CF_3$ | Ph-4-F |
| H | t-Bu | $CF_3$ | Ph-4-Cl |
| H | t-Bu | $CF_3$ | Ph-4-Br |
| H | t-Bu | $CF_3$ | Ph-4-I |
| H | t-Bu | $CF_3$ | Ph-4-$CF_3$ |
| H | t-Bu | $CF_3$ | Ph-4-$OCHF_2$ |
| H | t-Bu | $CF_3$ | Ph-4-$OCF_3$ |
| H | t-Bu | $CF_3$ | Ph-4-$OCF_2Br$ |
| H | t-Bu | $CF_3$ | Ph-4-$OCF_2CHF_2$ |
| H | t-Bu | $CF_3$ | Ph-4-$OCF_2CHFCl$ |
| H | t-Bu | $CF_3$ | Ph-4-$OCF_2CHFCF_3$ |
| H | t-Bu | $CF_3$ | Ph-4-$OCF_2CHFOCF_3$ |
| H | t-Bu | $CF_3$ | Ph-4-$OCF_2CHFOCF_2CF_2CF_3$ |
| H | t-Bu | $CF_3$ | Ph-4-O (L-45g) |
| H | t-Bu | $CF_3$ | Ph-3, 4-$Cl_2$ |
| H | t-Bu | $CF_3$ | Ph (-3-$OCF_2O$-4-) |
| H | t-Bu | $CF_3$ | Ph (-3-$OCF_2CF_2O$-4-) |
| H | t-Bu | $CF_3$ | (L-45c) Cl |
| H | t-Bu | $CF_3$ | (L-45c) Br |
| H | t-Bu | $CF_3$ | (L-45c) $CF_3$ |
| H | t-Bu | $CF_3$ | L-45e |
| H | t-Bu | $CF_3$ | L-45f |
| H | t-Bu | $CF_3$ | (L-46c) Cl |
| H | t-Bu | $CF_3$ | (L-46c) Br |
| H | t-Bu | $CF_3$ | (L-46c) $CF_3$ |
| H | t-Bu | $CF_3$ | L-46d |
| H | t-Bu | $CF_2Cl$ | Ph-4-$OCF_3$ |
| H | t-Bu | $CF_2Br$ | (L-45c) $CF_3$ |

| | | | |
|---|---|---|---|
| H | t-Bu | $CF_2CF_3$ | Ph-4-Br |
| H | t-Bu | $CF_2CF_2CF_3$ | Ph-4-$CF_3$ |
| H | t-Bu | $CF_2OCH_3$ | Ph-4-$OCF_3$ |
| H | t-Bu | $CF_2SCH_3$ | (L-45c) $CF_3$ |
| H | c-Bu | $CF_3$ | Ph-4-Br |
| H | n-Pen | $CF_3$ | Ph-4-$CF_3$ |
| H | $CH_2CH_2$Pr-i | $CF_3$ | Ph-4-$OCF_3$ |
| H | $CH_2CH(CH_3)$Et | $CF_3$ | (L-45c) $CF_3$ |
| H | $CH_2$Bu-t | $CF_3$ | Ph-4-Br |
| H | $CH(CH_3)$Pr-n | $CF_3$ | $CF_2O$(Ph-4-Cl) |
| H | $CH(CH_3)$Pr-n | $CF_3$ | CH=CH(Ph-4-Cl) |
| H | $CH(CH_3)$Pr-n | $CF_3$ | CH=CH(Ph-4-$OCF_3$) |
| H | $CH(CH_3)$Pr-n | $CF_3$ | CH=CH(Ph-3, 4-$Cl_2$) |
| H | $CH(CH_3)$Pr-n | $CF_3$ | Ph-4-F |
| H | $CH(CH_3)$Pr-n | $CF_3$ | Ph-4-Cl |
| H | $CH(CH_3)$Pr-n | $CF_3$ | Ph-4-Br |
| H | $CH(CH_3)$Pr-n | $CF_3$ | Ph-4-I |
| H | $CH(CH_3)$Pr-n | $CF_3$ | Ph-4-$CF_3$ |
| H | $CH(CH_3)$Pr-n | $CF_3$ | Ph-4-$OCHF_2$ |
| H | $CH(CH_3)$Pr-n | $CF_3$ | Ph-4-$OCF_3$ |
| H | $CH(CH_3)$Pr-n | $CF_3$ | Ph-4-$OCF_2Br$ |
| H | $CH(CH_3)$Pr-n | $CF_3$ | Ph-4-$OCF_2CHF_2$ |
| H | $CH(CH_3)$Pr-n | $CF_3$ | Ph-4-$OCF_2CHFCl$ |
| H | $CH(CH_3)$Pr-n | $CF_3$ | Ph-4-$OCF_2CHFCF_3$ |
| H | $CH(CH_3)$Pr-n | $CF_3$ | Ph-4-$OCF_2CHFOCF_3$ |
| H | $CH(CH_3)$Pr-n | $CF_3$ | Ph-4-$OCF_2CHFOCF_2CF_2CF_3$ |
| H | $CH(CH_3)$Pr-n | $CF_3$ | Ph-4-O(L-45g) |
| H | $CH(CH_3)$Pr-n | $CF_3$ | Ph-3, 4-$Cl_2$ |
| H | $CH(CH_3)$Pr-n | $CF_3$ | Ph(-3-$OCF_2O$-4-) |
| H | $CH(CH_3)$Pr-n | $CF_3$ | Ph(-3-$OCF_2CF_2O$-4-) |
| H | $CH(CH_3)$Pr-n | $CF_3$ | (L-45c) Cl |
| H | $CH(CH_3)$Pr-n | $CF_3$ | (L-45c) Br |
| H | $CH(CH_3)$Pr-n | $CF_3$ | (L-45c) $CF_3$ |
| H | $CH(CH_3)$Pr-n | $CF_3$ | L-45e |
| H | $CH(CH_3)$Pr-n | $CF_3$ | L-45f |
| H | $CH(CH_3)$Pr-n | $CF_3$ | (L-46c) Cl |
| H | $CH(CH_3)$Pr-n | $CF_3$ | (L-46c) Br |
| H | $CH(CH_3)$Pr-n | $CF_3$ | (L-46c) $CF_3$ |
| H | $CH(CH_3)$Pr-n | $CF_3$ | L-46d |
| H | $CH(CH_3)$Pr-i | $CF_3$ | Ph-4-$CF_3$ |
| H | $CH(Et)_2$ | $CF_3$ | Ph-4-$OCF_3$ |

| | | | |
|---|---|---|---|
| H | $C(CH_3)_2Et$ | $CF_3$ | Ph-4-Br |
| H | $C(CH_3)_2Et$ | $CF_3$ | Ph-4-$CF_3$ |
| H | $C(CH_3)_2Et$ | $CF_3$ | Ph-4-$OCF_3$ |
| H | $C(CH_3)_2Et$ | $CF_3$ | (L-45c) $CF_3$ |
| H | c-Pen | $CF_3$ | (L-45c) $CF_3$ |
| H | n-Hex | $CF_3$ | Ph-4-Br |
| H | $CH(CH_3)$Bu-i | $CF_3$ | Ph-4-$CF_3$ |
| H | $C(CH_3)_2$Pr-n | $CF_3$ | Ph-4-Br |
| H | $C(CH_3)_2$Pr-n | $CF_3$ | Ph-4-$CF_3$ |
| H | $C(CH_3)_2$Pr-n | $CF_3$ | Ph-4-$OCF_3$ |
| H | $C(CH_3)_2$Pr-n | $CF_3$ | (L-45c) $CF_3$ |
| H | c-Hex | $CF_3$ | Ph-4-$OCF_3$ |
| H | $CH_2$Hex-c | $CF_3$ | (L-45c) $CF_3$ |
| H | Oct | $CF_3$ | Ph-4-Br |
| H | $C(CH_3)_2CH_2$Bu-t | $CF_3$ | Ph-4-$CF_3$ |
| $-CH_2CH_2CH_2CH_2-$ | | $CF_3$ | Ph-4-$OCF_3$ |
| $-CH_2CH_2CH_2CH_2CH_2-$ | | $CF_3$ | (L-45c) $CF_3$ |
| $-CH_2CH_2CH(CH_3)CH_2CH_2-$ | | $CF_3$ | Ph-4-Br |
| $-CH_2CH(CH_3)CH_2CH(CH_3)CH_2-$ | | $CF_3$ | Ph-4-$CF_3$ |
| $-CH_2CH_2CH_2CH_2CH_2CH_2-$ | | $CF_3$ | Ph-4-$OCF_3$ |
| H | $CH_2CH_2F$ | $CF_3$ | (L-45c) $CF_3$ |
| H | $CH_2CH_2Cl$ | $CF_3$ | Ph-4-Br |
| H | $CH_2CF_3$ | $CF_3$ | Ph-4-$CF_3$ |
| H | $CH(CH_3)CH_2F$ | $CF_3$ | Ph-4-Br |
| H | $CH(CH_3)CH_2F$ | $CF_3$ | Ph-4-$CF_3$ |
| H | $CH(CH_3)CH_2F$ (S) | $CF_3$ | Ph-4-$CF_3$ |
| H | $CH(CH_3)CH_2F$ | $CF_3$ | Ph-4-$OCF_3$ |
| H | $CH(CH_3)CH_2F$ | $CF_3$ | (L-45c) $CF_3$ |
| H | $CH(CH_3)CH_2Cl$ | $CF_3$ | Ph-4-Br |
| H | $CH(CH_3)CH_2Cl$ | $CF_3$ | Ph-4-$CF_3$ |
| H | $CH(CH_3)CH_2Cl$ | $CF_3$ | Ph-4-$OCF_3$ |
| H | $CH(CH_3)CH_2Cl$ | $CF_3$ | (L-45c) $CF_3$ |
| H | $CH(CH_3)CH_2Br$ | $CF_3$ | Ph-4-$OCF_3$ |
| H | $CH(CH_3)CH_2Br$ (R) | $CF_3$ | Ph-4-$OCF_3$ |
| H | $CH(CH_3)CH_2Br$ (S) | $CF_3$ | Ph-4-$OCF_3$ |
| H | $C(CH_3)_2CH_2Cl$ | $CF_3$ | Ph-4-Br |
| H | $C(CH_3)_2CH_2Cl$ | $CF_3$ | Ph-4-$CF_3$ |
| H | $C(CH_3)_2CH_2Cl$ | $CF_3$ | Ph-4-$OCF_3$ |
| H | $C(CH_3)_2CH_2Cl$ | $CF_3$ | (L-45c) $CF_3$ |
| H | $C(CH_3)_2CH_2Br$ | $CF_3$ | (L-45c) $CF_3$ |
| H | $C(CH_3)_2CHBrCH_2Br$ | $CF_3$ | Ph-4-Br |

| | | | |
|---|---|---|---|
| H | $CH_2OCH_3$ | $CF_3$ | Ph-4-$CF_3$ |
| H | $CH_2CH_2OCH_3$ | $CF_3$ | Ph-4-$OCF_3$ |
| H | $CH_2CH_2OEt$ | $CF_3$ | (L-45c) $CF_3$ |
| $CH_2CH_2OEt$ | $CH_2CH_2OEt$ | $CF_3$ | Ph-4-Br |
| H | $CH_2CH_2OC(O)NHEt$ | $CF_3$ | Ph-4-$CF_3$ |
| H | $CH_2CH_2OPh$ | $CF_3$ | Ph-4-$OCF_3$ |
| H | $CH_2CH_2O$(Ph-2-Cl) | $CF_3$ | (L-45c) $CF_3$ |
| H | $CH_2CH_2O$(Ph-3-Cl) | $CF_3$ | Ph-4-Br |
| H | $CH_2CH_2O$(Ph-4-Cl) | $CF_3$ | Ph-4-$CF_3$ |
| H | $CH_2CH(OH)CH_3$ | $CF_3$ | Ph-4-$OCF_3$ |
| H | $CH_2CH(OH)Et$ | $CF_3$ | (L-45c) $CF_3$ |
| H | $CH_2CH(OH)Ph$ | $CF_3$ | Ph-4-Br |
| H | $CH_2CH(OH)CH_2Ph$ | $CF_3$ | Ph-4-$CF_3$ |
| H | $CH_2C(CH_3)_2OSi(CH_3)_3$ | $CF_3$ | CH=CH(Ph-4-Cl) |
| H | $CH_2C(CH_3)_2OSi(CH_3)_3$ | $CF_3$ | Ph-4-Cl |
| H | $CH_2C(CH_3)_2OSi(CH_3)_3$ | $CF_3$ | Ph-4-Br |
| H | $CH_2C(CH_3)_2OSi(CH_3)_3$ | $CF_3$ | Ph-4-$CF_3$ |
| H | $CH_2C(CH_3)_2OSi(CH_3)_3$ | $CF_3$ | Ph-4-$OCF_3$ |
| H | $CH_2C(CH_3)_2OSi(CH_3)_3$ | $CF_3$ | Ph-4-$OCF_2Br$ |
| H | $CH_2C(CH_3)_2OSi(CH_3)_3$ | $CF_3$ | Ph-4-$OCF_2CHFCF_3$ |
| H | $CH_2C(CH_3)_2OSi(CH_3)_3$ | $CF_3$ | Ph-4-$OCF_2CHFOCF_3$ |
| H | $CH_2C(CH_3)_2OSi(CH_3)_3$ | $CF_3$ | Ph-4-$OCF_2CHFOCF_2CF_2CF_3$ |
| H | $CH_2C(CH_3)_2OSi(CH_3)_3$ | $CF_3$ | Ph-4-O(L-45g) |
| H | $CH_2C(CH_3)_2OSi(CH_3)_3$ | $CF_3$ | Ph-3,4-$Cl_2$ |
| H | $CH_2C(CH_3)_2OSi(CH_3)_3$ | $CF_3$ | Ph(-3-$OCF_2O$-4-) |
| H | $CH_2C(CH_3)_2OSi(CH_3)_3$ | $CF_3$ | Ph(-3-$OCF_2CF_2O$-4-) |
| H | $CH_2C(CH_3)_2OSi(CH_3)_3$ | $CF_3$ | (L-45c) $CF_3$ |
| H | $CH_2C(CH_3)_2OSi(CH_3)_3$ | $CF_3$ | L-45f |
| H | $CH_2C(CH_3)_2OSi(CH_3)_3$ | $CF_3$ | (L-46c) $CF_3$ |
| H | $CH_2C(CH_3)_2OSi(CH_3)_3$ | $CF_3$ | L-46d |
| H | $CH_2CH(OEt)_2$ | $CF_3$ | Ph-4-$OCF_3$ |
| H | $CH(CH_3)CH_2OH$ | $CF_3$ | (L-45c) $CF_3$ |
| H | $CH(CH_3)CH_2OH$ (R) | $CF_3$ | (L-45c) $CF_3$ |
| H | $CH(CH_3)CH_2OH$ (S) | $CF_3$ | (L-45c) $CF_3$ |
| H | $CH(CH_3)CH_2OCH_3$ | $CH_3$ | Ph-4-Br |
| H | $CH(CH_3)CH_2OCH_3$ | $CH_3$ | Ph-4-$CF_3$ |
| H | $CH(CH_3)CH_2OCH_3$ | $CH_3$ | Ph-4-$OCF_3$ |
| H | $CH(CH_3)CH_2OCH_3$ | $CH_3$ | (L-45c) Cl |
| H | $CH(CH_3)CH_2OCH_3$ | $CH_3$ | (L-45c) Br |
| H | $CH(CH_3)CH_2OCH_3$ | $CH_3$ | (L-45c) $CF_3$ |
| H | $CH(CH_3)CH_2OCH_3$ | Et | Ph-4-Br |

| | | | |
|---|---|---|---|
| H | $CH(CH_3)CH_2OCH_3$ | n-Pr | Ph-4-$CF_3$ |
| H | $CH(CH_3)CH_2OCH_3$ | i-Pr | Ph-4-$OCF_3$ |
| H | $CH(CH_3)CH_2OCH_3$ | c-Pr | (L-45c) $CF_3$ |
| H | $CH(CH_3)CH_2OCH_3$ | $CHF_2$ | Ph-4-Br |
| H | $CH(CH_3)CH_2OCH_3$ | $CF_3$ | $CF_2O$ (Ph-4-Cl) |
| H | $CH(CH_3)CH_2OCH_3$ | $CF_3$ | CH=CH (Ph-4-Cl) |
| H | $CH(CH_3)CH_2OCH_3$ | $CF_3$ | CH=CH (Ph-4-$OCF_3$) |
| H | $CH(CH_3)CH_2OCH_3$ | $CF_3$ | CH=CH (Ph-3, 4-$Cl_2$) |
| H | $CH(CH_3)CH_2OCH_3$ | $CF_3$ | Ph-4-F |
| H | $CH(CH_3)CH_2OCH_3$ | $CF_3$ | Ph-4-Cl |
| H | $CH(CH_3)CH_2OCH_3$ | $CF_3$ | Ph-4-Br |
| H | $CH(CH_3)CH_2OCH_3$ | $CF_3$ | Ph-4-I |
| H | $CH(CH_3)CH_2OCH_3$ | $CF_3$ | Ph-4-$CF_3$ |
| H | $CH(CH_3)CH_2OCH_3$ | $CF_3$ | Ph-4-$OCHF_2$ |
| H | $CH(CH_3)CH_2OCH_3$ | $CF_3$ | Ph-4-$OCF_3$ |
| H | $CH(CH_3)CH_2OCH_3$ | $CF_3$ | Ph-4-$OCF_2Br$ |
| H | $CH(CH_3)CH_2OCH_3$ | $CF_3$ | Ph-4-$OCF_2CHF_2$ |
| H | $CH(CH_3)CH_2OCH_3$ | $CF_3$ | Ph-4-$OCF_2CHFCl$ |
| H | $CH(CH_3)CH_2OCH_3$ | $CF_3$ | Ph-4-$OCF_2CHFCF_3$ |
| H | $CH(CH_3)CH_2OCH_3$ | $CF_3$ | Ph-4-$OCF_2CHFOCF_3$ |
| H | $CH(CH_3)CH_2OCH_3$ | $CF_3$ | Ph-4-$OCF_2CHFOCF_2CF_2CF_3$ |
| H | $CH(CH_3)CH_2OCH_3$ | $CF_3$ | Ph-4-O (L-45g) |
| H | $CH(CH_3)CH_2OCH_3$ | $CF_3$ | Ph-3, 4-$Cl_2$ |
| H | $CH(CH_3)CH_2OCH_3$ | $CF_3$ | Ph (-3-$OCF_2O$-4-) |
| H | $CH(CH_3)CH_2OCH_3$ | $CF_3$ | Ph (-3-$OCF_2CF_2O$-4-) |
| H | $CH(CH_3)CH_2OCH_3$ | $CF_3$ | (L-45c) Cl |
| H | $CH(CH_3)CH_2OCH_3$ | $CF_3$ | (L-45c) Br |
| H | $CH(CH_3)CH_2OCH_3$ | $CF_3$ | (L-45c) $CF_3$ |
| H | $CH(CH_3)CH_2OCH_3$ | $CF_3$ | L-45e |
| H | $CH(CH_3)CH_2OCH_3$ | $CF_3$ | L-45f |
| H | $CH(CH_3)CH_2OCH_3$ | $CF_3$ | (L-46c) Cl |
| H | $CH(CH_3)CH_2OCH_3$ | $CF_3$ | (L-46c) Br |
| H | $CH(CH_3)CH_2OCH_3$ | $CF_3$ | (L-46c) $CF_3$ |
| H | $CH(CH_3)CH_2OCH_3$ | $CF_3$ | L-46d |
| H | $CH(CH_3)CH_2OCH_3$ | $CF_2Cl$ | Ph-4-$CF_3$ |
| H | $CH(CH_3)CH_2OCH_3$ | $CF_2Br$ | Ph-4-$OCF_3$ |
| H | $CH(CH_3)CH_2OCH_3$ | $CF_2CF_3$ | (L-45c) $CF_3$ |
| H | $CH(CH_3)CH_2OCH_3$ | $CF_2CF_2CF_3$ | Ph-4-Br |
| H | $CH(CH_3)CH_2OCH_3$ | $CF_2OCH_3$ | Ph-4-$CF_3$ |
| H | $CH(CH_3)CH_2OCH_3$ | $CF_2SCH_3$ | Ph-4-$OCF_3$ |
| H | $CH(CH_3)CH_2OEt$ | $CF_3$ | (L-45c) $CF_3$ |

| | | | |
|---|---|---|---|
| H | $CH(CH_3)CH_2OPr-n$ | $CF_3$ | Ph-4-Br |
| H | $CH(CH_3)CH_2OBu-i$ | $CF_3$ | $Ph-4-CF_3$ |
| H | $CH(CH_3)CH_2OCH_2CH_2OCH_3$ | $CF_3$ | $Ph-4-OCF_3$ |
| H | $CH(CH_3)CH_2OCH_2CH_2SCH_3$ | $CF_3$ | $(L-45c)CF_3$ |
| H | $CH(CH_3)CH_2OCH_2CH_2SEt$ | $CF_3$ | Ph-4-Br |
| H | $CH(CH_3)CH_2OCH_2Ph$ | $CF_3$ | $Ph-4-CF_3$ |
| H | $CH(CH_3)CH_2OC(O)CH_3$ | $CF_3$ | CH=CH(Ph-4-Cl) |
| H | $CH(CH_3)CH_2OC(O)CH_3$ | $CF_3$ | Ph-4-Cl |
| H | $CH(CH_3)CH_2OC(O)CH_3$ | $CF_3$ | Ph-4-Br |
| H | $CH(CH_3)CH_2OC(O)CH_3$ | $CF_3$ | $Ph-4-CF_3$ |
| H | $CH(CH_3)CH_2OC(O)CH_3$ | $CF_3$ | $Ph-4-OCF_3$ |
| H | $CH(CH_3)CH_2OC(O)CH_3$ | $CF_3$ | $Ph-4-OCF_2Br$ |
| H | $CH(CH_3)CH_2OC(O)CH_3$ | $CF_3$ | $Ph-4-OCF_2CHFCF_3$ |
| H | $CH(CH_3)CH_2OC(O)CH_3$ | $CF_3$ | $Ph-4-OCF_2CHFOCF_3$ |
| H | $CH(CH_3)CH_2OC(O)CH_3$ | $CF_3$ | $Ph-4-OCF_2CHFOCF_2CF_2CF_3$ |
| H | $CH(CH_3)CH_2OC(O)CH_3$ | $CF_3$ | Ph-4-O(L-45g) |
| H | $CH(CH_3)CH_2OC(O)CH_3$ | $CF_3$ | $Ph-3,4-Cl_2$ |
| H | $CH(CH_3)CH_2OC(O)CH_3$ | $CF_3$ | $Ph(-3-OCF_2O-4-)$ |
| H | $CH(CH_3)CH_2OC(O)CH_3$ | $CF_3$ | $Ph(-3-OCF_2CF_2O-4-)$ |
| H | $CH(CH_3)CH_2OC(O)CH_3$ | $CF_3$ | $(L-45c)CF_3$ |
| H | $CH(CH_3)CH_2OC(O)CH_3$ | $CF_3$ | L-45f |
| H | $CH(CH_3)CH_2OC(O)CH_3$ | $CF_3$ | $(L-46c)CF_3$ |
| H | $CH(CH_3)CH_2OC(O)CH_3$ | $CF_3$ | L-46d |
| H | $CH(CH_3)CH_2OC(O)CF_3$ | $CF_3$ | $Ph-4-OCF_3$ |
| H | $CH(CH_3)CH_2OC(O)NHCH_3$ | $CF_3$ | $CF_2O(Ph-4-Cl)$ |
| H | $CH(CH_3)CH_2OC(O)NHCH_3$ | $CF_3$ | CH=CH(Ph-4-Cl) |
| H | $CH(CH_3)CH_2OC(O)NHCH_3$ | $CF_3$ | CH=CH(Ph-4-Br) |
| H | $CH(CH_3)CH_2OC(O)NHCH_3$ | $CF_3$ | $CH=CH(Ph-4-CF_3)$ |
| H | $CH(CH_3)CH_2OC(O)NHCH_3$ | $CF_3$ | $CH=CH(Ph-4-OCF_3)$ |
| H | $CH(CH_3)CH_2OC(O)NHCH_3$ | $CF_3$ | $CH=CH(Ph-3,4-Cl_2)$ |
| H | $CH(CH_3)CH_2OC(O)NHCH_3$ | $CF_3$ | Ph-4-F |
| H | $CH(CH_3)CH_2OC(O)NHCH_3$ | $CF_3$ | Ph-4-Cl |
| H | $CH(CH_3)CH_2OC(O)NHCH_3$ | $CF_3$ | Ph-4-Br |
| H | $CH(CH_3)CH_2OC(O)NHCH_3$ | $CF_3$ | $Ph-4-CF_3$ |
| H | $CH(CH_3)CH_2OC(O)NHCH_3$ | $CF_3$ | $Ph-4-OCF_3$ |
| H | $CH(CH_3)CH_2OC(O)NHCH_3$ | $CF_3$ | $Ph-4-OCF_2Br$ |
| H | $CH(CH_3)CH_2OC(O)NHCH_3$ | $CF_3$ | $Ph-4-OCF_2CHF_2$ |
| H | $CH(CH_3)CH_2OC(O)NHCH_3$ | $CF_3$ | $Ph-4-OCF_2CHFCF_3$ |
| H | $CH(CH_3)CH_2OC(O)NHCH_3$ | $CF_3$ | $Ph-4-OCF_2CHFOCF_3$ |
| H | $CH(CH_3)CH_2OC(O)NHCH_3$ | $CF_3$ | $Ph-4-OCF_2CHFOCF_2CF_2CF_3$ |
| H | $CH(CH_3)CH_2OC(O)NHCH_3$ | $CF_3$ | Ph-4-O(L-45g) |

| | | | |
|---|---|---|---|
| H | $CH(CH_3)CH_2OC(O)NHCH_3$ | $CF_3$ | Ph-3, 4-$F_2$ |
| H | $CH(CH_3)CH_2OC(O)NHCH_3$ | $CF_3$ | Ph-3-F-4-Cl |
| H | $CH(CH_3)CH_2OC(O)NHCH_3$ | $CF_3$ | Ph-3, 4-$Cl_2$ |
| H | $CH(CH_3)CH_2OC(O)NHCH_3$ | $CF_3$ | Ph(-3-$OCF_2O$-4-) |
| H | $CH(CH_3)CH_2OC(O)NHCH_3$ | $CF_3$ | Ph(-3-$OCF_2CF_2O$-4-) |
| H | $CH(CH_3)CH_2OC(O)NHCH_3$ | $CF_3$ | (L-45c)$CF_3$ |
| H | $CH(CH_3)CH_2OC(O)NHCH_3$ | $CF_3$ | L-45f |
| H | $CH(CH_3)CH_2OC(O)NHCH_3$ | $CF_3$ | (L-46c)$CF_3$ |
| H | $CH(CH_3)CH_2OC(O)NHCH_3$ | $CF_3$ | L-46d |
| H | $CH(CH_3)CH_2OC(O)NHEt$ | $CH_3$ | Ph-4-Br |
| H | $CH(CH_3)CH_2OC(O)NHEt$ | $CH_3$ | Ph-4-$CF_3$ |
| H | $CH(CH_3)CH_2OC(O)NHEt$ | $CH_3$ | Ph-4-$OCF_3$ |
| H | $CH(CH_3)CH_2OC(O)NHEt$ | $CH_3$ | (L-45c)Cl |
| H | $CH(CH_3)CH_2OC(O)NHEt$ | $CH_3$ | (L-45c)Br |
| H | $CH(CH_3)CH_2OC(O)NHEt$ | $CH_3$ | (L-45c)$CF_3$ |
| H | $CH(CH_3)CH_2OC(O)NHEt$ | Et | (L-45c)$CF_3$ |
| H | $CH(CH_3)CH_2OC(O)NHEt$ | n-Pr | Ph-4-Br |
| H | $CH(CH_3)CH_2OC(O)NHEt$ | i-Pr | Ph-4-$CF_3$ |
| H | $CH(CH_3)CH_2OC(O)NHEt$ | c-Pr | Ph-4-$OCF_3$ |
| H | $CH(CH_3)CH_2OC(O)NHEt$ | $CHF_2$ | (L-45c)$CF_3$ |
| H | $CH(CH_3)CH_2OC(O)NHEt$ | $CF_3$ | $CF_2O$(Ph-4-Cl) |
| H | $CH(CH_3)CH_2OC(O)NHEt$ | $CF_3$ | CH=CH(Ph-4-Cl) |
| H | $CH(CH_3)CH_2OC(O)NHEt$ | $CF_3$ | CH=CH(Ph-4-Br) |
| H | $CH(CH_3)CH_2OC(O)NHEt$ | $CF_3$ | CH=CH(Ph-4-$CF_3$) |
| H | $CH(CH_3)CH_2OC(O)NHEt$ | $CF_3$ | CH=CH(Ph-4-$OCF_3$) |
| H | $CH(CH_3)CH_2OC(O)NHEt$ | $CF_3$ | CH=CH(Ph-3, 4-$Cl_2$) |
| H | $CH(CH_3)CH_2OC(O)NHEt$ | $CF_3$ | Ph-4-F |
| H | $CH(CH_3)CH_2OC(O)NHEt$ | $CF_3$ | Ph-4-Cl |
| H | $CH(CH_3)CH_2OC(O)NHEt$ | $CF_3$ | Ph-4-Br |
| H | $CH(CH_3)CH_2OC(O)NHEt$ | $CF_3$ | Ph-4-I |
| H | $CH(CH_3)CH_2OC(O)NHEt$ | $CF_3$ | Ph-4-$CF_3$ |
| H | $CH(CH_3)CH_2OC(O)NHEt$ | $CF_3$ | Ph-4-$OCHF_2$ |
| H | $CH(CH_3)CH_2OC(O)NHEt$ | $CF_3$ | Ph-4-$OCF_3$ |
| H | $CH(CH_3)CH_2OC(O)NHEt$ | $CF_3$ | Ph-4-$OCF_2Br$ |
| H | $CH(CH_3)CH_2OC(O)NHEt$ | $CF_3$ | Ph-4-$OCF_2CHF_2$ |
| H | $CH(CH_3)CH_2OC(O)NHEt$ | $CF_3$ | Ph-4-$OCF_2CHFCl$ |
| H | $CH(CH_3)CH_2OC(O)NHEt$ | $CF_3$ | Ph-4-$OCF_2CHFCF_3$ |
| H | $CH(CH_3)CH_2OC(O)NHEt$ | $CF_3$ | Ph-4-$OCF_2CHFOCF_3$ |
| H | $CH(CH_3)CH_2OC(O)NHEt$ | $CF_3$ | Ph-4-$OCF_2CHFOCF_2CF_2CF_3$ |
| H | $CH(CH_3)CH_2OC(O)NHEt$ | $CF_3$ | Ph-4-O(L-45g) |
| H | $CH(CH_3)CH_2OC(O)NHEt$ | $CF_3$ | Ph-3, 4-$F_2$ |

| | | | |
|---|---|---|---|
| H | $CH(CH_3)CH_2OC(O)NHEt$ | $CF_3$ | Ph-3-F-4-Cl |
| H | $CH(CH_3)CH_2OC(O)NHEt$ | $CF_3$ | Ph-3, 4-$Cl_2$ |
| H | $CH(CH_3)CH_2OC(O)NHEt$ | $CF_3$ | Ph(-3-$OCF_2O$-4-) |
| H | $CH(CH_3)CH_2OC(O)NHEt$ | $CF_3$ | Ph(-3-$OCF_2CF_2O$-4-) |
| H | $CH(CH_3)CH_2OC(O)NHEt$ | $CF_3$ | (L-45c)Cl |
| H | $CH(CH_3)CH_2OC(O)NHEt$ | $CF_3$ | (L-45c)Br |
| H | $CH(CH_3)CH_2OC(O)NHEt$ | $CF_3$ | (L-45c)$CF_3$ |
| H | $CH(CH_3)CH_2OC(O)NHEt$ | $CF_3$ | L-45e |
| H | $CH(CH_3)CH_2OC(O)NHEt$ | $CF_3$ | L-45f |
| H | $CH(CH_3)CH_2OC(O)NHEt$ | $CF_3$ | (L-46c)Cl |
| H | $CH(CH_3)CH_2OC(O)NHEt$ | $CF_3$ | (L-46c)Br |
| H | $CH(CH_3)CH_2OC(O)NHEt$ | $CF_3$ | (L-46c)$CF_3$ |
| H | $CH(CH_3)CH_2OC(O)NHEt$ | $CF_3$ | L-46d |
| H | $CH(CH_3)CH_2OC(O)NHEt$ | $CF_2Cl$ | Ph-4-Br |
| H | $CH(CH_3)CH_2OC(O)NHEt$ | $CF_2Br$ | Ph-4-$CF_3$ |
| H | $CH(CH_3)CH_2OC(O)NHEt$ | $CF_2CF_3$ | Ph-4-$OCF_3$ |
| H | $CH(CH_3)CH_2OC(O)NHEt$ | $CF_2CF_2CF_3$ | (L-45c)$CF_3$ |
| H | $CH(CH_3)CH_2OC(O)NHEt$ | $CF_2OCH_3$ | Ph-4-Br |
| H | $CH(CH_3)CH_2OC(O)NHEt$ | $CF_2SCH_3$ | Ph-4-$CF_3$ |
| H | $CH(CH_3)CH_2OC(O)NHPr$-n | $CF_3$ | CH=CH(Ph-4-Cl) |
| H | $CH(CH_3)CH_2OC(O)NHPr$-n | $CF_3$ | Ph-4-Cl |
| H | $CH(CH_3)CH_2OC(O)NHPr$-n | $CF_3$ | Ph-4-Br |
| H | $CH(CH_3)CH_2OC(O)NHPr$-n | $CF_3$ | Ph-4-$CF_3$ |
| H | $CH(CH_3)CH_2OC(O)NHPr$-n | $CF_3$ | Ph-4-$OCF_3$ |
| H | $CH(CH_3)CH_2OC(O)NHPr$-n | $CF_3$ | Ph-4-$OCF_2Br$ |
| H | $CH(CH_3)CH_2OC(O)NHPr$-n | $CF_3$ | Ph-4-$OCF_2CHFCF_3$ |
| H | $CH(CH_3)CH_2OC(O)NHPr$-n | $CF_3$ | Ph-4-$OCF_2CHFOCF_3$ |
| H | $CH(CH_3)CH_2OC(O)NHPr$-n | $CF_3$ | Ph-4-$OCF_2CHFOCF_2CF_2CF_3$ |
| H | $CH(CH_3)CH_2OC(O)NHPr$-n | $CF_3$ | Ph-4-O(L-45g) |
| H | $CH(CH_3)CH_2OC(O)NHPr$-n | $CF_3$ | Ph-3, 4-$Cl_2$ |
| H | $CH(CH_3)CH_2OC(O)NHPr$-n | $CF_3$ | Ph(-3-$OCF_2O$-4-) |
| H | $CH(CH_3)CH_2OC(O)NHPr$-n | $CF_3$ | Ph(-3-$OCF_2CF_2O$-4-) |
| H | $CH(CH_3)CH_2OC(O)NHPr$-n | $CF_3$ | (L-45c)$CF_3$ |
| H | $CH(CH_3)CH_2OC(O)NHPr$-n | $CF_3$ | L-45f |
| H | $CH(CH_3)CH_2OC(O)NHPr$-n | $CF_3$ | (L-46c)$CF_3$ |
| H | $CH(CH_3)CH_2OC(O)NHPr$-n | $CF_3$ | L-46d |
| H | $CH(CH_3)CH_2OC(O)NHPr$-i | $CF_3$ | CH=CH(Ph-4-Cl) |
| H | $CH(CH_3)CH_2OC(O)NHPr$-i | $CF_3$ | Ph-4-Cl |
| H | $CH(CH_3)CH_2OC(O)NHPr$-i | $CF_3$ | Ph-4-Br |
| H | $CH(CH_3)CH_2OC(O)NHPr$-i | $CF_3$ | Ph-4-$CF_3$ |
| H | $CH(CH_3)CH_2OC(O)NHPr$-i | $CF_3$ | Ph-4-$OCF_3$ |

| | | | |
|---|---|---|---|
| H | $CH(CH_3)CH_2OC(O)NHPr-i$ | $CF_3$ | $Ph-4-OCF_2Br$ |
| H | $CH(CH_3)CH_2OC(O)NHPr-i$ | $CF_3$ | $Ph-4-OCF_2CHFCF_3$ |
| H | $CH(CH_3)CH_2OC(O)NHPr-i$ | $CF_3$ | $Ph-4-OCF_2CHFOCF_3$ |
| H | $CH(CH_3)CH_2OC(O)NHPr-i$ | $CF_3$ | $Ph-4-OCF_2CHFOCF_2CF_2CF_3$ |
| H | $CH(CH_3)CH_2OC(O)NHPr-i$ | $CF_3$ | Ph-4-O(L-45g) |
| H | $CH(CH_3)CH_2OC(O)NHPr-i$ | $CF_3$ | $Ph-3,4-Cl_2$ |
| H | $CH(CH_3)CH_2OC(O)NHPr-i$ | $CF_3$ | $Ph(-3-OCF_2O-4-)$ |
| H | $CH(CH_3)CH_2OC(O)NHPr-i$ | $CF_3$ | $Ph(-3-OCF_2CF_2O-4-)$ |
| H | $CH(CH_3)CH_2OC(O)NHPr-i$ | $CF_3$ | (L-45c)$CF_3$ |
| H | $CH(CH_3)CH_2OC(O)NHPr-i$ | $CF_3$ | L-45f |
| H | $CH(CH_3)CH_2OC(O)NHPr-i$ | $CF_3$ | (L-46c)$CF_3$ |
| H | $CH(CH_3)CH_2OC(O)NHPr-i$ | $CF_3$ | L-46d |
| H | $CH(CH_3)CH_2OC(O)NHPr-c$ | $CF_3$ | CH=CH(Ph-4-Cl) |
| H | $CH(CH_3)CH_2OC(O)NHPr-c$ | $CF_3$ | Ph-4-Cl |
| H | $CH(CH_3)CH_2OC(O)NHPr-c$ | $CF_3$ | Ph-4-Br |
| H | $CH(CH_3)CH_2OC(O)NHPr-c$ | $CF_3$ | $Ph-4-CF_3$ |
| H | $CH(CH_3)CH_2OC(O)NHPr-c$ | $CF_3$ | $Ph-4-OCF_3$ |
| H | $CH(CH_3)CH_2OC(O)NHPr-c$ | $CF_3$ | $Ph-4-OCF_2Br$ |
| H | $CH(CH_3)CH_2OC(O)NHPr-c$ | $CF_3$ | $Ph-4-OCF_2CHFCF_3$ |
| H | $CH(CH_3)CH_2OC(O)NHPr-c$ | $CF_3$ | $Ph-4-OCF_2CHFOCF_3$ |
| H | $CH(CH_3)CH_2OC(O)NHPr-c$ | $CF_3$ | $Ph-4-OCF_2CHFOCF_2CF_2CF_3$ |
| H | $CH(CH_3)CH_2OC(O)NHPr-c$ | $CF_3$ | Ph-4-O(L-45g) |
| H | $CH(CH_3)CH_2OC(O)NHPr-c$ | $CF_3$ | $Ph-3,4-Cl_2$ |
| H | $CH(CH_3)CH_2OC(O)NHPr-c$ | $CF_3$ | $Ph(-3-OCF_2O-4-)$ |
| H | $CH(CH_3)CH_2OC(O)NHPr-c$ | $CF_3$ | $Ph(-3-OCF_2CF_2O-4-)$ |
| H | $CH(CH_3)CH_2OC(O)NHPr-c$ | $CF_3$ | (L-45c)$CF_3$ |
| H | $CH(CH_3)CH_2OC(O)NHPr-c$ | $CF_3$ | L-45f |
| H | $CH(CH_3)CH_2OC(O)NHPr-c$ | $CF_3$ | (L-46c)$CF_3$ |
| H | $CH(CH_3)CH_2OC(O)NHPr-c$ | $CF_3$ | L-46d |
| H | $CH(CH_3)CH_2OC(O)NHBu-t$ | $CF_3$ | $Ph-4-OCF_3$ |
| H | $CH(CH_3)CH_2OC(O)NHCH_2CF_3$ | $CF_3$ | (L-45c)$CF_3$ |
| H | $CH(CH_3)CH_2OC(O)NHCH_2CH_2OCH_3$ | $CF_3$ | Ph-4-Br |
| H | $CH(CH_3)CH_2OC(O)NHCH_2CH_2SCH_3$ | $CF_3$ | $Ph-4-CF_3$ |
| H | $CH(CH_3)CH_2OC(O)NHCH_2CH_2CH_2SCH_3$ | $CF_3$ | $Ph-4-OCF_3$ |
| H | $CH(CH_3)CH_2OC(O)NHCH_2CH=CH_2$ | $CF_3$ | (L-45c)$CF_3$ |
| H | $CH(CH_3)CH_2OC(O)NHCH_2Ph$ | $CF_3$ | CH=CH(Ph-4-Cl) |
| H | $CH(CH_3)CH_2OC(O)NHCH_2Ph$ | $CF_3$ | Ph-4-Cl |
| H | $CH(CH_3)CH_2OC(O)NHCH_2Ph$ | $CF_3$ | Ph-4-Br |
| H | $CH(CH_3)CH_2OC(O)NHCH_2Ph$ | $CF_3$ | $Ph-4-CF_3$ |
| H | $CH(CH_3)CH_2OC(O)NHCH_2Ph$ | $CF_3$ | $Ph-4-OCF_3$ |
| H | $CH(CH_3)CH_2OC(O)NHCH_2Ph$ | $CF_3$ | $Ph-4-OCF_2Br$ |

| | | | |
|---|---|---|---|
| H | $CH(CH_3)CH_2OC(O)NHCH_2Ph$ | $CF_3$ | $Ph-4-OCF_2CHFCF_3$ |
| H | $CH(CH_3)CH_2OC(O)NHCH_2Ph$ | $CF_3$ | $Ph-4-OCF_2CHFOCF_3$ |
| H | $CH(CH_3)CH_2OC(O)NHCH_2Ph$ | $CF_3$ | $Ph-4-OCF_2CHFOCF_2CF_2CF_3$ |
| H | $CH(CH_3)CH_2OC(O)NHCH_2Ph$ | $CF_3$ | Ph-4-O(L-45g) |
| H | $CH(CH_3)CH_2OC(O)NHCH_2Ph$ | $CF_3$ | $Ph-3,4-Cl_2$ |
| H | $CH(CH_3)CH_2OC(O)NHCH_2Ph$ | $CF_3$ | $Ph(-3-OCF_2O-4-)$ |
| H | $CH(CH_3)CH_2OC(O)NHCH_2Ph$ | $CF_3$ | $Ph(-3-OCF_2CF_2O-4-)$ |
| H | $CH(CH_3)CH_2OC(O)NHCH_2Ph$ | $CF_3$ | (L-45c)$CF_3$ |
| H | $CH(CH_3)CH_2OC(O)NHCH_2Ph$ | $CF_3$ | L-45f |
| H | $CH(CH_3)CH_2OC(O)NHCH_2Ph$ | $CF_3$ | (L-46c)$CF_3$ |
| H | $CH(CH_3)CH_2OC(O)NHCH_2Ph$ | $CF_3$ | L-46d |
| H | $CH(CH_3)CH_2OC(O)NHCH_2(Ph-4-Cl)$ | $CF_3$ | Ph-4-Br |
| H | $CH(CH_3)CH_2OC(O)NHCH_2(Ph-4-OCH_3)$ | $CF_3$ | $Ph-4-CF_3$ |
| H | $CH(CH_3)CH_2OC(O)NHCH_2$(L-46a) | $CF_3$ | $Ph-4-OCF_3$ |
| H | $CH(CH_3)CH_2OC(O)NHCH_2$(L-47a) | $CF_3$ | (L-45c)$CF_3$ |
| H | $CH(CH_3)CH_2OC(O)NHPh$ | $CF_3$ | CH=CH(Ph-4-Cl) |
| H | $CH(CH_3)CH_2OC(O)NHPh$ | $CF_3$ | Ph-4-Cl |
| H | $CH(CH_3)CH_2OC(O)NHPh$ | $CF_3$ | Ph-4-Br |
| H | $CH(CH_3)CH_2OC(O)NHPh$ | $CF_3$ | $Ph-4-CF_3$ |
| H | $CH(CH_3)CH_2OC(O)NHPh$ | $CF_3$ | $Ph-4-OCF_3$ |
| H | $CH(CH_3)CH_2OC(O)NHPh$ | $CF_3$ | $Ph-4-OCF_2Br$ |
| H | $CH(CH_3)CH_2OC(O)NHPh$ | $CF_3$ | $Ph-4-OCF_2CHFCF_3$ |
| H | $CH(CH_3)CH_2OC(O)NHPh$ | $CF_3$ | $Ph-4-OCF_2CHFOCF_3$ |
| H | $CH(CH_3)CH_2OC(O)NHPh$ | $CF_3$ | $Ph-4-OCF_2CHFOCF_2CF_2CF_3$ |
| H | $CH(CH_3)CH_2OC(O)NHPh$ | $CF_3$ | Ph-4-O(L-45g) |
| H | $CH(CH_3)CH_2OC(O)NHPh$ | $CF_3$ | $Ph-3,4-Cl_2$ |
| H | $CH(CH_3)CH_2OC(O)NHPh$ | $CF_3$ | $Ph(-3-OCF_2O-4-)$ |
| H | $CH(CH_3)CH_2OC(O)NHPh$ | $CF_3$ | $Ph(-3-OCF_2CF_2O-4-)$ |
| H | $CH(CH_3)CH_2OC(O)NHPh$ | $CF_3$ | (L-45c)$CF_3$ |
| H | $CH(CH_3)CH_2OC(O)NHPh$ | $CF_3$ | L-45f |
| H | $CH(CH_3)CH_2OC(O)NHPh$ | $CF_3$ | (L-46c)$CF_3$ |
| H | $CH(CH_3)CH_2OC(O)NHPh$ | $CF_3$ | L-46d |
| H | $CH(CH_3)CH_2OC(O)N(CH_3)_2$ | $CF_3$ | Ph-4-Br |
| H | $CH(CH_3)CH_2OC(O)N(Et)_2$ | $CF_3$ | CH=CH(Ph-4-Cl) |
| H | $CH(CH_3)CH_2OC(O)N(Et)_2$ | $CF_3$ | Ph-4-Cl |
| H | $CH(CH_3)CH_2OC(O)N(Et)_2$ | $CF_3$ | Ph-4-Br |
| H | $CH(CH_3)CH_2OC(O)N(Et)_2$ | $CF_3$ | $Ph-4-CF_3$ |
| H | $CH(CH_3)CH_2OC(O)N(Et)_2$ | $CF_3$ | $Ph-4-OCF_3$ |
| H | $CH(CH_3)CH_2OC(O)N(Et)_2$ | $CF_3$ | $Ph-4-OCF_2Br$ |
| H | $CH(CH_3)CH_2OC(O)N(Et)_2$ | $CF_3$ | $Ph-4-OCF_2CHFCF_3$ |
| H | $CH(CH_3)CH_2OC(O)N(Et)_2$ | $CF_3$ | $Ph-4-OCF_2CHFOCF_3$ |

| | | | |
|---|---|---|---|
| H | $CH(CH_3)CH_2OC(O)N(Et)_2$ | $CF_3$ | Ph-4-$OCF_2CHFOCF_2CF_2CF_3$ |
| H | $CH(CH_3)CH_2OC(O)N(Et)_2$ | $CF_3$ | Ph-4-O(L-45g) |
| H | $CH(CH_3)CH_2OC(O)N(Et)_2$ | $CF_3$ | Ph-3,4-$Cl_2$ |
| H | $CH(CH_3)CH_2OC(O)N(Et)_2$ | $CF_3$ | Ph(-3-$OCF_2O$-4-) |
| H | $CH(CH_3)CH_2OC(O)N(Et)_2$ | $CF_3$ | Ph(-3-$OCF_2CF_2O$-4-) |
| H | $CH(CH_3)CH_2OC(O)N(Et)_2$ | $CF_3$ | (L-45c)$CF_3$ |
| H | $CH(CH_3)CH_2OC(O)N(Et)_2$ | $CF_3$ | L-45f |
| H | $CH(CH_3)CH_2OC(O)N(Et)_2$ | $CF_3$ | (L-46c)$CF_3$ |
| H | $CH(CH_3)CH_2OC(O)N(Et)_2$ | $CF_3$ | L-46d |
| H | $CH(CH_3)CH_2OC(O)N(Pr\text{-}i)_2$ | $CF_3$ | Ph-4-$CF_3$ |
| H | $CH(CH_3)CH_2OC(O)N(CH_3)Ph$ | $CF_3$ | Ph-4-$OCF_3$ |
| H | $CH(CH_3)CH_2OC(O)$(T-16) | $CF_3$ | (L-45c)$CF_3$ |
| H | $CH(CH_3)CH_2OC(O)$(T-19) | $CF_3$ | Ph-4-Br |
| H | $CH(CH_3)CH_2OC(O)$(T-20) | $CF_3$ | Ph-4-$CF_3$ |
| H | $CH(CH_3)CH_2OC(O)$(T-21) | $CF_3$ | Ph-4-$OCF_3$ |
| H | $CH(CH_3)CH_2OP(O)(OEt)_2$ | $CF_3$ | (L-45c)$CF_3$ |
| H | $CH(CH_3)CH_2OP(S)(OCH_3)_2$ | $CF_3$ | CH=CH(Ph-4-Cl) |
| H | $CH(CH_3)CH_2OP(S)(OCH_3)_2$ | $CF_3$ | Ph-4-Cl |
| H | $CH(CH_3)CH_2OP(S)(OCH_3)_2$ | $CF_3$ | Ph-4-Br |
| H | $CH(CH_3)CH_2OP(S)(OCH_3)_2$ | $CF_3$ | Ph-4-$CF_3$ |
| H | $CH(CH_3)CH_2OP(S)(OCH_3)_2$ | $CF_3$ | Ph-4-$OCF_3$ |
| H | $CH(CH_3)CH_2OP(S)(OCH_3)_2$ | $CF_3$ | Ph-4-$OCF_2Br$ |
| H | $CH(CH_3)CH_2OP(S)(OCH_3)_2$ | $CF_3$ | Ph-4-$OCF_2CHFCF_3$ |
| H | $CH(CH_3)CH_2OP(S)(OCH_3)_2$ | $CF_3$ | Ph-4-$OCF_2CHFOCF_3$ |
| H | $CH(CH_3)CH_2OP(S)(OCH_3)_2$ | $CF_3$ | Ph-4-$OCF_2CHFOCF_2CF_2CF_3$ |
| H | $CH(CH_3)CH_2OP(S)(OCH_3)_2$ | $CF_3$ | Ph-4-O(L-45g) |
| H | $CH(CH_3)CH_2OP(S)(OCH_3)_2$ | $CF_3$ | Ph-3,4-$Cl_2$ |
| H | $CH(CH_3)CH_2OP(S)(OCH_3)_2$ | $CF_3$ | Ph(-3-$OCF_2O$-4-) |
| H | $CH(CH_3)CH_2OP(S)(OCH_3)_2$ | $CF_3$ | Ph(-3-$OCF_2CF_2O$-4-) |
| H | $CH(CH_3)CH_2OP(S)(OCH_3)_2$ | $CF_3$ | (L-45c)$CF_3$ |
| H | $CH(CH_3)CH_2OP(S)(OCH_3)_2$ | $CF_3$ | L-45f |
| H | $CH(CH_3)CH_2OP(S)(OCH_3)_2$ | $CF_3$ | (L-46c)$CF_3$ |
| H | $CH(CH_3)CH_2OP(S)(OCH_3)_2$ | $CF_3$ | L-46d |
| H | $CH(CH_3)CH_2OP(S)(OEt)_2$ | $CF_3$ | CH=CH(Ph-4-Cl) |
| H | $CH(CH_3)CH_2OP(S)(OEt)_2$ | $CF_3$ | Ph-4-Cl |
| H | $CH(CH_3)CH_2OP(S)(OEt)_2$ | $CF_3$ | Ph-4-Br |
| H | $CH(CH_3)CH_2OP(S)(OEt)_2$ | $CF_3$ | Ph-4-$CF_3$ |
| H | $CH(CH_3)CH_2OP(S)(OEt)_2$ | $CF_3$ | Ph-4-$OCF_3$ |
| H | $CH(CH_3)CH_2OP(S)(OEt)_2$ | $CF_3$ | Ph-4-$OCF_2Br$ |
| H | $CH(CH_3)CH_2OP(S)(OEt)_2$ | $CF_3$ | Ph-4-$OCF_2CHFCF_3$ |
| H | $CH(CH_3)CH_2OP(S)(OEt)_2$ | $CF_3$ | Ph-4-$OCF_2CHFOCF_3$ |

| | | | |
|---|---|---|---|
| H | $CH(CH_3)CH_2OP(S)(OEt)_2$ | $CF_3$ | Ph-4-$OCF_2CHFOCF_2CF_2CF_3$ |
| H | $CH(CH_3)CH_2OP(S)(OEt)_2$ | $CF_3$ | Ph-4-O(L-45g) |
| H | $CH(CH_3)CH_2OP(S)(OEt)_2$ | $CF_3$ | Ph-3,4-$Cl_2$ |
| H | $CH(CH_3)CH_2OP(S)(OEt)_2$ | $CF_3$ | Ph(-3-$OCF_2O$-4-) |
| H | $CH(CH_3)CH_2OP(S)(OEt)_2$ | $CF_3$ | Ph(-3-$OCF_2CF_2O$-4-) |
| H | $CH(CH_3)CH_2OP(S)(OEt)_2$ | $CF_3$ | (L-45c)$CF_3$ |
| H | $CH(CH_3)CH_2OP(S)(OEt)_2$ | $CF_3$ | L-45f |
| H | $CH(CH_3)CH_2OP(S)(OEt)_2$ | $CF_3$ | (L-46c)$CF_3$ |
| H | $CH(CH_3)CH_2OP(S)(OEt)_2$ | $CF_3$ | L-46d |
| H | $CH(CH_3)CH_2OPh$ | $CF_3$ | Ph-4-Br |
| H | $CH(CH_3)CH_2O$(Ph-4-Cl) | $CF_3$ | CH=CH(Ph-4-Cl) |
| H | $CH(CH_3)CH_2O$(Ph-4-Cl) | $CF_3$ | Ph-4-Cl |
| H | $CH(CH_3)CH_2O$(Ph-4-Cl) | $CF_3$ | Ph-4-Br |
| H | $CH(CH_3)CH_2O$(Ph-4-Cl) | $CF_3$ | Ph-4-$CF_3$ |
| H | $CH(CH_3)CH_2O$(Ph-4-Cl) | $CF_3$ | Ph-4-$OCF_3$ |
| H | $CH(CH_3)CH_2O$(Ph-4-Cl) | $CF_3$ | Ph-4-$OCF_2Br$ |
| H | $CH(CH_3)CH_2O$(Ph-4-Cl) | $CF_3$ | Ph-4-$OCF_2CHFCF_3$ |
| H | $CH(CH_3)CH_2O$(Ph-4-Cl) | $CF_3$ | Ph-4-$OCF_2CHFOCF_3$ |
| H | $CH(CH_3)CH_2O$(Ph-4-Cl) | $CF_3$ | Ph-4-$OCF_2CHFOCF_2CF_2CF_3$ |
| H | $CH(CH_3)CH_2O$(Ph-4-Cl) | $CF_3$ | Ph-4-O(L-45g) |
| H | $CH(CH_3)CH_2O$(Ph-4-Cl) | $CF_3$ | Ph-3,4-$Cl_2$ |
| H | $CH(CH_3)CH_2O$(Ph-4-Cl) | $CF_3$ | Ph(-3-$OCF_2O$-4-) |
| H | $CH(CH_3)CH_2O$(Ph-4-Cl) | $CF_3$ | Ph(-3-$OCF_2CF_2O$-4-) |
| H | $CH(CH_3)CH_2O$(Ph-4-Cl) | $CF_3$ | (L-45c)$CF_3$ |
| H | $CH(CH_3)CH_2O$(Ph-4-Cl) | $CF_3$ | L-45f |
| H | $CH(CH_3)CH_2O$(Ph-4-Cl) | $CF_3$ | (L-46c)$CF_3$ |
| H | $CH(CH_3)CH_2O$(Ph-4-Cl) | $CF_3$ | L-46d |
| H | $CH(CH_3)CH_2O$(Ph-3-$CF_3$) | $CF_3$ | Ph-4-$CF_3$ |
| H | $CH(Et)CH_2OH$ | $CF_3$ | Ph-4-$OCF_3$ |
| H | $CH(Et)CH_2OCH_3$ | $CF_3$ | (L-45c)$CF_3$ |
| H | $CH(Ph)CH_2OH$ | $CF_3$ | Ph-4-Br |
| H | $CH(Ph)CH_2OH$(R) | $CF_3$ | Ph-4-Br |
| H | CH(Ph-2-Cl)$CH_2OH$ | $CF_3$ | Ph-4-$CF_3$ |
| H | CH(Ph-4-Cl)$CH_2OH$ | $CF_3$ | Ph-4-$OCF_3$ |
| H | CH(Ph-4-Ph)$CH_2OH$ | $CF_3$ | (L-45c)$CF_3$ |
| H | $C(CH_3)_2CH_2OH$ | $CF_3$ | Ph-4-Br |
| H | $CH(CH_3)CH_2OCH_3$ | $CH_3$ | Ph-4-Br |
| H | $C(CH_3)_2CH_2OCH_3$ | $CH_3$ | Ph-4-$CF_3$ |
| H | $C(CH_3)_2CH_2OCH_3$ | $CH_3$ | Ph-4-$OCF_3$ |
| H | $C(CH_3)_2CH_2OCH_3$ | $CH_3$ | (L-45c)Cl |
| H | $C(CH_3)_2CH_2OCH_3$ | $CH_3$ | (L-45c)Br |

| | | | |
|---|---|---|---|
| H | $C(CH_3)_2CH_2OCH_3$ | $CH_3$ | (L-45c) $CF_3$ |
| H | $C(CH_3)_2CH_2OCH_3$ | Et | Ph-4-$CF_3$ |
| H | $C(CH_3)_2CH_2OCH_3$ | n-Pr | Ph-4-$OCF_3$ |
| H | $C(CH_3)_2CH_2OCH_3$ | i-Pr | (L-45c) $CF_3$ |
| H | $C(CH_3)_2CH_2OCH_3$ | c-Pr | Ph-4-Br |
| H | $C(CH_3)_2CH_2OCH_3$ | $CHF_2$ | Ph-4-$CF_3$ |
| H | $C(CH_3)_2CH_2OCH_3$ | $CF_3$ | $CF_2O$ (Ph-4-Cl) |
| H | $C(CH_3)_2CH_2OCH_3$ | $CF_3$ | CH=CH (Ph-4-Cl) |
| H | $C(CH_3)_2CH_2OCH_3$ | $CF_3$ | CH=CH (Ph-4-$OCF_3$) |
| H | $C(CH_3)_2CH_2OCH_3$ | $CF_3$ | CH=CH (Ph-3, 4-$Cl_2$) |
| H | $C(CH_3)_2CH_2OCH_3$ | $CF_3$ | Ph-4-F |
| H | $C(CH_3)_2CH_2OCH_3$ | $CF_3$ | Ph-4-Cl |
| H | $C(CH_3)_2CH_2OCH_3$ | $CF_3$ | Ph-4-Br |
| H | $C(CH_3)_2CH_2OCH_3$ | $CF_3$ | Ph-4-I |
| H | $C(CH_3)_2CH_2OCH_3$ | $CF_3$ | Ph-4-$CF_3$ |
| H | $C(CH_3)_2CH_2OCH_3$ | $CF_3$ | Ph-4-$OCHF_2$ |
| H | $C(CH_3)_2CH_2OCH_3$ | $CF_3$ | Ph-4-$OCF_3$ |
| H | $C(CH_3)_2CH_2OCH_3$ | $CF_3$ | Ph-4-$OCF_2Br$ |
| H | $C(CH_3)_2CH_2OCH_3$ | $CF_3$ | Ph-4-$OCF_2CHF_2$ |
| H | $C(CH_3)_2CH_2OCH_3$ | $CF_3$ | Ph-4-$OCF_2CHFCl$ |
| H | $C(CH_3)_2CH_2OCH_3$ | $CF_3$ | Ph-4-$OCF_2CHFCF_3$ |
| H | $C(CH_3)_2CH_2OCH_3$ | $CF_3$ | Ph-4-$OCF_2CHFOCF_3$ |
| H | $C(CH_3)_2CH_2OCH_3$ | $CF_3$ | Ph-4-$OCF_2CHFOCF_2CF_2CF_3$ |
| H | $C(CH_3)_2CH_2OCH_3$ | $CF_3$ | Ph-4-O (L-45g) |
| H | $C(CH_3)_2CH_2OCH_3$ | $CF_3$ | Ph-3, 4-$Cl_2$ |
| H | $C(CH_3)_2CH_2OCH_3$ | $CF_3$ | Ph (-3-$OCF_2O$-4-) |
| H | $C(CH_3)_2CH_2OCH_3$ | $CF_3$ | Ph (-3-$OCF_2CF_2O$-4-) |
| H | $C(CH_3)_2CH_2OCH_3$ | $CF_3$ | (L-45c) Cl |
| H | $C(CH_3)_2CH_2OCH_3$ | $CF_3$ | (L-45c) Br |
| H | $C(CH_3)_2CH_2OCH_3$ | $CF_3$ | (L-45c) $CF_3$ |
| H | $C(CH_3)_2CH_2OCH_3$ | $CF_3$ | L-45e |
| H | $C(CH_3)_2CH_2OCH_3$ | $CF_3$ | L-45f |
| H | $C(CH_3)_2CH_2OCH_3$ | $CF_3$ | (L-46c) Cl |
| H | $C(CH_3)_2CH_2OCH_3$ | $CF_3$ | (L-46c) Br |
| H | $C(CH_3)_2CH_2OCH_3$ | $CF_3$ | (L-46c) $CF_3$ |
| H | $C(CH_3)_2CH_2OCH_3$ | $CF_3$ | L-46d |
| H | $C(CH_3)_2CH_2OCH_3$ | $CF_2Cl$ | Ph-4-$OCF_3$ |
| H | $C(CH_3)_2CH_2OCH_3$ | $CF_2Br$ | (L-45c) $CF_3$ |
| H | $C(CH_3)_2CH_2OCH_3$ | $CF_2CF_3$ | Ph-4-Br |
| H | $C(CH_3)_2CH_2OCH_3$ | $CF_2CF_2CF_3$ | Ph-4-$CF_3$ |
| H | $C(CH_3)_2CH_2OCH_3$ | $CF_2OCH_3$ | Ph-4-$OCF_3$ |

| | | | |
|---|---|---|---|
| H | $C(CH_3)_2CH_2OCH_3$ | $CF_2SCH_3$ | (L-45c) $CF_3$ |
| H | $C(CH_3)_2CH_2OC(O)CH_3$ | $CF_3$ | Ph-4-Br |
| H | $C(CH_3)_2CH_2OC(O)CF_3$ | $CF_3$ | Ph-4-$CF_3$ |
| H | $C(CH_3)_2CH_2OC(O)NHCH_3$ | $CH_3$ | Ph-4-Br |
| H | $C(CH_3)_2CH_2OC(O)NHCH_3$ | $CH_3$ | Ph-4-$CF_3$ |
| H | $C(CH_3)_2CH_2OC(O)NHCH_3$ | $CH_3$ | Ph-4-$OCF_3$ |
| H | $C(CH_3)_2CH_2OC(O)NHCH_3$ | $CH_3$ | (L-45c) Cl |
| H | $C(CH_3)_2CH_2OC(O)NHCH_3$ | $CH_3$ | (L-45c) Br |
| H | $C(CH_3)_2CH_2OC(O)NHCH_3$ | $CH_3$ | (L-45c) $CF_3$ |
| H | $C(CH_3)_2CH_2OC(O)NHCH_3$ | Et | Ph-4-$OCF_3$ |
| H | $C(CH_3)_2CH_2OC(O)NHCH_3$ | n-Pr | (L-45c) $CF_3$ |
| H | $C(CH_3)_2CH_2OC(O)NHCH_3$ | i-Pr | Ph-4-Br |
| H | $C(CH_3)_2CH_2OC(O)NHCH_3$ | c-Pr | Ph-4-$CF_3$ |
| H | $C(CH_3)_2CH_2OC(O)NHCH_3$ | $CHF_2$ | Ph-4-$OCF_3$ |
| H | $C(CH_3)_2CH_2OC(O)NHCH_3$ | $CF_3$ | $CF_2O$(Ph-4-Cl) |
| H | $C(CH_3)_2CH_2OC(O)NHCH_3$ | $CF_3$ | CH=CH(Ph-4-Cl) |
| H | $C(CH_3)_2CH_2OC(O)NHCH_3$ | $CF_3$ | CH=CH(Ph-4-Br) |
| H | $C(CH_3)_2CH_2OC(O)NHCH_3$ | $CF_3$ | CH=CH(Ph-4-$CF_3$) |
| H | $C(CH_3)_2CH_2OC(O)NHCH_3$ | $CF_3$ | CH=CH(Ph-4-$OCF_3$) |
| H | $C(CH_3)_2CH_2OC(O)NHCH_3$ | $CF_3$ | CH=CH(Ph-3, 4-$Cl_2$) |
| H | $C(CH_3)_2CH_2OC(O)NHCH_3$ | $CF_3$ | Ph-4-F |
| H | $C(CH_3)_2CH_2OC(O)NHCH_3$ | $CF_3$ | Ph-4-Cl |
| H | $C(CH_3)_2CH_2OC(O)NHCH_3$ | $CF_3$ | Ph-4-Br |
| H | $C(CH_3)_2CH_2OC(O)NHCH_3$ | $CF_3$ | Ph-4-I |
| H | $C(CH_3)_2CH_2OC(O)NHCH_3$ | $CF_3$ | Ph-4-$CF_3$ |
| H | $C(CH_3)_2CH_2OC(O)NHCH_3$ | $CF_3$ | Ph-4-$OCHF_2$ |
| H | $C(CH_3)_2CH_2OC(O)NHCH_3$ | $CF_3$ | Ph-4-$OCF_3$ |
| H | $C(CH_3)_2CH_2OC(O)NHCH_3$ | $CF_3$ | Ph-4-$OCF_2Br$ |
| H | $C(CH_3)_2CH_2OC(O)NHCH_3$ | $CF_3$ | Ph-4-$OCF_2CHF_2$ |
| H | $C(CH_3)_2CH_2OC(O)NHCH_3$ | $CF_3$ | Ph-4-$OCF_2CHFCl$ |
| H | $C(CH_3)_2CH_2OC(O)NHCH_3$ | $CF_3$ | Ph-4-$OCF_2CHFCF_3$ |
| H | $C(CH_3)_2CH_2OC(O)NHCH_3$ | $CF_3$ | Ph-4-$OCF_2CHFOCF_3$ |
| H | $C(CH_3)_2CH_2OC(O)NHCH_3$ | $CF_3$ | Ph-4-$OCF_2CHFOCF_2CF_2CF_3$ |
| H | $C(CH_3)_2CH_2OC(O)NHCH_3$ | $CF_3$ | Ph-4-O(L-45g) |
| H | $C(CH_3)_2CH_2OC(O)NHCH_3$ | $CF_3$ | Ph-3, 4-$F_2$ |
| H | $C(CH_3)_2CH_2OC(O)NHCH_3$ | $CF_3$ | Ph-3-F-4-Cl |
| H | $C(CH_3)_2CH_2OC(O)NHCH_3$ | $CF_3$ | Ph-3, 4-$Cl_2$ |
| H | $C(CH_3)_2CH_2OC(O)NHCH_3$ | $CF_3$ | Ph(-3-$OCF_2O$-4-) |
| H | $C(CH_3)_2CH_2OC(O)NHCH_3$ | $CF_3$ | Ph(-3-$OCF_2CF_2O$-4-) |
| H | $C(CH_3)_2CH_2OC(O)NHCH_3$ | $CF_3$ | (L-45c) Cl |
| H | $C(CH_3)_2CH_2OC(O)NHCH_3$ | $CF_3$ | (L-45c) Br |

| | | | |
|---|---|---|---|
| H | $C(CH_3)_2CH_2OC(O)NHCH_3$ | $CF_3$ | (L-45c) $CF_3$ |
| H | $C(CH_3)_2CH_2OC(O)NHCH_3$ | $CF_3$ | L-45e |
| H | $C(CH_3)_2CH_2OC(O)NHCH_3$ | $CF_3$ | L-45f |
| H | $C(CH_3)_2CH_2OC(O)NHCH_3$ | $CF_3$ | (L-46c) Cl |
| H | $C(CH_3)_2CH_2OC(O)NHCH_3$ | $CF_3$ | (L-46c) Br |
| H | $C(CH_3)_2CH_2OC(O)NHCH_3$ | $CF_3$ | (L-46c) $CF_3$ |
| H | $C(CH_3)_2CH_2OC(O)NHCH_3$ | $CF_3$ | L-46d |
| H | $C(CH_3)_2CH_2OC(O)NHCH_3$ | $CF_2Cl$ | (L-45c) $CF_3$ |
| H | $C(CH_3)_2CH_2OC(O)NHCH_3$ | $CF_2Br$ | Ph-4-Br |
| H | $C(CH_3)_2CH_2OC(O)NHCH_3$ | $CF_2CF_3$ | Ph-4-$CF_3$ |
| H | $C(CH_3)_2CH_2OC(O)NHCH_3$ | $CF_2CF_2CF_3$ | Ph-4-$OCF_3$ |
| H | $C(CH_3)_2CH_2OC(O)NHCH_3$ | $CF_2OCH_3$ | (L-45c) $CF_3$ |
| H | $C(CH_3)_2CH_2OC(O)NHCH_3$ | $CF_2SCH_3$ | Ph-4-Br |
| H | $C(CH_3)_2CH_2OC(O)NHEt$ | $CF_3$ | $CF_2O$(Ph-4-Cl) |
| H | $C(CH_3)_2CH_2OC(O)NHEt$ | $CF_3$ | CH=CH(Ph-4-Cl) |
| H | $C(CH_3)_2CH_2OC(O)NHEt$ | $CF_3$ | CH=CH(Ph-4-Br) |
| H | $C(CH_3)_2CH_2OC(O)NHEt$ | $CF_3$ | CH=CH(Ph-4-$CF_3$) |
| H | $C(CH_3)_2CH_2OC(O)NHEt$ | $CF_3$ | CH=CH(Ph-4-$OCF_3$) |
| H | $C(CH_3)_2CH_2OC(O)NHEt$ | $CF_3$ | CH=CH(Ph-3, 4-$Cl_2$) |
| H | $C(CH_3)_2CH_2OC(O)NHEt$ | $CF_3$ | Ph-4-F |
| H | $C(CH_3)_2CH_2OC(O)NHEt$ | $CF_3$ | Ph-4-Cl |
| H | $C(CH_3)_2CH_2OC(O)NHEt$ | $CF_3$ | Ph-4-Br |
| H | $C(CH_3)_2CH_2OC(O)NHEt$ | $CF_3$ | Ph-4-$CF_3$ |
| H | $C(CH_3)_2CH_2OC(O)NHEt$ | $CF_3$ | Ph-4-$OCF_3$ |
| H | $C(CH_3)_2CH_2OC(O)NHEt$ | $CF_3$ | Ph-4-$OCF_2Br$ |
| H | $C(CH_3)_2CH_2OC(O)NHEt$ | $CF_3$ | Ph-4-$OCF_2CHF_2$ |
| H | $C(CH_3)_2CH_2OC(O)NHEt$ | $CF_3$ | Ph-4-$OCF_2CHFCF_3$ |
| H | $C(CH_3)_2CH_2OC(O)NHEt$ | $CF_3$ | Ph-4-$OCF_2CHFOCF_3$ |
| H | $C(CH_3)_2CH_2OC(O)NHEt$ | $CF_3$ | Ph-4-$OCF_2CHFOCF_2CF_2CF_3$ |
| H | $C(CH_3)_2CH_2OC(O)NHEt$ | $CF_3$ | Ph-4-O(L-45g) |
| H | $C(CH_3)_2CH_2OC(O)NHEt$ | $CF_3$ | Ph-3, 4-$F_2$ |
| H | $C(CH_3)_2CH_2OC(O)NHEt$ | $CF_3$ | Ph-3-F-4-Cl |
| H | $C(CH_3)_2CH_2OC(O)NHEt$ | $CF_3$ | Ph-3, 4-$Cl_2$ |
| H | $C(CH_3)_2CH_2OC(O)NHEt$ | $CF_3$ | Ph(-3-$OCF_2O$-4-) |
| H | $C(CH_3)_2CH_2OC(O)NHEt$ | $CF_3$ | Ph(-3-$OCF_2CF_2O$-4-) |
| H | $C(CH_3)_2CH_2OC(O)NHEt$ | $CF_3$ | (L-45c) $CF_3$ |
| H | $C(CH_3)_2CH_2OC(O)NHEt$ | $CF_3$ | L-45f |
| H | $C(CH_3)_2CH_2OC(O)NHEt$ | $CF_3$ | (L-46c) $CF_3$ |
| H | $C(CH_3)_2CH_2OC(O)NHEt$ | $CF_3$ | L-46d |
| H | $C(CH_3)_2CH_2OC(O)NHPr\text{-}n$ | $CF_3$ | CH=CH(Ph-4-Cl) |
| H | $C(CH_3)_2CH_2OC(O)NHPr\text{-}n$ | $CF_3$ | Ph-4-Cl |

| | | | |
|---|---|---|---|
| H | $C(CH_3)_2CH_2OC(O)NHPr-n$ | $CF_3$ | Ph-4-Br |
| H | $C(CH_3)_2CH_2OC(O)NHPr-n$ | $CF_3$ | $Ph-4-CF_3$ |
| H | $C(CH_3)_2CH_2OC(O)NHPr-n$ | $CF_3$ | $Ph-4-OCF_3$ |
| H | $C(CH_3)_2CH_2OC(O)NHPr-n$ | $CF_3$ | $Ph-4-OCF_2Br$ |
| H | $C(CH_3)_2CH_2OC(O)NHPr-n$ | $CF_3$ | $Ph-4-OCF_2CHFCF_3$ |
| H | $C(CH_3)_2CH_2OC(O)NHPr-n$ | $CF_3$ | $Ph-4-OCF_2CHFOCF_3$ |
| H | $C(CH_3)_2CH_2OC(O)NHPr-n$ | $CF_3$ | $Ph-4-OCF_2CHFOCF_2CF_2CF_3$ |
| H | $C(CH_3)_2CH_2OC(O)NHPr-n$ | $CF_3$ | Ph-4-O(L-45g) |
| H | $C(CH_3)_2CH_2OC(O)NHPr-n$ | $CF_3$ | $Ph-3,4-Cl_2$ |
| H | $C(CH_3)_2CH_2OC(O)NHPr-n$ | $CF_3$ | $Ph(-3-OCF_2O-4-)$ |
| H | $C(CH_3)_2CH_2OC(O)NHPr-n$ | $CF_3$ | $Ph(-3-OCF_2CF_2O-4-)$ |
| H | $C(CH_3)_2CH_2OC(O)NHPr-n$ | $CF_3$ | $(L-45c)CF_3$ |
| H | $C(CH_3)_2CH_2OC(O)NHPr-n$ | $CF_3$ | L-45f |
| H | $C(CH_3)_2CH_2OC(O)NHPr-n$ | $CF_3$ | $(L-46c)CF_3$ |
| H | $C(CH_3)_2CH_2OC(O)NHPr-n$ | $CF_3$ | L-46d |
| H | $C(CH_3)_2CH_2OC(O)NHPr-i$ | $CF_3$ | $Ph-4-CF_3$ |
| H | $C(CH_3)_2CH_2OC(O)NHPr-c$ | $CF_3$ | $Ph-4-OCF_3$ |
| H | $C(CH_3)_2CH_2OC(O)NHCH_2CF_3$ | $CF_3$ | $(L-45c)CF_3$ |
| H | $C(CH_3)_2CH_2OC(O)NHCH_2CH_2OCH_3$ | $CF_3$ | Ph-4-Br |
| H | $C(CH_3)_2CH_2OC(O)NHCH_2CH_2SCH_3$ | $CF_3$ | $Ph-4-CF_3$ |
| H | $C(CH_3)_2CH_2OC(O)NHCH_2CH_2CH_2SCH_3$ | $CF_3$ | $Ph-4-OCF_3$ |
| H | $C(CH_3)_2CH_2OC(O)NHCH_2CH=CH_2$ | $CF_3$ | $Ph-4-CF_3$ |
| H | $C(CH_3)_2CH_2OC(O)NHCH_2Ph$ | $CF_3$ | CH=CH(Ph-4-Cl) |
| H | $C(CH_3)_2CH_2OC(O)NHCH_2Ph$ | $CF_3$ | Ph-4-Cl |
| H | $C(CH_3)_2CH_2OC(O)NHCH_2Ph$ | $CF_3$ | Ph-4-Br |
| H | $C(CH_3)_2CH_2OC(O)NHCH_2Ph$ | $CF_3$ | $Ph-4-CF_3$ |
| H | $C(CH_3)_2CH_2OC(O)NHCH_2Ph$ | $CF_3$ | $Ph-4-OCF_3$ |
| H | $C(CH_3)_2CH_2OC(O)NHCH_2Ph$ | $CF_3$ | $Ph-4-OCF_2Br$ |
| H | $C(CH_3)_2CH_2OC(O)NHCH_2Ph$ | $CF_3$ | $Ph-4-OCF_2CHFCF_3$ |
| H | $C(CH_3)_2CH_2OC(O)NHCH_2Ph$ | $CF_3$ | $Ph-4-OCF_2CHFOCF_3$ |
| H | $C(CH_3)_2CH_2OC(O)NHCH_2Ph$ | $CF_3$ | $Ph-4-OCF_2CHFOCF_2CF_2CF_3$ |
| H | $C(CH_3)_2CH_2OC(O)NHCH_2Ph$ | $CF_3$ | Ph-4-O(L-45g) |
| H | $C(CH_3)_2CH_2OC(O)NHCH_2Ph$ | $CF_3$ | $Ph-3,4-Cl_2$ |
| H | $C(CH_3)_2CH_2OC(O)NHCH_2Ph$ | $CF_3$ | $Ph(-3-OCF_2O-4-)$ |
| H | $C(CH_3)_2CH_2OC(O)NHCH_2Ph$ | $CF_3$ | $Ph(-3-OCF_2CF_2O-4-)$ |
| H | $C(CH_3)_2CH_2OC(O)NHCH_2Ph$ | $CF_3$ | $(L-45c)CF_3$ |
| H | $C(CH_3)_2CH_2OC(O)NHCH_2Ph$ | $CF_3$ | L-45f |
| H | $C(CH_3)_2CH_2OC(O)NHCH_2Ph$ | $CF_3$ | $(L-46c)CF_3$ |
| H | $C(CH_3)_2CH_2OC(O)NHCH_2Ph$ | $CF_3$ | L-46d |
| H | $C(CH_3)_2CH_2OC(O)N(CH_3)_2$ | $CF_3$ | CH=CH(Ph-4-Cl) |
| H | $C(CH_3)_2CH_2OC(O)N(CH_3)_2$ | $CF_3$ | Ph-4-Cl |

| | | | |
|---|---|---|---|
| H | $C(CH_3)_2CH_2OC(O)N(CH_3)_2$ | $CF_3$ | Ph-4-Br |
| H | $C(CH_3)_2CH_2OC(O)N(CH_3)_2$ | $CF_3$ | Ph-4-$CF_3$ |
| H | $C(CH_3)_2CH_2OC(O)N(CH_3)_2$ | $CF_3$ | Ph-4-$OCF_3$ |
| H | $C(CH_3)_2CH_2OC(O)N(CH_3)_2$ | $CF_3$ | Ph-4-$OCF_2Br$ |
| H | $C(CH_3)_2CH_2OC(O)N(CH_3)_2$ | $CF_3$ | Ph-4-$OCF_2CHFCF_3$ |
| H | $C(CH_3)_2CH_2OC(O)N(CH_3)_2$ | $CF_3$ | Ph-4-$OCF_2CHFOCF_3$ |
| H | $C(CH_3)_2CH_2OC(O)N(CH_3)_2$ | $CF_3$ | Ph-4-$OCF_2CHFOCF_2CF_2CF_3$ |
| H | $C(CH_3)_2CH_2OC(O)N(CH_3)_2$ | $CF_3$ | Ph-4-O(L-45g) |
| H | $C(CH_3)_2CH_2OC(O)N(CH_3)_2$ | $CF_3$ | Ph-3, 4-$Cl_2$ |
| H | $C(CH_3)_2CH_2OC(O)N(CH_3)_2$ | $CF_3$ | Ph(-3-$OCF_2O$-4-) |
| H | $C(CH_3)_2CH_2OC(O)N(CH_3)_2$ | $CF_3$ | Ph(-3-$OCF_2CF_2O$-4-) |
| H | $C(CH_3)_2CH_2OC(O)N(CH_3)_2$ | $CF_3$ | (L-45c)$CF_3$ |
| H | $C(CH_3)_2CH_2OC(O)N(CH_3)_2$ | $CF_3$ | L-45f |
| H | $C(CH_3)_2CH_2OC(O)N(CH_3)_2$ | $CF_3$ | (L-46c)$CF_3$ |
| H | $C(CH_3)_2CH_2OC(O)N(CH_3)_2$ | $CF_3$ | L-46d |
| H | $C(CH_3)_2CH_2OP(S)(OCH_3)_2$ | $CF_3$ | Ph-4-$OCF_3$ |
| H | $CH_2CH_2CH_2OH$ | $CF_3$ | (L-45c)$CF_3$ |
| H | $CH_2CH_2CH_2OCH_3$ | $CF_3$ | Ph-4-Br |
| H | $CH(CH_3)CH_2CH_2OCH_3$ | $CF_3$ | Ph-4-$CF_3$ |
| H | $CH(CH_3)CH_2CH_2OEt$ | $CF_3$ | CH=CH(Ph-4-Cl) |
| H | $CH(CH_3)CH_2CH_2OEt$ | $CF_3$ | Ph-4-Cl |
| H | $CH(CH_3)CH_2CH_2OEt$ | $CF_3$ | Ph-4-Br |
| H | $CH(CH_3)CH_2CH_2OEt$ | $CF_3$ | Ph-4-$CF_3$ |
| H | $CH(CH_3)CH_2CH_2OEt$ | $CF_3$ | Ph-4-$OCF_3$ |
| H | $CH(CH_3)CH_2CH_2OEt$ | $CF_3$ | Ph-4-$OCF_2Br$ |
| H | $CH(CH_3)CH_2CH_2OEt$ | $CF_3$ | Ph-4-$OCF_2CHFCF_3$ |
| H | $CH(CH_3)CH_2CH_2OEt$ | $CF_3$ | Ph-4-$OCF_2CHFOCF_3$ |
| H | $CH(CH_3)CH_2CH_2OEt$ | $CF_3$ | Ph-4-$OCF_2CHFOCF_2CF_2CF_3$ |
| H | $CH(CH_3)CH_2CH_2OEt$ | $CF_3$ | Ph-4-O(L-45g) |
| H | $CH(CH_3)CH_2CH_2OEt$ | $CF_3$ | Ph-3, 4-$Cl_2$ |
| H | $CH(CH_3)CH_2CH_2OEt$ | $CF_3$ | Ph(-3-$OCF_2O$-4-) |
| H | $CH(CH_3)CH_2CH_2OEt$ | $CF_3$ | Ph(-3-$OCF_2CF_2O$-4-) |
| H | $CH(CH_3)CH_2CH_2OEt$ | $CF_3$ | (L-45c)$CF_3$ |
| H | $CH(CH_3)CH_2CH_2OEt$ | $CF_3$ | L-45f |
| H | $CH(CH_3)CH_2CH_2OEt$ | $CF_3$ | (L-46c)$CF_3$ |
| H | $CH(CH_3)CH_2CH_2OEt$ | $CF_3$ | L-46d |
| H | $CH(CH_3)CH_2CH_2OPr$-n | $CF_3$ | Ph-4-$OCF_3$ |
| H | $CH(CH_3)CH_2CH_2OBu$-i | $CF_3$ | (L-45c)$CF_3$ |
| H | $CH(CH_3)CH_2CH_2OCH_2CF_3$ | $CF_3$ | Ph-4-Br |
| H | $CH(CH_3)CH_2CH_2OCH_2CH_2OCH_3$ | $CF_3$ | Ph-4-$CF_3$ |
| H | $CH(CH_3)CH_2CH_2OC(O)NHEt$ | $CF_3$ | Ph-4-$OCF_3$ |

| | | | |
|---|---|---|---|
| H | $CH_2CH_2CH_2CH_2OC(O)NHEt$ | $CF_3$ | (L-45c) $CF_3$ |
| H | $CH_2CH_2CH_2CH_2OC(O)NHPr$-i | $CF_3$ | Ph-4-Br |
| H | $CH_2CH_2CH_2CH_2CH_2OC(O)NHEt$ | $CF_3$ | Ph-4-$CF_3$ |
| H | T-10 | $CF_3$ | Ph-4-$OCF_3$ |
| H | M-4a | $CF_3$ | (L-45c) $CF_3$ |
| H | M-5a | $CF_3$ | Ph-4-Br |
| H | $CH_2$ (M-7a) | $CF_3$ | Ph-4-$CF_3$ |
| H | $CH_2$ (M-16a) | $CF_3$ | Ph-4-$OCF_3$ |
| $-CH_2CH_2OCH_2CH_2-$ | | $CF_3$ | (L-45c) $CF_3$ |
| H | $CH_2SCH_3$ | $CF_3$ | Ph-4-Br |
| H | $CH_2CH_2SCH_3$ | $CF_3$ | Ph-4-$CF_3$ |
| H | $CH_2CH_2SEt$ | $CF_3$ | Ph-4-$OCF_3$ |
| H | $CH_2CH_2SPr$-i | $CF_3$ | (L-45c) $CF_3$ |
| H | $CH_2CH(CH_3)SCH_3$ | $CF_3$ | Ph-4-Br |
| H | $CH_2CH(CH_3)SO_2CH_3$ | $CF_3$ | Ph-4-$CF_3$ |
| H | $CH_2CH(CH_3)SEt$ | $CF_3$ | Ph-4-$OCF_3$ |
| H | $CH_2CH(CH_3)SO_2Et$ | $CF_3$ | (L-45c) $CF_3$ |
| H | $CH(CH_3)CH_2SH$ | $CF_3$ | Ph-4-Br |
| H | $CH(CH_3)CH_2SCH_3$ | $CH_3$ | $CF_2O$(Ph-4-Cl) |
| H | $CH(CH_3)CH_2SCH_3$ | $CH_3$ | CH=CH(Ph-4-Cl) |
| H | $CH(CH_3)CH_2SCH_3$ | $CH_3$ | CH=CH(Ph-4-$OCF_3$) |
| H | $CH(CH_3)CH_2SCH_3$ | $CH_3$ | CH=CH(Ph-3, 4-$Cl_2$) |
| H | $CH(CH_3)CH_2SCH_3$ | $CH_3$ | Ph-4-F |
| H | $CH(CH_3)CH_2SCH_3$ | $CH_3$ | Ph-4-Cl |
| H | $CH(CH_3)CH_2SCH_3$ | $CH_3$ | Ph-4-Br |
| H | $CH(CH_3)CH_2SCH_3$ | $CH_3$ | Ph-4-I |
| H | $CH(CH_3)CH_2SCH_3$ | $CH_3$ | Ph-4-$CF_3$ |
| H | $CH(CH_3)CH_2SCH_3$ | $CH_3$ | Ph-4-$OCHF_2$ |
| H | $CH(CH_3)CH_2SCH_3$ | $CH_3$ | Ph-4-$OCF_3$ |
| H | $CH(CH_3)CH_2SCH_3$ | $CH_3$ | Ph-4-$OCF_2Br$ |
| H | $CH(CH_3)CH_2SCH_3$ | $CH_3$ | Ph-4-$OCF_2CHF_2$ |
| H | $CH(CH_3)CH_2SCH_3$ | $CH_3$ | Ph-4-$OCF_2CHFCl$ |
| H | $CH(CH_3)CH_2SCH_3$ | $CH_3$ | Ph-4-$OCF_2CHFCF_3$ |
| H | $CH(CH_3)CH_2SCH_3$ | $CH_3$ | Ph-4-$OCF_2CHFOCF_3$ |
| H | $CH(CH_3)CH_2SCH_3$ | $CH_3$ | Ph-4-$OCF_2CHFOCF_2CF_2CF_3$ |
| H | $CH(CH_3)CH_2SCH_3$ | $CH_3$ | Ph-4-O(L-45g) |
| H | $CH(CH_3)CH_2SCH_3$ | $CH_3$ | Ph-3, 4-$Cl_2$ |
| H | $CH(CH_3)CH_2SCH_3$ | $CH_3$ | Ph(-3-$OCF_2O$-4-) |
| H | $CH(CH_3)CH_2SCH_3$ | $CH_3$ | Ph(-3-$OCF_2CF_2O$-4-) |
| H | $CH(CH_3)CH_2SCH_3$ | $CH_3$ | (L-45c) Cl |
| H | $CH(CH_3)CH_2SCH_3$ | $CH_3$ | (L-45c) Br |

| | | | |
|---|---|---|---|
| H | $CH(CH_3)CH_2SCH_3$ | $CH_3$ | (L-45c) $CF_3$ |
| H | $CH(CH_3)CH_2SCH_3$ | $CH_3$ | L-45e |
| H | $CH(CH_3)CH_2SCH_3$ | $CH_3$ | L-45f |
| H | $CH(CH_3)CH_2SCH_3$ | $CH_3$ | (L-46c) Cl |
| H | $CH(CH_3)CH_2SCH_3$ | $CH_3$ | (L-46c) Br |
| H | $CH(CH_3)CH_2SCH_3$ | $CH_3$ | (L-46c) $CF_3$ |
| H | $CH(CH_3)CH_2SCH_3$ | $CH_3$ | L-46d |
| H | $CH(CH_3)CH_2SCH_3$ | Et | Ph-4-Cl |
| H | $CH(CH_3)CH_2SCH_3$ | Et | Ph-4-Br |
| H | $CH(CH_3)CH_2SCH_3$ | Et | Ph-4-$CF_3$ |
| H | $CH(CH_3)CH_2SCH_3$ | Et | Ph-4-$OCF_3$ |
| H | $CH(CH_3)CH_2SCH_3$ | Et | Ph-4-$OCF_2Br$ |
| H | $CH(CH_3)CH_2SCH_3$ | Et | Ph-4-$OCF_2CHFCF_3$ |
| H | $CH(CH_3)CH_2SCH_3$ | Et | Ph-4-$OCF_2CHFOCF_3$ |
| H | $CH(CH_3)CH_2SCH_3$ | Et | Ph-4-$OCF_2CHFOCF_2CF_2CF_3$ |
| H | $CH(CH_3)CH_2SCH_3$ | Et | Ph-4-O(L-45g) |
| H | $CH(CH_3)CH_2SCH_3$ | Et | Ph-3, 4-$Cl_2$ |
| H | $CH(CH_3)CH_2SCH_3$ | Et | Ph(-3-$OCF_2O$-4-) |
| H | $CH(CH_3)CH_2SCH_3$ | Et | Ph(-3-$OCF_2CF_2O$-4-) |
| H | $CH(CH_3)CH_2SCH_3$ | Et | (L-45c) $CF_3$ |
| H | $CH(CH_3)CH_2SCH_3$ | n-Pr | Ph-4-Br |
| H | $CH(CH_3)CH_2SCH_3$ | n-Pr | Ph-4-$CF_3$ |
| H | $CH(CH_3)CH_2SCH_3$ | n-Pr | Ph-4-$OCF_3$ |
| H | $CH(CH_3)CH_2SCH_3$ | n-Pr | (L-45c) $CF_3$ |
| H | $CH(CH_3)CH_2SCH_3$ | i-Pr | Ph-4-Br |
| H | $CH(CH_3)CH_2SCH_3$ | i-Pr | Ph-4-$CF_3$ |
| H | $CH(CH_3)CH_2SCH_3$ | i-Pr | Ph-4-$OCF_3$ |
| H | $CH(CH_3)CH_2SCH_3$ | i-Pr | (L-45c) $CF_3$ |
| H | $CH(CH_3)CH_2SCH_3$ | c-Pr | Ph-4-Br |
| H | $CH(CH_3)CH_2SCH_3$ | c-Pr | Ph-4-$CF_3$ |
| H | $CH(CH_3)CH_2SCH_3$ | c-Pr | Ph-4-$OCF_3$ |
| H | $CH(CH_3)CH_2SCH_3$ | c-Pr | (L-45c) $CF_3$ |
| H | $CH(CH_3)CH_2SCH_3$ | n-Bu | Ph-4-$CF_3$ |
| H | $CH(CH_3)CH_2SCH_3$ | $CH_2F$ | Ph-4-$OCF_3$ |
| H | $CH(CH_3)CH_2SCH_3$ | $CH_2Cl$ | (L-45c) $CF_3$ |
| H | $CH(CH_3)CH_2SCH_3$ | $CH_2Br$ | Ph-4-Br |
| H | $CH(CH_3)CH_2SCH_3$ | $CHF_2$ | Ph-4-Cl |
| H | $CH(CH_3)CH_2SCH_3$ | $CHF_2$ | Ph-4-Br |
| H | $CH(CH_3)CH_2SCH_3$ | $CHF_2$ | Ph-4-$CF_3$ |
| H | $CH(CH_3)CH_2SCH_3$ | $CHF_2$ | Ph-4-$OCF_3$ |
| H | $CH(CH_3)CH_2SCH_3$ | $CHF_2$ | Ph-4-$OCF_2Br$ |

| | | | |
|---|---|---|---|
| H | $CH(CH_3)CH_2SCH_3$ | $CHF_2$ | Ph-4-$OCF_2CHFCF_3$ |
| H | $CH(CH_3)CH_2SCH_3$ | $CHF_2$ | Ph-4-$OCF_2CHFOCF_3$ |
| H | $CH(CH_3)CH_2SCH_3$ | $CHF_2$ | Ph-4-$OCF_2CHFOCF_2CF_2CF_3$ |
| H | $CH(CH_3)CH_2SCH_3$ | $CHF_2$ | Ph-4-O(L-45g) |
| H | $CH(CH_3)CH_2SCH_3$ | $CHF_2$ | Ph-3,4-$Cl_2$ |
| H | $CH(CH_3)CH_2SCH_3$ | $CHF_2$ | Ph(-3-$OCF_2O$-4-) |
| H | $CH(CH_3)CH_2SCH_3$ | $CHF_2$ | Ph(-3-$OCF_2CF_2O$-4-) |
| H | $CH(CH_3)CH_2SCH_3$ | $CHF_2$ | (L-45c)$CF_3$ |
| H | $CH(CH_3)CH_2SCH_3$ | $CHF_2$ | L-45f |
| H | $CH(CH_3)CH_2SCH_3$ | $CHF_2$ | (L-46c)$CF_3$ |
| H | $CH(CH_3)CH_2SCH_3$ | $CHF_2$ | L-46d |
| H | $CH(CH_3)CH_2SCH_3$ | CHFCl | Ph-4-$CF_3$ |
| H | $CH(CH_3)CH_2SCH_3$ | CHFBr | Ph-4-$OCF_3$ |
| H | $CH(CH_3)CH_2SCH_3$ | $CF_3$ | c-Pr |
| H | $CH(CH_3)CH_2SCH_3$ | $CF_3$ | c-Bu |
| H | $CH(CH_3)CH_2SCH_3$ | $CF_3$ | c-Pen |
| H | $CH(CH_3)CH_2SCH_3$ | $CF_3$ | c-Hex |
| H | $CH(CH_3)CH_2SCH_3$ | $CF_3$ | T-1 |
| H | $CH(CH_3)CH_2SCH_3$ | $CF_3$ | T-2 |
| H | $CH(CH_3)CH_2SCH_3$ | $CF_3$ | T-3 |
| H | $CH(CH_3)CH_2SCH_3$ | $CF_3$ | T-4 |
| H | $CH(CH_3)CH_2SCH_3$ | $CF_3$ | T-5 |
| H | $CH(CH_3)CH_2SCH_3$ | $CF_3$ | $CH_2OCH_3$ |
| H | $CH(CH_3)CH_2SCH_3$ | $CF_3$ | $CH_2OEt$ |
| H | $CH(CH_3)CH_2SCH_3$ | $CF_3$ | $CH_2OPr$-n |
| H | $CH(CH_3)CH_2SCH_3$ | $CF_3$ | $CH_2OPr$-i |
| H | $CH(CH_3)CH_2SCH_3$ | $CF_3$ | $CH_2OBu$-n |
| H | $CH(CH_3)CH_2SCH_3$ | $CF_3$ | $CH_2OC(O)$(Ph-2-Cl) |
| H | $CH(CH_3)CH_2SCH_3$ | $CF_3$ | $CH_2OC(O)$(Ph-3-Cl) |
| H | $CH(CH_3)CH_2SCH_3$ | $CF_3$ | $CH_2OC(O)$(Ph-4-Cl) |
| H | $CH(CH_3)CH_2SCH_3$ | $CF_3$ | $CH_2OPh$ |
| H | $CH(CH_3)CH_2SCH_3$ | $CF_3$ | $CH_2O$(Ph-2-F) |
| H | $CH(CH_3)CH_2SCH_3$ | $CF_3$ | $CH_2O$(Ph-3-F) |
| H | $CH(CH_3)CH_2SCH_3$ | $CF_3$ | $CH_2O$(Ph-4-F) |
| H | $CH(CH_3)CH_2SCH_3$ | $CF_3$ | $CH_2O$(Ph-2-Cl) |
| H | $CH(CH_3)CH_2SCH_3$ | $CF_3$ | $CH_2O$(Ph-3-Cl) |
| H | $CH(CH_3)CH_2SCH_3$ | $CF_3$ | $CH_2O$(Ph-4-Cl) |
| H | $CH(CH_3)CH_2SCH_3$ | $CF_3$ | $CH_2O$(Ph-2-Br) |
| H | $CH(CH_3)CH_2SCH_3$ | $CF_3$ | $CH_2O$(Ph-3-Br) |
| H | $CH(CH_3)CH_2SCH_3$ | $CF_3$ | $CH_2O$(Ph-4-Br) |
| H | $CH(CH_3)CH_2SCH_3$ | $CF_3$ | $CH_2O$(Ph-2-$CF_3$) |

| | | | |
|---|---|---|---|
| H | $CH(CH_3)CH_2SCH_3$ | $CF_3$ | $CH_2O(Ph-3-CF_3)$ |
| H | $CH(CH_3)CH_2SCH_3$ | $CF_3$ | $CH_2O(Ph-4-CF_3)$ |
| H | $CH(CH_3)CH_2SCH_3$ | $CF_3$ | $CH_2O(Ph-2-OCF_3)$ |
| H | $CH(CH_3)CH_2SCH_3$ | $CF_3$ | $CH_2O(Ph-3-OCF_3)$ |
| H | $CH(CH_3)CH_2SCH_3$ | $CF_3$ | $CH_2O(Ph-4-OCF_3)$ |
| H | $CH(CH_3)CH_2SCH_3$ | $CF_3$ | $CF_2OPh$ |
| H | $CH(CH_3)CH_2SCH_3$ | $CF_3$ | $CF_2O(Ph-2-F)$ |
| H | $CH(CH_3)CH_2SCH_3$ | $CF_3$ | $CF_2O(Ph-3-F)$ |
| H | $CH(CH_3)CH_2SCH_3$ | $CF_3$ | $CF_2O(Ph-4-F)$ |
| H | $CH(CH_3)CH_2SCH_3$ | $CF_3$ | $CF_2O(Ph-2-Cl)$ |
| H | $CH(CH_3)CH_2SCH_3$ | $CF_3$ | $CF_2O(Ph-3-Cl)$ |
| H | $CH(CH_3)CH_2SCH_3$ | $CF_3$ | $CF_2O(Ph-4-Cl)$ |
| H | $CH(CH_3)CH_2SCH_3$ | $CF_3$ | $CF_2O(Ph-2-Br)$ |
| H | $CH(CH_3)CH_2SCH_3$ | $CF_3$ | $CF_2O(Ph-3-Br)$ |
| H | $CH(CH_3)CH_2SCH_3$ | $CF_3$ | $CF_2O(Ph-4-Br)$ |
| H | $CH(CH_3)CH_2SCH_3$ | $CF_3$ | $CF_2O(Ph-2-CF_3)$ |
| H | $CH(CH_3)CH_2SCH_3$ | $CF_3$ | $CF_2O(Ph-3-CF_3)$ |
| H | $CH(CH_3)CH_2SCH_3$ | $CF_3$ | $CF_2O(Ph-4-CF_3)$ |
| H | $CH(CH_3)CH_2SCH_3$ | $CF_3$ | $CF_2O(Ph-2-OCF_3)$ |
| H | $CH(CH_3)CH_2SCH_3$ | $CF_3$ | $CF_2O(Ph-3-OCF_3)$ |
| H | $CH(CH_3)CH_2SCH_3$ | $CF_3$ | $CF_2O(Ph-4-OCF_3)$ |
| H | $CH(CH_3)CH_2SCH_3$ | $CF_3$ | $CH_2S(Ph-2-Cl)$ |
| H | $CH(CH_3)CH_2SCH_3$ | $CF_3$ | $CH_2S(Ph-3-Cl)$ |
| H | $CH(CH_3)CH_2SCH_3$ | $CF_3$ | $CH_2S(Ph-4-Cl)$ |
| H | $CH(CH_3)CH_2SCH_3$ | $CF_3$ | $CH_2SO_2(Ph-2-Cl)$ |
| H | $CH(CH_3)CH_2SCH_3$ | $CF_3$ | $CH_2SO_2(Ph-3-Cl)$ |
| H | $CH(CH_3)CH_2SCH_3$ | $CF_3$ | $CH_2SO_2(Ph-4-Cl)$ |
| H | $CH(CH_3)CH_2SCH_3$ | $CF_3$ | $CH_2N(CH_3)_2$ |
| H | $CH(CH_3)CH_2SCH_3$ | $CF_3$ | $CH_2NH(Ph-2-Cl)$ |
| H | $CH(CH_3)CH_2SCH_3$ | $CF_3$ | $CH_2NH(Ph-3-Cl)$ |
| H | $CH(CH_3)CH_2SCH_3$ | $CF_3$ | $CH_2NH(Ph-4-Cl)$ |
| H | $CH(CH_3)CH_2SCH_3$ | $CF_3$ | $CF_2C(O)OEt$ |
| H | $CH(CH_3)CH_2SCH_3$ | $CF_3$ | $CH_2(L-5a)$ |
| H | $CH(CH_3)CH_2SCH_3$ | $CF_3$ | $CH_2(L-14a)$ |
| H | $CH(CH_3)CH_2SCH_3$ | $CF_3$ | $CH_2(L-24a)$ |
| H | $CH(CH_3)CH_2SCH_3$ | $CF_3$ | $CH_2(L-36a)$ |
| H | $CH(CH_3)CH_2SCH_3$ | $CF_3$ | $CH_2CH_2Ph$ |
| H | $CH(CH_3)CH_2SCH_3$ | $CF_3$ | $CH_2CH_2(Ph-2-Cl)$ |
| H | $CH(CH_3)CH_2SCH_3$ | $CF_3$ | $CH_2CH_2(Ph-3-Cl)$ |
| H | $CH(CH_3)CH_2SCH_3$ | $CF_3$ | $CH_2CH_2(Ph-4-Cl)$ |
| H | $CH(CH_3)CH_2SCH_3$ | $CF_3$ | T-22 |

| | | | |
|---|---|---|---|
| H | $CH(CH_3)CH_2SCH_3$ | $CF_3$ | T-23 |
| H | $CH(CH_3)CH_2SCH_3$ | $CF_3$ | T-24 |
| H | $CH(CH_3)CH_2SCH_3$ | $CF_3$ | C(O)OEt |
| H | $CH(CH_3)CH_2SCH_3$ | $CF_3$ | C(O)OBu-t |
| H | $CH(CH_3)CH_2SCH_3$ | $CF_3$ | $C(O)OCH_2CF_3$ |
| H | $CH(CH_3)CH_2SCH_3$ | $CF_3$ | CH=CH(Ph-2-F) |
| H | $CH(CH_3)CH_2SCH_3$ | $CF_3$ | CH=CH(Ph-3-F) |
| H | $CH(CH_3)CH_2SCH_3$ | $CF_3$ | CH=CH(Ph-4-F) |
| H | $CH(CH_3)CH_2SCH_3$ | $CF_3$ | CH=CH(Ph-2-Cl) |
| H | $CH(CH_3)CH_2SCH_3$ | $CF_3$ | CH=CH(Ph-3-Cl) |
| H | $CH(CH_3)CH_2SCH_3$ | $CF_3$ | CH=CH(Ph-4-Cl) |
| H | $CH(CH_3)CH_2SCH_3$ | $CF_3$ | CH=CH(Ph-2-Br) |
| H | $CH(CH_3)CH_2SCH_3$ | $CF_3$ | CH=CH(Ph-3-Br) |
| H | $CH(CH_3)CH_2SCH_3$ | $CF_3$ | CH=CH(Ph-4-Br) |
| H | $CH(CH_3)CH_2SCH_3$ | $CF_3$ | $CH=CH(Ph-2-CF_3)$ |
| H | $CH(CH_3)CH_2SCH_3$ | $CF_3$ | $CH=CH(Ph-3-CF_3)$ |
| H | $CH(CH_3)CH_2SCH_3$ | $CF_3$ | $CH=CH(Ph-4-CF_3)$ |
| H | $CH(CH_3)CH_2SCH_3$ | $CF_3$ | $CH=CH(Ph-2-OCF_3)$ |
| H | $CH(CH_3)CH_2SCH_3$ | $CF_3$ | $CH=CH(Ph-3-OCF_3)$ |
| H | $CH(CH_3)CH_2SCH_3$ | $CF_3$ | $CH=CH(Ph-4-OCF_3)$ |
| H | $CH(CH_3)CH_2SCH_3$ | $CF_3$ | $CH=CH(Ph-2-SCH_3)$ |
| H | $CH(CH_3)CH_2SCH_3$ | $CF_3$ | $CH=CH(Ph-3-SCH_3)$ |
| H | $CH(CH_3)CH_2SCH_3$ | $CF_3$ | $CH=CH(Ph-4-SCH_3)$ |
| H | $CH(CH_3)CH_2SCH_3$ | $CF_3$ | $CH=CH(Ph-2-SO_2CH_3)$ |
| H | $CH(CH_3)CH_2SCH_3$ | $CF_3$ | $CH=CH(Ph-3-SO_2CH_3)$ |
| H | $CH(CH_3)CH_2SCH_3$ | $CF_3$ | $CH=CH(Ph-4-SO_2CH_3)$ |
| H | $CH(CH_3)CH_2SCH_3$ | $CF_3$ | $CH=CH(Ph-3,4-F_2)$ |
| H | $CH(CH_3)CH_2SCH_3$ | $CF_3$ | CH=CH(Ph-3-F-4-Cl) |
| H | $CH(CH_3)CH_2SCH_3$ | $CF_3$ | $CH=CH(Ph-3,4-Cl_2)$ |
| H | $CH(CH_3)CH_2SCH_3$ | $CF_3$ | $CH=CH(Ph-3,4-Br_2)$ |
| H | $CH(CH_3)CH_2SCH_3$ | $CF_3$ | $CH=CH(Ph-3-F-4-CF_3)$ |
| H | $CH(CH_3)CH_2SCH_3$ | $CF_3$ | $CH=CH(Ph-3-Cl-4-OCF_3)$ |
| H | $CH(CH_3)CH_2SCH_3$ | $CF_3$ | $CH=CH[Ph(-3-OCF_2O-4-)]$ |
| H | $CH(CH_3)CH_2SCH_3$ | $CF_3$ | Ph |
| H | $CH(CH_3)CH_2SCH_3$ | $CF_3$ | Ph-2-F |
| H | $CH(CH_3)CH_2SCH_3$ | $CF_3$ | Ph-3-F |
| H | $CH(CH_3)CH_2SCH_3$ | $CF_3$ | Ph-4-F |
| H | $CH(CH_3)CH_2SCH_3$ | $CF_3$ | Ph-2-Cl |
| H | $CH(CH_3)CH_2SCH_3$ | $CF_3$ | Ph-3-Cl |
| H | $CH(CH_3)CH_2SCH_3$ | $CF_3$ | Ph-4-Cl |
| H | $CH(CH_3)CH_2SCH_3$ | $CF_3$ | Ph-2-Br |

| | | | |
|---|---|---|---|
| H | $CH(CH_3)CH_2SCH_3$ | $CF_3$ | Ph-3-Br |
| H | $CH(CH_3)CH_2SCH_3$ | $CF_3$ | Ph-4-Br |
| H | $CH(CH_3)CH_2SCH_3$ | $CF_3$ | Ph-4-I |
| H | $CH(CH_3)CH_2SCH_3$ | $CF_3$ | Ph-4-Bu-t |
| H | $CH(CH_3)CH_2SCH_3$ | $CF_3$ | Ph-2-$CF_3$ |
| H | $CH(CH_3)CH_2SCH_3$ | $CF_3$ | Ph-3-$CF_3$ |
| H | $CH(CH_3)CH_2SCH_3$ | $CF_3$ | Ph-4-$CF_3$ |
| H | $CH(CH_3)CH_2SCH_3$ | $CF_3$ | Ph-4-$OCH_3$ |
| H | $CH(CH_3)CH_2SCH_3$ | $CF_3$ | Ph-4-$OCHF_2$ |
| H | $CH(CH_3)CH_2SCH_3$ | $CF_3$ | Ph-2-$OCF_3$ |
| H | $CH(CH_3)CH_2SCH_3$ | $CF_3$ | Ph-3-$OCF_3$ |
| H | $CH(CH_3)CH_2SCH_3$ | $CF_3$ | Ph-4-$OCF_3$ |
| H | $CH(CH_3)CH_2SCH_3$ (R) | $CF_3$ | Ph-4-$OCF_3$ |
| H | $CH(CH_3)CH_2SCH_3$ (S) | $CF_3$ | Ph-4-$OCF_3$ |
| H | $CH(CH_3)CH_2SCH_3$ | $CF_3$ | Ph-4-$OCF_2Br$ |
| H | $CH(CH_3)CH_2SCH_3$ | $CF_3$ | Ph-4-$OCH_2CF_3$ |
| H | $CH(CH_3)CH_2SCH_3$ | $CF_3$ | Ph-4-$OCF_2CHF_2$ |
| H | $CH(CH_3)CH_2SCH_3$ | $CF_3$ | Ph-4-$OCF_2CHFCl$ |
| H | $CH(CH_3)CH_2SCH_3$ | $CF_3$ | Ph-4-$OCF_2CHFBr$ |
| H | $CH(CH_3)CH_2SCH_3$ | $CF_3$ | Ph-4-$OCF_2CF_2Br$ |
| H | $CH(CH_3)CH_2SCH_3$ | $CF_3$ | Ph-4-$OCF_2CFCl_2$ |
| H | $CH(CH_3)CH_2SCH_3$ | $CF_3$ | Ph-4-$OCF_2CCl_3$ |
| H | $CH(CH_3)CH_2SCH_3$ | $CF_3$ | Ph-4-$OCH_2CF_2CHF_2$ |
| H | $CH(CH_3)CH_2SCH_3$ | $CF_3$ | Ph-3-$OCF_2CHFCF_3$ |
| H | $CH(CH_3)CH_2SCH_3$ | $CF_3$ | Ph-4-$OCF_2CHFCF_3$ |
| H | $CH(CH_3)CH_2SCH_3$ | $CF_3$ | Ph-4-$OCH(CF_3)_2$ |
| H | $CH(CH_3)CH_2SCH_3$ | $CF_3$ | Ph-4-$OCF_2CFBrCF_3$ |
| H | $CH(CH_3)CH_2SCH_3$ | $CF_3$ | Ph-3-$OCF_2CHFOCF_3$ |
| H | $CH(CH_3)CH_2SCH_3$ | $CF_3$ | Ph-4-$OCF_2CHFOCF_3$ |
| H | $CH(CH_3)CH_2SCH_3$ | $CF_3$ | Ph-4-$OCF_2CHFOCF_2CF_2CF_3$ |
| H | $CH(CH_3)CH_2SCH_3$ | $CF_3$ | Ph-4-$OSO_2CH_3$ |
| H | $CH(CH_3)CH_2SCH_3$ | $CF_3$ | Ph-4-$OSO_2CF_3$ |
| H | $CH(CH_3)CH_2SCH_3$ | $CF_3$ | Ph-3-O(Ph-4-Cl) |
| H | $CH(CH_3)CH_2SCH_3$ | $CF_3$ | Ph-4-O(Ph-4-Cl) |
| H | $CH(CH_3)CH_2SCH_3$ | $CF_3$ | Ph-4-O(Ph-4-Br) |
| H | $CH(CH_3)CH_2SCH_3$ | $CF_3$ | Ph-4-O(Ph-4-$CF_3$) |
| H | $CH(CH_3)CH_2SCH_3$ | $CF_3$ | Ph-4-O(L-45c)Br |
| H | $CH(CH_3)CH_2SCH_3$ | $CF_3$ | Ph-4-O(L-45c)$CF_3$ |
| H | $CH(CH_3)CH_2SCH_3$ | $CF_3$ | Ph-3-O(L-45e) |
| H | $CH(CH_3)CH_2SCH_3$ | $CF_3$ | Ph-4-O(L-45e) |
| H | $CH(CH_3)CH_2SCH_3$ | $CF_3$ | Ph-4-O(L-48b)Br |

| | | | |
|---|---|---|---|
| H | $CH(CH_3)CH_2SCH_3$ | $CF_3$ | Ph-4-$SCH_3$ |
| H | $CH(CH_3)CH_2SCH_3$ | $CF_3$ | Ph-4-$SO_2CH_3$ |
| H | $CH(CH_3)CH_2SCH_3$ | $CF_3$ | Ph-3-S(Ph-4-Cl) |
| H | $CH(CH_3)CH_2SCH_3$ | $CF_3$ | Ph-4-S(Ph-4-Cl) |
| H | $CH(CH_3)CH_2SCH_3$ | $CF_3$ | Ph-4-S(Ph-4-Br) |
| H | $CH(CH_3)CH_2SCH_3$ | $CF_3$ | Ph-4-S(Ph-4-$CF_3$) |
| H | $CH(CH_3)CH_2SCH_3$ | $CF_3$ | Ph-4-S(L-45c)Br |
| H | $CH(CH_3)CH_2SCH_3$ | $CF_3$ | Ph-4-S(L-45c)$CF_3$ |
| H | $CH(CH_3)CH_2SCH_3$ | $CF_3$ | Ph-4-S(L-45e) |
| H | $CH(CH_3)CH_2SCH_3$ | $CF_3$ | Ph-4-S(L-48b)Br |
| H | $CH(CH_3)CH_2SCH_3$ | $CF_3$ | Ph-4-$NO_2$ |
| H | $CH(CH_3)CH_2SCH_3$ | $CF_3$ | Ph-4-CN |
| H | $CH(CH_3)CH_2SCH_3$ | $CF_3$ | Ph-2, 3-$F_2$ |
| H | $CH(CH_3)CH_2SCH_3$ | $CF_3$ | Ph-2, 4-$F_2$ |
| H | $CH(CH_3)CH_2SCH_3$ | $CF_3$ | Ph-3, 4-$F_2$ |
| H | $CH(CH_3)CH_2SCH_3$ | $CF_3$ | Ph-2, 5-$F_2$ |
| H | $CH(CH_3)CH_2SCH_3$ | $CF_3$ | Ph-3, 5-$F_2$ |
| H | $CH(CH_3)CH_2SCH_3$ | $CF_3$ | Ph-2-Cl-4-F |
| H | $CH(CH_3)CH_2SCH_3$ | $CF_3$ | Ph-2-F-3-Cl |
| H | $CH(CH_3)CH_2SCH_3$ | $CF_3$ | Ph-3-Cl-4-F |
| H | $CH(CH_3)CH_2SCH_3$ | $CF_3$ | Ph-2-F-4-Cl |
| H | $CH(CH_3)CH_2SCH_3$ | $CF_3$ | Ph-3-F-4-Cl |
| H | $CH(CH_3)CH_2SCH_3$ | $CF_3$ | Ph-2, 3-$Cl_2$ |
| H | $CH(CH_3)CH_2SCH_3$ | $CF_3$ | Ph-2, 4-$Cl_2$ |
| H | $CH(CH_3)CH_2SCH_3$ | $CF_3$ | Ph-2, 5-$Cl_2$ |
| H | $CH(CH_3)CH_2SCH_3$ | $CF_3$ | Ph-3, 4-$Cl_2$ |
| H | $CH(CH_3)CH_2SCH_3$ | $CF_3$ | Ph-3, 5-$Cl_2$ |
| H | $CH(CH_3)CH_2SCH_3$ | $CF_3$ | Ph-3-Br-4-F |
| H | $CH(CH_3)CH_2SCH_3$ | $CF_3$ | Ph-2-F-4-Br |
| H | $CH(CH_3)CH_2SCH_3$ | $CF_3$ | Ph-2-F-5-Br |
| H | $CH(CH_3)CH_2SCH_3$ | $CF_3$ | Ph-3, 4-$Br_2$ |
| H | $CH(CH_3)CH_2SCH_3$ | $CF_3$ | Ph-3, 5-$Br_2$ |
| H | $CH(CH_3)CH_2SCH_3$ | $CF_3$ | Ph-3-$CH_3$-4-F |
| H | $CH(CH_3)CH_2SCH_3$ | $CF_3$ | Ph-3-F-4-$CH_3$ |
| H | $CH(CH_3)CH_2SCH_3$ | $CF_3$ | Ph-2-F-5-$CH_3$ |
| H | $CH(CH_3)CH_2SCH_3$ | $CF_3$ | Ph-2, 4-$(CH_3)_2$ |
| H | $CH(CH_3)CH_2SCH_3$ | $CF_3$ | Ph-3, 4-$(CH_3)_2$ |
| H | $CH(CH_3)CH_2SCH_3$ | $CF_3$ | Ph-2-F-3-$CF_3$ |
| H | $CH(CH_3)CH_2SCH_3$ | $CF_3$ | Ph-3-$CF_3$-4-F |
| H | $CH(CH_3)CH_2SCH_3$ | $CF_3$ | Ph-3-$CF_3$-4-Cl |
| H | $CH(CH_3)CH_2SCH_3$ | $CF_3$ | Ph-2-F-4-$CF_3$ |

| | | | |
|---|---|---|---|
| H | $CH(CH_3)CH_2SCH_3$ | $CF_3$ | Ph-3-F-4-$CF_3$ |
| H | $CH(CH_3)CH_2SCH_3$ | $CF_3$ | Ph-2-F-5-$CF_3$ |
| H | $CH(CH_3)CH_2SCH_3$ | $CF_3$ | Ph-3-F-5-$CF_3$ |
| H | $CH(CH_3)CH_2SCH_3$ | $CF_3$ | Ph-2-Cl-4-$CF_3$ |
| H | $CH(CH_3)CH_2SCH_3$ | $CF_3$ | Ph-3,5-$(CF_3)_2$ |
| H | $CH(CH_3)CH_2SCH_3$ | $CF_3$ | Ph-3-Br-4-$OCH_3$ |
| H | $CH(CH_3)CH_2SCH_3$ | $CF_3$ | Ph-3-F-4-$OCHF_2$ |
| H | $CH(CH_3)CH_2SCH_3$ | $CF_3$ | Ph-3-Cl-4-$OCHF_2$ |
| H | $CH(CH_3)CH_2SCH_3$ | $CF_3$ | Ph-3-Br-4-$OCHF_2$ |
| H | $CH(CH_3)CH_2SCH_3$ | $CF_3$ | Ph-3-F-4-$OCF_3$ |
| H | $CH(CH_3)CH_2SCH_3$ | $CF_3$ | Ph-3-Cl-4-$OCF_3$ |
| H | $CH(CH_3)CH_2SCH_3$ | $CF_3$ | Ph-3-Br-4-$OCF_3$ |
| H | $CH(CH_3)CH_2SCH_3$ | $CF_3$ | Ph-3-F-4-$OCF_2Br$ |
| H | $CH(CH_3)CH_2SCH_3$ | $CF_3$ | Ph-3-Cl-4-$OCF_2Br$ |
| H | $CH(CH_3)CH_2SCH_3$ | $CF_3$ | Ph-3-Br-4-$OCF_2Br$ |
| H | $CH(CH_3)CH_2SCH_3$ | $CF_3$ | Ph-3-F-4-$OCF_2CHF_2$ |
| H | $CH(CH_3)CH_2SCH_3$ | $CF_3$ | Ph-3-Cl-4-$OCF_2CHF_2$ |
| H | $CH(CH_3)CH_2SCH_3$ | $CF_3$ | Ph-3-Br-4-$OCF_2CHF_2$ |
| H | $CH(CH_3)CH_2SCH_3$ | $CF_3$ | Ph-3-F-4-$OCF_2CHFCl$ |
| H | $CH(CH_3)CH_2SCH_3$ | $CF_3$ | Ph-3-Cl-4-$OCF_2CHFCl$ |
| H | $CH(CH_3)CH_2SCH_3$ | $CF_3$ | Ph-3-Br-4-$OCF_2CHFCl$ |
| H | $CH(CH_3)CH_2SCH_3$ | $CF_3$ | Ph-3-F-4-$OCF_2CHFCF_3$ |
| H | $CH(CH_3)CH_2SCH_3$ | $CF_3$ | Ph-3-Cl-4-$OCF_2CHFCF_3$ |
| H | $CH(CH_3)CH_2SCH_3$ | $CF_3$ | Ph-3-Br-4-$OCF_2CHFCF_3$ |
| H | $CH(CH_3)CH_2SCH_3$ | $CF_3$ | Ph-3-F-4-$OCF_2CHFOCF_3$ |
| H | $CH(CH_3)CH_2SCH_3$ | $CF_3$ | Ph-3-Cl-4-$OCF_2CHFOCF_3$ |
| H | $CH(CH_3)CH_2SCH_3$ | $CF_3$ | Ph-3-Br-4-$OCF_2CHFOCF_3$ |
| H | $CH(CH_3)CH_2SCH_3$ | $CF_3$ | Ph-3-$CH_3$-4-$OCF_2CHFOCF_3$ |
| H | $CH(CH_3)CH_2SCH_3$ | $CF_3$ | Ph-3-F-4-$OCF_2CHFOCF_2CF_2CF_3$ |
| H | $CH(CH_3)CH_2SCH_3$ | $CF_3$ | Ph-3-Cl-4-$OCF_2CHFOCF_2CF_2CF_3$ |
| H | $CH(CH_3)CH_2SCH_3$ | $CF_3$ | Ph-3-Br-4-$OCF_2CHFOCF_2CF_2CF_3$ |
| H | $CH(CH_3)CH_2SCH_3$ | $CF_3$ | Ph(-3-$OCF_2O$-4-) |
| H | $CH(CH_3)CH_2SCH_3$ | $CF_3$ | Ph(-3-$OCF_2CF_2O$-4-) |
| H | $CH(CH_3)CH_2SCH_3$ | $CF_3$ | Ph-3-OPh-4-F |
| H | $CH(CH_3)CH_2SCH_3$ | $CF_3$ | Ph-3-$NO_2$-4-F |
| H | $CH(CH_3)CH_2SCH_3$ | $CF_3$ | Ph-3-$NO_2$-4-Cl |
| H | $CH(CH_3)CH_2SCH_3$ | $CF_3$ | Ph-2-F-5-$NO_2$ |
| H | $CH(CH_3)CH_2SCH_3$ | $CF_3$ | Ph-3-CN-4-F |
| H | $CH(CH_3)CH_2SCH_3$ | $CF_3$ | Ph-2,3,4-$F_3$ |
| H | $CH(CH_3)CH_2SCH_3$ | $CF_3$ | Ph-2,3,5-$F_3$ |
| H | $CH(CH_3)CH_2SCH_3$ | $CF_3$ | Ph-2,4,5-$F_3$ |

| | | | |
|---|---|---|---|
| H | $CH(CH_3)CH_2SCH_3$ | $CF_3$ | Ph-3, 4, 5-$F_3$ |
| H | $CH(CH_3)CH_2SCH_3$ | $CF_3$ | Ph-2, 3-$F_2$-4-$CH_3$ |
| H | $CH(CH_3)CH_2SCH_3$ | $CF_3$ | Ph-2, 3-$F_2$-4-$CF_3$ |
| H | $CH(CH_3)CH_2SCH_3$ | $CF_3$ | Ph-3, 4-$F_2$-5-$CF_3$ |
| H | $CH(CH_3)CH_2SCH_3$ | $CF_3$ | Ph-2-F-3-Cl-5-$CF_3$ |
| H | $CH(CH_3)CH_2SCH_3$ | $CF_3$ | Ph-3, 5-$Cl_2$-4-$OCH_3$ |
| H | $CH(CH_3)CH_2SCH_3$ | $CF_3$ | 1-Naph |
| H | $CH(CH_3)CH_2SCH_3$ | $CF_3$ | 2-Naph |
| H | $CH(CH_3)CH_2SCH_3$ | $CF_3$ | (L-1b) Br |
| H | $CH(CH_3)CH_2SCH_3$ | $CF_3$ | (L-1c) Cl |
| H | $CH(CH_3)CH_2SCH_3$ | $CF_3$ | (L-1c) Br |
| H | $CH(CH_3)CH_2SCH_3$ | $CF_3$ | (L-1c) I |
| H | $CH(CH_3)CH_2SCH_3$ | $CF_3$ | (L-1c) $CF_3$ |
| H | $CH(CH_3)CH_2SCH_3$ | $CF_3$ | (L-2b) Br |
| H | $CH(CH_3)CH_2SCH_3$ | $CF_3$ | (L-3b) Cl |
| H | $CH(CH_3)CH_2SCH_3$ | $CF_3$ | (L-3b) Br |
| H | $CH(CH_3)CH_2SCH_3$ | $CF_3$ | (L-3c) F |
| H | $CH(CH_3)CH_2SCH_3$ | $CF_3$ | (L-3c) Cl |
| H | $CH(CH_3)CH_2SCH_3$ | $CF_3$ | (L-3c) Br |
| H | $CH(CH_3)CH_2SCH_3$ | $CF_3$ | (L-3c) I |
| H | $CH(CH_3)CH_2SCH_3$ | $CF_3$ | (L-3c) $CF_3$ |
| H | $CH(CH_3)CH_2SCH_3$ | $CF_3$ | (L-3c) CN |
| H | $CH(CH_3)CH_2SCH_3$ | $CF_3$ | L-3d |
| H | $CH(CH_3)CH_2SCH_3$ | $CF_3$ | (L-4b) Cl |
| H | $CH(CH_3)CH_2SCH_3$ | $CF_3$ | (L-4b) Br |
| H | $CH(CH_3)CH_2SCH_3$ | $CF_3$ | (L-4b) CN |
| H | $CH(CH_3)CH_2SCH_3$ | $CF_3$ | (L-10b) Cl |
| H | $CH(CH_3)CH_2SCH_3$ | $CF_3$ | (L-10b) Br |
| H | $CH(CH_3)CH_2SCH_3$ | $CF_3$ | (L-15b) $CF_3$ |
| H | $CH(CH_3)CH_2SCH_3$ | $CF_3$ | (L-16a) $CHF_2$ |
| H | $CH(CH_3)CH_2SCH_3$ | $CF_3$ | (L-16a) $CF_2Br$ |
| H | $CH(CH_3)CH_2SCH_3$ | $CF_3$ | (L-17a) Cl |
| H | $CH(CH_3)CH_2SCH_3$ | $CF_3$ | (L-21b) Cl |
| H | $CH(CH_3)CH_2SCH_3$ | $CF_3$ | (L-21b) Br |
| H | $CH(CH_3)CH_2SCH_3$ | $CF_3$ | (L-21b) I |
| H | $CH(CH_3)CH_2SCH_3$ | $CF_3$ | (L-21b) $CF_3$ |
| H | $CH(CH_3)CH_2SCH_3$ | $CF_3$ | (L-22b) Cl |
| H | $CH(CH_3)CH_2SCH_3$ | $CF_3$ | (L-22b) Br |
| H | $CH(CH_3)CH_2SCH_3$ | $CF_3$ | (L-23b) Cl |
| H | $CH(CH_3)CH_2SCH_3$ | $CF_3$ | (L-23b) Br |
| H | $CH(CH_3)CH_2SCH_3$ | $CF_3$ | (L-23c) Cl |

| | | | |
|---|---|---|---|
| H | $CH(CH_3)CH_2SCH_3$ | $CF_3$ | (L-23c) Br |
| H | $CH(CH_3)CH_2SCH_3$ | $CF_3$ | (L-31a) Cl |
| H | $CH(CH_3)CH_2SCH_3$ | $CF_3$ | (L-31a) Br |
| H | $CH(CH_3)CH_2SCH_3$ | $CF_3$ | (L-45c) F |
| H | $CH(CH_3)CH_2SCH_3$ | $CF_3$ | (L-45c) Cl |
| H | $CH(CH_3)CH_2SCH_3$ | $CF_3$ | (L-45c) Br |
| H | $CH(CH_3)CH_2SCH_3$ | $CF_3$ | (L-45c) I |
| H | $CH(CH_3)CH_2SCH_3$ | $CF_3$ | (L-45c) $CF_3$ |
| H | $CH(CH_3)CH_2SCH_3$ | $CF_3$ | L-45e |
| H | $CH(CH_3)CH_2SCH_3$ | $CF_3$ | L-45f |
| H | $CH(CH_3)CH_2SCH_3$ | $CF_3$ | (L-46c) F |
| H | $CH(CH_3)CH_2SCH_3$ | $CF_3$ | (L-46c) Cl |
| H | $CH(CH_3)CH_2SCH_3$ | $CF_3$ | (L-46c) Br |
| H | $CH(CH_3)CH_2SCH_3$ | $CF_3$ | (L-46c) I |
| H | $CH(CH_3)CH_2SCH_3$ | $CF_3$ | (L-46c) $CF_3$ |
| H | $CH(CH_3)CH_2SCH_3$ | $CF_3$ | (L-46c) $OCH_2CF_3$ |
| H | $CH(CH_3)CH_2SCH_3$ | $CF_3$ | (L-46c) $OCH(CF_3)_2$ |
| H | $CH(CH_3)CH_2SCH_3$ | $CF_3$ | L-46d |
| H | $CH(CH_3)CH_2SCH_3$ | $CF_3$ | L-47a |
| H | $CH(CH_3)CH_2SCH_3$ | $CF_3$ | L-47d |
| H | $CH(CH_3)CH_2SCH_3$ | $CF_3$ | (L-48b) Br |
| H | $CH(CH_3)CH_2SCH_3$ | $CF_3$ | (L-50b) Cl |
| H | $CH(CH_3)CH_2SCH_3$ | $CF_3$ | (L-50b) Br |
| H | $CH(CH_3)CH_2SCH_3$ | $CF_3$ | (L-51b) Cl |
| H | $CH(CH_3)CH_2SCH_3$ | $CF_2Cl$ | Ph-4-Cl |
| H | $CH(CH_3)CH_2SCH_3$ | $CF_2Cl$ | Ph-4-Br |
| H | $CH(CH_3)CH_2SCH_3$ | $CF_2Cl$ | Ph-4-$CF_3$ |
| H | $CH(CH_3)CH_2SCH_3$ | $CF_2Cl$ | Ph-4-$OCF_3$ |
| H | $CH(CH_3)CH_2SCH_3$ | $CF_2Cl$ | Ph-4-$OCF_2Br$ |
| H | $CH(CH_3)CH_2SCH_3$ | $CF_2Cl$ | Ph-4-$OCF_2CHFCF_3$ |
| H | $CH(CH_3)CH_2SCH_3$ | $CF_2Cl$ | Ph-4-$OCF_2CHFOCF_3$ |
| H | $CH(CH_3)CH_2SCH_3$ | $CF_2Cl$ | Ph-4-$OCF_2CHFOCF_2CF_2CF_3$ |
| H | $CH(CH_3)CH_2SCH_3$ | $CF_2Cl$ | Ph-4-O(L-45g) |
| H | $CH(CH_3)CH_2SCH_3$ | $CF_2Cl$ | Ph-3, 4-$Cl_2$ |
| H | $CH(CH_3)CH_2SCH_3$ | $CF_2Cl$ | Ph(-3-$OCF_2O$-4-) |
| H | $CH(CH_3)CH_2SCH_3$ | $CF_2Cl$ | Ph(-3-$OCF_2CF_2O$-4-) |
| H | $CH(CH_3)CH_2SCH_3$ | $CF_2Cl$ | (L-45c) $CF_3$ |
| H | $CH(CH_3)CH_2SCH_3$ | $CF_2Cl$ | L-45f |
| H | $CH(CH_3)CH_2SCH_3$ | $CF_2Cl$ | (L-46c) $CF_3$ |
| H | $CH(CH_3)CH_2SCH_3$ | $CF_2Cl$ | L-46d |
| H | $CH(CH_3)CH_2SCH_3$ | $CFCl_2$ | (L-45c) $CF_3$ |

| | | | |
|---|---|---|---|
| H | $CH(CH_3)CH_2SCH_3$ | $CF_2Br$ | Ph-4-Cl |
| H | $CH(CH_3)CH_2SCH_3$ | $CF_2Br$ | Ph-4-Br |
| H | $CH(CH_3)CH_2SCH_3$ | $CF_2Br$ | Ph-4-$CF_3$ |
| H | $CH(CH_3)CH_2SCH_3$ | $CF_2Br$ | Ph-4-$OCF_3$ |
| H | $CH(CH_3)CH_2SCH_3$ | $CF_2Br$ | Ph-4-$OCF_2Br$ |
| H | $CH(CH_3)CH_2SCH_3$ | $CF_2Br$ | Ph-4-$OCF_2CHFCF_3$ |
| H | $CH(CH_3)CH_2SCH_3$ | $CF_2Br$ | Ph-4-$OCF_2CHFOCF_3$ |
| H | $CH(CH_3)CH_2SCH_3$ | $CF_2Br$ | Ph-4-$OCF_2CHFOCF_2CF_2CF_3$ |
| H | $CH(CH_3)CH_2SCH_3$ | $CF_2Br$ | Ph-4-O(L-45g) |
| H | $CH(CH_3)CH_2SCH_3$ | $CF_2Br$ | Ph-3, 4-$Cl_2$ |
| H | $CH(CH_3)CH_2SCH_3$ | $CF_2Br$ | Ph(-3-$OCF_2O$-4-) |
| H | $CH(CH_3)CH_2SCH_3$ | $CF_2Br$ | Ph(-3-$OCF_2CF_2O$-4-) |
| H | $CH(CH_3)CH_2SCH_3$ | $CF_2Br$ | (L-45c)$CF_3$ |
| H | $CH(CH_3)CH_2SCH_3$ | $CF_2Br$ | L-45f |
| H | $CH(CH_3)CH_2SCH_3$ | $CF_2Br$ | (L-46c)$CF_3$ |
| H | $CH(CH_3)CH_2SCH_3$ | $CF_2Br$ | L-46d |
| H | $CH(CH_3)CH_2SCH_3$ | CFClBr | Ph-4-Br |
| H | $CH(CH_3)CH_2SCH_3$ | $CFBr_2$ | Ph-4-$CF_3$ |
| H | $CH(CH_3)CH_2SCH_3$ | $CF_2CHF_2$ | Ph-4-$OCF_3$ |
| H | $CH(CH_3)CH_2SCH_3$ | $CF_2CF_3$ | Ph-4-Cl |
| H | $CH(CH_3)CH_2SCH_3$ | $CF_2CF_3$ | Ph-4-Br |
| H | $CH(CH_3)CH_2SCH_3$ | $CF_2CF_3$ | Ph-4-$CF_3$ |
| H | $CH(CH_3)CH_2SCH_3$ | $CF_2CF_3$ | Ph-4-$OCF_3$ |
| H | $CH(CH_3)CH_2SCH_3$ | $CF_2CF_3$ | Ph-4-$OCF_2Br$ |
| H | $CH(CH_3)CH_2SCH_3$ | $CF_2CF_3$ | Ph-4-$OCF_2CHFCF_3$ |
| H | $CH(CH_3)CH_2SCH_3$ | $CF_2CF_3$ | Ph-4-$OCF_2CHFOCF_3$ |
| H | $CH(CH_3)CH_2SCH_3$ | $CF_2CF_3$ | Ph-4-$OCF_2CHFOCF_2CF_2CF_3$ |
| H | $CH(CH_3)CH_2SCH_3$ | $CF_2CF_3$ | Ph-4-O(L-45g) |
| H | $CH(CH_3)CH_2SCH_3$ | $CF_2CF_3$ | Ph-3, 4-$Cl_2$ |
| H | $CH(CH_3)CH_2SCH_3$ | $CF_2CF_3$ | Ph(-3-$OCF_2O$-4-) |
| H | $CH(CH_3)CH_2SCH_3$ | $CF_2CF_3$ | Ph(-3-$OCF_2CF_2O$-4-) |
| H | $CH(CH_3)CH_2SCH_3$ | $CF_2CF_3$ | (L-45c)$CF_3$ |
| H | $CH(CH_3)CH_2SCH_3$ | $CF_2CF_3$ | L-45f |
| H | $CH(CH_3)CH_2SCH_3$ | $CF_2CF_3$ | (L-46c)$CF_3$ |
| H | $CH(CH_3)CH_2SCH_3$ | $CF_2CF_3$ | L-46d |
| H | $CH(CH_3)CH_2SCH_3$ | $CF_2CF_2Cl$ | (L-45c)$CF_3$ |
| H | $CH(CH_3)CH_2SCH_3$ | $CFClCF_3$ | Ph-4-Br |
| H | $CH(CH_3)CH_2SCH_3$ | $CFClCF_2Cl$ | Ph-4-$CF_3$ |
| H | $CH(CH_3)CH_2SCH_3$ | $CF_2CF_2Br$ | Ph-4-$OCF_3$ |
| H | $CH(CH_3)CH_2SCH_3$ | $CFBrCF_3$ | (L-45c)$CF_3$ |
| H | $CH(CH_3)CH_2SCH_3$ | $CF_2CHFCF_3$ | Ph-4-Br |

| | | | |
|---|---|---|---|
| H | $CH(CH_3)CH_2SCH_3$ | $CF_2CF_2CF_3$ | Ph-4-Cl |
| H | $CH(CH_3)CH_2SCH_3$ | $CF_2CF_2CF_3$ | Ph-4-Br |
| H | $CH(CH_3)CH_2SCH_3$ | $CF_2CF_2CF_3$ | Ph-4-$CF_3$ |
| H | $CH(CH_3)CH_2SCH_3$ | $CF_2CF_2CF_3$ | Ph-4-$OCF_3$ |
| H | $CH(CH_3)CH_2SCH_3$ | $CF_2CF_2CF_3$ | Ph-4-$OCF_2Br$ |
| H | $CH(CH_3)CH_2SCH_3$ | $CF_2CF_2CF_3$ | Ph-4-$OCF_2CHFCF_3$ |
| H | $CH(CH_3)CH_2SCH_3$ | $CF_2CF_2CF_3$ | Ph-4-$OCF_2CHFOCF_3$ |
| H | $CH(CH_3)CH_2SCH_3$ | $CF_2CF_2CF_3$ | Ph-4-$OCF_2CHFOCF_2CF_2CF_3$ |
| H | $CH(CH_3)CH_2SCH_3$ | $CF_2CF_2CF_3$ | Ph-4-O(L-45g) |
| H | $CH(CH_3)CH_2SCH_3$ | $CF_2CF_2CF_3$ | Ph-3, 4-$Cl_2$ |
| H | $CH(CH_3)CH_2SCH_3$ | $CF_2CF_2CF_3$ | Ph(-3-$OCF_2O$-4-) |
| H | $CH(CH_3)CH_2SCH_3$ | $CF_2CF_2CF_3$ | Ph(-3-$OCF_2CF_2O$-4-) |
| H | $CH(CH_3)CH_2SCH_3$ | $CF_2CF_2CF_3$ | (L-45c)$CF_3$ |
| H | $CH(CH_3)CH_2SCH_3$ | $CF_2CF_2CF_3$ | L-45f |
| H | $CH(CH_3)CH_2SCH_3$ | $CF_2CF_2CF_3$ | (L-46c)$CF_3$ |
| H | $CH(CH_3)CH_2SCH_3$ | $CF_2CF_2CF_3$ | L-46d |
| H | $CH(CH_3)CH_2SCH_3$ | $CF(CF_3)_2$ | Ph-4-$CF_3$ |
| H | $CH(CH_3)CH_2SCH_3$ | $CF_2CF_2CF_2CF_3$ | Ph-4-$OCF_3$ |
| H | $CH(CH_3)CH_2SCH_3$ | T-1 | (L-45c)$CF_3$ |
| H | $CH(CH_3)CH_2SCH_3$ | T-2 | Ph-4-Br |
| H | $CH(CH_3)CH_2SCH_3$ | $CH_2OCH_3$ | Ph-4-$CF_3$ |
| H | $CH(CH_3)CH_2SCH_3$ | $CH_2OEt$ | Ph-4-$OCF_3$ |
| H | $CH(CH_3)CH_2SCH_3$ | $CH_2OCH_2CF_3$ | (L-45c)$CF_3$ |
| H | $CH(CH_3)CH_2SCH_3$ | $CH_2OCH(CF_3)_2$ | Ph-4-Br |
| H | $CH(CH_3)CH_2SCH_3$ | $CF_2OCH_3$ | Ph-4-Cl |
| H | $CH(CH_3)CH_2SCH_3$ | $CF_2OCH_3$ | Ph-4-Br |
| H | $CH(CH_3)CH_2SCH_3$ | $CF_2OCH_3$ | Ph-4-$CF_3$ |
| H | $CH(CH_3)CH_2SCH_3$ | $CF_2OCH_3$ | Ph-4-$OCF_3$ |
| H | $CH(CH_3)CH_2SCH_3$ | $CF_2OCH_3$ | Ph-4-$OCF_2Br$ |
| H | $CH(CH_3)CH_2SCH_3$ | $CF_2OCH_3$ | Ph-4-$OCF_2CHFCF_3$ |
| H | $CH(CH_3)CH_2SCH_3$ | $CF_2OCH_3$ | Ph-4-$OCF_2CHFOCF_3$ |
| H | $CH(CH_3)CH_2SCH_3$ | $CF_2OCH_3$ | Ph-4-$OCF_2CHFOCF_2CF_2CF_3$ |
| H | $CH(CH_3)CH_2SCH_3$ | $CF_2OCH_3$ | Ph-4-O(L-45g) |
| H | $CH(CH_3)CH_2SCH_3$ | $CF_2OCH_3$ | Ph-3, 4-$Cl_2$ |
| H | $CH(CH_3)CH_2SCH_3$ | $CF_2OCH_3$ | Ph(-3-$OCF_2O$-4-) |
| H | $CH(CH_3)CH_2SCH_3$ | $CF_2OCH_3$ | Ph(-3-$OCF_2CF_2O$-4-) |
| H | $CH(CH_3)CH_2SCH_3$ | $CF_2OCH_3$ | (L-45c)$CF_3$ |
| H | $CH(CH_3)CH_2SCH_3$ | $CF_2OCH_3$ | L-45f |
| H | $CH(CH_3)CH_2SCH_3$ | $CF_2OCH_3$ | (L-46c)$CF_3$ |
| H | $CH(CH_3)CH_2SCH_3$ | $CF_2OCH_3$ | L-46d |
| H | $CH(CH_3)CH_2SCH_3$ | $CH_2SCH_3$ | Ph-4-$CF_3$ |

| | | | |
|---|---|---|---|
| H | $CH(CH_3)CH_2SCH_3$ | $CH_2SO_2CH_3$ | Ph-4-$OCF_3$ |
| H | $CH(CH_3)CH_2SCH_3$ | $CH_2SCF_3$ | (L-45c)$CF_3$ |
| H | $CH(CH_3)CH_2SCH_3$ | $CF_2SCH_3$ | Ph-4-Cl |
| H | $CH(CH_3)CH_2SCH_3$ | $CF_2SCH_3$ | Ph-4-Br |
| H | $CH(CH_3)CH_2SCH_3$ | $CF_2SCH_3$ | Ph-4-$CF_3$ |
| H | $CH(CH_3)CH_2SCH_3$ | $CF_2SCH_3$ | Ph-4-$OCF_3$ |
| H | $CH(CH_3)CH_2SCH_3$ | $CF_2SCH_3$ | Ph-4-$OCF_2Br$ |
| H | $CH(CH_3)CH_2SCH_3$ | $CF_2SCH_3$ | Ph-4-$OCF_2CHFCF_3$ |
| H | $CH(CH_3)CH_2SCH_3$ | $CF_2SCH_3$ | Ph-4-$OCF_2CHFOCF_3$ |
| H | $CH(CH_3)CH_2SCH_3$ | $CF_2SCH_3$ | Ph-4-$OCF_2CHFOCF_2CF_2CF_3$ |
| H | $CH(CH_3)CH_2SCH_3$ | $CF_2SCH_3$ | Ph-4-O(L-45g) |
| H | $CH(CH_3)CH_2SCH_3$ | $CF_2SCH_3$ | Ph-3,4-$Cl_2$ |
| H | $CH(CH_3)CH_2SCH_3$ | $CF_2SCH_3$ | Ph(-3-$OCF_2O$-4-) |
| H | $CH(CH_3)CH_2SCH_3$ | $CF_2SCH_3$ | Ph(-3-$OCF_2CF_2O$-4-) |
| H | $CH(CH_3)CH_2SCH_3$ | $CF_2SCH_3$ | (L-45c)$CF_3$ |
| H | $CH(CH_3)CH_2SCH_3$ | $CF_2SCH_3$ | L-45f |
| H | $CH(CH_3)CH_2SCH_3$ | $CF_2SCH_3$ | (L-46c)$CF_3$ |
| H | $CH(CH_3)CH_2SCH_3$ | $CF_2SCH_3$ | L-46d |
| H | $CH(CH_3)CH_2SCH_3$ | $CF_2SEt$ | Ph-4-Br |
| H | $CH(CH_3)CH_2SCH_3$ | $CF_2SPr$-n | Ph-4-$CF_3$ |
| H | $CH(CH_3)CH_2SCH_3$ | $CF_2SPr$-i | Ph-4-$OCF_3$ |
| H | $CH(CH_3)CH_2SCH_3$ | $CF_2SCH_2Ph$ | (L-45c)$CF_3$ |
| H | $CH(CH_3)CH_2SCH_3$ | $CF_2SPh$ | Ph-4-Br |
| H | $CH(CH_3)CH_2SCH_3$ | $CF_2C(O)OEt$ | Ph-4-$CF_3$ |
| H | $CH(CH_3)CH_2SCH_3$ | $CF_2SO_2N(CH_3)_2$ | Ph-4-$OCF_3$ |
| H | $CH(CH_3)CH_2SCH_3$ | C(O)OEt | (L-45c)$CF_3$ |
| H | $CH(CH_3)CH_2SCH_3$ | C(O)SEt | Ph-4-Br |
| $CH_3$ | $CH(CH_3)CH_2SCH_3$ | $CF_3$ | Ph-4-$CF_3$ |
| Et | $CH(CH_3)CH_2SCH_3$ | $CF_3$ | Ph-4-$OCF_3$ |
| H | $CH(CH_3)CH_2S(O)CH_3$ | $CH_3$ | Ph-4-Br |
| H | $CH(CH_3)CH_2S(O)CH_3$ | $CH_3$ | Ph-4-$CF_3$ |
| H | $CH(CH_3)CH_2S(O)CH_3$ | $CH_3$ | Ph-4-$OCF_3$ |
| H | $CH(CH_3)CH_2S(O)CH_3$ | $CH_3$ | (L-45c)$CF_3$ |
| H | $CH(CH_3)CH_2S(O)CH_3$ | $CF_3$ | $CH_2O$(Ph-4-Cl) |
| H | $CH(CH_3)CH_2S(O)CH_3$ | $CF_3$ | $CH_2O$(Ph-4-Br) |
| H | $CH(CH_3)CH_2S(O)CH_3$ | $CF_3$ | $CH_2O$(Ph-4-$CF_3$) |
| H | $CH(CH_3)CH_2S(O)CH_3$ | $CF_3$ | $CH_2O$(Ph-4-$OCF_3$) |
| H | $CH(CH_3)CH_2S(O)CH_3$ | $CF_3$ | $CF_2O$(Ph-4-Cl) |
| H | $CH(CH_3)CH_2S(O)CH_3$ | $CF_3$ | $CF_2O$(Ph-4-Br) |
| H | $CH(CH_3)CH_2S(O)CH_3$ | $CF_3$ | $CF_2O$(Ph-4-$CF_3$) |
| H | $CH(CH_3)CH_2S(O)CH_3$ | $CF_3$ | $CF_2O$(Ph-4-$OCF_3$) |

| | | | |
|---|---|---|---|
| H | $CH(CH_3)CH_2S(O)CH_3$ | $CF_3$ | CH=CH(Ph-4-F) |
| H | $CH(CH_3)CH_2S(O)CH_3$ | $CF_3$ | CH=CH(Ph-4-Cl) |
| H | $CH(CH_3)CH_2S(O)CH_3$ | $CF_3$ | CH=CH(Ph-4-Br) |
| H | $CH(CH_3)CH_2S(O)CH_3$ | $CF_3$ | CH=CH(Ph-4-$CF_3$) |
| H | $CH(CH_3)CH_2S(O)CH_3$ | $CF_3$ | CH=CH(Ph-4-$OCF_3$) |
| H | $CH(CH_3)CH_2S(O)CH_3$ | $CF_3$ | CH=CH(Ph-4-$SCH_3$) |
| H | $CH(CH_3)CH_2S(O)CH_3$ | $CF_3$ | CH=CH(Ph-4-$SO_2CH_3$) |
| H | $CH(CH_3)CH_2S(O)CH_3$ | $CF_3$ | CH=CH(Ph-3,4-$Cl_2$) |
| H | $CH(CH_3)CH_2S(O)CH_3$ | $CF_3$ | Ph-4-F |
| H | $CH(CH_3)CH_2S(O)CH_3$ | $CF_3$ | Ph-2-Cl |
| H | $CH(CH_3)CH_2S(O)CH_3$ | $CF_3$ | Ph-3-Cl |
| H | $CH(CH_3)CH_2S(O)CH_3$ | $CF_3$ | Ph-4-Cl |
| H | $CH(CH_3)CH_2S(O)CH_3$ | $CF_3$ | Ph-4-Br |
| H | $CH(CH_3)CH_2S(O)CH_3$ | $CF_3$ | Ph-4-I |
| H | $CH(CH_3)CH_2S(O)CH_3$ | $CF_3$ | Ph-4-$CF_3$ |
| H | $CH(CH_3)CH_2S(O)CH_3$ | $CF_3$ | Ph-4-$OCHF_2$ |
| H | $CH(CH_3)CH_2S(O)CH_3$ | $CF_3$ | Ph-4-$OCF_3$ |
| H | $CH(CH_3)CH_2S(O)CH_3$ | $CF_3$ | Ph-4-$OCF_2Br$ |
| H | $CH(CH_3)CH_2S(O)CH_3$ | $CF_3$ | Ph-4-$OCH_2CF_3$ |
| H | $CH(CH_3)CH_2S(O)CH_3$ | $CF_3$ | Ph-4-$OCF_2CHF_2$ |
| H | $CH(CH_3)CH_2S(O)CH_3$ | $CF_3$ | Ph-4-$OCF_2CHFCl$ |
| H | $CH(CH_3)CH_2S(O)CH_3$ | $CF_3$ | Ph-4-$OCF_2CHFCF_3$ |
| H | $CH(CH_3)CH_2S(O)CH_3$ | $CF_3$ | Ph-4-$OCF_2CHFOCF_3$ |
| H | $CH(CH_3)CH_2S(O)CH_3$ | $CF_3$ | Ph-4-$OCF_2CHFOCF_2CF_2CF_3$ |
| H | $CH(CH_3)CH_2S(O)CH_3$ | $CF_3$ | Ph-4-$OSO_2CH_3$ |
| H | $CH(CH_3)CH_2S(O)CH_3$ | $CF_3$ | Ph-4-$OSO_2CF_3$ |
| H | $CH(CH_3)CH_2S(O)CH_3$ | $CF_3$ | Ph-4-O(L-45e) |
| H | $CH(CH_3)CH_2S(O)CH_3$ | $CF_3$ | Ph-2,4-$F_2$ |
| H | $CH(CH_3)CH_2S(O)CH_3$ | $CF_3$ | Ph-3,4-$F_2$ |
| H | $CH(CH_3)CH_2S(O)CH_3$ | $CF_3$ | Ph-3-Cl-4-F |
| H | $CH(CH_3)CH_2S(O)CH_3$ | $CF_3$ | Ph-2-F-4-Cl |
| H | $CH(CH_3)CH_2S(O)CH_3$ | $CF_3$ | Ph-3-F-4-Cl |
| H | $CH(CH_3)CH_2S(O)CH_3$ | $CF_3$ | Ph-2,4-$Cl_2$ |
| H | $CH(CH_3)CH_2S(O)CH_3$ | $CF_3$ | Ph-3,4-$Cl_2$ |
| H | $CH(CH_3)CH_2S(O)CH_3$ | $CF_3$ | Ph-3,5-$Cl_2$ |
| H | $CH(CH_3)CH_2S(O)CH_3$ | $CF_3$ | Ph-3-Br-4-F |
| H | $CH(CH_3)CH_2S(O)CH_3$ | $CF_3$ | Ph-2-F-4-Br |
| H | $CH(CH_3)CH_2S(O)CH_3$ | $CF_3$ | Ph-3,4-$Br_2$ |
| H | $CH(CH_3)CH_2S(O)CH_3$ | $CF_3$ | Ph-3-$CH_3$-4-F |
| H | $CH(CH_3)CH_2S(O)CH_3$ | $CF_3$ | Ph-3-$CF_3$-4-F |
| H | $CH(CH_3)CH_2S(O)CH_3$ | $CF_3$ | Ph-3-$CF_3$-4-Cl |

| | | | |
|---|---|---|---|
| H | $CH(CH_3)CH_2S(O)CH_3$ | $CF_3$ | Ph-2-F-4-$CF_3$ |
| H | $CH(CH_3)CH_2S(O)CH_3$ | $CF_3$ | Ph-3-F-4-$CF_3$ |
| H | $CH(CH_3)CH_2S(O)CH_3$ | $CF_3$ | Ph-2-Cl-4-$CF_3$ |
| H | $CH(CH_3)CH_2S(O)CH_3$ | $CF_3$ | Ph-3-F-4-$OCHF_2$ |
| H | $CH(CH_3)CH_2S(O)CH_3$ | $CF_3$ | Ph-3-Cl-4-$OCHF_2$ |
| H | $CH(CH_3)CH_2S(O)CH_3$ | $CF_3$ | Ph-3-Br-4-$OCHF_2$ |
| H | $CH(CH_3)CH_2S(O)CH_3$ | $CF_3$ | Ph-3-F-4-$OCF_3$ |
| H | $CH(CH_3)CH_2S(O)CH_3$ | $CF_3$ | Ph-3-Cl-4-$OCF_3$ |
| H | $CH(CH_3)CH_2S(O)CH_3$ | $CF_3$ | Ph-3-Br-4-$OCF_3$ |
| H | $CH(CH_3)CH_2S(O)CH_3$ | $CF_3$ | Ph-3-F-4-$OCF_2Br$ |
| H | $CH(CH_3)CH_2S(O)CH_3$ | $CF_3$ | Ph-3-Cl-4-$OCF_2Br$ |
| H | $CH(CH_3)CH_2S(O)CH_3$ | $CF_3$ | Ph-3-Br-4-$OCF_2Br$ |
| H | $CH(CH_3)CH_2S(O)CH_3$ | $CF_3$ | Ph-3-F-4-$OCF_2CHF_2$ |
| H | $CH(CH_3)CH_2S(O)CH_3$ | $CF_3$ | Ph-3-Cl-4-$OCF_2CHF_2$ |
| H | $CH(CH_3)CH_2S(O)CH_3$ | $CF_3$ | Ph-3-Br-4-$OCF_2CHF_2$ |
| H | $CH(CH_3)CH_2S(O)CH_3$ | $CF_3$ | Ph-3-F-4-$OCF_2CHFCl$ |
| H | $CH(CH_3)CH_2S(O)CH_3$ | $CF_3$ | Ph-3-Cl-4-$OCF_2CHFCl$ |
| H | $CH(CH_3)CH_2S(O)CH_3$ | $CF_3$ | Ph-3-Br-4-$OCF_2CHFCl$ |
| H | $CH(CH_3)CH_2S(O)CH_3$ | $CF_3$ | Ph-3-F-4-$OCF_2CHFCF_3$ |
| H | $CH(CH_3)CH_2S(O)CH_3$ | $CF_3$ | Ph-3-Cl-4-$OCF_2CHFCF_3$ |
| H | $CH(CH_3)CH_2S(O)CH_3$ | $CF_3$ | Ph-3-Br-4-$OCF_2CHFCF_3$ |
| H | $CH(CH_3)CH_2S(O)CH_3$ | $CF_3$ | Ph-3-F-4-$OCF_2CHFOCF_3$ |
| H | $CH(CH_3)CH_2S(O)CH_3$ | $CF_3$ | Ph-3-Cl-4-$OCF_2CHFOCF_3$ |
| H | $CH(CH_3)CH_2S(O)CH_3$ | $CF_3$ | Ph-3-Br-4-$OCF_2CHFOCF_3$ |
| H | $CH(CH_3)CH_2S(O)CH_3$ | $CF_3$ | Ph-3-$CH_3$-4-$OCF_2CHFOCF_3$ |
| H | $CH(CH_3)CH_2S(O)CH_3$ | $CF_3$ | Ph-3-F-4-$OCF_2CHFOCF_2CF_2CF_3$ |
| H | $CH(CH_3)CH_2S(O)CH_3$ | $CF_3$ | Ph-3-Cl-4-$OCF_2CHFOCF_2CF_2CF_3$ |
| H | $CH(CH_3)CH_2S(O)CH_3$ | $CF_3$ | Ph-3-Br-4-$OCF_2CHFOCF_2CF_2CF_3$ |
| H | $CH(CH_3)CH_2S(O)CH_3$ | $CF_3$ | Ph(-3-$OCF_2O$-4-) |
| H | $CH(CH_3)CH_2S(O)CH_3$ | $CF_3$ | Ph(-3-$OCF_2CF_2O$-4-) |
| H | $CH(CH_3)CH_2S(O)CH_3$ | $CF_3$ | Ph-3,4-$F_2$-5-$CF_3$ |
| H | $CH(CH_3)CH_2S(O)CH_3$ | $CF_3$ | 2-Naph |
| H | $CH(CH_3)CH_2S(O)CH_3$ | $CF_3$ | (L-1b)Br |
| H | $CH(CH_3)CH_2S(O)CH_3$ | $CF_3$ | (L-1c)Cl |
| H | $CH(CH_3)CH_2S(O)CH_3$ | $CF_3$ | (L-1c)Br |
| H | $CH(CH_3)CH_2S(O)CH_3$ | $CF_3$ | (L-1c)$CF_3$ |
| H | $CH(CH_3)CH_2S(O)CH_3$ | $CF_3$ | (L-2b)Br |
| H | $CH(CH_3)CH_2S(O)CH_3$ | $CF_3$ | (L-3b)Cl |
| H | $CH(CH_3)CH_2S(O)CH_3$ | $CF_3$ | (L-3b)Br |
| H | $CH(CH_3)CH_2S(O)CH_3$ | $CF_3$ | (L-3c)Cl |
| H | $CH(CH_3)CH_2S(O)CH_3$ | $CF_3$ | (L-3c)Br |

| | | | |
|---|---|---|---|
| H | $CH(CH_3)CH_2S(O)CH_3$ | $CF_3$ | (L-3c) $CF_3$ |
| H | $CH(CH_3)CH_2S(O)CH_3$ | $CF_3$ | L-3d |
| H | $CH(CH_3)CH_2S(O)CH_3$ | $CF_3$ | (L-4b) Cl |
| H | $CH(CH_3)CH_2S(O)CH_3$ | $CF_3$ | (L-4b) Br |
| H | $CH(CH_3)CH_2S(O)CH_3$ | $CF_3$ | (L-10b) Cl |
| H | $CH(CH_3)CH_2S(O)CH_3$ | $CF_3$ | (L-10b) Br |
| H | $CH(CH_3)CH_2S(O)CH_3$ | $CF_3$ | (L-15b) $CF_3$ |
| H | $CH(CH_3)CH_2S(O)CH_3$ | $CF_3$ | (L-16a) $CHF_2$ |
| H | $CH(CH_3)CH_2S(O)CH_3$ | $CF_3$ | (L-16a) $CF_2Br$ |
| H | $CH(CH_3)CH_2S(O)CH_3$ | $CF_3$ | (L-17a) Cl |
| H | $CH(CH_3)CH_2S(O)CH_3$ | $CF_3$ | (L-21b) Cl |
| H | $CH(CH_3)CH_2S(O)CH_3$ | $CF_3$ | (L-21b) Br |
| H | $CH(CH_3)CH_2S(O)CH_3$ | $CF_3$ | (L-21b) $CF_3$ |
| H | $CH(CH_3)CH_2S(O)CH_3$ | $CF_3$ | (L-22b) Cl |
| H | $CH(CH_3)CH_2S(O)CH_3$ | $CF_3$ | (L-22b) Br |
| H | $CH(CH_3)CH_2S(O)CH_3$ | $CF_3$ | (L-23b) Cl |
| H | $CH(CH_3)CH_2S(O)CH_3$ | $CF_3$ | (L-23b) Br |
| H | $CH(CH_3)CH_2S(O)CH_3$ | $CF_3$ | (L-23c) Cl |
| H | $CH(CH_3)CH_2S(O)CH_3$ | $CF_3$ | (L-23c) Br |
| H | $CH(CH_3)CH_2S(O)CH_3$ | $CF_3$ | (L-31a) Cl |
| H | $CH(CH_3)CH_2S(O)CH_3$ | $CF_3$ | (L-31a) Br |
| H | $CH(CH_3)CH_2S(O)CH_3$ | $CF_3$ | (L-45c) F |
| H | $CH(CH_3)CH_2S(O)CH_3$ | $CF_3$ | (L-45c) Cl |
| H | $CH(CH_3)CH_2S(O)CH_3$ | $CF_3$ | (L-45c) Br |
| H | $CH(CH_3)CH_2S(O)CH_3$ | $CF_3$ | (L-45c) $CF_3$ |
| H | $CH(CH_3)CH_2S(O)CH_3$ | $CF_3$ | L-45e |
| H | $CH(CH_3)CH_2S(O)CH_3$ | $CF_3$ | L-45f |
| H | $CH(CH_3)CH_2S(O)CH_3$ | $CF_3$ | (L-46c) F |
| H | $CH(CH_3)CH_2S(O)CH_3$ | $CF_3$ | (L-46c) Cl |
| H | $CH(CH_3)CH_2S(O)CH_3$ | $CF_3$ | (L-46c) Br |
| H | $CH(CH_3)CH_2S(O)CH_3$ | $CF_3$ | (L-46c) $CF_3$ |
| H | $CH(CH_3)CH_2S(O)CH_3$ | $CF_3$ | L-46d |
| H | $CH(CH_3)CH_2S(O)CH_3$ | $CF_3$ | L-47a |
| H | $CH(CH_3)CH_2S(O)CH_3$ | $CF_3$ | L-47d |
| H | $CH(CH_3)CH_2S(O)CH_3$ | $CF_3$ | (L-48b) Br |
| H | $CH(CH_3)CH_2S(O)CH_3$ | $CF_3$ | (L-50b) Cl |
| H | $CH(CH_3)CH_2S(O)CH_3$ | $CF_3$ | (L-50b) Br |
| H | $CH(CH_3)CH_2S(O)CH_3$ | $CF_3$ | (L-51b) Cl |
| H | $CH(CH_3)CH_2SO_2CH_3$ | $CH_3$ | $CF_2O$ (Ph-4-Cl) |
| H | $CH(CH_3)CH_2SO_2CH_3$ | $CH_3$ | CH=CH (Ph-4-Cl) |
| H | $CH(CH_3)CH_2SO_2CH_3$ | $CH_3$ | CH=CH (Ph-4-$OCF_3$) |

| | | | |
|---|---|---|---|
| H | $CH(CH_3)CH_2SO_2CH_3$ | $CH_3$ | CH=CH(Ph-3, 4-$Cl_2$) |
| H | $CH(CH_3)CH_2SO_2CH_3$ | $CH_3$ | Ph-4-F |
| H | $CH(CH_3)CH_2SO_2CH_3$ | $CH_3$ | Ph-4-Cl |
| H | $CH(CH_3)CH_2SO_2CH_3$ | $CH_3$ | Ph-4-Br |
| H | $CH(CH_3)CH_2SO_2CH_3$ | $CH_3$ | Ph-4-I |
| H | $CH(CH_3)CH_2SO_2CH_3$ | $CH_3$ | Ph-4-$CF_3$ |
| H | $CH(CH_3)CH_2SO_2CH_3$ | $CH_3$ | Ph-4-$OCHF_2$ |
| H | $CH(CH_3)CH_2SO_2CH_3$ | $CH_3$ | Ph-4-$OCF_3$ |
| H | $CH(CH_3)CH_2SO_2CH_3$ | $CH_3$ | Ph-4-$OCF_2Br$ |
| H | $CH(CH_3)CH_2SO_2CH_3$ | $CH_3$ | Ph-4-$OCF_2CHF_2$ |
| H | $CH(CH_3)CH_2SO_2CH_3$ | $CH_3$ | Ph-4-$OCF_2CHFCl$ |
| H | $CH(CH_3)CH_2SO_2CH_3$ | $CH_3$ | Ph-4-$OCF_2CHFCF_3$ |
| H | $CH(CH_3)CH_2SO_2CH_3$ | $CH_3$ | Ph-4-$OCF_2CHFOCF_3$ |
| H | $CH(CH_3)CH_2SO_2CH_3$ | $CH_3$ | Ph-4-$OCF_2CHFOCF_2CF_2CF_3$ |
| H | $CH(CH_3)CH_2SO_2CH_3$ | $CH_3$ | Ph-4-O(L-45g) |
| H | $CH(CH_3)CH_2SO_2CH_3$ | $CH_3$ | Ph-3, 4-$Cl_2$ |
| H | $CH(CH_3)CH_2SO_2CH_3$ | $CH_3$ | Ph(-3-$OCF_2O$-4-) |
| H | $CH(CH_3)CH_2SO_2CH_3$ | $CH_3$ | Ph(-3-$OCF_2CF_2O$-4-) |
| H | $CH(CH_3)CH_2SO_2CH_3$ | $CH_3$ | (L-45c)Cl |
| H | $CH(CH_3)CH_2SO_2CH_3$ | $CH_3$ | (L-45c)Br |
| H | $CH(CH_3)CH_2SO_2CH_3$ | $CH_3$ | (L-45c)$CF_3$ |
| H | $CH(CH_3)CH_2SO_2CH_3$ | $CH_3$ | L-45e |
| H | $CH(CH_3)CH_2SO_2CH_3$ | $CH_3$ | L-45f |
| H | $CH(CH_3)CH_2SO_2CH_3$ | $CH_3$ | (L-46c)Cl |
| H | $CH(CH_3)CH_2SO_2CH_3$ | $CH_3$ | (L-46c)Br |
| H | $CH(CH_3)CH_2SO_2CH_3$ | $CH_3$ | (L-46c)$CF_3$ |
| H | $CH(CH_3)CH_2SO_2CH_3$ | $CH_3$ | L-46d |
| H | $CH(CH_3)CH_2SO_2CH_3$ | Et | Ph-4-Cl |
| H | $CH(CH_3)CH_2SO_2CH_3$ | Et | Ph-4-Br |
| H | $CH(CH_3)CH_2SO_2CH_3$ | Et | Ph-4-$CF_3$ |
| H | $CH(CH_3)CH_2SO_2CH_3$ | Et | Ph-4-$OCF_3$ |
| H | $CH(CH_3)CH_2SO_2CH_3$ | Et | Ph-4-$OCF_2Br$ |
| H | $CH(CH_3)CH_2SO_2CH_3$ | Et | Ph-4-$OCF_2CHFCF_3$ |
| H | $CH(CH_3)CH_2SO_2CH_3$ | Et | Ph-4-$OCF_2CHFOCF_3$ |
| H | $CH(CH_3)CH_2SO_2CH_3$ | Et | Ph-4-$OCF_2CHFOCF_2CF_2CF_3$ |
| H | $CH(CH_3)CH_2SO_2CH_3$ | Et | Ph-4-O(L-45g) |
| H | $CH(CH_3)CH_2SO_2CH_3$ | Et | Ph-3, 4-$Cl_2$ |
| H | $CH(CH_3)CH_2SO_2CH_3$ | Et | Ph(-3-$OCF_2O$-4-) |
| H | $CH(CH_3)CH_2SO_2CH_3$ | Et | Ph(-3-$OCF_2CF_2O$-4-) |
| H | $CH(CH_3)CH_2SO_2CH_3$ | Et | (L-45c)$CF_3$ |
| H | $CH(CH_3)CH_2SO_2CH_3$ | n-Pr | Ph-4-Br |

| | | | |
|---|---|---|---|
| H | $CH(CH_3)CH_2SO_2CH_3$ | n-Pr | Ph-4-$CF_3$ |
| H | $CH(CH_3)CH_2SO_2CH_3$ | n-Pr | Ph-4-$OCF_3$ |
| H | $CH(CH_3)CH_2SO_2CH_3$ | n-Pr | (L-45c) $CF_3$ |
| H | $CH(CH_3)CH_2SO_2CH_3$ | i-Pr | Ph-4-Br |
| H | $CH(CH_3)CH_2SO_2CH_3$ | i-Pr | Ph-4-$CF_3$ |
| H | $CH(CH_3)CH_2SO_2CH_3$ | i-Pr | Ph-4-$OCF_3$ |
| H | $CH(CH_3)CH_2SO_2CH_3$ | i-Pr | (L-45c) $CF_3$ |
| H | $CH(CH_3)CH_2SO_2CH_3$ | c-Pr | Ph-4-Br |
| H | $CH(CH_3)CH_2SO_2CH_3$ | c-Pr | Ph-4-$CF_3$ |
| H | $CH(CH_3)CH_2SO_2CH_3$ | c-Pr | Ph-4-$OCF_3$ |
| H | $CH(CH_3)CH_2SO_2CH_3$ | c-Pr | (L-45c) $CF_3$ |
| H | $CH(CH_3)CH_2SO_2CH_3$ | n-Bu | (L-45c) $CF_3$ |
| H | $CH(CH_3)CH_2SO_2CH_3$ | $CH_2F$ | Ph-4-Br |
| H | $CH(CH_3)CH_2SO_2CH_3$ | $CH_2Cl$ | Ph-4-$CF_3$ |
| H | $CH(CH_3)CH_2SO_2CH_3$ | $CH_2Br$ | Ph-4-$OCF_3$ |
| H | $CH(CH_3)CH_2SO_2CH_3$ | $CHF_2$ | Ph-4-Cl |
| H | $CH(CH_3)CH_2SO_2CH_3$ | $CHF_2$ | Ph-4-Br |
| H | $CH(CH_3)CH_2SO_2CH_3$ | $CHF_2$ | Ph-4-$CF_3$ |
| H | $CH(CH_3)CH_2SO_2CH_3$ | $CHF_2$ | Ph-4-$OCF_3$ |
| H | $CH(CH_3)CH_2SO_2CH_3$ | $CHF_2$ | Ph-4-$OCF_2Br$ |
| H | $CH(CH_3)CH_2SO_2CH_3$ | $CHF_2$ | Ph-4-$OCF_2CHFCF_3$ |
| H | $CH(CH_3)CH_2SO_2CH_3$ | $CHF_2$ | Ph-4-$OCF_2CHFOCF_3$ |
| H | $CH(CH_3)CH_2SO_2CH_3$ | $CHF_2$ | Ph-4-$OCF_2CHFOCF_2CF_2CF_3$ |
| H | $CH(CH_3)CH_2SO_2CH_3$ | $CHF_2$ | Ph-4-O (L-45g) |
| H | $CH(CH_3)CH_2SO_2CH_3$ | $CHF_2$ | Ph-3, 4-$Cl_2$ |
| H | $CH(CH_3)CH_2SO_2CH_3$ | $CHF_2$ | Ph (-3-$OCF_2O$-4-) |
| H | $CH(CH_3)CH_2SO_2CH_3$ | $CHF_2$ | Ph (-3-$OCF_2CF_2O$-4-) |
| H | $CH(CH_3)CH_2SO_2CH_3$ | $CHF_2$ | (L-45c) $CF_3$ |
| H | $CH(CH_3)CH_2SO_2CH_3$ | $CHF_2$ | L-45f |
| H | $CH(CH_3)CH_2SO_2CH_3$ | $CHF_2$ | (L-46c) $CF_3$ |
| H | $CH(CH_3)CH_2SO_2CH_3$ | $CHF_2$ | L-46d |
| H | $CH(CH_3)CH_2SO_2CH_3$ | CHFCl | (L-45c) $CF_3$ |
| H | $CH(CH_3)CH_2SO_2CH_3$ | CHFBr | Ph-4-Br |
| H | $CH(CH_3)CH_2SO_2CH_3$ | $CF_3$ | c-Pr |
| H | $CH(CH_3)CH_2SO_2CH_3$ | $CF_3$ | c-Bu |
| H | $CH(CH_3)CH_2SO_2CH_3$ | $CF_3$ | c-Pen |
| H | $CH(CH_3)CH_2SO_2CH_3$ | $CF_3$ | c-Hex |
| H | $CH(CH_3)CH_2SO_2CH_3$ | $CF_3$ | T-1 |
| H | $CH(CH_3)CH_2SO_2CH_3$ | $CF_3$ | T-2 |
| H | $CH(CH_3)CH_2SO_2CH_3$ | $CF_3$ | T-3 |
| H | $CH(CH_3)CH_2SO_2CH_3$ | $CF_3$ | T-4 |

| | | | |
|---|---|---|---|
| H | $CH(CH_3)CH_2SO_2CH_3$ | $CF_3$ | T-5 |
| H | $CH(CH_3)CH_2SO_2CH_3$ | $CF_3$ | $CH_2OCH_3$ |
| H | $CH(CH_3)CH_2SO_2CH_3$ | $CF_3$ | $CH_2OEt$ |
| H | $CH(CH_3)CH_2SO_2CH_3$ | $CF_3$ | $CH_2OPr$-n |
| H | $CH(CH_3)CH_2SO_2CH_3$ | $CF_3$ | $CH_2OPr$-i |
| H | $CH(CH_3)CH_2SO_2CH_3$ | $CF_3$ | $CH_2OBu$-n |
| H | $CH(CH_3)CH_2SO_2CH_3$ | $CF_3$ | $CH_2OC(O)$ (Ph-2-Cl) |
| H | $CH(CH_3)CH_2SO_2CH_3$ | $CF_3$ | $CH_2OC(O)$ (Ph-3-Cl) |
| H | $CH(CH_3)CH_2SO_2CH_3$ | $CF_3$ | $CH_2OC(O)$ (Ph-4-Cl) |
| H | $CH(CH_3)CH_2SO_2CH_3$ | $CF_3$ | $CH_2OPh$ |
| H | $CH(CH_3)CH_2SO_2CH_3$ | $CF_3$ | $CH_2O$(Ph-2-F) |
| H | $CH(CH_3)CH_2SO_2CH_3$ | $CF_3$ | $CH_2O$(Ph-3-F) |
| H | $CH(CH_3)CH_2SO_2CH_3$ | $CF_3$ | $CH_2O$(Ph-4-F) |
| H | $CH(CH_3)CH_2SO_2CH_3$ | $CF_3$ | $CH_2O$(Ph-2-Cl) |
| H | $CH(CH_3)CH_2SO_2CH_3$ | $CF_3$ | $CH_2O$(Ph-3-Cl) |
| H | $CH(CH_3)CH_2SO_2CH_3$ | $CF_3$ | $CH_2O$(Ph-4-Cl) |
| H | $CH(CH_3)CH_2SO_2CH_3$ | $CF_3$ | $CH_2O$(Ph-2-Br) |
| H | $CH(CH_3)CH_2SO_2CH_3$ | $CF_3$ | $CH_2O$(Ph-3-Br) |
| H | $CH(CH_3)CH_2SO_2CH_3$ | $CF_3$ | $CH_2O$(Ph-4-Br) |
| H | $CH(CH_3)CH_2SO_2CH_3$ | $CF_3$ | $CH_2O$(Ph-2-$CF_3$) |
| H | $CH(CH_3)CH_2SO_2CH_3$ | $CF_3$ | $CH_2O$(Ph-3-$CF_3$) |
| H | $CH(CH_3)CH_2SO_2CH_3$ | $CF_3$ | $CH_2O$(Ph-4-$CF_3$) |
| H | $CH(CH_3)CH_2SO_2CH_3$ | $CF_3$ | $CH_2O$(Ph-2-$OCF_3$) |
| H | $CH(CH_3)CH_2SO_2CH_3$ | $CF_3$ | $CH_2O$(Ph-3-$OCF_3$) |
| H | $CH(CH_3)CH_2SO_2CH_3$ | $CF_3$ | $CH_2O$(Ph-4-$OCF_3$) |
| H | $CH(CH_3)CH_2SO_2CH_3$ | $CF_3$ | $CF_2OPh$ |
| H | $CH(CH_3)CH_2SO_2CH_3$ | $CF_3$ | $CF_2O$(Ph-2-F) |
| H | $CH(CH_3)CH_2SO_2CH_3$ | $CF_3$ | $CF_2O$(Ph-3-F) |
| H | $CH(CH_3)CH_2SO_2CH_3$ | $CF_3$ | $CF_2O$(Ph-4-F) |
| H | $CH(CH_3)CH_2SO_2CH_3$ | $CF_3$ | $CF_2O$(Ph-2-Cl) |
| H | $CH(CH_3)CH_2SO_2CH_3$ | $CF_3$ | $CF_2O$(Ph-3-Cl) |
| H | $CH(CH_3)CH_2SO_2CH_3$ | $CF_3$ | $CF_2O$(Ph-4-Cl) |
| H | $CH(CH_3)CH_2SO_2CH_3$ | $CF_3$ | $CF_2O$(Ph-2-Br) |
| H | $CH(CH_3)CH_2SO_2CH_3$ | $CF_3$ | $CF_2O$(Ph-3-Br) |
| H | $CH(CH_3)CH_2SO_2CH_3$ | $CF_3$ | $CF_2O$(Ph-4-Br) |
| H | $CH(CH_3)CH_2SO_2CH_3$ | $CF_3$ | $CF_2O$(Ph-2-$CF_3$) |
| H | $CH(CH_3)CH_2SO_2CH_3$ | $CF_3$ | $CF_2O$(Ph-3-$CF_3$) |
| H | $CH(CH_3)CH_2SO_2CH_3$ | $CF_3$ | $CF_2O$(Ph-4-$CF_3$) |
| H | $CH(CH_3)CH_2SO_2CH_3$ | $CF_3$ | $CF_2O$(Ph-2-$OCF_3$) |
| H | $CH(CH_3)CH_2SO_2CH_3$ | $CF_3$ | $CF_2O$(Ph-3-$OCF_3$) |
| H | $CH(CH_3)CH_2SO_2CH_3$ | $CF_3$ | $CF_2O$(Ph-4-$OCF_3$) |

| | | | |
|---|---|---|---|
| H | $CH(CH_3)CH_2SO_2CH_3$ | $CF_3$ | $CH_2S(Ph-2-Cl)$ |
| H | $CH(CH_3)CH_2SO_2CH_3$ | $CF_3$ | $CH_2S(Ph-3-Cl)$ |
| H | $CH(CH_3)CH_2SO_2CH_3$ | $CF_3$ | $CH_2S(Ph-4-Cl)$ |
| H | $CH(CH_3)CH_2SO_2CH_3$ | $CF_3$ | $CH_2SO_2(Ph-2-Cl)$ |
| H | $CH(CH_3)CH_2SO_2CH_3$ | $CF_3$ | $CH_2SO_2(Ph-3-Cl)$ |
| H | $CH(CH_3)CH_2SO_2CH_3$ | $CF_3$ | $CH_2SO_2(Ph-4-Cl)$ |
| H | $CH(CH_3)CH_2SO_2CH_3$ | $CF_3$ | $CH_2N(CH_3)_2$ |
| H | $CH(CH_3)CH_2SO_2CH_3$ | $CF_3$ | $CH_2NH(Ph-2-Cl)$ |
| H | $CH(CH_3)CH_2SO_2CH_3$ | $CF_3$ | $CH_2NH(Ph-3-Cl)$ |
| H | $CH(CH_3)CH_2SO_2CH_3$ | $CF_3$ | $CH_2NH(Ph-4-Cl)$ |
| H | $CH(CH_3)CH_2SO_2CH_3$ | $CF_3$ | $CF_2C(O)OEt$ |
| H | $CH(CH_3)CH_2SO_2CH_3$ | $CF_3$ | $CH_2(L-5a)$ |
| H | $CH(CH_3)CH_2SO_2CH_3$ | $CF_3$ | $CH_2(L-14a)$ |
| H | $CH(CH_3)CH_2SO_2CH_3$ | $CF_3$ | $CH_2(L-24a)$ |
| H | $CH(CH_3)CH_2SO_2CH_3$ | $CF_3$ | $CH_2(L-36a)$ |
| H | $CH(CH_3)CH_2SO_2CH_3$ | $CF_3$ | $CH_2CH_2Ph$ |
| H | $CH(CH_3)CH_2SO_2CH_3$ | $CF_3$ | $CH_2CH_2(Ph-2-Cl)$ |
| H | $CH(CH_3)CH_2SO_2CH_3$ | $CF_3$ | $CH_2CH_2(Ph-3-Cl)$ |
| H | $CH(CH_3)CH_2SO_2CH_3$ | $CF_3$ | $CH_2CH_2(Ph-4-Cl)$ |
| H | $CH(CH_3)CH_2SO_2CH_3$ | $CF_3$ | T-22 |
| H | $CH(CH_3)CH_2SO_2CH_3$ | $CF_3$ | T-23 |
| H | $CH(CH_3)CH_2SO_2CH_3$ | $CF_3$ | T-24 |
| H | $CH(CH_3)CH_2SO_2CH_3$ | $CF_3$ | C(O)OEt |
| H | $CH(CH_3)CH_2SO_2CH_3$ | $CF_3$ | C(O)OBu-t |
| H | $CH(CH_3)CH_2SO_2CH_3$ | $CF_3$ | $C(O)OCH_2CF_3$ |
| H | $CH(CH_3)CH_2SO_2CH_3$ | $CF_3$ | CH=CH(Ph-2-F) |
| H | $CH(CH_3)CH_2SO_2CH_3$ | $CF_3$ | CH=CH(Ph-3-F) |
| H | $CH(CH_3)CH_2SO_2CH_3$ | $CF_3$ | CH=CH(Ph-4-F) |
| H | $CH(CH_3)CH_2SO_2CH_3$ | $CF_3$ | CH=CH(Ph-2-Cl) |
| H | $CH(CH_3)CH_2SO_2CH_3$ | $CF_3$ | CH=CH(Ph-3-Cl) |
| H | $CH(CH_3)CH_2SO_2CH_3$ | $CF_3$ | CH=CH(Ph-4-Cl) |
| H | $CH(CH_3)CH_2SO_2CH_3$ | $CF_3$ | CH=CH(Ph-2-Br) |
| H | $CH(CH_3)CH_2SO_2CH_3$ | $CF_3$ | CH=CH(Ph-3-Br) |
| H | $CH(CH_3)CH_2SO_2CH_3$ | $CF_3$ | CH=CH(Ph-4-Br) |
| H | $CH(CH_3)CH_2SO_2CH_3$ | $CF_3$ | $CH=CH(Ph-2-CF_3)$ |
| H | $CH(CH_3)CH_2SO_2CH_3$ | $CF_3$ | $CH=CH(Ph-3-CF_3)$ |
| H | $CH(CH_3)CH_2SO_2CH_3$ | $CF_3$ | $CH=CH(Ph-4-CF_3)$ |
| H | $CH(CH_3)CH_2SO_2CH_3$ | $CF_3$ | $CH=CH(Ph-2-OCF_3)$ |
| H | $CH(CH_3)CH_2SO_2CH_3$ | $CF_3$ | $CH=CH(Ph-3-OCF_3)$ |
| H | $CH(CH_3)CH_2SO_2CH_3$ | $CF_3$ | $CH=CH(Ph-4-OCF_3)$ |
| H | $CH(CH_3)CH_2SO_2CH_3$ | $CF_3$ | $CH=CH(Ph-2-SCH_3)$ |

| | | | |
|---|---|---|---|
| H | $CH(CH_3)CH_2SO_2CH_3$ | $CF_3$ | $CH=CH(Ph-3-SCH_3)$ |
| H | $CH(CH_3)CH_2SO_2CH_3$ | $CF_3$ | $CH=CH(Ph-4-SCH_3)$ |
| H | $CH(CH_3)CH_2SO_2CH_3$ | $CF_3$ | $CH=CH(Ph-2-SO_2CH_3)$ |
| H | $CH(CH_3)CH_2SO_2CH_3$ | $CF_3$ | $CH=CH(Ph-3-SO_2CH_3)$ |
| H | $CH(CH_3)CH_2SO_2CH_3$ | $CF_3$ | $CH=CH(Ph-4-SO_2CH_3)$ |
| H | $CH(CH_3)CH_2SO_2CH_3$ | $CF_3$ | $CH=CH(Ph-3,4-F_2)$ |
| H | $CH(CH_3)CH_2SO_2CH_3$ | $CF_3$ | $CH=CH(Ph-3-F-4-Cl)$ |
| H | $CH(CH_3)CH_2SO_2CH_3$ | $CF_3$ | $CH=CH(Ph-3,4-Cl_2)$ |
| H | $CH(CH_3)CH_2SO_2CH_3$ | $CF_3$ | $CH=CH(Ph-3,4-Br_2)$ |
| H | $CH(CH_3)CH_2SO_2CH_3$ | $CF_3$ | $CH=CH(Ph-3-F-4-CF_3)$ |
| H | $CH(CH_3)CH_2SO_2CH_3$ | $CF_3$ | $CH=CH(Ph-3-Cl-4-OCF_3)$ |
| H | $CH(CH_3)CH_2SO_2CH_3$ | $CF_3$ | $CH=CH[Ph(-3-OCF_2O-4-)]$ |
| H | $CH(CH_3)CH_2SO_2CH_3$ | $CF_3$ | Ph |
| H | $CH(CH_3)CH_2SO_2CH_3$ | $CF_3$ | Ph-2-F |
| H | $CH(CH_3)CH_2SO_2CH_3$ | $CF_3$ | Ph-3-F |
| H | $CH(CH_3)CH_2SO_2CH_3$ | $CF_3$ | Ph-4-F |
| H | $CH(CH_3)CH_2SO_2CH_3$ | $CF_3$ | Ph-2-Cl |
| H | $CH(CH_3)CH_2SO_2CH_3$ | $CF_3$ | Ph-3-Cl |
| H | $CH(CH_3)CH_2SO_2CH_3$ | $CF_3$ | Ph-4-Cl |
| H | $CH(CH_3)CH_2SO_2CH_3$ | $CF_3$ | Ph-2-Br |
| H | $CH(CH_3)CH_2SO_2CH_3$ | $CF_3$ | Ph-3-Br |
| H | $CH(CH_3)CH_2SO_2CH_3$ | $CF_3$ | Ph-4-Br |
| H | $CH(CH_3)CH_2SO_2CH_3$ | $CF_3$ | Ph-4-I |
| H | $CH(CH_3)CH_2SO_2CH_3$ | $CF_3$ | Ph-4-Bu-t |
| H | $CH(CH_3)CH_2SO_2CH_3$ | $CF_3$ | $Ph-2-CF_3$ |
| H | $CH(CH_3)CH_2SO_2CH_3$ | $CF_3$ | $Ph-3-CF_3$ |
| H | $CH(CH_3)CH_2SO_2CH_3$ | $CF_3$ | $Ph-4-CF_3$ |
| H | $CH(CH_3)CH_2SO_2CH_3$ | $CF_3$ | $Ph-4-OCH_3$ |
| H | $CH(CH_3)CH_2SO_2CH_3$ | $CF_3$ | $Ph-4-OCHF_2$ |
| H | $CH(CH_3)CH_2SO_2CH_3$ | $CF_3$ | $Ph-2-OCF_3$ |
| H | $CH(CH_3)CH_2SO_2CH_3$ | $CF_3$ | $Ph-3-OCF_3$ |
| H | $CH(CH_3)CH_2SO_2CH_3$ | $CF_3$ | $Ph-4-OCF_3$ |
| H | $CH(CH_3)CH_2SO_2CH_3$ | $CF_3$ | $Ph-4-OCF_2Br$ |
| H | $CH(CH_3)CH_2SO_2CH_3$ | $CF_3$ | $Ph-4-OCH_2CF_3$ |
| H | $CH(CH_3)CH_2SO_2CH_3$ | $CF_3$ | $Ph-4-OCF_2CHF_2$ |
| H | $CH(CH_3)CH_2SO_2CH_3$ | $CF_3$ | $Ph-4-OCF_2CHFCl$ |
| H | $CH(CH_3)CH_2SO_2CH_3$ | $CF_3$ | $Ph-4-OCF_2CHFBr$ |
| H | $CH(CH_3)CH_2SO_2CH_3$ | $CF_3$ | $Ph-4-OCF_2CF_2Br$ |
| H | $CH(CH_3)CH_2SO_2CH_3$ | $CF_3$ | $Ph-4-OCF_2CFCl_2$ |
| H | $CH(CH_3)CH_2SO_2CH_3$ | $CF_3$ | $Ph-4-OCF_2CCl_3$ |
| H | $CH(CH_3)CH_2SO_2CH_3$ | $CF_3$ | $Ph-4-OCH_2CF_2CHF_2$ |

| | | | |
|---|---|---|---|
| H | $CH(CH_3)CH_2SO_2CH_3$ | $CF_3$ | Ph-3-$OCF_2CHFCF_3$ |
| H | $CH(CH_3)CH_2SO_2CH_3$ | $CF_3$ | Ph-4-$OCF_2CHFCF_3$ |
| H | $CH(CH_3)CH_2SO_2CH_3$ | $CF_3$ | Ph-4-$OCH(CF_3)_2$ |
| H | $CH(CH_3)CH_2SO_2CH_3$ | $CF_3$ | Ph-4-$OCF_2CFBrCF_3$ |
| H | $CH(CH_3)CH_2SO_2CH_3$ | $CF_3$ | Ph-3-$OCF_2CHFOCF_3$ |
| H | $CH(CH_3)CH_2SO_2CH_3$ | $CF_3$ | Ph-4-$OCF_2CHFOCF_3$ |
| H | $CH(CH_3)CH_2SO_2CH_3$ | $CF_3$ | Ph-4-$OCF_2CHFOCF_2CF_2CF_3$ |
| H | $CH(CH_3)CH_2SO_2CH_3$ | $CF_3$ | Ph-4-$OSO_2CH_3$ |
| H | $CH(CH_3)CH_2SO_2CH_3$ | $CF_3$ | Ph-4-$OSO_2CF_3$ |
| H | $CH(CH_3)CH_2SO_2CH_3$ | $CF_3$ | Ph-3-O(Ph-4-Cl) |
| H | $CH(CH_3)CH_2SO_2CH_3$ | $CF_3$ | Ph-4-O(Ph-4-Cl) |
| H | $CH(CH_3)CH_2SO_2CH_3$ | $CF_3$ | Ph-4-O(Ph-4-Br) |
| H | $CH(CH_3)CH_2SO_2CH_3$ | $CF_3$ | Ph-4-O(Ph-4-$CF_3$) |
| H | $CH(CH_3)CH_2SO_2CH_3$ | $CF_3$ | Ph-4-O(L-45c)Br |
| H | $CH(CH_3)CH_2SO_2CH_3$ | $CF_3$ | Ph-4-O(L-45c)$CF_3$ |
| H | $CH(CH_3)CH_2SO_2CH_3$ | $CF_3$ | Ph-3-O(L-45e) |
| H | $CH(CH_3)CH_2SO_2CH_3$ | $CF_3$ | Ph-4-O(L-45e) |
| H | $CH(CH_3)CH_2SO_2CH_3$ | $CF_3$ | Ph-4-O(L-48b)Br |
| H | $CH(CH_3)CH_2SO_2CH_3$ | $CF_3$ | Ph-4-$SCH_3$ |
| H | $CH(CH_3)CH_2SO_2CH_3$ | $CF_3$ | Ph-4-$SO_2CH_3$ |
| H | $CH(CH_3)CH_2SO_2CH_3$ | $CF_3$ | Ph-3-S(Ph-4-Cl) |
| H | $CH(CH_3)CH_2SO_2CH_3$ | $CF_3$ | Ph-4-S(Ph-4-Cl) |
| H | $CH(CH_3)CH_2SO_2CH_3$ | $CF_3$ | Ph-4-S(Ph-4-Br) |
| H | $CH(CH_3)CH_2SO_2CH_3$ | $CF_3$ | Ph-4-S(Ph-4-$CF_3$) |
| H | $CH(CH_3)CH_2SO_2CH_3$ | $CF_3$ | Ph-4-S(L-45c)Br |
| H | $CH(CH_3)CH_2SO_2CH_3$ | $CF_3$ | Ph-4-S(L-45c)$CF_3$ |
| H | $CH(CH_3)CH_2SO_2CH_3$ | $CF_3$ | Ph-4-S(L-45e) |
| H | $CH(CH_3)CH_2SO_2CH_3$ | $CF_3$ | Ph-4-S(L-48b)Br |
| H | $CH(CH_3)CH_2SO_2CH_3$ | $CF_3$ | Ph-4-$NO_2$ |
| H | $CH(CH_3)CH_2SO_2CH_3$ | $CF_3$ | Ph-4-CN |
| H | $CH(CH_3)CH_2SO_2CH_3$ | $CF_3$ | Ph-2,3-$F_2$ |
| H | $CH(CH_3)CH_2SO_2CH_3$ | $CF_3$ | Ph-2,4-$F_2$ |
| H | $CH(CH_3)CH_2SO_2CH_3$ | $CF_3$ | Ph-3,4-$F_2$ |
| H | $CH(CH_3)CH_2SO_2CH_3$ | $CF_3$ | Ph-2,5-$F_2$ |
| H | $CH(CH_3)CH_2SO_2CH_3$ | $CF_3$ | Ph-3,5-$F_2$ |
| H | $CH(CH_3)CH_2SO_2CH_3$ | $CF_3$ | Ph-2-Cl-4-F |
| H | $CH(CH_3)CH_2SO_2CH_3$ | $CF_3$ | Ph-2-F-3-Cl |
| H | $CH(CH_3)CH_2SO_2CH_3$ | $CF_3$ | Ph-3-Cl-4-F |
| H | $CH(CH_3)CH_2SO_2CH_3$ | $CF_3$ | Ph-2-F-4-Cl |
| H | $CH(CH_3)CH_2SO_2CH_3$ | $CF_3$ | Ph-3-F-4-Cl |
| H | $CH(CH_3)CH_2SO_2CH_3$ | $CF_3$ | Ph-2,3-$Cl_2$ |

| | | | |
|---|---|---|---|
| H | $CH(CH_3)CH_2SO_2CH_3$ | $CF_3$ | Ph-2, 4-$Cl_2$ |
| H | $CH(CH_3)CH_2SO_2CH_3$ | $CF_3$ | Ph-2, 5-$Cl_2$ |
| H | $CH(CH_3)CH_2SO_2CH_3$ | $CF_3$ | Ph-3, 4-$Cl_2$ |
| H | $CH(CH_3)CH_2SO_2CH_3$ | $CF_3$ | Ph-3, 5-$Cl_2$ |
| H | $CH(CH_3)CH_2SO_2CH_3$ | $CF_3$ | Ph-3-Br-4-F |
| H | $CH(CH_3)CH_2SO_2CH_3$ | $CF_3$ | Ph-2-F-4-Br |
| H | $CH(CH_3)CH_2SO_2CH_3$ | $CF_3$ | Ph-2-F-5-Br |
| H | $CH(CH_3)CH_2SO_2CH_3$ | $CF_3$ | Ph-3, 4-$Br_2$ |
| H | $CH(CH_3)CH_2SO_2CH_3$ | $CF_3$ | Ph-3, 5-$Br_2$ |
| H | $CH(CH_3)CH_2SO_2CH_3$ | $CF_3$ | Ph-3-$CH_3$-4-F |
| H | $CH(CH_3)CH_2SO_2CH_3$ | $CF_3$ | Ph-3-F-4-$CH_3$ |
| H | $CH(CH_3)CH_2SO_2CH_3$ | $CF_3$ | Ph-2-F-5-$CH_3$ |
| H | $CH(CH_3)CH_2SO_2CH_3$ | $CF_3$ | Ph-2, 4-$(CH_3)_2$ |
| H | $CH(CH_3)CH_2SO_2CH_3$ | $CF_3$ | Ph-3, 4-$(CH_3)_2$ |
| H | $CH(CH_3)CH_2SO_2CH_3$ | $CF_3$ | Ph-2-F-3-$CF_3$ |
| H | $CH(CH_3)CH_2SO_2CH_3$ | $CF_3$ | Ph-3-$CF_3$-4-F |
| H | $CH(CH_3)CH_2SO_2CH_3$ | $CF_3$ | Ph-3-$CF_3$-4-Cl |
| H | $CH(CH_3)CH_2SO_2CH_3$ | $CF_3$ | Ph-2-F-4-$CF_3$ |
| H | $CH(CH_3)CH_2SO_2CH_3$ | $CF_3$ | Ph-3-F-4-$CF_3$ |
| H | $CH(CH_3)CH_2SO_2CH_3$ | $CF_3$ | Ph-2-F-5-$CF_3$ |
| H | $CH(CH_3)CH_2SO_2CH_3$ | $CF_3$ | Ph-3-F-5-$CF_3$ |
| H | $CH(CH_3)CH_2SO_2CH_3$ | $CF_3$ | Ph-2-Cl-4-$CF_3$ |
| H | $CH(CH_3)CH_2SO_2CH_3$ | $CF_3$ | Ph-3, 5-$(CF_3)_2$ |
| H | $CH(CH_3)CH_2SO_2CH_3$ | $CF_3$ | Ph-3-Br-4-$OCH_3$ |
| H | $CH(CH_3)CH_2SO_2CH_3$ | $CF_3$ | Ph-3-F-4-$OCHF_2$ |
| H | $CH(CH_3)CH_2SO_2CH_3$ | $CF_3$ | Ph-3-Cl-4-$OCHF_2$ |
| H | $CH(CH_3)CH_2SO_2CH_3$ | $CF_3$ | Ph-3-Br-4-$OCHF_2$ |
| H | $CH(CH_3)CH_2SO_2CH_3$ | $CF_3$ | Ph-3-F-4-$OCF_3$ |
| H | $CH(CH_3)CH_2SO_2CH_3$ | $CF_3$ | Ph-3-Cl-4-$OCF_3$ |
| H | $CH(CH_3)CH_2SO_2CH_3$ | $CF_3$ | Ph-3-Br-4-$OCF_3$ |
| H | $CH(CH_3)CH_2SO_2CH_3$ | $CF_3$ | Ph-3-F-4-$OCF_2Br$ |
| H | $CH(CH_3)CH_2SO_2CH_3$ | $CF_3$ | Ph-3-Cl-4-$OCF_2Br$ |
| H | $CH(CH_3)CH_2SO_2CH_3$ | $CF_3$ | Ph-3-Br-4-$OCF_2Br$ |
| H | $CH(CH_3)CH_2SO_2CH_3$ | $CF_3$ | Ph-3-F-4-$OCF_2CHF_2$ |
| H | $CH(CH_3)CH_2SO_2CH_3$ | $CF_3$ | Ph-3-Cl-4-$OCF_2CHF_2$ |
| H | $CH(CH_3)CH_2SO_2CH_3$ | $CF_3$ | Ph-3-Br-4-$OCF_2CHF_2$ |
| H | $CH(CH_3)CH_2SO_2CH_3$ | $CF_3$ | Ph-3-F-4-$OCF_2CHFCl$ |
| H | $CH(CH_3)CH_2SO_2CH_3$ | $CF_3$ | Ph-3-Cl-4-$OCF_2CHFCl$ |
| H | $CH(CH_3)CH_2SO_2CH_3$ | $CF_3$ | Ph-3-Br-4-$OCF_2CHFCl$ |
| H | $CH(CH_3)CH_2SO_2CH_3$ | $CF_3$ | Ph-3-F-4-$OCF_2CHFCF_3$ |
| H | $CH(CH_3)CH_2SO_2CH_3$ | $CF_3$ | Ph-3-Cl-4-$OCF_2CHFCF_3$ |

| | | | |
|---|---|---|---|
| H | $CH(CH_3)CH_2SO_2CH_3$ | $CF_3$ | Ph-3-Br-4-$OCF_2CHFCF_3$ |
| H | $CH(CH_3)CH_2SO_2CH_3$ | $CF_3$ | Ph-3-F-4-$OCF_2CHFOCF_3$ |
| H | $CH(CH_3)CH_2SO_2CH_3$ | $CF_3$ | Ph-3-Cl-4-$OCF_2CHFOCF_3$ |
| H | $CH(CH_3)CH_2SO_2CH_3$ | $CF_3$ | Ph-3-Br-4-$OCF_2CHFOCF_3$ |
| H | $CH(CH_3)CH_2SO_2CH_3$ | $CF_3$ | Ph-3-$CH_3$-4-$OCF_2CHFOCF_3$ |
| H | $CH(CH_3)CH_2SO_2CH_3$ | $CF_3$ | Ph-3-F-4-$OCF_2CHFOCF_2CF_2CF_3$ |
| H | $CH(CH_3)CH_2SO_2CH_3$ | $CF_3$ | Ph-3-Cl-4-$OCF_2CHFOCF_2CF_2CF_3$ |
| H | $CH(CH_3)CH_2SO_2CH_3$ | $CF_3$ | Ph-3-Br-4-$OCF_2CHFOCF_2CF_2CF_3$ |
| H | $CH(CH_3)CH_2SO_2CH_3$ | $CF_3$ | Ph(-3-$OCF_2O$-4-) |
| H | $CH(CH_3)CH_2SO_2CH_3$ | $CF_3$ | Ph(-3-$OCF_2CF_2O$-4-) |
| H | $CH(CH_3)CH_2SO_2CH_3$ | $CF_3$ | Ph-3-OPh-4-F |
| H | $CH(CH_3)CH_2SO_2CH_3$ | $CF_3$ | Ph-3-$NO_2$-4-F |
| H | $CH(CH_3)CH_2SO_2CH_3$ | $CF_3$ | Ph-3-$NO_2$-4-Cl |
| H | $CH(CH_3)CH_2SO_2CH_3$ | $CF_3$ | Ph-2-F-5-$NO_2$ |
| H | $CH(CH_3)CH_2SO_2CH_3$ | $CF_3$ | Ph-3-CN-4-F |
| H | $CH(CH_3)CH_2SO_2CH_3$ | $CF_3$ | Ph-2, 3, 4-$F_3$ |
| H | $CH(CH_3)CH_2SO_2CH_3$ | $CF_3$ | Ph-2, 3, 5-$F_3$ |
| H | $CH(CH_3)CH_2SO_2CH_3$ | $CF_3$ | Ph-2, 4, 5-$F_3$ |
| H | $CH(CH_3)CH_2SO_2CH_3$ | $CF_3$ | Ph-3, 4, 5-$F_3$ |
| H | $CH(CH_3)CH_2SO_2CH_3$ | $CF_3$ | Ph-2, 3-$F_2$-4-$CH_3$ |
| H | $CH(CH_3)CH_2SO_2CH_3$ | $CF_3$ | Ph-2, 3-$F_2$-4-$CF_3$ |
| H | $CH(CH_3)CH_2SO_2CH_3$ | $CF_3$ | Ph-3, 4-$F_2$-5-$CF_3$ |
| H | $CH(CH_3)CH_2SO_2CH_3$ | $CF_3$ | Ph-2-F-3-Cl-5-$CF_3$ |
| H | $CH(CH_3)CH_2SO_2CH_3$ | $CF_3$ | Ph-3, 5-$Cl_2$-4-$OCH_3$ |
| H | $CH(CH_3)CH_2SO_2CH_3$ | $CF_3$ | 1-Naph |
| H | $CH(CH_3)CH_2SO_2CH_3$ | $CF_3$ | 2-Naph |
| H | $CH(CH_3)CH_2SO_2CH_3$ | $CF_3$ | (L-1b) Br |
| H | $CH(CH_3)CH_2SO_2CH_3$ | $CF_3$ | (L-1c) Cl |
| H | $CH(CH_3)CH_2SO_2CH_3$ | $CF_3$ | (L-1c) Br |
| H | $CH(CH_3)CH_2SO_2CH_3$ | $CF_3$ | (L-1c) I |
| H | $CH(CH_3)CH_2SO_2CH_3$ | $CF_3$ | (L-1c) $CF_3$ |
| H | $CH(CH_3)CH_2SO_2CH_3$ | $CF_3$ | (L-2b) Br |
| H | $CH(CH_3)CH_2SO_2CH_3$ | $CF_3$ | (L-3b) Cl |
| H | $CH(CH_3)CH_2SO_2CH_3$ | $CF_3$ | (L-3b) Br |
| H | $CH(CH_3)CH_2SO_2CH_3$ | $CF_3$ | (L-3c) F |
| H | $CH(CH_3)CH_2SO_2CH_3$ | $CF_3$ | (L-3c) Cl |
| H | $CH(CH_3)CH_2SO_2CH_3$ | $CF_3$ | (L-3c) Br |
| H | $CH(CH_3)CH_2SO_2CH_3$ | $CF_3$ | (L-3c) I |
| H | $CH(CH_3)CH_2SO_2CH_3$ | $CF_3$ | (L-3c) $CF_3$ |
| H | $CH(CH_3)CH_2SO_2CH_3$ | $CF_3$ | (L-3c) CN |
| H | $CH(CH_3)CH_2SO_2CH_3$ | $CF_3$ | L-3d |

| | | | |
|---|---|---|---|
| H | $CH(CH_3)CH_2SO_2CH_3$ | $CF_3$ | (L-4b) Cl |
| H | $CH(CH_3)CH_2SO_2CH_3$ | $CF_3$ | (L-4b) Br |
| H | $CH(CH_3)CH_2SO_2CH_3$ | $CF_3$ | (L-4b) CN |
| H | $CH(CH_3)CH_2SO_2CH_3$ | $CF_3$ | (L-10b) Cl |
| H | $CH(CH_3)CH_2SO_2CH_3$ | $CF_3$ | (L-10b) Br |
| H | $CH(CH_3)CH_2SO_2CH_3$ | $CF_3$ | (L-15b) $CF_3$ |
| H | $CH(CH_3)CH_2SO_2CH_3$ | $CF_3$ | (L-16a) $CHF_2$ |
| H | $CH(CH_3)CH_2SO_2CH_3$ | $CF_3$ | (L-16a) $CF_2Br$ |
| H | $CH(CH_3)CH_2SO_2CH_3$ | $CF_3$ | (L-17a) Cl |
| H | $CH(CH_3)CH_2SO_2CH_3$ | $CF_3$ | (L-21b) Cl |
| H | $CH(CH_3)CH_2SO_2CH_3$ | $CF_3$ | (L-21b) Br |
| H | $CH(CH_3)CH_2SO_2CH_3$ | $CF_3$ | (L-21b) I |
| H | $CH(CH_3)CH_2SO_2CH_3$ | $CF_3$ | (L-21b) $CF_3$ |
| H | $CH(CH_3)CH_2SO_2CH_3$ | $CF_3$ | (L-22b) Cl |
| H | $CH(CH_3)CH_2SO_2CH_3$ | $CF_3$ | (L-22b) Br |
| H | $CH(CH_3)CH_2SO_2CH_3$ | $CF_3$ | (L-23b) Cl |
| H | $CH(CH_3)CH_2SO_2CH_3$ | $CF_3$ | (L-23b) Br |
| H | $CH(CH_3)CH_2SO_2CH_3$ | $CF_3$ | (L-23c) Cl |
| H | $CH(CH_3)CH_2SO_2CH_3$ | $CF_3$ | (L-23c) Br |
| H | $CH(CH_3)CH_2SO_2CH_3$ | $CF_3$ | (L-31a) Cl |
| H | $CH(CH_3)CH_2SO_2CH_3$ | $CF_3$ | (L-31a) Br |
| H | $CH(CH_3)CH_2SO_2CH_3$ | $CF_3$ | (L-45c) F |
| H | $CH(CH_3)CH_2SO_2CH_3$ | $CF_3$ | (L-45c) Cl |
| H | $CH(CH_3)CH_2SO_2CH_3$ | $CF_3$ | (L-45c) Br |
| H | $CH(CH_3)CH_2SO_2CH_3$ | $CF_3$ | (L-45c) I |
| H | $CH(CH_3)CH_2SO_2CH_3$ | $CF_3$ | (L-45c) $CF_3$ |
| H | $CH(CH_3)CH_2SO_2CH_3$ | $CF_3$ | L-45e |
| H | $CH(CH_3)CH_2SO_2CH_3$ | $CF_3$ | L-45f |
| H | $CH(CH_3)CH_2SO_2CH_3$ | $CF_3$ | (L-46c) F |
| H | $CH(CH_3)CH_2SO_2CH_3$ | $CF_3$ | (L-46c) Cl |
| H | $CH(CH_3)CH_2SO_2CH_3$ | $CF_3$ | (L-46c) Br |
| H | $CH(CH_3)CH_2SO_2CH_3$ | $CF_3$ | (L-46c) I |
| H | $CH(CH_3)CH_2SO_2CH_3$ | $CF_3$ | (L-46c) $CF_3$ |
| H | $CH(CH_3)CH_2SO_2CH_3$ | $CF_3$ | (L-46c) $OCH_2CF_3$ |
| H | $CH(CH_3)CH_2SO_2CH_3$ | $CF_3$ | (L-46c) $OCH(CF_3)_2$ |
| H | $CH(CH_3)CH_2SO_2CH_3$ | $CF_3$ | L-46d |
| H | $CH(CH_3)CH_2SO_2CH_3$ | $CF_3$ | L-47a |
| H | $CH(CH_3)CH_2SO_2CH_3$ | $CF_3$ | L-47d |
| H | $CH(CH_3)CH_2SO_2CH_3$ | $CF_3$ | (L-48b) Br |
| H | $CH(CH_3)CH_2SO_2CH_3$ | $CF_3$ | (L-50b) Cl |
| H | $CH(CH_3)CH_2SO_2CH_3$ | $CF_3$ | (L-50b) Br |

| | | | |
|---|---|---|---|
| H | $CH(CH_3)CH_2SO_2CH_3$ | $CF_3$ | (L-51b)Cl |
| H | $CH(CH_3)CH_2SO_2CH_3$ | $CF_2Cl$ | Ph-4-Cl |
| H | $CH(CH_3)CH_2SO_2CH_3$ | $CF_2Cl$ | Ph-4-Br |
| H | $CH(CH_3)CH_2SO_2CH_3$ | $CF_2Cl$ | Ph-4-$CF_3$ |
| H | $CH(CH_3)CH_2SO_2CH_3$ | $CF_2Cl$ | Ph-4-$OCF_3$ |
| H | $CH(CH_3)CH_2SO_2CH_3$ | $CF_2Cl$ | Ph-4-$OCF_2Br$ |
| H | $CH(CH_3)CH_2SO_2CH_3$ | $CF_2Cl$ | Ph-4-$OCF_2CHFCF_3$ |
| H | $CH(CH_3)CH_2SO_2CH_3$ | $CF_2Cl$ | Ph-4-$OCF_2CHFOCF_3$ |
| H | $CH(CH_3)CH_2SO_2CH_3$ | $CF_2Cl$ | Ph-4-$OCF_2CHFOCF_2CF_2CF_3$ |
| H | $CH(CH_3)CH_2SO_2CH_3$ | $CF_2Cl$ | Ph-4-O(L-45g) |
| H | $CH(CH_3)CH_2SO_2CH_3$ | $CF_2Cl$ | Ph-3,4-$Cl_2$ |
| H | $CH(CH_3)CH_2SO_2CH_3$ | $CF_2Cl$ | Ph(-3-$OCF_2O$-4-) |
| H | $CH(CH_3)CH_2SO_2CH_3$ | $CF_2Cl$ | Ph(-3-$OCF_2CF_2O$-4-) |
| H | $CH(CH_3)CH_2SO_2CH_3$ | $CF_2Cl$ | (L-45c)$CF_3$ |
| H | $CH(CH_3)CH_2SO_2CH_3$ | $CF_2Cl$ | L-45f |
| H | $CH(CH_3)CH_2SO_2CH_3$ | $CF_2Cl$ | (L-46c)$CF_3$ |
| H | $CH(CH_3)CH_2SO_2CH_3$ | $CF_2Cl$ | L-46d |
| H | $CH(CH_3)CH_2SO_2CH_3$ | $CFCl_2$ | Ph-4-$CF_3$ |
| H | $CH(CH_3)CH_2SO_2CH_3$ | $CF_2Br$ | Ph-4-Cl |
| H | $CH(CH_3)CH_2SO_2CH_3$ | $CF_2Br$ | Ph-4-Br |
| H | $CH(CH_3)CH_2SO_2CH_3$ | $CF_2Br$ | Ph-4-$CF_3$ |
| H | $CH(CH_3)CH_2SO_2CH_3$ | $CF_2Br$ | Ph-4-$OCF_3$ |
| H | $CH(CH_3)CH_2SO_2CH_3$ | $CF_2Br$ | Ph-4-$OCF_2Br$ |
| H | $CH(CH_3)CH_2SO_2CH_3$ | $CF_2Br$ | Ph-4-$OCF_2CHFCF_3$ |
| H | $CH(CH_3)CH_2SO_2CH_3$ | $CF_2Br$ | Ph-4-$OCF_2CHFOCF_3$ |
| H | $CH(CH_3)CH_2SO_2CH_3$ | $CF_2Br$ | Ph-4-$OCF_2CHFOCF_2CF_2CF_3$ |
| H | $CH(CH_3)CH_2SO_2CH_3$ | $CF_2Br$ | Ph-4-O(L-45g) |
| H | $CH(CH_3)CH_2SO_2CH_3$ | $CF_2Br$ | Ph-3,4-$Cl_2$ |
| H | $CH(CH_3)CH_2SO_2CH_3$ | $CF_2Br$ | Ph(-3-$OCF_2O$-4-) |
| H | $CH(CH_3)CH_2SO_2CH_3$ | $CF_2Br$ | Ph(-3-$OCF_2CF_2O$-4-) |
| H | $CH(CH_3)CH_2SO_2CH_3$ | $CF_2Br$ | (L-45c)$CF_3$ |
| H | $CH(CH_3)CH_2SO_2CH_3$ | $CF_2Br$ | L-45f |
| H | $CH(CH_3)CH_2SO_2CH_3$ | $CF_2Br$ | (L-46c)$CF_3$ |
| H | $CH(CH_3)CH_2SO_2CH_3$ | $CF_2Br$ | L-46d |
| H | $CH(CH_3)CH_2SO_2CH_3$ | $CFClBr$ | Ph-4-$OCF_3$ |
| H | $CH(CH_3)CH_2SO_2CH_3$ | $CFBr_2$ | (L-45c)$CF_3$ |
| H | $CH(CH_3)CH_2SO_2CH_3$ | $CF_2CHF_2$ | Ph-4-Br |
| H | $CH(CH_3)CH_2SO_2CH_3$ | $CF_2CF_3$ | Ph-4-Cl |
| H | $CH(CH_3)CH_2SO_2CH_3$ | $CF_2CF_3$ | Ph-4-Br |
| H | $CH(CH_3)CH_2SO_2CH_3$ | $CF_2CF_3$ | Ph-4-$CF_3$ |
| H | $CH(CH_3)CH_2SO_2CH_3$ | $CF_2CF_3$ | Ph-4-$OCF_3$ |

| | | | |
|---|---|---|---|
| H | $CH(CH_3)CH_2SO_2CH_3$ | $CF_2CF_3$ | Ph-4-$OCF_2Br$ |
| H | $CH(CH_3)CH_2SO_2CH_3$ | $CF_2CF_3$ | Ph-4-$OCF_2CHFCF_3$ |
| H | $CH(CH_3)CH_2SO_2CH_3$ | $CF_2CF_3$ | Ph-4-$OCF_2CHFOCF_3$ |
| H | $CH(CH_3)CH_2SO_2CH_3$ | $CF_2CF_3$ | Ph-4-$OCF_2CHFOCF_2CF_2CF_3$ |
| H | $CH(CH_3)CH_2SO_2CH_3$ | $CF_2CF_3$ | Ph-4-O(L-45g) |
| H | $CH(CH_3)CH_2SO_2CH_3$ | $CF_2CF_3$ | Ph-3, 4-$Cl_2$ |
| H | $CH(CH_3)CH_2SO_2CH_3$ | $CF_2CF_3$ | Ph(-3-$OCF_2O$-4-) |
| H | $CH(CH_3)CH_2SO_2CH_3$ | $CF_2CF_3$ | Ph(-3-$OCF_2CF_2O$-4-) |
| H | $CH(CH_3)CH_2SO_2CH_3$ | $CF_2CF_3$ | (L-45c)$CF_3$ |
| H | $CH(CH_3)CH_2SO_2CH_3$ | $CF_2CF_3$ | L-45f |
| H | $CH(CH_3)CH_2SO_2CH_3$ | $CF_2CF_3$ | (L-46c)$CF_3$ |
| H | $CH(CH_3)CH_2SO_2CH_3$ | $CF_2CF_3$ | L-46d |
| H | $CH(CH_3)CH_2SO_2CH_3$ | $CF_2CF_2Cl$ | Ph-4-$CF_3$ |
| H | $CH(CH_3)CH_2SO_2CH_3$ | $CFClCF_3$ | Ph-4-$OCF_3$ |
| H | $CH(CH_3)CH_2SO_2CH_3$ | $CFClCF_2Cl$ | (L-45c)$CF_3$ |
| H | $CH(CH_3)CH_2SO_2CH_3$ | $CF_2CF_2Br$ | Ph-4-Br |
| H | $CH(CH_3)CH_2SO_2CH_3$ | $CFBrCF_3$ | Ph-4-$CF_3$ |
| H | $CH(CH_3)CH_2SO_2CH_3$ | $CF_2CHFCF_3$ | Ph-4-$OCF_3$ |
| H | $CH(CH_3)CH_2SO_2CH_3$ | $CF_2CF_2CF_3$ | Ph-4-Cl |
| H | $CH(CH_3)CH_2SO_2CH_3$ | $CF_2CF_2CF_3$ | Ph-4-Br |
| H | $CH(CH_3)CH_2SO_2CH_3$ | $CF_2CF_2CF_3$ | Ph-4-$CF_3$ |
| H | $CH(CH_3)CH_2SO_2CH_3$ | $CF_2CF_2CF_3$ | Ph-4-$OCF_3$ |
| H | $CH(CH_3)CH_2SO_2CH_3$ | $CF_2CF_2CF_3$ | Ph-4-$OCF_2Br$ |
| H | $CH(CH_3)CH_2SO_2CH_3$ | $CF_2CF_2CF_3$ | Ph-4-$OCF_2CHFCF_3$ |
| H | $CH(CH_3)CH_2SO_2CH_3$ | $CF_2CF_2CF_3$ | Ph-4-$OCF_2CHFOCF_3$ |
| H | $CH(CH_3)CH_2SO_2CH_3$ | $CF_2CF_2CF_3$ | Ph-4-$OCF_2CHFOCF_2CF_2CF_3$ |
| H | $CH(CH_3)CH_2SO_2CH_3$ | $CF_2CF_2CF_3$ | Ph-4-O(L-45g) |
| H | $CH(CH_3)CH_2SO_2CH_3$ | $CF_2CF_2CF_3$ | Ph-3, 4-$Cl_2$ |
| H | $CH(CH_3)CH_2SO_2CH_3$ | $CF_2CF_2CF_3$ | Ph(-3-$OCF_2O$-4-) |
| H | $CH(CH_3)CH_2SO_2CH_3$ | $CF_2CF_2CF_3$ | Ph(-3-$OCF_2CF_2O$-4-) |
| H | $CH(CH_3)CH_2SO_2CH_3$ | $CF_2CF_2CF_3$ | (L-45c)$CF_3$ |
| H | $CH(CH_3)CH_2SO_2CH_3$ | $CF_2CF_2CF_3$ | L-45f |
| H | $CH(CH_3)CH_2SO_2CH_3$ | $CF_2CF_2CF_3$ | (L-46c)$CF_3$ |
| H | $CH(CH_3)CH_2SO_2CH_3$ | $CF_2CF_2CF_3$ | L-46d |
| H | $CH(CH_3)CH_2SO_2CH_3$ | $CF(CF_3)_2$ | (L-45c)$CF_3$ |
| H | $CH(CH_3)CH_2SO_2CH_3$ | $CF_2CF_2CF_2CF_3$ | Ph-4-Br |
| H | $CH(CH_3)CH_2SO_2CH_3$ | T-1 | Ph-4-$CF_3$ |
| H | $CH(CH_3)CH_2SO_2CH_3$ | T-2 | Ph-4-$OCF_3$ |
| H | $CH(CH_3)CH_2SO_2CH_3$ | $CH_2OCH_3$ | (L-45c)$CF_3$ |
| H | $CH(CH_3)CH_2SO_2CH_3$ | $CH_2OEt$ | Ph-4-Br |
| H | $CH(CH_3)CH_2SO_2CH_3$ | $CH_2OCH_2CF_3$ | Ph-4-$CF_3$ |

| | | | |
|---|---|---|---|
| H | $CH(CH_3)CH_2SO_2CH_3$ | $CH_2OCH(CF_3)_2$ | Ph-4-$OCF_3$ |
| H | $CH(CH_3)CH_2SO_2CH_3$ | $CF_2OCH_3$ | Ph-4-Cl |
| H | $CH(CH_3)CH_2SO_2CH_3$ | $CF_2OCH_3$ | Ph-4-Br |
| H | $CH(CH_3)CH_2SO_2CH_3$ | $CF_2OCH_3$ | Ph-4-$CF_3$ |
| H | $CH(CH_3)CH_2SO_2CH_3$ | $CF_2OCH_3$ | Ph-4-$OCF_3$ |
| H | $CH(CH_3)CH_2SO_2CH_3$ | $CF_2OCH_3$ | Ph-4-$OCF_2Br$ |
| H | $CH(CH_3)CH_2SO_2CH_3$ | $CF_2OCH_3$ | Ph-4-$OCF_2CHFCF_3$ |
| H | $CH(CH_3)CH_2SO_2CH_3$ | $CF_2OCH_3$ | Ph-4-$OCF_2CHFOCF_3$ |
| H | $CH(CH_3)CH_2SO_2CH_3$ | $CF_2OCH_3$ | Ph-4-$OCF_2CHFOCF_2CF_2CF_3$ |
| H | $CH(CH_3)CH_2SO_2CH_3$ | $CF_2OCH_3$ | Ph-4-O(L-45g) |
| H | $CH(CH_3)CH_2SO_2CH_3$ | $CF_2OCH_3$ | Ph-3,4-$Cl_2$ |
| H | $CH(CH_3)CH_2SO_2CH_3$ | $CF_2OCH_3$ | Ph(-3-$OCF_2O$-4-) |
| H | $CH(CH_3)CH_2SO_2CH_3$ | $CF_2OCH_3$ | Ph(-3-$OCF_2CF_2O$-4-) |
| H | $CH(CH_3)CH_2SO_2CH_3$ | $CF_2OCH_3$ | (L-45c)$CF_3$ |
| H | $CH(CH_3)CH_2SO_2CH_3$ | $CF_2OCH_3$ | L-45f |
| H | $CH(CH_3)CH_2SO_2CH_3$ | $CF_2OCH_3$ | (L-46c)$CF_3$ |
| H | $CH(CH_3)CH_2SO_2CH_3$ | $CF_2OCH_3$ | L-46d |
| H | $CH(CH_3)CH_2SO_2CH_3$ | $CH_2SCH_3$ | (L-45c)$CF_3$ |
| H | $CH(CH_3)CH_2SO_2CH_3$ | $CH_2SO_2CH_3$ | Ph-4-Br |
| H | $CH(CH_3)CH_2SO_2CH_3$ | $CH_2SCF_3$ | Ph-4-$CF_3$ |
| H | $CH(CH_3)CH_2SO_2CH_3$ | $CF_2SCH_3$ | Ph-4-Cl |
| H | $CH(CH_3)CH_2SO_2CH_3$ | $CF_2SCH_3$ | Ph-4-Br |
| H | $CH(CH_3)CH_2SO_2CH_3$ | $CF_2SCH_3$ | Ph-4-$CF_3$ |
| H | $CH(CH_3)CH_2SO_2CH_3$ | $CF_2SCH_3$ | Ph-4-$OCF_3$ |
| H | $CH(CH_3)CH_2SO_2CH_3$ | $CF_2SCH_3$ | Ph-4-$OCF_2Br$ |
| H | $CH(CH_3)CH_2SO_2CH_3$ | $CF_2SCH_3$ | Ph-4-$OCF_2CHFCF_3$ |
| H | $CH(CH_3)CH_2SO_2CH_3$ | $CF_2SCH_3$ | Ph-4-$OCF_2CHFOCF_3$ |
| H | $CH(CH_3)CH_2SO_2CH_3$ | $CF_2SCH_3$ | Ph-4-$OCF_2CHFOCF_2CF_2CF_3$ |
| H | $CH(CH_3)CH_2SO_2CH_3$ | $CF_2SCH_3$ | Ph-4-O(L-45g) |
| H | $CH(CH_3)CH_2SO_2CH_3$ | $CF_2SCH_3$ | Ph-3,4-$Cl_2$ |
| H | $CH(CH_3)CH_2SO_2CH_3$ | $CF_2SCH_3$ | Ph(-3-$OCF_2O$-4-) |
| H | $CH(CH_3)CH_2SO_2CH_3$ | $CF_2SCH_3$ | Ph(-3-$OCF_2CF_2O$-4-) |
| H | $CH(CH_3)CH_2SO_2CH_3$ | $CF_2SCH_3$ | (L-45c)$CF_3$ |
| H | $CH(CH_3)CH_2SO_2CH_3$ | $CF_2SCH_3$ | L-45f |
| H | $CH(CH_3)CH_2SO_2CH_3$ | $CF_2SCH_3$ | (L-46c)$CF_3$ |
| H | $CH(CH_3)CH_2SO_2CH_3$ | $CF_2SCH_3$ | L-46d |
| H | $CH(CH_3)CH_2SO_2CH_3$ | $CF_2SEt$ | Ph-4-$OCF_3$ |
| H | $CH(CH_3)CH_2SO_2CH_3$ | $CF_2SPr$-n | (L-45c)$CF_3$ |
| H | $CH(CH_3)CH_2SO_2CH_3$ | $CF_2SPr$-i | Ph-4-Br |
| H | $CH(CH_3)CH_2SO_2CH_3$ | $CF_2SCH_2Ph$ | Ph-4-$CF_3$ |
| H | $CH(CH_3)CH_2SO_2CH_3$ | $CF_2SPh$ | Ph-4-$OCF_3$ |

| | | | |
|---|---|---|---|
| H | $CH(CH_3)CH_2SO_2CH_3$ | $CF_2C(O)OEt$ | $(L-45c)CF_3$ |
| H | $CH(CH_3)CH_2SO_2CH_3$ | $CF_2SO_2N(CH_3)_2$ | Ph-4-Br |
| H | $CH(CH_3)CH_2SO_2CH_3$ | C(O)OEt | $Ph-4-CF_3$ |
| H | $CH(CH_3)CH_2SO_2CH_3$ | C(O)SEt | $Ph-4-OCF_3$ |
| H | $CH(CH_3)CH_2SEt$ | $CF_3$ | CH=CH(Ph-4-Cl) |
| H | $CH(CH_3)CH_2SEt$ | $CF_3$ | Ph-4-Cl |
| H | $CH(CH_3)CH_2SEt$ | $CF_3$ | Ph-4-Br |
| H | $CH(CH_3)CH_2SEt$ | $CF_3$ | $Ph-4-CF_3$ |
| H | $CH(CH_3)CH_2SEt$ | $CF_3$ | $Ph-4-OCF_3$ |
| H | $CH(CH_3)CH_2SEt$ (R) | $CF_3$ | $Ph-4-OCF_3$ |
| H | $CH(CH_3)CH_2SEt$ (S) | $CF_3$ | $Ph-4-OCF_3$ |
| H | $CH(CH_3)CH_2SEt$ | $CF_3$ | $Ph-4-OCF_2Br$ |
| H | $CH(CH_3)CH_2SEt$ | $CF_3$ | $Ph-4-OCF_2CHFCF_3$ |
| H | $CH(CH_3)CH_2SEt$ | $CF_3$ | $Ph-4-OCF_2CHFOCF_3$ |
| H | $CH(CH_3)CH_2SEt$ | $CF_3$ | $Ph-4-OCF_2CHFOCF_2CF_2CF_3$ |
| H | $CH(CH_3)CH_2SEt$ | $CF_3$ | Ph-4-O(L-45g) |
| H | $CH(CH_3)CH_2SEt$ | $CF_3$ | $Ph-3,4-Cl_2$ |
| H | $CH(CH_3)CH_2SEt$ | $CF_3$ | $Ph(-3-OCF_2O-4-)$ |
| H | $CH(CH_3)CH_2SEt$ | $CF_3$ | $Ph(-3-OCF_2CF_2O-4-)$ |
| H | $CH(CH_3)CH_2SEt$ | $CF_3$ | $(L-45c)CF_3$ |
| H | $CH(CH_3)CH_2SEt$ | $CF_3$ | L-45f |
| H | $CH(CH_3)CH_2SEt$ | $CF_3$ | $(L-46c)CF_3$ |
| H | $CH(CH_3)CH_2SEt$ | $CF_3$ | L-46d |
| H | $CH(CH_3)CH_2S(O)Et$ | $CF_3$ | CH=CH(Ph-4-Cl) |
| H | $CH(CH_3)CH_2S(O)Et$ | $CF_3$ | Ph-4-Cl |
| H | $CH(CH_3)CH_2S(O)Et$ | $CF_3$ | Ph-4-Br |
| H | $CH(CH_3)CH_2S(O)Et$ | $CF_3$ | $Ph-4-CF_3$ |
| H | $CH(CH_3)CH_2S(O)Et$ | $CF_3$ | $Ph-4-OCF_3$ |
| H | $CH(CH_3)CH_2S(O)Et$ | $CF_3$ | $Ph-4-OCF_2Br$ |
| H | $CH(CH_3)CH_2S(O)Et$ | $CF_3$ | $Ph-4-OCF_2CHFCF_3$ |
| H | $CH(CH_3)CH_2S(O)Et$ | $CF_3$ | $Ph-4-OCF_2CHFOCF_3$ |
| H | $CH(CH_3)CH_2S(O)Et$ | $CF_3$ | $Ph-4-OCF_2CHFOCF_2CF_2CF_3$ |
| H | $CH(CH_3)CH_2S(O)Et$ | $CF_3$ | Ph-4-O(L-45g) |
| H | $CH(CH_3)CH_2S(O)Et$ | $CF_3$ | $Ph-3,4-Cl_2$ |
| H | $CH(CH_3)CH_2S(O)Et$ | $CF_3$ | $Ph(-3-OCF_2O-4-)$ |
| H | $CH(CH_3)CH_2S(O)Et$ | $CF_3$ | $Ph(-3-OCF_2CF_2O-4-)$ |
| H | $CH(CH_3)CH_2S(O)Et$ | $CF_3$ | $(L-45c)CF_3$ |
| H | $CH(CH_3)CH_2S(O)Et$ | $CF_3$ | L-45f |
| H | $CH(CH_3)CH_2S(O)Et$ | $CF_3$ | $(L-46c)CF_3$ |
| H | $CH(CH_3)CH_2S(O)Et$ | $CF_3$ | L-46d |
| H | $CH(CH_3)CH_2SO_2Et$ | $CF_3$ | CH=CH(Ph-4-Cl) |

| | | | |
|---|---|---|---|
| H | $CH(CH_3)CH_2SO_2Et$ | $CF_3$ | Ph-4-Cl |
| H | $CH(CH_3)CH_2SO_2Et$ | $CF_3$ | Ph-4-Br |
| H | $CH(CH_3)CH_2SO_2Et$ | $CF_3$ | $Ph-4-CF_3$ |
| H | $CH(CH_3)CH_2SO_2Et$ | $CF_3$ | $Ph-4-OCF_3$ |
| H | $CH(CH_3)CH_2SO_2Et$ | $CF_3$ | $Ph-4-OCF_2Br$ |
| H | $CH(CH_3)CH_2SO_2Et$ | $CF_3$ | $Ph-4-OCF_2CHFCF_3$ |
| H | $CH(CH_3)CH_2SO_2Et$ | $CF_3$ | $Ph-4-OCF_2CHFOCF_3$ |
| H | $CH(CH_3)CH_2SO_2Et$ | $CF_3$ | $Ph-4-OCF_2CHFOCF_2CF_2CF_3$ |
| H | $CH(CH_3)CH_2SO_2Et$ | $CF_3$ | Ph-4-O(L-45g) |
| H | $CH(CH_3)CH_2SO_2Et$ | $CF_3$ | $Ph-3,4-Cl_2$ |
| H | $CH(CH_3)CH_2SO_2Et$ | $CF_3$ | $Ph(-3-OCF_2O-4-)$ |
| H | $CH(CH_3)CH_2SO_2Et$ | $CF_3$ | $Ph(-3-OCF_2CF_2O-4-)$ |
| H | $CH(CH_3)CH_2SO_2Et$ | $CF_3$ | $(L-45c)CF_3$ |
| H | $CH(CH_3)CH_2SO_2Et$ | $CF_3$ | L-45f |
| H | $CH(CH_3)CH_2SO_2Et$ | $CF_3$ | $(L-46c)CF_3$ |
| H | $CH(CH_3)CH_2SO_2Et$ | $CF_3$ | L-46d |
| H | $CH(CH_3)CH_2SPr-n$ | $CF_3$ | Ph-4-Br |
| H | $CH(CH_3)CH_2SPr-n$ | $CF_3$ | $Ph-4-CF_3$ |
| H | $CH(CH_3)CH_2SPr-n$ | $CF_3$ | $Ph-4-OCF_3$ |
| H | $CH(CH_3)CH_2SPr-n$ | $CF_3$ | $(L-45c)CF_3$ |
| H | $CH(CH_3)CH_2SPr-i$ | $CF_3$ | $(L-45c)CF_3$ |
| H | $CH(CH_3)CH_2SO_2Pr-i$ | $CF_3$ | Ph-4-Br |
| H | $CH(CH_3)CH_2SBu-n$ | $CF_3$ | $Ph-4-CF_3$ |
| H | $CH(CH_3)CH_2SBu-i$ | $CF_3$ | Ph-4-Br |
| H | $CH(CH_3)CH_2SBu-i$ | $CF_3$ | $Ph-4-CF_3$ |
| H | $CH(CH_3)CH_2SBu-i$ | $CF_3$ | $Ph-4-OCF_3$ |
| H | $CH(CH_3)CH_2SBu-i$ | $CF_3$ | $(L-45c)CF_3$ |
| H | $CH(CH_3)CH_2SBu-t$ | $CF_3$ | Ph-4-Br |
| H | $CH(CH_3)CH_2SBu-t$ | $CF_3$ | $Ph-4-CF_3$ |
| H | $CH(CH_3)CH_2SBu-t$ | $CF_3$ | $Ph-4-OCF_3$ |
| H | $CH(CH_3)CH_2SBu-t$ | $CF_3$ | $(L-45c)CF_3$ |
| H | $CH(CH_3)CH_2SO_2Bu-t$ | $CF_3$ | $Ph-4-OCF_3$ |
| H | $CH(CH_3)CH_2SHex-n$ | $CF_3$ | $(L-45c)CF_3$ |
| H | $CH(CH_3)CH_2SHex-c$ | $CF_3$ | Ph-4-Br |
| H | $CH(CH_3)CH_2SCH_2CF_3$ | $CF_3$ | $Ph-4-CF_3$ |
| H | $CH(CH_3)CH_2S(O)CH_2CF_3$ | $CF_3$ | $Ph-4-OCF_3$ |
| H | $CH(CH_3)CH_2SO_2CH_2CF_3$ | $CF_3$ | $(L-45c)CF_3$ |
| H | $CH(CH_3)CH_2SCH_2CH_2OH$ | $CF_3$ | Ph-4-Br |
| H | $CH(CH_3)CH_2SCH_2CH_2OCH_3$ | $CF_3$ | $Ph-4-CF_3$ |
| H | $CH(CH_3)CH_2S(O)CH_2CH_2OCH_3$ | $CF_3$ | $Ph-4-OCF_3$ |
| H | $CH(CH_3)CH_2SO_2CH_2CH_2OCH_3$ | $CF_3$ | $(L-45c)CF_3$ |

| | | | |
|---|---|---|---|
| H | $CH(CH_3)CH_2SCH_2CH_2OEt$ | $CF_3$ | Ph-4-Br |
| H | $CH(CH_3)CH_2S(O)CH_2CH_2OEt$ | $CF_3$ | Ph-4-$CF_3$ |
| H | $CH(CH_3)CH_2SO_2CH_2CH_2OEt$ | $CF_3$ | Ph-4-$OCF_3$ |
| H | $CH(CH_3)CH_2SCH_2CH_2OC(O)CF_3$ | $CF_3$ | (L-45c) $CF_3$ |
| H | $CH(CH_3)CH_2SCH_2CH_2SCH_3$ | $CF_3$ | Ph-4-Br |
| H | $CH(CH_3)CH_2SCH_2Si(CH_3)_3$ | $CF_3$ | Ph-4-Br |
| H | $CH(CH_3)CH_2SCH_2Si(CH_3)_3$ | $CF_3$ | Ph-4-$CF_3$ |
| H | $CH(CH_3)CH_2SCH_2Si(CH_3)_3$ | $CF_3$ | Ph-4-$OCF_3$ |
| H | $CH(CH_3)CH_2SCH_2Si(CH_3)_3$ | $CF_3$ | (L-45c) $CF_3$ |
| H | $CH(CH_3)CH_2SCH_2C(O)CH_3$ | $CF_3$ | Ph-4-$CF_3$ |
| H | $CH(CH_3)CH_2SCH_2CH_2C(O)OCH_3$ | $CF_3$ | Ph-4-$OCF_3$ |
| H | $CH(CH_3)CH_2S(O)CH_2CH_2C(O)OCH_3$ | $CF_3$ | (L-45c) $CF_3$ |
| H | $CH(CH_3)CH_2SO_2CH_2CH_2C(O)OCH_3$ | $CF_3$ | Ph-4-Br |
| H | $CH(CH_3)CH_2SCH_2C(O)N(Et)_2$ | $CF_3$ | Ph-4-$CF_3$ |
| H | $CH(CH_3)CH_2S(O)CH_2C(O)N(Et)_2$ | $CF_3$ | Ph-4-$OCF_3$ |
| H | $CH(CH_3)CH_2SO_2CH_2C(O)N(Et)_2$ | $CF_3$ | (L-45c) $CF_3$ |
| H | $CH(CH_3)CH_2SCH_2(Ph\text{-}2,4\text{-}Cl_2)$ | $CF_3$ | Ph-4-Br |
| H | $CH(CH_3)CH_2SCH_2CH{=}CH_2$ | $CF_3$ | Ph-4-$CF_3$ |
| H | $CH(CH_3)CH_2SCH_2C{\equiv}CH$ | $CF_3$ | Ph-4-$OCF_3$ |
| H | $CH(CH_3)CH_2SC(O)CH_3$ | $CF_3$ | (L-45c) $CF_3$ |
| H | $CH(CH_3)CH_2SSCH_3$ | $CF_3$ | CH=CH(Ph-4-Cl) |
| H | $CH(CH_3)CH_2SSCH_3$ | $CF_3$ | Ph-4-Cl |
| H | $CH(CH_3)CH_2SSCH_3$ | $CF_3$ | Ph-4-Br |
| H | $CH(CH_3)CH_2SSCH_3$ | $CF_3$ | Ph-4-$CF_3$ |
| H | $CH(CH_3)CH_2SSCH_3$ | $CF_3$ | Ph-4-$OCF_3$ |
| H | $CH(CH_3)CH_2SSCH_3$ | $CF_3$ | Ph-4-$OCF_2Br$ |
| H | $CH(CH_3)CH_2SSCH_3$ | $CF_3$ | Ph-4-$OCF_2CHFCF_3$ |
| H | $CH(CH_3)CH_2SSCH_3$ | $CF_3$ | Ph-4-$OCF_2CHFOCF_3$ |
| H | $CH(CH_3)CH_2SSCH_3$ | $CF_3$ | Ph-4-$OCF_2CHFOCF_2CF_2CF_3$ |
| H | $CH(CH_3)CH_2SSCH_3$ | $CF_3$ | Ph-4-O(L-45g) |
| H | $CH(CH_3)CH_2SSCH_3$ | $CF_3$ | Ph-3,4-$Cl_2$ |
| H | $CH(CH_3)CH_2SSCH_3$ | $CF_3$ | Ph(-3-$OCF_2O$-4-) |
| H | $CH(CH_3)CH_2SSCH_3$ | $CF_3$ | Ph(-3-$OCF_2CF_2O$-4-) |
| H | $CH(CH_3)CH_2SSCH_3$ | $CF_3$ | (L-45c) $CF_3$ |
| H | $CH(CH_3)CH_2SSCH_3$ | $CF_3$ | L-45f |
| H | $CH(CH_3)CH_2SSCH_3$ | $CF_3$ | (L-46c) $CF_3$ |
| H | $CH(CH_3)CH_2SSCH_3$ | $CF_3$ | L-46d |
| H | $CH(CH_3)CH_2SS(Ph\text{-}2\text{-}NO_2)$ | $CF_3$ | Ph-4-Br |
| H | $CH(CH_3)CH_2SO_2NH_2$ | $CF_3$ | Ph-4-$CF_3$ |
| H | $CH(CH_3)CH_2SO_2NHCH_3$ | $CF_3$ | Ph-4-Br |
| H | $CH(CH_3)CH_2SO_2NHCH_3$ | $CF_3$ | Ph-4-$CF_3$ |

| | | | |
|---|---|---|---|
| H | $CH(CH_3)CH_2SO_2NHCH_3$ | $CF_3$ | Ph-4-$OCF_3$ |
| H | $CH(CH_3)CH_2SO_2NHCH_3$ | $CF_3$ | (L-45c) $CF_3$ |
| H | $CH(CH_3)CH_2SO_2NHEt$ | $CF_3$ | $CF_2O$(Ph-4-Cl) |
| H | $CH(CH_3)CH_2SO_2NHEt$ | $CF_3$ | CH=CH(Ph-4-Cl) |
| H | $CH(CH_3)CH_2SO_2NHEt$ | $CF_3$ | CH=CH(Ph-4-Br) |
| H | $CH(CH_3)CH_2SO_2NHEt$ | $CF_3$ | CH=CH(Ph-4-$CF_3$) |
| H | $CH(CH_3)CH_2SO_2NHEt$ | $CF_3$ | CH=CH(Ph-4-$OCF_3$) |
| H | $CH(CH_3)CH_2SO_2NHEt$ | $CF_3$ | CH=CH(Ph-3, 4-$Cl_2$) |
| H | $CH(CH_3)CH_2SO_2NHEt$ | $CF_3$ | Ph-4-F |
| H | $CH(CH_3)CH_2SO_2NHEt$ | $CF_3$ | Ph-4-Cl |
| H | $CH(CH_3)CH_2SO_2NHEt$ | $CF_3$ | Ph-4-Br |
| H | $CH(CH_3)CH_2SO_2NHEt$ | $CF_3$ | Ph-4-$CF_3$ |
| H | $CH(CH_3)CH_2SO_2NHEt$ | $CF_3$ | Ph-4-$OCF_3$ |
| H | $CH(CH_3)CH_2SO_2NHEt$ (S) | $CF_3$ | Ph-4-$OCF_3$ |
| H | $CH(CH_3)CH_2SO_2NHEt$ | $CF_3$ | Ph-4-$OCF_2Br$ |
| H | $CH(CH_3)CH_2SO_2NHEt$ | $CF_3$ | Ph-4-$OCF_2CHF_2$ |
| H | $CH(CH_3)CH_2SO_2NHEt$ | $CF_3$ | Ph-4-$OCF_2CHFCF_3$ |
| H | $CH(CH_3)CH_2SO_2NHEt$ | $CF_3$ | Ph-4-$OCF_2CHFOCF_3$ |
| H | $CH(CH_3)CH_2SO_2NHEt$ | $CF_3$ | Ph-4-$OCF_2CHFOCF_2CF_2CF_3$ |
| H | $CH(CH_3)CH_2SO_2NHEt$ | $CF_3$ | Ph-4-O(L-45g) |
| H | $CH(CH_3)CH_2SO_2NHEt$ | $CF_3$ | Ph-3, 4-$F_2$ |
| H | $CH(CH_3)CH_2SO_2NHEt$ | $CF_3$ | Ph-3-F-4-Cl |
| H | $CH(CH_3)CH_2SO_2NHEt$ | $CF_3$ | Ph-3, 4-$Cl_2$ |
| H | $CH(CH_3)CH_2SO_2NHEt$ | $CF_3$ | Ph(-3-$OCF_2O$-4-) |
| H | $CH(CH_3)CH_2SO_2NHEt$ | $CF_3$ | Ph(-3-$OCF_2CF_2O$-4-) |
| H | $CH(CH_3)CH_2SO_2NHEt$ | $CF_3$ | (L-45c) $CF_3$ |
| H | $CH(CH_3)CH_2SO_2NHEt$ | $CF_3$ | L-45f |
| H | $CH(CH_3)CH_2SO_2NHEt$ | $CF_3$ | (L-46c) $CF_3$ |
| H | $CH(CH_3)CH_2SO_2NHEt$ | $CF_3$ | L-46d |
| H | $CH(CH_3)CH_2SO_2NHPr$-n | $CF_3$ | Ph-4-$OCF_3$ |
| H | $CH(CH_3)CH_2SO_2NHPr$-i | $CF_3$ | (L-45c) $CF_3$ |
| H | $CH(CH_3)CH_2SO_2NHBu$-n | $CF_3$ | Ph-4-Br |
| H | $CH(CH_3)CH_2SO_2NHBu$-t | $CF_3$ | Ph-4-$CF_3$ |
| H | $CH(CH_3)CH_2SO_2NHCH_2CH_2Cl$ | $CF_3$ | Ph-4-$OCF_3$ |
| H | $CH(CH_3)CH_2SO_2NHCH_2CH_2OH$ | $CF_3$ | (L-45c) $CF_3$ |
| H | $CH(CH_3)CH_2SO_2NHCH_2CH_2OCH_3$ | $CF_3$ | Ph-4-Br |
| H | $CH(CH_3)CH_2SO_2NHCH_2CH_2SCH_3$ | $CF_3$ | Ph-4-$CF_3$ |
| H | $CH(CH_3)CH_2SO_2NHCH_2CH_2S(O)CH_3$ | $CF_3$ | Ph-4-$OCF_3$ |
| H | $CH(CH_3)CH_2SO_2NHCH_2CH_2SO_2CH_3$ | $CF_3$ | (L-45c) $CF_3$ |
| H | $CH(CH_3)CH_2SO_2NHCH(CH_3)CH_2SCH_3$ | $CF_3$ | Ph-4-Br |
| H | $CH(CH_3)CH_2SO_2NHCH(CH_3)CH_2SCH_3$ (S, S) | $CF_3$ | Ph-4-$CF_3$ |

| | | | |
|---|---|---|---|
| H | $CH(CH_3)CH_2SO_2NHCH(CH_3)CH_2S(O)CH_3$ | $CF_3$ | Ph-4-$OCF_3$ |
| H | $CH(CH_3)CH_2SO_2NHCH(CH_3)CH_2S(O)CH_3$ (S, S) | $CF_3$ | (L-45c) $CF_3$ |
| H | $CH(CH_3)CH_2SO_2NHCH(CH_3)CH_2SO_2CH_3$ | $CF_3$ | Ph-4-Br |
| H | $CH(CH_3)CH_2SO_2NHCH(CH_3)CH_2SO_2CH_3$ (S, S) | $CF_3$ | Ph-4-$CF_3$ |
| H | $CH(CH_3)CH_2SO_2NHC(CH_3)_2CH_2SCH_3$ | $CF_3$ | Ph-4-$OCF_3$ |
| H | $CH(CH_3)CH_2SO_2NHCH(CH_3)C(O)NH_2$ | $CF_3$ | (L-45c) $CF_3$ |
| H | $CH(CH_3)CH_2SO_2NHCH(CH_3)C(O)NHCH_3$ | $CF_3$ | Ph-4-Br |
| H | $CH(CH_3)CH_2SO_2NHCH(CH_3)C(O)NHEt$ | $CF_3$ | Ph-4-$CF_3$ |
| H | $CH(CH_3)CH_2SO_2NHCH_2CH{=}CH_2$ | $CF_3$ | Ph-4-$OCF_3$ |
| H | $CH(CH_3)CH_2SO_2NHCH_2C{\equiv}CH$ | $CF_3$ | (L-45c) $CF_3$ |
| H | $CH(CH_3)CH_2SO_2NHCH_2Ph$ | $CF_3$ | Ph-4-Br |
| H | $CH(CH_3)CH_2SO_2NHCH_2Ph$ (S) | $CF_3$ | Ph-4-$CF_3$ |
| H | $CH(CH_3)CH_2SO_2NHCH_2$(Ph-2-F) | $CF_3$ | Ph-4-$OCF_3$ |
| H | $CH(CH_3)CH_2SO_2NHCH_2$(Ph-2-Cl) | $CF_3$ | (L-45c) $CF_3$ |
| H | $CH(CH_3)CH_2SO_2NHCH_2$(Ph-2-Br) | $CF_3$ | Ph-4-Br |
| H | $CH(CH_3)CH_2SO_2NHCH_2$(Ph-2-$OCH_3$) | $CF_3$ | Ph-4-$CF_3$ |
| H | $CH(CH_3)CH_2SO_2NHCH_2$(Ph-3-$OCH_3$) | $CF_3$ | Ph-4-$OCF_3$ |
| H | $CH(CH_3)CH_2SO_2NHCH_2$(Ph-4-$OCH_3$) | $CF_3$ | (L-45c) $CF_3$ |
| H | $CH(CH_3)CH_2SO_2NHCH_2$(Ph-4-$SCF_3$) | $CF_3$ | Ph-4-Br |
| H | $CH(CH_3)CH_2SO_2NHCH_2$(Ph-2-$NO_2$) | $CF_3$ | Ph-4-$CF_3$ |
| H | $CH(CH_3)CH_2SO_2NHCH_2$(Ph-3-$NO_2$) | $CF_3$ | Ph-4-$OCF_3$ |
| H | $CH(CH_3)CH_2SO_2NHCH_2$(Ph-4-$NO_2$) | $CF_3$ | (L-45c) $CF_3$ |
| H | $CH(CH_3)CH_2SO_2NHCH_2$(Ph-3-CN) | $CF_3$ | Ph-4-Br |
| H | $CH(CH_3)CH_2SO_2NHCH_2$(Ph-4-CN) | $CF_3$ | Ph-4-$CF_3$ |
| H | $CH(CH_3)CH_2SO_2NHCH_2$(L-45a) | $CF_3$ | Ph-4-$OCF_3$ |
| H | $CH(CH_3)CH_2SO_2NHCH_2$(L-46a) | $CF_3$ | (L-45c) $CF_3$ |
| H | $CH(CH_3)CH_2SO_2NHCH_2$(L-47a) | $CF_3$ | Ph-4-Br |
| H | $CH(CH_3)CH_2SO_2NHCH(CH_3)Ph$ | $CF_3$ | Ph-4-$CF_3$ |
| H | $CH(CH_3)CH_2SO_2NHCH(CH_3)Ph$ (R) | $CF_3$ | Ph-4-$OCF_3$ |
| H | $CH(CH_3)CH_2SO_2NHCH(CH_3)Ph$ (S) | $CF_3$ | (L-45c) $CF_3$ |
| H | $CH(CH_3)CH_2SO_2NHCH_2CH_2Ph$ | $CF_3$ | Ph-4-Br |
| H | $CH(CH_3)CH_2SO_2NH$(Ph-2-$OCH_3$) | $CF_3$ | Ph-4-$CF_3$ |
| H | $CH(CH_3)CH_2SO_2N(CH_3)_2$ | $CF_3$ | Ph-4-Br |
| H | $CH(CH_3)CH_2SO_2N(CH_3)_2$ | $CF_3$ | Ph-4-$CF_3$ |
| H | $CH(CH_3)CH_2SO_2N(CH_3)_2$ | $CF_3$ | Ph-4-$OCF_3$ |
| H | $CH(CH_3)CH_2SO_2N(CH_3)_2$ | $CF_3$ | (L-45c) $CF_3$ |
| H | $CH(CH_3)CH_2SO_2N(CH_3)Et$ | $CF_3$ | Ph-4-$OCF_3$ |
| H | $CH(CH_3)CH_2SO_2N(Et)_2$ | $CF_3$ | $CF_2O$(Ph-4-Cl) |
| H | $CH(CH_3)CH_2SO_2N(Et)_2$ | $CF_3$ | CH=CH(Ph-4-Cl) |
| H | $CH(CH_3)CH_2SO_2N(Et)_2$ | $CF_3$ | CH=CH(Ph-4-Br) |
| H | $CH(CH_3)CH_2SO_2N(Et)_2$ | $CF_3$ | CH=CH(Ph-4-$CF_3$) |

| | | | |
|---|---|---|---|
| H | CH ($CH_3$) $CH_2SO_2N$ (Et)$_2$ | $CF_3$ | CH=CH (Ph-4-$OCF_3$) |
| H | CH ($CH_3$) $CH_2SO_2N$ (Et)$_2$ | $CF_3$ | CH=CH (Ph-3, 4-$Cl_2$) |
| H | CH ($CH_3$) $CH_2SO_2N$ (Et)$_2$ | $CF_3$ | Ph-4-F |
| H | CH ($CH_3$) $CH_2SO_2N$ (Et)$_2$ | $CF_3$ | Ph-4-Cl |
| H | CH ($CH_3$) $CH_2SO_2N$ (Et)$_2$ | $CF_3$ | Ph-4-Br |
| H | CH ($CH_3$) $CH_2SO_2N$ (Et)$_2$ | $CF_3$ | Ph-4-$CF_3$ |
| H | CH ($CH_3$) $CH_2SO_2N$ (Et)$_2$ | $CF_3$ | Ph-4-$OCF_3$ |
| H | CH ($CH_3$) $CH_2SO_2N$ (Et)$_2$ (S) | $CF_3$ | Ph-4-$OCF_3$ |
| H | CH ($CH_3$) $CH_2SO_2N$ (Et)$_2$ | $CF_3$ | Ph-4-$OCF_2Br$ |
| H | CH ($CH_3$) $CH_2SO_2N$ (Et)$_2$ | $CF_3$ | Ph-4-$OCF_2CHF_2$ |
| H | CH ($CH_3$) $CH_2SO_2N$ (Et)$_2$ | $CF_3$ | Ph-4-$OCF_2CHFCF_3$ |
| H | CH ($CH_3$) $CH_2SO_2N$ (Et)$_2$ | $CF_3$ | Ph-4-$OCF_2CHFOCF_3$ |
| H | CH ($CH_3$) $CH_2SO_2N$ (Et)$_2$ | $CF_3$ | Ph-4-$OCF_2CHFOCF_2CF_2CF_3$ |
| H | CH ($CH_3$) $CH_2SO_2N$ (Et)$_2$ | $CF_3$ | Ph-4-O (L-45g) |
| H | CH ($CH_3$) $CH_2SO_2N$ (Et)$_2$ | $CF_3$ | Ph-3, 4-$F_2$ |
| H | CH ($CH_3$) $CH_2SO_2N$ (Et)$_2$ | $CF_3$ | Ph-3-F-4-Cl |
| H | CH ($CH_3$) $CH_2SO_2N$ (Et)$_2$ | $CF_3$ | Ph-3, 4-$Cl_2$ |
| H | CH ($CH_3$) $CH_2SO_2N$ (Et)$_2$ | $CF_3$ | Ph (-3-$OCF_2O$-4-) |
| H | CH ($CH_3$) $CH_2SO_2N$ (Et)$_2$ | $CF_3$ | Ph (-3-$OCF_2CF_2O$-4-) |
| H | CH ($CH_3$) $CH_2SO_2N$ (Et)$_2$ | $CF_3$ | (L-45c) $CF_3$ |
| H | CH ($CH_3$) $CH_2SO_2N$ (Et)$_2$ | $CF_3$ | L-45f |
| H | CH ($CH_3$) $CH_2SO_2N$ (Et)$_2$ | $CF_3$ | (L-46c) $CF_3$ |
| H | CH ($CH_3$) $CH_2SO_2N$ (Et)$_2$ | $CF_3$ | L-46d |
| H | CH ($CH_3$) $CH_2SO_2$ (T-19) | $CF_3$ | (L-45c) $CF_3$ |
| H | CH ($CH_3$) $CH_2SO_2$ (T-20) | $CF_3$ | Ph-4-Br |
| H | CH ($CH_3$) $CH_2SO_2$ (T-21) | $CF_3$ | Ph-4-$CF_3$ |
| H | CH ($CH_3$) $CH_2SO_2N$ ($CH_3$) $CH_2Ph$ | $CF_3$ | Ph-4-$OCF_3$ |
| H | CH ($CH_3$) $CH_2SO_2N$ (Et) $CH_2Ph$ | $CF_3$ | (L-45c) $CF_3$ |
| H | CH ($CH_3$) $CH_2SC$ (O) NHEt | $CF_3$ | Ph-4-Br |
| H | CH ($CH_3$) $CH_2SC$ (O) N ($CH_3$)$_2$ | $CF_3$ | Ph-4-$CF_3$ |
| H | CH ($CH_3$) $CH_2SC$ (O) N (Et)$_2$ | $CF_3$ | Ph-4-$OCF_3$ |
| H | CH ($CH_3$) $CH_2SC$ (S) $NHCH_3$ | $CF_3$ | (L-45c) $CF_3$ |
| H | CH ($CH_3$) $CH_2SC$ (S) NHEt | $CF_3$ | Ph-4-Br |
| H | CH ($CH_3$) $CH_2SC$ (S) N ($CH_3$)$_2$ | $CF_3$ | Ph-4-$CF_3$ |
| H | CH ($CH_3$) $CH_2SPh$ | $CF_3$ | Ph-4-Br |
| H | CH ($CH_3$) $CH_2SPh$ | $CF_3$ | Ph-4-$CF_3$ |
| H | CH ($CH_3$) $CH_2SPh$ | $CF_3$ | Ph-4-$OCF_3$ |
| H | CH ($CH_3$) $CH_2SPh$ | $CF_3$ | (L-45c) $CF_3$ |
| H | CH ($CH_3$) $CH_2S$ (L-21a) | $CF_3$ | Ph-4-$OCF_3$ |
| H | CH ($CH_3$) $CH_2SO_2$ (L-21a) | $CF_3$ | (L-45c) $CF_3$ |
| H | CH ($CH_3$) $CH_2S$ (L-45a) | $CF_3$ | Ph-4-Br |

| | | | |
|---|---|---|---|
| H | $CH(CH_3)CH_2S$ (L-45a) | $CF_3$ | Ph-4-$CF_3$ |
| H | $CH(CH_3)CH_2S$ (L-45a) | $CF_3$ | Ph-4-$OCF_3$ |
| H | $CH(CH_3)CH_2S$ (L-45a) | $CF_3$ | (L-45c) $CF_3$ |
| H | $CH(CH_3)CH_2S(O)$ (L-45a) | $CF_3$ | Ph-4-Br |
| H | $CH(CH_3)CH_2S(O)$ (L-45a) | $CF_3$ | Ph-4-$CF_3$ |
| H | $CH(CH_3)CH_2S(O)$ (L-45a) | $CF_3$ | Ph-4-$OCF_3$ |
| H | $CH(CH_3)CH_2S(O)$ (L-45a) | $CF_3$ | (L-45c) $CF_3$ |
| H | $CH(CH_3)CH_2SO_2$ (L-45a) | $CF_3$ | Ph-4-Br |
| H | $CH(CH_3)CH_2SO_2$ (L-45a) | $CF_3$ | Ph-4-$CF_3$ |
| H | $CH(CH_3)CH_2SO_2$ (L-45a) | $CF_3$ | Ph-4-$OCF_3$ |
| H | $CH(CH_3)CH_2SO_2$ (L-45a) | $CF_3$ | (L-45c) $CF_3$ |
| H | $CH(CH_3)CH_2S$ (L-48a) | $CF_3$ | Ph-4-Br |
| H | $CH(CH_3)CH_2S(O)$ (L-48a) | $CF_3$ | Ph-4-$CF_3$ |
| H | $CH(CH_3)CH_2SO_2$ (L-48a) | $CF_3$ | Ph-4-$OCF_3$ |
| H | $CH(CH_3)CH_2S$ (L-48b) | $CF_3$ | (L-45c) $CF_3$ |
| H | $CH(CH_3)CH_2S(O)$ (L-48b) | $CF_3$ | Ph-4-Br |
| H | $CH(CH_3)CH_2SO_2$ (L-48b) | $CF_3$ | Ph-4-$CF_3$ |
| H | $CH(Et)CH_2SCH_3$ | $CF_3$ | (L-45c) $CF_3$ |
| H | $CH(CH_2SCH_3)_2$ | $CF_3$ | Ph-4-Br |
| H | $CH(Ph)CH_2SCH_3$ | $CF_3$ | Ph-4-$CF_3$ |
| H | $CH(Ph)CH_2S(O)CH_3$ | $CF_3$ | Ph-4-$OCF_3$ |
| H | $CH(Ph)CH_2SO_2CH_3$ | $CF_3$ | (L-45c) $CF_3$ |
| H | $CH(CH_3)CH(CH_3)SCH_3$ | $CF_3$ | Ph-4-Br |
| H | $CH(CH_3)CH(CH_3)SO_2CH_3$ | $CF_3$ | Ph-4-$CF_3$ |
| H | $CH(CH_3)CH(CH_3)SEt$ | $CF_3$ | Ph-4-$OCF_3$ |
| H | $CH(CH_3)CH(CH_3)SO_2Et$ | $CF_3$ | (L-45c) $CF_3$ |
| H | $C(CH_3)_2CH_2SCH_3$ | $CH_3$ | $CF_2O$(Ph-4-Cl) |
| H | $C(CH_3)_2CH_2SCH_3$ | $CH_3$ | CH=CH(Ph-4-Cl) |
| H | $C(CH_3)_2CH_2SCH_3$ | $CH_3$ | CH=CH(Ph-4-$OCF_3$) |
| H | $C(CH_3)_2CH_2SCH_3$ | $CH_3$ | CH=CH(Ph-3,4-$Cl_2$) |
| H | $C(CH_3)_2CH_2SCH_3$ | $CH_3$ | Ph-4-F |
| H | $C(CH_3)_2CH_2SCH_3$ | $CH_3$ | Ph-4-Cl |
| H | $C(CH_3)_2CH_2SCH_3$ | $CH_3$ | Ph-4-Br |
| H | $C(CH_3)_2CH_2SCH_3$ | $CH_3$ | Ph-4-I |
| H | $C(CH_3)_2CH_2SCH_3$ | $CH_3$ | Ph-4-$CF_3$ |
| H | $C(CH_3)_2CH_2SCH_3$ | $CH_3$ | Ph-4-$OCHF_2$ |
| H | $C(CH_3)_2CH_2SCH_3$ | $CH_3$ | Ph-4-$OCF_3$ |
| H | $C(CH_3)_2CH_2SCH_3$ | $CH_3$ | Ph-4-$OCF_2Br$ |
| H | $C(CH_3)_2CH_2SCH_3$ | $CH_3$ | Ph-4-$OCF_2CHF_2$ |
| H | $C(CH_3)_2CH_2SCH_3$ | $CH_3$ | Ph-4-$OCF_2CHFCl$ |
| H | $C(CH_3)_2CH_2SCH_3$ | $CH_3$ | Ph-4-$OCF_2CHFCF_3$ |

| | | | |
|---|---|---|---|
| H | $C(CH_3)_2CH_2SCH_3$ | $CH_3$ | $Ph-4-OCF_2CHFOCF_3$ |
| H | $C(CH_3)_2CH_2SCH_3$ | $CH_3$ | $Ph-4-OCF_2CHFOCF_2CF_2CF_3$ |
| H | $C(CH_3)_2CH_2SCH_3$ | $CH_3$ | Ph-4-O (L-45g) |
| H | $C(CH_3)_2CH_2SCH_3$ | $CH_3$ | $Ph-3,4-Cl_2$ |
| H | $C(CH_3)_2CH_2SCH_3$ | $CH_3$ | $Ph(-3-OCF_2O-4-)$ |
| H | $C(CH_3)_2CH_2SCH_3$ | $CH_3$ | $Ph(-3-OCF_2CF_2O-4-)$ |
| H | $C(CH_3)_2CH_2SCH_3$ | $CH_3$ | (L-45c) Cl |
| H | $C(CH_3)_2CH_2SCH_3$ | $CH_3$ | (L-45c) Br |
| H | $C(CH_3)_2CH_2SCH_3$ | $CH_3$ | (L-45c) $CF_3$ |
| H | $C(CH_3)_2CH_2SCH_3$ | $CH_3$ | L-45e |
| H | $C(CH_3)_2CH_2SCH_3$ | $CH_3$ | L-45f |
| H | $C(CH_3)_2CH_2SCH_3$ | $CH_3$ | (L-46c) Cl |
| H | $C(CH_3)_2CH_2SCH_3$ | $CH_3$ | (L-46c) Br |
| H | $C(CH_3)_2CH_2SCH_3$ | $CH_3$ | (L-46c) $CF_3$ |
| H | $C(CH_3)_2CH_2SCH_3$ | $CH_3$ | L-46d |
| H | $C(CH_3)_2CH_2SCH_3$ | Et | Ph-4-Cl |
| H | $C(CH_3)_2CH_2SCH_3$ | Et | Ph-4-Br |
| H | $C(CH_3)_2CH_2SCH_3$ | Et | $Ph-4-CF_3$ |
| H | $C(CH_3)_2CH_2SCH_3$ | Et | $Ph-4-OCF_3$ |
| H | $C(CH_3)_2CH_2SCH_3$ | Et | $Ph-4-OCF_2Br$ |
| H | $C(CH_3)_2CH_2SCH_3$ | Et | $Ph-4-OCF_2CHFCF_3$ |
| H | $C(CH_3)_2CH_2SCH_3$ | Et | $Ph-4-OCF_2CHFOCF_3$ |
| H | $C(CH_3)_2CH_2SCH_3$ | Et | $Ph-4-OCF_2CHFOCF_2CF_2CF_3$ |
| H | $C(CH_3)_2CH_2SCH_3$ | Et | Ph-4-O (L-45g) |
| H | $C(CH_3)_2CH_2SCH_3$ | Et | $Ph-3,4-Cl_2$ |
| H | $C(CH_3)_2CH_2SCH_3$ | Et | $Ph(-3-OCF_2O-4-)$ |
| H | $C(CH_3)_2CH_2SCH_3$ | Et | $Ph(-3-OCF_2CF_2O-4-)$ |
| H | $C(CH_3)_2CH_2SCH_3$ | Et | (L-45c) $CF_3$ |
| H | $C(CH_3)_2CH_2SCH_3$ | n-Pr | Ph-4-Br |
| H | $C(CH_3)_2CH_2SCH_3$ | n-Pr | $Ph-4-CF_3$ |
| H | $C(CH_3)_2CH_2SCH_3$ | n-Pr | $Ph-4-OCF_3$ |
| H | $C(CH_3)_2CH_2SCH_3$ | n-Pr | (L-45c) $CF_3$ |
| H | $C(CH_3)_2CH_2SCH_3$ | i-Pr | Ph-4-Br |
| H | $C(CH_3)_2CH_2SCH_3$ | i-Pr | $Ph-4-CF_3$ |
| H | $C(CH_3)_2CH_2SCH_3$ | i-Pr | $Ph-4-OCF_3$ |
| H | $C(CH_3)_2CH_2SCH_3$ | i-Pr | (L-45c) $CF_3$ |
| H | $C(CH_3)_2CH_2SCH_3$ | c-Pr | Ph-4-Br |
| H | $C(CH_3)_2CH_2SCH_3$ | c-Pr | $Ph-4-CF_3$ |
| H | $C(CH_3)_2CH_2SCH_3$ | c-Pr | $Ph-4-OCF_3$ |
| H | $C(CH_3)_2CH_2SCH_3$ | c-Pr | (L-45c) $CF_3$ |
| H | $C(CH_3)_2CH_2SCH_3$ | n-Bu | Ph-4-Br |

| | | | |
|---|---|---|---|
| H | $C(CH_3)_2CH_2SCH_3$ | $CH_2F$ | Ph-4-$CF_3$ |
| H | $C(CH_3)_2CH_2SCH_3$ | $CH_2Cl$ | Ph-4-$OCF_3$ |
| H | $C(CH_3)_2CH_2SCH_3$ | $CH_2Br$ | (L-45c) $CF_3$ |
| H | $C(CH_3)_2CH_2SCH_3$ | $CHF_2$ | Ph-4-Cl |
| H | $C(CH_3)_2CH_2SCH_3$ | $CHF_2$ | Ph-4-Br |
| H | $C(CH_3)_2CH_2SCH_3$ | $CHF_2$ | Ph-4-$CF_3$ |
| H | $C(CH_3)_2CH_2SCH_3$ | $CHF_2$ | Ph-4-$OCF_3$ |
| H | $C(CH_3)_2CH_2SCH_3$ | $CHF_2$ | Ph-4-$OCF_2Br$ |
| H | $C(CH_3)_2CH_2SCH_3$ | $CHF_2$ | Ph-4-$OCF_2CHFCF_3$ |
| H | $C(CH_3)_2CH_2SCH_3$ | $CHF_2$ | Ph-4-$OCF_2CHFOCF_3$ |
| H | $C(CH_3)_2CH_2SCH_3$ | $CHF_2$ | Ph-4-$OCF_2CHFOCF_2CF_2CF_3$ |
| H | $C(CH_3)_2CH_2SCH_3$ | $CHF_2$ | Ph-4-O (L-45g) |
| H | $C(CH_3)_2CH_2SCH_3$ | $CHF_2$ | Ph-3, 4-$Cl_2$ |
| H | $C(CH_3)_2CH_2SCH_3$ | $CHF_2$ | Ph (-3-$OCF_2O$-4-) |
| H | $C(CH_3)_2CH_2SCH_3$ | $CHF_2$ | Ph (-3-$OCF_2CF_2O$-4-) |
| H | $C(CH_3)_2CH_2SCH_3$ | $CHF_2$ | (L-45c) $CF_3$ |
| H | $C(CH_3)_2CH_2SCH_3$ | $CHF_2$ | L-45f |
| H | $C(CH_3)_2CH_2SCH_3$ | $CHF_2$ | (L-46c) $CF_3$ |
| H | $C(CH_3)_2CH_2SCH_3$ | $CHF_2$ | L-46d |
| H | $C(CH_3)_2CH_2SCH_3$ | CHFCl | Ph-4-Br |
| H | $C(CH_3)_2CH_2SCH_3$ | CHFBr | Ph-4-$CF_3$ |
| H | $C(CH_3)_2CH_2SCH_3$ | $CF_3$ | c-Pr |
| H | $C(CH_3)_2CH_2SCH_3$ | $CF_3$ | c-Bu |
| H | $C(CH_3)_2CH_2SCH_3$ | $CF_3$ | c-Pen |
| H | $C(CH_3)_2CH_2SCH_3$ | $CF_3$ | c-Hex |
| H | $C(CH_3)_2CH_2SCH_3$ | $CF_3$ | T-1 |
| H | $C(CH_3)_2CH_2SCH_3$ | $CF_3$ | T-2 |
| H | $C(CH_3)_2CH_2SCH_3$ | $CF_3$ | T-3 |
| H | $C(CH_3)_2CH_2SCH_3$ | $CF_3$ | T-4 |
| H | $C(CH_3)_2CH_2SCH_3$ | $CF_3$ | T-5 |
| H | $C(CH_3)_2CH_2SCH_3$ | $CF_3$ | $CH_2OCH_3$ |
| H | $C(CH_3)_2CH_2SCH_3$ | $CF_3$ | $CH_2OEt$ |
| H | $C(CH_3)_2CH_2SCH_3$ | $CF_3$ | $CH_2OPr$-n |
| H | $C(CH_3)_2CH_2SCH_3$ | $CF_3$ | $CH_2OPr$-i |
| H | $C(CH_3)_2CH_2SCH_3$ | $CF_3$ | $CH_2OBu$-n |
| H | $C(CH_3)_2CH_2SCH_3$ | $CF_3$ | $CH_2OC(O)$ (Ph-2-Cl) |
| H | $C(CH_3)_2CH_2SCH_3$ | $CF_3$ | $CH_2OC(O)$ (Ph-3-Cl) |
| H | $C(CH_3)_2CH_2SCH_3$ | $CF_3$ | $CH_2OC(O)$ (Ph-4-Cl) |
| H | $C(CH_3)_2CH_2SCH_3$ | $CF_3$ | $CH_2OPh$ |
| H | $C(CH_3)_2CH_2SCH_3$ | $CF_3$ | $CH_2O$ (Ph-2-F) |
| H | $C(CH_3)_2CH_2SCH_3$ | $CF_3$ | $CH_2O$ (Ph-3-F) |

| | | | |
|---|---|---|---|
| H | $C(CH_3)_2CH_2SCH_3$ | $CF_3$ | $CH_2O$ (Ph-4-F) |
| H | $C(CH_3)_2CH_2SCH_3$ | $CF_3$ | $CH_2O$ (Ph-2-Cl) |
| H | $C(CH_3)_2CH_2SCH_3$ | $CF_3$ | $CH_2O$ (Ph-3-Cl) |
| H | $C(CH_3)_2CH_2SCH_3$ | $CF_3$ | $CH_2O$ (Ph-4-Cl) |
| H | $C(CH_3)_2CH_2SCH_3$ | $CF_3$ | $CH_2O$ (Ph-2-Br) |
| H | $C(CH_3)_2CH_2SCH_3$ | $CF_3$ | $CH_2O$ (Ph-3-Br) |
| H | $C(CH_3)_2CH_2SCH_3$ | $CF_3$ | $CH_2O$ (Ph-4-Br) |
| H | $C(CH_3)_2CH_2SCH_3$ | $CF_3$ | $CH_2O$ (Ph-2-$CF_3$) |
| H | $C(CH_3)_2CH_2SCH_3$ | $CF_3$ | $CH_2O$ (Ph-3-$CF_3$) |
| H | $C(CH_3)_2CH_2SCH_3$ | $CF_3$ | $CH_2O$ (Ph-4-$CF_3$) |
| H | $C(CH_3)_2CH_2SCH_3$ | $CF_3$ | $CH_2O$ (Ph-2-$OCF_3$) |
| H | $C(CH_3)_2CH_2SCH_3$ | $CF_3$ | $CH_2O$ (Ph-3-$OCF_3$) |
| H | $C(CH_3)_2CH_2SCH_3$ | $CF_3$ | $CH_2O$ (Ph-4-$OCF_3$) |
| H | $C(CH_3)_2CH_2SCH_3$ | $CF_3$ | $CF_2OPh$ |
| H | $C(CH_3)_2CH_2SCH_3$ | $CF_3$ | $CF_2O$ (Ph-2-F) |
| H | $C(CH_3)_2CH_2SCH_3$ | $CF_3$ | $CF_2O$ (Ph-3-F) |
| H | $C(CH_3)_2CH_2SCH_3$ | $CF_3$ | $CF_2O$ (Ph-4-F) |
| H | $C(CH_3)_2CH_2SCH_3$ | $CF_3$ | $CF_2O$ (Ph-2-Cl) |
| H | $C(CH_3)_2CH_2SCH_3$ | $CF_3$ | $CF_2O$ (Ph-3-Cl) |
| H | $C(CH_3)_2CH_2SCH_3$ | $CF_3$ | $CF_2O$ (Ph-4-Cl) |
| H | $C(CH_3)_2CH_2SCH_3$ | $CF_3$ | $CF_2O$ (Ph-2-Br) |
| H | $C(CH_3)_2CH_2SCH_3$ | $CF_3$ | $CF_2O$ (Ph-3-Br) |
| H | $C(CH_3)_2CH_2SCH_3$ | $CF_3$ | $CF_2O$ (Ph-4-Br) |
| H | $C(CH_3)_2CH_2SCH_3$ | $CF_3$ | $CF_2O$ (Ph-2-$CF_3$) |
| H | $C(CH_3)_2CH_2SCH_3$ | $CF_3$ | $CF_2O$ (Ph-3-$CF_3$) |
| H | $C(CH_3)_2CH_2SCH_3$ | $CF_3$ | $CF_2O$ (Ph-4-$CF_3$) |
| H | $C(CH_3)_2CH_2SCH_3$ | $CF_3$ | $CF_2O$ (Ph-2-$OCF_3$) |
| H | $C(CH_3)_2CH_2SCH_3$ | $CF_3$ | $CF_2O$ (Ph-3-$OCF_3$) |
| H | $C(CH_3)_2CH_2SCH_3$ | $CF_3$ | $CF_2O$ (Ph-4-$OCF_3$) |
| H | $C(CH_3)_2CH_2SCH_3$ | $CF_3$ | $CH_2S$ (Ph-2-Cl) |
| H | $C(CH_3)_2CH_2SCH_3$ | $CF_3$ | $CH_2S$ (Ph-3-Cl) |
| H | $C(CH_3)_2CH_2SCH_3$ | $CF_3$ | $CH_2S$ (Ph-4-Cl) |
| H | $C(CH_3)_2CH_2SCH_3$ | $CF_3$ | $CH_2SO_2$ (Ph-2-Cl) |
| H | $C(CH_3)_2CH_2SCH_3$ | $CF_3$ | $CH_2SO_2$ (Ph-3-Cl) |
| H | $C(CH_3)_2CH_2SCH_3$ | $CF_3$ | $CH_2SO_2$ (Ph-4-Cl) |
| H | $C(CH_3)_2CH_2SCH_3$ | $CF_3$ | $CH_2N(CH_3)_2$ |
| H | $C(CH_3)_2CH_2SCH_3$ | $CF_3$ | $CH_2NH$ (Ph-2-Cl) |
| H | $C(CH_3)_2CH_2SCH_3$ | $CF_3$ | $CH_2NH$ (Ph-3-Cl) |
| H | $C(CH_3)_2CH_2SCH_3$ | $CF_3$ | $CH_2NH$ (Ph-4-Cl) |
| H | $C(CH_3)_2CH_2SCH_3$ | $CF_3$ | $CF_2C(O)OEt$ |
| H | $C(CH_3)_2CH_2SCH_3$ | $CF_3$ | $CH_2$ (L-5a) |

| | | | |
|---|---|---|---|
| H | $C(CH_3)_2CH_2SCH_3$ | $CF_3$ | $CH_2$(L-14a) |
| H | $C(CH_3)_2CH_2SCH_3$ | $CF_3$ | $CH_2$(L-24a) |
| H | $C(CH_3)_2CH_2SCH_3$ | $CF_3$ | $CH_2$(L-36a) |
| H | $C(CH_3)_2CH_2SCH_3$ | $CF_3$ | $CH_2CH_2Ph$ |
| H | $C(CH_3)_2CH_2SCH_3$ | $CF_3$ | $CH_2CH_2$(Ph-2-Cl) |
| H | $C(CH_3)_2CH_2SCH_3$ | $CF_3$ | $CH_2CH_2$(Ph-3-Cl) |
| H | $C(CH_3)_2CH_2SCH_3$ | $CF_3$ | $CH_2CH_2$(Ph-4-Cl) |
| H | $C(CH_3)_2CH_2SCH_3$ | $CF_3$ | T-22 |
| H | $C(CH_3)_2CH_2SCH_3$ | $CF_3$ | T-23 |
| H | $C(CH_3)_2CH_2SCH_3$ | $CF_3$ | T-24 |
| H | $C(CH_3)_2CH_2SCH_3$ | $CF_3$ | C(O)OEt |
| H | $C(CH_3)_2CH_2SCH_3$ | $CF_3$ | C(O)OBu-t |
| H | $C(CH_3)_2CH_2SCH_3$ | $CF_3$ | $C(O)OCH_2CF_3$ |
| H | $C(CH_3)_2CH_2SCH_3$ | $CF_3$ | CH=CH(Ph-2-F) |
| H | $C(CH_3)_2CH_2SCH_3$ | $CF_3$ | CH=CH(Ph-3-F) |
| H | $C(CH_3)_2CH_2SCH_3$ | $CF_3$ | CH=CH(Ph-4-F) |
| H | $C(CH_3)_2CH_2SCH_3$ | $CF_3$ | CH=CH(Ph-2-Cl) |
| H | $C(CH_3)_2CH_2SCH_3$ | $CF_3$ | CH=CH(Ph-3-Cl) |
| H | $C(CH_3)_2CH_2SCH_3$ | $CF_3$ | CH=CH(Ph-4-Cl) |
| H | $C(CH_3)_2CH_2SCH_3$ | $CF_3$ | CH=CH(Ph-2-Br) |
| H | $C(CH_3)_2CH_2SCH_3$ | $CF_3$ | CH=CH(Ph-3-Br) |
| H | $C(CH_3)_2CH_2SCH_3$ | $CF_3$ | CH=CH(Ph-4-Br) |
| H | $C(CH_3)_2CH_2SCH_3$ | $CF_3$ | CH=CH(Ph-2-$CF_3$) |
| H | $C(CH_3)_2CH_2SCH_3$ | $CF_3$ | CH=CH(Ph-3-$CF_3$) |
| H | $C(CH_3)_2CH_2SCH_3$ | $CF_3$ | CH=CH(Ph-4-$CF_3$) |
| H | $C(CH_3)_2CH_2SCH_3$ | $CF_3$ | CH=CH(Ph-2-$OCF_3$) |
| H | $C(CH_3)_2CH_2SCH_3$ | $CF_3$ | CH=CH(Ph-3-$OCF_3$) |
| H | $C(CH_3)_2CH_2SCH_3$ | $CF_3$ | CH=CH(Ph-4-$OCF_3$) |
| H | $C(CH_3)_2CH_2SCH_3$ | $CF_3$ | CH=CH(Ph-2-$SCH_3$) |
| H | $C(CH_3)_2CH_2SCH_3$ | $CF_3$ | CH=CH(Ph-3-$SCH_3$) |
| H | $C(CH_3)_2CH_2SCH_3$ | $CF_3$ | CH=CH(Ph-4-$SCH_3$) |
| H | $C(CH_3)_2CH_2SCH_3$ | $CF_3$ | CH=CH(Ph-2-$SO_2CH_3$) |
| H | $C(CH_3)_2CH_2SCH_3$ | $CF_3$ | CH=CH(Ph-3-$SO_2CH_3$) |
| H | $C(CH_3)_2CH_2SCH_3$ | $CF_3$ | CH=CH(Ph-4-$SO_2CH_3$) |
| H | $C(CH_3)_2CH_2SCH_3$ | $CF_3$ | CH=CH(Ph-3,4-$F_2$) |
| H | $C(CH_3)_2CH_2SCH_3$ | $CF_3$ | CH=CH(Ph-3-F-4-Cl) |
| H | $C(CH_3)_2CH_2SCH_3$ | $CF_3$ | CH=CH(Ph-3,4-$Cl_2$) |
| H | $C(CH_3)_2CH_2SCH_3$ | $CF_3$ | CH=CH(Ph-3,4-$Br_2$) |
| H | $C(CH_3)_2CH_2SCH_3$ | $CF_3$ | CH=CH(Ph-3-F-4-$CF_3$) |
| H | $C(CH_3)_2CH_2SCH_3$ | $CF_3$ | CH=CH(Ph-3-Cl-4-$OCF_3$) |
| H | $C(CH_3)_2CH_2SCH_3$ | $CF_3$ | CH=CH[Ph(-3-$OCF_2O$-4-)] |

| | | | |
|---|---|---|---|
| H | $C(CH_3)_2CH_2SCH_3$ | $CF_3$ | Ph |
| H | $C(CH_3)_2CH_2SCH_3$ | $CF_3$ | Ph-2-F |
| H | $C(CH_3)_2CH_2SCH_3$ | $CF_3$ | Ph-3-F |
| H | $C(CH_3)_2CH_2SCH_3$ | $CF_3$ | Ph-4-F |
| H | $C(CH_3)_2CH_2SCH_3$ | $CF_3$ | Ph-2-Cl |
| H | $C(CH_3)_2CH_2SCH_3$ | $CF_3$ | Ph-3-Cl |
| H | $C(CH_3)_2CH_2SCH_3$ | $CF_3$ | Ph-4-Cl |
| H | $C(CH_3)_2CH_2SCH_3$ | $CF_3$ | Ph-2-Br |
| H | $C(CH_3)_2CH_2SCH_3$ | $CF_3$ | Ph-3-Br |
| H | $C(CH_3)_2CH_2SCH_3$ | $CF_3$ | Ph-4-Br |
| H | $C(CH_3)_2CH_2SCH_3$ | $CF_3$ | Ph-4-I |
| H | $C(CH_3)_2CH_2SCH_3$ | $CF_3$ | Ph-4-Bu-t |
| H | $C(CH_3)_2CH_2SCH_3$ | $CF_3$ | Ph-2-$CF_3$ |
| H | $C(CH_3)_2CH_2SCH_3$ | $CF_3$ | Ph-3-$CF_3$ |
| H | $C(CH_3)_2CH_2SCH_3$ | $CF_3$ | Ph-4-$CF_3$ |
| H | $C(CH_3)_2CH_2SCH_3$ | $CF_3$ | Ph-4-$OCH_3$ |
| H | $C(CH_3)_2CH_2SCH_3$ | $CF_3$ | Ph-4-$OCHF_2$ |
| H | $C(CH_3)_2CH_2SCH_3$ | $CF_3$ | Ph-2-$OCF_3$ |
| H | $C(CH_3)_2CH_2SCH_3$ | $CF_3$ | Ph-3-$OCF_3$ |
| H | $C(CH_3)_2CH_2SCH_3$ | $CF_3$ | Ph-4-$OCF_3$ |
| H | $C(CH_3)_2CH_2SCH_3$ | $CF_3$ | Ph-4-$OCF_2Br$ |
| H | $C(CH_3)_2CH_2SCH_3$ | $CF_3$ | Ph-4-$OCH_2CF_3$ |
| H | $C(CH_3)_2CH_2SCH_3$ | $CF_3$ | Ph-4-$OCF_2CHF_2$ |
| H | $C(CH_3)_2CH_2SCH_3$ | $CF_3$ | Ph-4-$OCF_2CHFCl$ |
| H | $C(CH_3)_2CH_2SCH_3$ | $CF_3$ | Ph-4-$OCF_2CHFBr$ |
| H | $C(CH_3)_2CH_2SCH_3$ | $CF_3$ | Ph-4-$OCF_2CF_2Br$ |
| H | $C(CH_3)_2CH_2SCH_3$ | $CF_3$ | Ph-4-$OCF_2CFCl_2$ |
| H | $C(CH_3)_2CH_2SCH_3$ | $CF_3$ | Ph-4-$OCF_2CCl_3$ |
| H | $C(CH_3)_2CH_2SCH_3$ | $CF_3$ | Ph-4-$OCH_2CF_2CHF_2$ |
| H | $C(CH_3)_2CH_2SCH_3$ | $CF_3$ | Ph-3-$OCF_2CHFCF_3$ |
| H | $C(CH_3)_2CH_2SCH_3$ | $CF_3$ | Ph-4-$OCF_2CHFCF_3$ |
| H | $C(CH_3)_2CH_2SCH_3$ | $CF_3$ | Ph-4-$OCH(CF_3)_2$ |
| H | $C(CH_3)_2CH_2SCH_3$ | $CF_3$ | Ph-4-$OCF_2CFBrCF_3$ |
| H | $C(CH_3)_2CH_2SCH_3$ | $CF_3$ | Ph-3-$OCF_2CHFOCF_3$ |
| H | $C(CH_3)_2CH_2SCH_3$ | $CF_3$ | Ph-4-$OCF_2CHFOCF_3$ |
| H | $C(CH_3)_2CH_2SCH_3$ | $CF_3$ | Ph-4-$OCF_2CHFOCF_2CF_2CF_3$ |
| H | $C(CH_3)_2CH_2SCH_3$ | $CF_3$ | Ph-4-$OSO_2CH_3$ |
| H | $C(CH_3)_2CH_2SCH_3$ | $CF_3$ | Ph-4-$OSO_2CF_3$ |
| H | $C(CH_3)_2CH_2SCH_3$ | $CF_3$ | Ph-3-O(Ph-4-Cl) |
| H | $C(CH_3)_2CH_2SCH_3$ | $CF_3$ | Ph-4-O(Ph-4-Cl) |
| H | $C(CH_3)_2CH_2SCH_3$ | $CF_3$ | Ph-4-O(Ph-4-Br) |

| | | | |
|---|---|---|---|
| H | $C(CH_3)_2CH_2SCH_3$ | $CF_3$ | Ph-4-O(Ph-4-$CF_3$) |
| H | $C(CH_3)_2CH_2SCH_3$ | $CF_3$ | Ph-4-O(L-45c)Br |
| H | $C(CH_3)_2CH_2SCH_3$ | $CF_3$ | Ph-4-O(L-45c)$CF_3$ |
| H | $C(CH_3)_2CH_2SCH_3$ | $CF_3$ | Ph-3-O(L-45e) |
| H | $C(CH_3)_2CH_2SCH_3$ | $CF_3$ | Ph-4-O(L-45e) |
| H | $C(CH_3)_2CH_2SCH_3$ | $CF_3$ | Ph-4-O(L-48b)Br |
| H | $C(CH_3)_2CH_2SCH_3$ | $CF_3$ | Ph-4-$SCH_3$ |
| H | $C(CH_3)_2CH_2SCH_3$ | $CF_3$ | Ph-4-$SO_2CH_3$ |
| H | $C(CH_3)_2CH_2SCH_3$ | $CF_3$ | Ph-3-S(Ph-4-Cl) |
| H | $C(CH_3)_2CH_2SCH_3$ | $CF_3$ | Ph-4-S(Ph-4-Cl) |
| H | $C(CH_3)_2CH_2SCH_3$ | $CF_3$ | Ph-4-S(Ph-4-Br) |
| H | $C(CH_3)_2CH_2SCH_3$ | $CF_3$ | Ph-4-S(Ph-4-$CF_3$) |
| H | $C(CH_3)_2CH_2SCH_3$ | $CF_3$ | Ph-4-S(L-45c)Br |
| H | $C(CH_3)_2CH_2SCH_3$ | $CF_3$ | Ph-4-S(L-45c)$CF_3$ |
| H | $C(CH_3)_2CH_2SCH_3$ | $CF_3$ | Ph-4-S(L-45e) |
| H | $C(CH_3)_2CH_2SCH_3$ | $CF_3$ | Ph-4-S(L-48b)Br |
| H | $C(CH_3)_2CH_2SCH_3$ | $CF_3$ | Ph-4-$NO_2$ |
| H | $C(CH_3)_2CH_2SCH_3$ | $CF_3$ | Ph-4-CN |
| H | $C(CH_3)_2CH_2SCH_3$ | $CF_3$ | Ph-2,3-$F_2$ |
| H | $C(CH_3)_2CH_2SCH_3$ | $CF_3$ | Ph-2,4-$F_2$ |
| H | $C(CH_3)_2CH_2SCH_3$ | $CF_3$ | Ph-3,4-$F_2$ |
| H | $C(CH_3)_2CH_2SCH_3$ | $CF_3$ | Ph-2,5-$F_2$ |
| H | $C(CH_3)_2CH_2SCH_3$ | $CF_3$ | Ph-3,5-$F_2$ |
| H | $C(CH_3)_2CH_2SCH_3$ | $CF_3$ | Ph-2-Cl-4-F |
| H | $C(CH_3)_2CH_2SCH_3$ | $CF_3$ | Ph-2-F-3-Cl |
| H | $C(CH_3)_2CH_2SCH_3$ | $CF_3$ | Ph-3-Cl-4-F |
| H | $C(CH_3)_2CH_2SCH_3$ | $CF_3$ | Ph-2-F-4-Cl |
| H | $C(CH_3)_2CH_2SCH_3$ | $CF_3$ | Ph-3-F-4-Cl |
| H | $C(CH_3)_2CH_2SCH_3$ | $CF_3$ | Ph-2,3-$Cl_2$ |
| H | $C(CH_3)_2CH_2SCH_3$ | $CF_3$ | Ph-2,4-$Cl_2$ |
| H | $C(CH_3)_2CH_2SCH_3$ | $CF_3$ | Ph-2,5-$Cl_2$ |
| H | $C(CH_3)_2CH_2SCH_3$ | $CF_3$ | Ph-3,4-$Cl_2$ |
| H | $C(CH_3)_2CH_2SCH_3$ | $CF_3$ | Ph-3,5-$Cl_2$ |
| H | $C(CH_3)_2CH_2SCH_3$ | $CF_3$ | Ph-3-Br-4-F |
| H | $C(CH_3)_2CH_2SCH_3$ | $CF_3$ | Ph-2-F-4-Br |
| H | $C(CH_3)_2CH_2SCH_3$ | $CF_3$ | Ph-2-F-5-Br |
| H | $C(CH_3)_2CH_2SCH_3$ | $CF_3$ | Ph-3,4-$Br_2$ |
| H | $C(CH_3)_2CH_2SCH_3$ | $CF_3$ | Ph-3,5-$Br_2$ |
| H | $C(CH_3)_2CH_2SCH_3$ | $CF_3$ | Ph-3-$CH_3$-4-F |
| H | $C(CH_3)_2CH_2SCH_3$ | $CF_3$ | Ph-3-F-4-$CH_3$ |
| H | $C(CH_3)_2CH_2SCH_3$ | $CF_3$ | Ph-2-F-5-$CH_3$ |

| | | | |
|---|---|---|---|
| H | $C(CH_3)_2CH_2SCH_3$ | $CF_3$ | Ph-2, 4-$(CH_3)_2$ |
| H | $C(CH_3)_2CH_2SCH_3$ | $CF_3$ | Ph-3, 4-$(CH_3)_2$ |
| H | $C(CH_3)_2CH_2SCH_3$ | $CF_3$ | Ph-2-F-3-$CF_3$ |
| H | $C(CH_3)_2CH_2SCH_3$ | $CF_3$ | Ph-3-$CF_3$-4-F |
| H | $C(CH_3)_2CH_2SCH_3$ | $CF_3$ | Ph-3-$CF_3$-4-Cl |
| H | $C(CH_3)_2CH_2SCH_3$ | $CF_3$ | Ph-2-F-4-$CF_3$ |
| H | $C(CH_3)_2CH_2SCH_3$ | $CF_3$ | Ph-3-F-4-$CF_3$ |
| H | $C(CH_3)_2CH_2SCH_3$ | $CF_3$ | Ph-2-F-5-$CF_3$ |
| H | $C(CH_3)_2CH_2SCH_3$ | $CF_3$ | Ph-3-F-5-$CF_3$ |
| H | $C(CH_3)_2CH_2SCH_3$ | $CF_3$ | Ph-2-Cl-4-$CF_3$ |
| H | $C(CH_3)_2CH_2SCH_3$ | $CF_3$ | Ph-3, 5-$(CF_3)_2$ |
| H | $C(CH_3)_2CH_2SCH_3$ | $CF_3$ | Ph-3-Br-4-$OCH_3$ |
| H | $C(CH_3)_2CH_2SCH_3$ | $CF_3$ | Ph-3-F-4-$OCHF_2$ |
| H | $C(CH_3)_2CH_2SCH_3$ | $CF_3$ | Ph-3-Cl-4-$OCHF_2$ |
| H | $C(CH_3)_2CH_2SCH_3$ | $CF_3$ | Ph-3-Br-4-$OCHF_2$ |
| H | $C(CH_3)_2CH_2SCH_3$ | $CF_3$ | Ph-3-F-4-$OCF_3$ |
| H | $C(CH_3)_2CH_2SCH_3$ | $CF_3$ | Ph-3-Cl-4-$OCF_3$ |
| H | $C(CH_3)_2CH_2SCH_3$ | $CF_3$ | Ph-3-Br-4-$OCF_3$ |
| H | $C(CH_3)_2CH_2SCH_3$ | $CF_3$ | Ph-3-F-4-$OCF_2Br$ |
| H | $C(CH_3)_2CH_2SCH_3$ | $CF_3$ | Ph-3-Cl-4-$OCF_2Br$ |
| H | $C(CH_3)_2CH_2SCH_3$ | $CF_3$ | Ph-3-Br-4-$OCF_2Br$ |
| H | $C(CH_3)_2CH_2SCH_3$ | $CF_3$ | Ph-3-F-4-$OCF_2CHF_2$ |
| H | $C(CH_3)_2CH_2SCH_3$ | $CF_3$ | Ph-3-Cl-4-$OCF_2CHF_2$ |
| H | $C(CH_3)_2CH_2SCH_3$ | $CF_3$ | Ph-3-Br-4-$OCF_2CHF_2$ |
| H | $C(CH_3)_2CH_2SCH_3$ | $CF_3$ | Ph-3-F-4-$OCF_2CHFCl$ |
| H | $C(CH_3)_2CH_2SCH_3$ | $CF_3$ | Ph-3-Cl-4-$OCF_2CHFCl$ |
| H | $C(CH_3)_2CH_2SCH_3$ | $CF_3$ | Ph-3-Br-4-$OCF_2CHFCl$ |
| H | $C(CH_3)_2CH_2SCH_3$ | $CF_3$ | Ph-3-F-4-$OCF_2CHFCF_3$ |
| H | $C(CH_3)_2CH_2SCH_3$ | $CF_3$ | Ph-3-Cl-4-$OCF_2CHFCF_3$ |
| H | $C(CH_3)_2CH_2SCH_3$ | $CF_3$ | Ph-3-Br-4-$OCF_2CHFCF_3$ |
| H | $C(CH_3)_2CH_2SCH_3$ | $CF_3$ | Ph-3-F-4-$OCF_2CHFOCF_3$ |
| H | $C(CH_3)_2CH_2SCH_3$ | $CF_3$ | Ph-3-Cl-4-$OCF_2CHFOCF_3$ |
| H | $C(CH_3)_2CH_2SCH_3$ | $CF_3$ | Ph-3-Br-4-$OCF_2CHFOCF_3$ |
| H | $C(CH_3)_2CH_2SCH_3$ | $CF_3$ | Ph-3-$CH_3$-4-$OCF_2CHFOCF_3$ |
| H | $C(CH_3)_2CH_2SCH_3$ | $CF_3$ | Ph-3-F-4-$OCF_2CHFOCF_2CF_2CF_3$ |
| H | $C(CH_3)_2CH_2SCH_3$ | $CF_3$ | Ph-3-Cl-4-$OCF_2CHFOCF_2CF_2CF_3$ |
| H | $C(CH_3)_2CH_2SCH_3$ | $CF_3$ | Ph-3-Br-4-$OCF_2CHFOCF_2CF_2CF_3$ |
| H | $C(CH_3)_2CH_2SCH_3$ | $CF_3$ | Ph(-3-$OCF_2O$-4-) |
| H | $C(CH_3)_2CH_2SCH_3$ | $CF_3$ | Ph(-3-$OCF_2CF_2O$-4-) |
| H | $C(CH_3)_2CH_2SCH_3$ | $CF_3$ | Ph-3-OPh-4-F |
| H | $C(CH_3)_2CH_2SCH_3$ | $CF_3$ | Ph-3-$NO_2$-4-F |

| | | | |
|---|---|---|---|
| H | $C(CH_3)_2CH_2SCH_3$ | $CF_3$ | Ph-3-$NO_2$-4-Cl |
| H | $C(CH_3)_2CH_2SCH_3$ | $CF_3$ | Ph-2-F-5-$NO_2$ |
| H | $C(CH_3)_2CH_2SCH_3$ | $CF_3$ | Ph-3-CN-4-F |
| H | $C(CH_3)_2CH_2SCH_3$ | $CF_3$ | Ph-2, 3, 4-$F_3$ |
| H | $C(CH_3)_2CH_2SCH_3$ | $CF_3$ | Ph-2, 3, 5-$F_3$ |
| H | $C(CH_3)_2CH_2SCH_3$ | $CF_3$ | Ph-2, 4, 5-$F_3$ |
| H | $C(CH_3)_2CH_2SCH_3$ | $CF_3$ | Ph-3, 4, 5-$F_3$ |
| H | $C(CH_3)_2CH_2SCH_3$ | $CF_3$ | Ph-2, 3-$F_2$-4-$CH_3$ |
| H | $C(CH_3)_2CH_2SCH_3$ | $CF_3$ | Ph-2, 3-$F_2$-4-$CF_3$ |
| H | $C(CH_3)_2CH_2SCH_3$ | $CF_3$ | Ph-3, 4-$F_2$-5-$CF_3$ |
| H | $C(CH_3)_2CH_2SCH_3$ | $CF_3$ | Ph-2-F-3-Cl-5-$CF_3$ |
| H | $C(CH_3)_2CH_2SCH_3$ | $CF_3$ | Ph-3, 5-$Cl_2$-4-$OCH_3$ |
| H | $C(CH_3)_2CH_2SCH_3$ | $CF_3$ | 1-Naph |
| H | $C(CH_3)_2CH_2SCH_3$ | $CF_3$ | 2-Naph |
| H | $C(CH_3)_2CH_2SCH_3$ | $CF_3$ | (L-1b) Br |
| H | $C(CH_3)_2CH_2SCH_3$ | $CF_3$ | (L-1c) Cl |
| H | $C(CH_3)_2CH_2SCH_3$ | $CF_3$ | (L-1c) Br |
| H | $C(CH_3)_2CH_2SCH_3$ | $CF_3$ | (L-1c) I |
| H | $C(CH_3)_2CH_2SCH_3$ | $CF_3$ | (L-1c) $CF_3$ |
| H | $C(CH_3)_2CH_2SCH_3$ | $CF_3$ | (L-2b) Br |
| H | $C(CH_3)_2CH_2SCH_3$ | $CF_3$ | (L-3b) Cl |
| H | $C(CH_3)_2CH_2SCH_3$ | $CF_3$ | (L-3b) Br |
| H | $C(CH_3)_2CH_2SCH_3$ | $CF_3$ | (L-3c) F |
| H | $C(CH_3)_2CH_2SCH_3$ | $CF_3$ | (L-3c) Cl |
| H | $C(CH_3)_2CH_2SCH_3$ | $CF_3$ | (L-3c) Br |
| H | $C(CH_3)_2CH_2SCH_3$ | $CF_3$ | (L-3c) I |
| H | $C(CH_3)_2CH_2SCH_3$ | $CF_3$ | (L-3c) $CF_3$ |
| H | $C(CH_3)_2CH_2SCH_3$ | $CF_3$ | (L-3c) CN |
| H | $C(CH_3)_2CH_2SCH_3$ | $CF_3$ | L-3d |
| H | $C(CH_3)_2CH_2SCH_3$ | $CF_3$ | (L-4b) Cl |
| H | $C(CH_3)_2CH_2SCH_3$ | $CF_3$ | (L-4b) Br |
| H | $C(CH_3)_2CH_2SCH_3$ | $CF_3$ | (L-4b) CN |
| H | $C(CH_3)_2CH_2SCH_3$ | $CF_3$ | (L-10b) Cl |
| H | $C(CH_3)_2CH_2SCH_3$ | $CF_3$ | (L-10b) Br |
| H | $C(CH_3)_2CH_2SCH_3$ | $CF_3$ | (L-15b) $CF_3$ |
| H | $C(CH_3)_2CH_2SCH_3$ | $CF_3$ | (L-16a) $CHF_2$ |
| H | $C(CH_3)_2CH_2SCH_3$ | $CF_3$ | (L-16a) $CF_2Br$ |
| H | $C(CH_3)_2CH_2SCH_3$ | $CF_3$ | (L-17a) Cl |
| H | $C(CH_3)_2CH_2SCH_3$ | $CF_3$ | (L-21b) Cl |
| H | $C(CH_3)_2CH_2SCH_3$ | $CF_3$ | (L-21b) Br |
| H | $C(CH_3)_2CH_2SCH_3$ | $CF_3$ | (L-21b) I |

| | | | |
|---|---|---|---|
| H | $C(CH_3)_2CH_2SCH_3$ | $CF_3$ | (L-21b) $CF_3$ |
| H | $C(CH_3)_2CH_2SCH_3$ | $CF_3$ | (L-22b) Cl |
| H | $C(CH_3)_2CH_2SCH_3$ | $CF_3$ | (L-22b) Br |
| H | $C(CH_3)_2CH_2SCH_3$ | $CF_3$ | (L-23b) Cl |
| H | $C(CH_3)_2CH_2SCH_3$ | $CF_3$ | (L-23b) Br |
| H | $C(CH_3)_2CH_2SCH_3$ | $CF_3$ | (L-23c) Cl |
| H | $C(CH_3)_2CH_2SCH_3$ | $CF_3$ | (L-23c) Br |
| H | $C(CH_3)_2CH_2SCH_3$ | $CF_3$ | (L-31a) Cl |
| H | $C(CH_3)_2CH_2SCH_3$ | $CF_3$ | (L-31a) Br |
| H | $C(CH_3)_2CH_2SCH_3$ | $CF_3$ | (L-45c) F |
| H | $C(CH_3)_2CH_2SCH_3$ | $CF_3$ | (L-45c) Cl |
| H | $C(CH_3)_2CH_2SCH_3$ | $CF_3$ | (L-45c) Br |
| H | $C(CH_3)_2CH_2SCH_3$ | $CF_3$ | (L-45c) I |
| H | $C(CH_3)_2CH_2SCH_3$ | $CF_3$ | (L-45c) $CF_3$ |
| H | $C(CH_3)_2CH_2SCH_3$ | $CF_3$ | L-45e |
| H | $C(CH_3)_2CH_2SCH_3$ | $CF_3$ | L-45f |
| H | $C(CH_3)_2CH_2SCH_3$ | $CF_3$ | (L-46c) F |
| H | $C(CH_3)_2CH_2SCH_3$ | $CF_3$ | (L-46c) Cl |
| H | $C(CH_3)_2CH_2SCH_3$ | $CF_3$ | (L-46c) Br |
| H | $C(CH_3)_2CH_2SCH_3$ | $CF_3$ | (L-46c) I |
| H | $C(CH_3)_2CH_2SCH_3$ | $CF_3$ | (L-46c) $CF_3$ |
| H | $C(CH_3)_2CH_2SCH_3$ | $CF_3$ | (L-46c) $OCH_2CF_3$ |
| H | $C(CH_3)_2CH_2SCH_3$ | $CF_3$ | (L-46c) $OCH(CF_3)_2$ |
| H | $C(CH_3)_2CH_2SCH_3$ | $CF_3$ | L-46d |
| H | $C(CH_3)_2CH_2SCH_3$ | $CF_3$ | L-47a |
| H | $C(CH_3)_2CH_2SCH_3$ | $CF_3$ | L-47d |
| H | $C(CH_3)_2CH_2SCH_3$ | $CF_3$ | (L-48b) Br |
| H | $C(CH_3)_2CH_2SCH_3$ | $CF_3$ | (L-50b) Cl |
| H | $C(CH_3)_2CH_2SCH_3$ | $CF_3$ | (L-50b) Br |
| H | $C(CH_3)_2CH_2SCH_3$ | $CF_3$ | (L-51b) Cl |
| H | $C(CH_3)_2CH_2SCH_3$ | $CF_2Cl$ | Ph-4-Cl |
| H | $C(CH_3)_2CH_2SCH_3$ | $CF_2Cl$ | Ph-4-Br |
| H | $C(CH_3)_2CH_2SCH_3$ | $CF_2Cl$ | Ph-4-$CF_3$ |
| H | $C(CH_3)_2CH_2SCH_3$ | $CF_2Cl$ | Ph-4-$OCF_3$ |
| H | $C(CH_3)_2CH_2SCH_3$ | $CF_2Cl$ | Ph-4-$OCF_2Br$ |
| H | $C(CH_3)_2CH_2SCH_3$ | $CF_2Cl$ | Ph-4-$OCF_2CHFCF_3$ |
| H | $C(CH_3)_2CH_2SCH_3$ | $CF_2Cl$ | Ph-4-$OCF_2CHFOCF_3$ |
| H | $C(CH_3)_2CH_2SCH_3$ | $CF_2Cl$ | Ph-4-$OCF_2CHFOCF_2CF_2CF_3$ |
| H | $C(CH_3)_2CH_2SCH_3$ | $CF_2Cl$ | Ph-4-O(L-45g) |
| H | $C(CH_3)_2CH_2SCH_3$ | $CF_2Cl$ | Ph-3, 4-$Cl_2$ |
| H | $C(CH_3)_2CH_2SCH_3$ | $CF_2Cl$ | Ph(-3-$OCF_2O$-4-) |

| | | | |
|---|---|---|---|
| H | $C(CH_3)_2CH_2SCH_3$ | $CF_2Cl$ | Ph(-3-$OCF_2CF_2O$-4-) |
| H | $C(CH_3)_2CH_2SCH_3$ | $CF_2Cl$ | (L-45c)$CF_3$ |
| H | $C(CH_3)_2CH_2SCH_3$ | $CF_2Cl$ | L-45f |
| H | $C(CH_3)_2CH_2SCH_3$ | $CF_2Cl$ | (L-46c)$CF_3$ |
| H | $C(CH_3)_2CH_2SCH_3$ | $CF_2Cl$ | L-46d |
| H | $C(CH_3)_2CH_2SCH_3$ | $CFCl_2$ | Ph-4-$OCF_3$ |
| H | $C(CH_3)_2CH_2SCH_3$ | $CF_2Br$ | Ph-4-Cl |
| H | $C(CH_3)_2CH_2SCH_3$ | $CF_2Br$ | Ph-4-Br |
| H | $C(CH_3)_2CH_2SCH_3$ | $CF_2Br$ | Ph-4-$CF_3$ |
| H | $C(CH_3)_2CH_2SCH_3$ | $CF_2Br$ | Ph-4-$OCF_3$ |
| H | $C(CH_3)_2CH_2SCH_3$ | $CF_2Br$ | Ph-4-$OCF_2Br$ |
| H | $C(CH_3)_2CH_2SCH_3$ | $CF_2Br$ | Ph-4-$OCF_2CHFCF_3$ |
| H | $C(CH_3)_2CH_2SCH_3$ | $CF_2Br$ | Ph-4-$OCF_2CHFOCF_3$ |
| H | $C(CH_3)_2CH_2SCH_3$ | $CF_2Br$ | Ph-4-$OCF_2CHFOCF_2CF_2CF_3$ |
| H | $C(CH_3)_2CH_2SCH_3$ | $CF_2Br$ | Ph-4-O(L-45g) |
| H | $C(CH_3)_2CH_2SCH_3$ | $CF_2Br$ | Ph-3,4-$Cl_2$ |
| H | $C(CH_3)_2CH_2SCH_3$ | $CF_2Br$ | Ph(-3-$OCF_2O$-4-) |
| H | $C(CH_3)_2CH_2SCH_3$ | $CF_2Br$ | Ph(-3-$OCF_2CF_2O$-4-) |
| H | $C(CH_3)_2CH_2SCH_3$ | $CF_2Br$ | (L-45c)$CF_3$ |
| H | $C(CH_3)_2CH_2SCH_3$ | $CF_2Br$ | L-45f |
| H | $C(CH_3)_2CH_2SCH_3$ | $CF_2Br$ | (L-46c)$CF_3$ |
| H | $C(CH_3)_2CH_2SCH_3$ | $CF_2Br$ | L-46d |
| H | $C(CH_3)_2CH_2SCH_3$ | $CFClBr$ | (L-45c)$CF_3$ |
| H | $C(CH_3)_2CH_2SCH_3$ | $CFBr_2$ | Ph-4-Br |
| H | $C(CH_3)_2CH_2SCH_3$ | $CF_2CHF_2$ | Ph-4-$CF_3$ |
| H | $C(CH_3)_2CH_2SCH_3$ | $CF_2CF_3$ | Ph-4-Cl |
| H | $C(CH_3)_2CH_2SCH_3$ | $CF_2CF_3$ | Ph-4-Br |
| H | $C(CH_3)_2CH_2SCH_3$ | $CF_2CF_3$ | Ph-4-$CF_3$ |
| H | $C(CH_3)_2CH_2SCH_3$ | $CF_2CF_3$ | Ph-4-$OCF_3$ |
| H | $C(CH_3)_2CH_2SCH_3$ | $CF_2CF_3$ | Ph-4-$OCF_2Br$ |
| H | $C(CH_3)_2CH_2SCH_3$ | $CF_2CF_3$ | Ph-4-$OCF_2CHFCF_3$ |
| H | $C(CH_3)_2CH_2SCH_3$ | $CF_2CF_3$ | Ph-4-$OCF_2CHFOCF_3$ |
| H | $C(CH_3)_2CH_2SCH_3$ | $CF_2CF_3$ | Ph-4-$OCF_2CHFOCF_2CF_2CF_3$ |
| H | $C(CH_3)_2CH_2SCH_3$ | $CF_2CF_3$ | Ph-4-O(L-45g) |
| H | $C(CH_3)_2CH_2SCH_3$ | $CF_2CF_3$ | Ph-3,4-$Cl_2$ |
| H | $C(CH_3)_2CH_2SCH_3$ | $CF_2CF_3$ | Ph(-3-$OCF_2O$-4-) |
| H | $C(CH_3)_2CH_2SCH_3$ | $CF_2CF_3$ | Ph(-3-$OCF_2CF_2O$-4-) |
| H | $C(CH_3)_2CH_2SCH_3$ | $CF_2CF_3$ | (L-45c)$CF_3$ |
| H | $C(CH_3)_2CH_2SCH_3$ | $CF_2CF_3$ | L-45f |
| H | $C(CH_3)_2CH_2SCH_3$ | $CF_2CF_3$ | (L-46c)$CF_3$ |
| H | $C(CH_3)_2CH_2SCH_3$ | $CF_2CF_3$ | L-46d |

| | | | |
|---|---|---|---|
| H | $C(CH_3)_2CH_2SCH_3$ | $CF_2CF_2Cl$ | Ph-4-$OCF_3$ |
| H | $C(CH_3)_2CH_2SCH_3$ | $CFClCF_3$ | (L-45c) $CF_3$ |
| H | $C(CH_3)_2CH_2SCH_3$ | $CFClCF_2Cl$ | Ph-4-Br |
| H | $C(CH_3)_2CH_2SCH_3$ | $CF_2CF_2Br$ | Ph-4-$CF_3$ |
| H | $C(CH_3)_2CH_2SCH_3$ | $CFBrCF_3$ | Ph-4-$OCF_3$ |
| H | $C(CH_3)_2CH_2SCH_3$ | $CF_2CHFCF_3$ | (L-45c) $CF_3$ |
| H | $C(CH_3)_2CH_2SCH_3$ | $CF_2CF_2CF_3$ | Ph-4-Cl |
| H | $C(CH_3)_2CH_2SCH_3$ | $CF_2CF_2CF_3$ | Ph-4-Br |
| H | $C(CH_3)_2CH_2SCH_3$ | $CF_2CF_2CF_3$ | Ph-4-$CF_3$ |
| H | $C(CH_3)_2CH_2SCH_3$ | $CF_2CF_2CF_3$ | Ph-4-$OCF_3$ |
| H | $C(CH_3)_2CH_2SCH_3$ | $CF_2CF_2CF_3$ | Ph-4-$OCF_2Br$ |
| H | $C(CH_3)_2CH_2SCH_3$ | $CF_2CF_2CF_3$ | Ph-4-$OCF_2CHFCF_3$ |
| H | $C(CH_3)_2CH_2SCH_3$ | $CF_2CF_2CF_3$ | Ph-4-$OCF_2CHFOCF_3$ |
| H | $C(CH_3)_2CH_2SCH_3$ | $CF_2CF_2CF_3$ | Ph-4-$OCF_2CHFOCF_2CF_2CF_3$ |
| H | $C(CH_3)_2CH_2SCH_3$ | $CF_2CF_2CF_3$ | Ph-4-O(L-45g) |
| H | $C(CH_3)_2CH_2SCH_3$ | $CF_2CF_2CF_3$ | Ph-3,4-$Cl_2$ |
| H | $C(CH_3)_2CH_2SCH_3$ | $CF_2CF_2CF_3$ | Ph(-3-$OCF_2O$-4-) |
| H | $C(CH_3)_2CH_2SCH_3$ | $CF_2CF_2CF_3$ | Ph(-3-$OCF_2CF_2O$-4-) |
| H | $C(CH_3)_2CH_2SCH_3$ | $CF_2CF_2CF_3$ | (L-45c) $CF_3$ |
| H | $C(CH_3)_2CH_2SCH_3$ | $CF_2CF_2CF_3$ | L-45f |
| H | $C(CH_3)_2CH_2SCH_3$ | $CF_2CF_2CF_3$ | (L-46c) $CF_3$ |
| H | $C(CH_3)_2CH_2SCH_3$ | $CF_2CF_2CF_3$ | L-46d |
| H | $C(CH_3)_2CH_2SCH_3$ | $CF(CF_3)_2$ | Ph-4-Br |
| H | $C(CH_3)_2CH_2SCH_3$ | $CF_2CF_2CF_2CF_3$ | Ph-4-$CF_3$ |
| H | $C(CH_3)_2CH_2SCH_3$ | T-1 | Ph-4-$OCF_3$ |
| H | $C(CH_3)_2CH_2SCH_3$ | T-2 | (L-45c) $CF_3$ |
| H | $C(CH_3)_2CH_2SCH_3$ | $CH_2OCH_3$ | Ph-4-Br |
| H | $C(CH_3)_2CH_2SCH_3$ | $CH_2OEt$ | Ph-4-$CF_3$ |
| H | $C(CH_3)_2CH_2SCH_3$ | $CH_2OCH_2CF_3$ | Ph-4-$OCF_3$ |
| H | $C(CH_3)_2CH_2SCH_3$ | $CH_2OCH(CF_3)_2$ | (L-45c) $CF_3$ |
| H | $C(CH_3)_2CH_2SCH_3$ | $CF_2OCH_3$ | Ph-4-Cl |
| H | $C(CH_3)_2CH_2SCH_3$ | $CF_2OCH_3$ | Ph-4-Br |
| H | $C(CH_3)_2CH_2SCH_3$ | $CF_2OCH_3$ | Ph-4-$CF_3$ |
| H | $C(CH_3)_2CH_2SCH_3$ | $CF_2OCH_3$ | Ph-4-$OCF_3$ |
| H | $C(CH_3)_2CH_2SCH_3$ | $CF_2OCH_3$ | Ph-4-$OCF_2Br$ |
| H | $C(CH_3)_2CH_2SCH_3$ | $CF_2OCH_3$ | Ph-4-$OCF_2CHFCF_3$ |
| H | $C(CH_3)_2CH_2SCH_3$ | $CF_2OCH_3$ | Ph-4-$OCF_2CHFOCF_3$ |
| H | $C(CH_3)_2CH_2SCH_3$ | $CF_2OCH_3$ | Ph-4-$OCF_2CHFOCF_2CF_2CF_3$ |
| H | $C(CH_3)_2CH_2SCH_3$ | $CF_2OCH_3$ | Ph-4-O(L-45g) |
| H | $C(CH_3)_2CH_2SCH_3$ | $CF_2OCH_3$ | Ph-3,4-$Cl_2$ |
| H | $C(CH_3)_2CH_2SCH_3$ | $CF_2OCH_3$ | Ph(-3-$OCF_2O$-4-) |

| | | | |
|---|---|---|---|
| H | $C(CH_3)_2CH_2SCH_3$ | $CF_2OCH_3$ | Ph(-3-$OCF_2CF_2O$-4-) |
| H | $C(CH_3)_2CH_2SCH_3$ | $CF_2OCH_3$ | (L-45c)$CF_3$ |
| H | $C(CH_3)_2CH_2SCH_3$ | $CF_2OCH_3$ | L-45f |
| H | $C(CH_3)_2CH_2SCH_3$ | $CF_2OCH_3$ | (L-46c)$CF_3$ |
| H | $C(CH_3)_2CH_2SCH_3$ | $CF_2OCH_3$ | L-46d |
| H | $C(CH_3)_2CH_2SCH_3$ | $CH_2SCH_3$ | Ph-4-Br |
| H | $C(CH_3)_2CH_2SCH_3$ | $CH_2SO_2CH_3$ | Ph-4-$CF_3$ |
| H | $C(CH_3)_2CH_2SCH_3$ | $CH_2SCF_3$ | Ph-4-$OCF_3$ |
| H | $C(CH_3)_2CH_2SCH_3$ | $CF_2SCH_3$ | Ph-4-Cl |
| H | $C(CH_3)_2CH_2SCH_3$ | $CF_2SCH_3$ | Ph-4-Br |
| H | $C(CH_3)_2CH_2SCH_3$ | $CF_2SCH_3$ | Ph-4-$CF_3$ |
| H | $C(CH_3)_2CH_2SCH_3$ | $CF_2SCH_3$ | Ph-4-$OCF_3$ |
| H | $C(CH_3)_2CH_2SCH_3$ | $CF_2SCH_3$ | Ph-4-$OCF_2Br$ |
| H | $C(CH_3)_2CH_2SCH_3$ | $CF_2SCH_3$ | Ph-4-$OCF_2CHFCF_3$ |
| H | $C(CH_3)_2CH_2SCH_3$ | $CF_2SCH_3$ | Ph-4-$OCF_2CHFOCF_3$ |
| H | $C(CH_3)_2CH_2SCH_3$ | $CF_2SCH_3$ | Ph-4-$OCF_2CHFOCF_2CF_2CF_3$ |
| H | $C(CH_3)_2CH_2SCH_3$ | $CF_2SCH_3$ | Ph-4-O(L-45g) |
| H | $C(CH_3)_2CH_2SCH_3$ | $CF_2SCH_3$ | Ph-3,4-$Cl_2$ |
| H | $C(CH_3)_2CH_2SCH_3$ | $CF_2SCH_3$ | Ph(-3-$OCF_2O$-4-) |
| H | $C(CH_3)_2CH_2SCH_3$ | $CF_2SCH_3$ | Ph(-3-$OCF_2CF_2O$-4-) |
| H | $C(CH_3)_2CH_2SCH_3$ | $CF_2SCH_3$ | (L-45c)$CF_3$ |
| H | $C(CH_3)_2CH_2SCH_3$ | $CF_2SCH_3$ | L-45f |
| H | $C(CH_3)_2CH_2SCH_3$ | $CF_2SCH_3$ | (L-46c)$CF_3$ |
| H | $C(CH_3)_2CH_2SCH_3$ | $CF_2SCH_3$ | L-46d |
| H | $C(CH_3)_2CH_2SCH_3$ | $CF_2SEt$ | (L-45c)$CF_3$ |
| H | $C(CH_3)_2CH_2SCH_3$ | $CF_2SPr$-n | Ph-4-Br |
| H | $C(CH_3)_2CH_2SCH_3$ | $CF_2SPr$-i | Ph-4-$CF_3$ |
| H | $C(CH_3)_2CH_2SCH_3$ | $CF_2SCH_2Ph$ | Ph-4-$OCF_3$ |
| H | $C(CH_3)_2CH_2SCH_3$ | $CF_2SPh$ | (L-45c)$CF_3$ |
| H | $C(CH_3)_2CH_2SCH_3$ | $CF_2C(O)OEt$ | Ph-4-Br |
| H | $C(CH_3)_2CH_2SCH_3$ | $CF_2SO_2N(CH_3)_2$ | Ph-4-$CF_3$ |
| H | $C(CH_3)_2CH_2SCH_3$ | C(O)OEt | Ph-4-$OCF_3$ |
| H | $C(CH_3)_2CH_2SCH_3$ | C(O)SEt | (L-45c)$CF_3$ |
| H | $C(CH_3)_2CH_2S(O)CH_3$ | $CH_3$ | Ph-4-Br |
| H | $C(CH_3)_2CH_2S(O)CH_3$ | $CH_3$ | Ph-4-$CF_3$ |
| H | $C(CH_3)_2CH_2S(O)CH_3$ | $CH_3$ | Ph-4-$OCF_3$ |
| H | $C(CH_3)_2CH_2S(O)CH_3$ | $CH_3$ | (L-45c)$CF_3$ |
| H | $C(CH_3)_2CH_2S(O)CH_3$ | $CF_3$ | $CH_2O$(Ph-4-Cl) |
| H | $C(CH_3)_2CH_2S(O)CH_3$ | $CF_3$ | $CH_2O$(Ph-4-Br) |
| H | $C(CH_3)_2CH_2S(O)CH_3$ | $CF_3$ | $CH_2O$(Ph-4-$CF_3$) |
| H | $C(CH_3)_2CH_2S(O)CH_3$ | $CF_3$ | $CH_2O$(Ph-4-$OCF_3$) |

| | | | |
|---|---|---|---|
| H | $C(CH_3)_2CH_2S(O)CH_3$ | $CF_3$ | $CF_2O(Ph\text{-}4\text{-}Cl)$ |
| H | $C(CH_3)_2CH_2S(O)CH_3$ | $CF_3$ | $CF_2O(Ph\text{-}4\text{-}Br)$ |
| H | $C(CH_3)_2CH_2S(O)CH_3$ | $CF_3$ | $CF_2O(Ph\text{-}4\text{-}CF_3)$ |
| H | $C(CH_3)_2CH_2S(O)CH_3$ | $CF_3$ | $CF_2O(Ph\text{-}4\text{-}OCF_3)$ |
| H | $C(CH_3)_2CH_2S(O)CH_3$ | $CF_3$ | $CH{=}CH(Ph\text{-}4\text{-}F)$ |
| H | $C(CH_3)_2CH_2S(O)CH_3$ | $CF_3$ | $CH{=}CH(Ph\text{-}4\text{-}Cl)$ |
| H | $C(CH_3)_2CH_2S(O)CH_3$ | $CF_3$ | $CH{=}CH(Ph\text{-}4\text{-}Br)$ |
| H | $C(CH_3)_2CH_2S(O)CH_3$ | $CF_3$ | $CH{=}CH(Ph\text{-}4\text{-}CF_3)$ |
| H | $C(CH_3)_2CH_2S(O)CH_3$ | $CF_3$ | $CH{=}CH(Ph\text{-}4\text{-}OCF_3)$ |
| H | $C(CH_3)_2CH_2S(O)CH_3$ | $CF_3$ | $CH{=}CH(Ph\text{-}4\text{-}SCH_3)$ |
| H | $C(CH_3)_2CH_2S(O)CH_3$ | $CF_3$ | $CH{=}CH(Ph\text{-}4\text{-}SO_2CH_3)$ |
| H | $C(CH_3)_2CH_2S(O)CH_3$ | $CF_3$ | $CH{=}CH(Ph\text{-}3,4\text{-}Cl_2)$ |
| H | $C(CH_3)_2CH_2S(O)CH_3$ | $CF_3$ | Ph-4-F |
| H | $C(CH_3)_2CH_2S(O)CH_3$ | $CF_3$ | Ph-2-Cl |
| H | $C(CH_3)_2CH_2S(O)CH_3$ | $CF_3$ | Ph-3-Cl |
| H | $C(CH_3)_2CH_2S(O)CH_3$ | $CF_3$ | Ph-4-Cl |
| H | $C(CH_3)_2CH_2S(O)CH_3$ | $CF_3$ | Ph-4-Br |
| H | $C(CH_3)_2CH_2S(O)CH_3$ | $CF_3$ | Ph-4-I |
| H | $C(CH_3)_2CH_2S(O)CH_3$ | $CF_3$ | $Ph\text{-}4\text{-}CF_3$ |
| H | $C(CH_3)_2CH_2S(O)CH_3$ | $CF_3$ | $Ph\text{-}4\text{-}OCHF_2$ |
| H | $C(CH_3)_2CH_2S(O)CH_3$ | $CF_3$ | $Ph\text{-}4\text{-}OCF_3$ |
| H | $C(CH_3)_2CH_2S(O)CH_3$ (-) | $CF_3$ | $Ph\text{-}4\text{-}OCF_3$ |
| H | $C(CH_3)_2CH_2S(O)CH_3$ (+) | $CF_3$ | $Ph\text{-}4\text{-}OCF_3$ |
| H | $C(CH_3)_2CH_2S(O)CH_3$ | $CF_3$ | $Ph\text{-}4\text{-}OCF_2Br$ |
| H | $C(CH_3)_2CH_2S(O)CH_3$ | $CF_3$ | $Ph\text{-}4\text{-}OCH_2CF_3$ |
| H | $C(CH_3)_2CH_2S(O)CH_3$ | $CF_3$ | $Ph\text{-}4\text{-}OCF_2CHF_2$ |
| H | $C(CH_3)_2CH_2S(O)CH_3$ | $CF_3$ | $Ph\text{-}4\text{-}OCF_2CHFCl$ |
| H | $C(CH_3)_2CH_2S(O)CH_3$ | $CF_3$ | $Ph\text{-}4\text{-}OCF_2CHFCF_3$ |
| H | $C(CH_3)_2CH_2S(O)CH_3$ | $CF_3$ | $Ph\text{-}4\text{-}OCF_2CHFOCF_3$ |
| H | $C(CH_3)_2CH_2S(O)CH_3$ | $CF_3$ | $Ph\text{-}4\text{-}OCF_2CHFOCF_2CF_2CF_3$ |
| H | $C(CH_3)_2CH_2S(O)CH_3$ | $CF_3$ | $Ph\text{-}4\text{-}OSO_2CH_3$ |
| H | $C(CH_3)_2CH_2S(O)CH_3$ | $CF_3$ | $Ph\text{-}4\text{-}OSO_2CF_3$ |
| H | $C(CH_3)_2CH_2S(O)CH_3$ | $CF_3$ | Ph-4-O(L-45e) |
| H | $C(CH_3)_2CH_2S(O)CH_3$ | $CF_3$ | $Ph\text{-}2,4\text{-}F_2$ |
| H | $C(CH_3)_2CH_2S(O)CH_3$ | $CF_3$ | $Ph\text{-}3,4\text{-}F_2$ |
| H | $C(CH_3)_2CH_2S(O)CH_3$ | $CF_3$ | Ph-3-Cl-4-F |
| H | $C(CH_3)_2CH_2S(O)CH_3$ | $CF_3$ | Ph-2-F-4-Cl |
| H | $C(CH_3)_2CH_2S(O)CH_3$ | $CF_3$ | Ph-3-F-4-Cl |
| H | $C(CH_3)_2CH_2S(O)CH_3$ | $CF_3$ | $Ph\text{-}2,4\text{-}Cl_2$ |
| H | $C(CH_3)_2CH_2S(O)CH_3$ | $CF_3$ | $Ph\text{-}3,4\text{-}Cl_2$ |
| H | $C(CH_3)_2CH_2S(O)CH_3$ | $CF_3$ | $Ph\text{-}3,5\text{-}Cl_2$ |

| | | | |
|---|---|---|---|
| H | $C(CH_3)_2CH_2S(O)CH_3$ | $CF_3$ | Ph-3-Br-4-F |
| H | $C(CH_3)_2CH_2S(O)CH_3$ | $CF_3$ | Ph-2-F-4-Br |
| H | $C(CH_3)_2CH_2S(O)CH_3$ | $CF_3$ | Ph-3, 4-$Br_2$ |
| H | $C(CH_3)_2CH_2S(O)CH_3$ | $CF_3$ | Ph-3-$CH_3$-4-F |
| H | $C(CH_3)_2CH_2S(O)CH_3$ | $CF_3$ | Ph-3-$CF_3$-4-F |
| H | $C(CH_3)_2CH_2S(O)CH_3$ | $CF_3$ | Ph-3-$CF_3$-4-Cl |
| H | $C(CH_3)_2CH_2S(O)CH_3$ | $CF_3$ | Ph-2-F-4-$CF_3$ |
| H | $C(CH_3)_2CH_2S(O)CH_3$ | $CF_3$ | Ph-3-F-4-$CF_3$ |
| H | $C(CH_3)_2CH_2S(O)CH_3$ | $CF_3$ | Ph-2-Cl-4-$CF_3$ |
| H | $C(CH_3)_2CH_2S(O)CH_3$ | $CF_3$ | Ph-3-F-4-$OCHF_2$ |
| H | $C(CH_3)_2CH_2S(O)CH_3$ | $CF_3$ | Ph-3-Cl-4-$OCHF_2$ |
| H | $C(CH_3)_2CH_2S(O)CH_3$ | $CF_3$ | Ph-3-Br-4-$OCHF_2$ |
| H | $C(CH_3)_2CH_2S(O)CH_3$ | $CF_3$ | Ph-3-F-4-$OCF_3$ |
| H | $C(CH_3)_2CH_2S(O)CH_3$ | $CF_3$ | Ph-3-Cl-4-$OCF_3$ |
| H | $C(CH_3)_2CH_2S(O)CH_3$ | $CF_3$ | Ph-3-Br-4-$OCF_3$ |
| H | $C(CH_3)_2CH_2S(O)CH_3$ | $CF_3$ | Ph-3-F-4-$OCF_2Br$ |
| H | $C(CH_3)_2CH_2S(O)CH_3$ | $CF_3$ | Ph-3-Cl-4-$OCF_2Br$ |
| H | $C(CH_3)_2CH_2S(O)CH_3$ | $CF_3$ | Ph-3-Br-4-$OCF_2Br$ |
| H | $C(CH_3)_2CH_2S(O)CH_3$ | $CF_3$ | Ph-3-F-4-$OCF_2CHF_2$ |
| H | $C(CH_3)_2CH_2S(O)CH_3$ | $CF_3$ | Ph-3-Cl-4-$OCF_2CHF_2$ |
| H | $C(CH_3)_2CH_2S(O)CH_3$ | $CF_3$ | Ph-3-Br-4-$OCF_2CHF_2$ |
| H | $C(CH_3)_2CH_2S(O)CH_3$ | $CF_3$ | Ph-3-F-4-$OCF_2CHFCl$ |
| H | $C(CH_3)_2CH_2S(O)CH_3$ | $CF_3$ | Ph-3-Cl-4-$OCF_2CHFCl$ |
| H | $C(CH_3)_2CH_2S(O)CH_3$ | $CF_3$ | Ph-3-Br-4-$OCF_2CHFCl$ |
| H | $C(CH_3)_2CH_2S(O)CH_3$ | $CF_3$ | Ph-3-F-4-$OCF_2CHFCF_3$ |
| H | $C(CH_3)_2CH_2S(O)CH_3$ | $CF_3$ | Ph-3-Cl-4-$OCF_2CHFCF_3$ |
| H | $C(CH_3)_2CH_2S(O)CH_3$ | $CF_3$ | Ph-3-Br-4-$OCF_2CHFCF_3$ |
| H | $C(CH_3)_2CH_2S(O)CH_3$ | $CF_3$ | Ph-3-F-4-$OCF_2CHFOCF_3$ |
| H | $C(CH_3)_2CH_2S(O)CH_3$ | $CF_3$ | Ph-3-Cl-4-$OCF_2CHFOCF_3$ |
| H | $C(CH_3)_2CH_2S(O)CH_3$ | $CF_3$ | Ph-3-Br-4-$OCF_2CHFOCF_3$ |
| H | $C(CH_3)_2CH_2S(O)CH_3$ | $CF_3$ | Ph-3-$CH_3$-4-$OCF_2CHFOCF_3$ |
| H | $C(CH_3)_2CH_2S(O)CH_3$ | $CF_3$ | Ph-3-F-4-$OCF_2CHFOCF_2CF_2CF_3$ |
| H | $C(CH_3)_2CH_2S(O)CH_3$ | $CF_3$ | Ph-3-Cl-4-$OCF_2CHFOCF_2CF_2CF_3$ |
| H | $C(CH_3)_2CH_2S(O)CH_3$ | $CF_3$ | Ph-3-Br-4-$OCF_2CHFOCF_2CF_2CF_3$ |
| H | $C(CH_3)_2CH_2S(O)CH_3$ | $CF_3$ | Ph(-3-$OCF_2O$-4-) |
| H | $C(CH_3)_2CH_2S(O)CH_3$ | $CF_3$ | Ph(-3-$OCF_2CF_2O$-4-) |
| H | $C(CH_3)_2CH_2S(O)CH_3$ | $CF_3$ | Ph-3, 4-$F_2$-5-$CF_3$ |
| H | $C(CH_3)_2CH_2S(O)CH_3$ | $CF_3$ | 2-Naph |
| H | $C(CH_3)_2CH_2S(O)CH_3$ | $CF_3$ | (L-1b)Br |
| H | $C(CH_3)_2CH_2S(O)CH_3$ | $CF_3$ | (L-1c)Cl |
| H | $C(CH_3)_2CH_2S(O)CH_3$ | $CF_3$ | (L-1c)Br |

| | | | |
|---|---|---|---|
| H | $C(CH_3)_2CH_2S(O)CH_3$ | $CF_3$ | (L-1c) $CF_3$ |
| H | $C(CH_3)_2CH_2S(O)CH_3$ | $CF_3$ | (L-2b) Br |
| H | $C(CH_3)_2CH_2S(O)CH_3$ | $CF_3$ | (L-3b) Cl |
| H | $C(CH_3)_2CH_2S(O)CH_3$ | $CF_3$ | (L-3b) Br |
| H | $C(CH_3)_2CH_2S(O)CH_3$ | $CF_3$ | (L-3c) Cl |
| H | $C(CH_3)_2CH_2S(O)CH_3$ | $CF_3$ | (L-3c) Br |
| H | $C(CH_3)_2CH_2S(O)CH_3$ | $CF_3$ | (L-3c) $CF_3$ |
| H | $C(CH_3)_2CH_2S(O)CH_3$ | $CF_3$ | L-3d |
| H | $C(CH_3)_2CH_2S(O)CH_3$ | $CF_3$ | (L-4b) Cl |
| H | $C(CH_3)_2CH_2S(O)CH_3$ | $CF_3$ | (L-4b) Br |
| H | $C(CH_3)_2CH_2S(O)CH_3$ | $CF_3$ | (L-10b) Cl |
| H | $C(CH_3)_2CH_2S(O)CH_3$ | $CF_3$ | (L-10b) Br |
| H | $C(CH_3)_2CH_2S(O)CH_3$ | $CF_3$ | (L-15b) $CF_3$ |
| H | $C(CH_3)_2CH_2S(O)CH_3$ | $CF_3$ | (L-16a) $CHF_2$ |
| H | $C(CH_3)_2CH_2S(O)CH_3$ | $CF_3$ | (L-16a) $CF_2Br$ |
| H | $C(CH_3)_2CH_2S(O)CH_3$ | $CF_3$ | (L-17a) Cl |
| H | $C(CH_3)_2CH_2S(O)CH_3$ | $CF_3$ | (L-21b) Cl |
| H | $C(CH_3)_2CH_2S(O)CH_3$ | $CF_3$ | (L-21b) Br |
| H | $C(CH_3)_2CH_2S(O)CH_3$ | $CF_3$ | (L-21b) $CF_3$ |
| H | $C(CH_3)_2CH_2S(O)CH_3$ | $CF_3$ | (L-22b) Cl |
| H | $C(CH_3)_2CH_2S(O)CH_3$ | $CF_3$ | (L-22b) Br |
| H | $C(CH_3)_2CH_2S(O)CH_3$ | $CF_3$ | (L-23b) Cl |
| H | $C(CH_3)_2CH_2S(O)CH_3$ | $CF_3$ | (L-23b) Br |
| H | $C(CH_3)_2CH_2S(O)CH_3$ | $CF_3$ | (L-23c) Cl |
| H | $C(CH_3)_2CH_2S(O)CH_3$ | $CF_3$ | (L-23c) Br |
| H | $C(CH_3)_2CH_2S(O)CH_3$ | $CF_3$ | (L-31a) Cl |
| H | $C(CH_3)_2CH_2S(O)CH_3$ | $CF_3$ | (L-31a) Br |
| H | $C(CH_3)_2CH_2S(O)CH_3$ | $CF_3$ | (L-45c) F |
| H | $C(CH_3)_2CH_2S(O)CH_3$ | $CF_3$ | (L-45c) Cl |
| H | $C(CH_3)_2CH_2S(O)CH_3$ | $CF_3$ | (L-45c) Br |
| H | $C(CH_3)_2CH_2S(O)CH_3$ | $CF_3$ | (L-45c) $CF_3$ |
| H | $C(CH_3)_2CH_2S(O)CH_3$ | $CF_3$ | L-45e |
| H | $C(CH_3)_2CH_2S(O)CH_3$ | $CF_3$ | L-45f |
| H | $C(CH_3)_2CH_2S(O)CH_3$ | $CF_3$ | (L-46c) F |
| H | $C(CH_3)_2CH_2S(O)CH_3$ | $CF_3$ | (L-46c) Cl |
| H | $C(CH_3)_2CH_2S(O)CH_3$ | $CF_3$ | (L-46c) Br |
| H | $C(CH_3)_2CH_2S(O)CH_3$ | $CF_3$ | (L-46c) $CF_3$ |
| H | $C(CH_3)_2CH_2S(O)CH_3$ | $CF_3$ | L-46d |
| H | $C(CH_3)_2CH_2S(O)CH_3$ | $CF_3$ | L-47a |
| H | $C(CH_3)_2CH_2S(O)CH_3$ | $CF_3$ | L-47d |
| H | $C(CH_3)_2CH_2S(O)CH_3$ | $CF_3$ | (L-48b) Br |

| | | | |
|---|---|---|---|
| H | $C(CH_3)_2CH_2S(O)CH_3$ | $CF_3$ | (L-50b) Cl |
| H | $C(CH_3)_2CH_2S(O)CH_3$ | $CF_3$ | (L-50b) Br |
| H | $C(CH_3)_2CH_2S(O)CH_3$ | $CF_3$ | (L-51b) Cl |
| H | $C(CH_3)_2CH_2SO_2CH_3$ | $CH_3$ | $CF_2O$ (Ph-4-Cl) |
| H | $C(CH_3)_2CH_2SO_2CH_3$ | $CH_3$ | CH=CH (Ph-4-Cl) |
| H | $C(CH_3)_2CH_2SO_2CH_3$ | $CH_3$ | CH=CH (Ph-4-$OCF_3$) |
| H | $C(CH_3)_2CH_2SO_2CH_3$ | $CH_3$ | CH=CH (Ph-3, 4-$Cl_2$) |
| H | $C(CH_3)_2CH_2SO_2CH_3$ | $CH_3$ | Ph-4-F |
| H | $C(CH_3)_2CH_2SO_2CH_3$ | $CH_3$ | Ph-4-Cl |
| H | $C(CH_3)_2CH_2SO_2CH_3$ | $CH_3$ | Ph-4-Br |
| H | $C(CH_3)_2CH_2SO_2CH_3$ | $CH_3$ | Ph-4-I |
| H | $C(CH_3)_2CH_2SO_2CH_3$ | $CH_3$ | Ph-4-$CF_3$ |
| H | $C(CH_3)_2CH_2SO_2CH_3$ | $CH_3$ | Ph-4-$OCHF_2$ |
| H | $C(CH_3)_2CH_2SO_2CH_3$ | $CH_3$ | Ph-4-$OCF_3$ |
| H | $C(CH_3)_2CH_2SO_2CH_3$ | $CH_3$ | Ph-4-$OCF_2Br$ |
| H | $C(CH_3)_2CH_2SO_2CH_3$ | $CH_3$ | Ph-4-$OCF_2CHF_2$ |
| H | $C(CH_3)_2CH_2SO_2CH_3$ | $CH_3$ | Ph-4-$OCF_2CHFCl$ |
| H | $C(CH_3)_2CH_2SO_2CH_3$ | $CH_3$ | Ph-4-$OCF_2CHFCF_3$ |
| H | $C(CH_3)_2CH_2SO_2CH_3$ | $CH_3$ | Ph-4-$OCF_2CHFOCF_3$ |
| H | $C(CH_3)_2CH_2SO_2CH_3$ | $CH_3$ | Ph-4-$OCF_2CHFOCF_2CF_2CF_3$ |
| H | $C(CH_3)_2CH_2SO_2CH_3$ | $CH_3$ | Ph-4-O (L-45g) |
| H | $C(CH_3)_2CH_2SO_2CH_3$ | $CH_3$ | Ph-3, 4-$Cl_2$ |
| H | $C(CH_3)_2CH_2SO_2CH_3$ | $CH_3$ | Ph (-3-$OCF_2O$-4-) |
| H | $C(CH_3)_2CH_2SO_2CH_3$ | $CH_3$ | Ph (-3-$OCF_2CF_2O$-4-) |
| H | $C(CH_3)_2CH_2SO_2CH_3$ | $CH_3$ | (L-45c) Cl |
| H | $C(CH_3)_2CH_2SO_2CH_3$ | $CH_3$ | (L-45c) Br |
| H | $C(CH_3)_2CH_2SO_2CH_3$ | $CH_3$ | (L-45c) $CF_3$ |
| H | $C(CH_3)_2CH_2SO_2CH_3$ | $CH_3$ | L-45e |
| H | $C(CH_3)_2CH_2SO_2CH_3$ | $CH_3$ | L-45f |
| H | $C(CH_3)_2CH_2SO_2CH_3$ | $CH_3$ | (L-46c) Cl |
| H | $C(CH_3)_2CH_2SO_2CH_3$ | $CH_3$ | (L-46c) Br |
| H | $C(CH_3)_2CH_2SO_2CH_3$ | $CH_3$ | (L-46c) $CF_3$ |
| H | $C(CH_3)_2CH_2SO_2CH_3$ | $CH_3$ | L-46d |
| H | $C(CH_3)_2CH_2SO_2CH_3$ | Et | Ph-4-Cl |
| H | $C(CH_3)_2CH_2SO_2CH_3$ | Et | Ph-4-Br |
| H | $C(CH_3)_2CH_2SO_2CH_3$ | Et | Ph-4-$CF_3$ |
| H | $C(CH_3)_2CH_2SO_2CH_3$ | Et | Ph-4-$OCF_3$ |
| H | $C(CH_3)_2CH_2SO_2CH_3$ | Et | Ph-4-$OCF_2Br$ |
| H | $C(CH_3)_2CH_2SO_2CH_3$ | Et | Ph-4-$OCF_2CHFCF_3$ |
| H | $C(CH_3)_2CH_2SO_2CH_3$ | Et | Ph-4-$OCF_2CHFOCF_3$ |
| H | $C(CH_3)_2CH_2SO_2CH_3$ | Et | Ph-4-$OCF_2CHFOCF_2CF_2CF_3$ |

| | | | |
|---|---|---|---|
| H | $C(CH_3)_2CH_2SO_2CH_3$ | Et | Ph-4-O(L-45g) |
| H | $C(CH_3)_2CH_2SO_2CH_3$ | Et | Ph-3,4-$Cl_2$ |
| H | $C(CH_3)_2CH_2SO_2CH_3$ | Et | Ph(-3-$OCF_2O$-4-) |
| H | $C(CH_3)_2CH_2SO_2CH_3$ | Et | Ph(-3-$OCF_2CF_2O$-4-) |
| H | $C(CH_3)_2CH_2SO_2CH_3$ | Et | (L-45c)$CF_3$ |
| H | $C(CH_3)_2CH_2SO_2CH_3$ | n-Pr | Ph-4-Br |
| H | $C(CH_3)_2CH_2SO_2CH_3$ | n-Pr | Ph-4-$CF_3$ |
| H | $C(CH_3)_2CH_2SO_2CH_3$ | n-Pr | Ph-4-$OCF_3$ |
| H | $C(CH_3)_2CH_2SO_2CH_3$ | n-Pr | (L-45c)$CF_3$ |
| H | $C(CH_3)_2CH_2SO_2CH_3$ | i-Pr | Ph-4-Br |
| H | $C(CH_3)_2CH_2SO_2CH_3$ | i-Pr | Ph-4-$CF_3$ |
| H | $C(CH_3)_2CH_2SO_2CH_3$ | i-Pr | Ph-4-$OCF_3$ |
| H | $C(CH_3)_2CH_2SO_2CH_3$ | i-Pr | (L-45c)$CF_3$ |
| H | $C(CH_3)_2CH_2SO_2CH_3$ | c-Pr | Ph-4-Br |
| H | $C(CH_3)_2CH_2SO_2CH_3$ | c-Pr | Ph-4-$CF_3$ |
| H | $C(CH_3)_2CH_2SO_2CH_3$ | c-Pr | Ph-4-$OCF_3$ |
| H | $C(CH_3)_2CH_2SO_2CH_3$ | c-Pr | (L-45c)$CF_3$ |
| H | $C(CH_3)_2CH_2SO_2CH_3$ | n-Bu | Ph-4-$OCF_3$ |
| H | $C(CH_3)_2CH_2SO_2CH_3$ | $CH_2F$ | (L-45c)$CF_3$ |
| H | $C(CH_3)_2CH_2SO_2CH_3$ | $CH_2Cl$ | Ph-4-Br |
| H | $C(CH_3)_2CH_2SO_2CH_3$ | $CH_2Br$ | Ph-4-$CF_3$ |
| H | $C(CH_3)_2CH_2SO_2CH_3$ | $CHF_2$ | Ph-4-Cl |
| H | $C(CH_3)_2CH_2SO_2CH_3$ | $CHF_2$ | Ph-4-Br |
| H | $C(CH_3)_2CH_2SO_2CH_3$ | $CHF_2$ | Ph-4-$CF_3$ |
| H | $C(CH_3)_2CH_2SO_2CH_3$ | $CHF_2$ | Ph-4-$OCF_3$ |
| H | $C(CH_3)_2CH_2SO_2CH_3$ | $CHF_2$ | Ph-4-$OCF_2Br$ |
| H | $C(CH_3)_2CH_2SO_2CH_3$ | $CHF_2$ | Ph-4-$OCF_2CHFCF_3$ |
| H | $C(CH_3)_2CH_2SO_2CH_3$ | $CHF_2$ | Ph-4-$OCF_2CHFOCF_3$ |
| H | $C(CH_3)_2CH_2SO_2CH_3$ | $CHF_2$ | Ph-4-$OCF_2CHFOCF_2CF_2CF_3$ |
| H | $C(CH_3)_2CH_2SO_2CH_3$ | $CHF_2$ | Ph-4-O(L-45g) |
| H | $C(CH_3)_2CH_2SO_2CH_3$ | $CHF_2$ | Ph-3,4-$Cl_2$ |
| H | $C(CH_3)_2CH_2SO_2CH_3$ | $CHF_2$ | Ph(-3-$OCF_2O$-4-) |
| H | $C(CH_3)_2CH_2SO_2CH_3$ | $CHF_2$ | Ph(-3-$OCF_2CF_2O$-4-) |
| H | $C(CH_3)_2CH_2SO_2CH_3$ | $CHF_2$ | (L-45c)$CF_3$ |
| H | $C(CH_3)_2CH_2SO_2CH_3$ | $CHF_2$ | L-45f |
| H | $C(CH_3)_2CH_2SO_2CH_3$ | $CHF_2$ | (L-46c)$CF_3$ |
| H | $C(CH_3)_2CH_2SO_2CH_3$ | $CHF_2$ | L-46d |
| H | $C(CH_3)_2CH_2SO_2CH_3$ | CHFCl | Ph-4-$OCF_3$ |
| H | $C(CH_3)_2CH_2SO_2CH_3$ | CHFBr | (L-45c)$CF_3$ |
| H | $C(CH_3)_2CH_2SO_2CH_3$ | $CF_3$ | c-Pr |
| H | $C(CH_3)_2CH_2SO_2CH_3$ | $CF_3$ | c-Bu |

| | | | |
|---|---|---|---|
| H | $C(CH_3)_2CH_2SO_2CH_3$ | $CF_3$ | c-Pen |
| H | $C(CH_3)_2CH_2SO_2CH_3$ | $CF_3$ | c-Hex |
| H | $C(CH_3)_2CH_2SO_2CH_3$ | $CF_3$ | T-1 |
| H | $C(CH_3)_2CH_2SO_2CH_3$ | $CF_3$ | T-2 |
| H | $C(CH_3)_2CH_2SO_2CH_3$ | $CF_3$ | T-3 |
| H | $C(CH_3)_2CH_2SO_2CH_3$ | $CF_3$ | T-4 |
| H | $C(CH_3)_2CH_2SO_2CH_3$ | $CF_3$ | T-5 |
| H | $C(CH_3)_2CH_2SO_2CH_3$ | $CF_3$ | $CH_2OCH_3$ |
| H | $C(CH_3)_2CH_2SO_2CH_3$ | $CF_3$ | $CH_2OEt$ |
| H | $C(CH_3)_2CH_2SO_2CH_3$ | $CF_3$ | $CH_2OPr$-n |
| H | $C(CH_3)_2CH_2SO_2CH_3$ | $CF_3$ | $CH_2OPr$-i |
| H | $C(CH_3)_2CH_2SO_2CH_3$ | $CF_3$ | $CH_2OBu$-n |
| H | $C(CH_3)_2CH_2SO_2CH_3$ | $CF_3$ | $CH_2OC(O)$ (Ph-2-Cl) |
| H | $C(CH_3)_2CH_2SO_2CH_3$ | $CF_3$ | $CH_2OC(O)$ (Ph-3-Cl) |
| H | $C(CH_3)_2CH_2SO_2CH_3$ | $CF_3$ | $CH_2OC(O)$ (Ph-4-Cl) |
| H | $C(CH_3)_2CH_2SO_2CH_3$ | $CF_3$ | $CH_2OPh$ |
| H | $C(CH_3)_2CH_2SO_2CH_3$ | $CF_3$ | $CH_2O$ (Ph-2-F) |
| H | $C(CH_3)_2CH_2SO_2CH_3$ | $CF_3$ | $CH_2O$ (Ph-3-F) |
| H | $C(CH_3)_2CH_2SO_2CH_3$ | $CF_3$ | $CH_2O$ (Ph-4-F) |
| H | $C(CH_3)_2CH_2SO_2CH_3$ | $CF_3$ | $CH_2O$ (Ph-2-Cl) |
| H | $C(CH_3)_2CH_2SO_2CH_3$ | $CF_3$ | $CH_2O$ (Ph-3-Cl) |
| H | $C(CH_3)_2CH_2SO_2CH_3$ | $CF_3$ | $CH_2O$ (Ph-4-Cl) |
| H | $C(CH_3)_2CH_2SO_2CH_3$ | $CF_3$ | $CH_2O$ (Ph-2-Br) |
| H | $C(CH_3)_2CH_2SO_2CH_3$ | $CF_3$ | $CH_2O$ (Ph-3-Br) |
| H | $C(CH_3)_2CH_2SO_2CH_3$ | $CF_3$ | $CH_2O$ (Ph-4-Br) |
| H | $C(CH_3)_2CH_2SO_2CH_3$ | $CF_3$ | $CH_2O$ (Ph-2-$CF_3$) |
| H | $C(CH_3)_2CH_2SO_2CH_3$ | $CF_3$ | $CH_2O$ (Ph-3-$CF_3$) |
| H | $C(CH_3)_2CH_2SO_2CH_3$ | $CF_3$ | $CH_2O$ (Ph-4-$CF_3$) |
| H | $C(CH_3)_2CH_2SO_2CH_3$ | $CF_3$ | $CH_2O$ (Ph-2-$OCF_3$) |
| H | $C(CH_3)_2CH_2SO_2CH_3$ | $CF_3$ | $CH_2O$ (Ph-3-$OCF_3$) |
| H | $C(CH_3)_2CH_2SO_2CH_3$ | $CF_3$ | $CH_2O$ (Ph-4-$OCF_3$) |
| H | $C(CH_3)_2CH_2SO_2CH_3$ | $CF_3$ | $CF_2OPh$ |
| H | $C(CH_3)_2CH_2SO_2CH_3$ | $CF_3$ | $CF_2O$ (Ph-2-F) |
| H | $C(CH_3)_2CH_2SO_2CH_3$ | $CF_3$ | $CF_2O$ (Ph-3-F) |
| H | $C(CH_3)_2CH_2SO_2CH_3$ | $CF_3$ | $CF_2O$ (Ph-4-F) |
| H | $C(CH_3)_2CH_2SO_2CH_3$ | $CF_3$ | $CF_2O$ (Ph-2-Cl) |
| H | $C(CH_3)_2CH_2SO_2CH_3$ | $CF_3$ | $CF_2O$ (Ph-3-Cl) |
| H | $C(CH_3)_2CH_2SO_2CH_3$ | $CF_3$ | $CF_2O$ (Ph-4-Cl) |
| H | $C(CH_3)_2CH_2SO_2CH_3$ | $CF_3$ | $CF_2O$ (Ph-2-Br) |
| H | $C(CH_3)_2CH_2SO_2CH_3$ | $CF_3$ | $CF_2O$ (Ph-3-Br) |
| H | $C(CH_3)_2CH_2SO_2CH_3$ | $CF_3$ | $CF_2O$ (Ph-4-Br) |

| | | | |
|---|---|---|---|
| H | $C(CH_3)_2CH_2SO_2CH_3$ | $CF_3$ | $CF_2O(Ph-2-CF_3)$ |
| H | $C(CH_3)_2CH_2SO_2CH_3$ | $CF_3$ | $CF_2O(Ph-3-CF_3)$ |
| H | $C(CH_3)_2CH_2SO_2CH_3$ | $CF_3$ | $CF_2O(Ph-4-CF_3)$ |
| H | $C(CH_3)_2CH_2SO_2CH_3$ | $CF_3$ | $CF_2O(Ph-2-OCF_3)$ |
| H | $C(CH_3)_2CH_2SO_2CH_3$ | $CF_3$ | $CF_2O(Ph-3-OCF_3)$ |
| H | $C(CH_3)_2CH_2SO_2CH_3$ | $CF_3$ | $CF_2O(Ph-4-OCF_3)$ |
| H | $C(CH_3)_2CH_2SO_2CH_3$ | $CF_3$ | $CH_2S(Ph-2-Cl)$ |
| H | $C(CH_3)_2CH_2SO_2CH_3$ | $CF_3$ | $CH_2S(Ph-3-Cl)$ |
| H | $C(CH_3)_2CH_2SO_2CH_3$ | $CF_3$ | $CH_2S(Ph-4-Cl)$ |
| H | $C(CH_3)_2CH_2SO_2CH_3$ | $CF_3$ | $CH_2SO_2(Ph-2-Cl)$ |
| H | $C(CH_3)_2CH_2SO_2CH_3$ | $CF_3$ | $CH_2SO_2(Ph-3-Cl)$ |
| H | $C(CH_3)_2CH_2SO_2CH_3$ | $CF_3$ | $CH_2SO_2(Ph-4-Cl)$ |
| H | $C(CH_3)_2CH_2SO_2CH_3$ | $CF_3$ | $CH_2N(CH_3)_2$ |
| H | $C(CH_3)_2CH_2SO_2CH_3$ | $CF_3$ | $CH_2NH(Ph-2-Cl)$ |
| H | $C(CH_3)_2CH_2SO_2CH_3$ | $CF_3$ | $CH_2NH(Ph-3-Cl)$ |
| H | $C(CH_3)_2CH_2SO_2CH_3$ | $CF_3$ | $CH_2NH(Ph-4-Cl)$ |
| H | $C(CH_3)_2CH_2SO_2CH_3$ | $CF_3$ | $CF_2C(O)OEt$ |
| H | $C(CH_3)_2CH_2SO_2CH_3$ | $CF_3$ | $CH_2$(L-5a) |
| H | $C(CH_3)_2CH_2SO_2CH_3$ | $CF_3$ | $CH_2$(L-14a) |
| H | $C(CH_3)_2CH_2SO_2CH_3$ | $CF_3$ | $CH_2$(L-24a) |
| H | $C(CH_3)_2CH_2SO_2CH_3$ | $CF_3$ | $CH_2$(L-36a) |
| H | $C(CH_3)_2CH_2SO_2CH_3$ | $CF_3$ | $CH_2CH_2Ph$ |
| H | $C(CH_3)_2CH_2SO_2CH_3$ | $CF_3$ | $CH_2CH_2(Ph-2-Cl)$ |
| H | $C(CH_3)_2CH_2SO_2CH_3$ | $CF_3$ | $CH_2CH_2(Ph-3-Cl)$ |
| H | $C(CH_3)_2CH_2SO_2CH_3$ | $CF_3$ | $CH_2CH_2(Ph-4-Cl)$ |
| H | $C(CH_3)_2CH_2SO_2CH_3$ | $CF_3$ | T-22 |
| H | $C(CH_3)_2CH_2SO_2CH_3$ | $CF_3$ | T-23 |
| H | $C(CH_3)_2CH_2SO_2CH_3$ | $CF_3$ | T-24 |
| H | $C(CH_3)_2CH_2SO_2CH_3$ | $CF_3$ | C(O)OEt |
| H | $C(CH_3)_2CH_2SO_2CH_3$ | $CF_3$ | C(O)OBu-t |
| H | $C(CH_3)_2CH_2SO_2CH_3$ | $CF_3$ | $C(O)OCH_2CF_3$ |
| H | $C(CH_3)_2CH_2SO_2CH_3$ | $CF_3$ | CH=CH(Ph-2-F) |
| H | $C(CH_3)_2CH_2SO_2CH_3$ | $CF_3$ | CH=CH(Ph-3-F) |
| H | $C(CH_3)_2CH_2SO_2CH_3$ | $CF_3$ | CH=CH(Ph-4-F) |
| H | $C(CH_3)_2CH_2SO_2CH_3$ | $CF_3$ | CH=CH(Ph-2-Cl) |
| H | $C(CH_3)_2CH_2SO_2CH_3$ | $CF_3$ | CH=CH(Ph-3-Cl) |
| H | $C(CH_3)_2CH_2SO_2CH_3$ | $CF_3$ | CH=CH(Ph-4-Cl) |
| H | $C(CH_3)_2CH_2SO_2CH_3$ | $CF_3$ | CH=CH(Ph-2-Br) |
| H | $C(CH_3)_2CH_2SO_2CH_3$ | $CF_3$ | CH=CH(Ph-3-Br) |
| H | $C(CH_3)_2CH_2SO_2CH_3$ | $CF_3$ | CH=CH(Ph-4-Br) |
| H | $C(CH_3)_2CH_2SO_2CH_3$ | $CF_3$ | $CH=CH(Ph-2-CF_3)$ |

| | | | |
|---|---|---|---|
| H | $C(CH_3)_2CH_2SO_2CH_3$ | $CF_3$ | CH=CH (Ph-3-$CF_3$) |
| H | $C(CH_3)_2CH_2SO_2CH_3$ | $CF_3$ | CH=CH (Ph-4-$CF_3$) |
| H | $C(CH_3)_2CH_2SO_2CH_3$ | $CF_3$ | CH=CH (Ph-2-$OCF_3$) |
| H | $C(CH_3)_2CH_2SO_2CH_3$ | $CF_3$ | CH=CH (Ph-3-$OCF_3$) |
| H | $C(CH_3)_2CH_2SO_2CH_3$ | $CF_3$ | CH=CH (Ph-4-$OCF_3$) |
| H | $C(CH_3)_2CH_2SO_2CH_3$ | $CF_3$ | CH=CH (Ph-2-$SCH_3$) |
| H | $C(CH_3)_2CH_2SO_2CH_3$ | $CF_3$ | CH=CH (Ph-3-$SCH_3$) |
| H | $C(CH_3)_2CH_2SO_2CH_3$ | $CF_3$ | CH=CH (Ph-4-$SCH_3$) |
| H | $C(CH_3)_2CH_2SO_2CH_3$ | $CF_3$ | CH=CH (Ph-2-$SO_2CH_3$) |
| H | $C(CH_3)_2CH_2SO_2CH_3$ | $CF_3$ | CH=CH (Ph-3-$SO_2CH_3$) |
| H | $C(CH_3)_2CH_2SO_2CH_3$ | $CF_3$ | CH=CH (Ph-4-$SO_2CH_3$) |
| H | $C(CH_3)_2CH_2SO_2CH_3$ | $CF_3$ | CH=CH (Ph-3, 4-$F_2$) |
| H | $C(CH_3)_2CH_2SO_2CH_3$ | $CF_3$ | CH=CH (Ph-3-F-4-Cl) |
| H | $C(CH_3)_2CH_2SO_2CH_3$ | $CF_3$ | CH=CH (Ph-3, 4-$Cl_2$) |
| H | $C(CH_3)_2CH_2SO_2CH_3$ | $CF_3$ | CH=CH (Ph-3, 4-$Br_2$) |
| H | $C(CH_3)_2CH_2SO_2CH_3$ | $CF_3$ | CH=CH (Ph-3-F-4-$CF_3$) |
| H | $C(CH_3)_2CH_2SO_2CH_3$ | $CF_3$ | CH=CH (Ph-3-Cl-4-$OCF_3$) |
| H | $C(CH_3)_2CH_2SO_2CH_3$ | $CF_3$ | CH=CH [Ph (-3-$OCF_2O$-4-) ] |
| H | $C(CH_3)_2CH_2SO_2CH_3$ | $CF_3$ | Ph |
| H | $C(CH_3)_2CH_2SO_2CH_3$ | $CF_3$ | Ph-2-F |
| H | $C(CH_3)_2CH_2SO_2CH_3$ | $CF_3$ | Ph-3-F |
| H | $C(CH_3)_2CH_2SO_2CH_3$ | $CF_3$ | Ph-4-F |
| H | $C(CH_3)_2CH_2SO_2CH_3$ | $CF_3$ | Ph-2-Cl |
| H | $C(CH_3)_2CH_2SO_2CH_3$ | $CF_3$ | Ph-3-Cl |
| H | $C(CH_3)_2CH_2SO_2CH_3$ | $CF_3$ | Ph-4-Cl |
| H | $C(CH_3)_2CH_2SO_2CH_3$ | $CF_3$ | Ph-2-Br |
| H | $C(CH_3)_2CH_2SO_2CH_3$ | $CF_3$ | Ph-3-Br |
| H | $C(CH_3)_2CH_2SO_2CH_3$ | $CF_3$ | Ph-4-Br |
| H | $C(CH_3)_2CH_2SO_2CH_3$ | $CF_3$ | Ph-4-I |
| H | $C(CH_3)_2CH_2SO_2CH_3$ | $CF_3$ | Ph-4-Bu-t |
| H | $C(CH_3)_2CH_2SO_2CH_3$ | $CF_3$ | Ph-2-$CF_3$ |
| H | $C(CH_3)_2CH_2SO_2CH_3$ | $CF_3$ | Ph-3-$CF_3$ |
| H | $C(CH_3)_2CH_2SO_2CH_3$ | $CF_3$ | Ph-4-$CF_3$ |
| H | $C(CH_3)_2CH_2SO_2CH_3$ | $CF_3$ | Ph-4-$OCH_3$ |
| H | $C(CH_3)_2CH_2SO_2CH_3$ | $CF_3$ | Ph-4-$OCHF_2$ |
| H | $C(CH_3)_2CH_2SO_2CH_3$ | $CF_3$ | Ph-2-$OCF_3$ |
| H | $C(CH_3)_2CH_2SO_2CH_3$ | $CF_3$ | Ph-3-$OCF_3$ |
| H | $C(CH_3)_2CH_2SO_2CH_3$ | $CF_3$ | Ph-4-$OCF_3$ |
| H | $C(CH_3)_2CH_2SO_2CH_3$ | $CF_3$ | Ph-4-$OCF_2Br$ |
| H | $C(CH_3)_2CH_2SO_2CH_3$ | $CF_3$ | Ph-4-$OCH_2CF_3$ |
| H | $C(CH_3)_2CH_2SO_2CH_3$ | $CF_3$ | Ph-4-$OCF_2CHF_2$ |

| | | | |
|---|---|---|---|
| H | $C(CH_3)_2CH_2SO_2CH_3$ | $CF_3$ | Ph-4-$OCF_2CHFCl$ |
| H | $C(CH_3)_2CH_2SO_2CH_3$ | $CF_3$ | Ph-4-$OCF_2CHFBr$ |
| H | $C(CH_3)_2CH_2SO_2CH_3$ | $CF_3$ | Ph-4-$OCF_2CF_2Br$ |
| H | $C(CH_3)_2CH_2SO_2CH_3$ | $CF_3$ | Ph-4-$OCF_2CFCl_2$ |
| H | $C(CH_3)_2CH_2SO_2CH_3$ | $CF_3$ | Ph-4-$OCF_2CCl_3$ |
| H | $C(CH_3)_2CH_2SO_2CH_3$ | $CF_3$ | Ph-4-$OCH_2CF_2CHF_2$ |
| H | $C(CH_3)_2CH_2SO_2CH_3$ | $CF_3$ | Ph-3-$OCF_2CHFCF_3$ |
| H | $C(CH_3)_2CH_2SO_2CH_3$ | $CF_3$ | Ph-4-$OCF_2CHFCF_3$ |
| H | $C(CH_3)_2CH_2SO_2CH_3$ | $CF_3$ | Ph-4-$OCH(CF_3)_2$ |
| H | $C(CH_3)_2CH_2SO_2CH_3$ | $CF_3$ | Ph-4-$OCF_2CFBrCF_3$ |
| H | $C(CH_3)_2CH_2SO_2CH_3$ | $CF_3$ | Ph-3-$OCF_2CHFOCF_3$ |
| H | $C(CH_3)_2CH_2SO_2CH_3$ | $CF_3$ | Ph-4-$OCF_2CHFOCF_3$ |
| H | $C(CH_3)_2CH_2SO_2CH_3$ | $CF_3$ | Ph-4-$OCF_2CHFOCF_2CF_2CF_3$ |
| H | $C(CH_3)_2CH_2SO_2CH_3$ | $CF_3$ | Ph-4-$OSO_2CH_3$ |
| H | $C(CH_3)_2CH_2SO_2CH_3$ | $CF_3$ | Ph-4-$OSO_2CF_3$ |
| H | $C(CH_3)_2CH_2SO_2CH_3$ | $CF_3$ | Ph-3-O(Ph-4-Cl) |
| H | $C(CH_3)_2CH_2SO_2CH_3$ | $CF_3$ | Ph-4-O(Ph-4-Cl) |
| H | $C(CH_3)_2CH_2SO_2CH_3$ | $CF_3$ | Ph-4-O(Ph-4-Br) |
| H | $C(CH_3)_2CH_2SO_2CH_3$ | $CF_3$ | Ph-4-O(Ph-4-$CF_3$) |
| H | $C(CH_3)_2CH_2SO_2CH_3$ | $CF_3$ | Ph-4-O(L-45c)Br |
| H | $C(CH_3)_2CH_2SO_2CH_3$ | $CF_3$ | Ph-4-O(L-45c)$CF_3$ |
| H | $C(CH_3)_2CH_2SO_2CH_3$ | $CF_3$ | Ph-3-O(L-45e) |
| H | $C(CH_3)_2CH_2SO_2CH_3$ | $CF_3$ | Ph-4-O(L-45e) |
| H | $C(CH_3)_2CH_2SO_2CH_3$ | $CF_3$ | Ph-4-O(L-48b)Br |
| H | $C(CH_3)_2CH_2SO_2CH_3$ | $CF_3$ | Ph-4-$SCH_3$ |
| H | $C(CH_3)_2CH_2SO_2CH_3$ | $CF_3$ | Ph-4-$SO_2CH_3$ |
| H | $C(CH_3)_2CH_2SO_2CH_3$ | $CF_3$ | Ph-3-S(Ph-4-Cl) |
| H | $C(CH_3)_2CH_2SO_2CH_3$ | $CF_3$ | Ph-4-S(Ph-4-Cl) |
| H | $C(CH_3)_2CH_2SO_2CH_3$ | $CF_3$ | Ph-4-S(Ph-4-Br) |
| H | $C(CH_3)_2CH_2SO_2CH_3$ | $CF_3$ | Ph-4-S(Ph-4-$CF_3$) |
| H | $C(CH_3)_2CH_2SO_2CH_3$ | $CF_3$ | Ph-4-S(L-45c)Br |
| H | $C(CH_3)_2CH_2SO_2CH_3$ | $CF_3$ | Ph-4-S(L-45c)$CF_3$ |
| H | $C(CH_3)_2CH_2SO_2CH_3$ | $CF_3$ | Ph-4-S(L-45e) |
| H | $C(CH_3)_2CH_2SO_2CH_3$ | $CF_3$ | Ph-4-S(L-48b)Br |
| H | $C(CH_3)_2CH_2SO_2CH_3$ | $CF_3$ | Ph-4-$NO_2$ |
| H | $C(CH_3)_2CH_2SO_2CH_3$ | $CF_3$ | Ph-4-CN |
| H | $C(CH_3)_2CH_2SO_2CH_3$ | $CF_3$ | Ph-2,3-$F_2$ |
| H | $C(CH_3)_2CH_2SO_2CH_3$ | $CF_3$ | Ph-2,4-$F_2$ |
| H | $C(CH_3)_2CH_2SO_2CH_3$ | $CF_3$ | Ph-3,4-$F_2$ |
| H | $C(CH_3)_2CH_2SO_2CH_3$ | $CF_3$ | Ph-2,5-$F_2$ |
| H | $C(CH_3)_2CH_2SO_2CH_3$ | $CF_3$ | Ph-3,5-$F_2$ |

| | | | |
|---|---|---|---|
| H | $C(CH_3)_2CH_2SO_2CH_3$ | $CF_3$ | Ph-2-Cl-4-F |
| H | $C(CH_3)_2CH_2SO_2CH_3$ | $CF_3$ | Ph-2-F-3-Cl |
| H | $C(CH_3)_2CH_2SO_2CH_3$ | $CF_3$ | Ph-3-Cl-4-F |
| H | $C(CH_3)_2CH_2SO_2CH_3$ | $CF_3$ | Ph-2-F-4-Cl |
| H | $C(CH_3)_2CH_2SO_2CH_3$ | $CF_3$ | Ph-3-F-4-Cl |
| H | $C(CH_3)_2CH_2SO_2CH_3$ | $CF_3$ | Ph-2, 3-$Cl_2$ |
| H | $C(CH_3)_2CH_2SO_2CH_3$ | $CF_3$ | Ph-2, 4-$Cl_2$ |
| H | $C(CH_3)_2CH_2SO_2CH_3$ | $CF_3$ | Ph-2, 5-$Cl_2$ |
| H | $C(CH_3)_2CH_2SO_2CH_3$ | $CF_3$ | Ph-3, 4-$Cl_2$ |
| H | $C(CH_3)_2CH_2SO_2CH_3$ | $CF_3$ | Ph-3, 5-$Cl_2$ |
| H | $C(CH_3)_2CH_2SO_2CH_3$ | $CF_3$ | Ph-3-Br-4-F |
| H | $C(CH_3)_2CH_2SO_2CH_3$ | $CF_3$ | Ph-2-F-4-Br |
| H | $C(CH_3)_2CH_2SO_2CH_3$ | $CF_3$ | Ph-2-F-5-Br |
| H | $C(CH_3)_2CH_2SO_2CH_3$ | $CF_3$ | Ph-3, 4-$Br_2$ |
| H | $C(CH_3)_2CH_2SO_2CH_3$ | $CF_3$ | Ph-3, 5-$Br_2$ |
| H | $C(CH_3)_2CH_2SO_2CH_3$ | $CF_3$ | Ph-3-$CH_3$-4-F |
| H | $C(CH_3)_2CH_2SO_2CH_3$ | $CF_3$ | Ph-3-F-4-$CH_3$ |
| H | $C(CH_3)_2CH_2SO_2CH_3$ | $CF_3$ | Ph-2-F-5-$CH_3$ |
| H | $C(CH_3)_2CH_2SO_2CH_3$ | $CF_3$ | Ph-2, 4-$(CH_3)_2$ |
| H | $C(CH_3)_2CH_2SO_2CH_3$ | $CF_3$ | Ph-3, 4-$(CH_3)_2$ |
| H | $C(CH_3)_2CH_2SO_2CH_3$ | $CF_3$ | Ph-2-F-3-$CF_3$ |
| H | $C(CH_3)_2CH_2SO_2CH_3$ | $CF_3$ | Ph-3-$CF_3$-4-F |
| H | $C(CH_3)_2CH_2SO_2CH_3$ | $CF_3$ | Ph-3-$CF_3$-4-Cl |
| H | $C(CH_3)_2CH_2SO_2CH_3$ | $CF_3$ | Ph-2-F-4-$CF_3$ |
| H | $C(CH_3)_2CH_2SO_2CH_3$ | $CF_3$ | Ph-3-F-4-$CF_3$ |
| H | $C(CH_3)_2CH_2SO_2CH_3$ | $CF_3$ | Ph-2-F-5-$CF_3$ |
| H | $C(CH_3)_2CH_2SO_2CH_3$ | $CF_3$ | Ph-3-F-5-$CF_3$ |
| H | $C(CH_3)_2CH_2SO_2CH_3$ | $CF_3$ | Ph-2-Cl-4-$CF_3$ |
| H | $C(CH_3)_2CH_2SO_2CH_3$ | $CF_3$ | Ph-3, 5-$(CF_3)_2$ |
| H | $C(CH_3)_2CH_2SO_2CH_3$ | $CF_3$ | Ph-3-Br-4-$OCH_3$ |
| H | $C(CH_3)_2CH_2SO_2CH_3$ | $CF_3$ | Ph-3-F-4-$OCHF_2$ |
| H | $C(CH_3)_2CH_2SO_2CH_3$ | $CF_3$ | Ph-3-Cl-4-$OCHF_2$ |
| H | $C(CH_3)_2CH_2SO_2CH_3$ | $CF_3$ | Ph-3-Br-4-$OCHF_2$ |
| H | $C(CH_3)_2CH_2SO_2CH_3$ | $CF_3$ | Ph-3-F-4-$OCF_3$ |
| H | $C(CH_3)_2CH_2SO_2CH_3$ | $CF_3$ | Ph-3-Cl-4-$OCF_3$ |
| H | $C(CH_3)_2CH_2SO_2CH_3$ | $CF_3$ | Ph-3-Br-4-$OCF_3$ |
| H | $C(CH_3)_2CH_2SO_2CH_3$ | $CF_3$ | Ph-3-F-4-$OCF_2Br$ |
| H | $C(CH_3)_2CH_2SO_2CH_3$ | $CF_3$ | Ph-3-Cl-4-$OCF_2Br$ |
| H | $C(CH_3)_2CH_2SO_2CH_3$ | $CF_3$ | Ph-3-Br-4-$OCF_2Br$ |
| H | $C(CH_3)_2CH_2SO_2CH_3$ | $CF_3$ | Ph-3-F-4-$OCF_2CHF_2$ |
| H | $C(CH_3)_2CH_2SO_2CH_3$ | $CF_3$ | Ph-3-Cl-4-$OCF_2CHF_2$ |

| | | | |
|---|---|---|---|
| H | $C(CH_3)_2CH_2SO_2CH_3$ | $CF_3$ | Ph-3-Br-4-$OCF_2CHF_2$ |
| H | $C(CH_3)_2CH_2SO_2CH_3$ | $CF_3$ | Ph-3-F-4-$OCF_2CHFCl$ |
| H | $C(CH_3)_2CH_2SO_2CH_3$ | $CF_3$ | Ph-3-Cl-4-$OCF_2CHFCl$ |
| H | $C(CH_3)_2CH_2SO_2CH_3$ | $CF_3$ | Ph-3-Br-4-$OCF_2CHFCl$ |
| H | $C(CH_3)_2CH_2SO_2CH_3$ | $CF_3$ | Ph-3-F-4-$OCF_2CHFCF_3$ |
| H | $C(CH_3)_2CH_2SO_2CH_3$ | $CF_3$ | Ph-3-Cl-4-$OCF_2CHFCF_3$ |
| H | $C(CH_3)_2CH_2SO_2CH_3$ | $CF_3$ | Ph-3-Br-4-$OCF_2CHFCF_3$ |
| H | $C(CH_3)_2CH_2SO_2CH_3$ | $CF_3$ | Ph-3-F-4-$OCF_2CHFOCF_3$ |
| H | $C(CH_3)_2CH_2SO_2CH_3$ | $CF_3$ | Ph-3-Cl-4-$OCF_2CHFOCF_3$ |
| H | $C(CH_3)_2CH_2SO_2CH_3$ | $CF_3$ | Ph-3-Br-4-$OCF_2CHFOCF_3$ |
| H | $C(CH_3)_2CH_2SO_2CH_3$ | $CF_3$ | Ph-3-$CH_3$-4-$OCF_2CHFOCF_3$ |
| H | $C(CH_3)_2CH_2SO_2CH_3$ | $CF_3$ | Ph-3-F-4-$OCF_2CHFOCF_2CF_2CF_3$ |
| H | $C(CH_3)_2CH_2SO_2CH_3$ | $CF_3$ | Ph-3-Cl-4-$OCF_2CHFOCF_2CF_2CF_3$ |
| H | $C(CH_3)_2CH_2SO_2CH_3$ | $CF_3$ | Ph-3-Br-4-$OCF_2CHFOCF_2CF_2CF_3$ |
| H | $C(CH_3)_2CH_2SO_2CH_3$ | $CF_3$ | Ph(-3-$OCF_2O$-4-) |
| H | $C(CH_3)_2CH_2SO_2CH_3$ | $CF_3$ | Ph(-3-$OCF_2CF_2O$-4-) |
| H | $C(CH_3)_2CH_2SO_2CH_3$ | $CF_3$ | Ph-3-OPh-4-F |
| H | $C(CH_3)_2CH_2SO_2CH_3$ | $CF_3$ | Ph-3-$NO_2$-4-F |
| H | $C(CH_3)_2CH_2SO_2CH_3$ | $CF_3$ | Ph-3-$NO_2$-4-Cl |
| H | $C(CH_3)_2CH_2SO_2CH_3$ | $CF_3$ | Ph-2-F-5-$NO_2$ |
| H | $C(CH_3)_2CH_2SO_2CH_3$ | $CF_3$ | Ph-3-CN-4-F |
| H | $C(CH_3)_2CH_2SO_2CH_3$ | $CF_3$ | Ph-2, 3, 4-$F_3$ |
| H | $C(CH_3)_2CH_2SO_2CH_3$ | $CF_3$ | Ph-2, 3, 5-$F_3$ |
| H | $C(CH_3)_2CH_2SO_2CH_3$ | $CF_3$ | Ph-2, 4, 5-$F_3$ |
| H | $C(CH_3)_2CH_2SO_2CH_3$ | $CF_3$ | Ph-3, 4, 5-$F_3$ |
| H | $C(CH_3)_2CH_2SO_2CH_3$ | $CF_3$ | Ph-2, 3-$F_2$-4-$CH_3$ |
| H | $C(CH_3)_2CH_2SO_2CH_3$ | $CF_3$ | Ph-2, 3-$F_2$-4-$CF_3$ |
| H | $C(CH_3)_2CH_2SO_2CH_3$ | $CF_3$ | Ph-3, 4-$F_2$-5-$CF_3$ |
| H | $C(CH_3)_2CH_2SO_2CH_3$ | $CF_3$ | Ph-2-F-3-Cl-5-$CF_3$ |
| H | $C(CH_3)_2CH_2SO_2CH_3$ | $CF_3$ | Ph-3, 5-$Cl_2$-4-$OCH_3$ |
| H | $C(CH_3)_2CH_2SO_2CH_3$ | $CF_3$ | 1-Naph |
| H | $C(CH_3)_2CH_2SO_2CH_3$ | $CF_3$ | 2-Naph |
| H | $C(CH_3)_2CH_2SO_2CH_3$ | $CF_3$ | (L-1b) Br |
| H | $C(CH_3)_2CH_2SO_2CH_3$ | $CF_3$ | (L-1c) Cl |
| H | $C(CH_3)_2CH_2SO_2CH_3$ | $CF_3$ | (L-1c) Br |
| H | $C(CH_3)_2CH_2SO_2CH_3$ | $CF_3$ | (L-1c) I |
| H | $C(CH_3)_2CH_2SO_2CH_3$ | $CF_3$ | (L-1c) $CF_3$ |
| H | $C(CH_3)_2CH_2SO_2CH_3$ | $CF_3$ | (L-2b) Br |
| H | $C(CH_3)_2CH_2SO_2CH_3$ | $CF_3$ | (L-3b) Cl |
| H | $C(CH_3)_2CH_2SO_2CH_3$ | $CF_3$ | (L-3b) Br |
| H | $C(CH_3)_2CH_2SO_2CH_3$ | $CF_3$ | (L-3c) F |

| | | | |
|---|---|---|---|
| H | $C(CH_3)_2CH_2SO_2CH_3$ | $CF_3$ | (L-3c) Cl |
| H | $C(CH_3)_2CH_2SO_2CH_3$ | $CF_3$ | (L-3c) Br |
| H | $C(CH_3)_2CH_2SO_2CH_3$ | $CF_3$ | (L-3c) I |
| H | $C(CH_3)_2CH_2SO_2CH_3$ | $CF_3$ | (L-3c) $CF_3$ |
| H | $C(CH_3)_2CH_2SO_2CH_3$ | $CF_3$ | (L-3c) CN |
| H | $C(CH_3)_2CH_2SO_2CH_3$ | $CF_3$ | L-3d |
| H | $C(CH_3)_2CH_2SO_2CH_3$ | $CF_3$ | (L-4b) Cl |
| H | $C(CH_3)_2CH_2SO_2CH_3$ | $CF_3$ | (L-4b) Br |
| H | $C(CH_3)_2CH_2SO_2CH_3$ | $CF_3$ | (L-4b) CN |
| H | $C(CH_3)_2CH_2SO_2CH_3$ | $CF_3$ | (L-10b) Cl |
| H | $C(CH_3)_2CH_2SO_2CH_3$ | $CF_3$ | (L-10b) Br |
| H | $C(CH_3)_2CH_2SO_2CH_3$ | $CF_3$ | (L-15b) $CF_3$ |
| H | $C(CH_3)_2CH_2SO_2CH_3$ | $CF_3$ | (L-16a) $CHF_2$ |
| H | $C(CH_3)_2CH_2SO_2CH_3$ | $CF_3$ | (L-16a) $CF_2Br$ |
| H | $C(CH_3)_2CH_2SO_2CH_3$ | $CF_3$ | (L-17a) Cl |
| H | $C(CH_3)_2CH_2SO_2CH_3$ | $CF_3$ | (L-21b) Cl |
| H | $C(CH_3)_2CH_2SO_2CH_3$ | $CF_3$ | (L-21b) Br |
| H | $C(CH_3)_2CH_2SO_2CH_3$ | $CF_3$ | (L-21b) I |
| H | $C(CH_3)_2CH_2SO_2CH_3$ | $CF_3$ | (L-21b) $CF_3$ |
| H | $C(CH_3)_2CH_2SO_2CH_3$ | $CF_3$ | (L-22b) Cl |
| H | $C(CH_3)_2CH_2SO_2CH_3$ | $CF_3$ | (L-22b) Br |
| H | $C(CH_3)_2CH_2SO_2CH_3$ | $CF_3$ | (L-23b) Cl |
| H | $C(CH_3)_2CH_2SO_2CH_3$ | $CF_3$ | (L-23b) Br |
| H | $C(CH_3)_2CH_2SO_2CH_3$ | $CF_3$ | (L-23c) Cl |
| H | $C(CH_3)_2CH_2SO_2CH_3$ | $CF_3$ | (L-23c) Br |
| H | $C(CH_3)_2CH_2SO_2CH_3$ | $CF_3$ | (L-31a) Cl |
| H | $C(CH_3)_2CH_2SO_2CH_3$ | $CF_3$ | (L-31a) Br |
| H | $C(CH_3)_2CH_2SO_2CH_3$ | $CF_3$ | (L-45c) F |
| H | $C(CH_3)_2CH_2SO_2CH_3$ | $CF_3$ | (L-45c) Cl |
| H | $C(CH_3)_2CH_2SO_2CH_3$ | $CF_3$ | (L-45c) Br |
| H | $C(CH_3)_2CH_2SO_2CH_3$ | $CF_3$ | (L-45c) I |
| H | $C(CH_3)_2CH_2SO_2CH_3$ | $CF_3$ | (L-45c) $CF_3$ |
| H | $C(CH_3)_2CH_2SO_2CH_3$ | $CF_3$ | L-45e |
| H | $C(CH_3)_2CH_2SO_2CH_3$ | $CF_3$ | L-45f |
| H | $C(CH_3)_2CH_2SO_2CH_3$ | $CF_3$ | (L-46c) F |
| H | $C(CH_3)_2CH_2SO_2CH_3$ | $CF_3$ | (L-46c) Cl |
| H | $C(CH_3)_2CH_2SO_2CH_3$ | $CF_3$ | (L-46c) Br |
| H | $C(CH_3)_2CH_2SO_2CH_3$ | $CF_3$ | (L-46c) I |
| H | $C(CH_3)_2CH_2SO_2CH_3$ | $CF_3$ | (L-46c) $CF_3$ |
| H | $C(CH_3)_2CH_2SO_2CH_3$ | $CF_3$ | (L-46c) $OCH_2CF_3$ |
| H | $C(CH_3)_2CH_2SO_2CH_3$ | $CF_3$ | (L-46c) $OCH(CF_3)_2$ |

| | | | |
|---|---|---|---|
| H | $C(CH_3)_2CH_2SO_2CH_3$ | $CF_3$ | L-46d |
| H | $C(CH_3)_2CH_2SO_2CH_3$ | $CF_3$ | L-47a |
| H | $C(CH_3)_2CH_2SO_2CH_3$ | $CF_3$ | L-47d |
| H | $C(CH_3)_2CH_2SO_2CH_3$ | $CF_3$ | (L-48b) Br |
| H | $C(CH_3)_2CH_2SO_2CH_3$ | $CF_3$ | (L-50b) Cl |
| H | $C(CH_3)_2CH_2SO_2CH_3$ | $CF_3$ | (L-50b) Br |
| H | $C(CH_3)_2CH_2SO_2CH_3$ | $CF_3$ | (L-51b) Cl |
| H | $C(CH_3)_2CH_2SO_2CH_3$ | $CF_2Cl$ | Ph-4-Cl |
| H | $C(CH_3)_2CH_2SO_2CH_3$ | $CF_2Cl$ | Ph-4-Br |
| H | $C(CH_3)_2CH_2SO_2CH_3$ | $CF_2Cl$ | Ph-4-$CF_3$ |
| H | $C(CH_3)_2CH_2SO_2CH_3$ | $CF_2Cl$ | Ph-4-$OCF_3$ |
| H | $C(CH_3)_2CH_2SO_2CH_3$ | $CF_2Cl$ | Ph-4-$OCF_2Br$ |
| H | $C(CH_3)_2CH_2SO_2CH_3$ | $CF_2Cl$ | Ph-4-$OCF_2CHFCF_3$ |
| H | $C(CH_3)_2CH_2SO_2CH_3$ | $CF_2Cl$ | Ph-4-$OCF_2CHFOCF_3$ |
| H | $C(CH_3)_2CH_2SO_2CH_3$ | $CF_2Cl$ | Ph-4-$OCF_2CHFOCF_2CF_2CF_3$ |
| H | $C(CH_3)_2CH_2SO_2CH_3$ | $CF_2Cl$ | Ph-4-O (L-45g) |
| H | $C(CH_3)_2CH_2SO_2CH_3$ | $CF_2Cl$ | Ph-3, 4-$Cl_2$ |
| H | $C(CH_3)_2CH_2SO_2CH_3$ | $CF_2Cl$ | Ph (-3-$OCF_2O$-4-) |
| H | $C(CH_3)_2CH_2SO_2CH_3$ | $CF_2Cl$ | Ph (-3-$OCF_2CF_2O$-4-) |
| H | $C(CH_3)_2CH_2SO_2CH_3$ | $CF_2Cl$ | (L-45c) $CF_3$ |
| H | $C(CH_3)_2CH_2SO_2CH_3$ | $CF_2Cl$ | L-45f |
| H | $C(CH_3)_2CH_2SO_2CH_3$ | $CF_2Cl$ | (L-46c) $CF_3$ |
| H | $C(CH_3)_2CH_2SO_2CH_3$ | $CF_2Cl$ | L-46d |
| H | $C(CH_3)_2CH_2SO_2CH_3$ | $CFCl_2$ | Ph-4-Br |
| H | $C(CH_3)_2CH_2SO_2CH_3$ | $CF_2Br$ | Ph-4-Cl |
| H | $C(CH_3)_2CH_2SO_2CH_3$ | $CF_2Br$ | Ph-4-Br |
| H | $C(CH_3)_2CH_2SO_2CH_3$ | $CF_2Br$ | Ph-4-$CF_3$ |
| H | $C(CH_3)_2CH_2SO_2CH_3$ | $CF_2Br$ | Ph-4-$OCF_3$ |
| H | $C(CH_3)_2CH_2SO_2CH_3$ | $CF_2Br$ | Ph-4-$OCF_2Br$ |
| H | $C(CH_3)_2CH_2SO_2CH_3$ | $CF_2Br$ | Ph-4-$OCF_2CHFCF_3$ |
| H | $C(CH_3)_2CH_2SO_2CH_3$ | $CF_2Br$ | Ph-4-$OCF_2CHFOCF_3$ |
| H | $C(CH_3)_2CH_2SO_2CH_3$ | $CF_2Br$ | Ph-4-$OCF_2CHFOCF_2CF_2CF_3$ |
| H | $C(CH_3)_2CH_2SO_2CH_3$ | $CF_2Br$ | Ph-4-O (L-45g) |
| H | $C(CH_3)_2CH_2SO_2CH_3$ | $CF_2Br$ | Ph-3, 4-$Cl_2$ |
| H | $C(CH_3)_2CH_2SO_2CH_3$ | $CF_2Br$ | Ph (-3-$OCF_2O$-4-) |
| H | $C(CH_3)_2CH_2SO_2CH_3$ | $CF_2Br$ | Ph (-3-$OCF_2CF_2O$-4-) |
| H | $C(CH_3)_2CH_2SO_2CH_3$ | $CF_2Br$ | (L-45c) $CF_3$ |
| H | $C(CH_3)_2CH_2SO_2CH_3$ | $CF_2Br$ | L-45f |
| H | $C(CH_3)_2CH_2SO_2CH_3$ | $CF_2Br$ | (L-46c) $CF_3$ |
| H | $C(CH_3)_2CH_2SO_2CH_3$ | $CF_2Br$ | L-46d |
| H | $C(CH_3)_2CH_2SO_2CH_3$ | CFClBr | Ph-4-$CF_3$ |

| | | | |
|---|---|---|---|
| H | $C(CH_3)_2CH_2SO_2CH_3$ | $CFBr_2$ | Ph-4-$OCF_3$ |
| H | $C(CH_3)_2CH_2SO_2CH_3$ | $CF_2CHF_2$ | (L-45c) $CF_3$ |
| H | $C(CH_3)_2CH_2SO_2CH_3$ | $CF_2CF_3$ | Ph-4-Cl |
| H | $C(CH_3)_2CH_2SO_2CH_3$ | $CF_2CF_3$ | Ph-4-Br |
| H | $C(CH_3)_2CH_2SO_2CH_3$ | $CF_2CF_3$ | Ph-4-$CF_3$ |
| H | $C(CH_3)_2CH_2SO_2CH_3$ | $CF_2CF_3$ | Ph-4-$OCF_3$ |
| H | $C(CH_3)_2CH_2SO_2CH_3$ | $CF_2CF_3$ | Ph-4-$OCF_2Br$ |
| H | $C(CH_3)_2CH_2SO_2CH_3$ | $CF_2CF_3$ | Ph-4-$OCF_2CHFCF_3$ |
| H | $C(CH_3)_2CH_2SO_2CH_3$ | $CF_2CF_3$ | Ph-4-$OCF_2CHFOCF_3$ |
| H | $C(CH_3)_2CH_2SO_2CH_3$ | $CF_2CF_3$ | Ph-4-$OCF_2CHFOCF_2CF_2CF_3$ |
| H | $C(CH_3)_2CH_2SO_2CH_3$ | $CF_2CF_3$ | Ph-4-O (L-45g) |
| H | $C(CH_3)_2CH_2SO_2CH_3$ | $CF_2CF_3$ | Ph-3, 4-$Cl_2$ |
| H | $C(CH_3)_2CH_2SO_2CH_3$ | $CF_2CF_3$ | Ph (-3-$OCF_2O$-4-) |
| H | $C(CH_3)_2CH_2SO_2CH_3$ | $CF_2CF_3$ | Ph (-3-$OCF_2CF_2O$-4-) |
| H | $C(CH_3)_2CH_2SO_2CH_3$ | $CF_2CF_3$ | (L-45c) $CF_3$ |
| H | $C(CH_3)_2CH_2SO_2CH_3$ | $CF_2CF_3$ | L-45f |
| H | $C(CH_3)_2CH_2SO_2CH_3$ | $CF_2CF_3$ | (L-46c) $CF_3$ |
| H | $C(CH_3)_2CH_2SO_2CH_3$ | $CF_2CF_3$ | L-46d |
| H | $C(CH_3)_2CH_2SO_2CH_3$ | $CF_2CF_2Cl$ | Ph-4-Br |
| H | $C(CH_3)_2CH_2SO_2CH_3$ | $CFClCF_3$ | Ph-4-$CF_3$ |
| H | $C(CH_3)_2CH_2SO_2CH_3$ | $CFClCF_2Cl$ | Ph-4-$OCF_3$ |
| H | $C(CH_3)_2CH_2SO_2CH_3$ | $CF_2CF_2Br$ | (L-45c) $CF_3$ |
| H | $C(CH_3)_2CH_2SO_2CH_3$ | $CFBrCF_3$ | Ph-4-Br |
| H | $C(CH_3)_2CH_2SO_2CH_3$ | $CF_2CHFCF_3$ | Ph-4-$CF_3$ |
| H | $C(CH_3)_2CH_2SO_2CH_3$ | $CF_2CF_2CF_3$ | Ph-4-Cl |
| H | $C(CH_3)_2CH_2SO_2CH_3$ | $CF_2CF_2CF_3$ | Ph-4-Br |
| H | $C(CH_3)_2CH_2SO_2CH_3$ | $CF_2CF_2CF_3$ | Ph-4-$CF_3$ |
| H | $C(CH_3)_2CH_2SO_2CH_3$ | $CF_2CF_2CF_3$ | Ph-4-$OCF_3$ |
| H | $C(CH_3)_2CH_2SO_2CH_3$ | $CF_2CF_2CF_3$ | Ph-4-$OCF_2Br$ |
| H | $C(CH_3)_2CH_2SO_2CH_3$ | $CF_2CF_2CF_3$ | Ph-4-$OCF_2CHFCF_3$ |
| H | $C(CH_3)_2CH_2SO_2CH_3$ | $CF_2CF_2CF_3$ | Ph-4-$OCF_2CHFOCF_3$ |
| H | $C(CH_3)_2CH_2SO_2CH_3$ | $CF_2CF_2CF_3$ | Ph-4-$OCF_2CHFOCF_2CF_2CF_3$ |
| H | $C(CH_3)_2CH_2SO_2CH_3$ | $CF_2CF_2CF_3$ | Ph-4-O (L-45g) |
| H | $C(CH_3)_2CH_2SO_2CH_3$ | $CF_2CF_2CF_3$ | Ph-3, 4-$Cl_2$ |
| H | $C(CH_3)_2CH_2SO_2CH_3$ | $CF_2CF_2CF_3$ | Ph (-3-$OCF_2O$-4-) |
| H | $C(CH_3)_2CH_2SO_2CH_3$ | $CF_2CF_2CF_3$ | Ph (-3-$OCF_2CF_2O$-4-) |
| H | $C(CH_3)_2CH_2SO_2CH_3$ | $CF_2CF_2CF_3$ | (L-45c) $CF_3$ |
| H | $C(CH_3)_2CH_2SO_2CH_3$ | $CF_2CF_2CF_3$ | L-45f |
| H | $C(CH_3)_2CH_2SO_2CH_3$ | $CF_2CF_2CF_3$ | (L-46c) $CF_3$ |
| H | $C(CH_3)_2CH_2SO_2CH_3$ | $CF_2CF_2CF_3$ | L-46d |
| H | $C(CH_3)_2CH_2SO_2CH_3$ | $CF(CF_3)_2$ | Ph-4-$OCF_3$ |

| | | | |
|---|---|---|---|
| H | $C(CH_3)_2CH_2SO_2CH_3$ | $CF_2CF_2CF_2CF_3$ | (L-45c) $CF_3$ |
| H | $C(CH_3)_2CH_2SO_2CH_3$ | T-1 | Ph-4-Br |
| H | $C(CH_3)_2CH_2SO_2CH_3$ | T-2 | Ph-4-$CF_3$ |
| H | $C(CH_3)_2CH_2SO_2CH_3$ | $CH_2OCH_3$ | Ph-4-$OCF_3$ |
| H | $C(CH_3)_2CH_2SO_2CH_3$ | $CH_2OEt$ | (L-45c) $CF_3$ |
| H | $C(CH_3)_2CH_2SO_2CH_3$ | $CH_2OCH_2CF_3$ | Ph-4-Br |
| H | $C(CH_3)_2CH_2SO_2CH_3$ | $CH_2OCH(CF_3)_2$ | Ph-4-$CF_3$ |
| H | $C(CH_3)_2CH_2SO_2CH_3$ | $CF_2OCH_3$ | Ph-4-Cl |
| H | $C(CH_3)_2CH_2SO_2CH_3$ | $CF_2OCH_3$ | Ph-4-Br |
| H | $C(CH_3)_2CH_2SO_2CH_3$ | $CF_2OCH_3$ | Ph-4-$CF_3$ |
| H | $C(CH_3)_2CH_2SO_2CH_3$ | $CF_2OCH_3$ | Ph-4-$OCF_3$ |
| H | $C(CH_3)_2CH_2SO_2CH_3$ | $CF_2OCH_3$ | Ph-4-$OCF_2Br$ |
| H | $C(CH_3)_2CH_2SO_2CH_3$ | $CF_2OCH_3$ | Ph-4-$OCF_2CHFCF_3$ |
| H | $C(CH_3)_2CH_2SO_2CH_3$ | $CF_2OCH_3$ | Ph-4-$OCF_2CHFOCF_3$ |
| H | $C(CH_3)_2CH_2SO_2CH_3$ | $CF_2OCH_3$ | Ph-4-$OCF_2CHFOCF_2CF_2CF_3$ |
| H | $C(CH_3)_2CH_2SO_2CH_3$ | $CF_2OCH_3$ | Ph-4-O(L-45g) |
| H | $C(CH_3)_2CH_2SO_2CH_3$ | $CF_2OCH_3$ | Ph-3,4-$Cl_2$ |
| H | $C(CH_3)_2CH_2SO_2CH_3$ | $CF_2OCH_3$ | Ph(-3-$OCF_2O$-4-) |
| H | $C(CH_3)_2CH_2SO_2CH_3$ | $CF_2OCH_3$ | Ph(-3-$OCF_2CF_2O$-4-) |
| H | $C(CH_3)_2CH_2SO_2CH_3$ | $CF_2OCH_3$ | (L-45c) $CF_3$ |
| H | $C(CH_3)_2CH_2SO_2CH_3$ | $CF_2OCH_3$ | L-45f |
| H | $C(CH_3)_2CH_2SO_2CH_3$ | $CF_2OCH_3$ | (L-46c) $CF_3$ |
| H | $C(CH_3)_2CH_2SO_2CH_3$ | $CF_2OCH_3$ | L-46d |
| H | $C(CH_3)_2CH_2SO_2CH_3$ | $CH_2SCH_3$ | Ph-4-$OCF_3$ |
| H | $C(CH_3)_2CH_2SO_2CH_3$ | $CH_2SO_2CH_3$ | (L-45c) $CF_3$ |
| H | $C(CH_3)_2CH_2SO_2CH_3$ | $CH_2SCF_3$ | Ph-4-Br |
| H | $C(CH_3)_2CH_2SO_2CH_3$ | $CF_2SCH_3$ | Ph-4-Cl |
| H | $C(CH_3)_2CH_2SO_2CH_3$ | $CF_2SCH_3$ | Ph-4-Br |
| H | $C(CH_3)_2CH_2SO_2CH_3$ | $CF_2SCH_3$ | Ph-4-$CF_3$ |
| H | $C(CH_3)_2CH_2SO_2CH_3$ | $CF_2SCH_3$ | Ph-4-$OCF_3$ |
| H | $C(CH_3)_2CH_2SO_2CH_3$ | $CF_2SCH_3$ | Ph-4-$OCF_2Br$ |
| H | $C(CH_3)_2CH_2SO_2CH_3$ | $CF_2SCH_3$ | Ph-4-$OCF_2CHFCF_3$ |
| H | $C(CH_3)_2CH_2SO_2CH_3$ | $CF_2SCH_3$ | Ph-4-$OCF_2CHFOCF_3$ |
| H | $C(CH_3)_2CH_2SO_2CH_3$ | $CF_2SCH_3$ | Ph-4-$OCF_2CHFOCF_2CF_2CF_3$ |
| H | $C(CH_3)_2CH_2SO_2CH_3$ | $CF_2SCH_3$ | Ph-4-O(L-45g) |
| H | $C(CH_3)_2CH_2SO_2CH_3$ | $CF_2SCH_3$ | Ph-3,4-$Cl_2$ |
| H | $C(CH_3)_2CH_2SO_2CH_3$ | $CF_2SCH_3$ | Ph(-3-$OCF_2O$-4-) |
| H | $C(CH_3)_2CH_2SO_2CH_3$ | $CF_2SCH_3$ | Ph(-3-$OCF_2CF_2O$-4-) |
| H | $C(CH_3)_2CH_2SO_2CH_3$ | $CF_2SCH_3$ | (L-45c) $CF_3$ |
| H | $C(CH_3)_2CH_2SO_2CH_3$ | $CF_2SCH_3$ | L-45f |
| H | $C(CH_3)_2CH_2SO_2CH_3$ | $CF_2SCH_3$ | (L-46c) $CF_3$ |

| | | | |
|---|---|---|---|
| H | $C(CH_3)_2CH_2SO_2CH_3$ | $CF_2SCH_3$ | L-46d |
| H | $C(CH_3)_2CH_2SO_2CH_3$ | $CF_2SEt$ | Ph-4-$CF_3$ |
| H | $C(CH_3)_2CH_2SO_2CH_3$ | $CF_2SPr$-n | Ph-4-$OCF_3$ |
| H | $C(CH_3)_2CH_2SO_2CH_3$ | $CF_2SPr$-i | (L-45c) $CF_3$ |
| H | $C(CH_3)_2CH_2SO_2CH_3$ | $CF_2SCH_2Ph$ | Ph-4-Br |
| H | $C(CH_3)_2CH_2SO_2CH_3$ | $CF_2SPh$ | Ph-4-$CF_3$ |
| H | $C(CH_3)_2CH_2SO_2CH_3$ | $CF_2C(O)OEt$ | Ph-4-$OCF_3$ |
| H | $C(CH_3)_2CH_2SO_2CH_3$ | $CF_2SO_2N(CH_3)_2$ | (L-45c) $CF_3$ |
| H | $C(CH_3)_2CH_2SO_2CH_3$ | C(O)OEt | Ph-4-Br |
| H | $C(CH_3)_2CH_2SO_2CH_3$ | C(O)SEt | Ph-4-$CF_3$ |
| H | $C(CH_3)_2CH_2SEt$ | $CF_3$ | CH=CH(Ph-4-Cl) |
| H | $C(CH_3)_2CH_2SEt$ | $CF_3$ | Ph-4-Cl |
| H | $C(CH_3)_2CH_2SEt$ | $CF_3$ | Ph-4-Br |
| H | $C(CH_3)_2CH_2SEt$ | $CF_3$ | Ph-4-$CF_3$ |
| H | $C(CH_3)_2CH_2SEt$ | $CF_3$ | Ph-4-$OCF_3$ |
| H | $C(CH_3)_2CH_2SEt$ | $CF_3$ | Ph-4-$OCF_2Br$ |
| H | $C(CH_3)_2CH_2SEt$ | $CF_3$ | Ph-4-$OCF_2CHFCF_3$ |
| H | $C(CH_3)_2CH_2SEt$ | $CF_3$ | Ph-4-$OCF_2CHFOCF_3$ |
| H | $C(CH_3)_2CH_2SEt$ | $CF_3$ | Ph-4-$OCF_2CHFOCF_2CF_2CF_3$ |
| H | $C(CH_3)_2CH_2SEt$ | $CF_3$ | Ph-4-O(L-45g) |
| H | $C(CH_3)_2CH_2SEt$ | $CF_3$ | Ph-3,4-$Cl_2$ |
| H | $C(CH_3)_2CH_2SEt$ | $CF_3$ | Ph(-3-$OCF_2O$-4-) |
| H | $C(CH_3)_2CH_2SEt$ | $CF_3$ | Ph(-3-$OCF_2CF_2O$-4-) |
| H | $C(CH_3)_2CH_2SEt$ | $CF_3$ | (L-45c) $CF_3$ |
| H | $C(CH_3)_2CH_2SEt$ | $CF_3$ | L-45f |
| H | $C(CH_3)_2CH_2SEt$ | $CF_3$ | (L-46c) $CF_3$ |
| H | $C(CH_3)_2CH_2SEt$ | $CF_3$ | L-46d |
| H | $C(CH_3)_2CH_2S(O)Et$ | $CF_3$ | CH=CH(Ph-4-Cl) |
| H | $C(CH_3)_2CH_2S(O)Et$ | $CF_3$ | Ph-4-Cl |
| H | $C(CH_3)_2CH_2S(O)Et$ | $CF_3$ | Ph-4-Br |
| H | $C(CH_3)_2CH_2S(O)Et$ | $CF_3$ | Ph-4-$CF_3$ |
| H | $C(CH_3)_2CH_2S(O)Et$ | $CF_3$ | Ph-4-$OCF_3$ |
| H | $C(CH_3)_2CH_2S(O)Et$ | $CF_3$ | Ph-4-$OCF_2Br$ |
| H | $C(CH_3)_2CH_2S(O)Et$ | $CF_3$ | Ph-4-$OCF_2CHFCF_3$ |
| H | $C(CH_3)_2CH_2S(O)Et$ | $CF_3$ | Ph-4-$OCF_2CHFOCF_3$ |
| H | $C(CH_3)_2CH_2S(O)Et$ | $CF_3$ | Ph-4-$OCF_2CHFOCF_2CF_2CF_3$ |
| H | $C(CH_3)_2CH_2S(O)Et$ | $CF_3$ | Ph-4-O(L-45g) |
| H | $C(CH_3)_2CH_2S(O)Et$ | $CF_3$ | Ph-3,4-$Cl_2$ |
| H | $C(CH_3)_2CH_2S(O)Et$ | $CF_3$ | Ph(-3-$OCF_2O$-4-) |
| H | $C(CH_3)_2CH_2S(O)Et$ | $CF_3$ | Ph(-3-$OCF_2CF_2O$-4-) |
| H | $C(CH_3)_2CH_2S(O)Et$ | $CF_3$ | (L-45c) $CF_3$ |

| | | | |
|---|---|---|---|
| H | $C(CH_3)_2CH_2S(O)Et$ | $CF_3$ | L-45f |
| H | $C(CH_3)_2CH_2S(O)Et$ | $CF_3$ | (L-46c) $CF_3$ |
| H | $C(CH_3)_2CH_2S(O)Et$ | $CF_3$ | L-46d |
| H | $C(CH_3)_2CH_2SO_2Et$ | $CF_3$ | CH=CH(Ph-4-Cl) |
| H | $C(CH_3)_2CH_2SO_2Et$ | $CF_3$ | Ph-4-Cl |
| H | $C(CH_3)_2CH_2SO_2Et$ | $CF_3$ | Ph-4-Br |
| H | $C(CH_3)_2CH_2SO_2Et$ | $CF_3$ | Ph-4-$CF_3$ |
| H | $C(CH_3)_2CH_2SO_2Et$ | $CF_3$ | Ph-4-$OCF_3$ |
| H | $C(CH_3)_2CH_2SO_2Et$ | $CF_3$ | Ph-4-$OCF_2Br$ |
| H | $C(CH_3)_2CH_2SO_2Et$ | $CF_3$ | Ph-4-$OCF_2CHFCF_3$ |
| H | $C(CH_3)_2CH_2SO_2Et$ | $CF_3$ | Ph-4-$OCF_2CHFOCF_3$ |
| H | $C(CH_3)_2CH_2SO_2Et$ | $CF_3$ | Ph-4-$OCF_2CHFOCF_2CF_2CF_3$ |
| H | $C(CH_3)_2CH_2SO_2Et$ | $CF_3$ | Ph-4-O(L-45g) |
| H | $C(CH_3)_2CH_2SO_2Et$ | $CF_3$ | Ph-3, 4-$Cl_2$ |
| H | $C(CH_3)_2CH_2SO_2Et$ | $CF_3$ | Ph(-3-$OCF_2O$-4-) |
| H | $C(CH_3)_2CH_2SO_2Et$ | $CF_3$ | Ph(-3-$OCF_2CF_2O$-4-) |
| H | $C(CH_3)_2CH_2SO_2Et$ | $CF_3$ | (L-45c) $CF_3$ |
| H | $C(CH_3)_2CH_2SO_2Et$ | $CF_3$ | L-45f |
| H | $C(CH_3)_2CH_2SO_2Et$ | $CF_3$ | (L-46c) $CF_3$ |
| H | $C(CH_3)_2CH_2SO_2Et$ | $CF_3$ | L-46d |
| H | $C(CH_3)_2CH_2SPr$-n | $CF_3$ | Ph-4-Br |
| H | $C(CH_3)_2CH_2SPr$-n | $CF_3$ | Ph-4-$CF_3$ |
| H | $C(CH_3)_2CH_2SPr$-n | $CF_3$ | Ph-4-$OCF_3$ |
| H | $C(CH_3)_2CH_2SPr$-n | $CF_3$ | (L-45c) $CF_3$ |
| H | $C(CH_3)_2CH_2S(O)Pr$-n | $CF_3$ | Ph-4-$OCF_3$ |
| H | $C(CH_3)_2CH_2SO_2Pr$-n | $CF_3$ | Ph-4-Br |
| H | $C(CH_3)_2CH_2SO_2Pr$-n | $CF_3$ | Ph-4-$CF_3$ |
| H | $C(CH_3)_2CH_2SO_2Pr$-n | $CF_3$ | Ph-4-$OCF_3$ |
| H | $C(CH_3)_2CH_2SO_2Pr$-n | $CF_3$ | (L-45c) $CF_3$ |
| H | $C(CH_3)_2CH_2SPr$-i | $CF_3$ | Ph-4-Br |
| H | $C(CH_3)_2CH_2SPr$-i | $CF_3$ | Ph-4-$CF_3$ |
| H | $C(CH_3)_2CH_2SPr$-i | $CF_3$ | Ph-4-$OCF_3$ |
| H | $C(CH_3)_2CH_2SPr$-i | $CF_3$ | (L-45c) $CF_3$ |
| H | $C(CH_3)_2CH_2S(O)Pr$-i | $CF_3$ | (L-45c) $CF_3$ |
| H | $C(CH_3)_2CH_2SO_2Pr$-i | $CF_3$ | Ph-4-Br |
| H | $C(CH_3)_2CH_2SBu$-t | $CF_3$ | Ph-4-$CF_3$ |
| H | $C(CH_3)_2CH_2S(O)Bu$-t | $CF_3$ | Ph-4-$OCF_3$ |
| H | $C(CH_3)_2CH_2SCH_2$(Ph-4-Cl) | $CF_3$ | (L-45c) $CF_3$ |
| H | $C(CH_3)_2CH_2SO_2NH_2$ | $CF_3$ | Ph-4-Br |
| H | $C(CH_3)_2CH_2SO_2NHCH_3$ | $CF_3$ | Ph-4-$CF_3$ |
| H | $C(CH_3)_2CH_2SO_2NHEt$ | $CF_3$ | Ph-4-Br |

| | | | |
|---|---|---|---|
| H | $C(CH_3)_2CH_2SO_2NHEt$ | $CF_3$ | Ph-4-$CF_3$ |
| H | $C(CH_3)_2CH_2SO_2NHEt$ | $CF_3$ | Ph-4-$OCF_3$ |
| H | $C(CH_3)_2CH_2SO_2NHEt$ | $CF_3$ | (L-45c) $CF_3$ |
| H | $C(CH_3)_2CH_2SO_2NHCH_2CH_2OCH_3$ | $CF_3$ | Ph-4-$OCF_3$ |
| H | $C(CH_3)_2CH_2SO_2NHCH_2CH_2OC(O)CH_3$ | $CF_3$ | (L-45c) $CF_3$ |
| H | $C(CH_3)_2CH_2SO_2NHCH_2CH_2SCH_3$ | $CF_3$ | Ph-4-Br |
| H | $C(CH_3)_2CH_2SO_2NHCH_2CH_2S(O)CH_3$ | $CF_3$ | Ph-4-$CF_3$ |
| H | $C(CH_3)_2CH_2SO_2NHCH_2CH_2SO_2CH_3$ | $CF_3$ | Ph-4-$OCF_3$ |
| H | $C(CH_3)_2CH_2SO_2NHCH_2CH_2SEt$ | $CF_3$ | (L-45c) $CF_3$ |
| H | $C(CH_3)_2CH_2SO_2NHCH_2CH_2S(O)Et$ | $CF_3$ | Ph-4-Br |
| H | $C(CH_3)_2CH_2SO_2NHCH_2CH_2SO_2Et$ | $CF_3$ | Ph-4-$CF_3$ |
| H | $C(CH_3)_2CH_2SO_2NHCH_2CH_2SPh$ | $CF_3$ | Ph-4-$OCF_3$ |
| H | $C(CH_3)_2CH_2SO_2NHCH_2CH_2S(O)Ph$ | $CF_3$ | (L-45c) $CF_3$ |
| H | $C(CH_3)_2CH_2SO_2NHCH_2CH_2SO_2Ph$ | $CF_3$ | Ph-4-Br |
| H | $C(CH_3)_2CH_2SO_2NHCH_2Ph$ | $CF_3$ | Ph-4-$CF_3$ |
| H | $C(CH_3)_2CH_2SO_2N(CH_3)_2$ | $CF_3$ | Ph-4-Br |
| H | $C(CH_3)_2CH_2SO_2N(CH_3)_2$ | $CF_3$ | Ph-4-$CF_3$ |
| H | $C(CH_3)_2CH_2SO_2N(CH_3)_2$ | $CF_3$ | Ph-4-$OCF_3$ |
| H | $C(CH_3)_2CH_2SO_2N(CH_3)_2$ | $CF_3$ | (L-45c) $CF_3$ |
| H | $C(CH_3)_2CH_2SO_2N(Et)_2$ | $CF_3$ | $CF_2O$(Ph-4-Cl) |
| H | $C(CH_3)_2CH_2SO_2N(Et)_2$ | $CF_3$ | CH=CH(Ph-4-Cl) |
| H | $C(CH_3)_2CH_2SO_2N(Et)_2$ | $CF_3$ | CH=CH(Ph-4-Br) |
| H | $C(CH_3)_2CH_2SO_2N(Et)_2$ | $CF_3$ | CH=CH(Ph-4-$CF_3$) |
| H | $C(CH_3)_2CH_2SO_2N(Et)_2$ | $CF_3$ | CH=CH(Ph-4-$OCF_3$) |
| H | $C(CH_3)_2CH_2SO_2N(Et)_2$ | $CF_3$ | CH=CH(Ph-3, 4-$Cl_2$) |
| H | $C(CH_3)_2CH_2SO_2N(Et)_2$ | $CF_3$ | Ph-4-F |
| H | $C(CH_3)_2CH_2SO_2N(Et)_2$ | $CF_3$ | Ph-4-Cl |
| H | $C(CH_3)_2CH_2SO_2N(Et)_2$ | $CF_3$ | Ph-4-Br |
| H | $C(CH_3)_2CH_2SO_2N(Et)_2$ | $CF_3$ | Ph-4-$CF_3$ |
| H | $C(CH_3)_2CH_2SO_2N(Et)_2$ | $CF_3$ | Ph-4-$OCF_3$ |
| H | $C(CH_3)_2CH_2SO_2N(Et)_2$ | $CF_3$ | Ph-4-$OCF_2Br$ |
| H | $C(CH_3)_2CH_2SO_2N(Et)_2$ | $CF_3$ | Ph-4-$OCF_2CHF_2$ |
| H | $C(CH_3)_2CH_2SO_2N(Et)_2$ | $CF_3$ | Ph-4-$OCF_2CHFCF_3$ |
| H | $C(CH_3)_2CH_2SO_2N(Et)_2$ | $CF_3$ | Ph-4-$OCF_2CHFOCF_3$ |
| H | $C(CH_3)_2CH_2SO_2N(Et)_2$ | $CF_3$ | Ph-4-$OCF_2CHFOCF_2CF_2CF_3$ |
| H | $C(CH_3)_2CH_2SO_2N(Et)_2$ | $CF_3$ | Ph-4-O(L-45g) |
| H | $C(CH_3)_2CH_2SO_2N(Et)_2$ | $CF_3$ | Ph-3, 4-$F_2$ |
| H | $C(CH_3)_2CH_2SO_2N(Et)_2$ | $CF_3$ | Ph-3-F-4-Cl |
| H | $C(CH_3)_2CH_2SO_2N(Et)_2$ | $CF_3$ | Ph-3, 4-$Cl_2$ |
| H | $C(CH_3)_2CH_2SO_2N(Et)_2$ | $CF_3$ | Ph(-3-$OCF_2O$-4-) |
| H | $C(CH_3)_2CH_2SO_2N(Et)_2$ | $CF_3$ | Ph(-3-$OCF_2CF_2O$-4-) |

| | | | |
|---|---|---|---|
| H | $C(CH_3)_2CH_2SO_2N(Et)_2$ | $CF_3$ | (L-45c) $CF_3$ |
| H | $C(CH_3)_2CH_2SO_2N(Et)_2$ | $CF_3$ | L-45f |
| H | $C(CH_3)_2CH_2SO_2N(Et)_2$ | $CF_3$ | (L-46c) $CF_3$ |
| H | $C(CH_3)_2CH_2SO_2N(Et)_2$ | $CF_3$ | L-46d |
| H | $C(CH_3)_2CH_2SO_2$ (T-19) | $CF_3$ | Ph-4-$OCF_3$ |
| H | $C(CH_3)_2CH_2SO_2$ (T-21) | $CF_3$ | (L-46c) $CF_3$ |
| H | $C(CH_3)_2CH_2SPh$ | $CF_3$ | Ph-4-Br |
| H | $C(CH_3)_2CH_2S(O)Ph$ | $CF_3$ | Ph-4-$CF_3$ |
| H | $C(CH_3)_2CH_2SO_2Ph$ | $CF_3$ | Ph-4-$OCF_3$ |
| H | $C(CH_3)_2CH_2S$ (L-45a) | $CF_3$ | (L-45c) $CF_3$ |
| H | $C(CH_3)_2CH_2S(O)$ (L-45a) | $CF_3$ | Ph-4-Br |
| H | $C(CH_3)_2CH_2SO_2$ (L-45a) | $CF_3$ | Ph-4-$CF_3$ |
| H | $CH_2CH_2CH_2SCH_3$ | $CF_3$ | Ph-4-$OCF_3$ |
| H | $CH(CH_3)CH_2CH_2SCH_3$ | $CF_3$ | Ph-4-Br |
| H | $CH(CH_3)CH_2CH_2SCH_3$ | $CF_3$ | Ph-4-$CF_3$ |
| H | $CH(CH_3)CH_2CH_2SCH_3$ | $CF_3$ | Ph-4-$OCF_3$ |
| H | $CH(CH_3)CH_2CH_2SCH_3$ | $CF_3$ | (L-45c) $CF_3$ |
| H | $CH(CH_3)CH_2CH_2S(O)CH_3$ | $CF_3$ | Ph-4-Br |
| H | $CH(CH_3)CH_2CH_2S(O)CH_3$ | $CF_3$ | Ph-4-$CF_3$ |
| H | $CH(CH_3)CH_2CH_2S(O)CH_3$ | $CF_3$ | Ph-4-$OCF_3$ |
| H | $CH(CH_3)CH_2CH_2S(O)CH_3$ | $CF_3$ | (L-45c) $CF_3$ |
| H | $CH(CH_3)CH_2CH_2SO_2CH_3$ | $CF_3$ | Ph-4-Br |
| H | $CH(CH_3)CH_2CH_2SO_2CH_3$ | $CF_3$ | Ph-4-$CF_3$ |
| H | $CH(CH_3)CH_2CH_2SO_2CH_3$ | $CF_3$ | Ph-4-$OCF_3$ |
| H | $CH(CH_3)CH_2CH_2SO_2CH_3$ | $CF_3$ | (L-45c) $CF_3$ |
| H | $CH(CH_3)CH_2CH_2SEt$ | $CF_3$ | Ph-4-Br |
| H | $CH(CH_3)CH_2CH_2SEt$ | $CF_3$ | Ph-4-$CF_3$ |
| H | $CH(CH_3)CH_2CH_2SEt$ | $CF_3$ | Ph-4-$OCF_3$ |
| H | $CH(CH_3)CH_2CH_2SEt$ | $CF_3$ | (L-45c) $CF_3$ |
| H | $CH(CH_3)CH_2CH_2S(O)Et$ | $CF_3$ | Ph-4-Br |
| H | $CH(CH_3)CH_2CH_2S(O)Et$ | $CF_3$ | Ph-4-$CF_3$ |
| H | $CH(CH_3)CH_2CH_2S(O)Et$ | $CF_3$ | Ph-4-$OCF_3$ |
| H | $CH(CH_3)CH_2CH_2S(O)Et$ | $CF_3$ | (L-45c) $CF_3$ |
| H | $CH(CH_3)CH_2CH_2SO_2Et$ | $CF_3$ | Ph-4-Br |
| H | $CH(CH_3)CH_2CH_2SO_2Et$ | $CF_3$ | Ph-4-$CF_3$ |
| H | $CH(CH_3)CH_2CH_2SO_2Et$ | $CF_3$ | Ph-4-$OCF_3$ |
| H | $CH(CH_3)CH_2CH_2SO_2Et$ | $CF_3$ | (L-45c) $CF_3$ |
| H | $CH(Et)CH_2CH_2SCH_3$ | $CF_3$ | (L-45c) $CF_3$ |
| H | $CH(CH_2OH)CH_2CH_2SCH_3$ | $CF_3$ | Ph-4-Br |
| H | $C(CH_3)_2CH_2CH_2SCH_3$ | $CF_3$ | Ph-4-$CF_3$ |
| H | $C(CH_3)_2CH_2CH_2SEt$ | $CF_3$ | Ph-4-$OCF_3$ |

| | | | |
|---|---|---|---|
| H | $CH(CH_3)(CH_2)_3SCH_3$ | $CF_3$ | Ph-4-Br |
| H | $CH(CH_3)(CH_2)_3SCH_3$ | $CF_3$ | Ph-4-$CF_3$ |
| H | $CH(CH_3)(CH_2)_3SCH_3$ | $CF_3$ | Ph-4-$OCF_3$ |
| H | $CH(CH_3)(CH_2)_3SCH_3$ | $CF_3$ | (L-45c)$CF_3$ |
| H | $CH(CH_3)(CH_2)_3S(O)CH_3$ | $CF_3$ | (L-45c)$CF_3$ |
| H | $CH(CH_3)(CH_2)_3SO_2CH_3$ | $CF_3$ | Ph-4-Br |
| H | $CH(CH_3)(CH_2)_3SO_2CH_3$ | $CF_3$ | Ph-4-$CF_3$ |
| H | $CH(CH_3)(CH_2)_3SO_2CH_3$ | $CF_3$ | Ph-4-$OCF_3$ |
| H | $CH(CH_3)(CH_2)_3SO_2CH_3$ | $CF_3$ | (L-45c)$CF_3$ |
| H | $CH(CH_3)(CH_2)_3SEt$ | $CF_3$ | Ph-4-Br |
| H | $CH(CH_3)(CH_2)_3SEt$ | $CF_3$ | Ph-4-$CF_3$ |
| H | $CH(CH_3)(CH_2)_3SEt$ | $CF_3$ | Ph-4-$OCF_3$ |
| H | $CH(CH_3)(CH_2)_3SEt$ | $CF_3$ | (L-45c)$CF_3$ |
| H | $CH(CH_3)(CH_2)_3S(O)Et$ | $CF_3$ | Ph-4-Br |
| H | $CH(CH_3)(CH_2)_3SO_2Et$ | $CF_3$ | Ph-4-Br |
| H | $CH(CH_3)(CH_2)_3SO_2Et$ | $CF_3$ | Ph-4-$CF_3$ |
| H | $CH(CH_3)(CH_2)_3SO_2Et$ | $CF_3$ | Ph-4-$OCF_3$ |
| H | $CH(CH_3)(CH_2)_3SO_2Et$ | $CF_3$ | (L-45c)$CF_3$ |
| H | $C(CH_3)_2(CH_2)_3SCH_3$ | $CF_3$ | Ph-4-$CF_3$ |
| H | $C(CH_3)_2(CH_2)_3S(O)CH_3$ | $CF_3$ | Ph-4-$OCF_3$ |
| H | $CH(CH_3)CH_2SO_2CH_3$ | $CF_3$ | (L-45c)$CF_3$ |
| H | $C(CH_3)_2(CH_2)_3SEt$ | $CF_3$ | Ph-4-Br |
| H | $C(CH_3)_2(CH_2)_3S(O)Et$ | $CF_3$ | Ph-4-$CF_3$ |
| H | $C(CH_3)_2(CH_2)_3SO_2Et$ | $CF_3$ | Ph-4-$OCF_3$ |
| H | $C(CH_3)_2(CH_2)_3SO_2NHCH_3$ | $CF_3$ | (L-45c)$CF_3$ |
| H | $C(CH_3)_2(CH_2)_3SO_2NHEt$ | $CF_3$ | Ph-4-Br |
| H | $C(CH_3)_2(CH_2)_3SO_2N(CH_3)_2$ | $CF_3$ | Ph-4-$CF_3$ |
| H | $C(CH_3)_2(CH_2)_3SO_2N(Et)_2$ | $CF_3$ | Ph-4-$OCF_3$ |
| H | $C(CH_3)_2CH_2CH_2CH(CH_3)SCH_3$ | $CF_3$ | (L-45c)$CF_3$ |
| H | $CH(CH_3)(CH_2)_4SCH_3$ | $CF_3$ | Ph-4-Br |
| H | $CH(CH_3)(CH_2)_4SO_2CH_3$ | $CF_3$ | Ph-4-$CF_3$ |
| H | $CH(CH_3)(CH_2)_4SEt$ | $CF_3$ | Ph-4-$OCF_3$ |
| H | $CH(CH_3)(CH_2)_4S(O)Et$ | $CF_3$ | (L-45c)$CF_3$ |
| H | $CH(CH_3)(CH_2)_4SO_2Et$ | $CF_3$ | Ph-4-Br |
| H | T-6 | $CF_3$ | Ph-4-$CF_3$ |
| H | T-7 | $CF_3$ | Ph-4-$OCF_3$ |
| H | T-8 | $CF_3$ | (L-45c)$CF_3$ |
| H | T-9 | $CF_3$ | Ph-4-Br |
| H | T-11 | $CF_3$ | Ph-4-$CF_3$ |
| H | T-12 | $CF_3$ | Ph-4-$OCF_3$ |
| H | T-13 | $CF_3$ | (L-45c)$CF_3$ |

| | | | |
|---|---|---|---|
| H | T-14 | $CF_3$ | Ph-4-Br |
| H | T-15 | $CF_3$ | Ph-4-$CF_3$ |
| H | M-8a | $CF_3$ | Ph-4-$OCF_3$ |
| H | M-9a | $CF_3$ | (L-45c) $CF_3$ |
| H | M-9b | $CF_3$ | Ph-4-Br |
| H | M-9c | $CF_3$ | Ph-4-$CF_3$ |
| H | M-19a | $CF_3$ | Ph-4-$OCF_3$ |
| H | $CH_2NHC(O)OCH_3$ | $CF_3$ | (L-45c) $CF_3$ |
| H | $CH_2NHC(O)OEt$ | $CF_3$ | Ph-4-Br |
| H | $CH_2NHC(O)OPr$-i | $CF_3$ | Ph-4-Br |
| H | $CH_2NHC(O)OPr$-i | $CF_3$ | Ph-4-$CF_3$ |
| H | $CH_2NHC(O)OPr$-i | $CF_3$ | Ph-4-$OCF_3$ |
| H | $CH_2NHC(O)OPr$-i | $CF_3$ | (L-45c) $CF_3$ |
| H | $CH_2CH_2NHC(O)CH_3$ | $CF_3$ | Ph-4-$CF_3$ |
| H | $CH_2CH_2NHC(O)N(CH_3)_2$ | $CF_3$ | Ph-4-$OCF_3$ |
| H | $CH_2CH_2NHC(O)Ph$ | $CF_3$ | (L-45c) $CF_3$ |
| H | $CH_2CH_2N(CH_3)OCH_3$ | $CF_3$ | Ph-4-Br |
| H | $CH(CH_3)CH_2NHC(O)CH_3$ | $CF_3$ | Ph-4-Br |
| H | $CH(CH_3)CH_2NHC(O)CH_3$ | $CF_3$ | Ph-4-$CF_3$ |
| H | $CH(CH_3)CH_2NHC(O)CH_3$ | $CF_3$ | Ph-4-$OCF_3$ |
| H | $CH(CH_3)CH_2NHC(O)CH_3$ | $CF_3$ | (L-45c) $CF_3$ |
| H | $CH(CH_3)CH_2NHC(O)Et$ | $CF_3$ | Ph-4-$CF_3$ |
| H | $CH(CH_3)CH_2NHC(O)OCH_3$ | $CF_3$ | Ph-4-$OCF_3$ |
| H | $CH(CH_3)CH_2NHC(O)N(CH_3)_2$ | $CF_3$ | (L-45c) $CF_3$ |
| H | $CH(CH_3)CH_2NHC(S)NHEt$ | $CF_3$ | Ph-4-Br |
| H | $CH(CH_3)CH_2NHSO_2CH_3$ | $CF_3$ | Ph-4-$CF_3$ |
| H | $CH(CH_3)CH_2NHSO_2Et$ | $CF_3$ | Ph-4-$OCF_3$ |
| H | $CH(CH_3)CH_2NHSO_2Ph$ | $CF_3$ | (L-45c) $CF_3$ |
| H | $CH(CH_3)CH_2NHSO_2N(CH_3)_2$ | $CF_3$ | Ph-4-Br |
| H | $CH(CH_3)CH_2NHP(S)(OCH_3)_2$ | $CF_3$ | Ph-4-$CF_3$ |
| H | $CH(CH_3)CH_2NHP(S)(OEt)_2$ | $CF_3$ | Ph-4-$OCF_3$ |
| H | $CH(CH_3)CH_2N(CH_3)_2$ | $CF_3$ | (L-45c) $CF_3$ |
| H | $CH(CH_3)CH_2N(CH_3)SO_2CH_3$ | $CH_3$ | Ph-4-Br |
| H | $CH(CH_3)CH_2N(CH_3)SO_2CH_3$ | $CH_3$ | Ph-4-$CF_3$ |
| H | $CH(CH_3)CH_2N(CH_3)SO_2CH_3$ | $CH_3$ | Ph-4-$OCF_3$ |
| H | $CH(CH_3)CH_2N(CH_3)SO_2CH_3$ | $CH_3$ | (L-45c) Cl |
| H | $CH(CH_3)CH_2N(CH_3)SO_2CH_3$ | $CH_3$ | (L-45c) Br |
| H | $CH(CH_3)CH_2N(CH_3)SO_2CH_3$ | $CH_3$ | (L-45c) $CF_3$ |
| H | $CH(CH_3)CH_2N(CH_3)SO_2CH_3$ | Et | Ph-4-Br |
| H | $CH(CH_3)CH_2N(CH_3)SO_2CH_3$ | n-Pr | Ph-4-$CF_3$ |
| H | $CH(CH_3)CH_2N(CH_3)SO_2CH_3$ | i-Pr | Ph-4-$OCF_3$ |

| | | | |
|---|---|---|---|
| H | $CH(CH_3)CH_2N(CH_3)SO_2CH_3$ | c-Pr | (L-45c) $CF_3$ |
| H | $CH(CH_3)CH_2N(CH_3)SO_2CH_3$ | $CHF_2$ | Ph-4-Br |
| H | $CH(CH_3)CH_2N(CH_3)SO_2CH_3$ | $CF_3$ | $CF_2O$ (Ph-4-Cl) |
| H | $CH(CH_3)CH_2N(CH_3)SO_2CH_3$ | $CF_3$ | CH=CH (Ph-4-Cl) |
| H | $CH(CH_3)CH_2N(CH_3)SO_2CH_3$ | $CF_3$ | CH=CH (Ph-4-Br) |
| H | $CH(CH_3)CH_2N(CH_3)SO_2CH_3$ | $CF_3$ | CH=CH (Ph-4-$CF_3$) |
| H | $CH(CH_3)CH_2N(CH_3)SO_2CH_3$ | $CF_3$ | CH=CH (Ph-4-$OCF_3$) |
| H | $CH(CH_3)CH_2N(CH_3)SO_2CH_3$ | $CF_3$ | CH=CH (Ph-3, 4-$Cl_2$) |
| H | $CH(CH_3)CH_2N(CH_3)SO_2CH_3$ | $CF_3$ | Ph-4-F |
| H | $CH(CH_3)CH_2N(CH_3)SO_2CH_3$ | $CF_3$ | Ph-4-Cl |
| H | $CH(CH_3)CH_2N(CH_3)SO_2CH_3$ | $CF_3$ | Ph-4-Br |
| H | $CH(CH_3)CH_2N(CH_3)SO_2CH_3$ | $CF_3$ | Ph-4-I |
| H | $CH(CH_3)CH_2N(CH_3)SO_2CH_3$ | $CF_3$ | Ph-4-$CF_3$ |
| H | $CH(CH_3)CH_2N(CH_3)SO_2CH_3$ | $CF_3$ | Ph-4-$OCHF_2$ |
| H | $CH(CH_3)CH_2N(CH_3)SO_2CH_3$ | $CF_3$ | Ph-4-$OCF_3$ |
| H | $CH(CH_3)CH_2N(CH_3)SO_2CH_3$ | $CF_3$ | Ph-4-$OCF_2Br$ |
| H | $CH(CH_3)CH_2N(CH_3)SO_2CH_3$ | $CF_3$ | Ph-4-$OCF_2CHF_2$ |
| H | $CH(CH_3)CH_2N(CH_3)SO_2CH_3$ | $CF_3$ | Ph-4-$OCF_2CHFCl$ |
| H | $CH(CH_3)CH_2N(CH_3)SO_2CH_3$ | $CF_3$ | Ph-4-$OCF_2CHFCF_3$ |
| H | $CH(CH_3)CH_2N(CH_3)SO_2CH_3$ | $CF_3$ | Ph-4-$OCF_2CHFOCF_3$ |
| H | $CH(CH_3)CH_2N(CH_3)SO_2CH_3$ | $CF_3$ | Ph-4-$OCF_2CHFOCF_2CF_2CF_3$ |
| H | $CH(CH_3)CH_2N(CH_3)SO_2CH_3$ | $CF_3$ | Ph-4-O (L-45g) |
| H | $CH(CH_3)CH_2N(CH_3)SO_2CH_3$ | $CF_3$ | Ph-3, 4-$F_2$ |
| H | $CH(CH_3)CH_2N(CH_3)SO_2CH_3$ | $CF_3$ | Ph-3-F-4-Cl |
| H | $CH(CH_3)CH_2N(CH_3)SO_2CH_3$ | $CF_3$ | Ph-3, 4-$Cl_2$ |
| H | $CH(CH_3)CH_2N(CH_3)SO_2CH_3$ | $CF_3$ | Ph (-3-$OCF_2O$-4-) |
| H | $CH(CH_3)CH_2N(CH_3)SO_2CH_3$ | $CF_3$ | Ph (-3-$OCF_2CF_2O$-4-) |
| H | $CH(CH_3)CH_2N(CH_3)SO_2CH_3$ | $CF_3$ | (L-45c) Cl |
| H | $CH(CH_3)CH_2N(CH_3)SO_2CH_3$ | $CF_3$ | (L-45c) Br |
| H | $CH(CH_3)CH_2N(CH_3)SO_2CH_3$ | $CF_3$ | (L-45c) $CF_3$ |
| H | $CH(CH_3)CH_2N(CH_3)SO_2CH_3$ | $CF_3$ | L-45e |
| H | $CH(CH_3)CH_2N(CH_3)SO_2CH_3$ | $CF_3$ | L-45f |
| H | $CH(CH_3)CH_2N(CH_3)SO_2CH_3$ | $CF_3$ | (L-46c) Cl |
| H | $CH(CH_3)CH_2N(CH_3)SO_2CH_3$ | $CF_3$ | (L-46c) Br |
| H | $CH(CH_3)CH_2N(CH_3)SO_2CH_3$ | $CF_3$ | (L-46c) $CF_3$ |
| H | $CH(CH_3)CH_2N(CH_3)SO_2CH_3$ | $CF_3$ | L-46d |
| H | $CH(CH_3)CH_2N(CH_3)SO_2CH_3$ | $CF_2Cl$ | Ph-4-$CF_3$ |
| H | $CH(CH_3)CH_2N(CH_3)SO_2CH_3$ | $CF_2Br$ | Ph-4-$OCF_3$ |
| H | $CH(CH_3)CH_2N(CH_3)SO_2CH_3$ | $CF_2CF_3$ | (L-45c) $CF_3$ |
| H | $CH(CH_3)CH_2N(CH_3)SO_2CH_3$ | $CF_2CF_2CF_3$ | Ph-4-Br |
| H | $CH(CH_3)CH_2N(CH_3)SO_2CH_3$ | $CF_2OCH_3$ | Ph-4-$CF_3$ |

| | | | |
|---|---|---|---|
| H | $CH(CH_3)CH_2N(CH_3)SO_2CH_3$ | $CF_2SCH_3$ | Ph-4-$OCF_3$ |
| H | $C(CH_3)_2CH_2NHC(O)CH_3$ | $CF_3$ | (L-45c) $CF_3$ |
| H | $C(CH_3)_2CH_2NHC(O)Et$ | $CF_3$ | Ph-4-Br |
| H | $C(CH_3)_2CH_2NHC(O)Pr$-c | $CF_3$ | Ph-4-$CF_3$ |
| H | $C(CH_3)_2CH_2NHC(O)Bu$-t | $CF_3$ | Ph-4-$OCF_3$ |
| H | $C(CH_3)_2CH_2NHC(O)CF_3$ | $CF_3$ | (L-45c) $CF_3$ |
| H | $C(CH_3)_2CH_2NHC(O)Ph$ | $CF_3$ | Ph-4-Br |
| H | $C(CH_3)_2CH_2NHC(O)OCH_3$ | $CF_3$ | $CF_2O$(Ph-4-Cl) |
| H | $C(CH_3)_2CH_2NHC(O)OCH_3$ | $CF_3$ | CH=CH(Ph-4-Cl) |
| H | $C(CH_3)_2CH_2NHC(O)OCH_3$ | $CF_3$ | CH=CH(Ph-4-Br) |
| H | $C(CH_3)_2CH_2NHC(O)OCH_3$ | $CF_3$ | CH=CH(Ph-4-$CF_3$) |
| H | $C(CH_3)_2CH_2NHC(O)OCH_3$ | $CF_3$ | CH=CH(Ph-4-$OCF_3$) |
| H | $C(CH_3)_2CH_2NHC(O)OCH_3$ | $CF_3$ | CH=CH(Ph-3,4-$Cl_2$) |
| H | $C(CH_3)_2CH_2NHC(O)OCH_3$ | $CF_3$ | Ph-4-F |
| H | $C(CH_3)_2CH_2NHC(O)OCH_3$ | $CF_3$ | Ph-4-Cl |
| H | $C(CH_3)_2CH_2NHC(O)OCH_3$ | $CF_3$ | Ph-4-Br |
| H | $C(CH_3)_2CH_2NHC(O)OCH_3$ | $CF_3$ | Ph-4-$CF_3$ |
| H | $C(CH_3)_2CH_2NHC(O)OCH_3$ | $CF_3$ | Ph-4-$OCF_3$ |
| H | $C(CH_3)_2CH_2NHC(O)OCH_3$ | $CF_3$ | Ph-4-$OCF_2Br$ |
| H | $C(CH_3)_2CH_2NHC(O)OCH_3$ | $CF_3$ | Ph-4-$OCF_2CHF_2$ |
| H | $C(CH_3)_2CH_2NHC(O)OCH_3$ | $CF_3$ | Ph-4-$OCF_2CHFCF_3$ |
| H | $C(CH_3)_2CH_2NHC(O)OCH_3$ | $CF_3$ | Ph-4-$OCF_2CHFOCF_3$ |
| H | $C(CH_3)_2CH_2NHC(O)OCH_3$ | $CF_3$ | Ph-4-$OCF_2CHFOCF_2CF_2CF_3$ |
| H | $C(CH_3)_2CH_2NHC(O)OCH_3$ | $CF_3$ | Ph-4-O(L-45g) |
| H | $C(CH_3)_2CH_2NHC(O)OCH_3$ | $CF_3$ | Ph-3,4-$F_2$ |
| H | $C(CH_3)_2CH_2NHC(O)OCH_3$ | $CF_3$ | Ph-3-F-4-Cl |
| H | $C(CH_3)_2CH_2NHC(O)OCH_3$ | $CF_3$ | Ph-3,4-$Cl_2$ |
| H | $C(CH_3)_2CH_2NHC(O)OCH_3$ | $CF_3$ | Ph(-3-$OCF_2O$-4-) |
| H | $C(CH_3)_2CH_2NHC(O)OCH_3$ | $CF_3$ | Ph(-3-$OCF_2CF_2O$-4-) |
| H | $C(CH_3)_2CH_2NHC(O)OCH_3$ | $CF_3$ | (L-45c) $CF_3$ |
| H | $C(CH_3)_2CH_2NHC(O)OCH_3$ | $CF_3$ | L-45f |
| H | $C(CH_3)_2CH_2NHC(O)OCH_3$ | $CF_3$ | (L-46c) $CF_3$ |
| H | $C(CH_3)_2CH_2NHC(O)OCH_3$ | $CF_3$ | L-46d |
| H | $C(CH_3)_2CH_2NHC(O)OEt$ | $CF_3$ | Ph-4-$CF_3$ |
| H | $C(CH_3)_2CH_2NHC(O)OPr$-n | $CF_3$ | Ph-4-$OCF_3$ |
| H | $CH_2CH_2CH_2NHC(O)OCH_3$ | $CF_3$ | (L-45c) $CF_3$ |
| H | $CH_2CH_2CH_2NHC(O)OBu$-t | $CF_3$ | Ph-4-Br |
| H | $CH_2CH_2CH_2N(CH_3)_2$ | $CF_3$ | Ph-4-$CF_3$ |
| H | $CH_2CH_2CH_2N(CH_3)OCH_3$ | $CF_3$ | Ph-4-$OCF_3$ |
| H | (M-22a) CHO | $CF_3$ | (L-45c) $CF_3$ |
| H | (M-22a) $C(O)CH_3$ | $CF_3$ | Ph-4-Br |

| | | | |
|---|---|---|---|
| H | (M-22a) C (O) $OCH_3$ | $CF_3$ | Ph-4-$CF_3$ |
| H | (M-22a) C (O) OEt | $CF_3$ | Ph-4-$OCF_3$ |
| H | (M-22a) C (O) $SCH_3$ | $CF_3$ | (L-45c) $CF_3$ |
| H | $CH_2Si(CH_3)_3$ | $CF_3$ | Ph-4-Br |
| H | $C(CH_3)_2CHO$ | $CF_3$ | Ph-4-$CF_3$ |
| H | $CH(CH_3)C(O)CH_3$ | $CF_3$ | Ph-4-$OCF_3$ |
| H | $CH(CHO)CH_2SO_2CH_3$ | $CF_3$ | (L-45c) $CF_3$ |
| H | $C(CH_3)(CHO)CH_2SO_2CH_3$ | $CF_3$ | Ph-4-Br |
| H | $C(CH_3)(CHO)CH_2SO_2Et$ | $CF_3$ | Ph-4-$CF_3$ |
| H | $CH_2CH=NOCH_3$ | $CF_3$ | Ph-4-$OCF_3$ |
| H | $CH_2C(Ph)=NOCH_3$ | $CF_3$ | (L-45c) $CF_3$ |
| H | $CH(CH_3)CH=NOCH_3$ | $CH_3$ | Ph-4-Br |
| H | $CH(CH_3)CH=NOCH_3$ | $CH_3$ | Ph-4-$CF_3$ |
| H | $CH(CH_3)CH=NOCH_3$ | $CH_3$ | Ph-4-$OCF_3$ |
| H | $CH(CH_3)CH=NOCH_3$ | $CH_3$ | (L-45c) Cl |
| H | $CH(CH_3)CH=NOCH_3$ | $CH_3$ | (L-45c) Br |
| H | $CH(CH_3)CH=NOCH_3$ | $CH_3$ | (L-45c) $CF_3$ |
| H | $CH(CH_3)CH=NOCH_3$ | Et | Ph-4-Br |
| H | $CH(CH_3)CH=NOCH_3$ | n-Pr | Ph-4-$CF_3$ |
| H | $CH(CH_3)CH=NOCH_3$ | i-Pr | Ph-4-$OCF_3$ |
| H | $CH(CH_3)CH=NOCH_3$ | c-Pr | (L-45c) $CF_3$ |
| H | $CH(CH_3)CH=NOCH_3$ | $CHF_2$ | Ph-4-Br |
| H | $CH(CH_3)CH=NOCH_3$ | $CF_3$ | $CF_2O$ (Ph-4-Cl) |
| H | $CH(CH_3)CH=NOCH_3$ | $CF_3$ | CH=CH (Ph-4-Cl) |
| H | $CH(CH_3)CH=NOCH_3$ | $CF_3$ | CH=CH (Ph-4-Br) |
| H | $CH(CH_3)CH=NOCH_3$ | $CF_3$ | CH=CH (Ph-4-$CF_3$) |
| H | $CH(CH_3)CH=NOCH_3$ | $CF_3$ | CH=CH (Ph-4-$OCF_3$) |
| H | $CH(CH_3)CH=NOCH_3$ | $CF_3$ | CH=CH (Ph-3, 4-$Cl_2$) |
| H | $CH(CH_3)CH=NOCH_3$ | $CF_3$ | Ph-4-F |
| H | $CH(CH_3)CH=NOCH_3$ | $CF_3$ | Ph-4-Cl |
| H | $CH(CH_3)CH=NOCH_3$ | $CF_3$ | Ph-4-Br |
| H | $CH(CH_3)CH=NOCH_3$ | $CF_3$ | Ph-4-I |
| H | $CH(CH_3)CH=NOCH_3$ | $CF_3$ | Ph-4-$CF_3$ |
| H | $CH(CH_3)CH=NOCH_3$ | $CF_3$ | Ph-4-$OCHF_2$ |
| H | $CH(CH_3)CH=NOCH_3$ | $CF_3$ | Ph-4-$OCF_3$ |
| H | $CH(CH_3)CH=NOCH_3$ | $CF_3$ | Ph-4-$OCF_2Br$ |
| H | $CH(CH_3)CH=NOCH_3$ | $CF_3$ | Ph-4-$OCF_2CHF_2$ |
| H | $CH(CH_3)CH=NOCH_3$ | $CF_3$ | Ph-4-$OCF_2CHFCl$ |
| H | $CH(CH_3)CH=NOCH_3$ | $CF_3$ | Ph-4-$OCF_2CHFCF_3$ |
| H | $CH(CH_3)CH=NOCH_3$ | $CF_3$ | Ph-4-$OCF_2CHFOCF_3$ |
| H | $CH(CH_3)CH=NOCH_3$ | $CF_3$ | Ph-4-$OCF_2CHFOCF_2CF_2CF_3$ |

| | | | |
|---|---|---|---|
| H | $CH(CH_3)CH=NOCH_3$ | $CF_3$ | Ph-4-O (L-45g) |
| H | $CH(CH_3)CH=NOCH_3$ | $CF_3$ | Ph-3, 4-$F_2$ |
| H | $CH(CH_3)CH=NOCH_3$ | $CF_3$ | Ph-3-F-4-Cl |
| H | $CH(CH_3)CH=NOCH_3$ | $CF_3$ | Ph-3, 4-$Cl_2$ |
| H | $CH(CH_3)CH=NOCH_3$ | $CF_3$ | Ph (-3-$OCF_2O$-4-) |
| H | $CH(CH_3)CH=NOCH_3$ | $CF_3$ | Ph (-3-$OCF_2CF_2O$-4-) |
| H | $CH(CH_3)CH=NOCH_3$ | $CF_3$ | (L-45c) Cl |
| H | $CH(CH_3)CH=NOCH_3$ | $CF_3$ | (L-45c) Br |
| H | $CH(CH_3)CH=NOCH_3$ | $CF_3$ | (L-45c) $CF_3$ |
| H | $CH(CH_3)CH=NOCH_3$ | $CF_3$ | L-45e |
| H | $CH(CH_3)CH=NOCH_3$ | $CF_3$ | L-45f |
| H | $CH(CH_3)CH=NOCH_3$ | $CF_3$ | (L-46c) Cl |
| H | $CH(CH_3)CH=NOCH_3$ | $CF_3$ | (L-46c) Br |
| H | $CH(CH_3)CH=NOCH_3$ | $CF_3$ | (L-46c) $CF_3$ |
| H | $CH(CH_3)CH=NOCH_3$ | $CF_3$ | L-46d |
| H | $CH(CH_3)CH=NOCH_3$ | $CF_2Cl$ | Ph-4-$CF_3$ |
| H | $CH(CH_3)CH=NOCH_3$ | $CF_2Br$ | Ph-4-$OCF_3$ |
| H | $CH(CH_3)CH=NOCH_3$ | $CF_2CF_3$ | (L-45c) $CF_3$ |
| H | $CH(CH_3)CH=NOCH_3$ | $CF_2CF_2CF_3$ | Ph-4-Br |
| H | $CH(CH_3)CH=NOCH_3$ | $CF_2OCH_3$ | Ph-4-$CF_3$ |
| H | $CH(CH_3)CH=NOCH_3$ | $CF_2SCH_3$ | Ph-4-$OCF_3$ |
| H | $CH(CH_3)CH=NOPr$-n | $CF_3$ | Ph-4-Br |
| H | $CH(CH_3)CH=NOPr$-n | $CF_3$ | Ph-4-$CF_3$ |
| H | $CH(CH_3)CH=NOPr$-n | $CF_3$ | Ph-4-$OCF_3$ |
| H | $CH(CH_3)CH=NOPr$-n | $CF_3$ | (L-45c) $CF_3$ |
| H | $CH(CH_3)CH=NOCH_2Pr$-c | $CF_3$ | (L-45c) $CF_3$ |
| H | $CH(CH_3)CH=NOCH_2CH_2OEt$ | $CF_3$ | Ph-4-Br |
| H | $CH(CH_3)CH=NOCH_2CH_2SEt$ | $CF_3$ | Ph-4-$CF_3$ |
| H | $CH(CH_3)CH=NOCH_2CH=CH_2$ | $CF_3$ | Ph-4-$OCF_3$ |
| H | $CH(CH_3)CH=NOCH_2Ph$ | $CF_3$ | (L-45c) $CF_3$ |
| H | $CH(CH_3)C(CH_3)=NOCH_3$ | $CF_3$ | Ph-4-Br |
| H | $C(CH_3)_2CH=NOH$ | $CF_3$ | Ph-4-Br |
| H | $C(CH_3)_2CH=NOH$ | $CF_3$ | Ph-4-$CF_3$ |
| H | $C(CH_3)_2CH=NOH$ | $CF_3$ | Ph-4-$OCF_3$ |
| H | $C(CH_3)_2CH=NOH$ | $CF_3$ | (L-45c) $CF_3$ |
| H | $C(CH_3)_2CH=NOCH_3$ | $CF_3$ | $CF_2O$ (Ph-4-Cl) |
| H | $C(CH_3)_2CH=NOCH_3$ | $CF_3$ | CH=CH (Ph-4-Cl) |
| H | $C(CH_3)_2CH=NOCH_3$ | $CF_3$ | CH=CH (Ph-4-Br) |
| H | $C(CH_3)_2CH=NOCH_3$ | $CF_3$ | CH=CH (Ph-4-$CF_3$) |
| H | $C(CH_3)_2CH=NOCH_3$ | $CF_3$ | CH=CH (Ph-4-$OCF_3$) |
| H | $C(CH_3)_2CH=NOCH_3$ | $CF_3$ | CH=CH (Ph-3, 4-$Cl_2$) |

| | | | |
|---|---|---|---|
| H | $C(CH_3)_2CH=NOCH_3$ | $CF_3$ | Ph-4-F |
| H | $C(CH_3)_2CH=NOCH_3$ | $CF_3$ | Ph-4-Cl |
| H | $C(CH_3)_2CH=NOCH_3$ | $CF_3$ | Ph-4-Br |
| H | $C(CH_3)_2CH=NOCH_3$ | $CF_3$ | Ph-4-$CF_3$ |
| H | $C(CH_3)_2CH=NOCH_3$ | $CF_3$ | Ph-4-$OCF_3$ |
| H | $C(CH_3)_2CH=NOCH_3$ | $CF_3$ | Ph-4-$OCF_2Br$ |
| H | $C(CH_3)_2CH=NOCH_3$ | $CF_3$ | Ph-4-$OCF_2CHF_2$ |
| H | $C(CH_3)_2CH=NOCH_3$ | $CF_3$ | Ph-4-$OCF_2CHFCF_3$ |
| H | $C(CH_3)_2CH=NOCH_3$ | $CF_3$ | Ph-4-$OCF_2CHFOCF_3$ |
| H | $C(CH_3)_2CH=NOCH_3$ | $CF_3$ | Ph-4-$OCF_2CHFOCF_2CF_2CF_3$ |
| H | $C(CH_3)_2CH=NOCH_3$ | $CF_3$ | Ph-4-O(L-45g) |
| H | $C(CH_3)_2CH=NOCH_3$ | $CF_3$ | Ph-3,4-$F_2$ |
| H | $C(CH_3)_2CH=NOCH_3$ | $CF_3$ | Ph-3-F-4-Cl |
| H | $C(CH_3)_2CH=NOCH_3$ | $CF_3$ | Ph-3,4-$Cl_2$ |
| H | $C(CH_3)_2CH=NOCH_3$ | $CF_3$ | Ph(-3-$OCF_2O$-4-) |
| H | $C(CH_3)_2CH=NOCH_3$ | $CF_3$ | Ph(-3-$OCF_2CF_2O$-4-) |
| H | $C(CH_3)_2CH=NOCH_3$ | $CF_3$ | (L-45c)$CF_3$ |
| H | $C(CH_3)_2CH=NOCH_3$ | $CF_3$ | L-45f |
| H | $C(CH_3)_2CH=NOCH_3$ | $CF_3$ | (L-46c)$CF_3$ |
| H | $C(CH_3)_2CH=NOCH_3$ | $CF_3$ | L-46d |
| H | $C(CH_3)_2CH=NOEt$ | $CF_3$ | Ph-4-$CF_3$ |
| H | $C(CH_3)_2CH=NOCH_2C(O)OBu$-t | $CF_3$ | Ph-4-$OCF_3$ |
| H | $C(CH_3)_2CH=NOCH_2C(O)N(Et)_2$ | $CF_3$ | (L-45c)$CF_3$ |
| H | $C(CH_3)(CH_2SO_2CH_3)CH=NOH$ | $CF_3$ | Ph-4-Br |
| H | $C(CH_3)(CH_2SO_2Et)CH=NOH$ | $CF_3$ | Ph-4-$CF_3$ |
| H | $CH_2CH_2CH=NOCH_3$ | $CF_3$ | Ph-4-$OCF_3$ |
| H | $CH(CH_3)CH_2CH=NOCH_3$ | $CF_3$ | (L-45c)$CF_3$ |
| H | $CH_2CH_2CH_2CH=NOEt$ | $CF_3$ | Ph-4-Br |
| H | $CH_2C(O)OEt$ | $CF_3$ | Ph-4-$CF_3$ |
| H | $CH(CH_3)C(O)OCH_3$ | $CF_3$ | Ph-4-$OCF_3$ |
| H | $CH(CH_3)C(O)OEt$ | $CF_3$ | (L-45c)$CF_3$ |
| H | $CH_2CH_2C(O)OEt$ | $CF_3$ | Ph-4-Br |
| H | $CH(CH_3)CH_2C(O)OEt$ | $CF_3$ | Ph-4-$CF_3$ |
| H | $CH(CH_3)C(O)NHEt$ | $CF_3$ | Ph-4-$OCF_3$ |
| H | $CH(CH_3)C(O)NHPr$-n | $CF_3$ | (L-45c)$CF_3$ |
| H | $CH(CH_3)C(O)NHBu$-n | $CF_3$ | Ph-4-Br |
| H | $CH(CH_3)C(O)NHCH_2Ph$ | $CF_3$ | Ph-4-$CF_3$ |
| H | $CH(CH_3)C(O)N(CH_3)_2$ | $CF_3$ | Ph-4-$OCF_3$ |
| H | $CH(CH_3)C(O)N(Et)_2$ | $CF_3$ | (L-45c)$CF_3$ |
| H | $CH(CH_3)C(O)N(Pr\text{-}n)_2$ | $CF_3$ | Ph-4-Br |
| H | $CH(CH_3)C(O)$(T-16) | $CF_3$ | Ph-4-$CF_3$ |

| | | | |
|---|---|---|---|
| H | CH ($CH_3$) C (O) (T-17) | $CF_3$ | Ph-4-O$CF_3$ |
| H | CH ($CH_3$) C (O) (T-18) | $CF_3$ | (L-45c) $CF_3$ |
| H | CH ($CH_3$) C (O) (T-19) | $CF_3$ | Ph-4-Br |
| H | CH ($CH_3$) C (O) (T-20) | $CF_3$ | Ph-4-$CF_3$ |
| H | CH ($CH_3$) C (O) (T-21) | $CF_3$ | Ph-4-O$CF_3$ |
| H | CH ($CH_3$) $CH_2$C (O) NH$CH_3$ | $CF_3$ | (L-45c) $CF_3$ |
| H | CH ($CH_3$) $CH_2$C (O) NHEt | $CF_3$ | Ph-4-Br |
| H | CH ($CH_3$) $CH_2$C (O) N ($CH_3$)$_2$ | $CF_3$ | Ph-4-$CF_3$ |
| H | CH ($CH_3$) $CH_2$C (O) N (Et)$_2$ | $CF_3$ | Ph-4-O$CF_3$ |
| H | CH ($CH_3$) $CH_2$C (O) N ($CH_3$) Ph | $CF_3$ | (L-45c) $CF_3$ |
| H | CH ($CH_3$) CN | $CF_3$ | Ph-4-Br |
| H | C ($CH_3$)$_2$CN | $CF_3$ | Ph-4-$CF_3$ |
| H | $CH_2CH=CH_2$ | $CF_3$ | Ph-4-O$CF_3$ |
| $CH_2CH=CH_2$ | $CH_2CH=CH_2$ | $CF_3$ | (L-45c) $CF_3$ |
| H | C ($CH_3$)$_2$CH=$CH_2$ | $CF_3$ | CH=CH (Ph-4-Cl) |
| H | C ($CH_3$)$_2$CH=$CH_2$ | $CF_3$ | Ph-4-Cl |
| H | C ($CH_3$)$_2$CH=$CH_2$ | $CF_3$ | Ph-4-Br |
| H | C ($CH_3$)$_2$CH=$CH_2$ | $CF_3$ | Ph-4-$CF_3$ |
| H | C ($CH_3$)$_2$CH=$CH_2$ | $CF_3$ | Ph-4-O$CF_3$ |
| H | C ($CH_3$)$_2$CH=$CH_2$ | $CF_3$ | Ph-4-O$CF_2$Br |
| H | C ($CH_3$)$_2$CH=$CH_2$ | $CF_3$ | Ph-4-O$CF_2$CHF$CF_3$ |
| H | C ($CH_3$)$_2$CH=$CH_2$ | $CF_3$ | Ph-4-O$CF_2$CHFO$CF_3$ |
| H | C ($CH_3$)$_2$CH=$CH_2$ | $CF_3$ | Ph-4-O$CF_2$CHFO$CF_2CF_2CF_3$ |
| H | C ($CH_3$)$_2$CH=$CH_2$ | $CF_3$ | Ph-4-O (L-45g) |
| H | C ($CH_3$)$_2$CH=$CH_2$ | $CF_3$ | Ph-3, 4-$Cl_2$ |
| H | C ($CH_3$)$_2$CH=$CH_2$ | $CF_3$ | Ph (-3-O$CF_2$O-4-) |
| H | C ($CH_3$)$_2$CH=$CH_2$ | $CF_3$ | Ph (-3-O$CF_2CF_2$O-4-) |
| H | C ($CH_3$)$_2$CH=$CH_2$ | $CF_3$ | (L-45c) $CF_3$ |
| H | C ($CH_3$)$_2$CH=$CH_2$ | $CF_3$ | L-45f |
| H | C ($CH_3$)$_2$CH=$CH_2$ | $CF_3$ | (L-46c) $CF_3$ |
| H | C ($CH_3$)$_2$CH=$CH_2$ | $CF_3$ | L-46d |
| H | C ($CH_3$)$_2$CH=CHC (O) NHEt | $CF_3$ | Ph-4-Br |
| H | C ($CH_3$)$_2$CH=CHPh (E) | $CF_3$ | Ph-4-$CF_3$ |
| H | $CH_2C\equiv CH$ | $CF_3$ | Ph-4-O$CF_3$ |
| $CH_3$ | $CH_2C\equiv CH$ | $CF_3$ | Ph-4-Br |
| $CH_3$ | $CH_2C\equiv CH$ | $CF_3$ | Ph-4-$CF_3$ |
| $CH_3$ | $CH_2C\equiv CH$ | $CF_3$ | Ph-4-O$CF_3$ |
| $CH_3$ | $CH_2C\equiv CH$ | $CF_3$ | (L-45c) $CF_3$ |
| H | C ($CH_3$)$_2$C≡CH | $CF_3$ | CH=CH (Ph-4-Cl) |
| H | C ($CH_3$)$_2$C≡CH | $CF_3$ | Ph-4-Cl |
| H | C ($CH_3$)$_2$C≡CH | $CF_3$ | Ph-4-Br |

| | | | |
|---|---|---|---|
| H | $C(CH_3)_2C\equiv CH$ | $CF_3$ | Ph-4-$CF_3$ |
| H | $C(CH_3)_2C\equiv CH$ | $CF_3$ | Ph-4-$OCF_3$ |
| H | $C(CH_3)_2C\equiv CH$ | $CF_3$ | Ph-4-$OCF_2Br$ |
| H | $C(CH_3)_2C\equiv CH$ | $CF_3$ | Ph-4-$OCF_2CHFCF_3$ |
| H | $C(CH_3)_2C\equiv CH$ | $CF_3$ | Ph-4-$OCF_2CHFOCF_3$ |
| H | $C(CH_3)_2C\equiv CH$ | $CF_3$ | Ph-4-$OCF_2CHFOCF_2CF_2CF_3$ |
| H | $C(CH_3)_2C\equiv CH$ | $CF_3$ | Ph-4-O(L-45g) |
| H | $C(CH_3)_2C\equiv CH$ | $CF_3$ | Ph-3, 4-$Cl_2$ |
| H | $C(CH_3)_2C\equiv CH$ | $CF_3$ | Ph(-3-$OCF_2O$-4-) |
| H | $C(CH_3)_2C\equiv CH$ | $CF_3$ | Ph(-3-$OCF_2CF_2O$-4-) |
| H | $C(CH_3)_2C\equiv CH$ | $CF_3$ | (L-45c)$CF_3$ |
| H | $C(CH_3)_2C\equiv CH$ | $CF_3$ | L-45f |
| H | $C(CH_3)_2C\equiv CH$ | $CF_3$ | (L-46c)$CF_3$ |
| H | $C(CH_3)_2C\equiv CH$ | $CF_3$ | L-46d |
| H | $C(CH_3)_2C\equiv CPh$ | $CF_3$ | (L-45c)$CF_3$ |
| H | $C(CH_3)_2C\equiv C$(Ph-4-$CH_3$) | $CF_3$ | Ph-4-Br |
| H | $C(CH_3)_2C\equiv C$(Ph-4-$CF_3$) | $CF_3$ | Ph-4-$CF_3$ |
| H | $C(CH_3)_2C\equiv C$(Ph-4-$OCH_3$) | $CF_3$ | Ph-4-$OCF_3$ |
| H | $C(CH_3)_2C\equiv C$(Ph-4-$OCF_3$) | $CF_3$ | (L-45c)$CF_3$ |
| H | $C(CH_3)_2C\equiv C$(Ph-2, 4-$F_2$) | $CF_3$ | Ph-4-Br |
| H | $C(CH_3)_2C\equiv C$(Ph-2, 4-$Cl_2$) | $CF_3$ | Ph-4-$CF_3$ |
| H | $C(CH_3)_2C\equiv C$(Ph-2, 6-$Cl_2$) | $CF_3$ | Ph-4-$OCF_3$ |
| H | $C(CH_3)_2C\equiv C$(1-Naph) | $CF_3$ | (L-45c)$CF_3$ |
| H | $C(CH_3)_2C\equiv C$(L-3a) | $CF_3$ | Ph-4-Br |
| H | $C(CH_3)_2C\equiv C$(L-4a) | $CF_3$ | Ph-4-$CF_3$ |
| H | $C(CH_3)_2C\equiv C$(L-45a) | $CF_3$ | Ph-4-$OCF_3$ |
| H | $C(CH_3)_2C\equiv C$(L-45g) | $CF_3$ | (L-45c)$CF_3$ |
| H | $C(CH_3)_2C\equiv C$(L-46a) | $CF_3$ | Ph-4-Br |
| H | $CH_2Ph$ | $CF_3$ | Ph-4-$CF_3$ |
| $CH_3$ | $CH_2Ph$ | $CF_3$ | Ph-4-$OCF_3$ |
| Et | $CH_2Ph$ | $CF_3$ | (L-45c)$CF_3$ |
| H | $CH_2$(Ph-2-F) | $CF_3$ | Ph-4-Br |
| $CH_3$ | $CH_2$(Ph-2-F) | $CF_3$ | Ph-4-$CF_3$ |
| H | $CH_2$(Ph-2-Cl) | $CF_3$ | Ph-4-$OCF_3$ |
| H | $CH_2$(Ph-3-Cl) | $CF_3$ | (L-45c)$CF_3$ |
| $CH_3$ | $CH_2$(Ph-3-Cl) | $CF_3$ | Ph-4-Br |
| H | $CH_2$(Ph-4-Cl) | $CF_3$ | Ph-4-$CF_3$ |
| H | $CH_2$(Ph-2-$CH_3$) | $CF_3$ | Ph-4-$OCF_3$ |
| H | $CH_2$(Ph-3-$CH_3$) | $CF_3$ | (L-45c)$CF_3$ |
| H | $CH_2$(Ph-4-$CH_3$) | $CF_3$ | Ph-4-Br |
| H | $CH_2$(Ph-2-$CF_3$) | $CF_3$ | Ph-4-$CF_3$ |

| | | | |
|---|---|---|---|
| H | $CH_2$ (Ph-2-$OCH_3$) | $CF_3$ | Ph-4-$OCF_3$ |
| H | $CH_2$ (Ph-3-$OCH_3$) | $CF_3$ | (L-45c) $CF_3$ |
| H | $CH_2$ (Ph-4-$OCH_3$) | $CF_3$ | Ph-4-Br |
| H | $CH_2$ (Ph-4-$OCF_3$) | $CF_3$ | Ph-4-$CF_3$ |
| H | $CH_2$ (Ph-2, 3-$Cl_2$) | $CF_3$ | Ph-4-$OCF_3$ |
| H | $CH_2$ (Ph-2, 4-$Cl_2$) | $CF_3$ | (L-45c) $CF_3$ |
| H | $CH_2$ (Ph-3, 4-$Cl_2$) | $CF_3$ | Ph-4-Br |
| H | $CH_2$ (L-45a) | $CF_3$ | Ph-4-$CF_3$ |
| H | $CH_2$ (L-46a) | $CF_3$ | Ph-4-$OCF_3$ |
| H | $CH_2$ (L-47a) | $CF_3$ | (L-45c) $CF_3$ |
| H | CH ($CH_3$) Ph | $CF_3$ | Ph-4-Br |
| H | CH ($CH_3$) Ph (R) | $CF_3$ | Ph-4-$CF_3$ |
| H | CH ($CH_3$) Ph (S) | $CF_3$ | Ph-4-$OCF_3$ |
| H | CH ($CH_3$) (Ph-2-Cl) | $CF_3$ | (L-45c) $CF_3$ |
| H | CH ($CH_3$) (Ph-3-Cl) | $CF_3$ | Ph-4-Br |
| H | CH ($CH_3$) (Ph-4-Cl) | $CF_3$ | Ph-4-$CF_3$ |
| H | CH ($CH_3$) (L-1a) | $CF_3$ | Ph-4-$OCF_3$ |
| H | CH ($CH_3$) (L-3a) | $CF_3$ | (L-45c) $CF_3$ |
| H | CH ($CH_3$) (L-45a) | $CF_3$ | Ph-4-Br |
| H | C $(CH_3)_2$Ph | $CF_3$ | Ph-4-$CF_3$ |
| H | C $(CH_3)_2$ (Ph-3-Cl) | $CF_3$ | Ph-4-$OCF_3$ |
| H | C $(CH_3)_2$ (Ph-4-Cl) | $CF_3$ | (L-45c) $CF_3$ |
| H | $CH_2CH_2Ph$ | $CF_3$ | Ph-4-Br |
| H | $CH_2CH_2$ (Ph-2-Cl) | $CF_3$ | Ph-4-$CF_3$ |
| H | $CH_2CH_2$ (Ph-3-Cl) | $CF_3$ | Ph-4-$OCF_3$ |
| H | $CH_2CH_2$ (Ph-4-Cl) | $CF_3$ | (L-45c) $CF_3$ |
| H | $CH_2CH_2$ (L-46a) | $CF_3$ | Ph-4-Br |
| H | C $(CH_3)_2CH_2Ph$ | $CF_3$ | Ph-4-$CF_3$ |
| H | $CH_2CH_2CH_2Ph$ | $CF_3$ | Ph-4-$OCF_3$ |
| H | $OCH_3$ | $CF_3$ | (L-45c) $CF_3$ |
| H | OPr-n | $CF_3$ | Ph-4-Br |
| H | $OCH_2CH$=CHCl | $CF_3$ | Ph-4-$CF_3$ |
| H | $OCH_2Ph$ | $CF_3$ | Ph-4-$OCF_3$ |
| H | NHCHO | $CF_3$ | (L-45c) $CF_3$ |
| H | NHC (O) $CH_3$ | $CF_3$ | Ph-4-Br |
| H | NHC (O) Ph | $CF_3$ | Ph-4-$CF_3$ |
| H | NHC (O) $OCH_3$ | $CF_3$ | Ph-4-$OCF_3$ |
| H | NHC (O) OPh | $CF_3$ | (L-45c) $CF_3$ |
| H | NHC (O) $OCH_2Ph$ | $CF_3$ | Ph-4-Br |
| H | N $(CH_3)_2$ | $CF_3$ | Ph-4-$CF_3$ |
| H | N ($CH_3$) CHO | $CF_3$ | Ph-4-$OCF_3$ |

| | | | |
|---|---|---|---|
| H | N (CH$_3$) C (O) CH$_3$ | CF$_3$ | (L-45c) CF$_3$ |
| H | N (CH$_3$) C (O) OCH$_3$ | CF$_3$ | Ph-4-Br |

Table 3

In the table, the number(s) showing the position(s) of the substituent $(X)_m$ and $(Y)_n$ correspond to the number(s) shown in the following structural formulae, and the symbol – means unsubstituted.

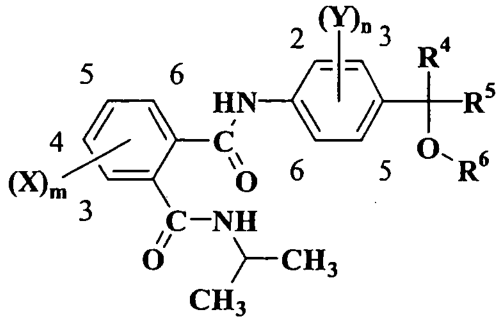

[2] - 1 ,

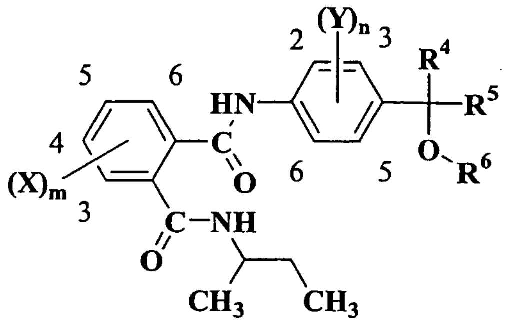

[2] - 2 ,

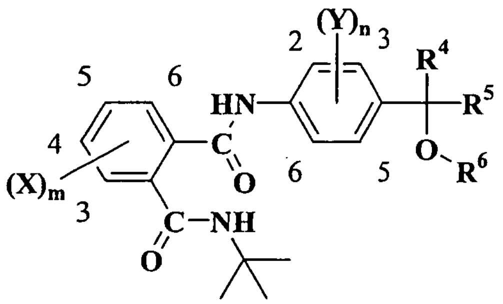

[2] - 3 ,

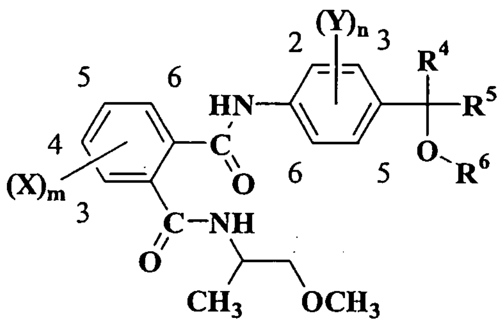

[2] - 4 ,

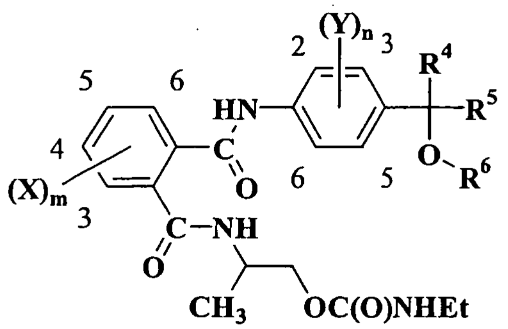

[2] - 5 ,

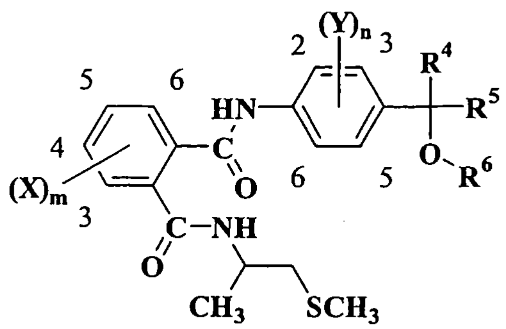

[2] - 6

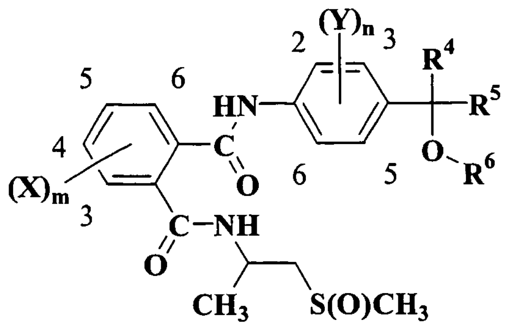

[2] - 7 ,

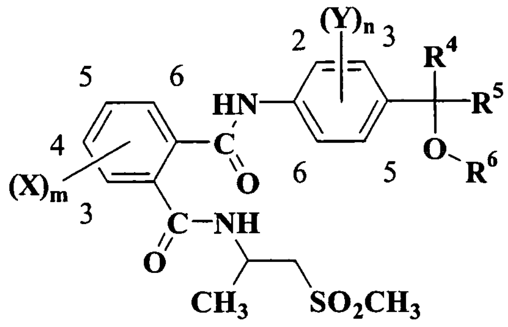

[2] - 8

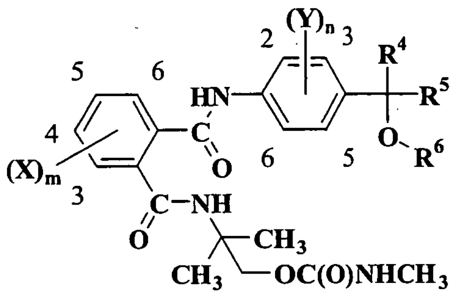

[2] - 9 ,

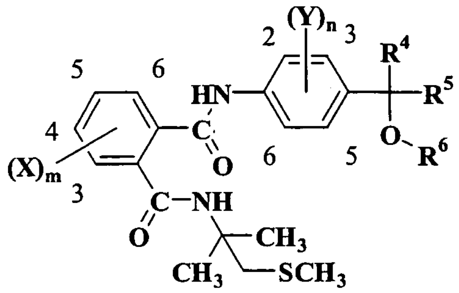

[2] - 10 ,

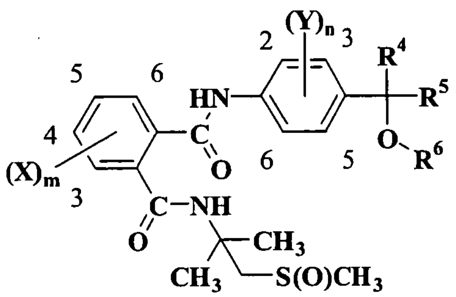

[2] - 11 or

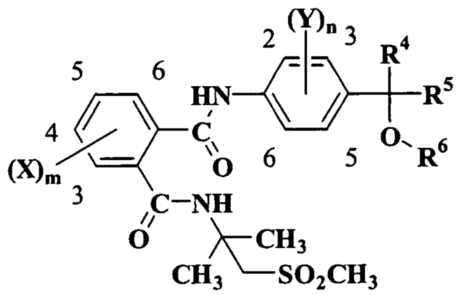

[2] - 12

| $(X)_m$ | $(Y)_n$ | $R^4$ | $R^5$ | $R^6$ |
|---|---|---|---|---|
| - | $2-CH_3$ | $CF_3$ | Ph-4-Br | H |
| 5-F | $2-CH_3$ | $CF_3$ | $Ph-4-CF_3$ | H |
| 6-F | $2-CH_3$ | $CF_3$ | $Ph-4-OCF_3$ | H |
| $3,4-F_2$ | $2-CH_3$ | $CF_3$ | Ph-4-Br | H |
| $3,4-F_2$ | $2-CH_3$ | $CF_3$ | $Ph-4-CF_3$ | H |
| $3,4-F_2$ | $2-CH_3$ | $CF_3$ | $Ph-4-OCF_3$ | H |
| $3,4-F_2$ | $2-CH_3$ | $CF_3$ | (L-45c) $CF_3$ | H |
| $3,6-F_2$ | $2-CH_3$ | $CF_3$ | (L-45c) $CF_3$ | H |
| $4,5-F_2$ | $2-CH_3$ | $CF_3$ | Ph-4-Br | H |
| 4-Cl | $2-CH_3$ | $CF_3$ | $Ph-4-CF_3$ | H |

| | | | | |
|---|---|---|---|---|
| 5-Cl | 2-$CH_3$ | $CF_3$ | Ph-4-$OCF_3$ | H |
| 6-Cl | 2-$CH_3$ | $CF_3$ | (L-45c) $CF_3$ | H |
| 3-Cl-4-F | 2-$CH_3$ | $CF_3$ | Ph-4-Br | H |
| 3-Cl-4-F | 2-$CH_3$ | $CF_3$ | Ph-4-$CF_3$ | H |
| 3-Cl-4-F | 2-$CH_3$ | $CF_3$ | Ph-4-$OCF_3$ | H |
| 3-Cl-4-F | 2-$CH_3$ | $CF_3$ | (L-45c) $CF_3$ | H |
| 3, 4-$Cl_2$ | 2-$CH_3$ | $CH_3$ | Ph-4-Br | H |
| 3, 4-$Cl_2$ | 2-$CH_3$ | $CH_3$ | Ph-4-$CF_3$ | H |
| 3, 4-$Cl_2$ | 2-$CH_3$ | $CH_3$ | Ph-4-$OCF_3$ | H |
| 3, 4-$Cl_2$ | 2-$CH_3$ | $CH_3$ | (L-45c) Cl | H |
| 3, 4-$Cl_2$ | 2-$CH_3$ | $CH_3$ | (L-45c) Br | H |
| 3, 4-$Cl_2$ | 2-$CH_3$ | $CH_3$ | (L-45c) $CF_3$ | H |
| 3, 4-$Cl_2$ | 2-$CH_3$ | $CH_3$ | (L-45c) $CF_3$ | H |
| 3, 4-$Cl_2$ | 2-$CH_3$ | n-Pr | Ph-4-Br | H |
| 3, 4-$Cl_2$ | 2-$CH_3$ | i-Pr | Ph-4-$CF_3$ | H |
| 3, 4-$Cl_2$ | 2-$CH_3$ | c-Pr | Ph-4-$OCF_3$ | H |
| 3, 4-$Cl_2$ | 2-$CH_3$ | $CHF_2$ | (L-45c) $CF_3$ | H |
| 3, 4-$Cl_2$ | 2-$CH_3$ | $CF_3$ | $CF_2O$ (Ph-4-Cl) | H |
| 3, 4-$Cl_2$ | 2-$CH_3$ | $CF_3$ | CH=CH (Ph-4-Cl) | H |
| 3, 4-$Cl_2$ | 2-$CH_3$ | $CF_3$ | CH=CH (Ph-4-Br) | H |
| 3, 4-$Cl_2$ | 2-$CH_3$ | $CF_3$ | CH=CH (Ph-4-$CF_3$) | H |
| 3, 4-$Cl_2$ | 2-$CH_3$ | $CF_3$ | CH=CH (Ph-4-$OCF_3$) | H |
| 3, 4-$Cl_2$ | 2-$CH_3$ | $CF_3$ | CH=CH (Ph-3, 4-$Cl_2$) | H |
| 3, 4-$Cl_2$ | 2-$CH_3$ | $CF_3$ | Ph-4-F | H |
| 3, 4-$Cl_2$ | 2-$CH_3$ | $CF_3$ | Ph-4-Cl | H |
| 3, 4-$Cl_2$ | 2-$CH_3$ | $CF_3$ | Ph-4-Br | H |
| 3, 4-$Cl_2$ | 2-$CH_3$ | $CF_3$ | Ph-4-I | H |
| 3, 4-$Cl_2$ | 2-$CH_3$ | $CF_3$ | Ph-4-$CF_3$ | H |
| 3, 4-$Cl_2$ | 2-$CH_3$ | $CF_3$ | Ph-4-$OCHF_2$ | H |
| 3, 4-$Cl_2$ | 2-$CH_3$ | $CF_3$ | Ph-4-$OCF_3$ | H |
| 3, 4-$Cl_2$ | 2-$CH_3$ | $CF_3$ | Ph-4-$OCF_2Br$ | H |
| 3, 4-$Cl_2$ | 2-$CH_3$ | $CF_3$ | Ph-4-$OCF_2CHF_2$ | H |
| 3, 4-$Cl_2$ | 2-$CH_3$ | $CF_3$ | Ph-4-$OCF_2CHFCl$ | H |
| 3, 4-$Cl_2$ | 2-$CH_3$ | $CF_3$ | Ph-4-$OCF_2CHFCF_3$ | H |
| 3, 4-$Cl_2$ | 2-$CH_3$ | $CF_3$ | Ph-4-$OCF_2CHFOCF_3$ | H |
| 3, 4-$Cl_2$ | 2-$CH_3$ | $CF_3$ | Ph-4-$OCF_2CHFOCF_2CF_2CF_3$ | H |
| 3, 4-$Cl_2$ | 2-$CH_3$ | $CF_3$ | Ph-4-O (L-45g) | H |
| 3, 4-$Cl_2$ | 2-$CH_3$ | $CF_3$ | Ph-3, 4-$F_2$ | H |
| 3, 4-$Cl_2$ | 2-$CH_3$ | $CF_3$ | Ph-3-F-4-Cl | H |
| 3, 4-$Cl_2$ | 2-$CH_3$ | $CF_3$ | Ph-3, 4-$Cl_2$ | H |
| 3, 4-$Cl_2$ | 2-$CH_3$ | $CF_3$ | Ph (-3-$OCF_2O$-4-) | H |

| | | | | |
|---|---|---|---|---|
| 3, 4-$Cl_2$ | 2-$CH_3$ | $CF_3$ | Ph (-3-$OCF_2CF_2O$-4-) | H |
| 3, 4-$Cl_2$ | 2-$CH_3$ | $CF_3$ | (L-45c) Cl | H |
| 3, 4-$Cl_2$ | 2-$CH_3$ | $CF_3$ | (L-45c) Br | H |
| 3, 4-$Cl_2$ | 2-$CH_3$ | $CF_3$ | (L-45c) $CF_3$ | H |
| 3, 4-$Cl_2$ | 2-$CH_3$ | $CF_3$ | L-45e | H |
| 3, 4-$Cl_2$ | 2-$CH_3$ | $CF_3$ | L-45f | H |
| 3, 4-$Cl_2$ | 2-$CH_3$ | $CF_3$ | (L-46c) Cl | H |
| 3, 4-$Cl_2$ | 2-$CH_3$ | $CF_3$ | (L-46c) Br | H |
| 3, 4-$Cl_2$ | 2-$CH_3$ | $CF_3$ | (L-46c) $CF_3$ | H |
| 3, 4-$Cl_2$ | 2-$CH_3$ | $CF_3$ | L-46d | H |
| 3, 4-$Cl_2$ | 2-$CH_3$ | $CF_2Cl$ | Ph-4-Br | H |
| 3, 4-$Cl_2$ | 2-$CH_3$ | $CF_2Br$ | Ph-4-$CF_3$ | H |
| 3, 4-$Cl_2$ | 2-$CH_3$ | $CF_2CF_3$ | Ph-4-$OCF_3$ | H |
| 3, 4-$Cl_2$ | 2-$CH_3$ | $CF_2CF_2CF_3$ | (L-45c) $CF_3$ | H |
| 3, 4-$Cl_2$ | 2-$CH_3$ | $CF_2OCH_3$ | Ph-4-Br | H |
| 3, 4-$Cl_2$ | 2-$CH_3$ | $CF_2SCH_3$ | Ph-4-$CF_3$ | H |
| 3, 5-$Cl_2$ | 2-$CH_3$ | $CF_3$ | Ph-4-$OCF_3$ | H |
| 3, 6-$Cl_2$ | 2-$CH_3$ | $CF_3$ | (L-45c) $CF_3$ | H |
| 5, 6-$Cl_2$ | 2-$CH_3$ | $CF_3$ | Ph-4-Br | H |
| 6-Br | 2-$CH_3$ | $CF_3$ | Ph-4-$CF_3$ | H |
| 3, 4-$Br_2$ | 2-$CH_3$ | $CF_3$ | Ph-4-$OCF_3$ | H |
| 3, 6-$Br_2$ | 2-$CH_3$ | $CF_3$ | (L-45c) $CF_3$ | H |
| 5, 6-$Br_2$ | 2-$CH_3$ | $CF_3$ | Ph-4-Br | H |
| 3-I | 2-I | $CF_3$ | Ph-4-$CF_3$ | H |
| 3-I | 2-$CH_3$ | $CF_3$ | $CF_2O$ (Ph-4-Cl) | $CH_3$ |
| 3-I | 2-$CH_3$ | $CF_3$ | $CF_2O$ (Ph-4-Cl) | $CH_2OCH_3$ |
| 3-I | 2-$CH_3$ | $CF_3$ | $CF_2O$ (Ph-4-Cl) | C (O) $CH_3$ |
| 3-I | 2-$CH_3$ | $CF_3$ | $CF_2O$ (Ph-4-Cl) | Si $(CH_3)_3$ |
| 3-I | 2-$CH_3$ | $CF_3$ | CH=CH (Ph-4-Cl) | $CH_3$ |
| 3-I | 2-$CH_3$ | $CF_3$ | CH=CH (Ph-4-Cl) | $CH_2OCH_3$ |
| 3-I | 2-$CH_3$ | $CF_3$ | CH=CH (Ph-4-Cl) | C (O) $CH_3$ |
| 3-I | 2-$CH_3$ | $CF_3$ | CH=CH (Ph-4-Cl) | Si $(CH_3)_3$ |
| 3-I | 2-$CH_3$ | $CF_3$ | CH=CH (Ph-4-Br) | $CH_3$ |
| 3-I | 2-$CH_3$ | $CF_3$ | CH=CH (Ph-4-Br) | $CH_2OCH_3$ |
| 3-I | 2-$CH_3$ | $CF_3$ | CH=CH (Ph-4-Br) | C (O) $CH_3$ |
| 3-I | 2-$CH_3$ | $CF_3$ | CH=CH (Ph-4-Br) | Si $(CH_3)_3$ |
| 3-I | 2-$CH_3$ | $CF_3$ | CH=CH (Ph-4-$CF_3$) | $CH_3$ |
| 3-I | 2-$CH_3$ | $CF_3$ | CH=CH (Ph-4-$CF_3$) | $CH_2OCH_3$ |
| 3-I | 2-$CH_3$ | $CF_3$ | CH=CH (Ph-4-$CF_3$) | C (O) $CH_3$ |
| 3-I | 2-$CH_3$ | $CF_3$ | CH=CH (Ph-4-$CF_3$) | Si $(CH_3)_3$ |
| 3-I | 2-$CH_3$ | $CF_3$ | CH=CH (Ph-4-$OCF_3$) | $CH_3$ |

| | | | | |
|---|---|---|---|---|
| 3-I | 2-$CH_3$ | $CF_3$ | CH=CH (Ph-4-$OCF_3$) | $CH_2OCH_3$ |
| 3-I | 2-$CH_3$ | $CF_3$ | CH=CH (Ph-4-$OCF_3$) | C (O) $CH_3$ |
| 3-I | 2-$CH_3$ | $CF_3$ | CH=CH (Ph-4-$OCF_3$) | Si $(CH_3)_3$ |
| 3-I | 2-$CH_3$ | $CF_3$ | CH=CH (Ph-3, 4-$Cl_2$) | $CH_3$ |
| 3-I | 2-$CH_3$ | $CF_3$ | CH=CH (Ph-3, 4-$Cl_2$) | $CH_2OCH_3$ |
| 3-I | 2-$CH_3$ | $CF_3$ | CH=CH (Ph-3, 4-$Cl_2$) | C (O) $CH_3$ |
| 3-I | 2-$CH_3$ | $CF_3$ | CH=CH (Ph-3, 4-$Cl_2$) | Si $(CH_3)_3$ |
| 3-I | 2-$CH_3$ | $CF_3$ | Ph-4-F | $CH_3$ |
| 3-I | 2-$CH_3$ | $CF_3$ | Ph-4-F | $CH_2OCH_3$ |
| 3-I | 2-$CH_3$ | $CF_3$ | Ph-4-F | C (O) $CH_3$ |
| 3-I | 2-$CH_3$ | $CF_3$ | Ph-4-F | Si $(CH_3)_3$ |
| 3-I | 2-$CH_3$ | $CF_3$ | Ph-4-Cl | $CH_3$ |
| 3-I | 2-$CH_3$ | $CF_3$ | Ph-4-Cl | $CH_2OCH_3$ |
| 3-I | 2-$CH_3$ | $CF_3$ | Ph-4-Cl | C (O) $CH_3$ |
| 3-I | 2-$CH_3$ | $CF_3$ | Ph-4-Cl | Si $(CH_3)_3$ |
| 3-I | 2-$CH_3$ | $CF_3$ | Ph-4-Br | $CH_3$ |
| 3-I | 2-$CH_3$ | $CF_3$ | Ph-4-Br | Et |
| 3-I | 2-$CH_3$ | $CF_3$ | Ph-4-Br | n-Pr |
| 3-I | 2-$CH_3$ | $CF_3$ | Ph-4-Br | i-Pr |
| 3-I | 2-$CH_3$ | $CF_3$ | Ph-4-Br | n-Bu |
| 3-I | 2-$CH_3$ | $CF_3$ | Ph-4-Br | $CH_2CF_3$ |
| 3-I | 2-$CH_3$ | $CF_3$ | Ph-4-Br | $CH_2OCH_3$ |
| 3-I | 2-$CH_3$ | $CF_3$ | Ph-4-Br | $CH_2OEt$ |
| 3-I | 2-$CH_3$ | $CF_3$ | Ph-4-Br | $CH_2SCH_3$ |
| 3-I | 2-$CH_3$ | $CF_3$ | Ph-4-Br | $CH_2CH=CH_2$ |
| 3-I | 2-$CH_3$ | $CF_3$ | Ph-4-Br | $CH_2CH=CF_2$ |
| 3-I | 2-$CH_3$ | $CF_3$ | Ph-4-Br | $CH_2CH=CCl_2$ |
| 3-I | 2-$CH_3$ | $CF_3$ | Ph-4-Br | $CH_2CF=CF_2$ |
| 3-I | 2-$CH_3$ | $CF_3$ | Ph-4-Br | $CH_2CCl=CCl_2$ |
| 3-I | 2-$CH_3$ | $CF_3$ | Ph-4-Br | $CH_2C\equiv CH$ |
| 3-I | 2-$CH_3$ | $CF_3$ | Ph-4-Br | $CH_2Ph$ |
| 3-I | 2-$CH_3$ | $CF_3$ | Ph-4-Br | C (O) $CH_3$ |
| 3-I | 2-$CH_3$ | $CF_3$ | Ph-4-Br | C (O) Bu-t |
| 3-I | 2-$CH_3$ | $CF_3$ | Ph-4-Br | C (O) $SCH_3$ |
| 3-I | 2-$CH_3$ | $CF_3$ | Ph-4-Br | C (S) SEt |
| 3-I | 2-$CH_3$ | $CF_3$ | Ph-4-Br | Si $(CH_3)_3$ |
| 3-I | 2-$CH_3$ | $CF_3$ | Ph-4-Br | Si $(CH_3)_2$Bu-t |
| 3-I | 2-$CH_3$ | $CF_3$ | Ph-4-Br | Si $(CH_3)_2$Ph |
| 3-I | 2-$CH_3$ | $CF_3$ | Ph-4-I | $CH_3$ |
| 3-I | 2-$CH_3$ | $CF_3$ | Ph-4-I | $CH_2OCH_3$ |
| 3-I | 2-$CH_3$ | $CF_3$ | Ph-4-I | C (O) $CH_3$ |

| | | | | |
|---|---|---|---|---|
| 3-I | 2-$CH_3$ | $CF_3$ | Ph-4-I | Si ($CH_3$)$_3$ |
| 3-I | 2-$CH_3$ | $CF_3$ | Ph-4-$CF_3$ | $CH_3$ |
| 3-I | 2-$CH_3$ | $CF_3$ | Ph-4-$CF_3$ | Et |
| 3-I | 2-$CH_3$ | $CF_3$ | Ph-4-$CF_3$ | n-Pr |
| 3-I | 2-$CH_3$ | $CF_3$ | Ph-4-$CF_3$ | i-Pr |
| 3-I | 2-$CH_3$ | $CF_3$ | Ph-4-$CF_3$ | n-Bu |
| 3-I | 2-$CH_3$ | $CF_3$ | Ph-4-$CF_3$ | $CH_2CF_3$ |
| 3-I | 2-$CH_3$ | $CF_3$ | Ph-4-$CF_3$ | $CH_2OCH_3$ |
| 3-I | 2-$CH_3$ | $CF_3$ | Ph-4-$CF_3$ | $CH_2OEt$ |
| 3-I | 2-$CH_3$ | $CF_3$ | Ph-4-$CF_3$ | $CH_2SCH_3$ |
| 3-I | 2-$CH_3$ | $CF_3$ | Ph-4-$CF_3$ | $CH_2CH=CH_2$ |
| 3-I | 2-$CH_3$ | $CF_3$ | Ph-4-$CF_3$ | $CH_2CH=CF_2$ |
| 3-I | 2-$CH_3$ | $CF_3$ | Ph-4-$CF_3$ | $CH_2CH=CCl_2$ |
| 3-I | 2-$CH_3$ | $CF_3$ | Ph-4-$CF_3$ | $CH_2CF=CF_2$ |
| 3-I | 2-$CH_3$ | $CF_3$ | Ph-4-$CF_3$ | $CH_2CCl=CCl_2$ |
| 3-I | 2-$CH_3$ | $CF_3$ | Ph-4-$CF_3$ | $CH_2C\equiv CH$ |
| 3-I | 2-$CH_3$ | $CF_3$ | Ph-4-$CF_3$ | $CH_2Ph$ |
| 3-I | 2-$CH_3$ | $CF_3$ | Ph-4-$CF_3$ | C (O) $CH_3$ |
| 3-I | 2-$CH_3$ | $CF_3$ | Ph-4-$CF_3$ | C (O) Bu-t |
| 3-I | 2-$CH_3$ | $CF_3$ | Ph-4-$CF_3$ | C (O) $SCH_3$ |
| 3-I | 2-$CH_3$ | $CF_3$ | Ph-4-$CF_3$ | C (S) SEt |
| 3-I | 2-$CH_3$ | $CF_3$ | Ph-4-$CF_3$ | Si ($CH_3$)$_3$ |
| 3-I | 2-$CH_3$ | $CF_3$ | Ph-4-$CF_3$ | Si ($CH_3$)$_2$Bu-t |
| 3-I | 2-$CH_3$ | $CF_3$ | Ph-4-$CF_3$ | Si ($CH_3$)$_2$Ph |
| 3-I | 2-$CH_3$ | $CF_3$ | Ph-4-$CHF_2$ | $CH_3$ |
| 3-I | 2-$CH_3$ | $CF_3$ | Ph-4-$CHF_2$ | $CH_2OCH_3$ |
| 3-I | 2-$CH_3$ | $CF_3$ | Ph-4-$CHF_2$ | C (O) $CH_3$ |
| 3-I | 2-$CH_3$ | $CF_3$ | Ph-4-$CHF_2$ | Si ($CH_3$)$_3$ |
| 3-I | 2-$CH_3$ | $CF_3$ | Ph-4-$OCF_3$ | $CH_3$ |
| 3-I | 2-$CH_3$ | $CF_3$ | Ph-4-$OCF_3$ | Et |
| 3-I | 2-$CH_3$ | $CF_3$ | Ph-4-$OCF_3$ | n-Pr |
| 3-I | 2-$CH_3$ | $CF_3$ | Ph-4-$OCF_3$ | i-Pr |
| 3-I | 2-$CH_3$ | $CF_3$ | Ph-4-$OCF_3$ | n-Bu |
| 3-I | 2-$CH_3$ | $CF_3$ | Ph-4-$OCF_3$ | $CH_2CF_3$ |
| 3-I | 2-$CH_3$ | $CF_3$ | Ph-4-$OCF_3$ | $CH_2OCH_3$ |
| 3-I | 2-$CH_3$ | $CF_3$ | Ph-4-$OCF_3$ | $CH_2OEt$ |
| 3-I | 2-$CH_3$ | $CF_3$ | Ph-4-$OCF_3$ | $CH_2SCH_3$ |
| 3-I | 2-$CH_3$ | $CF_3$ | Ph-4-$OCF_3$ | $CH_2CH=CH_2$ |
| 3-I | 2-$CH_3$ | $CF_3$ | Ph-4-$OCF_3$ | $CH_2CH=CF_2$ |
| 3-I | 2-$CH_3$ | $CF_3$ | Ph-4-$OCF_3$ | $CH_2CH=CCl_2$ |
| 3-I | 2-$CH_3$ | $CF_3$ | Ph-4-$OCF_3$ | $CH_2CF=CF_2$ |

| | | | | |
|---|---|---|---|---|
| 3-I | 2-$CH_3$ | $CF_3$ | Ph-4-$OCF_3$ | $CH_2CCl=CCl_2$ |
| 3-I | 2-$CH_3$ | $CF_3$ | Ph-4-$OCF_3$ | $CH_2C\equiv CH$ |
| 3-I | 2-$CH_3$ | $CF_3$ | Ph-4-$OCF_3$ | $CH_2Ph$ |
| 3-I | 2-$CH_3$ | $CF_3$ | Ph-4-$OCF_3$ | C(O)$CH_3$ |
| 3-I | 2-$CH_3$ | $CF_3$ | Ph-4-$OCF_3$ | C(O)Bu-t |
| 3-I | 2-$CH_3$ | $CF_3$ | Ph-4-$OCF_3$ | C(O)$SCH_3$ |
| 3-I | 2-$CH_3$ | $CF_3$ | Ph-4-$OCF_3$ | C(S)SEt |
| 3-I | 2-$CH_3$ | $CF_3$ | Ph-4-$OCF_3$ | Si$(CH_3)_3$ |
| 3-I | 2-$CH_3$ | $CF_3$ | Ph-4-$OCF_3$ | Si$(CH_3)_2$Bu-t |
| 3-I | 2-$CH_3$ | $CF_3$ | Ph-4-$OCF_3$ | Si$(CH_3)_2$Ph |
| 3-I | 2-$CH_3$ | $CF_3$ | Ph-4-$OCF_2Br$ | $CH_3$ |
| 3-I | 2-$CH_3$ | $CF_3$ | Ph-4-$OCF_2Br$ | $CH_2OCH_3$ |
| 3-I | 2-$CH_3$ | $CF_3$ | Ph-4-$OCF_2Br$ | C(O)$CH_3$ |
| 3-I | 2-$CH_3$ | $CF_3$ | Ph-4-$OCF_2Br$ | Si$(CH_3)_3$ |
| 3-I | 2-$CH_3$ | $CF_3$ | Ph-4-$OCF_2CHF_2$ | $CH_3$ |
| 3-I | 2-$CH_3$ | $CF_3$ | Ph-4-$OCF_2CHF_2$ | $CH_2OCH_3$ |
| 3-I | 2-$CH_3$ | $CF_3$ | Ph-4-$OCF_2CHF_2$ | C(O)$CH_3$ |
| 3-I | 2-$CH_3$ | $CF_3$ | Ph-4-$OCF_2CHF_2$ | Si$(CH_3)_3$ |
| 3-I | 2-$CH_3$ | $CF_3$ | Ph-4-$OCF_2CHFCl$ | $CH_3$ |
| 3-I | 2-$CH_3$ | $CF_3$ | Ph-4-$OCF_2CHFCl$ | $CH_2OCH_3$ |
| 3-I | 2-$CH_3$ | $CF_3$ | Ph-4-$OCF_2CHFCl$ | C(O)$CH_3$ |
| 3-I | 2-$CH_3$ | $CF_3$ | Ph-4-$OCF_2CHFCl$ | Si$(CH_3)_3$ |
| 3-I | 2-$CH_3$ | $CF_3$ | Ph-4-$OCF_2CHFCF_3$ | $CH_3$ |
| 3-I | 2-$CH_3$ | $CF_3$ | Ph-4-$OCF_2CHFCF_3$ | $CH_2OCH_3$ |
| 3-I | 2-$CH_3$ | $CF_3$ | Ph-4-$OCF_2CHFCF_3$ | C(O)$CH_3$ |
| 3-I | 2-$CH_3$ | $CF_3$ | Ph-4-$OCF_2CHFCF_3$ | Si$(CH_3)_3$ |
| 3-I | 2-$CH_3$ | $CF_3$ | Ph-4-$OCF_2CHFOCF_3$ | $CH_3$ |
| 3-I | 2-$CH_3$ | $CF_3$ | Ph-4-$OCF_2CHFOCF_3$ | $CH_2OCH_3$ |
| 3-I | 2-$CH_3$ | $CF_3$ | Ph-4-$OCF_2CHFOCF_3$ | C(O)$CH_3$ |
| 3-I | 2-$CH_3$ | $CF_3$ | Ph-4-$OCF_2CHFOCF_3$ | Si$(CH_3)_3$ |
| 3-I | 2-$CH_3$ | $CF_3$ | Ph-4-$OCF_2CHFOCF_2CF_2CF_3$ | $CH_3$ |
| 3-I | 2-$CH_3$ | $CF_3$ | Ph-4-$OCF_2CHFOCF_2CF_2CF_3$ | $CH_2OCH_3$ |
| 3-I | 2-$CH_3$ | $CF_3$ | Ph-4-$OCF_2CHFOCF_2CF_2CF_3$ | C(O)$CH_3$ |
| 3-I | 2-$CH_3$ | $CF_3$ | Ph-4-$OCF_2CHFOCF_2CF_2CF_3$ | Si$(CH_3)_3$ |
| 3-I | 2-$CH_3$ | $CF_3$ | Ph-4-O(L-45g) | $CH_3$ |
| 3-I | 2-$CH_3$ | $CF_3$ | Ph-4-O(L-45g) | $CH_2OCH_3$ |
| 3-I | 2-$CH_3$ | $CF_3$ | Ph-4-O(L-45g) | C(O)$CH_3$ |
| 3-I | 2-$CH_3$ | $CF_3$ | Ph-4-O(L-45g) | Si$(CH_3)_3$ |
| 3-I | 2-$CH_3$ | $CF_3$ | Ph-3,4-$F_2$ | $CH_3$ |
| 3-I | 2-$CH_3$ | $CF_3$ | Ph-3,4-$F_2$ | $CH_2OCH_3$ |
| 3-I | 2-$CH_3$ | $CF_3$ | Ph-3,4-$F_2$ | C(O)$CH_3$ |

| | | | | |
|---|---|---|---|---|
| 3-I | 2-$CH_3$ | $CF_3$ | Ph-3, 4-$F_2$ | Si ($CH_3$)$_3$ |
| 3-I | 2-$CH_3$ | $CF_3$ | Ph-3-F-4-Cl | $CH_3$ |
| 3-I | 2-$CH_3$ | $CF_3$ | Ph-3-F-4-Cl | $CH_2OCH_3$ |
| 3-I | 2-$CH_3$ | $CF_3$ | Ph-3-F-4-Cl | C (O) $CH_3$ |
| 3-I | 2-$CH_3$ | $CF_3$ | Ph-3-F-4-Cl | Si ($CH_3$)$_3$ |
| 3-I | 2-$CH_3$ | $CF_3$ | Ph-3, 4-$Cl_2$ | $CH_3$ |
| 3-I | 2-$CH_3$ | $CF_3$ | Ph-3, 4-$Cl_2$ | $CH_2OCH_3$ |
| 3-I | 2-$CH_3$ | $CF_3$ | Ph-3, 4-$Cl_2$ | C (O) $CH_3$ |
| 3-I | 2-$CH_3$ | $CF_3$ | Ph-3, 4-$Cl_2$ | Si ($CH_3$)$_3$ |
| 3-I | 2-$CH_3$ | $CF_3$ | Ph (-3-$OCF_2O$-4-) | $CH_3$ |
| 3-I | 2-$CH_3$ | $CF_3$ | Ph (-3-$OCF_2O$-4-) | $CH_2OCH_3$ |
| 3-I | 2-$CH_3$ | $CF_3$ | Ph (-3-$OCF_2O$-4-) | C (O) $CH_3$ |
| 3-I | 2-$CH_3$ | $CF_3$ | Ph (-3-$OCF_2O$-4-) | Si ($CH_3$)$_3$ |
| 3-I | 2-$CH_3$ | $CF_3$ | Ph (-3-$OCF_2CF_2O$-4-) | $CH_3$ |
| 3-I | 2-$CH_3$ | $CF_3$ | Ph (-3-$OCF_2CF_2O$-4-) | $CH_2OCH_3$ |
| 3-I | 2-$CH_3$ | $CF_3$ | Ph (-3-$OCF_2CF_2O$-4-) | C (O) $CH_3$ |
| 3-I | 2-$CH_3$ | $CF_3$ | Ph (-3-$OCF_2CF_2O$-4-) | Si ($CH_3$)$_3$ |
| 3-I | 2-$CH_3$ | $CF_3$ | (L-45c) Cl | $CH_3$ |
| 3-I | 2-$CH_3$ | $CF_3$ | (L-45c) Cl | $CH_2OCH_3$ |
| 3-I | 2-$CH_3$ | $CF_3$ | (L-45c) Cl | C (O) $CH_3$ |
| 3-I | 2-$CH_3$ | $CF_3$ | (L-45c) Cl | Si ($CH_3$)$_3$ |
| 3-I | 2-$CH_3$ | $CF_3$ | (L-45c) Br | $CH_3$ |
| 3-I | 2-$CH_3$ | $CF_3$ | (L-45c) Br | $CH_2OCH_3$ |
| 3-I | 2-$CH_3$ | $CF_3$ | (L-45c) Br | C (O) $CH_3$ |
| 3-I | 2-$CH_3$ | $CF_3$ | (L-45c) Br | Si ($CH_3$)$_3$ |
| 3-I | 2-$CH_3$ | $CF_3$ | (L-45c) $CF_3$ | $CH_3$ |
| 3-I | 2-$CH_3$ | $CF_3$ | (L-45c) $CF_3$ | Et |
| 3-I | 2-$CH_3$ | $CF_3$ | (L-45c) $CF_3$ | n-Pr |
| 3-I | 2-$CH_3$ | $CF_3$ | (L-45c) $CF_3$ | i-Pr |
| 3-I | 2-$CH_3$ | $CF_3$ | (L-45c) $CF_3$ | n-Bu |
| 3-I | 2-$CH_3$ | $CF_3$ | (L-45c) $CF_3$ | $CH_2CF_3$ |
| 3-I | 2-$CH_3$ | $CF_3$ | (L-45c) $CF_3$ | $CH_2OCH_3$ |
| 3-I | 2-$CH_3$ | $CF_3$ | (L-45c) $CF_3$ | $CH_2OEt$ |
| 3-I | 2-$CH_3$ | $CF_3$ | (L-45c) $CF_3$ | $CH_2SCH_3$ |
| 3-I | 2-$CH_3$ | $CF_3$ | (L-45c) $CF_3$ | $CH_2CH=CH_2$ |
| 3-I | 2-$CH_3$ | $CF_3$ | (L-45c) $CF_3$ | $CH_2CH=CF_2$ |
| 3-I | 2-$CH_3$ | $CF_3$ | (L-45c) $CF_3$ | $CH_2CH=CCl_2$ |
| 3-I | 2-$CH_3$ | $CF_3$ | (L-45c) $CF_3$ | $CH_2CF=CF_2$ |
| 3-I | 2-$CH_3$ | $CF_3$ | (L-45c) $CF_3$ | $CH_2CCl=CCl_2$ |
| 3-I | 2-$CH_3$ | $CF_3$ | (L-45c) $CF_3$ | $CH_2C\equiv CH$ |
| 3-I | 2-$CH_3$ | $CF_3$ | (L-45c) $CF_3$ | $CH_2Ph$ |

| | | | | |
|---|---|---|---|---|
| 3-I | 2-$CH_3$ | $CF_3$ | (L-45c) $CF_3$ | C(O)$CH_3$ |
| 3-I | 2-$CH_3$ | $CF_3$ | (L-45c) $CF_3$ | C(O)Bu-t |
| 3-I | 2-$CH_3$ | $CF_3$ | (L-45c) $CF_3$ | C(O)$SCH_3$ |
| 3-I | 2-$CH_3$ | $CF_3$ | (L-45c) $CF_3$ | C(S)SEt |
| 3-I | 2-$CH_3$ | $CF_3$ | (L-45c) $CF_3$ | Si$(CH_3)_3$ |
| 3-I | 2-$CH_3$ | $CF_3$ | (L-45c) $CF_3$ | Si$(CH_3)_2$Bu-t |
| 3-I | 2-$CH_3$ | $CF_3$ | (L-45c) $CF_3$ | Si$(CH_3)_2$Ph |
| 3-I | 2-$CH_3$ | $CF_3$ | L-45e | $CH_3$ |
| 3-I | 2-$CH_3$ | $CF_3$ | L-45e | $CH_2OCH_3$ |
| 3-I | 2-$CH_3$ | $CF_3$ | L-45e | C(O)$CH_3$ |
| 3-I | 2-$CH_3$ | $CF_3$ | L-45e | Si$(CH_3)_3$ |
| 3-I | 2-$CH_3$ | $CF_3$ | L-45f | $CH_3$ |
| 3-I | 2-$CH_3$ | $CF_3$ | L-45f | $CH_2OCH_3$ |
| 3-I | 2-$CH_3$ | $CF_3$ | L-45f | C(O)$CH_3$ |
| 3-I | 2-$CH_3$ | $CF_3$ | L-45f | Si$(CH_3)_3$ |
| 3-I | 2-$CH_3$ | $CF_3$ | (L-46c) Cl | $CH_3$ |
| 3-I | 2-$CH_3$ | $CF_3$ | (L-46c) Cl | $CH_2OCH_3$ |
| 3-I | 2-$CH_3$ | $CF_3$ | (L-46c) Cl | C(O)$CH_3$ |
| 3-I | 2-$CH_3$ | $CF_3$ | (L-46c) Cl | Si$(CH_3)_3$ |
| 3-I | 2-$CH_3$ | $CF_3$ | (L-46c) Br | $CH_3$ |
| 3-I | 2-$CH_3$ | $CF_3$ | (L-46c) Br | $CH_2OCH_3$ |
| 3-I | 2-$CH_3$ | $CF_3$ | (L-46c) Br | C(O)$CH_3$ |
| 3-I | 2-$CH_3$ | $CF_3$ | (L-46c) Br | Si$(CH_3)_3$ |
| 3-I | 2-$CH_3$ | $CF_3$ | (L-46c) $CF_3$ | $CH_3$ |
| 3-I | 2-$CH_3$ | $CF_3$ | (L-46c) $CF_3$ | $CH_2OCH_3$ |
| 3-I | 2-$CH_3$ | $CF_3$ | (L-46c) $CF_3$ | C(O)$CH_3$ |
| 3-I | 2-$CH_3$ | $CF_3$ | (L-46c) $CF_3$ | Si$(CH_3)_3$ |
| 3-I | 2-$CH_3$ | $CF_3$ | L-46d | $CH_3$ |
| 3-I | 2-$CH_3$ | $CF_3$ | L-46d | $CH_2OCH_3$ |
| 3-I | 2-$CH_3$ | $CF_3$ | L-46d | C(O)$CH_3$ |
| 3-I | 2-$CH_3$ | $CF_3$ | L-46d | Si$(CH_3)_3$ |
| 3-I | 2-Pr-n | $CF_3$ | Ph-4-$OCF_3$ | H |
| 3-I | 2-Pr-i | $CF_3$ | (L-45c) $CF_3$ | H |
| 3-I | 2-Bu-n | $CF_3$ | Ph-4-Br | H |
| 3-I | 2-$CF_3$ | $CF_3$ | Ph-4-$CF_3$ | H |
| 3-I | 2-$CF_2CF_3$ | $CF_3$ | Ph-4-$OCF_3$ | H |
| 3-I | 2-$CH_2OH$ | $CF_3$ | Ph-4-Br | H |
| 3-I | 2-$CH_2OH$ | $CF_3$ | Ph-4-$CF_3$ | H |
| 3-I | 2-$CH_2OH$ | $CF_3$ | Ph-4-$OCF_3$ | H |
| 3-I | 2-$CH_2OH$ | $CF_3$ | (L-45c) $CF_3$ | H |
| 3-I | 2-$CH_2OCH_3$ | $CF_3$ | (L-45c) $CF_3$ | H |

| | | | | |
|---|---|---|---|---|
| 3-I | 2-$CH_2SCH_3$ | $CF_3$ | Ph-4-Br | H |
| 3-I | 2-$OCH_3$ | $CF_3$ | Ph-4-Br | H |
| 3-I | 2-$OCH_3$ | $CF_3$ | Ph-4-$CF_3$ | H |
| 3-I | 2-$OCH_3$ | $CF_3$ | Ph-4-$OCF_3$ | H |
| 3-I | 2-$OCH_3$ | $CF_3$ | (L-45c) $CF_3$ | H |
| 3-I | 2-OEt | $CF_3$ | Ph-4-$CF_3$ | H |
| 3-I | 2-OPr-i | $CF_3$ | Ph-4-$OCF_3$ | H |
| 3-I | 2-$OCF_3$ | $CF_3$ | (L-45c) $CF_3$ | H |
| 3-I | 2-OPh | $CF_3$ | Ph-4-Br | H |
| 3-I | 2-$SCH_3$ | $CF_3$ | Ph-4-Br | H |
| 3-I | 2-$SCH_3$ | $CF_3$ | Ph-4-$CF_3$ | H |
| 3-I | 2-$SCH_3$ | $CF_3$ | Ph-4-$OCF_3$ | H |
| 3-I | 2-$SCH_3$ | $CF_3$ | (L-45c) $CF_3$ | H |
| 3-I | 2-$SO_2CH_3$ | $CF_3$ | Ph-4-$CF_3$ | H |
| 3-I | 2-SEt | $CF_3$ | Ph-4-$OCF_3$ | H |
| 3-I | 2-SPr-i | $CF_3$ | (L-45c) $CF_3$ | H |
| 3-I | 2-$SCHF_2$ | $CF_3$ | Ph-4-Br | H |
| 3-I | 2-N$(CH_3)_2$ | $CF_3$ | Ph-4-$CF_3$ | H |
| 3-I | 2-CN | $CF_3$ | Ph-4-$OCF_3$ | H |
| 3-I | 2-Ph | $CF_3$ | (L-45c) $CF_3$ | H |
| 3-I | 2-(L-14a) | $CF_3$ | Ph-4-Br | H |
| 3-I | 2-(L-14b) | $CF_3$ | Ph-4-$CF_3$ | H |
| 3-I | 2-(L-14c) | $CF_3$ | Ph-4-$OCF_3$ | H |
| 3-I | 2-(L-14d) | $CF_3$ | (L-45c) $CF_3$ | H |
| 3-I | 2-(L-14e) | $CF_3$ | Ph-4-Br | H |
| 3-I | 2-(L-14f) | $CF_3$ | Ph-4-$CF_3$ | H |
| 3-I | 2-$CH_3$-3-F | $CF_3$ | Ph-4-$OCF_3$ | H |
| 3-I | 2-$CH_3$-5-F | $CF_3$ | (L-45c) $CF_3$ | H |
| 3-I | 2-$CH_3$-3-Cl | $CF_3$ | Ph-4-Br | H |
| 3-I | 2-$CH_3$-5-Cl | $CF_3$ | Ph-4-$CF_3$ | H |
| 3-I | 2, 3-$(CH_3)_2$ | $CF_3$ | Ph-4-$OCF_3$ | H |
| 3-I | 2, 5-$(CH_3)_2$ | $CF_3$ | (L-45c) $CF_3$ | H |
| 3-I | 2, 6-$(CH_3)_2$ | $CF_3$ | Ph-4-Br | H |
| 3-I | 2-$CH_3$-3-$OCH_3$ | $CF_3$ | Ph-4-$CF_3$ | H |
| 3-I | 2-$CH_3$-3-$OCHF_2$ | $CF_3$ | Ph-4-$OCF_3$ | H |
| 3-I | 2-$CH_3$-3, 5-$Cl_2$ | $CF_3$ | (L-45c) $CF_3$ | H |
| 4-I | 2-$CH_3$ | $CF_3$ | Ph-4-Br | H |
| 5-I | 2-$CH_3$ | $CF_3$ | Ph-4-$CF_3$ | H |
| 6-I | 2-$CH_3$ | $CF_3$ | Ph-4-$OCF_3$ | H |
| 3-I-4-Cl | 2-$CH_3$ | $CF_3$ | (L-45c) $CF_3$ | H |
| 4-Cl-6-I | 2-$CH_3$ | $CF_3$ | Ph-4-Br | H |

| | | | | |
|---|---|---|---|---|
| 5-$CH_3$ | 2-$CH_3$ | $CF_3$ | Ph-4-$CF_3$ | H |
| 6-$CH_3$ | 2-$CH_3$ | $CF_3$ | Ph-4-$OCF_3$ | H |
| 3-Cl-4-$CH_3$ | 2-$CH_3$ | $CF_3$ | Ph-4-Br | H |
| 3-Cl-4-$CH_3$ | 2-$CH_3$ | $CF_3$ | Ph-4-$CF_3$ | H |
| 3-Cl-4-$CH_3$ | 2-$CH_3$ | $CF_3$ | Ph-4-$OCF_3$ | H |
| 3-Cl-4-$CH_3$ | 2-$CH_3$ | $CF_3$ | (L-45c) $CF_3$ | H |
| 6-Et | 2-$CH_3$ | $CF_3$ | (L-45c) $CF_3$ | H |
| 4-$CF_3$ | 2-$CH_3$ | $CF_3$ | Ph-4-Br | H |
| 6-$CF_3$ | 2-$CH_3$ | $CF_3$ | Ph-4-$CF_3$ | H |
| 3-$CF_2CF_3$ | 2-$CH_3$ | $CF_3$ | Ph-4-$OCF_3$ | H |
| 3-$OCH_3$ | 2-$CH_3$ | $CF_3$ | (L-45c) $CF_3$ | H |
| 6-$OCH_3$ | 2-$CH_3$ | $CF_3$ | Ph-4-Br | H |
| 3-Cl-4-$OCH_3$ | 2-$CH_3$ | $CF_3$ | Ph-4-$CF_3$ | H |
| 3-$OCH_2O$-4 | 2-$CH_3$ | $CF_3$ | Ph-4-$OCF_3$ | H |
| 5-$OCHF_2$ | 2-$CH_3$ | $CF_3$ | (L-45c) $CF_3$ | H |
| 6-$OCHF_2$ | 2-$CH_3$ | $CF_3$ | Ph-4-Br | H |
| 5-$OCF_3$ | 2-$CH_3$ | $CF_3$ | Ph-4-$CF_3$ | H |
| 3-$OSO_2CH_3$ | 2-$CH_3$ | $CF_3$ | $CF_2O$ (Ph-4-Cl) | H |
| 3-$OSO_2CH_3$ | 2-$CH_3$ | $CF_3$ | CH=CH (Ph-4-Cl) | H |
| 3-$OSO_2CH_3$ | 2-$CH_3$ | $CF_3$ | CH=CH (Ph-4-Br) | H |
| 3-$OSO_2CH_3$ | 2-$CH_3$ | $CF_3$ | CH=CH (Ph-4-$CF_3$) | H |
| 3-$OSO_2CH_3$ | 2-$CH_3$ | $CF_3$ | CH=CH (Ph-4-$OCF_3$) | H |
| 3-$OSO_2CH_3$ | 2-$CH_3$ | $CF_3$ | CH=CH (Ph-3, 4-$Cl_2$) | H |
| 3-$OSO_2CH_3$ | 2-$CH_3$ | $CF_3$ | Ph-4-F | H |
| 3-$OSO_2CH_3$ | 2-$CH_3$ | $CF_3$ | Ph-4-Cl | H |
| 3-$OSO_2CH_3$ | 2-$CH_3$ | $CF_3$ | Ph-4-Br | H |
| 3-$OSO_2CH_3$ | 2-$CH_3$ | $CF_3$ | Ph-4-$CF_3$ | H |
| 3-$OSO_2CH_3$ | 2-$CH_3$ | $CF_3$ | Ph-4-$OCF_3$ | H |
| 3-$OSO_2CH_3$ | 2-$CH_3$ | $CF_3$ | Ph-4-$OCF_2Br$ | H |
| 3-$OSO_2CH_3$ | 2-$CH_3$ | $CF_3$ | Ph-4-$OCF_2CHF_2$ | H |
| 3-$OSO_2CH_3$ | 2-$CH_3$ | $CF_3$ | Ph-4-$OCF_2CHFCF_3$ | H |
| 3-$OSO_2CH_3$ | 2-$CH_3$ | $CF_3$ | Ph-4-$OCF_2CHFOCF_3$ | H |
| 3-$OSO_2CH_3$ | 2-$CH_3$ | $CF_3$ | Ph-4-$OCF_2CHFOCF_2CF_2CF_3$ | H |
| 3-$OSO_2CH_3$ | 2-$CH_3$ | $CF_3$ | Ph-4-O (L-45g) | H |
| 3-$OSO_2CH_3$ | 2-$CH_3$ | $CF_3$ | Ph-3, 4-$F_2$ | H |
| 3-$OSO_2CH_3$ | 2-$CH_3$ | $CF_3$ | Ph-3-F-4-Cl | H |
| 3-$OSO_2CH_3$ | 2-$CH_3$ | $CF_3$ | Ph-3, 4-$Cl_2$ | H |
| 3-$OSO_2CH_3$ | 2-$CH_3$ | $CF_3$ | Ph (-3-$OCF_2O$-4-) | H |
| 3-$OSO_2CH_3$ | 2-$CH_3$ | $CF_3$ | Ph (-3-$OCF_2CF_2O$-4-) | H |
| 3-$OSO_2CH_3$ | 2-$CH_3$ | $CF_3$ | (L-45c) $CF_3$ | H |
| 3-$OSO_2CH_3$ | 2-$CH_3$ | $CF_3$ | L-45f | H |

| | | | | |
|---|---|---|---|---|
| $3-OSO_2CH_3$ | $2-CH_3$ | $CF_3$ | (L-46c) $CF_3$ | H |
| $3-OSO_2CH_3$ | $2-CH_3$ | $CF_3$ | L-46d | H |
| $3-OSO_2Et$ | $2-CH_3$ | $CF_3$ | Ph-4-$OCF_3$ | H |
| $3-OSO_2CF_3$ | $2-CH_3$ | $CF_3$ | (L-45c) $CF_3$ | H |
| $3-OCF_2O-4$ | $2-CH_3$ | $CF_3$ | $CF_2O$ (Ph-4-Cl) | H |
| $3-OCF_2O-4$ | $2-CH_3$ | $CF_3$ | CH=CH (Ph-4-Cl) | H |
| $3-OCF_2O-4$ | $2-CH_3$ | $CF_3$ | CH=CH (Ph-4-Br) | H |
| $3-OCF_2O-4$ | $2-CH_3$ | $CF_3$ | CH=CH (Ph-4-$CF_3$) | H |
| $3-OCF_2O-4$ | $2-CH_3$ | $CF_3$ | CH=CH (Ph-4-$OCF_3$) | H |
| $3-OCF_2O-4$ | $2-CH_3$ | $CF_3$ | CH=CH (Ph-3, 4-$Cl_2$) | H |
| $3-OCF_2O-4$ | $2-CH_3$ | $CF_3$ | Ph-4-F | H |
| $3-OCF_2O-4$ | $2-CH_3$ | $CF_3$ | Ph-4-Cl | H |
| $3-OCF_2O-4$ | $2-CH_3$ | $CF_3$ | Ph-4-Br | H |
| $3-OCF_2O-4$ | $2-CH_3$ | $CF_3$ | Ph-4-$CF_3$ | H |
| $3-OCF_2O-4$ | $2-CH_3$ | $CF_3$ | Ph-4-$OCF_3$ | H |
| $3-OCF_2O-4$ | $2-CH_3$ | $CF_3$ | Ph-4-$OCF_2Br$ | H |
| $3-OCF_2O-4$ | $2-CH_3$ | $CF_3$ | Ph-4-$OCF_2CHF_2$ | H |
| $3-OCF_2O-4$ | $2-CH_3$ | $CF_3$ | Ph-4-$OCF_2CHFCF_3$ | H |
| $3-OCF_2O-4$ | $2-CH_3$ | $CF_3$ | Ph-4-$OCF_2CHFOCF_3$ | H |
| $3-OCF_2O-4$ | $2-CH_3$ | $CF_3$ | Ph-4-$OCF_2CHFOCF_2CF_2CF_3$ | H |
| $3-OCF_2O-4$ | $2-CH_3$ | $CF_3$ | Ph-4-O (L-45g) | H |
| $3-OCF_2O-4$ | $2-CH_3$ | $CF_3$ | Ph-3, 4-$F_2$ | H |
| $3-OCF_2O-4$ | $2-CH_3$ | $CF_3$ | Ph-3-F-4-Cl | H |
| $3-OCF_2O-4$ | $2-CH_3$ | $CF_3$ | Ph-3, 4-$Cl_2$ | H |
| $3-OCF_2O-4$ | $2-CH_3$ | $CF_3$ | Ph (-3-$OCF_2O$-4-) | H |
| $3-OCF_2O-4$ | $2-CH_3$ | $CF_3$ | Ph (-3-$OCF_2CF_2O$-4-) | H |
| $3-OCF_2O-4$ | $2-CH_3$ | $CF_3$ | (L-45c) $CF_3$ | H |
| $3-OCF_2O-4$ | $2-CH_3$ | $CF_3$ | L-45f | H |
| $3-OCF_2O-4$ | $2-CH_3$ | $CF_3$ | (L-46c) $CF_3$ | H |
| $3-OCF_2O-4$ | $2-CH_3$ | $CF_3$ | L-46d | H |
| $3-OCF_2CF_2O-4$ | $2-CH_3$ | $CF_3$ | CH=CH (Ph-4-Cl) | H |
| $3-OCF_2CF_2O-4$ | $2-CH_3$ | $CF_3$ | Ph-4-Cl | H |
| $3-OCF_2CF_2O-4$ | $2-CH_3$ | $CF_3$ | Ph-4-Br | H |
| $3-OCF_2CF_2O-4$ | $2-CH_3$ | $CF_3$ | Ph-4-$CF_3$ | H |
| $3-OCF_2CF_2O-4$ | $2-CH_3$ | $CF_3$ | Ph-4-$OCF_3$ | H |
| $3-OCF_2CF_2O-4$ | $2-CH_3$ | $CF_3$ | Ph-4-$OCF_2Br$ | H |
| $3-OCF_2CF_2O-4$ | $2-CH_3$ | $CF_3$ | Ph-4-$OCF_2CHFCF_3$ | H |
| $3-OCF_2CF_2O-4$ | $2-CH_3$ | $CF_3$ | Ph-4-$OCF_2CHFOCF_3$ | H |
| $3-OCF_2CF_2O-4$ | $2-CH_3$ | $CF_3$ | Ph-4-$OCF_2CHFOCF_2CF_2CF_3$ | H |
| $3-OCF_2CF_2O-4$ | $2-CH_3$ | $CF_3$ | Ph-4-O (L-45g) | H |
| $3-OCF_2CF_2O-4$ | $2-CH_3$ | $CF_3$ | Ph-3, 4-$Cl_2$ | H |

| | | | | |
|---|---|---|---|---|
| 3-$OCF_2CF_2O$-4 | 2-$CH_3$ | $CF_3$ | Ph (-3-$OCF_2O$-4-) | H |
| 3-$OCF_2CF_2O$-4 | 2-$CH_3$ | $CF_3$ | Ph (-3-$OCF_2CF_2O$-4-) | H |
| 3-$OCF_2CF_2O$-4 | 2-$CH_3$ | $CF_3$ | (L-45c) $CF_3$ | H |
| 3-$OCF_2CF_2O$-4 | 2-$CH_3$ | $CF_3$ | L-45f | H |
| 3-$OCF_2CF_2O$-4 | 2-$CH_3$ | $CF_3$ | (L-46c) $CF_3$ | H |
| 3-$OCF_2CF_2O$-4 | 2-$CH_3$ | $CF_3$ | L-46d | H |
| 6-$SCH_3$ | 2-$CH_3$ | $CF_3$ | Ph-4-Br | H |
| 6-$SO_2CH_3$ | 2-$CH_3$ | $CF_3$ | Ph-4-$CF_3$ | H |
| 3-SEt | 2-$CH_3$ | $CF_3$ | Ph-4-$OCF_3$ | H |
| 3-$SO_2$Et | 2-$CH_3$ | $CF_3$ | (L-45c) $CF_3$ | H |
| 3-SPr-i | 2-$CH_3$ | $CF_3$ | Ph-4-Br | H |
| 3-S (O) Pr-i | 2-$CH_3$ | $CF_3$ | Ph-4-$CF_3$ | H |
| 3-$SO_2$Pr-i | 2-$CH_3$ | $CF_3$ | Ph-4-$OCF_3$ | H |
| 6-S (O) $CF_3$ | 2-$CH_3$ | $CF_3$ | (L-45c) $CF_3$ | H |
| 3-$SCH_2CF_3$ | 2-$CH_3$ | $CF_3$ | Ph-4-Br | H |
| 6-$SCH_2CF_3$ | 2-$CH_3$ | $CF_3$ | Ph-4-$CF_3$ | H |
| 3-S (O) $CH_2CF_3$ | 2-$CH_3$ | $CF_3$ | Ph-4-$OCF_3$ | H |
| 6-S (O) $CH_2CF_3$ | 2-$CH_3$ | $CF_3$ | (L-45c) $CF_3$ | H |
| 3-$SO_2CH_2CF_3$ | 2-$CH_3$ | $CF_3$ | Ph-4-Br | H |
| 3-Cl-6-$SCF_3$ | 2-$CH_3$ | $CF_3$ | Ph-4-$CF_3$ | H |
| 6-$NO_2$ | 2-$CH_3$ | $CF_3$ | Ph-4-$OCF_3$ | H |
| 3-CN | 2-$CH_3$ | $CF_3$ | (L-45c) $CF_3$ | H |
| 6-C (O) $OCH_3$ | 2-$CH_3$ | $CF_3$ | Ph-4-Br | H |
| 3-C (O) NHPr-i | 2-$CH_3$ | $CF_3$ | Ph-4-$CF_3$ | H |
| 3-C≡CH | 2-$CH_3$ | $CF_3$ | Ph-4-$OCF_3$ | H |
| 3-C≡CSi $(CH_3)_3$ | 2-$CH_3$ | $CF_3$ | (L-45c) $CF_3$ | H |
| 6-C≡CH | 2-$CH_3$ | $CF_3$ | Ph-4-Br | H |
| 6-Ph | 2-$CH_3$ | $CF_3$ | Ph-4-$CF_3$ | H |
| 3-CH=CH-CH=CH-4 | 2-$CH_3$ | $CF_3$ | Ph-4-$OCF_3$ | H |
| 4-CH=CH-CH=CH-5 | 2-$CH_3$ | $CF_3$ | (L-45c) $CF_3$ | H |
| 3-Si $(CH_3)_3$ | 2-$CH_3$ | $CF_3$ | Ph-4-Br | H |

Table 4

In the table, the number(s) showing the position(s) of the substituent $(X)_m$ and $(Y)_n$ correspond to the number(s) shown in the following structural formulae, and the symbol – means unsubstituted.

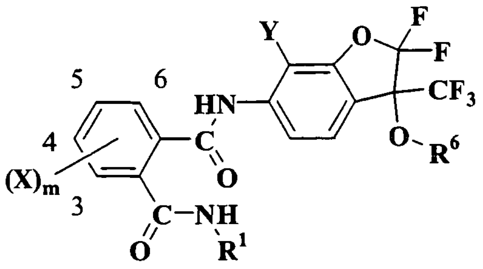

[3] - 1

or

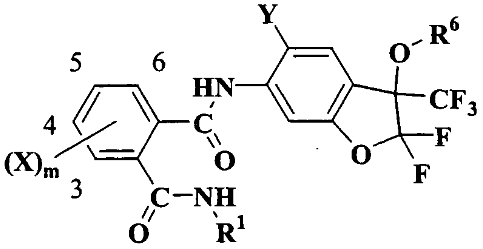

[3] - 2

| $(X)_m$ | Y | $R^1$ | $R^6$ |
|---|---|---|---|
| 3-F | $CH_3$ | i-Pr | H |
| 3-F | $CH_3$ | $CH(CH_3)CH_2SCH_3$ | H |
| 3-F | $CH_3$ | $CH(CH_3)CH_2S(O)CH_3$ | H |
| 3-F | $CH_3$ | $CH(CH_3)CH_2SO_2CH_3$ | H |
| 3-F | $CH_3$ | $C(CH_3)_2CH_2SCH_3$ | H |
| 3-F | $CH_3$ | $C(CH_3)_2CH_2S(O)CH_3$ | H |
| 3-F | $CH_3$ | $C(CH_3)_2CH_2SO_2CH_3$ | H |
| 3-Cl | $CH_3$ | i-Pr | H |
| 3-Cl | $CH_3$ | $CH(CH_3)CH_2SCH_3$ | H |
| 3-Cl | $CH_3$ | $CH(CH_3)CH_2S(O)CH_3$ | H |
| 3-Cl | $CH_3$ | $CH(CH_3)CH_2SO_2CH_3$ | H |
| 3-Cl | $CH_3$ | $C(CH_3)_2CH_2SCH_3$ | H |
| 3-Cl | $CH_3$ | $C(CH_3)_2CH_2S(O)CH_3$ | H |
| 3-Cl | $CH_3$ | $C(CH_3)_2CH_2SO_2CH_3$ | H |
| 3, 4-$Cl_2$ | $CH_3$ | i-Pr | H |
| 3, 4-$Cl_2$ | $CH_3$ | $CH(CH_3)CH_2SCH_3$ | H |
| 3, 4-$Cl_2$ | $CH_3$ | $CH(CH_3)CH_2S(O)CH_3$ | H |
| 3, 4-$Cl_2$ | $CH_3$ | $CH(CH_3)CH_2SO_2CH_3$ | H |
| 3, 4-$Cl_2$ | $CH_3$ | $C(CH_3)_2CH_2SCH_3$ | H |
| 3, 4-$Cl_2$ | $CH_3$ | $C(CH_3)_2CH_2S(O)CH_3$ | H |
| 3, 4-$Cl_2$ | $CH_3$ | $C(CH_3)_2CH_2SO_2CH_3$ | H |
| 3-Br | $CH_3$ | i-Pr | H |
| 3-Br | $CH_3$ | $CH(CH_3)CH_2SCH_3$ | H |
| 3-Br | $CH_3$ | $CH(CH_3)CH_2S(O)CH_3$ | H |
| 3-Br | $CH_3$ | $CH(CH_3)CH_2SO_2CH_3$ | H |
| 3-Br | $CH_3$ | $C(CH_3)_2CH_2SCH_3$ | H |
| 3-Br | $CH_3$ | $C(CH_3)_2CH_2S(O)CH_3$ | H |
| 3-Br | $CH_3$ | $C(CH_3)_2CH_2SO_2CH_3$ | H |
| 3-I | - | i-Pr | H |
| 3-I | - | $CH(CH_3)CH_2SCH_3$ | H |

| | | | |
|---|---|---|---|
| 3-I | - | $CH(CH_3)CH_2S(O)CH_3$ | H |
| 3-I | - | $CH(CH_3)CH_2SO_2CH_3$ | H |
| 3-I | - | $C(CH_3)_2CH_2SCH_3$ | H |
| 3-I | - | $C(CH_3)_2CH_2S(O)CH_3$ | H |
| 3-I | - | $C(CH_3)_2CH_2SO_2CH_3$ | H |
| 3-I | F | i-Pr | H |
| 3-I | F | $CH(CH_3)CH_2SCH_3$ | H |
| 3-I | F | $CH(CH_3)CH_2S(O)CH_3$ | H |
| 3-I | F | $CH(CH_3)CH_2SO_2CH_3$ | H |
| 3-I | F | $C(CH_3)_2CH_2SCH_3$ | H |
| 3-I | F | $C(CH_3)_2CH_2S(O)CH_3$ | H |
| 3-I | F | $C(CH_3)_2CH_2SO_2CH_3$ | H |
| 3-I | Cl | i-Pr | H |
| 3-I | Cl | $CH(CH_3)CH_2SCH_3$ | H |
| 3-I | Cl | $CH(CH_3)CH_2S(O)CH_3$ | H |
| 3-I | Cl | $CH(CH_3)CH_2SO_2CH_3$ | H |
| 3-I | Cl | $C(CH_3)_2CH_2SCH_3$ | H |
| 3-I | Cl | $C(CH_3)_2CH_2S(O)CH_3$ | H |
| 3-I | Cl | $C(CH_3)_2CH_2SO_2CH_3$ | H |
| 3-I | Br | i-Pr | H |
| 3-I | Br | $CH(CH_3)CH_2SCH_3$ | H |
| 3-I | Br | $CH(CH_3)CH_2S(O)CH_3$ | H |
| 3-I | Br | $CH(CH_3)CH_2SO_2CH_3$ | H |
| 3-I | Br | $C(CH_3)_2CH_2SCH_3$ | H |
| 3-I | Br | $C(CH_3)_2CH_2S(O)CH_3$ | H |
| 3-I | Br | $C(CH_3)_2CH_2SO_2CH_3$ | H |
| 3-I | $CH_3$ | $CH_3$ | H |
| 3-I | $CH_3$ | Et | H |
| 3-I | $CH_3$ | i-Pr | H |
| 3-I | $CH_3$ | i-Pr | $CH_3$ |
| 3-I | $CH_3$ | i-Pr | $CH_2OCH_3$ |
| 3-I | $CH_3$ | i-Pr | $C(O)CH_3$ |
| 3-I | $CH_3$ | i-Pr | $Si(CH_3)_3$ |
| 3-I | $CH_3$ | s-Bu | H |
| 3-I | $CH_3$ | t-Bu | H |
| 3-I | $CH_3$ | $CH(CH_3)Pr\text{-}n$ | H |
| 3-I | $CH_3$ | $CH(CH_3)CH_2OCH_3$ | H |
| 3-I | $CH_3$ | $CH(CH_3)CH_2OC(O)CH_3$ | H |
| 3-I | $CH_3$ | $CH(CH_3)CH_2OC(O)NHCH_3$ | H |
| 3-I | $CH_3$ | $CH(CH_3)CH_2OC(O)NHEt$ | H |
| 3-I | $CH_3$ | $CH(CH_3)CH_2OC(O)NHPr\text{-}n$ | H |

| | | | |
|---|---|---|---|
| 3-I | $CH_3$ | $CH(CH_3)CH_2OC(O)NHPr$-i | H |
| 3-I | $CH_3$ | $CH(CH_3)CH_2OC(O)NHPr$-c | H |
| 3-I | $CH_3$ | $CH(CH_3)CH_2OC(O)NHCH_2Ph$ | H |
| 3-I | $CH_3$ | $CH(CH_3)CH_2OC(O)NHPh$ | H |
| 3-I | $CH_3$ | $CH(CH_3)CH_2OC(O)N(Et)_2$ | H |
| 3-I | $CH_3$ | $CH(CH_3)CH_2OP(S)(OCH_3)_2$ | H |
| 3-I | $CH_3$ | $CH(CH_3)CH_2OP(S)(OEt)_2$ | H |
| 3-I | $CH_3$ | $C(CH_3)_2CH_2OCH_3$ | H |
| 3-I | $CH_3$ | $C(CH_3)_2CH_2OC(O)NHCH_3$ | H |
| 3-I | $CH_3$ | $C(CH_3)_2CH_2OC(O)NHEt$ | H |
| 3-I | $CH_3$ | $C(CH_3)_2CH_2OC(O)NHPr$-n | H |
| 3-I | $CH_3$ | $C(CH_3)_2CH_2OC(O)NHCH_2Ph$ | H |
| 3-I | $CH_3$ | $C(CH_3)_2CH_2OC(O)N(CH_3)_2$ | H |
| 3-I | $CH_3$ | $CH(CH_3)CH_2CH_2OEt$ | H |
| 3-I | $CH_3$ | $CH(CH_3)CH_2SCH_3$ | H |
| 3-I | $CH_3$ | $CH(CH_3)CH_2SCH_3$ | $CH_3$ |
| 3-I | $CH_3$ | $CH(CH_3)CH_2SCH_3$ | $CH_2OCH_3$ |
| 3-I | $CH_3$ | $CH(CH_3)CH_2SCH_3$ | $C(O)CH_3$ |
| 3-I | $CH_3$ | $CH(CH_3)CH_2SCH_3$ | $Si(CH_3)_3$ |
| 3-I | $CH_3$ | $CH(CH_3)CH_2S(O)CH_3$ | H |
| 3-I | $CH_3$ | $CH(CH_3)CH_2S(O)CH_3$ | $CH_3$ |
| 3-I | $CH_3$ | $CH(CH_3)CH_2S(O)CH_3$ | $CH_2OCH_3$ |
| 3-I | $CH_3$ | $CH(CH_3)CH_2S(O)CH_3$ | $C(O)CH_3$ |
| 3-I | $CH_3$ | $CH(CH_3)CH_2S(O)CH_3$ | $Si(CH_3)_3$ |
| 3-I | $CH_3$ | $CH(CH_3)CH_2SO_2CH_3$ | H |
| 3-I | $CH_3$ | $CH(CH_3)CH_2SO_2CH_3$ | $CH_3$ |
| 3-I | $CH_3$ | $CH(CH_3)CH_2SO_2CH_3$ | $CH_2OCH_3$ |
| 3-I | $CH_3$ | $CH(CH_3)CH_2SO_2CH_3$ | $C(O)CH_3$ |
| 3-I | $CH_3$ | $CH(CH_3)CH_2SO_2CH_3$ | $Si(CH_3)_3$ |
| 3-I | $CH_3$ | $CH(CH_3)CH_2SEt$ | H |
| 3-I | $CH_3$ | $CH(CH_3)CH_2S(O)Et$ | H |
| 3-I | $CH_3$ | $CH(CH_3)CH_2SO_2Et$ | H |
| 3-I | $CH_3$ | $CH(CH_3)CH_2SCH_2Si(CH_3)_3$ | H |
| 3-I | $CH_3$ | $CH(CH_3)CH_2SSCH_3$ | H |
| 3-I | $CH_3$ | $CH(CH_3)CH_2SO_2NHEt$ | H |
| 3-I | $CH_3$ | $CH(CH_3)CH_2SO_2N(Et)_2$ | H |
| 3-I | $CH_3$ | $C(CH_3)_2CH_2SCH_3$ | H |
| 3-I | $CH_3$ | $C(CH_3)_2CH_2SCH_3$ | $CH_3$ |
| 3-I | $CH_3$ | $C(CH_3)_2CH_2SCH_3$ | $CH_2OCH_3$ |
| 3-I | $CH_3$ | $C(CH_3)_2CH_2SCH_3$ | $C(O)CH_3$ |
| 3-I | $CH_3$ | $C(CH_3)_2CH_2SCH_3$ | $Si(CH_3)_3$ |

| | | | |
|---|---|---|---|
| 3-I | $CH_3$ | $C(CH_3)_2CH_2S(O)CH_3$ | H |
| 3-I | $CH_3$ | $C(CH_3)_2CH_2S(O)CH_3$ | $CH_3$ |
| 3-I | $CH_3$ | $C(CH_3)_2CH_2S(O)CH_3$ | $CH_2OCH_3$ |
| 3-I | $CH_3$ | $C(CH_3)_2CH_2S(O)CH_3$ | $C(O)CH_3$ |
| 3-I | $CH_3$ | $C(CH_3)_2CH_2S(O)CH_3$ | $Si(CH_3)_3$ |
| 3-I | $CH_3$ | $C(CH_3)_2CH_2SO_2CH_3$ | H |
| 3-I | $CH_3$ | $C(CH_3)_2CH_2SO_2CH_3$ | $CH_3$ |
| 3-I | $CH_3$ | $C(CH_3)_2CH_2SO_2CH_3$ | $CH_2OCH_3$ |
| 3-I | $CH_3$ | $C(CH_3)_2CH_2SO_2CH_3$ | $C(O)CH_3$ |
| 3-I | $CH_3$ | $C(CH_3)_2CH_2SO_2CH_3$ | $Si(CH_3)_3$ |
| 3-I | $CH_3$ | $C(CH_3)_2CH_2SEt$ | H |
| 3-I | $CH_3$ | $C(CH_3)_2CH_2S(O)Et$ | H |
| 3-I | $CH_3$ | $C(CH_3)_2CH_2SO_2Et$ | H |
| 3-I | $CH_3$ | $C(CH_3)_2CH_2SO_2N(Et)_2$ | H |
| 3-I | $CH_3$ | $CH(CH_3)CH_2N(CH_3)SO_2CH_3$ | H |
| 3-I | $CH_3$ | $C(CH_3)_2CH_2NHC(O)OCH_3$ | H |
| 3-I | $CH_3$ | $CH(CH_3)CH=NOCH_3$ | H |
| 3-I | $CH_3$ | $C(CH_3)_2CH=NOCH_3$ | H |
| 3-I | $CH_3$ | $C(CH_3)_2CH=CH_2$ | H |
| 3-I | $CH_3$ | $C(CH_3)_2C\equiv CH$ | H |
| 3-I | Et | i-Pr | H |
| 3-I | Et | $CH(CH_3)CH_2SCH_3$ | H |
| 3-I | Et | $CH(CH_3)CH_2S(O)CH_3$ | H |
| 3-I | Et | $CH(CH_3)CH_2SO_2CH_3$ | H |
| 3-I | Et | $C(CH_3)_2CH_2SCH_3$ | H |
| 3-I | Et | $C(CH_3)_2CH_2S(O)CH_3$ | H |
| 3-I | Et | $C(CH_3)_2CH_2SO_2CH_3$ | H |
| 3-I | $OCH_3$ | i-Pr | H |
| 3-I | $OCH_3$ | $CH(CH_3)CH_2SCH_3$ | H |
| 3-I | $OCH_3$ | $CH(CH_3)CH_2S(O)CH_3$ | H |
| 3-I | $OCH_3$ | $CH(CH_3)CH_2SO_2CH_3$ | H |
| 3-I | $OCH_3$ | $C(CH_3)_2CH_2SCH_3$ | H |
| 3-I | $OCH_3$ | $C(CH_3)_2CH_2S(O)CH_3$ | H |
| 3-I | $OCH_3$ | $C(CH_3)_2CH_2SO_2CH_3$ | H |
| 3-I | $SCH_3$ | i-Pr | H |
| 3-I | $SCH_3$ | $CH(CH_3)CH_2SCH_3$ | H |
| 3-I | $SCH_3$ | $CH(CH_3)CH_2S(O)CH_3$ | H |
| 3-I | $SCH_3$ | $CH(CH_3)CH_2SO_2CH_3$ | H |
| 3-I | $SCH_3$ | $C(CH_3)_2CH_2SCH_3$ | H |
| 3-I | $SCH_3$ | $C(CH_3)_2CH_2S(O)CH_3$ | H |
| 3-I | $SCH_3$ | $C(CH_3)_2CH_2SO_2CH_3$ | H |

| | | | |
|---|---|---|---|
| 3-$CH_3$ | $CH_3$ | i-Pr | H |
| 3-$CH_3$ | $CH_3$ | $CH(CH_3)CH_2SCH_3$ | H |
| 3-$CH_3$ | $CH_3$ | $CH(CH_3)CH_2S(O)CH_3$ | H |
| 3-$CH_3$ | $CH_3$ | $CH(CH_3)CH_2SO_2CH_3$ | H |
| 3-$CH_3$ | $CH_3$ | $C(CH_3)_2CH_2SCH_3$ | H |
| 3-$CH_3$ | $CH_3$ | $C(CH_3)_2CH_2S(O)CH_3$ | H |
| 3-$CH_3$ | $CH_3$ | $C(CH_3)_2CH_2SO_2CH_3$ | H |
| 3-$CF_3$ | $CH_3$ | i-Pr | H |
| 3-$CF_3$ | $CH_3$ | $CH(CH_3)CH_2SCH_3$ | H |
| 3-$CF_3$ | $CH_3$ | $CH(CH_3)CH_2S(O)CH_3$ | H |
| 3-$CF_3$ | $CH_3$ | $CH(CH_3)CH_2SO_2CH_3$ | H |
| 3-$CF_3$ | $CH_3$ | $C(CH_3)_2CH_2SCH_3$ | H |
| 3-$CF_3$ | $CH_3$ | $C(CH_3)_2CH_2S(O)CH_3$ | H |
| 3-$CF_3$ | $CH_3$ | $C(CH_3)_2CH_2SO_2CH_3$ | H |
| 3-$OCHF_2$ | $CH_3$ | i-Pr | H |
| 3-$OCHF_2$ | $CH_3$ | $CH(CH_3)CH_2SCH_3$ | H |
| 3-$OCHF_2$ | $CH_3$ | $CH(CH_3)CH_2S(O)CH_3$ | H |
| 3-$OCHF_2$ | $CH_3$ | $CH(CH_3)CH_2SO_2CH_3$ | H |
| 3-$OCHF_2$ | $CH_3$ | $C(CH_3)_2CH_2SCH_3$ | H |
| 3-$OCHF_2$ | $CH_3$ | $C(CH_3)_2CH_2S(O)CH_3$ | H |
| 3-$OCHF_2$ | $CH_3$ | $C(CH_3)_2CH_2SO_2CH_3$ | H |
| 3-$OCF_3$ | $CH_3$ | i-Pr | H |
| 3-$OCF_3$ | $CH_3$ | $CH(CH_3)CH_2SCH_3$ | H |
| 3-$OCF_3$ | $CH_3$ | $CH(CH_3)CH_2S(O)CH_3$ | H |
| 3-$OCF_3$ | $CH_3$ | $CH(CH_3)CH_2SO_2CH_3$ | H |
| 3-$OCF_3$ | $CH_3$ | $C(CH_3)_2CH_2SCH_3$ | H |
| 3-$OCF_3$ | $CH_3$ | $C(CH_3)_2CH_2S(O)CH_3$ | H |
| 3-$OCF_3$ | $CH_3$ | $C(CH_3)_2CH_2SO_2CH_3$ | H |
| 3-$OSO_2CH_3$ | $CH_3$ | i-Pr | H |
| 3-$OSO_2CH_3$ | $CH_3$ | $CH(CH_3)CH_2SCH_3$ | H |
| 3-$OSO_2CH_3$ | $CH_3$ | $CH(CH_3)CH_2S(O)CH_3$ | H |
| 3-$OSO_2CH_3$ | $CH_3$ | $CH(CH_3)CH_2SO_2CH_3$ | H |
| 3-$OSO_2CH_3$ | $CH_3$ | $C(CH_3)_2CH_2SCH_3$ | H |
| 3-$OSO_2CH_3$ | $CH_3$ | $C(CH_3)_2CH_2S(O)CH_3$ | H |
| 3-$OSO_2CH_3$ | $CH_3$ | $C(CH_3)_2CH_2SO_2CH_3$ | H |
| 3-$OCF_2O$-4 | $CH_3$ | i-Pr | H |
| 3-$OCF_2O$-4 | $CH_3$ | $CH(CH_3)CH_2SCH_3$ | H |
| 3-$OCF_2O$-4 | $CH_3$ | $CH(CH_3)CH_2S(O)CH_3$ | H |
| 3-$OCF_2O$-4 | $CH_3$ | $CH(CH_3)CH_2SO_2CH_3$ | H |
| 3-$OCF_2O$-4 | $CH_3$ | $C(CH_3)_2CH_2SCH_3$ | H |
| 3-$OCF_2O$-4 | $CH_3$ | $C(CH_3)_2CH_2S(O)CH_3$ | H |

| | | | |
|---|---|---|---|
| $3\text{-}OCF_2O\text{-}4$ | $CH_3$ | $C(CH_3)_2CH_2SO_2CH_3$ | H |
| $3\text{-}SCH_3$ | $CH_3$ | i-Pr | H |
| $3\text{-}SCH_3$ | $CH_3$ | $CH(CH_3)CH_2SCH_3$ | H |
| $3\text{-}SCH_3$ | $CH_3$ | $CH(CH_3)CH_2S(O)CH_3$ | H |
| $3\text{-}SCH_3$ | $CH_3$ | $CH(CH_3)CH_2SO_2CH_3$ | H |
| $3\text{-}SCH_3$ | $CH_3$ | $C(CH_3)_2CH_2SCH_3$ | H |
| $3\text{-}SCH_3$ | $CH_3$ | $C(CH_3)_2CH_2S(O)CH_3$ | H |
| $3\text{-}SCH_3$ | $CH_3$ | $C(CH_3)_2CH_2SO_2CH_3$ | H |
| $3\text{-}S(O)CH_3$ | $CH_3$ | i-Pr | H |
| $3\text{-}S(O)CH_3$ | $CH_3$ | $CH(CH_3)CH_2SCH_3$ | H |
| $3\text{-}S(O)CH_3$ | $CH_3$ | $CH(CH_3)CH_2S(O)CH_3$ | H |
| $3\text{-}S(O)CH_3$ | $CH_3$ | $CH(CH_3)CH_2SO_2CH_3$ | H |
| $3\text{-}S(O)CH_3$ | $CH_3$ | $C(CH_3)_2CH_2SCH_3$ | H |
| $3\text{-}S(O)CH_3$ | $CH_3$ | $C(CH_3)_2CH_2S(O)CH_3$ | H |
| $3\text{-}S(O)CH_3$ | $CH_3$ | $C(CH_3)_2CH_2SO_2CH_3$ | H |
| $3\text{-}SO_2CH_3$ | $CH_3$ | i-Pr | H |
| $3\text{-}SO_2CH_3$ | $CH_3$ | $CH(CH_3)CH_2SCH_3$ | H |
| $3\text{-}SO_2CH_3$ | $CH_3$ | $CH(CH_3)CH_2S(O)CH_3$ | H |
| $3\text{-}SO_2CH_3$ | $CH_3$ | $CH(CH_3)CH_2SO_2CH_3$ | H |
| $3\text{-}SO_2CH_3$ | $CH_3$ | $C(CH_3)_2CH_2SCH_3$ | H |
| $3\text{-}SO_2CH_3$ | $CH_3$ | $C(CH_3)_2CH_2S(O)CH_3$ | H |
| $3\text{-}SO_2CH_3$ | $CH_3$ | $C(CH_3)_2CH_2SO_2CH_3$ | H |
| $3\text{-}SCHF_2$ | $CH_3$ | i-Pr | H |
| $3\text{-}SCHF_2$ | $CH_3$ | $CH(CH_3)CH_2SCH_3$ | H |
| $3\text{-}SCHF_2$ | $CH_3$ | $CH(CH_3)CH_2S(O)CH_3$ | H |
| $3\text{-}SCHF_2$ | $CH_3$ | $CH(CH_3)CH_2SO_2CH_3$ | H |
| $3\text{-}SCHF_2$ | $CH_3$ | $C(CH_3)_2CH_2SCH_3$ | H |
| $3\text{-}SCHF_2$ | $CH_3$ | $C(CH_3)_2CH_2S(O)CH_3$ | H |
| $3\text{-}SCHF_2$ | $CH_3$ | $C(CH_3)_2CH_2SO_2CH_3$ | H |
| $3\text{-}S(O)CHF_2$ | $CH_3$ | i-Pr | H |
| $3\text{-}S(O)CHF_2$ | $CH_3$ | $CH(CH_3)CH_2SCH_3$ | H |
| $3\text{-}S(O)CHF_2$ | $CH_3$ | $CH(CH_3)CH_2S(O)CH_3$ | H |
| $3\text{-}S(O)CHF_2$ | $CH_3$ | $CH(CH_3)CH_2SO_2CH_3$ | H |
| $3\text{-}S(O)CHF_2$ | $CH_3$ | $C(CH_3)_2CH_2SCH_3$ | H |
| $3\text{-}S(O)CHF_2$ | $CH_3$ | $C(CH_3)_2CH_2S(O)CH_3$ | H |
| $3\text{-}S(O)CHF_2$ | $CH_3$ | $C(CH_3)_2CH_2SO_2CH_3$ | H |
| $3\text{-}SO_2CHF_2$ | $CH_3$ | i-Pr | H |
| $3\text{-}SO_2CHF_2$ | $CH_3$ | $CH(CH_3)CH_2SCH_3$ | H |
| $3\text{-}SO_2CHF_2$ | $CH_3$ | $CH(CH_3)CH_2S(O)CH_3$ | H |
| $3\text{-}SO_2CHF_2$ | $CH_3$ | $CH(CH_3)CH_2SO_2CH_3$ | H |
| $3\text{-}SO_2CHF_2$ | $CH_3$ | $C(CH_3)_2CH_2SCH_3$ | H |

| | | | |
|---|---|---|---|
| 3-$SO_2CHF_2$ | $CH_3$ | $C(CH_3)_2CH_2S(O)CH_3$ | H |
| 3-$SO_2CHF_2$ | $CH_3$ | $C(CH_3)_2CH_2SO_2CH_3$ | H |
| 3-$SCF_3$ | $CH_3$ | i-Pr | H |
| 3-$SCF_3$ | $CH_3$ | $CH(CH_3)CH_2SCH_3$ | H |
| 3-$SCF_3$ | $CH_3$ | $CH(CH_3)CH_2S(O)CH_3$ | H |
| 3-$SCF_3$ | $CH_3$ | $CH(CH_3)CH_2SO_2CH_3$ | H |
| 3-$SCF_3$ | $CH_3$ | $C(CH_3)_2CH_2SCH_3$ | H |
| 3-$SCF_3$ | $CH_3$ | $C(CH_3)_2CH_2S(O)CH_3$ | H |
| 3-$SCF_3$ | $CH_3$ | $C(CH_3)_2CH_2SO_2CH_3$ | H |
| 3-$S(O)CF_3$ | $CH_3$ | i-Pr | H |
| 3-$S(O)CF_3$ | $CH_3$ | $CH(CH_3)CH_2SCH_3$ | H |
| 3-$S(O)CF_3$ | $CH_3$ | $CH(CH_3)CH_2S(O)CH_3$ | H |
| 3-$S(O)CF_3$ | $CH_3$ | $CH(CH_3)CH_2SO_2CH_3$ | H |
| 3-$S(O)CF_3$ | $CH_3$ | $C(CH_3)_2CH_2SCH_3$ | H |
| 3-$S(O)CF_3$ | $CH_3$ | $C(CH_3)_2CH_2S(O)CH_3$ | H |
| 3-$S(O)CF_3$ | $CH_3$ | $C(CH_3)_2CH_2SO_2CH_3$ | H |
| 3-$SO_2CF_3$ | $CH_3$ | i-Pr | H |
| 3-$SO_2CF_3$ | $CH_3$ | $CH(CH_3)CH_2SCH_3$ | H |
| 3-$SO_2CF_3$ | $CH_3$ | $CH(CH_3)CH_2S(O)CH_3$ | H |
| 3-$SO_2CF_3$ | $CH_3$ | $CH(CH_3)CH_2SO_2CH_3$ | H |
| 3-$SO_2CF_3$ | $CH_3$ | $C(CH_3)_2CH_2SCH_3$ | H |
| 3-$SO_2CF_3$ | $CH_3$ | $C(CH_3)_2CH_2S(O)CH_3$ | H |
| 3-$SO_2CF_3$ | $CH_3$ | $C(CH_3)_2CH_2SO_2CH_3$ | H |
| 3-$NO_2$ | $CH_3$ | i-Pr | H |
| 3-$NO_2$ | $CH_3$ | $CH(CH_3)CH_2SCH_3$ | H |
| 3-$NO_2$ | $CH_3$ | $CH(CH_3)CH_2S(O)CH_3$ | H |
| 3-$NO_2$ | $CH_3$ | $CH(CH_3)CH_2SO_2CH_3$ | H |
| 3-$NO_2$ | $CH_3$ | $C(CH_3)_2CH_2SCH_3$ | H |
| 3-$NO_2$ | $CH_3$ | $C(CH_3)_2CH_2S(O)CH_3$ | H |
| 3-$NO_2$ | $CH_3$ | $C(CH_3)_2CH_2SO_2CH_3$ | H |

Table5

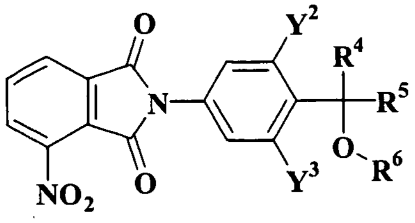

[4] - 1 ,

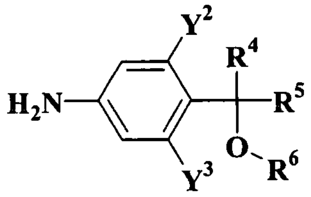

[4] - 2 ,

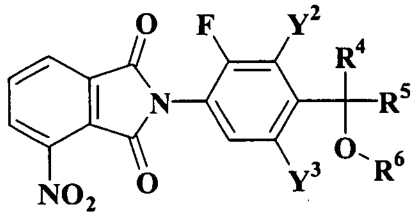

[4] - 3 ,

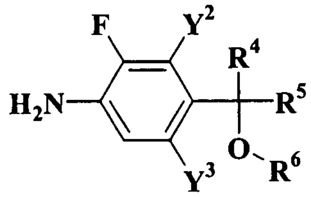

[4] - 4 ,

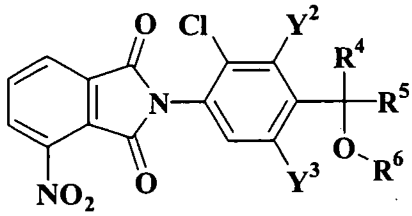

[4] - 5 ,

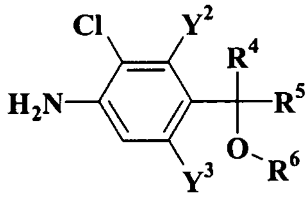

[4] - 6 ,

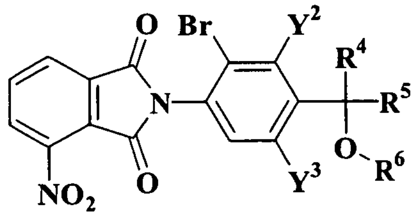

[4] - 7 ,

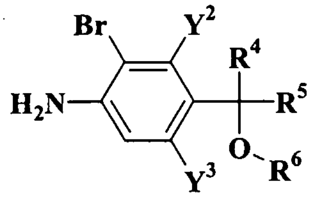

[4] - 8 ,

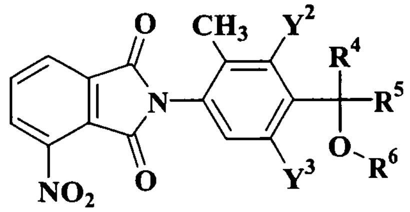

[4] - 9 ,

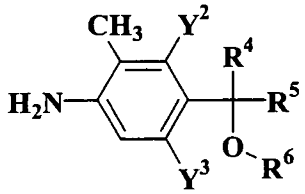

[4] - 10 ,

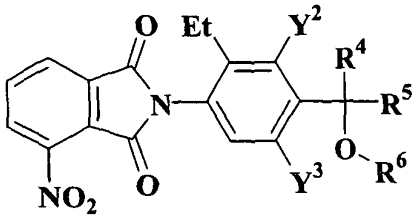

[4] - 11

or

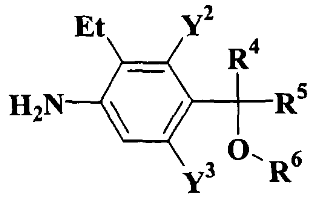

[4] - 12

| $R^4$ | $Y^2$ | $R^5$ | $Y^3$ | $R^6$ |
|---|---|---|---|---|
| $CH_3$ | H | $CF_2O$ (Ph-4-Cl) | H | H |
| $CH_3$ | H | CH=CH (Ph-4-Cl) | H | H |
| $CH_3$ | H | CH=CH (Ph-4-$OCF_3$) | H | H |
| $CH_3$ | H | CH=CH (Ph-3, 4-$Cl_2$) | H | H |
| $CH_3$ | H | Ph-4-F | H | H |
| $CH_3$ | H | Ph-4-Cl | H | H |
| $CH_3$ | H | Ph-4-Br | H | H |
| $CH_3$ | H | Ph-4-I | H | H |
| $CH_3$ | H | Ph-4-$CF_3$ | H | H |
| $CH_3$ | H | Ph-4-$OCHF_2$ | H | H |
| $CH_3$ | H | Ph-4-$OCF_3$ | H | H |
| $CH_3$ | H | Ph-4-$OCF_2Br$ | H | H |
| $CH_3$ | H | Ph-4-$OCF_2CHF_2$ | H | H |
| $CH_3$ | H | Ph-4-$OCF_2CHFCl$ | H | H |
| $CH_3$ | H | Ph-4-$OCF_2CHFCF_3$ | H | H |
| $CH_3$ | H | Ph-4-$OCF_2CHFOCF_3$ | H | H |
| $CH_3$ | H | Ph-4-$OCF_2CHFOCF_2CF_2CF_3$ | H | H |
| $CH_3$ | H | Ph-4-O (L-45g) | H | H |
| $CH_3$ | H | Ph-3, 4-$Cl_2$ | H | H |
| $CH_3$ | H | Ph (-3-$OCF_2O$-4-) | H | H |
| $CH_3$ | H | Ph (-3-$OCF_2CF_2O$-4-) | H | H |
| $CH_3$ | H | (L-45c) Cl | H | H |
| $CH_3$ | H | (L-45c) Br | H | H |
| $CH_3$ | H | (L-45c) $CF_3$ | H | H |
| $CH_3$ | H | L-45e | H | H |
| $CH_3$ | H | L-45f | H | H |
| $CH_3$ | H | (L-46c) Cl | H | H |
| $CH_3$ | H | (L-46c) Br | H | H |
| $CH_3$ | H | (L-46c) $CF_3$ | H | H |
| $CH_3$ | H | L-46d | H | H |

| | | | | |
|---|---|---|---|---|
| Et | H | $CF_2O$ (Ph-4-Cl) | H | H |
| Et | H | CH=CH (Ph-4-Cl) | H | H |
| Et | H | CH=CH (Ph-4-$OCF_3$) | H | H |
| Et | H | CH=CH (Ph-3, 4-$Cl_2$) | H | H |
| Et | H | Ph-4-Cl | H | H |
| Et | H | Ph-4-Br | H | H |
| Et | H | Ph-4-$CF_3$ | H | H |
| Et | H | Ph-4-$OCF_3$ | H | H |
| Et | H | Ph-4-$OCF_2Br$ | H | H |
| Et | H | Ph-4-$OCF_2CHFCF_3$ | H | H |
| Et | H | Ph-4-$OCF_2CHFOCF_3$ | H | H |
| Et | H | Ph-4-$OCF_2CHFOCF_2CF_2CF_3$ | H | H |
| Et | H | Ph-4-O (L-45g) | H | H |
| Et | H | Ph-3, 4-$Cl_2$ | H | H |
| Et | H | Ph (-3-$OCF_2O$-4-) | H | H |
| Et | H | Ph (-3-$OCF_2CF_2O$-4-) | H | H |
| Et | H | (L-45c) Br | H | H |
| Et | H | (L-45c) $CF_3$ | H | H |
| Et | H | L-45e | H | H |
| Et | H | L-45f | H | H |
| Et | H | (L-46c) Br | H | H |
| Et | H | (L-46c) $CF_3$ | H | H |
| Et | H | L-46d | H | H |
| n-Pr | H | Ph-4-Br | H | H |
| n-Pr | H | Ph-4-$CF_3$ | H | H |
| n-Pr | H | Ph-4-$OCF_3$ | H | H |
| n-Pr | H | (L-45c) $CF_3$ | H | H |
| i-Pr | H | Ph-4-Br | H | H |
| i-Pr | H | Ph-4-$CF_3$ | H | H |
| i-Pr | H | Ph-4-$OCF_3$ | H | H |
| i-Pr | H | (L-45c) $CF_3$ | H | H |
| c-Pr | H | Ph-4-Br | H | H |
| c-Pr | H | Ph-4-$CF_3$ | H | H |
| c-Pr | H | Ph-4-$OCF_3$ | H | H |
| c-Pr | H | (L-45c) $CF_3$ | H | H |
| n-Bu | H | Ph-4-Br | H | H |
| $CH_2F$ | H | Ph-4-$CF_3$ | H | H |
| $CH_2Cl$ | H | Ph-4-$OCF_3$ | H | H |
| $CH_2Br$ | H | (L-45c) $CF_3$ | H | H |
| $CHF_2$ | H | $CF_2O$ (Ph-4-Cl) | H | H |
| $CHF_2$ | H | CH=CH (Ph-4-Cl) | H | H |

| | | | | |
|---|---|---|---|---|
| $CHF_2$ | H | CH=CH (Ph-4-$OCF_3$) | H | H |
| $CHF_2$ | H | CH=CH (Ph-3, 4-$Cl_2$) | H | H |
| $CHF_2$ | H | Ph-4-F | H | H |
| $CHF_2$ | H | Ph-4-Cl | H | H |
| $CHF_2$ | H | Ph-4-Br | H | H |
| $CHF_2$ | H | Ph-4-I | H | H |
| $CHF_2$ | H | Ph-4-$CF_3$ | H | H |
| $CHF_2$ | H | Ph-4-$OCHF_2$ | H | H |
| $CHF_2$ | H | Ph-4-$OCF_3$ | H | H |
| $CHF_2$ | H | Ph-4-$OCF_2Br$ | H | H |
| $CHF_2$ | H | Ph-4-$OCF_2CHF_2$ | H | H |
| $CHF_2$ | H | Ph-4-$OCF_2CHFCl$ | H | H |
| $CHF_2$ | H | Ph-4-$OCF_2CHFCF_3$ | H | H |
| $CHF_2$ | H | Ph-4-$OCF_2CHFOCF_3$ | H | H |
| $CHF_2$ | H | Ph-4-$OCF_2CHFOCF_2CF_2CF_3$ | H | H |
| $CHF_2$ | H | Ph-4-O (L-45g) | H | H |
| $CHF_2$ | H | Ph-3, 4-$Cl_2$ | H | H |
| $CHF_2$ | H | Ph (-3-$OCF_2O$-4-) | H | H |
| $CHF_2$ | H | Ph (-3-$OCF_2CF_2O$-4-) | H | H |
| $CHF_2$ | H | (L-45c) Cl | H | H |
| $CHF_2$ | H | (L-45c) Br | H | H |
| $CHF_2$ | H | (L-45c) $CF_3$ | H | H |
| $CHF_2$ | H | L-45e | H | H |
| $CHF_2$ | H | L-45f | H | H |
| $CHF_2$ | H | (L-46c) Cl | H | H |
| $CHF_2$ | H | (L-46c) Br | H | H |
| $CHF_2$ | H | (L-46c) $CF_3$ | H | H |
| $CHF_2$ | H | L-46d | H | H |
| CHFCl | H | Ph-4-Br | H | H |
| CHFBr | H | Ph-4-$CF_3$ | H | H |
| $CF_3$ | H | $CH_2OCH_3$ | H | H |
| $CF_3$ | H | $CH_2OEt$ | H | H |
| $CF_3$ | H | $CH_2OPr$-n | H | H |
| $CF_3$ | H | $CH_2OPr$-i | H | H |
| $CF_3$ | H | $CH_2OBu$-n | H | H |
| $CF_3$ | H | $CH_2OCH_2CF_3$ | H | H |
| $CF_3$ | H | $CH_2OCH_2CF_2CF_3$ | H | H |
| $CF_3$ | H | $CH_2OCH(CF_3)_2$ | H | H |
| $CF_3$ | H | $CH_2OPh$ | H | H |
| $CF_3$ | H | $CH_2O$ (Ph-4-F) | H | H |
| $CF_3$ | H | $CH_2O$ (Ph-2-Cl) | H | H |

| | | | | |
|---|---|---|---|---|
| $CF_3$ | H | $CH_2O$ (Ph-3-Cl) | H | H |
| $CF_3$ | H | $CH_2O$ (Ph-4-Cl) | H | H |
| $CF_3$ | H | $CH_2O$ (Ph-4-Br) | H | H |
| $CF_3$ | H | $CH_2O$ (Ph-4-$CF_3$) | H | H |
| $CF_3$ | H | $CH_2O$ (Ph-2-$OCF_3$) | H | H |
| $CF_3$ | H | $CH_2O$ (Ph-3-$OCF_3$) | H | H |
| $CF_3$ | H | $CH_2O$ (Ph-4-$OCF_3$) | H | H |
| $CF_3$ | H | $CF_2OPh$ | H | H |
| $CF_3$ | H | $CF_2O$ (Ph-3-F) | H | H |
| $CF_3$ | H | $CF_2O$ (Ph-4-F) | H | H |
| $CF_3$ | H | $CF_2O$ (Ph-2-Cl) | H | H |
| $CF_3$ | H | $CF_2O$ (Ph-3-Cl) | H | H |
| $CF_3$ | H | $CF_2O$ (Ph-4-Cl) | H | H |
| $CF_3$ | H | $CF_2O$ (Ph-3-Br) | H | H |
| $CF_3$ | H | $CF_2O$ (Ph-4-Br) | H | H |
| $CF_3$ | H | $CF_2O$ (Ph-3-$CF_3$) | H | H |
| $CF_3$ | H | $CF_2O$ (Ph-4-$CF_3$) | H | H |
| $CF_3$ | H | $CF_2O$ (Ph-3-$OCF_3$) | H | H |
| $CF_3$ | H | $CF_2O$ (Ph-4-$OCF_3$) | H | H |
| $CF_3$ | H | $CH_2SPh$ | H | H |
| $CF_3$ | H | $CH_2S$ (Ph-3-Cl) | H | H |
| $CF_3$ | H | $CH_2S$ (Ph-4-Cl) | H | H |
| $CF_3$ | H | $CH_2SO_2$ (Ph-3-Cl) | H | H |
| $CF_3$ | H | $CH_2SO_2$ (Ph-4-Cl) | H | H |
| $CF_3$ | H | $CH_2N(CH_3)_2$ | H | H |
| $CF_3$ | H | $CH_2NHPh$ | H | H |
| $CF_3$ | H | $CH_2NH$ (Ph-3-Cl) | H | H |
| $CF_3$ | H | $CH_2NH$ (Ph-4-Cl) | H | H |
| $CF_3$ | H | $CF_2C(O)OEt$ | H | H |
| $CF_3$ | H | $CH_2$ (L-5a) | H | H |
| $CF_3$ | H | $CH_2$ (L-14a) | H | H |
| $CF_3$ | H | $CH_2$ (L-24a) | H | H |
| $CF_3$ | H | $CH_2$ (L-36a) | H | H |
| $CF_3$ | H | C(O)OEt | H | H |
| $CF_3$ | H | C(O)OBu-t | H | H |
| $CF_3$ | H | $C(O)OCH_2CF_3$ | H | H |
| $CF_3$ | H | CH=CHPh | H | H |
| $CF_3$ | H | CH=CH (Ph-3-F) | H | H |
| $CF_3$ | H | CH=CH (Ph-4-F) | H | H |
| $CF_3$ | H | CH=CH (Ph-2-Cl) | H | H |
| $CF_3$ | H | CH=CH (Ph-3-Cl) | H | H |

| | | | | |
|---|---|---|---|---|
| $CF_3$ | H | CH=CH (Ph-4-Cl) | H | H |
| $CF_3$ | H | CH=CH (Ph-3-Br) | H | H |
| $CF_3$ | H | CH=CH (Ph-4-Br) | H | H |
| $CF_3$ | H | CH=CH (Ph-3-$CF_3$) | H | H |
| $CF_3$ | H | CH=CH (Ph-4-$CF_3$) | H | H |
| $CF_3$ | H | CH=CH (Ph-3-$OCF_3$) | H | H |
| $CF_3$ | H | CH=CH (Ph-4-$OCF_3$) | H | H |
| $CF_3$ | H | CH=CH (Ph-3-$SCH_3$) | H | H |
| $CF_3$ | H | CH=CH (Ph-4-$SCH_3$) | H | H |
| $CF_3$ | H | CH=CH (Ph-3-$SO_2CH_3$) | H | H |
| $CF_3$ | H | CH=CH (Ph-4-$SO_2CH_3$) | H | H |
| $CF_3$ | H | CH=CH (Ph-3, 4-$F_2$) | H | H |
| $CF_3$ | H | CH=CH (Ph-3-F-4-Cl) | H | H |
| $CF_3$ | H | CH=CH (Ph-3, 4-$Cl_2$) | H | H |
| $CF_3$ | H | CH=CH (Ph-3, 4-$Br_2$) | H | H |
| $CF_3$ | H | CH=CH (Ph-3-F-4-$CF_3$) | H | H |
| $CF_3$ | H | CH=CH (Ph-3-Cl-4-$OCF_3$) | H | H |
| $CF_3$ | H | CH=CH [Ph (-3-$OCF_2O$-4-) ] | H | H |
| $CF_3$ | H | Ph | H | H |
| $CF_3$ | H | Ph-2-F | H | H |
| $CF_3$ | H | Ph-3-F | H | H |
| $CF_3$ | H | Ph-4-F | H | H |
| $CF_3$ | H | Ph-2-Cl | H | H |
| $CF_3$ | H | Ph-3-Cl | H | H |
| $CF_3$ | H | Ph-4-Cl | H | H |
| $CF_3$ | H | Ph-3-Br | H | H |
| $CF_3$ | H | Ph-4-Br | H | H |
| $CF_3$ | H | Ph-4-Br | H | $CH_3$ |
| $CF_3$ | H | Ph-4-Br | H | Et |
| $CF_3$ | H | Ph-4-Br | H | n-Pr |
| $CF_3$ | H | Ph-4-Br | H | i-Pr |
| $CF_3$ | H | Ph-4-Br | H | n-Bu |
| $CF_3$ | H | Ph-4-Br | H | $CH_2CF_3$ |
| $CF_3$ | H | Ph-4-Br | H | $CH_2OCH_3$ |
| $CF_3$ | H | Ph-4-Br | H | $CH_2OEt$ |
| $CF_3$ | H | Ph-4-Br | H | $CH_2SCH_3$ |
| $CF_3$ | H | Ph-4-Br | H | $CH_2CH=CH_2$ |
| $CF_3$ | H | Ph-4-Br | H | $CH_2CH=CF_2$ |
| $CF_3$ | H | Ph-4-Br | H | $CH_2CH=CCl_2$ |
| $CF_3$ | H | Ph-4-Br | H | $CH_2CF=CF_2$ |
| $CF_3$ | H | Ph-4-Br | H | $CH_2CCl=CCl_2$ |

| | | | | |
|---|---|---|---|---|
| $CF_3$ | H | Ph-4-Br | H | $CH_2C{\equiv}CH$ |
| $CF_3$ | H | Ph-4-Br | H | $CH_2Ph$ |
| $CF_3$ | H | Ph-4-Br | H | $Si(CH_3)_3$ |
| $CF_3$ | H | Ph-4-Br | H | $Si(CH_3)_2Bu$-t |
| $CF_3$ | H | Ph-4-Br | H | $Si(CH_3)_2Ph$ |
| $CF_3$ | H | Ph-4-I | H | H |
| $CF_3$ | H | Ph-4-Bu-t | H | H |
| $CF_3$ | H | Ph-3-$CF_3$ | H | H |
| $CF_3$ | H | Ph-4-$CF_3$ | H | H |
| $CF_3$ | H | Ph-4-$CF_3$ | H | $CH_3$ |
| $CF_3$ | H | Ph-4-$CF_3$ | H | Et |
| $CF_3$ | H | Ph-4-$CF_3$ | H | n-Pr |
| $CF_3$ | H | Ph-4-$CF_3$ | H | i-Pr |
| $CF_3$ | H | Ph-4-$CF_3$ | H | n-Bu |
| $CF_3$ | H | Ph-4-$CF_3$ | H | $CH_2CF_3$ |
| $CF_3$ | H | Ph-4-$CF_3$ | H | $CH_2OCH_3$ |
| $CF_3$ | H | Ph-4-$CF_3$ | H | $CH_2OEt$ |
| $CF_3$ | H | Ph-4-$CF_3$ | H | $CH_2SCH_3$ |
| $CF_3$ | H | Ph-4-$CF_3$ | H | $CH_2CH=CH_2$ |
| $CF_3$ | H | Ph-4-$CF_3$ | H | $CH_2CH=CF_2$ |
| $CF_3$ | H | Ph-4-$CF_3$ | H | $CH_2CH=CCl_2$ |
| $CF_3$ | H | Ph-4-$CF_3$ | H | $CH_2CF=CF_2$ |
| $CF_3$ | H | Ph-4-$CF_3$ | H | $CH_2CCl=CCl_2$ |
| $CF_3$ | H | Ph-4-$CF_3$ | H | $CH_2C{\equiv}CH$ |
| $CF_3$ | H | Ph-4-$CF_3$ | H | $CH_2Ph$ |
| $CF_3$ | H | Ph-4-$CF_3$ | H | $Si(CH_3)_3$ |
| $CF_3$ | H | Ph-4-$CF_3$ | H | $Si(CH_3)_2Bu$-t |
| $CF_3$ | H | Ph-4-$CF_3$ | H | $Si(CH_3)_2Ph$ |
| $CF_3$ | H | Ph-4-$OCH_3$ | H | H |
| $CF_3$ | H | Ph-4-$OCHF_2$ | H | H |
| $CF_3$ | H | Ph-3-$OCF_3$ | H | H |
| $CF_3$ | H | Ph-4-$OCF_3$ | H | H |
| $CF_3$ | H | Ph-4-$OCF_3$ | H | $CH_3$ |
| $CF_3$ | H | Ph-4-$OCF_3$ | H | Et |
| $CF_3$ | H | Ph-4-$OCF_3$ | H | n-Pr |
| $CF_3$ | H | Ph-4-$OCF_3$ | H | i-Pr |
| $CF_3$ | H | Ph-4-$OCF_3$ | H | n-Bu |
| $CF_3$ | H | Ph-4-$OCF_3$ | H | $CH_2CF_3$ |
| $CF_3$ | H | Ph-4-$OCF_3$ | H | $CH_2OCH_3$ |
| $CF_3$ | H | Ph-4-$OCF_3$ | H | $CH_2OEt$ |
| $CF_3$ | H | Ph-4-$OCF_3$ | H | $CH_2SCH_3$ |

| | | | | |
|---|---|---|---|---|
| $CF_3$ | H | Ph-4-$OCF_3$ | H | $CH_2CH=CH_2$ |
| $CF_3$ | H | Ph-4-$OCF_3$ | H | $CH_2CH=CF_2$ |
| $CF_3$ | H | Ph-4-$OCF_3$ | H | $CH_2CH=CCl_2$ |
| $CF_3$ | H | Ph-4-$OCF_3$ | H | $CH_2CF=CF_2$ |
| $CF_3$ | H | Ph-4-$OCF_3$ | H | $CH_2CCl=CCl_2$ |
| $CF_3$ | H | Ph-4-$OCF_3$ | H | $CH_2C\equiv CH$ |
| $CF_3$ | H | Ph-4-$OCF_3$ | H | $CH_2Ph$ |
| $CF_3$ | H | Ph-4-$OCF_3$ | H | $Si(CH_3)_3$ |
| $CF_3$ | H | Ph-4-$OCF_3$ | H | $Si(CH_3)_2$Bu-t |
| $CF_3$ | H | Ph-4-$OCF_3$ | H | $Si(CH_3)_2$Ph |
| $CF_3$ | H | Ph-4-$OCF_2Br$ | H | H |
| $CF_3$ | H | Ph-4-$OCH_2CF_3$ | H | H |
| $CF_3$ | H | Ph-4-$OCF_2CHF_2$ | H | H |
| $CF_3$ | H | Ph-4-$OCF_2CHFCl$ | H | H |
| $CF_3$ | H | Ph-4-$OCF_2CHFBr$ | H | H |
| $CF_3$ | H | Ph-4-$OCF_2CF_2Br$ | H | H |
| $CF_3$ | H | Ph-4-$OCF_2CFCl_2$ | H | H |
| $CF_3$ | H | Ph-4-$OCF_2CCl_3$ | H | H |
| $CF_3$ | H | Ph-4-$OCH_2CF_2CHF_2$ | H | H |
| $CF_3$ | H | Ph-3-$OCF_2CHFCF_3$ | H | H |
| $CF_3$ | H | Ph-4-$OCF_2CHFCF_3$ | H | H |
| $CF_3$ | H | Ph-4-$OCH(CF_3)_2$ | H | H |
| $CF_3$ | H | Ph-4-$OCF_2CFBrCF_3$ | H | H |
| $CF_3$ | H | Ph-3-$OCF_2CHFOCF_3$ | H | H |
| $CF_3$ | H | Ph-4-$OCF_2CHFOCF_3$ | H | H |
| $CF_3$ | H | Ph-4-$OCF_2CHFOCF_2CF_2CF_3$ | H | H |
| $CF_3$ | H | Ph-4-$OSO_2CH_3$ | H | H |
| $CF_3$ | H | Ph-4-$OSO_2CF_3$ | H | H |
| $CF_3$ | H | Ph-3-O(Ph-4-Cl) | H | H |
| $CF_3$ | H | Ph-4-O(Ph-4-Cl) | H | H |
| $CF_3$ | H | Ph-4-O(Ph-4-Br) | H | H |
| $CF_3$ | H | Ph-4-O(Ph-4-$CF_3$) | H | H |
| $CF_3$ | H | Ph-4-O(L-45c)Br | H | H |
| $CF_3$ | H | Ph-4-O(L-45c)$CF_3$ | H | H |
| $CF_3$ | H | Ph-3-O(L-45e) | H | H |
| $CF_3$ | H | Ph-4-O(L-45e) | H | H |
| $CF_3$ | H | Ph-4-O(L-48b)Br | H | H |
| $CF_3$ | H | Ph-4-$SCH_3$ | H | H |
| $CF_3$ | H | Ph-4-$SO_2CH_3$ | H | H |
| $CF_3$ | H | Ph-3-S(Ph-4-Cl) | H | H |
| $CF_3$ | H | Ph-4-S(Ph-4-Cl) | H | H |

| | | | | |
|---|---|---|---|---|
| $CF_3$ | H | Ph-4-S (Ph-4-Br) | H | H |
| $CF_3$ | H | Ph-4-S (Ph-4-$CF_3$) | H | H |
| $CF_3$ | H | Ph-4-S (L-45c) Br | H | H |
| $CF_3$ | H | Ph-4-S (L-45c) $CF_3$ | H | H |
| $CF_3$ | H | Ph-4-S (L-45e) | H | H |
| $CF_3$ | H | Ph-4-S (L-48b) Br | H | H |
| $CF_3$ | H | Ph-4-$NO_2$ | H | H |
| $CF_3$ | H | Ph-4-CN | H | H |
| $CF_3$ | H | Ph-2, 3-$F_2$ | H | H |
| $CF_3$ | H | Ph-2, 4-$F_2$ | H | H |
| $CF_3$ | H | Ph-3, 4-$F_2$ | H | H |
| $CF_3$ | H | Ph-2, 5-$F_2$ | H | H |
| $CF_3$ | H | Ph-3, 5-$F_2$ | H | H |
| $CF_3$ | H | Ph-2-Cl-4-F | H | H |
| $CF_3$ | H | Ph-2-F-3-Cl | H | H |
| $CF_3$ | H | Ph-3-Cl-4-F | H | H |
| $CF_3$ | H | Ph-2-F-4-Cl | H | H |
| $CF_3$ | H | Ph-3-F-4-Cl | H | H |
| $CF_3$ | H | Ph-2, 3-$Cl_2$ | H | H |
| $CF_3$ | H | Ph-2, 4-$Cl_2$ | H | H |
| $CF_3$ | H | Ph-2, 5-$Cl_2$ | H | H |
| $CF_3$ | H | Ph-3, 4-$Cl_2$ | H | H |
| $CF_3$ | H | Ph-3, 5-$Cl_2$ | H | H |
| $CF_3$ | H | Ph-3-Br-4-F | H | H |
| $CF_3$ | H | Ph-2-F-4-Br | H | H |
| $CF_3$ | H | Ph-2-F-5-Br | H | H |
| $CF_3$ | H | Ph-3, 4-$Br_2$ | H | H |
| $CF_3$ | H | Ph-3, 5-$Br_2$ | H | H |
| $CF_3$ | H | Ph-3-$CH_3$-4-F | H | H |
| $CF_3$ | H | Ph-3-F-4-$CH_3$ | H | H |
| $CF_3$ | H | Ph-2-F-5-$CH_3$ | H | H |
| $CF_3$ | H | Ph-2, 4- $(CH_3)_2$ | H | H |
| $CF_3$ | H | Ph-3, 4- $(CH_3)_2$ | H | H |
| $CF_3$ | H | Ph-2-F-3-$CF_3$ | H | H |
| $CF_3$ | H | Ph-3-$CF_3$-4-F | H | H |
| $CF_3$ | H | Ph-3-$CF_3$-4-Cl | H | H |
| $CF_3$ | H | Ph-2-F-4-$CF_3$ | H | H |
| $CF_3$ | H | Ph-3-F-4-$CF_3$ | H | H |
| $CF_3$ | H | Ph-2-F-5-$CF_3$ | H | H |
| $CF_3$ | H | Ph-3-F-5-$CF_3$ | H | H |
| $CF_3$ | H | Ph-2-Cl-4-$CF_3$ | H | H |

| | | | | |
|---|---|---|---|---|
| $CF_3$ | H | Ph-3, 5-$(CF_3)_2$ | H | H |
| $CF_3$ | H | Ph-3-Br-4-$OCH_3$ | H | H |
| $CF_3$ | H | Ph-3-F-4-$OCHF_2$ | H | H |
| $CF_3$ | H | Ph-3-Cl-4-$OCHF_2$ | H | H |
| $CF_3$ | H | Ph-3-Br-4-$OCHF_2$ | H | H |
| $CF_3$ | H | Ph-3-F-4-$OCF_3$ | H | H |
| $CF_3$ | H | Ph-3-Cl-4-$OCF_3$ | H | H |
| $CF_3$ | H | Ph-3-Br-4-$OCF_3$ | H | H |
| $CF_3$ | H | Ph-3-F-4-$OCF_2Br$ | H | H |
| $CF_3$ | H | Ph-3-Cl-4-$OCF_2Br$ | H | H |
| $CF_3$ | H | Ph-3-Br-4-$OCF_2Br$ | H | H |
| $CF_3$ | H | Ph-3-F-4-$OCF_2CHF_2$ | H | H |
| $CF_3$ | H | Ph-3-Cl-4-$OCF_2CHF_2$ | H | H |
| $CF_3$ | H | Ph-3-Br-4-$OCF_2CHF_2$ | H | H |
| $CF_3$ | H | Ph-3-F-4-$OCF_2CHFCl$ | H | H |
| $CF_3$ | H | Ph-3-Cl-4-$OCF_2CHFCl$ | H | H |
| $CF_3$ | H | Ph-3-Br-4-$OCF_2CHFCl$ | H | H |
| $CF_3$ | H | Ph-3-F-4-$OCF_2CHFCF_3$ | H | H |
| $CF_3$ | H | Ph-3-Cl-4-$OCF_2CHFCF_3$ | H | H |
| $CF_3$ | H | Ph-3-Br-4-$OCF_2CHFCF_3$ | H | H |
| $CF_3$ | H | Ph-3-F-4-$OCF_2CHFOCF_3$ | H | H |
| $CF_3$ | H | Ph-3-Cl-4-$OCF_2CHFOCF_3$ | H | H |
| $CF_3$ | H | Ph-3-Br-4-$OCF_2CHFOCF_3$ | H | H |
| $CF_3$ | H | Ph-3-$CH_3$-4-$OCF_2CHFOCF_3$ | H | H |
| $CF_3$ | H | Ph-3-F-4-$OCF_2CHFOCF_2CF_2CF_3$ | H | H |
| $CF_3$ | H | Ph-3-Cl-4-$OCF_2CHFOCF_2CF_2CF_3$ | H | H |
| $CF_3$ | H | Ph-3-Br-4-$OCF_2CHFOCF_2CF_2CF_3$ | H | H |
| $CF_3$ | H | Ph(-3-$OCF_2O$-4-) | H | H |
| $CF_3$ | H | Ph(-3-$OCF_2CF_2O$-4-) | H | H |
| $CF_3$ | H | Ph-3-OPh-4-F | H | H |
| $CF_3$ | H | Ph-3-$NO_2$-4-F | H | H |
| $CF_3$ | H | Ph-3-$NO_2$-4-Cl | H | H |
| $CF_3$ | H | Ph-2-F-5-$NO_2$ | H | H |
| $CF_3$ | H | Ph-3-CN-4-F | H | H |
| $CF_3$ | H | Ph-2, 3, 4-$F_3$ | H | H |
| $CF_3$ | H | Ph-2, 3, 5-$F_3$ | H | H |
| $CF_3$ | H | Ph-2, 4, 5-$F_3$ | H | H |
| $CF_3$ | H | Ph-3, 4, 5-$F_3$ | H | H |
| $CF_3$ | H | Ph-2, 3-$F_2$-4-$CH_3$ | H | H |
| $CF_3$ | H | Ph-2, 3-$F_2$-4-$CF_3$ | H | H |
| $CF_3$ | H | Ph-3, 4-$F_2$-5-$CF_3$ | H | H |

| | | | | |
|---|---|---|---|---|
| $CF_3$ | H | Ph-2-F-3-Cl-5-$CF_3$ | H | H |
| $CF_3$ | H | Ph-3, 5-$Cl_2$-4-$OCH_3$ | H | H |
| $CF_3$ | H | 1-Naph | H | H |
| $CF_3$ | H | 2-Naph | H | H |
| $CF_3$ | H | (L-1b) Br | H | H |
| $CF_3$ | H | (L-1c) Cl | H | H |
| $CF_3$ | H | (L-1c) Br | H | H |
| $CF_3$ | H | (L-1c) I | H | H |
| $CF_3$ | H | (L-1c) $CF_3$ | H | H |
| $CF_3$ | H | (L-2b) Br | H | H |
| $CF_3$ | H | (L-3b) Cl | H | H |
| $CF_3$ | H | (L-3b) Br | H | H |
| $CF_3$ | H | (L-3c) F | H | H |
| $CF_3$ | H | (L-3c) Cl | H | H |
| $CF_3$ | H | (L-3c) Br | H | H |
| $CF_3$ | H | (L-3c) I | H | H |
| $CF_3$ | H | (L-3c) $CF_3$ | H | H |
| $CF_3$ | H | (L-3c) CN | H | H |
| $CF_3$ | H | L-3d | H | H |
| $CF_3$ | H | (L-4b) Cl | H | H |
| $CF_3$ | H | (L-4b) Br | H | H |
| $CF_3$ | H | (L-4b) CN | H | H |
| $CF_3$ | H | (L-10b) Cl | H | H |
| $CF_3$ | H | (L-10b) Br | H | H |
| $CF_3$ | H | (L-15b) $CF_3$ | H | H |
| $CF_3$ | H | (L-16a) $CHF_2$ | H | H |
| $CF_3$ | H | (L-16a) $CF_2Br$ | H | H |
| $CF_3$ | H | (L-17a) Cl | H | H |
| $CF_3$ | H | (L-21b) Cl | H | H |
| $CF_3$ | H | (L-21b) Br | H | H |
| $CF_3$ | H | (L-21b) I | H | H |
| $CF_3$ | H | (L-21b) $CF_3$ | H | H |
| $CF_3$ | H | (L-22b) Cl | H | H |
| $CF_3$ | H | (L-22b) Br | H | H |
| $CF_3$ | H | (L-23b) Cl | H | H |
| $CF_3$ | H | (L-23b) Br | H | H |
| $CF_3$ | H | (L-23c) Cl | H | H |
| $CF_3$ | H | (L-23c) Br | H | H |
| $CF_3$ | H | (L-31a) Cl | H | H |
| $CF_3$ | H | (L-31a) Br | H | H |
| $CF_3$ | H | (L-45c) F | H | H |

| | | | | |
|---|---|---|---|---|
| $CF_3$ | H | (L-45c) Cl | H | H |
| $CF_3$ | H | (L-45c) Br | H | H |
| $CF_3$ | H | (L-45c) I | H | H |
| $CF_3$ | H | (L-45c) $CF_3$ | H | H |
| $CF_3$ | H | (L-45c) $CF_3$ | H | $CH_3$ |
| $CF_3$ | H | (L-45c) $CF_3$ | H | Et |
| $CF_3$ | H | (L-45c) $CF_3$ | H | n-Pr |
| $CF_3$ | H | (L-45c) $CF_3$ | H | i-Pr |
| $CF_3$ | H | (L-45c) $CF_3$ | H | n-Bu |
| $CF_3$ | H | (L-45c) $CF_3$ | H | $CH_2CF_3$ |
| $CF_3$ | H | (L-45c) $CF_3$ | H | $CH_2OCH_3$ |
| $CF_3$ | H | (L-45c) $CF_3$ | H | $CH_2OEt$ |
| $CF_3$ | H | (L-45c) $CF_3$ | H | $CH_2SCH_3$ |
| $CF_3$ | H | (L-45c) $CF_3$ | H | $CH_2CH=CH_2$ |
| $CF_3$ | H | (L-45c) $CF_3$ | H | $CH_2CH=CF_2$ |
| $CF_3$ | H | (L-45c) $CF_3$ | H | $CH_2CH=CCl_2$ |
| $CF_3$ | H | (L-45c) $CF_3$ | H | $CH_2CF=CF_2$ |
| $CF_3$ | H | (L-45c) $CF_3$ | H | $CH_2CCl=CCl_2$ |
| $CF_3$ | H | (L-45c) $CF_3$ | H | $CH_2C\equiv CH$ |
| $CF_3$ | H | (L-45c) $CF_3$ | H | $CH_2Ph$ |
| $CF_3$ | H | (L-45c) $CF_3$ | H | $Si(CH_3)_3$ |
| $CF_3$ | H | (L-45c) $CF_3$ | H | $Si(CH_3)_2Bu$-t |
| $CF_3$ | H | (L-45c) $CF_3$ | H | $Si(CH_3)_2Ph$ |
| $CF_3$ | H | L-45e | H | H |
| $CF_3$ | H | L-45f | H | H |
| $CF_3$ | H | (L-46c) F | H | H |
| $CF_3$ | H | (L-46c) Cl | H | H |
| $CF_3$ | H | (L-46c) Br | H | H |
| $CF_3$ | H | (L-46c) I | H | H |
| $CF_3$ | H | (L-46c) $CF_3$ | H | H |
| $CF_3$ | H | (L-46c) $OCH_2CF_3$ | H | H |
| $CF_3$ | H | (L-46c) $OCH(CF_3)_2$ | H | H |
| $CF_3$ | H | L-46d | H | H |
| $CF_3$ | H | L-47a | H | H |
| $CF_3$ | H | L-47d | H | H |
| $CF_3$ | H | (L-48b) Br | H | H |
| $CF_3$ | H | (L-50b) Cl | H | H |
| $CF_3$ | H | (L-50b) Br | H | H |
| $CF_3$ | H | (L-51b) Cl | H | H |
| $CF_3$ | $-OCF_2-$ | | H | H |
| $CF_3$ | $-OCF_2-$ | | H | $CH_3$ |

| | | | | |
|---|---|---|---|---|
| $CF_3$ | $-OCF_2-$ | | H | $CH_2OCH_3$ |
| $CF_3$ | $-OCF_2-$ | | H | $Si(CH_3)_3$ |
| $CF_3$ | H | $-CF_2O-$ | | H |
| $CF_3$ | H | $-CF_2O-$ | | $CH_3$ |
| $CF_3$ | H | $-CF_2O-$ | | $CH_2OCH_3$ |
| $CF_3$ | H | $-CF_2O-$ | | $Si(CH_3)_3$ |
| $CF_2Cl$ | H | $CF_2O(Ph-4-Cl)$ | H | H |
| $CF_2Cl$ | H | $CH=CH(Ph-4-Cl)$ | H | H |
| $CF_2Cl$ | H | $CH=CH(Ph-4-OCF_3)$ | H | H |
| $CF_2Cl$ | H | $CH=CH(Ph-3,4-Cl_2)$ | H | H |
| $CF_2Cl$ | H | Ph-4-F | H | H |
| $CF_2Cl$ | H | Ph-4-Cl | H | H |
| $CF_2Cl$ | H | Ph-4-Br | H | H |
| $CF_2Cl$ | H | Ph-4-I | H | H |
| $CF_2Cl$ | H | $Ph-4-CF_3$ | H | H |
| $CF_2Cl$ | H | $Ph-4-OCHF_2$ | H | H |
| $CF_2Cl$ | H | $Ph-4-OCF_3$ | H | H |
| $CF_2Cl$ | H | $Ph-4-OCF_2Br$ | H | H |
| $CF_2Cl$ | H | $Ph-4-OCF_2CHF_2$ | H | H |
| $CF_2Cl$ | H | $Ph-4-OCF_2CHFCl$ | H | H |
| $CF_2Cl$ | H | $Ph-4-OCF_2CHFCF_3$ | H | H |
| $CF_2Cl$ | H | $Ph-4-OCF_2CHFOCF_3$ | H | H |
| $CF_2Cl$ | H | $Ph-4-OCF_2CHFOCF_2CF_2CF_3$ | H | H |
| $CF_2Cl$ | H | Ph-4-O(L-45g) | H | H |
| $CF_2Cl$ | H | $Ph-3,4-Cl_2$ | H | H |
| $CF_2Cl$ | H | $Ph(-3-OCF_2O-4-)$ | H | H |
| $CF_2Cl$ | H | $Ph(-3-OCF_2CF_2O-4$ | H | H |
| $CF_2Cl$ | H | (L-45c)Cl | H | H |
| $CF_2Cl$ | H | (L-45c)Br | H | H |
| $CF_2Cl$ | H | $(L-45c)CF_3$ | H | H |
| $CF_2Cl$ | H | L-45e | H | H |
| $CF_2Cl$ | H | L-45f | H | H |
| $CF_2Cl$ | H | (L-46c)Cl | H | H |
| $CF_2Cl$ | H | (L-46c)Br | H | H |
| $CF_2Cl$ | H | $(L-46c)CF_3$ | H | H |
| $CF_2Cl$ | H | L-46d | H | H |
| $CFCl_2$ | H | $Ph-4-OCF_3$ | H | H |
| $CF_2Br$ | H | Ph-4-Cl | H | H |
| $CF_2Br$ | H | Ph-4-Br | H | H |
| $CF_2Br$ | H | $Ph-4-CF_3$ | H | H |
| $CF_2Br$ | H | $Ph-4-OCF_3$ | H | H |

| | | | | |
|---|---|---|---|---|
| $CF_2Br$ | H | Ph-4-$OCF_2Br$ | H | H |
| $CF_2Br$ | H | Ph-4-$OCF_2CHFCF_3$ | H | H |
| $CF_2Br$ | H | Ph-4-$OCF_2CHFOCF_3$ | H | H |
| $CF_2Br$ | H | Ph-4-$OCF_2CHFOCF_2CF_2CF_3$ | H | H |
| $CF_2Br$ | H | Ph-4-O (L-45g) | H | H |
| $CF_2Br$ | H | Ph-3, 4-$Cl_2$ | H | H |
| $CF_2Br$ | H | Ph (-3-$OCF_2O$-4-) | H | H |
| $CF_2Br$ | H | Ph (-3-$OCF_2CF_2O$-4-) | H | H |
| $CF_2Br$ | H | (L-45c) $CF_3$ | H | H |
| $CF_2Br$ | H | L-45f | H | H |
| $CF_2Br$ | H | (L-46c) $CF_3$ | H | H |
| $CF_2Br$ | H | L-46d | H | H |
| CFClBr | H | (L-45c) $CF_3$ | H | H |
| $CFBr_2$ | H | Ph-4-Br | H | H |
| $CF_2CHF_2$ | H | Ph-4-$CF_3$ | H | H |
| $CF_2CF_3$ | H | $CF_2O$ (Ph-4-Cl) | H | H |
| $CF_2CF_3$ | H | CH=CH (Ph-4-Cl) | H | H |
| $CF_2CF_3$ | H | CH=CH (Ph-4-$OCF_3$) | H | H |
| $CF_2CF_3$ | H | CH=CH (Ph-3, 4-$Cl_2$) | H | H |
| $CF_2CF_3$ | H | Ph-4-F | H | H |
| $CF_2CF_3$ | H | Ph-4-Cl | H | H |
| $CF_2CF_3$ | H | Ph-4-Br | H | H |
| $CF_2CF_3$ | H | Ph-4-I | H | H |
| $CF_2CF_3$ | H | Ph-4-$CF_3$ | H | H |
| $CF_2CF_3$ | H | Ph-4-$OCHF_2$ | H | H |
| $CF_2CF_3$ | H | Ph-4-$OCF_3$ | H | H |
| $CF_2CF_3$ | H | Ph-4-$OCF_2Br$ | H | H |
| $CF_2CF_3$ | H | Ph-4-$OCF_2CHF_2$ | H | H |
| $CF_2CF_3$ | H | Ph-4-$OCF_2CHFCl$ | H | H |
| $CF_2CF_3$ | H | Ph-4-$OCF_2CHFCF_3$ | H | H |
| $CF_2CF_3$ | H | Ph-4-$OCF_2CHFOCF_3$ | H | H |
| $CF_2CF_3$ | H | Ph-4-$OCF_2CHFOCF_2CF_2CF_3$ | H | H |
| $CF_2CF_3$ | H | Ph-4-O (L-45g) | H | H |
| $CF_2CF_3$ | H | Ph-3, 4-$Cl_2$ | H | H |
| $CF_2CF_3$ | H | Ph (-3-$OCF_2O$-4-) | H | H |
| $CF_2CF_3$ | H | Ph (-3-$OCF_2CF_2O$-4-) | H | H |
| $CF_2CF_3$ | H | (L-45c) Cl | H | H |
| $CF_2CF_3$ | H | (L-45c) Br | H | H |
| $CF_2CF_3$ | H | (L-45c) $CF_3$ | H | H |
| $CF_2CF_3$ | H | L-45e | H | H |
| $CF_2CF_3$ | H | L-45f | H | H |

| | | | | |
|---|---|---|---|---|
| $CF_2CF_3$ | H | (L-46c) Cl | H | H |
| $CF_2CF_3$ | H | (L-46c) Br | H | H |
| $CF_2CF_3$ | H | (L-46c) $CF_3$ | H | H |
| $CF_2CF_3$ | H | L-46d | H | H |
| $CF_2CF_2Cl$ | H | Ph-4-$OCF_3$ | H | H |
| $CFClCF_3$ | H | (L-45c) $CF_3$ | H | H |
| $CFClCF_2Cl$ | H | Ph-4-Br | H | H |
| $CF_2CF_2Br$ | H | Ph-4-$CF_3$ | H | H |
| $CFBrCF_3$ | H | Ph-4-$OCF_3$ | H | H |
| $CF_2CHFCF_3$ | H | (L-45c) $CF_3$ | H | H |
| $CF_2CF_2CF_3$ | H | Ph-4-Cl | H | H |
| $CF_2CF_2CF_3$ | H | Ph-4-Br | H | H |
| $CF_2CF_2CF_3$ | H | Ph-4-$CF_3$ | H | H |
| $CF_2CF_2CF_3$ | H | Ph-4-$OCF_3$ | H | H |
| $CF_2CF_2CF_3$ | H | Ph-4-$OCF_2Br$ | H | H |
| $CF_2CF_2CF_3$ | H | Ph-4-$OCF_2CHFCF_3$ | H | H |
| $CF_2CF_2CF_3$ | H | Ph-4-$OCF_2CHFOCF_3$ | H | H |
| $CF_2CF_2CF_3$ | H | Ph-4-$OCF_2CHFOCF_2CF_2CF_3$ | H | H |
| $CF_2CF_2CF_3$ | H | Ph-4-O (L-45g) | H | H |
| $CF_2CF_2CF_3$ | H | Ph-3, 4-$Cl_2$ | H | H |
| $CF_2CF_2CF_3$ | H | Ph (-3-$OCF_2O$-4-) | H | H |
| $CF_2CF_2CF_3$ | H | Ph (-3-$OCF_2CF_2O$-4-) | H | H |
| $CF_2CF_2CF_3$ | H | (L-45c) $CF_3$ | H | H |
| $CF_2CF_2CF_3$ | H | L-45f | H | H |
| $CF_2CF_2CF_3$ | H | (L-46c) $CF_3$ | H | H |
| $CF_2CF_2CF_3$ | H | L-46d | H | H |
| $CF(CF_3)_2$ | H | Ph-4-Br | H | H |
| $CF_2CFClCF_2Cl$ | H | Ph-4-$CF_3$ | H | H |
| $CF_2CFBrCF_2Cl$ | H | Ph-4-$OCF_3$ | H | H |
| $CF_2CF_2CF_2CHF_2$ | H | (L-45c) $CF_3$ | H | H |
| $CF_2CF_2CF_2CF_3$ | H | Ph-4-Br | H | H |
| $CF(CF_3)CF_2CF_3$ | H | Ph-4-$CF_3$ | H | H |
| $CF_2CF_2CF_2CF_2Cl$ | H | Ph-4-$OCF_3$ | H | H |
| T-1 | H | (L-45c) $CF_3$ | H | H |
| T-2 | H | Ph-4-Br | H | H |
| $CF_2OCH_3$ | H | Ph-4-Cl | H | H |
| $CF_2OCH_3$ | H | Ph-4-Br | H | H |
| $CF_2OCH_3$ | H | Ph-4-$CF_3$ | H | H |
| $CF_2OCH_3$ | H | Ph-4-$OCF_3$ | H | H |
| $CF_2OCH_3$ | H | Ph-4-$OCF_2Br$ | H | H |
| $CF_2OCH_3$ | H | Ph-4-$OCF_2CHFCF_3$ | H | H |

| | | | | |
|---|---|---|---|---|
| $CF_2OCH_3$ | H | Ph-4-$OCF_2CHFOCF_3$ | H | H |
| $CF_2OCH_3$ | H | Ph-4-$OCF_2CHFOCF_2CF_2CF_3$ | H | H |
| $CF_2OCH_3$ | H | Ph-4-O (L-45g) | H | H |
| $CF_2OCH_3$ | H | Ph-3, 4-$Cl_2$ | H | H |
| $CF_2OCH_3$ | H | Ph (-3-$OCF_2O$-4-) | H | H |
| $CF_2OCH_3$ | H | Ph (-3-$OCF_2CF_2O$-4-) | H | H |
| $CF_2OCH_3$ | H | (L-45c) $CF_3$ | H | H |
| $CF_2OCH_3$ | H | L-45f | H | H |
| $CF_2OCH_3$ | H | (L-46c) $CF_3$ | H | H |
| $CF_2OCH_3$ | H | L-46d | H | H |
| $CF_2SCH_3$ | H | Ph-4-Cl | H | H |
| $CF_2SCH_3$ | H | Ph-4-Br | H | H |
| $CF_2SCH_3$ | H | Ph-4-$CF_3$ | H | H |
| $CF_2SCH_3$ | H | Ph-4-$OCF_3$ | H | H |
| $CF_2SCH_3$ | H | Ph-4-$OCF_2Br$ | H | H |
| $CF_2SCH_3$ | H | Ph-4-$OCF_2CHFCF_3$ | H | H |
| $CF_2SCH_3$ | H | Ph-4-$OCF_2CHFOCF_3$ | H | H |
| $CF_2SCH_3$ | H | Ph-4-$OCF_2CHFOCF_2CF_2CF_3$ | H | H |
| $CF_2SCH_3$ | H | Ph-4-O (L-45g) | H | H |
| $CF_2SCH_3$ | H | Ph-3, 4-$Cl_2$ | H | H |
| $CF_2SCH_3$ | H | Ph (-3-$OCF_2O$-4-) | H | H |
| $CF_2SCH_3$ | H | Ph (-3-$OCF_2CF_2O$-4-) | H | H |
| $CF_2SCH_3$ | H | (L-45c) $CF_3$ | H | H |
| $CF_2SCH_3$ | H | L-45f | H | H |
| $CF_2SCH_3$ | H | (L-46c) $CF_3$ | H | H |
| $CF_2SCH_3$ | H | L-46d | H | H |
| $CF_2SPr$-n | H | Ph-4-$CF_3$ | H | H |
| $CF_2SPr$-I | H | Ph-4-$OCF_3$ | H | H |

The compound of the present invention can prevent from and exterminate either of harmful insects with a low concentration such as the so-called agricultural harmful insects which injure agricultural and horticultural crops and trees, the so-called harmful insects against domestic animals which are parasitic on domestic animals and domestic dowls, the so-called hygiene harmful insects which provide various bad influences on a human life environment such as houses, etc., the so-called harmful insects against stored grains which injure grains stored in a storehouse, and mites, nematodes, mollusks and crustaceans which generate in the same situation and injure.

In the insects, mites, nematodes, molluscs and crustaceans which can be prevented and exterminated by using the compound of the present invention, there may be specifically mentioned, for example,

Lepidoptera harmful insects such as diamondback moth (Plutella xylostella), black cutworm (Agrotis ipsilon), Turnip moth (Agrotis segetum), com earworm (Helicoverpa armigera), Oriental tobacco budworm (Helicoverpa assulta), cotton bollworm (Helicoverpa zea), tabacco budworm (Heliothis virescens), cabbage armyworm (Mamestra brassicae), green rice caterpillar (Naranga aenescens), beet semilooper (Autographa nigrisigna), rice armyworm (Mythimna separata), beet armyworm (Spodoptera exigua), common cutworm (Spodoptera litura), cotton leafworm (Spodoptera littoralis), fall armyworm (Spodoptera frugiperda), southern armyworm (Spodoptera eridania), tomato homworm (Manduca quinquemaculata), tabacco hornworm (Manduca sexta), grapeberry moth (Endopiza viteana), apple lyonetid (Lyonetia prunifoliella malinella), apple leafminer (Phyllonorycter ringoniella), citrus leafminer (Phyllocnistis citrella), pink bollworm (Pectinophora gossypiella), peach fruit moth (Carposina sasakii), summer fruit tortrix (Adoxophyes orana faciata), smaller tea tortrix (Adoxophyes honmai), oriental tea tortrix (Homona magnamina), codling moth (Cydla pomonella), Oriental fruit moth (Grapholita molesta), rice stem borer (Chilo suppressalis), rice leafroller (Cnaphalocrocis medinalis), cabbage webworm (Hellula undalis), European com borer (Ostrinia nubilalis), soybean looper (Pseudoplusia includens), cabbage looper (Trichoplusia ni), fall wedworm (Hyphantria cunea), common white (Pieris rapae crucivora), rice skipper (Parnara guttata), etc.,

Coleoptera harmful insects such as cupreous chafer beetle (Anomala cuprea), soybean beetle (Anomala rufocuprea), Japanese beetle (Popillia japonica), Colorado potato beetle (Lepinotarsa decemlineata), Mexican bean beetle (Epilachna varivestis), sugarcane wireworm (Melanotus tamsuyensis), cigarette beetle (Lasioderma serricorne), Himehirata Nitidulidae (Epuraea domina), twentyeight-spotted ladybird (Epilachna vigintioctopunctata), yellow mealworm (Tenebrio molitor), red flour beetle (Tribolium castaneum), whitespotted longicom beetle (Anoplophora malasiaca), japanese pine sawyer (Monochamus altematus), azuki bean weevil (Callosobruchus chinensis), cucurbit leaf beetle (Aulacophora femoralis), rice leaf beetle (Oulema oryzae), striped flea beetle (Phyllotreta striolata), sweetpotato weevil (Cylas formicarius), boll weevil (Anthonomus grandis), rice curculio (Echinocnemus squameus), alfalfa weevil (Hypera postica), rice water weevil (Lissohoptrus oryzophilus), maize weevil (Sitophilus zeamais), hunting billbug (Sphenophorus venatus vestitus), granary weevil (Sitophilus granarius), southem com rootworm (Diabrotica undecimpunctata), westem com rootworm (Diabrotica virgifera), northern com rootworm (Diabrotica barberi), rove beetle (Paederus fuscipes), etc.,

Hemiptera harmful insects such as cabbage bug (Eurydema rugosum), whitespotted bug (Eysarcoris ventralis), brown marmorated stink bug (Halyomorpha halys), southem green stink bug (Nezara viridula), rice bug (Leptocorisa chinensis), bean bug (Riptortus clavatus), small wing gourd bug (Togo hemipterus), tamished plant bug (Lygus lineolaris), cotton fleahopper (Pseudatomoscelis seriatus), azalea lace bug (Stephanitis pyrioides), grape leafhopper (Epiacanthus stramineus), tea green leafhopper (Empoasca onukii), potato leafhopper (Empoasca fabae), green rice leafhopper (Nephotettix cinctinceps), small brown planthopper (Laodelphax striatella), brown rice planthopper (Nilaparvata lugens), whitebacked rice planthopper (Sogatella furcifera), Asiatic citrus psylla (Diaphorina citri), pear psylla (Psylla pyrisuga), silverleaf whitefly (Bemisia argentifolii), sweetpotato whitefly (Bemisia tabaci), citrus whitefly (Dialeurodes citri), greenhouse whitefly (Trialeurodes vaporariorum), cotton aphid (Aphis gossypii), spiraea aphid (Aphis spiraecola), green peach aphid (Myzus persicae), giant margarodid mealybug (Drosicha corpulenta), cottony cushion scale (Icerya purchasi), citrus mealybug (Planococcus citri), comstock mealybug (Pseudococcus comstocki), red wax scale (Ceroplastes rubens), arrowhead scale (Unaspis yanonensis), bed bug (Cimex lectularius), etc.,

Thysanoptera harmful insects such as western flower thrips (Frankliniella occidentalis), flower thrips (Frankliniella intonsa), yellow tea thrips (Scirtothrips dorsalis), melon thrips (Thrips palmi), onion thrips (Thrips tabaci), etc.,

Diptera harmful insects such as oriental fruit fly (Dacus dorsalis), melon fly (Dacus cucurbitae), mediterranean fruit fly (Ceratitis capitata), rice leaf miner (Hydrellia griseola), bryony leaf miner (Liriomyza bryoniae), serpentine leaf miner (Liriomyza trifolii), seedcom maggot (Delia platura), apple maggot (Rhagoletis pomonella), hessian fly (Mayetiola destructor), house fly (Musca domestica), stable fly (Stomoxys calcitrans), sheep ked (Melophagus ovinus), northern cattle grub (Hypoderma bovis), common cattle grub (Hypoderma lineatum), sheep nasal bot fly (Oestrus ovis), tsetse fly (Glossina palpalis, Glossina morsitans), yellow-leg giant gnat (Prosimulium yezoensis), cattle hoursefly (Tabanus trigonus), filter fly or bathroom_fly (Telmatoscopus albipunctatus), tokunaganuka mosquito (Leptoconops nipponensis), brown house mosquito (Culex pipiens pallens), yellow fever mosquito (Aedes aegypti), asian tiger mosquito (Aedes albopictus), chinese spotted mosquito (Anopheles hyracanus sinesis), etc.,

Hymenoptera harmful insects such as chestnut sawfly (Apethymus kuri), tumip sawfly (Athalia rosae japonensis), european pine sawfly (Neodiprion sertifer), american army ants (Eciton burchelli, Eciton schmitti), red carpenter ant (Camponotus japonicus), giant asian homet (Vespa mandarina), bulldog ants (Myrmecia spp.), fire ants (Solenopsis spp.), pharaoh ant (Monomorium pharaonis), etc.,

Blattaria harmful insects such as smokybrown cockroach (Periplaneta fuliginosa), Japanese cockroach (Periplaneta japonica), German cockroach (Blattella germanica), etc.,

Orthoptera harmful insects such as emma field cricket (Teleogryllus emma), mole cricket (Gryllotalpa orientalis), migratory locust (Locusta migratoria), rice grasshopper (Oxya yezoensis), desert locust (Schistocerca gregaria), etc.,

Isoptera harmful insects such as formosan subterranean termite (Coptotermes formosanus), Japanese termite (Reticulitermes speratus), Taiwanese termite (Odontotermes formosanus), etc.,

Isoptera harmful insects such as cat flea (Ctenocephalides felis), human flea (Pulex irritans), Oriental rat flea (Xenopsylla cheopis), etc.,

Mallophage harmful insects such as chicken body louse (Menacanthus stramineus), cattlebiting louse (Bovicola bovis), etc.,

Anoplura harmful insects such as shortnosed cattle louse (Haematopinus eurysternus), pig louse (Haematopinus suis), longnosed cattle louse (Linognathus vituli), tubercle-bearing louse (Solenopotes capillatus), etc.,

Tetranychidae such as citrus red mite (Panonychus citri), European red mite (Panonychus ulmi), kanzawa spider mite

(Tetranychus kanzawai), two-spotted spider mite (Tetranychus urticae), etc.,
Eriophyidae such as pink tea rust mite (Acaphylla theae), pink citrus rust mite (Aculops pelekassi), Japanese pear rust mite (Eriophyes chibaensis), wheat curl mite (Aceria tulipae), etc.,
Tarsonemidae such as broad mite (Polyphagotarsonemus latus), cyclamen mite (Steneotarsonemus pallidus), etc.,
Acaridae such as mould mite (Tyrophagus putrescentiae), bulb mite (Rhizoglyphus robini), etc.,
Varroidae such as honeybee varroa mite (Varroa jacobsoni), etc.,
Metastigmata such as southem cattle tick (Boophilus microplus), bush tick (Haemaphysalis longicomis), etc.,
Scab mites such as sheep scab mite (Psoroptes ovis), etc.,
Sarcoptes scabiei such as scabies mite (Sarcoptes scabiei), etc.,
Crustaceans such as pill bug (Armadillidium vulgare), etc.,
Nematodes such as cobb's root-lesion nematode (Pratylenchus penetrans), walnut root-lesion nematode (Pratylenchus vulnus), potato cyst nematode (Globodera rostochiensis), soybean cyst nematode (Heterodera glycines), northern root-knot nematode (Meloidogyne hapla), southern root-knot nematode (Meloidogyne incognita), pinewood nematode (Bursaphelenchus lignicolus), etc.,
Mollusks such as channeled apple snail (Ponacea canaliculata), slug (Incilaria bilineatum), Korean round snail (Acusta despecta sieboldiana), Japanese land snail (Euhadra peliomphala), etc.,
but the present invention is not limited by these alone.

Moreover, the compound of the present invention is also effective to harmful insects which are improved in resistance against already presenting insecticides such as organophosphurus type compounds, carbamate type compounds or pyrethroid type compounds, etc.

That is, the compound of the present invention can be effectively present and exterminate harmful insects of Orthoptera, Order Thysanoptera, Hemiptera, Lepidoptera, Coleoptera, Hymenoptera, Thysanoptera, Blattaria, Isoptera, Isoptera, mites and lice, and nematodes with a low concentration. On the other hand, the compound of the present invention has extremely useful characteristics that it causes substantially no bad effect against mammals, foshes, crustaceans and useful insects.

For the purpose of using the compound of the present invention, by mixing with a suitable solid carrier or a liquid carrier, and further, if desired, by adding a surfactant, a penetamt, a spreading agent, a thicknening agent, an antifreezing agent, a binder, a non-caking agent, a discipient, an antifoaming agent, an antiseptic agent and a decomposition preventing agent, etc., it can be practically applied in an optional formulations such as soluble concentrate, emulsifiable concentrate, wettable powder, water soluble powder, water dispersible granule, water soluble granule, suspension concentrate, concentrated emulsion, suspoemulsion, microemulsion, dustable powder, granule, tablet and emulsifiable gel, etc. Also, in the viewpoints of labor saving and improvement in safety, the above-mentioned formulations in an optional form are encapsulated in a water-soluble container such as a bag of a water-soluble capsule and water-soluble film, etc. and applied practically.

As the solid carrier, there may be mentioned, for example, natural minerals such as quartz, calcite, sepiolite, dolomite, chalk, kaolinite, pyrophyylite, sericite, halocite, metahalocite, Kibushi clay, Gaerome clay, kaolin, zeeklite, allophane, white sand (loam), mica, talc, bentonite, active china clay, acidic china clay, pumice, attapulgite, zeolite and diatomaceous earth, etc., calcined products of natural minerals such as calcined clay, perlite, white sand balloon (loam balloon), vermiculite, attapulgus clay and calcined diatomaceous earth, etc., inorganic salts such as magnesium carbonate, calcium carbonate, sodium carbonate, sodium hydrogen carbonate , ammonium sulfate, sodium sulfate, magnesium sulfate, diammonium hydrogen phosphate, ammonium dihydrogen phosphate and potassium chloride, etc., saccharides such as glucose, fructose, sucrose and lactose, etc., polysaccharides such as starch, powder cellulose and dextrin, etc., organic materials such as urea, urea derivatives, benzoic acid and a salt of benzoic acid, etc., plants such as wood powder, cork powder, com head stem, walnut shell and tobacco stem, etc., fly ash, white carbon (e.g., hydrated synthetic silica, anhydrous synthetic silica and hydrated synthetic silicate, etc.) and feritilizers, etc.

As the liquid carrier, there may be mentioned, for example, aromatic hydrocarbons such as xylene, alkyl($C_9$ or $C_{10}$, etc.)benzene, phenylxylylethane and alkyl($C_1$ or $C_3$, etc.)naphthalene, etc., aliphatic hydrocarbons such as machine oil, normal paraffin, isoparaffin and naphthene, etc., a mixture of aromatic hydrocarbons and aliphatic hydrocarbons such as kerosene, etc., alcohols such as ethanol, isopropanol, cyclohexanol, phenoxyethanol and benzylalcohol, etc., polyvalent alcohols such as ethylene glycol, propyleneglycol, diethylene glycol, hexylene glycol, polyethylene glycol and polypropyleneglycol, etc., ethers such as propyl cellosolve, butyl cellosolve, phenyl cellosolve, propyleneglycol monomethyl ether, propyleneglycol monoethyl ether, propyleneglycol monopropyl ether, propyleneglycol monobutyl ether and propyleneglycol monophenyl ether, etc., ketones such as acetophenone, cyclohexanone and $\gamma$-butyrolactone, etc., esters such as aliphatic acid methyl ester, dialkyl succinate, dialkyl glutamate, dialkyl adipate and dialkyl phthalate, etc., acid amides such as N-alkyl($C_1$, $C_8$ or $C_{12}$, etc.)pyrrolidone, etc., oil and fats such as soybean oil, linseed oil, rapeseed oil, coconut oil, cottonseed oil and caster oil, etc., dimethylsulfoxide and water.

These solid and liquid carriers may be used alone or in combination of two or more kinds in combination.

As the surfactant, there may be mentioned, for example, nonionic surfactants such as polyoxyethylene alkyl

ether, polyoxyethylene alkyl (mono- or di-)phenyl ether, polyoxyethylene (mono-, di- or tri-)styrylphenyl ether, polyoxyethylene polyoxypropylene block copolymer, polyoxyethylene fatty acid (mono- or di-)ester, sorbitan fatty acid ester, polyoxyethylene sorbitan fatty acid ester, caster oil-ethylene oxide adducts, acetylene glycol, acetylene alcohol, ethylene oxide adducts of acetylene glycol, ethylene oxide adducts of acetylene alcohol and alkyl glycoside, etc., anionic surfactants such as alkyl sulfate, alkylbenzenesulfonate, lignine sulfonate, alkylsulfosuccinate, naphthalene sulfonate, alkylnaphthalene sulfonate, formalin condensate salt of naphthalene sulfonic acid, formalin condensate salt of alkylnaphthalene sulfonic acid, polyoxyethylene alkyl ether sulfate or phosphate, polyoxyethylene (mono- or di-)alkylphenyl ether sulfate or phosphate, polyoxyethylene (mono-, di- or tri-)styrylphenyl ether sulfate or phosphate, polycarboxylate (e.g., polyacryaltes, polymaleates and copolymer materials of maleic acid and olefin, etc.) and polystyrenesulfonate, etc., cationic surfactants such as alkylamine salt and alkyl quaternary ammonium salt, etc., amphoteric surfactants such as amino acid type and betaine type, etc., silicone type surfactants and fluorine type surfactants.

A content of these surfactants is not specifically limited, and it is desirably in the range of 0.05 to 20 parts by weight in general based on 100 parts by weight of the preparation according to the present invention. Also, these surfactants may be used alone or in combination of two or more kinds in combination.

A dose of the compound of the present invention to be applied may vary depending on the place to be applied, time to be applied, method to be applied, crops to cultivate, etc., and in general, it is suitable in an amount of about 0.005 to 50 kg or so per a hectare (ha) as an amount of the effective ingredient.

Next, Formulation examples of the preparation when the compound of the present invention is used are shown below. Provided that Formulation examples of the present invention are not limited by these. Incidentally, in the following Formulation examples, all "part(s)" mean part(s) by weight.

[Wettable powder]

| | |
|---|---|
| Compound of the present invention | 0.1 to 80 parts |
| Solid carrier | 5 to 98.9 parts |
| Surfactant | 1 to 10 parts |
| Others | 0 to 5 parts |

As other components, there may be mentioned, for example, a non-caking agent, a decomposition preventing agent, and the like.

[Emulsifiable concentrate]

| | |
|---|---|
| Compound of the present invention | 0.1 to 30 parts |
| Liquid carrier | 45 to 95 parts |
| Surfactant | 4.9 to 15 parts |
| Others | 0 to 10 parts |

As other components, there may be mentioned, for example, a spreading agent, a decomposition preventing agent, and the like.

[Suspension concentrate]

| | |
|---|---|
| Compound of the present invention | 0.1 to 70 parts |
| Liquid carrier | 15 to 98.89 parts |
| Surfactant | 1 to 12 parts |
| Others | 0.01 to 30 parts |

As other components, there may be mentioned, for example, an antifreezing agent, a thicknening agent, and the like.

[Water dispersible granule]

| | |
|---|---|
| Compound of the present invention | 0.1 to 90 parts |
| Solid carrier | 0 to 98.9 parts |
| Surfactant | 1 to 20 parts |
| Others | 0 to 10 parts |

As other components, there may be mentioned, for example, a binder, a decomposition preventing agent, and the like.

[Soluble concentrate]

| | |
|---|---|
| Compound of the present invention | 0.01 to 70 parts |
| Liquid carrier | 20 to 99.99 parts |
| Others | 0 to 10 parts |

As other components, there may be mentioned, for example, an antifreezing agent, a spreading agent, and the like.

[Granule]

| | |
|---|---|
| Compound of the present invention | 0.01 to 80 parts |
| Solid carrier | 10 to 99.99 parts |
| Others | 0 to 10 parts |

As other components, there may be mentioned, for example, a binder, a decomposition preventing agent, and the like.

[Dustable powder]

| | |
|---|---|
| Compound of the present invention | 0.01 to 30 parts |
| Solid carrier | 65 to 99.99 parts |
| Others | 0 to 5 parts |

As other components, there may be mentioned, for example, a drift preventing agent, a decomposition preventing agent, and the like.
Next, Formulation examples using the compound of the present invention as an effective ingredient are described in more detail, but the present invention is not limited by these.
Incidentally, in the following Formulation examples, "part(s)" means part(s) by weight.

[Formulation example 1] Wettable powder

| | |
|---|---|
| Present compound No. 1-038 | 20 parts |
| Pyrophyylite | 74 parts |
| Solpol 5039 | 4 parts |
| (a mixture of a nonionic surfactant and an anionic surfactant: available from TOHO Chemical Industry Co., LTD, Tradename) | |
| CARPREX #80D | 2 parts |

(Synthetic hydrated silicic acid: available from Shionogi & Co., Ltd., Tradename)

The above materials are uniformly mixed and pulverized to make wettable powder.

[Formulation example 2] Emulsion

| | |
|---|---|
| Present compound No. 1-038 | 5 parts |
| xylene | 75 parts |
| N-methylpyrrolidone | 15 parts |
| Solpol 2680 | 5 parts |

(a mixture of a nonionic surfactant and an anionic surfactant: available from TOHO Chemical Industry Co., LTD, Tradename)

The above materials are uniformly mixed to make emulsifiable concentrate. [Formulation example 3] Suspension concentrate

| | |
|---|---|
| Present compound No. 1-038 | 25 parts |
| Agrisol S-710 | 10 parts |
| (a nonionic surfactant: available from KAO CORPORATION, Tradename) | |
| Lunox 1000C | 0.5 part |
| (an anionic surfactant: available from TOHO Chemical Industry Co., LTD, Tradename) | |
| Xanthan gum | 0.2 part |
| Water | 64.3 parts |

The above materials are uniformly mixed and pulverized, and then, wet pulverized to make suspension concentrate.

[Formulation example 4] Water dispersible granule

| | |
|---|---|
| Present compound No. 1-038 | 75 parts |
| HITENOL NE-15 | 5 parts |
| (an anionic surfactant: available from DAI-ICHI KOGYO SEIYAKU CO., LTD., Tradename) | |
| VANILLEX N | 10 parts |
| (an anionic surfactant: available from Nippon Paper Chemicals Co., Ltd., Tradename) | |
| CARPREX #80D | 10 parts |

(Synthetic hydrated silicic acid: available from Shionogi & Co., Ltd., Tradename)

The above materials are uniformly mixed and pulverized, and then, a small amount of water is added to the mixture and the resulting mixture is mixed under stirring, granulated by an extrusion granulator, and dried to make water dispersible granule.

[Formulation example 5] Granule

| | |
|---|---|
| Present compound No. 1-038 | 5 parts |
| Bentonite | 50 parts |
| Talc | 45 parts |

The above materials are uniformly mixed and pulverized, and then, a small amount of water is added to the mixture and the resulting mixture is mixed under stirring, granulated by an extrusion granulator, and dried to make granule.

[Formulation example 6] Dustable powder

| | |
|---|---|
| Present compound No. 1-038 | 3 parts |
| CARPREX #80D | 0.5 parts |
| (Synthetic hydrated silicic acid: available from Shionogi & Co., Ltd., Tradename) | |
| Caolinite | 95 parts |
| Diisopropyl phosphate | 1.5 parts |

The above materials are uniformly mixed and pulverized to make dustable powder.

For the purpose of use, the above-mentioned formulations are spread by diluting to 1 to 10000-folds with water, or directly spread without dilution.

Also, when the compound of the present invention is used as an agricultural chemicals, it may be mixed with other kinds of herbicides, various kinds of insecticides, acaricides, nematocides, fungicides, vegetable growth regulators, synergists, fertilizers, soil improvers, etc., and applied, at the time of preparing the formulation or at the time of spreading, if necessary.

In particular, by mixing with the other agricultural chemicals or plant hormones and applying the mixture, it can be expected that a cost is reduced due to reduction in a dose to be applied, enlargement in insecticidal spectrum or higher prevention and extinction effect of noxious organisms due to synergistic effect by mixing agricultural chemicals. At this time, it is possible to use the compound with a plural number of the conventionally known agricultural chemicals in combination simultaneously. As the kinds of the agricultural chemicals to be used in admixture with the compound of the present invention, there may be mentioned, for example, the compounds described in Farm Chemicals Handbook,

1999th Edition and the like. Specific examples of the general names can be enumerated below, but the invention is not necessarily limited by these alone.

Fungicide: acibenzolar-S-methyl, acylaminobenzamide, ambam (amobam), ampropylfos (ampropyfos), anilazine, azaconazole, azoxystrobin, benalaxyl, benodanil, benomyl, benthiazole, benzamacril, binapacryl, biphenyl, bitertanol, bethoxazine, bordeaux mixture, blasticidin-S, bromoconazole, bupirimate, buthiobate, calcium polysulfide, captafol, captan, copper oxychloride, carpropamid, carbendazim, carboxin, CGA-279202 (Test name), chinomethionat, chlobenthiazone, chlorfenazol, chloroneb, chlorothalonil, chlozolinate, cufraneb, cymoxanil, cyproconazol, cyprodinil, cyprofuram, dazomet, debacarb, dichlorophen, diclobutrazol, dichlorfluanid, diclomedine, dicloran, diethofencarb, diclocymet, difenoconazole, diflumetorim, dimethirimol, dimethomorph, diniconazole, diniconazole-M, dinocap, diphenylamine, dipyrithione, ditalimfos, dithianon, dodemorph, dodine, drazoxolon, edifenphos, epoxiconazole, etaconazole, ethirimol, etridianole (etridiazole), famoxazone (famoxadone), fenarimol, febuconazole, fenamidone, fendazosulam, fenfuram, fenhexamid, fenpiclonil, fenpropidin, fenpropimorph, fentin, ferbam, ferimzone, fluazinam, fludioxonil, fluoroimide, fluquinconazole, flusilazole, flusulfamide, flutolanil, flutriafol, folpet, fosetyl-aluminium, fuberidazole, furalaxyl, furametpyr, guazatine, hexachlorobenzene, hexaconazole, hymexazol, imazalil, imibenconazole, iminoctadine, ipconazole, iprobenfos, iprodione, isoprothiolane, iprovalicarb, kasugamycin, kresoxim-methyl, mancopper, mancozeb, maneb, mepanipyrim, mepronil, metalaxyl, metconazole, methasulfocarb, metiram, metominostrobin, myclobutanil, MTF-753 (Test name), nabam, nickel bis(dimethyldithiocarbamate), nitrothal-isopropyl, nuarimol, NNF-9425 (Test name), octhilinone, ofurace, oxadixyl, oxycarboxin, oxpoconazole fumarate, pefurzoate, penconazole, pencycuron, phthalide, piperalin, polyoxins, potassium hydrogen carbonate, probenazole, prochloraz, procymidone, propamocarb hydrochloride, propiconazole, propineb, pyrazophos, pyrifenox, pyrimethanil, pyroquilon, quinomethionate, quinoxyfen, quintozene, RH7281 (Test name), sodium hydrogen carbonate, sodium hypochlorite, sulfur, spiroxamine, tebuconazole, tecnazene, tetraconazole, thiabendazole, thiadiazin/milneb, thifluzamide, thiophanate-methyl, thiram, tolclofos-methyl, tolyfluranid, triadimefon, triadimenol (toriadimenol), triazoxide, tricyclazole, tridemorph, triflumizole, triforine, triticonazole, validamycin, vinclozolin, zinc sulfate, zineb, ziram and shiitake mushroom hyphae extract, etc.

Bactericide: streptomycin, tecloftalam, oxytetracyclin and oxolinic acid, etc.

Nematocide: aldoxycarb, cadusafos, fosthiazate, fosthietan, oxamyl and fenamiphos, etc.

Acaricide: acequinocyl, amitraz, bifenazate, bromopropylate, chinomethionat, chlorobezilate, clofentezine, cyhexatine, dicofol, dienochlor, etoxazole, fenazaquin, fenbutatin oxide, fenpropathrin, fenproximate, halfenprox, hexythiazox, milbemectin, propargite, pyridaben, pyrimidifen and tebufenpyrad, etc.

Insecticide: abamectin, acephate, acetamipirid, aldicarb, allethrin, azinphosmethyl, bendiocarb, benfuracarb, bensultap, bifenthrin, buprofezin, butocarboxim, carbaryl, carbofuran, carbosulfan, cartap, chlorfenapyr, chlorpyrifos, chlorfenvinphos, chlorfluazuron, clothianidin, chromafenozide, chlorpyrifos-methyl, cycloprothrin, cyfluthrin, beta-cyfluthrin, cypermethrin, cyromazine, cyhalothrin, lambda-cyhalothrin, deltamethrin, diafenthiuron, diazinon, diacloden, diflubenzuron, dimethylvinphos, diofenolan, disulfoton, dimethoate, emamectin-benzoate, EPN, esfenvalerate, ethiofencarb, ethiprole, etofenprox, etrimfos, fenitrothion, fenobucarb, fenoxycarb, fenpropathrin, fenvalerate, fipronil, fluacrypyrim, flucythrinate, flufenoxuron, flufenprox, tau-fluvalinate, fonophos, formetanate, formothion, furathiocarb, halofenozide, hexaflumuron, hydramethylnon, imidacloprid, isofenphos, indoxacarb, isoprocarb, isoxathion, lufenuron, malathion, metaldehyde, methamidophos, methidathion, methacrifos, metalcarb, methomyl, methoprene, methoxychlor, methoxyfenozide, monocrotophos, muscalure, nidinotefuran, nitenpyram, omethoate, oxydemeton-methyl, oxamyl, parathion, parathion-methyl, permethrin, phenthoate, phoxim, phorate, phosalone, phosmet, phosphamidon, pirimicarb, pirimiphos-methyl, profenofos, protrifenbute, pymetrozine, pyraclofos, pyriproxyfen, rotenone, sulprofos, silafluofen, spinosad, sulfotep, tebfenozide, teflubenzuron, tefluthorin, terbufos, tetrachlorvinphos, thiacloprid, thiocyclam, thiodicarb, thiamethoxam, thiofanox, thiometon, tolfenpyrad, tralomethrin, trichlorfon, triazuron, triflumuron and vamidothion, etc.

## Example

In the following, the present invention will be explained in more detail by specifically referring to Synthetic examples and Test Examples of the compound of the present invention as Examples, but the present invention is not limited by these.

[Synthetic examples]

Synthetic example 1

$N^1$-[4-[1-hydroxy-1-(5-trifluoromethylpyridin-2-yl)ethyl]-2-methylphenyl]-3-iodo-$N^2$-isopropylphthalic diamide (Present compound No.2-001).

Step 1; Preparation of 2-cyano-5-trifluoromethylpyridine

To 150ml N,N-dimethylformamide solution containing 15.0 g of 2-chloro-5-trifluoromethylpyridine were added 19.4 g of zinc cyanide and 9.6 g of tetrakistriphenylphosphine palladium, and the mixture was stirred under nitrogen atmosphere at 80°C for 3 hours. After completion of the reaction, the reaction mixture was cooled to room temperature by allowing to stand, poured into 300 ml of dil. aqueous ammonia, and extracted with 300 ml of diethyl ether. The organic layer was washed with 200 ml of a saturated saline solution, then dried over anhydrous magnesium sulfate, the solvent was removed under reduced pressure, and the residue was purified by distillation under reduced pressure (96.0 to 99.0°C/40 mmHg) to obtain 10.9 g of the the objective product as white crystals.
Melting point: 36.0 to 38.0°C
$^1$H NMR ($CDCl_3$, $Me_4Si$, 300MHz) δ 9.00 (bs, 1H), 8.13 (dd, J=8.3, 2.1Hz, 1H), 7.87 (d, J=8.3Hz, 1H).

Step 2; Preparation of 2-acetyl-5-trifluoromethylpyridine

Under nitrogen atmosphere, to 40 ml of a tetrahydrofuran solution containing 4.7 g of 2-cyano-5-trifluoromethylpyridine was added dropwise 35.0 ml of a tetrahydrofuran solution containing methyl magnesium bromide (0.93 M) at -78°C under stirring, and after completion of the dropwise addition, the temperature of the mixture was raised to room temperature, and stirring was continued at room temperature for further 1 hour. After completion of the reaction, the reaction mixture was poured into 100 ml of 2N hydrochloric acid, and extracted with 200 ml of ethyl acetate. The organic layer was dehydrated and dried by saturated saline solution and then anhydrous magnesium sulfate in this order, and the solvent was removed under reduced pressure to obtain 4.5 g of the objective product as brownish oily substance.
$^1$H NMR ($CDCl_3$, $Me_4Si$, 300MHz) δ 8.95 (bs, 1H), 8.16 (d, J=8.4Hz, 1H), 8.08 (dd, J=8.4, 2.1Hz, 1H), 2.76 (s, 3H).

Step 3; Preparation of t-butyl 4-[1-hydroxy-1-(5-trifluoromethylpyridin-2-yl)ethyl]-2-methylcarbanilate

Under nitrogen atmosphere, to 40 ml of a t-butylmethylether solution containing 3.0 g of t-butyl 4-iodo-2-methylcarbanilate was added dropwise 12.5 ml of n-butyl lithium (1.57M hexane solution) at -50°C under stirring, and after completion of the dropwise addition, the temperature of the mixture was raised to 0°C, and the mixture was stirred for further 30 minutes. Then, the reaction mixture was cooled to -78°C, 1.7 g of 2-acetyl-5-trifluoromethylpyridine was added thereto, the temperature of the mixture was gradually raised to 0°C, and stirring was continued at the same temperature for further 14 hours. After completion of the reaction, to the reaction mixture was added 100 ml of a saturated aqueous ammonium chloride solution, the organic layer was collected by separation, and the aqueous layer was extracted with 100 ml of ethyl acetate. The organic layers were combined, dried over anhydrous magnesium sulfate, and the solvent was removed under reduced pressure. The residue was purified by silica gel chromatography eluting with ethyl acetate-hexane (1:9 to 2:3) to obtain 1.3 g of the objective product as brown solid.
Melting point: 132.0 to 134.5°C
$^1$H NMR ($CDCl_3$, $Me_4Si$, 300MHz) δ 8.79 (bs, 1H), 7.86 (dd, J=8.3, 2.1Hz, 1H), 7.77 (d, J=8.3Hz, 1H), 7.42 (d, J=8.3Hz, 1H), 7.2-7.3 (m, 2H), 6.24 (bs, 1H). 5.23 (s, 1H), 2.22 (s, 3H), 1.92 (s, 3H), 1.51 (s, 9H).

Step 4; Preparation of 1-(4-amino-3-methylphenyl)-1-(5-trifluoromethylpyridin-2-yl)ethanol

To 0.4 g of t-butyl 4-[1-hydroxy-1-(5-trifluoromethylpyridin-2-yl)ethyl]-2-methylcarbanilate was added dropwise 3.0 ml of trifluoroacetic acid under ice cooling and stirring. After stirring was continued at room temperature for 20 minutes, 50 ml of a saturated aqueous potassium carbonate solution was added to the mixture under ice cooling, the mixture was extracted with 30 ml of chloroform, the organic layer was dried over anhydrous sodium sulfate, and the solvent was removed under reduced pressure to obtain 0.27 g of the objective product as brownish oily substance.
$^1$H NMR ($CDCl_3$, $Me_4Si$, 300MHz) δ 8.78 (bs, 1H), 7.84 (dd, J=8.3, 2.1 Hz, 1H), 7.43 (d, J=8.3Hz, 1H), 7.05-7.15 (m, 2H), 6.61 (d, J=8.3Hz, 1H), 5.15 (s, 1H), 3.59 (bs, 2H), 2.13 (s, 3H), 1.90 (s, 3H).

Step 5; Preparation of $N^1$-[4-[1-hydroxy-1-(4-trifluoromethylpyridin-2-yl)ethyl]-2-methylphenyl]-3-iodo-$N^2$-isopropylphthalic diamide

To 5 ml of a toluene solution containing 0.36 g of 3-iodo-N-isopropylphthalamidic acid was added dropwise 0.26 g of trifluoroacetic anhydride at room temperature under stirring. The mixture was stirred at the same temperature for 2 hours, then, the solvent was removed under reduced pressure, the residue was dissolved in 3.0 ml of acetonitrile, 0.27 g of 1-(4-amino-3-methylphenyl)-1-(5-trifluoromethylpyridin-2-yl)ethanol was added to the mixture, and stirring was continued at room temperature for 2 hours. After completion of the reaction, the solvent was removed under reduced pressure, and the residue was purified by silica gel chromatography eluting with ethyl acetate-hexane (3: 2) to obtain

0.43 g of the objective product as brownish oily substance.
$^1$H NMR ($CDCl_3$, $Me_4Si$, 300MHz) δ 8.80 (bs, 1H), 8.29 (bs, 1H), 7.7-8.05 (m, 4H), 7.1-7.5 (m, 4H), 5.84 (d, J=8.3Hz, 1H), 5.28 (s, 1H), 4.1-4.3 (m, 1H), 2.28 (s, 3H), 1.94 (s, 3H), 1.17 (d, J=6.6Hz, 6H).

Synthetic example 2

N$^1$-[4-[1-(4-bromodifluoromethoxyphenyl)-2,2,2-trifluoro-1-hydroxyethyl]-2-methylphenyl]-3-iodo-N$^2$-isopropylphthalic diamide (Present compound No.1-010).

Step 1; Preparation of 4'-bromodifluoromethoxy-2,2,2-trifluoroacetophenone

To 10 ml of a 1,3-dimethyl-2-imidazolidinone solution containing 2.0 g of 2,2,2-trifluoro-4'-hydroxyacetophenone was added 0.51 g of 55% oily sodium hydride under ice cooling and stirring, and the mixture was stirred at room temperature for 30 minutes. Then, the reaction mixture was added dropwise to 10 ml of a 1,3-dimethyl-2-imidazolidinone solution containing 11.0 g of dibromodifluoromethane under ice cooling and stirring with a rate in which an innter temperature was maintained at 10°C or lower. After completion of the dropwise addition, the temperature of the mixture was raised to room temperature, 0.12 g of potassium t-butoxide was added to the mixture, and then, stirring was continued at the same temperature for further 1 hour. After completion of the reaction, the reaction mixture was poured into 150 ml of ice-water, extracted with 100 ml of diethyl ether, the organic layer was washed with water, dried over anhydrous magnesium sulfate, and the solvent was removed under reduced pressure. The residue was purified by silica gel chromatography eluting with ethyl acetate-hexane (1:19 to 1:9) to obtain 1.83 g of the objective product as yellow oily substance.
$^1$H NMR ($CDCl_3$, $Me_4Si$, 300MHz) δ 8.16 (d, J=8.8Hz, 2H), 7.43 (d, J=8.8Hz, 2H).

Step 2; Preparation of t-butyl 4-[1-(4-bromodifluoromethoxyphenyl)-2,2,2-trifluoro-1-hydroxyethyl]-2-methylcarbanilate

Under nitrogen atmosphere to 25 ml of t-butyl methyl ether solution containing 1.91 g of t-butyl 4-iodo-2-methylcarbanilate was added dropwise 8.0 ml of n-butyl lithium (1.58M hexane solution) at -60°C under stirring, and after completion of the dropwise addition, the temperature of the mixture was raised to 0°C, and the mixture was stirred for further 30 minutes. Then, the reaction mixture was cooled to -78°C, 7.0 g of 4'-bromodifluoromethoxy-2,2,2-trifluoroacetophenone was added, and the temperature of the mixture was gradually raised to 0°C, and stirring was further continued at the same temperature for 30 minutes. After completion of the reaction, the reaction mixture was poured into 100 ml of a saturated aqueous ammonium chloride solution, the organic layer was collected by separation, and the aqueous layer was extracted with 50 ml of ethyl acetate. The organic layers were combined, dried over anhydrous magnesium sulfate, and the solvent was removed under reduced pressure. The residue was purified by silica gel chromatography eluting with ethyl acetate-hexane (1:19 to 1:4) to obtain 1.0 g of the objective product as brownish oily substance.
$^1$H NMR ($CDCl_3$, $Me_4Si$, 300MHz) δ 7.87 (d, J=8.3Hz, 1H), 7.51 (d, J=8.8Hz, 2H), 7.15-7.35 (m, 4H), 6.32 (bs, 1H), 3.00 (bs, 1H), 2.24 (s, 3H), 1.52 (s, 9H).

Step 3; Preparation of 1-(4-amino-3-methylphenyl)-1-(4-bromodifluoromethoxyphenyl)-2,2,2-trifluoroethanol

To 1.0 g of t-butyl 4-[1-(4-bromodifluoromethoxyphenyl)-2,2,2-trifluoro-1-hydroxyethyl]-2-methylcarbanilate was added dropwise 3.0 ml of trifluoroacetic acid under ice cooling and stirring. After stirring was continued at room temperature for 20 minutes, under ice cooling, 50 ml of a saturated aqueous potassium carbonate solution was added to the mixture, the mixture was extracted with 50 ml of chloroform, the organic layer was dried over anhydrous sodium sulfate, and the solvent was removed under reduced pressure to obtain 0.6 g of the objective product as brown crystals.
Melting point: 77.5 to 80.5°C
$^1$H NMR ($CDCl_3$, $Me_4Si$, 300MHz) δ 7.53 (d, J=8.5Hz, 2H), 7.0-7.25 (m, 4H), 6.61 (d, J=8.0Hz, 1H), 2.65-3.15 (bs, 3H), 2.13 (s, 3H).

Step 4; Preparation of N$^1$-[4-[1-(4-bromodifluoromethoxyphenyl)-2,2,2-trifluoro-1-hydroxyethyl]-2-methylphenyl]-3-iodo-N$^2$-isopropylphthalic diamide

To 5 ml of a toluene solution containing 0.25 g of 3-iodo-N-isopropylphthalamidic acid was added dropwise 0.18 g of trifluoroacetic anhydride at room temperature under stirring. After the mixture was stirred at the same temperature for 1 hour, the solvent was removed under reduced pressure, the residue was dissolved in 3 ml of acetonitrile, 0.27 g of 1-(4-amino-3-methylphenyl)-1-(4-bromodifluoromethoxyphenyl)-2,2,2-trifluoroethanol was added to the mixture, and

stirring was continued at room temperature for 15 hours. After completion of the reaction, the solvent was removed under reduced pressure, and the residue was purified by silica gel chromatography eluting with ethyl acetate-hexane (2: 3) to obtain 0.13 g of the objective product as brown crystals.
Melting point: 97.0 to 102.5°C
$^1$H NMR ($CDCl_3$, $Me_4Si$, 300MHz) δ 8.39 (bs, 1H), 8.06 (d, J=8.8Hz, 1H), 7.96 (d, J=7.8Hz, 1H), 7.78 (d, J=8.0Hz, 1H), 7.53 (d, J=8.8Hz, 2H), 7.1-7.35 (m, 5H), 5.86 (d, J=8.0Hz, 1H), 4.15-4.3 (m, 1H), 3.16 (s, 1H), 2.30 (s, 3H), 1.17 (d, J=6.6Hz, 6H).

Synthetic example 3

$N^1$-[4-[2,2,2- trifluoro- 1- hydroxy- 1-(4- trifluoromethoxyphenyl) ethyl]- 2- methylphenyl]- 3- iodo- $N^2$-isopropylphthalic diamide (Present compound No.1-009).

Step 1; Preparation of [2,2,2-trifluoro-1-methoxy-1-(4-trifluoromethoxyphenyl)ethyl]-trimethylsilane

To 55 ml of a 1,2-dimethoxyethane solution containing 10.0 g of methyl 4-trifluoromethoxybenzoate were added 13.6 g of (trifluoromethyl)trimethylsilane and 0.1 g of cesium fluoride under ice-cooling and stirring, and the temperature of the mixture was raised to room temperature over 2 hours under stirring. After stirring was continued at room temperature for further 1 hour, the reaction mixture was poured into 200 ml of ice-water, extracted with 200 ml of ethyl acetate, the organic layer was dried over anhydrous magnesium sulfate, and then, the solvent was removed under reduced pressure. The residue was purified by silica gel chromatography eluting with hexane to obtain 16.0 g of the objective product as colorless oily substance.
$^1$H NMR ($CDCl_3$, $Me_4Si$, 300MHz) δ 7.62 (d, J=8.5Hz, 2H), 7.23 (d, J=8.5Hz, 2H), 3.21 (s, 3H), 0.27 (s, 9H).

Step 2; Preparation of 2,2,2-trifluoro-4'-trifluoromethoxyacetophenone

To 55 ml of a tetrahydrofuran solution containing 16.0 g of [2,2,2-trifluoro-1-methoxy-1-(4-trifluoromethoxyphenyl)ethyl]trimethylsilane was added 9.5 ml of tetrahydrofuran solution containing 1M of tetrabutyl ammonium fluoride at room temperature under stirring, and the mixture was stirred at room temperature for 20 minutes. After completion of the reaction, to the reaction mixture was added 200 ml of saturated aqueous sodium hydrogen carbonate solution, the reaction mixture was extracted with 200 ml of ethyl acetate, the organic layer was dried over anhydrous magnesium sulfate, and then, the solvent was removed under reduced pressure to obtain 10.8 g of the objective product as brownish oily substance.
$^1$H NMR ($CDCl_3$, $Me_4Si$, 300MHz) δ 8.15 (d, J=8.8Hz, 2H), 7.37 (d, J=8.8Hz, 2H).

Step 3; Preparation of t-butyl 4-[2,2,2-trifluoro-1-hydroxy-1-(4-trifluoromethoxyphenyl)-ethyl]-2-methylcarbanilate

Under nitrogen atmosphere, to 130 ml of a t-butyl methyl ether solution containing 10.0 g of t-butyl 4-iodo-2-methylcarbanilate was added dropwise 41.8 ml of n-butyl lithium (1.58M hexane solution) at -60°C under stirring, and after completion of the dropwise addition, the temperature of the mixture was raised to 0°C, and the mixture was stirred for further 30 minutes. Then, the reaction mixture was cooled to -78°C, 7.75 g of 2,2,2-trifluoro-4'-trifluoromethoxyacetophenone was added to the mixture, the temperature of the mixture was gradually raised to 0°C, and stirring of the mixture was continued at the same temperature for further 30 minutes. After completion of the reaction, the reaction mixture was poured into 300 ml of a saturated aqueous ammonium chloride solution, the organic layer was collected by separation, and the aqueous layer was extracted with 100 ml of ethyl acetate. The organic layers were combined, dried over anhydrous magnesium sulfate, and the solvent was removed under reduced pressure. The residue was purified by silica gel chromatography eluting with ethyl acetate-hexane (1: 19 to 1: 4) to obtain 9.0 g of the objective product as colorless oily substance.
$^1$H NMR ($CDCl_3$, $Me_4Si$, 300MHz) δ 7.87 (d, J=8.5Hz, 1H), 7.50 (d, J=8.8Hz, 2H), 7.1-7.35 (m, 4H), 6.31 (bs, 1H), 2.95 (bs, 1H), 2.23 (s, 3H), 1.52 (s, 9H).

Step 4; Preparation of 1-(4-amino-3-methylphenyl)-2,2,2-trifluoro-1-(4-trifluoromethoxyphenyl)ethanol

To 9.0 g of t-butyl 4-[2,2,2-trifluoro-1-hydroxy-1-(4-trifluoromethoxyphenyl)ethyl]-2-methylcarbanilate was added dropwise 16.0 ml of trifluoroacetic acid under ice cooling and stirring. After stirring was continued at room temperature for 30 minutes, 200 ml of a saturated aqueous potassium carbonate solution was added to the mixture under ice cooling, the mixture was extracted with 100 ml of chloroform and 100 ml of ethyl acetate, the organic layers were combined, dried over anhydrous sodium sulfate, and the solvent was removed under reduced pressure to obtain 6.8 g of the

objective product as yellow crystals.
Melting point: 127.0 to 128.5°C
$^1$H NMR ($CDCl_3$, $Me_4Si$, 300MHz) δ 7.51 (d, J=8.5Hz, 2H), 7.0-7.25 (m, 4H), 6.60 (d, J=8.3Hz, 1H), 3.06 (bs, 3H), 2.13 (s, 3H).

Step 5; Preparation of $N^1$-[4-[2,2,2-trifluoro-1-hydroxy-1-(4-trifluoromethoxyphenyl)ethyl]-2-methylphenyl]-3-iodo-$N^2$-isopropylphthalic diamide

To 5 ml of a toluene solution containing 0.2 g of 3-iodo-N-isopropylphthalamidic acid was added dropwise 1.0 g of trifluoroacetic anhydride at room temperature under stirring. After stirring the mixture at the same temperature for 30 minutes, the solvent was removed under reduced pressure, the residue was dissolved in 5 ml of acetonitrile, 0.2 g of 1-(4-amino-3-methylphenyl)-2,2,2-trifluoro-1-(4-trifluoromethoxyphenyl)ethanol and one drop of trifluoroacetic acid were added to the mixture, and stirring was continued at room temperature for 4 hours. After completion of the reaction, the solvent was removed under reduced pressure, and the residue was purified by silica gel chromatography eluting with ethyl acetate-hexane (1:1) to obtain 0.33 g of the objective product as colorless resinous substance.
$^1$H NMR ($CDCl_3$, $Me_4Si$, 400MHz) δ 8.39 (bs, 1H), 8.10 (d, J=8.8Hz, 1H), 7.95 (d, J=7.6Hz, 1H), 7.80 (d, J=8.0Hz, 1H), 7.52 (d, J=8.0Hz, 2H), 7.2-7.35 (m, 5H), 5.90 (d, J=8.0Hz, 1H), 4.15-4.3 (m, 1H), 3.01 (s, 1H), 2.33 (s, 3H), 1.17 (d, J=6.6Hz, 6H).

Synthetic example 4

$N^1$-[4-[2,2,2-trifluoro-1-hydroxy-1-(2,2,3,3-tetrafluoro-1,4-dioxa-2,3-dihydronaphthalen-6-yl)ethyl]-2-methylphenyl]-3-iodo-$N^2$-isopropylphthalic diamide (Present compound No.1-028).

Step 1; Preparation of t-butyl 2-methyl-4-trifluoroacetylcarbanilate

Under nitrogen atmosphere, to 300 ml of a diethyl ether solution containing 10.0 g of t-butyl 4-iodo-2-methylcarbanilate was added dropwise 45.0 ml of n-butyl lithium (1.5M hexane solution) at -10°C under stirring, and after completion of the dropwise addition, the mixture was stirred at the same temperature for 30 minutes. Then, the reaction mixture was cooled to -78°C, 9.5 g of trifluoroethyl acetate was added dropwise to the mixture, and after completion of the dropwise addition, stirring was continued at the same temperature for further 1 hour. After completion of the reaction, the temperature of the reaction mixture was raised to -10°C, 100 ml of 2N hydrochloric acid was added and the mixture wsa vigorously stirred, then, the organic layer was collected by separation, dehydrated and dried by saturated saline solution and then anhydrous magnesium sulfate in this order, and the solvent was removed under reduced pressure. The residue was purified by silica gel chromatography eluting with diethyl ether-hexane (1:4) to obtain 3.2 g of the objective product as white crystals.
Melting point: 85.0 to 87.0°C
$^1$H NMR ($CDCl_3$, $Me_4Si$, 300MHz) δ 8.25 (d, J=8.8Hz, 1H), 7.8-8.0 (m, 2H), 6.62 (bs, 1H), 2.32 (s, 3H), 1.55 (s, 9H).

Step 2; Preparation of t-butyl 4-[2,2,2-trifluoro-1-hydroxy-1-(2,2,3,3-tetrafluoro-1,4-dioxa-2,3-dihydronaphthalen-6-yl) ethyl]-2-methylcarbanilate

Under nitrogen atmosphere, to 30 ml of a t-butylmethylether solution containing 2.37 g of 6-bromo-2,2,3,3-tetrafluoro-1,4-dioxa-2,3-dihydronaphthalene was added dropwise 5.4 ml of n-butyl lithium (1.58M hexane solution) at -40°C under stirring, and after completion of the dropwise addition, the temperature of the mixture was raised to 0°C, and the mixture was stirred for further 1 hour. Then, the reaction mixture was cooled to -50°C, 20 ml of a t-butylmethylether solution containing 1.0 g of t-butyl 2-methyl-4-trifluoroacetylcarbanilate was added dropwise to the mixture, the temperature of the mixture was gradually raised to 0°C, and stirring was continued at the same temperature for further 1 hour. After completion of the reaction, the reaction mixture was poured into 100 ml of a saturated aqueous ammonium chloride solution, the organic layer was collected by separation, and the aqueous layer was extracted with 50 ml of ethyl acetate. The organic layers were combined, dehydrated and dried by saturated saline solution and then anhydrous magnesium sulfate in this order, and the solvent was removed under reduced pressure. The residue was purified by silica gel chromatography eluting with ethyl acetate-hexane (1: 4) to obtain 2.3 g of the objective product as yellow oily substance.

Step 3; Preparation of 1-(4-amino-3-methylphenyl)-2,2,2-trifluoro-1-(2,2,3,3-tetrafluoro-1,4-dioxa-2,3-dihydronaphthalen-6-yl)ethanol

To 2.3 g of t-butyl 4-[2,2,2-trifluoro-1-hydroxy-1-(2,2,3,3-tetrafluoro-1 ,4-dioxa-2,3-dihydronaphthalen-6-yl)ethyl]-2-methylcarbanilate was added dropwise 15.0 ml of trifluoroacetic acid under ice cooling and stirring. After stirring was continued at room temperature for 30 minutes, excess trifluoroacetic acid was removed under reduced pressure, the residue was dissolved in 100 ml of ethyl acetate, washed with 100 ml of saturated aqueous sodium carbonate solution, dehydrated and dried by saturated saline solution and then anhydrous magnesium sulfate in this order, and the solvent was removed under reduced pressure. The residue was purified by silica gel chromatography eluting with ethyl acetate-hexane (2: 3) and high performance liquid chromatography eluting with acetonitrile-water (80: 20) to obtain 0.5 g of the objective product as white crystals.
Melting point: 147.5 to 150.0°C
$^1$H NMR ($CDCl_3$, $Me_4Si$, 300MHz) δ 7.33 (s, 1H), 7.24 (d, J=9.9Hz, 1H), 7.05-7.1 (m, 3H), 6.58 (d, J=9.1 Hz, 1H), 3.72 (bs, 2H), 3.45 (bs, 1H), 2.12 (s, 3H).

Step 4; Preparation of $N^1$-[4-[2,2,2-trifluoro-1-hydroxy-1-(2,2,3,3-tetrafluoro-1,4-dioxa-2,3-dihydronaphthalen-6-yl) ethyl]-2-methylphenyl]-3-iodo-$N^2$-isopropylphthalic diamide

To 10 ml of a toluene solution containing 0.15 g of 3-iodo-N-isopropylphthalamidic acid was added dropwise 0.15 g of trifluoroacetic anhydride at room temperature under stirring. After stirring was continued at the same temperature for 30 minutes, the solvent was removed under reduced pressure, the residue was dissolved in 3 ml of acetonitrile, 5 ml of an acetonitrile solution containing 0.15 g of 1-(4-amino-3-methylphenyl)-2,2,2-trifluoro-1-(2,2,3,3-tetrafluoro-1,4-dioxa-2,3-dihydronaphthalen-6-yl)ethanol was added dropwise to the mixture, and after completion of the dropwise addition, stirring was continued at room temperature for 1 hour. After completion of the reaction, the solvent was removed under reduced pressure, the residue was purified by silica gel chromatography eluting with ethyl acetate-hexane (2: 3) to obtain 0.25 g of the objective product as colorless resinous substance.
$^1$H NMR ($CDCl_3$, $Me_4Si$, 300MHz) δ 8.42 (s, 1H), 8.10 (d, J=8.8Hz, 1H), 7.9-8.05 (m, 2H), 7.73 (d, J=7.8Hz, 1H), 7.05-7.35 (m, 6H), 5.98 (d, J=8.1Hz, 1H), 4.1-4.25 (m, 1H), 3.69 (s, 1H), 2.28 (s, 3H), 1.16 (d, J=6.6Hz, 6H).

Synthetic example 5

$N^1$-[4-[2,2,2-trifluoro-1-hydroxy-1-(4-methanesulfonyloxyphenyl]ethyl]-2-methylphenyl]-3-iodo-$N^2$-isopropylphthalic diamide(Present compound No.1-015).

Step 1; Preparation of 4'-hydroxy-3-methyl-4-nitrobenzophenone

To 30ml of a dichloromethane solution containing 2.0 g of 4'-methoxy-3-methyl-4-nitrobenzophenone was added 2.1 ml of boron tribromide under ice cooling and stirring, and the mixture was stirred at room temperature for 20 hours. After completion of the reaction, 2 ml of diethyl ether was added to the reaction mixture, and the resulting mixture was stirred for 10 minutes. Then, the mixture was poured into 30 ml of water, the organic layer was collected by separation, washed with water, dehydrated and dried by saturated saline solution and then anhydrous sodium sulfate in this order, and the solvent was removed under reduced pressure. The remained solid was washed with a mixed solvent of diisopropyl ether-hexane to obtain 1.8 g of the objective product as pale purple crystals.
Melting point: 146.0 to 148.0°C
$^1$H NMR ($CDCl_3$-DMSO-$d_6$, $Me_4Si$, 300MHz) δ 9.71 (bs, 1H), 8.02 (d, J=8.3Hz, 1H), 7.55-7.85 (m, 4H), 6.94 (d, J=8.3Hz, 2H), 2.68 (s, 3H).

Step 2; Preparation of 4-(3-methyl-4-nitrobenzoyl)phenyl methanesulfonate

To 5 ml of an N,N-dimethylformamide solution containing 0.50 g of 4'-hydroxy-3-methyl-4-nitrobenzophenone and 0.20 g of triethylamine was added 0.22 g of methanesulfonyl chloride under ice cooling and stirring, and the mixture was stirred at the same temperature for 20 minutes. After completion of the reaction, the reaction mixture was poured into 30 ml of water, extracted with 50 ml of diethyl ether, the organic layer was dehydrated and dried by saturated saline solution and then anhydrous magnesium sulfate in this order, and the solvent was removed under reduced pressure. The residue was purified by silica gel chromatography eluting with ethyl acetate-hexane (2: 5) to obtain 0.54 g of the objective product as brownish oily substance.
$^1$H NMR ($CDCl_3$, $Me_4Si$, 300MHz) δ 8.05 (d, J=8.3Hz, 1H), 7.89 (d, J=8.8Hz, 2H), 7.75 (s, 1H), 7.72 (d, J=8.3Hz, 1H), 7.45 (d, J=8.8Hz, 2H), 3.25 (s, 3H), 2.66 (s, 3H).

Step 3; Preparation of 4-[1-(4-amino-3-methylphenyl)-2,2,2-trifluoro-1-hydroxyethyl]-phenyl methanesulfonate

To 3 ml of a 1,2-dimethoxyethane solution containing 0.54 g of 4-(3-methyl-4-nitrobenzoyl)phenyl methanesulfonate and 1.5 ml of (trifluoromethyl)trimethylsilane was added 0.06 g of cesium fluoride under ice cooling and stirring, and then, the temperature of the mixture was raized to room temperature, and stirring was continued for further 18 hours. After completion of the reaction, the reaction mixture was poured into 30 ml of water, extracted with 50 ml of diethyl ether, the organic layer was dehydrated and dried by saturated saline solution and then anhydrous magnesium sulfate in this order, and the solvent was removed under reduced pressure. The residue was dissolved in 3 ml of tetrahydrofuran, 0.3 ml of a tetrahydrofuran solution containing 1 M of tetrabutyl ammonium fluoride was added to the mixture at room temperature under stirring, and the resulting mixture was stirred at room temperature for 10 minutes. After completion of the reaction, the solvent was removed under reduced pressure, 30 ml of water was added to the residue, the mixture was extracted with 30 ml of ethyl acetate, dehydrated and dried by saturated saline solution and then anhydrous magnesium sulfate in this order, and the dryer was removed by filtration. To the filtrate was added 0.2 g of 5% palladium-carbon, and the mixture was stirred under hydrogen atmosphere at room temperature for 20 hours. After completion of the reaction, palladium-carbon was filtered off with Celite, the solvent was removed under reduced pressure, and the residue was purified by silica gel chromatography eluting with ethyl acetate-hexane (1:2 to 2:3) to obtain 0.24 g of the objective product as white crystals. Melting point: 137.0 to 139.0°C
$^1$H NMR ($CDCl_3$, $Me_4Si$, 300MHz) δ 7.54 (d, J=8.8Hz, 2H), 7.25 (d, J=7.7Hz, 2H), 7.11 (s, 1H), 7.09 (d, J=8.3Hz, 1H), 6.62 (d, J=9.0Hz, 1H), 3.72 (bs, 2H), 3.16 (s, 3H), 2.94 (bs, 1H), 2.14 (s, 3H).

Step 4; Preparation of $N^1$-[4-[2,2,2-trifluoro-1-hydroxy-1-(4-methanesulfonyloxyphenyl)-ethyl]-2-methylphenyl]-3-iodo-$N^2$-isopropylphthalic diamide

To 5 ml of a toluene solution containing 0.10 g of 3-iodo-N-isopropylphthalamidic acid was added dropwise 0.09 g of trifluoroacetic anhydride at room temperature under stirring. After stirring at the same temperature for 30 minutes, the solvent was removed under reduced pressure, the residue was dissolved in 4 ml of acetonitrile, 0.11 g of 4-[1-(4-amino-3-methylphenyl)-2,2,2-trifluoro-1-hydroxyethyl]phenyl methanesulfonate and 1 drop of trifluoroacetic acid were added to the mixture, and the resulting mixture was stirred at room temperature for 18 hours. After completion of the reaction, the solvent was removed under reduced pressure, and the residue was purified by silica gel chromatography eluting with ethyl acetate-hexane (1:1) to obtain 0.20.g of the objective product as colorless resinous solid.
Melting point: 111.0 to 114.0°C
$^1$H NMR ($CDCl_3$, $Me_4Si$, 300MHz) δ 8.39 (bs, 1H), 8.08 (d, J=9.1 Hz, 1H), 7.97 (d, J=8.0Hz, 1H), 7.79 (d, J=7.1 Hz, 1H), 7.54 (d, J=8.8Hz, 2H), 7.15-7.4 (m, 5H), 5.17 (d, J=8.0Hz, 1H), 4.15-4.3 (m, 1H), 3.17 (s, 3H), 3.07 (s, 1H), 2.30 (s, 3H), 1.17 (d, J=6.6Hz, 6H).

Synthetic example 6

$N^1$-[4-p-(4-chlorophenyl)-1-hydroxy-1-trifluoromethyl-2-propenyl]-2-methylphenyl]-3-iodo-$N^2$-isopropylphthalic diamide(Present compound No.2-018).

Step 1; Preparation of t-butyl 4-[3-(4-chlorophenyl)-1-hydroxy-1-trifluoromethyl-2-propenyl]-2-methylcarbanilate

Under nitrogen atmosphere, to 140 ml of a t-butylmethylether solution containing 10.0 g of t-butyl 4-iodo-2-methylcarbanilate was added dropwise 41.8 ml of n-butyl lithium (1.58M hexane solution) at -60°C under stirring, and after completion of the dropwise addition, the temperature of the mixture was raised to 0°C, and the mixture was stirred for further 30 minutes. Then, the reaction mixture was cooled to -78°C, 7.0 g of 4-(4-chlorophenyl)-1,1,1-trifluoro-3-buten-2-one was added, and the temperature of the mixture was gradually raised to 0°C, and stirring was continued at the same temperature for further 30 minutes. After completion of the reaction, the reaction mixture was poured into 300 ml of a saturated aqueous ammonium chloride solution, the organic layer was collected by separation, and the aqueous layer was extracted with 100 ml of ethyl acetate. The organic layers were combined, dried over anhydrous magnesium sulfate, and the solvent was removed under reduced pressure. The residue was purified by silica gel chromatography eluting with ethyl acetate-hexane (1:9 to 2:3) and alumina column chromatography eluting with chloroform to obtain 4.2 g of the objective product as colorless oily substance.
$^1$H NMR ($CDCl_3$, $Me_4Si$, 300MHz) δ 7.90 (d, J=8.1Hz, 1H), 7.25-7.5 (m, 6H), 6.82 (d, J=16.0Hz, 1H), 6.63 (d, J=16.0Hz, 1H), 6.31 (bs, 1H), 2.73 (s, 1H), 2.27 (s, 3H), 1.52 (s, 9H).

Step 2; Preparation of 1-(4-amino-3-methylphenyl)-3-(4-chlorophenyl)-1-trifluoromethyl-2-propen-1-ol

To 4.2 g of t-butyl 4-[3-(4-chlorophenyl)-1-hydroxy-1-trifluoromethyl-2 propenyl]-2-methylcarbanilate was added dropwise 8.0 ml of trifluoroacetic acid under ice cooling and stirring. After stirring was continued at room temperature for 30 minutes, under ice cooling, 150 ml of a saturated aqueous potassium carbonate solution was added to the mixture,

the resulting mixture was extracted with 100 ml of chloroform, the organic layer was dried over anhydrous sodium sulfate, and the solvent was removed under reduced pressure. The remained solid was washed with 10 ml of diisopropyl ether to obtain 2.4 g of the objective product as white crystals.
Melting point: 126.5 to 129.0°C
$^1$H NMR ($CDCl_3$, $Me_4Si$, 300MHz) δ 7.15-7.4 (m, 6H), 6.83 (d, J=16.0Hz, 1H), 6.55-6.75 (m, 2H), 2.8-3.5 (broad, 3H), 2.17 (s, 3H).

Step 3; Preparation of N$^1$-[4-[3-(4-chlorophenyl)-1-hydroxy-1-trifluoromethyl-2-propenyl]-2-methylphenyl]-3-iodo-N$^2$-isopropylphthalic diamide

To 20 ml of a toluene solution containing 1.17 g of 3-iodo-N-isopropylphthalamidic acid was added dropwise 0.86 g of trifluoroacetic anhydride at room temperature under stirring. After the mixture was stirred at the same temperature for 1 hour, the solvent was removed under reduced pressure, the residue was dissolved in 10 ml of acetonitrile, 1.0 g of 1-(4-amino-3-methylphenyl)-3-(4-chlorophenyl)-1-trifluoromethyl-2-propen-1-ol was added to the solution, and stirring of the mixture was continued at room temperature for 3 hours. After completion of the reaction, the solvent was removed under reduced pressure, the residue was purified by silica gel chromatography eluting with ethyl acetate-hexane (1: 9 to 2: 3) to obtain 1.50 g of the objective product as white crystals.
Melting point: 108.0 to 113.0°C
$^1$H NMR ($CDCl_3$, $Me_4Si$, 400MHz) δ 8.38 (s, 1H), 8.07 (d, J=8.8Hz, 1H), 7.96 (d, J=8.0Hz, 1H), 7.78 (d, J=8.0Hz, 1H), 7.15-7.5 (m, 7H), 6.83 (d, J=16.0Hz, 1H), 6.64 (d, J=16.0Hz. 1H), 5.85 (d, J=8.4Hz, 1H), 4.15-4.3 (m, 1H), 2.99 (s, 1H), 2.34 (s, 3H), 1.16 (d, J=6.4Hz, 6H).

Synthetic example 7

N$^1$-[4-[1-(4-chlorophenyl)-2,2,2-trifluoro-1-methoxyethyl]-2-methylphenyl]-3-iodo-N$^2$-isopropylphthalic diamide(Present compound No.3-002).

Step 1; Preparation of t-butyl 4-[1-(4-chlorophenyl)-2,2,2-trifluoro-1-hydroxyethyl]-2-methylcarbanilate

Under nitrogen atmosphere, to 40 ml of a t-butylmethylether solution containing 3.0 g of t-butyl 4-iodo-2-methylcarbanilate was added dropwise 12.5 ml of n-butyl lithium (1.58M hexane solution) at -50°C under stirring, and after completion of the dropwise addition, the temperature of the mixture was raised to 0°C, and the mixture was stirred for further 30 minutes. Then, the reaction mixture was cooled to -78°C, 1.88 g of 4'-chloro-2,2,2-trifluoroacetophenone was added to the mixture, the temperature of the mixture was gradually raised to 0°C, and stirring was continued at the same temperature for further 30 minutes. After completion of the reaction, to the reaction mixture was added 100 ml of a saturated aqueous ammonium chloride solution, the organic layer was collected by separation, and the aqueous layer was extracted with 100 ml of ethyl acetate. The organic layers were combined, dried over anhydrous magnesium sulfate, and the solvent was removed under reduced pressure. The residue was purified by silica gel chromatography eluting with ethyl acetate-hexane (1:9 to 2:3) to obtain 2.96 g of the objective product as colorless transparent oily substance. $^1$H NMR ($CDCl_3$, $Me_4Si$, 300MHz) δ 7.86 (d, J=8.7Hz, 1H), 7.40 (d, J=8.4Hz, 2H), 7.15-7.35 (m, 4H), 6.30 (bs, 1H), 2.93 (s, 1H), 2.22 (s, 3H), 1.52 (s, 9H).

Step 2; Preparation of 1-(4-amino-3-methylphenyl)-1-(4-chlorophenyl)-2,2,2-trifluoroethanol

To 2.96 g of t-butyl 4-[1-(4-chlorophenyl)-2,2,2-trifluoro-1-hydroxyethyl]-2-methylcarbanilate was added dropwise 6.0 ml of trifluoroacetic acid under ice cooling and stirring. After stirring was continued at room temperature for 20 minutes, 70 ml of a saturated aqueous potassium carbonate solution was added to the mixture under ice cooling, the mixture was extracted with 100 ml of chloroform, the organic layer was dried over anhydrous sodium sulfate, and the solvent was removed under reduced pressure to obtain 1.80 g of the objective product as brown solid.
Melting point: 157.0 to 160.5°C
$^1$H NMR ($CDCl_3$, $Me_4Si$, 300MHz) δ 7.45 (d, J=8.8Hz, 2H), 7.28 (d, J=8.8Hz, 2H), 7.05-7.15 (m, 2H), 6.60 (d, J=8.3Hz, 1H), 5.03 (bs, 1H), 3.74 (bs, 2H), 2.12 (s, 3H).

Step 3; Preparation of 4-[1-(4-chlorophenyl)-2,2,2-trifluoro-1-methoxyethyl]-2-methylaniline

To 3 ml of an N,N-dimethylformamide solution containing 0.5 g of 1-(4-amino-3-methylphenyl)-1-(4-chlorophenyl)-2,2,2-trifluoroethanol was added 0.073 g of 55% oily sodium hydride under ice cooling and stirring, the mixture was stirred at room temperature for 20 minutes, then, 0.24 g of methyl iodide was added to the mixture, and stirring was

continued at the same temperature for further 2 hours. After completion of the reaction, the reaction mixture was poured into 20 ml of ice-water, extracted with 30 ml of ethyl acetate, the organic layer was dried over anhydrous magnesium sulfate, and then, the solvent was removed under reduced pressure. The residue was purified by silica gel chromatography eluting with ethyl acetate-hexane (2:3) to obtain 0.44 g of the objective product as brownish oily substance.
$^1$H NMR ($CDCl_3$, $Me_4Si$, 300MHz) δ 7.2-7.4 (m, 4H), 6.9-7.1 (m, 3H), 6.62 (d, J=8.3Hz, 1H), 3.71 (bs, 2H), 3.27 (s, 3H), 2.14 (s, 3H).

Step 4; Preparation of $N^1$-[4-[1-(4-chlorophenyl)-2,2,2-trifluoro-1-methoxyethyl]-2-methylphenyl]-3-iodo-$N^2$-isopropylphthalic diamide

To 5 ml of a toluene solution containing 0.25 g of 3-iodo-N-isopropylphthalamidic acid was added dropwise 0.18 g of trifluoroacetic anhydride at room temperature under stirring. After the mixture was stirred at the same temperature for 1 hour, the solvent was removed under reduced pressure, the residue was dissolved in 3.0 ml of acetonitrile, 0.21 g of 4-[1-(4-chlorophenyl)-2,2,2-trifluoro-1-methoxyethyl]-2-methylaniline was added to the mixture, and stirring was continued at room temperature for 14 hours. After completion of the reaction, precipitated crystals were collected by filtration and washed with a small amount of acetonitrile to obtain 0.2 g of the objective product as white crystals.
Melting point: 211.5 to 214.0°C
$^1$H NMR ($CDCl_3$, $Me_4Si$, 300MHz) δ 8.35 (bs, 1H), 8.09 (d, J=8.8Hz, 1H), 7.97 (d, J=7.6Hz, 1H), 7.79 (d, J=8.0Hz, 1H), 7.15-7.35 (m, 7H), 5.83 (d, J=8.0Hz, 1H), 4.15-4.3 (m, 1H), 3.30 (s, 3H), 2.31 (s, 3H), 1.17 (d, J=6.6Hz, 6H).

Synthetic example 8

$N^1$-[4-[2-(4-chlorophenoxy)-1-hydroxy-1-trifluoromethylethyl]-2-methylphenyl]-3-iodo-$N^2$-isopropylphthalic diamide (Present compound No.2-004).

Step 1; Preparation of t-butyl 4-[2-(4-chlorophenoxy)-1-hydroxy-1-trifluoromethylethyl]-2-methylcarbanilate

To 30 ml of an N,N-dimethylformamide solution containing 2.0 g of t-butyl 2-methyl-4-(1-trifluoromethyloxirane-1-yl)carbanilate and 1.5 g of 4-chlorophenol was added 0.45 g of 60% oily sodium hydride under ice cooling and stirring, and the mixture was stirred at room temperature for 24 hours. After completion of the reaction, the reaction mixture was poured into 150 ml of ice-water and extracted with 100 ml of diethyl ether, and the organic layer was dehydrated and dried by a saturated saline solution and then anhydrous magnesium sulfate in this order, and the solvent was removed under reduced pressure. The residue was purified by silica gel chromatography eluting with ethyl acetate-hexane (1:4) to obtain 2.1 g of the objective product as colorless oily substance.

Step 2; Preparation of $N^1$-[4-[2-(4-chlorophenoxy)-1-hydroxy-1-trifluoromethylethyl]-2-methylphenyl]-3-iodo-$N^2$-isopropylphthalic diamide

To 1.0 g of t-butyl 4-[2-(4-chlorophenoxy)-1-hydroxy-1-trifluoromethylethyl]-2-methylcarbanilate was added dropwise 5.0 ml of trifluoroacetic acid under ice cooling and stirring. Stirring was continued at room temperature for 10 minutes, then, excess trifluoroacetic acid was removed under reduced pressure to obtain crude 1-(4-amino-3-methylphenyl)-2-(4-chlorophenoxy)-1-trifluoromethylethanol.
To 10 ml of a toluene solution containing 0.8 g of 3-iodo-N-isopropylphthalamidic acid was added dropwise 0.8 g of trifluoroacetic anhydride at room temperature under stirring. After stirring at the same temperature for 30 minutes, the solvent was removed under reduced pressure, the residue was dissolved in 20 ml of acetonitrile, and the above-mentioned crude 1-(4-amino-3-methylphenyl)-2-(4-chlorophenoxy)-1-trifluoromethylethanol was added thereto, and stirring of the mixture was continued at room temperature for 3 hours. After completion of the reaction, the solvent was removed under reduced pressure, the residue was purified by silica gel chromatography eluting with ethyl acetate-chloroform (2: 3) to obtain 0.7 g of the objective product as white crystals.
Melting point: 90.0 to 95.0°C.

Synthetic example 9

N'-[4-[2-(4-ch lorophenoxy)-2,2-d ifluoro-1-hyd roxy-1-trifluoromethylethyl]-2-methylphenyl]-3-iodo-$N^2$-isopropylphthalic diamide (Present compound No.2-008).

Step 1; Preparation of 1-(2-bromo-1,1,2,3,3,3-hexafluoropropyloxy)-4-chlorobenzene

To 100 ml of an N,N-dimethylformamide solution containing 20.0 g of 2,2-dibromo-1,1,2,3,3,3-hexafluoropropane and 7.0 g of 4-chlorophenol was added 2.2 g of 60% oily sodium hydride under ice cooling and stirring, and the mixture was stirred at room temperature for 12 hours. After completion of the reaction, the reaction mixture was poured into 300 ml of ice-water, extracted with 150 ml of diethyl ether, the organic layer was dehydrated and dried by saturated saline solution and then anhydrous magnesium sulfate in this order, and the solvent was removed under reduced pressure. The residue was purified by silica gel chromatography eluting with ethyl acetate-hexane (1:4) to obtain 18.0 g of the objective product as colorless oily substance.

Step 2; Preparation of 4-[2-(4-chlorophenoxy)-1,2,2-trifluoro-1-trifluoromethylethyl]-2-methylaniline

To 50 ml of a dimethylsulfoxide solution containing 5.0 g of 1-(2-bromo-1,1,2,3,3,3-hexafluoropropyloxy)-4-chlorobenzene and 1.5 g of o-toluidine were added 1.8 g of sodium hydrogen carbonate and 3.8 g of sodium dithionite under stirring, and the mixture was stirred at 70°C for 3 hours. After completion of the reaction, the reaction mixture was poured into 300 ml of ice-water, extracted with 150 ml of diethyl ether, the organic layer was dehydrated and dried by saturated saline solution and then anhydrous magnesium sulfate in this order, and the solvent was removed under reduced pressure. The residue was purified by silica gel chromatography eluting with ethyl acetate-hexane (1:4) to obtain 3.8 g of the objective product as colorless oily substance.

Step 3; Preparation of 1-(4-amino-3-methylphenyl)-2-(4-chlorophenoxy)-2,2-difluoro-1-trifluoromethylethanol

To 20 ml of a 1,4-dioxane solution containing 2;0 g of 4-[2-(4-chlorophenoxy)-1,2,2-trifluoro-1-trifluoromethylethyl]-2-methylaniline was added 3.0 g of potassium hydroxide, and the mixture was stirred under reflux for 2 hours. After completion of the reaction, the solvent was removed under reduced pressure, the residue was poured into 100 ml of ice-water and extracted with 100 ml of diethyl ether, the organic layer was dehydrated and dried by saturated saline solution and then anhydrous magnesium sulfate in this order, and the solvent was removed under reduced pressure. The residue was purified by silica gel chromatography eluting with ethyl acetate-hexane (2:3) to obtain 0.6 g of the objective product as pale yellowish oily substance.

Step 4; Preparation of $N^1$-[4-[3-(4-chlorophenyl)-1-hydroxy-1-trifluoromethyl-2-propenyl]-2-methylphenyl]-3-iodo-$N^2$-isopropylphthalic diamide

To 5 ml of a toluene solution containing 0.6 g of 3-iodo-N-isopropylphthalamidic acid was added dropwise 0.6 g of trifluoroacetic anhydride at room temperature under stirring. After the mixture was stirred at the same temperature for 30 minutes, the solvent was removed under reduced pressure, the residue was dissolved in 10 ml of acetonitrile, 0.5 g of 1-(4-amino-3-methylphenyl)-2-(4-chlorophenoxy)-2,2-difluoro-1-trifluoromethylethanol was added to the mixture, and stirring was continued at room temperature for 5 hours. After completion of the reaction, the solvent was removed under reduced pressure, and the residue was purified by silica gel chromatography eluting with ethyl acetate-chloroform (2:3) to obtain 0.3 g of the objective product as colorless crystals.
Melting point: 65.0 to 75.0°C.

Synthetic example 10

$N^1$-(2,2-difluoro-3-hydroxy-5-methyl-3-trifluoromethyl-2,3-dihydrobenzofuran-6-yl)-3-iodo-$N^2$-isopropylphthalic diamide(Present compound No.4-002).

Step 1; Preparation of 1-(2-bromo-1,1,2,3,3,3-hexafluoropropyloxy)-4-methyl-3-nitrobenzene

To 100 ml of an N,N-dimethylformamide solution containing 10.0 g of 2,2-dibromo-1,1,2,3,3,3-hexafluoropropane and 5.0 g of 4-methyl-3-nitrophenol was added 1.4 g of 60% oily sodium hydride under ice cooling and stirring, and the mixture was stirred at room temperature for 12 hours. After completion of the reaction, the reaction mixture was poured into 300 ml of ice-water, extracted with 150 ml of diethyl ether, the organic layer was dehydrated and dried by saturated saline solution and then anhydrous magnesium sulfate in this order, and the solvent was removed under reduced pressure. The residue was purified by silica gel chromatography eluting with ethyl acetate-hexane (1:4) to obtain 8.0 g of the objective product as pale yellowish oily substance.

Step 2; Preparation of 5-(2-bromo-1,1,2,3,3,3-hexafluoropropyloxy)-2-methylaniline

To 100 ml of an ethanol solution containing 8.0 g of 1-(2-bromo-1,1,2,3,3,3-hexafluoropropyloxy)-4-methyl-3-nitrobenzene were added 9.5 g of tin chloride dehydrate and 15 ml of conc. hydrochloric acid at room temperature under stirring, and the mixture was stirred at the same temperature for 12 hours. After completion of the reaction, the solvent was removed under reduced pressure, the residue was poured into 100 ml of 2N aqueous sodium hydroxide solution, extracted with 100 ml of diethyl ether, the organic layer was dehydrated and dried by saturated saline solution and then anhydrous magnesium sulfate in this order, and the solvent was removed under reduced pressure. The residue was purified by silica gel chromatography eluting with ethyl acetate-hexane (1:4) to obtain 6.0 g of the objective product as pale yellowish oily substance.

Step 3; Preparation of 6-amino-2,2,3-trifluoro-5-methyl-3-trifluoromethyl-2,3-dihydrobenzofuran

To 50 ml of a dimethylsulfoxide solution containing 6.0 g of 5-(2-bromo-1,1,2,3,3,3-hexafluoropropyloxy)-2-methylaniline were added 2.2 g of sodium hydrogen carbonate and 4.5 g of sodium dithionite under stirring, and the mixture was stirred at 70°C for 3 hours. After completion of the reaction, the reaction mixture was poured into 300 ml of ice-water and extracted with 150 ml of diethyl ether, the organic layer was dehydrated and dried by saturated saline solution and then anhydrous magnesium sulfate in this order, and the solvent was removed under reduced pressure. The residue was purified by silica gel chromatography eluting with ethyl acetate-hexane (3:7) to obtain 3.0 g of the objective product as colorless oily substance.

Step 4; Preparation of 6-amino-2,2-difluoro-3-hydroxy-5-methyl-3-trifluoromethyl-2,3-dihydrobenzofuran

1.0 g of 6-amino-2,2,3-trifluoro-5-methyl-3-trifluoromethyl-2,3-dihydrobenzofuran was treated by alumina column chromatography eluting with ethyl acetate-methanol (7:3) to obtain 0.6 g of the objective product as white crystals.

Step 5; Preparation of $N^1$-(2,2-difluoro-3-hydroxy-5-methyl-3-trifluoromethyl-2,3-dihydrobenzofuran-6-yl)-3-iodo-$N^2$-isopropylphthalic diamide

To 5 ml of a toluene solution containing 0.6 g of 3-iodo-N-isopropylphthalamidic acid was added dropwise 0.6 g of trifluoroacetic anhydride at room temperature under stirring. After the mixture was stirred at the same temperature for 30 minutes, the solvent was removed under reduced pressure, the residue was dissolved in 10 ml of acetonitrile, 0.5 g of 6-amino-2,2-difluoro-3-hydroxy-5-methyl-3-trifluoromethyl-2,3-dihydrobenzofuran was added to the mixture, and stirring was continued at room temperature for 5 hours. After completion of the reaction, the solvent was removed under reduced pressure, and the residue was purified by silica gel chromatography eluting with ethyl acetate-chloroform (1:1) to obtain 0.4 g of the objective product as colorless crystals.
Melting point: 144.0 to 146.0°C.

Present compounds can be prepared according to the above-mentioned Preparation method and Examples. Examples of such compounds are shown in Table 6 to Table 10, but the present invention is not limited by these.

Incidentally, in the Tables, i-Pr means an isopropyl group, and in the same manner, s-Bu means a secondary butyl group, t-Bu means a tertiary butyl group, Ph means a phenyl group, and 2-Naph means a 2-naphthyl group, respectively,

in the Tables, aromatic heterocyclic rings represented by L-15b to L-45e have the following structures, respectively.

L-15b : 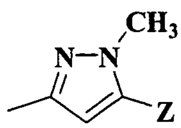

L-36a : 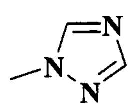

L-45c : 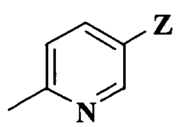

L-45e : 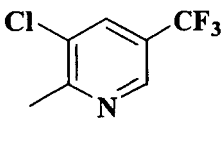

For example, the expression [(D-15b)$CF_3$] means 1-methyl-5-trifluoromethylpyrazol-3-yl group, and the expression [$CH_2$(L-36a)] means 1,2,4-triazol-1-yl methyl group.

Also, in the tables, *1 means "resinous state", and *2 means "oily state", respectively.

Table 6

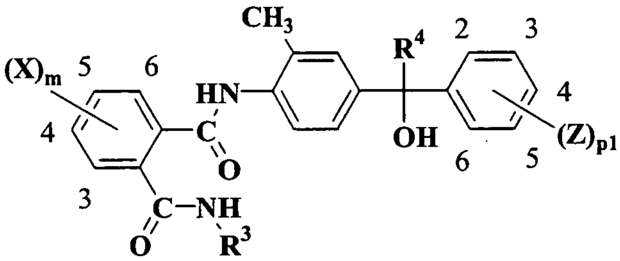

| No. | $(X)_m$ | $R^3$ | $R^4$ | $(Z)_{p1}$ | m.p. (°C) |
|---|---|---|---|---|---|
| 1-001 | 3-I | i-Pr | $CH_3$ | 4-Cl | 177.0-179.0 |
| 1-002 | 3-I | i-Pr | i-Pr | 4-Cl | 112.0-115.0 |
| 1-003 | 3-I | i-Pr | $CF_3$ | 4-F | 192.0-194.0 |
| 1-004 | 3-I | i-Pr | $CF_3$ | 3-Cl | *1 |
| 1-005 | 3-I | i-Pr | $CF_3$ | 4-Cl | 107.5-109.5 |
| 1-006 | 3-I | i-Pr | $CF_3$ | 4-Br | 110.0-113.0 |
| 1-007 | 3-I | i-Pr | $CF_3$ | 4-$CF_3$ | 112.0-114.0 |
| 1-008 | 3-I | i-Pr | $CF_3$ | 4-0$CH_3$ | 102.0-105.0 |
| 1-009 | 3-I | i-Pr | $CF_3$ | 4-0$CF_3$ | *1 |
| 1-010 | 3-I | i-Pr | $CF_3$ | 4-0$CF_2$Br | 97.0-102.5 |
| 1-011 | 3-I | i-Pr | $CF_3$ | 4-0$CF_2CHF_2$ | 98.0-101.0 |
| 1-012 | 3-I | i-Pr | $CF_3$ | 4-0$CF_2CHFCF_3$ | 98.0-101.0 |
| 1-013 | 3-I | i-Pr | $CF_3$ | 4-0$CF_2$CHF0$CF_3$ | 93.0-96.0 |
| 1-014 | 3-I | i-Pr | $CF_3$ | 4-0$CF_2$CHF0$CF_2CF_2CF_3$ | 78.0-80.0 |
| 1-015 | 3-I | i-Pr | $CF_3$ | 4-0S$0_2CH_3$ | 111.0-114.0 |
| 1-016 | 3-I | i-Pr | $CF_3$ | 3-0(L-45e) | 103.5-107.0 |
| 1-017 | 3-I | i-Pr | $CF_3$ | 4-0(L-45e) | 107.0-109.0 |
| 1-018 | 3-I | i-Pr | $CF_3$ | 4-S$CH_3$ | 100.0-107.5 |
| 1-019 | 3-I | i-Pr | $CF_3$ | 4-S$0_2CH_3$ | 150.0-162.0 |
| 1-020 | 3-I | i-Pr | $CF_3$ | 2-F-4-Cl | *1 |
| 1-021 | 3-I | i-Pr | $CF_3$ | 2,4-$Cl_2$ | 120.0-124.0 |
| 1-022 | 3-I | i-Pr | $CF_3$ | 3,4-$Cl_2$ | 112.0-113.0 |
| 1-023 | 3-I | i-Pr | $CF_3$ | 3,5-$Cl_2$ | 195.0-197.0 |
| 1-024 | 3-I | i-Pr | $CF_3$ | 3,4-$Br_2$ | 125.0-131.5 |
| 1-025 | 3-I | i-Pr | $CF_3$ | 3-Cl-4-0$CHF_2$ | *1 |
| 1-026 | 3-I | i-Pr | $CF_3$ | 3-$CH_3$-4-0$CF_2$CHF0$CF_3$ | 92.0-94.0 |

Table continued

| No. | $(X)_m$ | $R^3$ | $R^4$ | $(Z)_{p1}$ | m.p. (°C) |
|---|---|---|---|---|---|
| 1-027 | 3-I | i-Pr | $CF_3$ | $3\text{-}0CF_20\text{-}4$ | *1 |
| 1-028 | 3-I | i-Pr | $CF_3$ | $3\text{-}0CF_2CF_20\text{-}4$ | *1 |
| 1-029 | 3-I | i-Pr | $CF_3$ | $3,5\text{-}Cl_2\text{-}4\text{-}0CH_3$ | 110.0-116.5 |
| 1-030 | 3-I | i-Pr | $C(0)0CH_3$ | 4-Cl | 133.5-135.0 |
| 1-031 | 3-I | s-Bu | $CF_3$ | $4\text{-}0CF_3$ | *1 |
| 1-032 | 3-I | t-Bu | $CF_3$ | $4\text{-}0CF_3$ | 151.0-154.0 |
| 1-033 | 3-I | $CH(CH_3)CH_2SCH_3$ | i-Pr | 4-Cl | 105.0-107.0 |
| 1-034 | 3-I | $CH(CH_3)CH_2SCH_3$ | $CF_3$ | 4-F | 127.0-130.0 |
| 1-035 | 3-I | $CH(CH_3)CH_2SCH_3$ | $CF_3$ | 3-Cl | *1 |
| 1-036 | 3-I | $CH(CH_3)CH_2SCH_3$ | $CF_3$ | 4-Cl | 95.5-99.0 |
| 1-037 | 3-I | $CH(CH_3)CH_2SCH_3$ | $CF_3$ | 4-Bu-t | 107.0-111.0 |
| 1-038 | 3-I | $CH(CH_3)CH_2SCH_3$ | $CF_3$ | $4\text{-}CF_3$ | 101.0-103.0 |
| 1-039 | 3-I | $CH(CH_3)CH_2SCH_3$ | $CF_3$ | $4\text{-}0CH_3$ | 97.0-99.0 |
| 1-040 | 3-I | $CH(CH_3)CH_2SCH_3$ | $CF_3$ | $4\text{-}0CF_3$ | 112.0-115.0 |
| 1-041 | 3-I | $CH(CH_3)CH_2SCH_3$ | $CF_3$ | $4\text{-}0CF_2Br$ | *1 |
| 1-042 | 3-I | $CH(CH_3)CH_2SCH_3$ | $CF_3$ | $4\text{-}0CF_2CHF_2$ | 93.0-96.0 |
| 1-043 | 3-I | $CH(CH_3)CH_2SCH_3$ | $CF_3$ | $4\text{-}0CF_2CHFCF_3$ | 91.0-95.0 |
| 1-044 | 3-I | $CH(CH_3)CH_2SCH_3$ | $CF_3$ | $4\text{-}0CF_2CHF0CF_3$ | 86.0-89.0 |
| 1-045 | 3-I | $CH(CH_3)CH_2SCH_3$ | $CF_3$ | $4\text{-}0CF_2CHF0CF_2CF_2CF_3$ | 71.0-72.0 |
| 1-046 | 3-I | $CH(CH_3)CH_2SCH_3$ | $CF_3$ | $4\text{-}0S0_2CH_3$ | 97.0-100.0 |
| 1-047 | 3-I | $CH(CH_3)CH_2SCH_3$ | $CF_3$ | 3-0 (L-45e) | *1 |
| 1-048 | 3-I | $CH(CH_3)CH_2SCH_3$ | $CF_3$ | 4-0 (L-45e) | 105.0-107.0 |
| 1-049 | 3-I | $CH(CH_3)CH_2SCH_3$ | $CF_3$ | $4\text{-}S0_2CH_3$ | 117.0-120.0 |
| 1-050 | 3-I | $CH(CH_3)CH_2SCH_3$ | $CF_3$ | 2-F-4-Cl | 116.0-120.0 |
| 1-051 | 3-I | $CH(CH_3)CH_2SCH_3$ | $CF_3$ | $2,4\text{-}Cl_2$ | 112.0-116.0 |
| 1-052 | 3-I | $CH(CH_3)CH_2SCH_3$ | $CF_3$ | $3,4\text{-}Cl_2$ | 99.0-103.0 |
| 1-053 | 3-I | $CH(CH_3)CH_2SCH_3$ | $CF_3$ | $3, 5\text{-}Cl_2$ | 121.0-123.5 |
| 1-054 | 3-I | $CH(CH_3)CH_2SCH_3$ | $CF_3$ | $3,4\text{-}Br_2$ | 108.0-110.0 |
| 1-055 | 3-I | $CH(CH_3)CH_2SCH_3$ | $CF_3$ | $3\text{-}Cl\text{-}4\text{-}0CHF_2$ | *1 |
| 1-056 | 3-I | $CH(CH_3)CH_2SCH_3$ | $CF_3$ | $3\text{-}Cl\text{-}4\text{-}0CF_3$ | 83.0-89.0 |
| 1-057 | 3-I | $CH(CH_3)CH_2SCH_3$ | $CF_3$ | $3\text{-}CH_3\text{-}4\text{-}0CF_2CHF0CF_3$ | 86.0-89.0 |
| 1-058 | 3-I | $CH(CH_3)CH_2SCH_3$ | $CF_3$ | $3\text{-}0CF_20\text{-}4$ | *1 |
| 1-059 | 3-I | $CH(CH_3)CH_2SCH_3$ | $CF_3$ | $3\text{-}0CF_2CF_20\text{-}4$ | *1 |
| 1-060 | 3-I | $CH(CH_3)CH_2SCH_3$ | $CF_3$ | $3,5\text{-}Cl_2\text{-}4\text{-}0CH_3$ | 110.0-113.5 |
| 1-061 | 3-I | $CH(CH_3)CH_2SCH_3$ | $C(0)0CH_3$ | 4-Cl | *1 |
| 1-062 | 3-I | $CH(CH_3)CH_2S(0)CH_3$ | $CF_3$ | 3-Cl | 182.0-184.0 |
| 1-063 | 3-I | $CH(CH_3)CH_2S(0)CH_3$ | $CF_3$ | $4\text{-}CF_3$ | 229.0-231.0 |
| 1-064 | 3-I | $CH(CH_3)CH_2S0_2CH_3$ | i-Pr | 4-Cl | 122.0-124.0 |

Table continued

| No. | $(X)_m$ | $R^3$ | $R^4$ | $(Z)_{p1}$ | m.p. (°C) |
|---|---|---|---|---|---|
| 1-065 | 3-I | CH ($CH_3$) $CH_2S0_2CH_3$ | $CF_3$ | 4-F | 142.0-144.0 |
| 1-066 | 3-I | CH ($CH_3$) $CH_2S0_2CH_3$ | $CF_3$ | 4-Cl | 180.0-184.0 |
| 1-067 | 3-I | CH ($CH_3$) $CH_2S0_2CH_3$ | $CF_3$ | 4-$CF_3$ | 111.0-113.0 |
| 1-068 | 3-I | CH ($CH_3$) $CH_2S0_2CH_3$ | $CF_3$ | 4-$0CH_3$ | 123.0-126.0 |
| 1-069 | 3-I | CH ($CH_3$) $CH_2S0_2CH_3$ | $CF_3$ | 4-$0CF_3$ | 218.0-220.0 |
| 1-070 | 3-I | CH ($CH_3$) $CH_2S0_2CH_3$ | $CF_3$ | 4-$0CF_2Br$ | 115.0-117.5 |
| 1-071 | 3-I | CH ($CH_3$) $CH_2S0_2CH_3$ | $CF_3$ | 4-$0CF_2CHF_2$ | 112.0-115.0 |
| 1-072 | 3-I | CH ($CH_3$) $CH_2S0_2CH_3$ | $CF_3$ | 4-$0CF_2CHFCF_3$ | 144.0-146.0 |
| 1-073 | 3-I | CH ($CH_3$) $CH_2S0_2CH_3$ | $CF_3$ | 4-$0CF_2CHFOCF_3$ | 102.0-105.0 |
| 1-074 | 3-I | CH ($CH_3$) $CH_2S0_2CH_3$ | $CF_3$ | 4-0 (L-45e) | 123.0-124.0 |
| 1-075 | 3-I | CH ($CH_3$) $CH_2S0_2CH_3$ | $CF_3$ | 2-F-4-Cl | 228.0-230.0 |
| 1-076 | 3-I | CH ($CH_3$) $CH_2S0_2CH_3$ | $CF_3$ | 2,4-$Cl_2$ | 125.0-128.0 |
| 1-077 | 3-I | CH ($CH_3$) $CH_2S0_2CH_3$ | $CF_3$ | 3,4-$Cl_2$ | 112.0-114.0 |
| 1-078 | 3-I | CH ($CH_3$) $CH_2S0_2CH_3$ | $CF_3$ | 3,5-$Cl_2$ | 127.0-132.0 |
| 1-079 | 3-I | CH ($CH_3$) $CH_2S0_2CH_3$ | $CF_3$ | 3,4-$Br_2$ | 133.0-145.0 |
| 1-080 | 3-I | CH ($CH_3$) $CH_2S0_2CH_3$ | $CF_3$ | 3-Cl-4-$CHF_2$ | *1 |
| 1-081 | 3-I | CH ($CH_3$) $CH_2S0_2CH_3$ | $CF_3$ | 3-$CH_3$-4-$CF_2CHF0CF_3$ | 116.0-118.0 |
| 1-082 | 3-I | CH ($CH_3$) $CH_2S0_2CH_3$ | $CF_3$ | 3-$0CF_20$-4 | 193.0-203.0 |
| 1-083 | 3-I | CH ($CH_3$) $CH_2S0_2CH_3$ | $CF_3$ | 3-$OCF_2CF_20$-4 | *1 |
| 1-084 | 3-I | C $(CH_3)_2CH_2SCH_3$ | $CF_3$ | 4-Cl | 172.0-175.0 |
| 1-085 | 3-I | C $(CH_3)_2CH_2SCH_3$ | $CF_3$ | 4-$0CF_3$ | *1 |
| 1-086 | 3-I | C $(CH_3)_2CH_2SCH_3$ | $CF_3$ | 3,4-$Cl_2$ | 96.0-98.0 |
| 1-087 | 3-I | C $(CH_3)_2CH_2S0_2CH_3$ | $CF_3$ | 4-Cl | 126.0-128.0 |
| 1-088 | 3-I | C $(CH_3)_2CH_2S0_2CH_3$ | $CF_3$ | 4-$0CF_3$ | *1 |
| 1-089 | 3-I | C $(CH_3)_2CH_2S0_2CH_3$ | $CF_3$ | 3,4-$Cl_2$ | 109.0-113.0 |
| 1-090 | 3-I | C $(CH_3)_2C{\equiv}CH$ | $CF_3$ | 4-$0CF_3$ | *1 |

Table 7

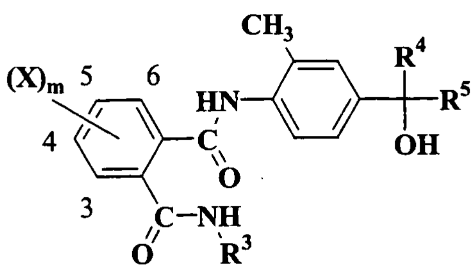

| No. | $(X)_m$ | $R^3$ | $R^4$ | $R^5$ | m. p. (°C) |
|---|---|---|---|---|---|
| 2-001 | 3-I | i-Pr | $CH_3$ | (L-45c) $CF_3$ | *1 |
| 2-002 | 3-I | i-Pr | $CF_3$ | $CH_2CH_2$ (Ph-4-Cl) | 103.5-106.0 |
| 2-003 | 3-I | i-Pr | $CF_3$ | $CH_20CH_3$ | 181.0-184.0 |
| 2-004 | 3-I | i-Pr | $CF_3$ | $CH_20$(Ph-4-Cl) | 90.0-95.0 |
| 2-005 | 3-I | i-Pr | $CF_3$ | $CH_20C(0)$ (Ph-4-Cl) | 180.0-183.0 |
| 2-006 | 3-I | i-Pr | $CF_3$ | $CF_20$(Ph-2-Cl) | 86.0-88.0 |
| 2-007 | 3-I | i-Pr | $CF_3$ | $CF_20$(Ph-3-Cl) | 82.0-85.0 |
| 2-008 | 3-I | i-Pr | $CF_3$ | $CF_20$(Ph-4-Cl) | 65.0-75.0 |
| 2-009 | 3-I | i-Pr | $CF_3$ | $CH_2S$(Ph-4-Cl) | 80.0-83.0 |
| 2-010 | 3-I | i-Pr | $CF_3$ | $CH_2S0_2$(Ph-4-Cl) | 111.0-113.0 |
| 2-011 | 3-I | i-Pr | $CF_3$ | $CH_2N(CH_3)_2$ | 204.0-207.0 |
| 2-012 | 3-I | i-Pr | $CF_3$ | $CH_2NH$(Ph-4-Cl) | 120.0-122.0 |
| 2-013 | 3-I | i-Pr | $CF_3$ | $CF_2C(0)0Et$ | *1 |
| 2-014 | 3-I | i-Pr | $CF_3$ | $CH_2$(L-36a) | 149.0-152.0 |
| 2-015 | 3-I | i-Pr | $CF_3$ | C(0)0Et | 177.0-178.0 |
| 2-016 | 3-I | i-Pr | $CF_3$ | CH=CH(Ph-4-F) | 109.0-111.0 |
| 2-017 | 3-I | i-Pr | $CF_3$ | CH=CH(Ph-3-Cl) | *1 |
| 2-018 | 3-I | i-Pr | $CF_3$ | CH=CH(Ph-4-Cl) | 108.0-113.0 |
| 2-019 | 3-I | i-Pr | $CF_3$ | CH=CH(Ph-4-$OCF_3$) | 104.0-108.5 |
| 2-020 | 3-I | i-Pr | $CF_3$ | CH=CH(Ph-4-$SCH_3$) | * 1 |
| 2-021 | 3-I | i-Pr | $CF_3$ | CH=CH(Ph-4-$SO_2CH_3$) | 133.0-138.0 |
| 2-022 | 3-I | i-Pr | $CF_3$ | CH=CH(Ph-3, 4-$Cl_2$) | 111.0-113.0 |
| 2-023 | 3-I | i-Pr | $CF_3$ | 2-Naph | 113.0-117.0 |
| 2-024 | 3-I | i-Pr | $CF_3$ | (L-45c)$CF_3$ | 107.0-110.0 |
| 2-025 | 3-I | $CH(CH_3)$ $CH_2SCH_3$ | $CF_3$ | $CF_20$(Ph-4-Cl) | *1 |
| 2-026 | 3-I | $CH(CH_3)$ $CH_2SCH_3$ | $CF_3$ | CH=CH(Ph-4-F) | 98.0-111.0 |
| 2-027 | 3-I | $CH(CH_3)$ $CH_2SCH_3$ | $CF_3$ | CH=CH(Ph-3-Cl) | *1 |
| 2-028 | 3-I | $CH(CH_3)$ $CH_2SCH_3$ | $CF_3$ | CH=CH(Ph-4-Cl) | 103.5-108.0 |
| 2-029 | 3-I | $CH(CH_3)$ $CH_2SCH_3$ | $CF_3$ | CH=CH(Ph-4-$OCF_3$) | *1 |
| 2-030 | 3-I | $CH(CH_3)$ $CH_2SCH_3$ | $CF_3$ | CH=CH (Ph-3, 4-$Cl_2$) | 98.0-101.0 |
| 2-031 | 3-I | $CH(CH_3)$ $CH_2SCH_3$ | $CF_3$ | 2-Naph | *1 |
| 2-032 | 3-I | CH $(CH_3)$ $CH_2SCH_3$ | $CF_3$ | (L-15b) $CF_3$ | 94.0-97.0 |
| 2-033 | 3-I | CH $(CH_3)$ $CH_2S0_2CH_3$ | $CF_3$ | $CF_2O$ (Ph-4-Cl) | 131.5-135.0 |
| 2-034 | 3-I | CH $(CH_3)$ $CH_2S0_2CH_3$ | $CF_3$ | CH=CH (Ph-4-F) | 123.0-125.0 |
| 2-035 | 3-I | CH $(CH_3)$ $CH_2S0_2CH_3$ | $CF_3$ | CH=CH (Ph-4-Cl) | 128.0-130.5 |
| 2-036 | 3-I | CH $(CH_3)$ $CH_2S0_2CH_3$ | $CF_3$ | CH=CH (Ph-4-$OCF_3$) | 120.5-125.0 |
| 2-037 | 3-I | CH $(CH_3)$ $CH_2S0_2CH_3$ | $CF_3$ | CH=CH (Ph-3, 4-$Cl_2$) | 124.0-128.0 |
| 2-038 | 3-I | CH $(CH_3)$ $CH_2S0_2CH_3$ | $CF_3$ | 2-Naph | *1 |

Table 8

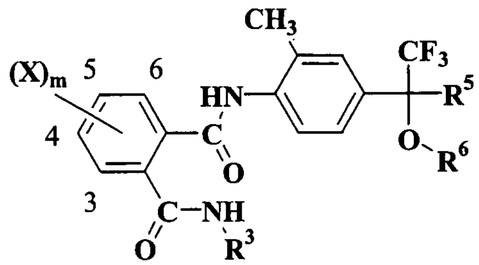

| No. | $(X)_m$ | $R^3$ | $R^5$ | $R^6$ | m.p. (°C) |
|---|---|---|---|---|---|
| 3-001 | 3-I | i-Pr | $CF_20$ (Ph-4-Cl) | $CH_3$ | 55.0-65.0 |
| 3-002 | 3-I | i-Pr | Ph-4-C1 | $CH_3$ | 211.5-214.0 |
| 3-003 | 3-I | i-Pr | Ph-4-C1 | $CH_20CH_3$ | 226.5-229.0 |
| 3-004 | 3-I | i-Pr | Ph-4-C1 | $C(0)CH_3$ | 212.0-214.0 |
| 3-005 | 3-I | i-Pr | CH=CH (Ph-4-Cl) | $CH_3$ | *1 |
| 3-006 | 3-I | CH $(CH_3)$ $CH_2SCH_3$ | CX=CH (Ph-4-Cl) | $CH_3$ | *1 |
| 3-007 | 3-I | CH $(CH_3)$ $CH_2SCH_3$ | CH=CH (Ph-4-Cl) | $CH_20CH_3$ | *1 |
| 3-008 | 3-I | CH $(CH_3)$ $CH_2SCH_3$ | Ph-4-Cl | $CH_3$ | 103.0-107.0 |
| 3-009 | 3-I | CH $(CH_3)$ $CH_2SCH_3$ | Ph-4-Cl | $CH_20CH_3$ | 185.0-188.5 |
| 3-010 | 3-I | CH $(CH_3)$ $CH_2SCH_3$ | Ph-4-Cl | Si $(CH_3)_3$ | 113.0-116.0 |
| 3-011 | 3-I | CH $(CH_3)$ $CH_2SCH_3$ | Ph-4-$0CH_3$ | Si $(CH_3)_3$ | 99.0-103.0 |
| 3-012 | 3-I | CH $(CH_3)$ $CH_2S0_2CH_3$ | CH=CH (Ph-4-Cl) | $CH_3$ | *1 |
| 3-013 | 3-I | CH $(CH_3)$ $CH_2S0_2CH_3$ | CH=CH (Ph-4-Cl) | $CH_20CH_3$ | *1 |
| 3-014 | 3-I | CH $(CH_3)$ $CH_2S0_2CH_3$ | Ph-4-Cl | $CH_3$ | 111.0-114.0 |
| 3-015 | 3-I | CH $(CH_3)$ $CH_2S0_2CH_3$ | Ph-4-Cl | $CH_20CH_3$ | **1 |
| 3-016 | 3-I | CH $(CH_3)$ $CH_2S0_2CH_3$ | Ph-4-$0CH_3$ | S $(CH_3)_3$ | 115.0-119.0 |

Table 9

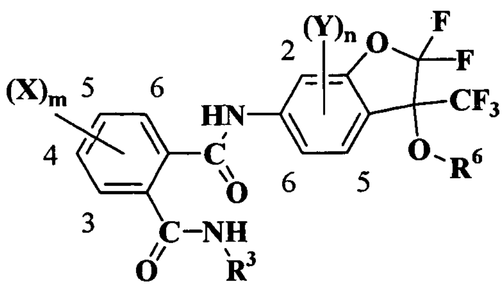

| No. | $(X)_m$ | $R^3$ | $(Y)_n$ | $R^6$ | m. p. (°C) |
|---|---|---|---|---|---|
| 4-001 | 3-I | i-Pr | 2-$CH_3$ | H | 123.0-126.0 |
| 4-002 | 3-I | i-Pr | 6-$CH_3$ | H | 144.0-146.0 |
| 4-003 | 3-I | i-Pr | 6-$CH_3$ | Ph-4-Cl | 195.0-198.0 |

Table 10

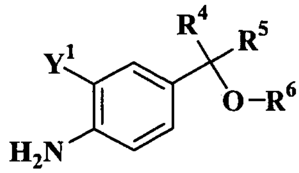

| No. | $Y^1$ | $R^4$ | $R^5$ | $R^6$ | m. p. (°C) |
|---|---|---|---|---|---|
| 5-01 | $CH_3$ | $CH_3$ | (L-45c) $CF_3$ | H | *2 |
| 5-02 | $CH_3$ | $CF_3$ | C(0)0Et | H | *2 |
| 5-03 | $CH_3$ | $CF_3$ | CH=CH (Ph-4-F) | H | 97.0-99.0 |
| 5-04 | $CH_3$ | $CF_3$ | Ph-4-Cl | H | 157.0-160.5 |
| 5-05 | $CH_3$ | $CF_3$ | Ph-4-Cl | $CH_3$ | *2 |
| 5-06 | $CH_3$ | $CF_3$ | Ph-4-0$CH_3$ | $Si(CH_3)_3$ | *2 |
| 5-07 | $CH_3$ | $CF_3$ | Ph-4-0$CF_2CHF_2$ | H | 69.0-72.0 |
| 5-08 | $CH_3$ | $CF_3$ | Ph-4-0$CF_2CHFOCF_3$ | H | 70.0-75.0 |
| 5-09 | $CH_3$ | $CF_3$ | Ph-4-0$CF_2CHFOCF_2CF_2CF_3$ | H | *2 |
| 5-10 | $CH_3$ | $CF_3$ | Ph-4-0$S0_2CH_3$ | H | 137.0-139.0 |
| 5-11 | $CH_3$ | $CF_3$ | Ph-2,4-$Cl_2$ | H | 149.0-151.0 |
| 5-12 | $CH_3$ | $CF_3$ | Ph-3-$CH_3$-4-0$CF_2CHF0CF_3$ | H | 84.0-87.0 |
| 5-13 | $CH_3$ | $CF_3$ | Ph-3-0$CF_2CF_2$0-4 | H | 147.5-150.0 |
| 5-14 | $CH_3$ | $CF_3$ | (L-45c) $CF_3$ | H | 136.0-138.5 |

[Test examples]

Next, usefulness of the compound of the present invention as a noxious organism controlling agent is specifically explained in the following Test Examples, but the present invention is not limited by these alone.

Test Example 1 Insecticidal test against diamondback moth

A 10% emulsifiable concentrate (depending on the compounds, 25% wettable powder was applied for the test) of the compound of the present invention was diluted with water containing a spreading agent to prepare a chemical solution with a concentration of 100 ppm. To the chemical solution was dipped leaves of Chinese olive for about 10 seconds, and after air-drying, they were placed in a laboratory dish, then, 10 diamondback moths (Plutella xylostella) with second instar larvae per the dish were released therein, and the dish was covered with a lid having holes and contained at a thermostat chamber at 25°C. A number of dead insect(s) after 6 days was counted and a rate of dead insects was calculated in the same manner as in Test Example 1. Incidentally, the test was carried out with two districts.

As a result, the following compounds showed 100% of insecticidal rate. The compounds of the present invention: No.1-001, 1-002, 1-003, 1-004, 1-005, 1-006, 1-007, 1-008, 1-009, 1-010, 1-011, 1-012, 1-013, 1-014, 1-015, 1-016, 1-017, 1-018, 1-019, 1-020, 1-021, 1-022, 1-023, 1-024, 1-025, 1-026, 1-027, 1-028, 1-029, 1-030, 1-031, 1-032, 1-033, 1-034, 1-035, 1-036, 1-037, 1-038, 1-039, 1-040, 1-041, 1-042, 1-043, 1-044, 1-045, 1-046, 1-047, 1-048, 1-050, 1-051, 1-052, 1-053, 1-054, 1-055, 1-056, 1-057, 1-058, 1-059, 1-060, 1-061, 1-062, 1-063, 1-064, 1-065, 1-066, 1-067, 1-068, 1-069, 1-070, 1-071, 1-072, 1-073, 1-074, 1-075, 1-076, 1-077, 1-078, 1-079, 1-080, 1-081, 1-082, 1-083, 1-084, 1-085, 1-086, 1-087, 1-088, 1-089, 1-090, 2-001, 2-002, 2-003, 2-004, 2-005, 2-006, 2-007, 2-008, 2-009, 2-010, 2-011, 2-012, 2-013, 2-014, 2-015, 2-016, 2-017, 2-018, 2-019, 2-020, 2-021, 2-022, 2-023, 2-024, 2-025, 2-026, 2-027, 2-028, 2-029, 2-030, 2-031, 2-032, 2-033, 2-034, 2-035, 2-036, 2-037, 2-038, 3-001, 3-002, 3-004, 3-005, 3-006, 3-007, 3-008, 3-009, 3-010, 3-011, 3-012, 3-013, 3-014, 3-015, 4-001, 4-002.

Test Example 2 Insecticidal test against Common cutworm

A 10% emulsifiable concentrate (depending on the compounds, 25% wettable powder was applied for the test) of the compound of the present invention was diluted with water containing a spreading agent to prepare a chemical solution with a concentration of 100 ppm. To the chemical solution was dipped leaves of Chinese olive for about 10 seconds, and after air-drying, they were placed in a laboratory dish, then, 10 Common cutworms (Spodoptera litura) with second instar larvae per the dish were released therein, and the dish was covered with a lid having holes and contained at a thermostat chamber at 25°C. A number of dead insect(s) after 6 days was counted and a rate of dead insects was calculated by the following calculation formula. Incidentally, the test was carried out with two districts.

Rate of dead insects (%) = (Number of dead insects/Number of released insects)x100

As a result, the following compounds showed 80% or more of insecticidal rate. The compounds of the present invention: No.1-001, 1-003, 1-005, 1-006, 1-007, 1-009, 1-010, 1-011, 1-012, 1-013, 1-014, 1-015, 1-016, 1-017, 1-020, 1-021, 1-022, 1-023, 1-024, 1-025, 1-026, 1-027, 1-028, 1-029, 1-031, 1-032, 1-034, 1-035, 1-036, 1-038, 1-040, 1-041, 1-042, 1-043, 1-044, 1-045, 1-047, 1-048, 1-050, 1-051, 1-052, 1-053, 1-054, 1-055, 1-056, 1-057, 1-058, 1-059, 1-063, 1-065, 1-066, 1-067, 1-069, 1-070, 1-071, 1-072, 1-073, 1-074, 1-075, 1-076, 1-077, 1-078, 1-079, 1-080, 1-081, 1-082, 1-083, 1-084, 1-085, 1-086, 1-087, 1-088, 1-089, 1-090, 2-001, 2-004, 2-007, 2-008, 2-012, 2-016, 2-017, 2-018, 2-019, 2-020, 2-022, 2-024, 2-025, 2-026, 2-027, 2-028, 2-029, 2-030, 2-033, 2-034, 2-035, 2-036, 2-037, 3-001, 3-002, 3-004, 3-005, 3-006, 3-007, 3-008, 3-009, 3-010, 3-012, 3-013, 3-014, 3-015, 4-001, 4-002.

Test example 3 Insecticidal test against Oriental tea tortrix

A 10% emulsifiable concentrate (depending on the compounds, 10% wettable powder was applied for the test) of the compound of the present invention was diluted with water containing a spreading agent to prepare a chemical solution with a concentration of 100 ppm. To the chemical solution was dipped leaves of Chinese olive for about 10 seconds, and after air-drying, they were placed in a laboratory dish, then, 5 Oriental tea tortrix (Homona magnanima) with second instar larvae per the dish were released therein, and the dish was covered with a lid having holes and contained at a thermostat chamber at 25°C. A number of dead insect(s) after 6 days was counted and a rate of dead insects was calculated in the same manner as in Test Example 1. Incidentally, the test was carried out with two districts.

As a result, the following compounds showed 80% or more of insecticidal rate. The compounds of the present invention: No.1-001, 1-003, 1-004, 1-005, 1-006, 1-007, 1-009, 1-010, 1-011, 1-012, 1-013, 1-017, 1-020, 1-021, 1-022, 1-023, 1-024, 1-025, 1-026, 1-027, 1-028, 1-032, 1-034, 1-035, 1-036, 1-038, 1-040, 1-041, 1-042, 1-043, 1-044, 1-045, 1-048, 1-050, 1-051, 1-052, 1-053, 1-054, 1-056, 1-057, 1-058, 1-059, 1-062, 1-063, 1-065, 1-066, 1-067, 1-069, 1-070, 1-071, 1-072, 1-073, 1-075, 1-076, 1-077, 1-078, 1-079, 1-081, 1-082, 1-083, 1-084, 1-085, 1-086, 1-088, 1-089, 2-008, 2-016, 2-018, 2-019, 2-024, 2-029, 2-033, 2-035, 3-001, 3-002, 3-008, 3-009, 3-014, 3-015, 4-001, 4-002.

Test example 4 Insecticidal test against Cucurbit leaf beetle

A 10% emulsifiable concentrate (depending on the compounds, 10% wettable powder was applied for the test) of the compound of the present invention was diluted with water containing a spreading agent to prepare a chemical solution with a concentration of 500 ppm. To the chemical solution was dipped leaves of cucumber for about 10 seconds, and after air-drying, they were placed in a laboratory dish, then, 5 Cucurbit leaf beetle (Aulacophora femoralis) with second instar larvae per the dish were released therein, and the dish was covered with a lid having holes and contained at a thermostat chamber at 25°C. A number of dead insect(s) after 6 days was counted and a rate of dead insects was calculated in the same manner as in Test Example 1. Incidentally, the test was carried out with two districts.

As a result, the following compounds showed 100% of insecticidal rate. The compounds of the present invention: No.1-001, 1-004, 1-005, 1-006, 1-007, 1-009, 1-010, 1-011, 1-012, 1-013, 1-014, 1-016, 1-017, 1-018, 1-020, 1-022, 1-023, 1-024, 1-025, 1-026, 1-027, 1-028, 1-031, 1-032, 1-035, 1-036, 1-038, 1-040, 1-041, 1-042, 1-043, 1-044, 1-045,

1-047, 1-048, 1-050, 1-052, 1-053, 1-054, 1-055, 1-056, 1-057, 1-058, 1-059, 1-062, 1-063, 1-064, 1-066, 1-067, 1-068, 1-069, 1-070, 1-071, 1-072, 1-073, 1-074, 1-075, 1-077, 1-078, 1-079, 1-080, 1-081, 1-082, 1-083, 1-084, 1-085, 1-086, 1-087, 1-088, 1-089, 2-001, 2-004, 2-008, 2-009, 2-010, 2-016, 2-017, 2-018, 2-019, 2-021, 2-022, 2-024, 2-025, 2-026, 2-028, 2-029, 2-030, 2-033, 2-034, 2-035, 2-036, 2-037, 3-001, 3-005, 3-006, 3-007, 3-008, 3-010, 3-012, 3-013, 3-014, 4-001, 4-002.

Test example 5 Insecticidal test (Comparative test) against Common cutworm

10% emulsifiable concentrates of the compound of the present invention and comparative compound were diluted with water containing a spreading agent to prepare chemical solutions with a predetermined concentration. To the chemical solutions was dipped leaves of Chinese olive for about 10 seconds, and after air-drying, they were placed in a laboratory dish, then, 7 Common cutworm (Spodoptera litura) with third instar larvae per the dish were released therein, and the dish was covered with a lid having holes and contained at a thermostat chamber at 25°C. Artificial feed (1 $cm^3$) was added after 2 days, a number of dead insect(s) was counted after 6 days and a rate of dead insects was calculated by the following calculation formula. Incidentally, the test was carried out with two districts.

Rate of dead insects (%) = (Number of dead insects/Number of released insects)x100

Rate of dead insects at the respective concentrations of the respective test compounds are shown in Table 11.

Table 11

| Test compounds | Concentration (ppm) | | | | | |
|---|---|---|---|---|---|---|
| | 33 | 10 | 3 | 1 | 0.3 | 0.1 |
| Present compound No.1-036 | 100 | 100 | 100 | 85.7 | 28.6 | 14.3 |
| Present compound No.1-084 | 100 | 100 | 71.4 | 21.3 | 0 | |
| Present compound No.1-087 | 100 | 100 | 100 | 71.4 | 21.3 | 0 |
| Comparative compound A | 0 | | | | | |

Comparative compound A: EP 1006107 A, Compound No.472

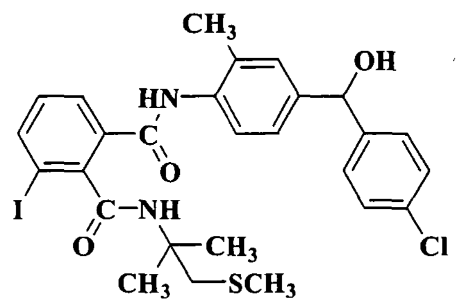

Utilizable field in industry

The substituted benzanilide compound according to the present invention is an extremely useful compound which shows excellent noxious organism controlling activity, in particular insecticidal and acaricidal activities, and causes substantially no bad effect on non-target creatures such as mammals, fishes and useful insects, etc.

**Claims**

1. A substituted benzanilide compound represented by the formula (1):

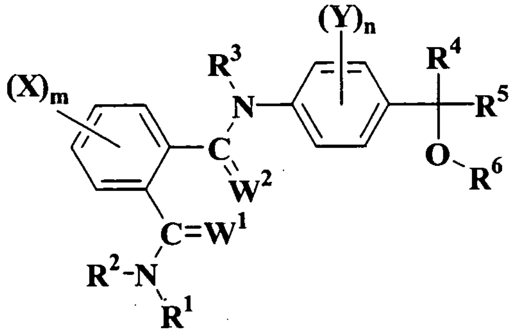

(1)

wherein $W^1$ and $W^2$ each independently represent an oxygen atom or a sulfur atom,
X represents a halogen atom, cyano, nitro, azide, -SCN, $-SF_5$, a $C_1$ to $C_6$ alkyl, a ($C_1$ to $C_6$) alkyl optionally substituted by $R^7$, a $C_3$ to $C_8$ cycloalkyl, a ($C_3$ to $C_8$) cycloalkyl optionally substituted by $R^7$, a $C_2$ to $C_6$ alkenyl, a ($C_2$ to $C_6$) alkenyl optionally substituted by $R^7$, a $C_3$ to $C_8$ cycloalkenyl, a $C_3$ to $C_8$ halocycloalkenyl, a $C_2$ to $C_6$ alkynyl, a ($C_2$ to $C_6$) alkynyl optionally substituted by $R^7$, -OH, $-OR^8$, $-OS(O)_2R^8$, -SH, $-S(O)_rR^8$, -CHO, $-C(O)_R{}^9$, $-C(O)OR^9$, -C (O)$SR^9$, $-C(O)NHR^{10}$, $-C(O)N(R^{10})R^9$, $-C(S)OR^9$, $-C(S)SR^9$, $-C(S)NHR^{10}$, $-C(S)N(R^{10})R^9$, $-CH=NOR^{11}$, $-C(R^9)$ $=NOR^{11}$, $-S(O)_2OR^9$, $-S(O)_2NHR^{10}$, $-S(O)_2N(R^{10})R^9$, $-Si(R^{13})(R^{14})R^{12}$, phenyl, a phenyl substituted by $(Z)_{p1}$, L or M, when m is 2, 3 or 4, each X may be the same or different from each other, and when two Xs are adjacent to each other, the adjacent two Xs may form a 5-membered ring or 6-membered ring with the carbon atoms to which two Xs are bonded by forming $-CH_2CH_2CH_2-$, $-CH_2CH_2O-$, $-CH_2OCH_2-$, $-OCH_2O-$, $-CH_2CH_2S-$, $-CH_2SCH_2-$, $-CH_2CH_2N$ $(R^{15})-$, $-CH_2N(R^{15})CH_2-$, $-CH_2CH_2CH_2CH_2-$, $-CH_2CH_2CH_2O-$, $-CH_2CH_2OCH_2-$, $-CH_2OCH_2O-$, $-OCH_2CH_2O-$, $-OCH_2CH_2S-$, $-CH_2CH=CH-$, -OCH=CH-, -SCH=CH-, $-N(R^{15})CH=CH-$, -OCH=N-, -SCH=N-, $-N(R^{15})CH=N-$, -N $(R^{15})N=CH-$, -CH=CHCH=CH-, $-OCH_2CH=CH-$, -N=CHCH=CH-, -N=CHCH=N- or -N=CHN=CH-, and at this time, each hydrogen atom bonded to the respective carbon atoms which form the ring may be optionally substituted by Z, and further when it is substituted by two or more Zs at the same time, each Z may be the same or different from each other,
Y represents a halogen atom, cyano, nitro, a $C_1$ to $C_6$ alkyl, a ($C_1$ to $C_6$) alkyl optionally substituted by $R^7$, a $C_3$ to $C_8$ cycloalkyl, $-OR^8$, $-S(O)_rR^8$, $-NH_2$, a $C_1$ to $C_6$ alkylamino, a di($C_1$ to $C_6$ alkyl)amino or $-Si(R^{13})(R^{14})R^{12}$, when n is 2, 3 or 4, each Y may be the same or different from each other, and when two Ys are adjacent to each other, the adjacent two Ys may form a 5-membered ring or 6-membered ring with the carbon atoms to which two Ys are bonded by forming $-CH_2CH_2CH_2-$, $-CH_2CH_2O-$, $-CH_2OCH_2-$ $-OCH_2O-$, $-CH_2CH_2S-$, $-CH_2SCH_2-$, $-SCH_2S-$, $-CH_2CH_2CH_2CH_2-$, $-CH_2CH_2CH_2O-$, $-CH_2CH_2OCH_2-$. $-CH_2OCH_2O-$, $-OCH_2CH_2O-$ $-OCH_2CH_2S-$, $-SCH_2CH_2S-$, -OCH=N- or -SCH=N-, and at this time, each hydrogen atom bonded to the respective carbon atoms which form the ring may be optionally substituted by Z, and further when it is substituted by two or more Zs at the same time, each Z may be the same or different from each other,
$R^1$ represents a hydrogen atom, cyano, a $C_1$ to $C_{12}$ alkyl, a ($C_1$ to $C_{12}$) alkyl optionally substituted by $R^{16}$, a $C_3$ to $C_{12}$ cycloalkyl, a ($C_3$ to $C_{12}$) cycloalkyl optionally substituted by $R^{16}$, a $C_3$ to $C_{12}$ alkenyl, a ($C_3$ to $C_{12}$) alkenyl optionally substituted by $R^{16}$, a $C_3$ to $C_{12}$ cycloalkenyl, a $C_3$ to $C_{12}$ halocycloalkenyl, a $C_3$ to $C_{12}$ alkynyl, a ($C_3$ to $C_{12}$) alkynyl optionally substituted by $R^{16}$, -OH, a $C_1$ to $C_8$ alkoxy, a $C_3$ to $C_8$ alkenyloxy, a $C_3$ to $C_8$ haloalkenyloxy, phenoxy, a phenoxy substituted by $(Z)_{p1}$, a phenyl($C_1$ to $C_4$) alkoxy, a phenyl($C_1$ to $C_4$) alkoxy substituted by $(Z)_{p1}$, $-N(R^{20})R^{19}$, phenyl, a phenyl substituted by $(Z)_{p1}$, Lor M,
$R^2$ and $R^3$ each independently represent a hydrogen atom, cyano, a $C_1$ to $C_{12}$ alkyl, a ($C_1$ to $C_{12}$) alkyl optionally substituted by $R^{16}$, a $C_3$ to $C_{12}$ alkenyl, a $C_3$ to $C_{12}$ haloalkenyl, a $C_3$ to $C_{12}$ alkynyl, a $C_3$ to $C_{12}$ haloalkynyl, -OH, a $C_1$ to $C_8$ alkoxy, a $C_1$ to $C_6$ alkylthio, a $C_1$ to $C_6$ haloalkylthio, phenylthio, a phenylthio substituted by $(Z)_{p1}$, -S $(O)_2R^9$, $-SN(R^{18})R^{17}$, $-S(O)_2N(R^{10})R^9$, $-N(R^{20})R^{19}$, $-C(O)R^9$, $-C(O)OR^9$, $-C(O)SR^9$, $-C(O)N(R^{10})R^9$, $-C(S)OR^9$, -C (S)$SR^9$, $-C(S)N(R^{10})R^9$, phenyl or a phenyl substituted by $(Z)_{p1}$, or $R^2$ is combined with $R^1$ to form a $C_2$ to $C_6$ alkylene chain whereby it may form a 3 to 7-membered ring with the nitrogen atom to which they are bonded, and the alkylene chain at this time may contain one oxygen atom, sulfur atom or nitrogen atom, and may be optionally substituted by a halogen atom, a $C_1$ to $C_6$ alkyl group, a $C_1$ to $C_6$ haloalkyl group, a $C_1$ to $C_6$ alkoxy group, a $C_1$ to $C_6$ alkylcarbonyl group or a $C_1$ to $C_6$ alkoxycarbonyl group,
$R^4$ represents a $C_1$ to $C_6$ alkyl, a $C_1$ to $C_6$ haloalkyl, a ($C_1$ to $C_6$) alkyl optionally substituted by $R^{21}$, a ($C_1$ to $C_6$) haloalkyl optionally substituted by $R^{21}$, a $C_3$ to $C_8$ cycloalkyl, a $C_3$ to $C_8$ halocycloalkyl, a ($C_3$ to $C_8$) cycloalkyl optionally substituted by $R^{21}$, a ($C_3$ to $C_8$) halocycloalkyl optionally substituted by $R^{21}$, a $C_3$ to $C_6$ alkenyl, a $C_3$ to $C_6$ haloalkenyl, a $C_3$ to $C_6$ alkynyl, a $C_3$ to $C_6$ haloalkynyl, phenyl, a phenyl substituted by $(Z)_{p1}$, L or M,
$R^5$ represents cyano, a ($C_1$ to $C_6$) alkyl optionally substituted by $R^{21}$, a ($C_1$ to $C_6$) haloalkyl optionally substituted

by $R^{21}$, a ($C_3$ to $C_8$) cycloalkyl optionally substituted by $R^{21}$, a ($C_3$ to $C_8$) halocycloalkyl optionally substituted by $R^{21}$, a ($C_2$ to $C_6$) alkenyl optionally substituted by $R^{21}$, a $C_3$ to $C_8$ cycloalkenyl, a $C_3$ to $C_8$ halocycloalkenyl, a ($C_2$ to $C_6$) alkynyl optionally substituted by $R^{21}$, $-OR^8$, $-S(O)_rR^8$, $-N(R^{10})R^9$, -CHO, $-C(O)R^9$, $-CH=NOR^{11}$, $-C(R^9)=NOR^{11}$, $-C(O)OR^9$, $-C(O)SR^9$, $-C(O)NHR^{10}$, $-C(O)N(R^{10})R^9$, $-C(S)OR^9$, $-C(S)SR^9$, $-C(S)NHR^{10}$, $-C(S)N(R^{10})R^9$, phenyl, a phenyl substituted by $(Z)_{p1}$, biphenyl, a biphenyl substituted by $(Z)_{p1}$, phenoxyphenyl, a phenoxyphenyl substituted by $(Z)_{P1}$, pyridyloxyphenyl, a pyridyloxyphenyl substituted by $(Z)p_1$, phenylthiophenyl, a phenylthiophenyl substituted by $(Z)_{p1}$, phenylsulfinylphenyl, a phenylsulfinylphenyl substituted by $(Z)_{P1}$, phenylsulfonylphenyl, a phenylsulfonylphenyl substituted by $(Z)_{p1}$, L or M, or it forms a $C_2$ to $C_3$ alkylene chain with Y present at the adjacent position in combination whereby it may form a 5 to 6-membered ring which fuses with a benzene ring, and the alkylene chain at this time may contain one oxygen atom, sulfur atom or nitrogen atom, and may be optionally substituted by a halogen atom or a $C_1$ to $C_6$ haloalkyl group,

$R^6$ represents a hydrogen atom, a $C_1$ to $C_6$ alkyl, a $C_1$ to $C_6$ haloalkyl, a ($C_1$ to $C_6$) alkyl optionally substituted by $R^{21}$, a ($C_1$ to $C_6$) haloalkyl optionally substituted by $R^{21}$, a $C_3$ to $C_8$ cycloalkyl, a $C_3$ to $C_6$ alkenyl, a $C_3$ to $C_6$ haloalkenyl, a phenyl($C_3$ to $C_6$) alkenyl, a phenyl($C_3$ to $C_6$) alkenyl substituted by $(Z)_{p1}$, a $C_3$ to $C_8$ cycloalkenyl, a $C_3$ to $C_6$ alkynyl, a $C_3$ to $C_6$ haloalkynyl, a phenyl($C_3$ to $C_6$) alkynyl, a phenyl($C_3$ to $C_6$) alkynyl substituted by $(Z)_{P1}$, $-S(O)_2R^9$, $-C(O)R^9$, $-C(O)OR^9$, $-C(O)SR^9$, $-C(S)OR^9$, $-C(S)SR^9$, $-C(O)NHR^{10}$, $-C(O)N(R^{10})R^9$, $-C(S)NHR^{10}$, $-C(S)N(R^{10})R^9$, $-Si(R^{13})(R^{14})R^{12}$, $-P(O)(OR^{22})_2$, $-P(S)(OR^{22})_2$ or M,

L represents an aromatic heterocyclic ring represented by any of the formula L-1 to the formula L-58,

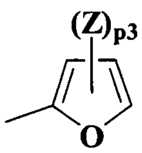
L-1

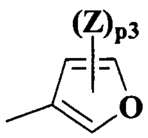
L-2

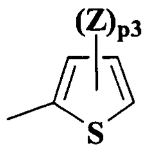
L-3

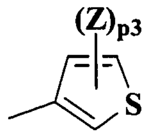
L-4

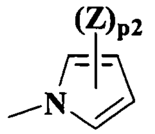
L-5

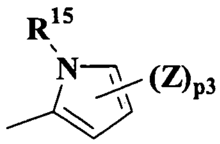
L-6

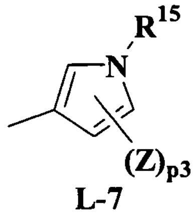
L-7

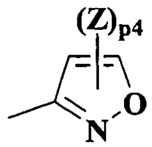
L-8

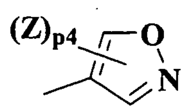
L-9

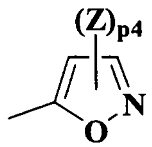
L-10

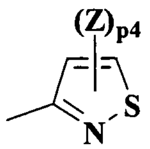
L-11

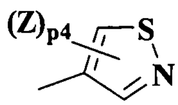
L-12

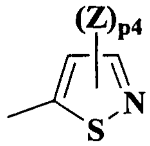
L-13

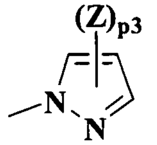
L-14

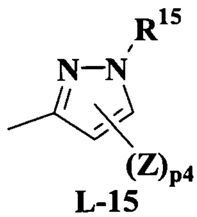
L-15

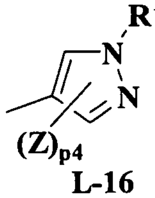
L-16

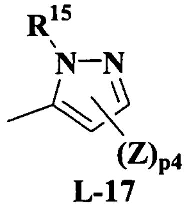
L-17

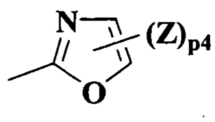
L-18

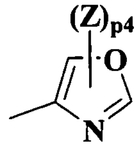
L-19

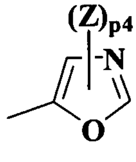
L-20

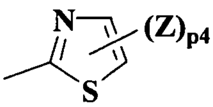
L-21

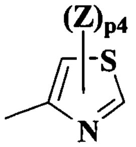
L-22

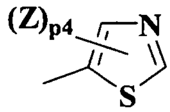
L-23

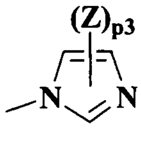
L-24

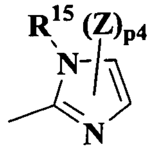
L-25

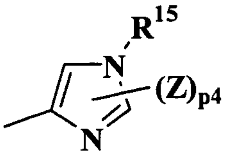
L-26

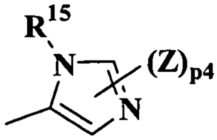
L-27

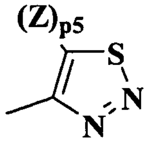
L-28

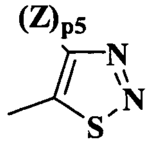
L-29

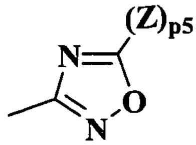
L-30

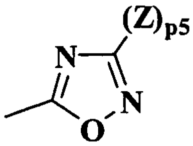
L-31

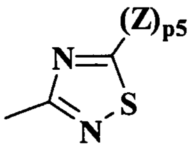
L-32

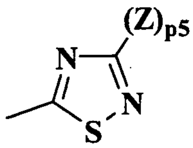
L-33

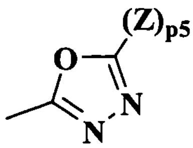
L-34

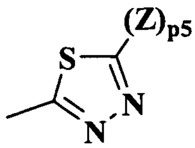
L-35

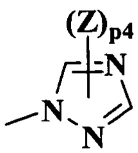
L-36

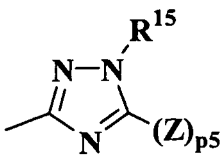
L-37

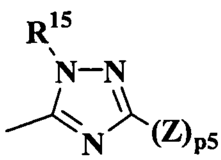
L-38

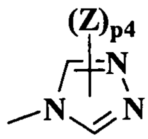
L-39

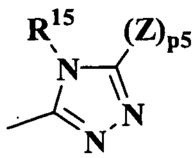
L-40

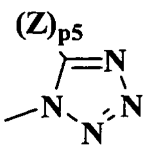
L-41

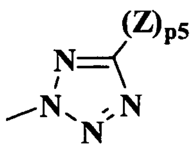
L-42

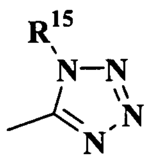
L-43

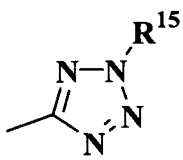
L-44

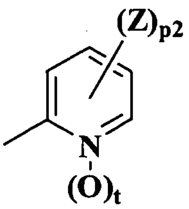
L-45

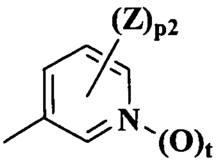
L-46

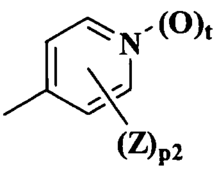
L-47

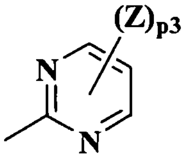
L-48

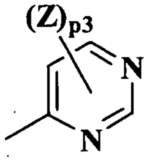
L-49

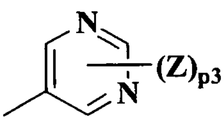
L-50

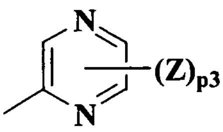
L-51

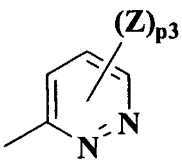
L-52

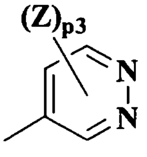
L-53

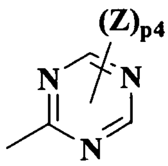
L-54

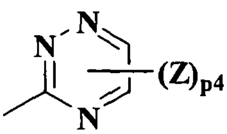
L-55

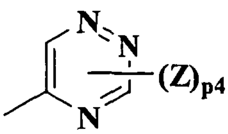
L-56

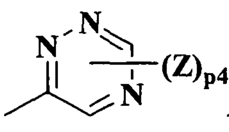
L-57

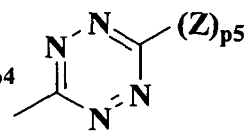
L-58

M represents an aromatic heterocyclic ring represented by any of the formula M-1 to the formula M-28,

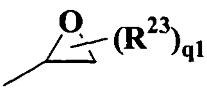
M-1

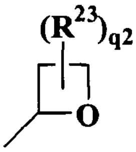
M-2

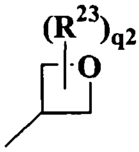
M-3

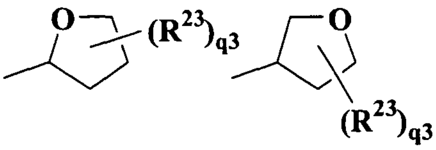
M-4 M-5

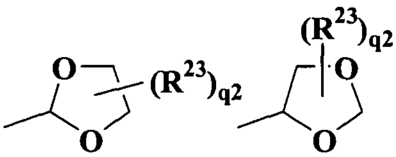
M-6 M-7

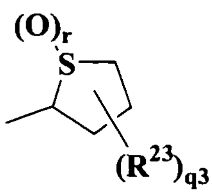
M-8

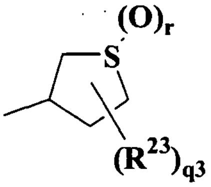
M-9

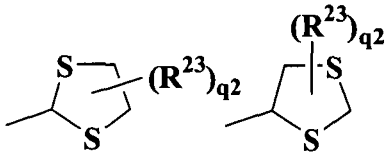
M-10 M-11 .

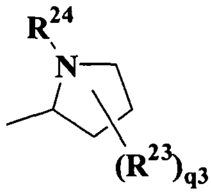
M-12

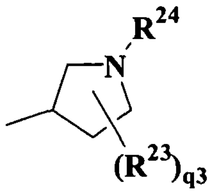
M-13

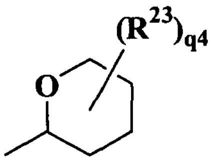
M-14

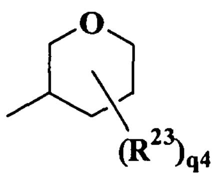
M-15

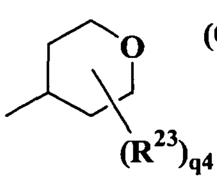
M-16

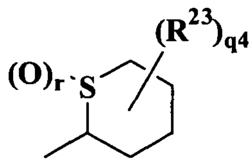
M-17

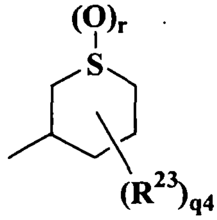
M-18

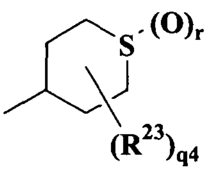
M-19

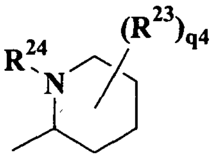
M-20

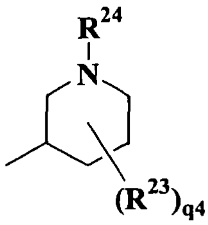
M-21

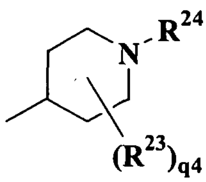
M-22

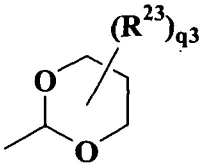
M-23

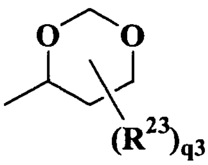
M-24

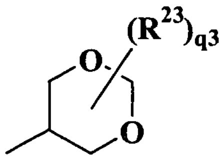
M-25

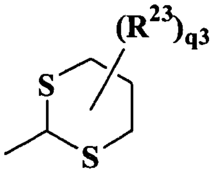
M-26

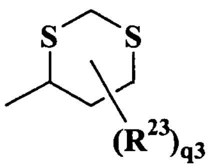
M-27

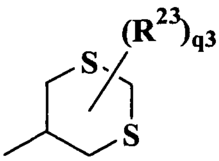
M-28

Z represents a halogen atom, cyano, nitro, azide, -SCN, -$SF_5$, a $C_1$ to $C_6$ alkyl, a $C_1$ to $C_6$ haloalkyl, a $C_1$ to $C_3$ alkoxy($C_1$ to $C_3$) alkyl, a $C_1$ to $C_3$ haloalkoxy($C_1$ to $C_3$) alkyl, a cyano($C_1$ to $C_6$) alkyl, a hydroxy($C_1$ to $C_3$) haloalkyl, a $C_1$ to $C_3$ alkoxy($C_1$ to $C_3$) haloalkyl, a $C_1$ to $C_3$ haloalkoxy($C_1$ to $C_3$) haloalkyl, a $C_1$ to $C_3$ alkylthio ($C_1$ to $C_3$) alkyl, a $C_1$ to $C_3$ haloalkylthio ($C_1$ to $C_3$) alkyl, a $C_1$ to $C_3$ alkylsulfinyl($C_1$ to $C_3$) alkyl, a $C_1$ to $C_3$ haloalkylsulfinyl($C_1$ to $C_3$) alkyl, a $C_1$ to $C_3$ alkylsulfonyl($C_1$ to $C_3$) alkyl, a $C_1$ to $C_3$ haloalkylsulfonyl-($C_1$ to $C_3$) alkyl, a $C_3$ to $C_8$ cycloalkyl, a $C_3$ to $C_8$ halocycloalkyl, a $C_2$ to $C_6$ alkenyl, a $C_2$ to $C_6$ haloalkenyl, a $C_3$ to $C_8$ cycloalkenyl, a $C_3$ to $C_8$ halocycloalkenyl, a $C_2$ to $C_6$ alkynyl, a $C_2$ to $C_6$ haloalkynyl, -OH, a $C_1$ to $C_6$ alkoxy, a $C_1$ to $C_6$ haloalkoxy, a $C_1$ to $C_3$ haloalkoxy-($C_1$ to $C_3$) haloalkoxy, a $C_2$ to $C_6$ alkenyloxy, a $C_2$ to $C_6$ haloalkenyloxy, a $C_3$ to $C_6$ alkynyloxy, a $C_3$ to $C_6$ haloalkynyloxy, a $C_1$ to $C_6$ alkylsulfonyloxy, a $C_1$ to $C_6$ haloalkylsulfonyloxy, -SH, a $C_1$ to $C_6$ alkylthio, a $C_1$ to $C_6$ haloalkylthio, a $C_1$ to $C_6$ alkylsulfinyl, a $C_1$ to $C_6$ haloalkylsulfinyl, a $C_1$ to $C_6$ alkylsulfonyl, a $C_1$ to $C_6$ haloalkylsulfonyl, -$NH_2$, a $C_1$ to $C_6$ alkylamino, a di($C_1$ to $C_6$ alkyl)amino, a $C_1$ to $C_6$ alkylsulfonylamino, a $C_1$ to $C_6$ haloalkylsulfonylamino, a $C_1$ to $C_6$ alkoxycarbonyl, a $C_1$ to $C_6$ haloalkoxycarbonyl, -C(O)$NH_2$, a $C_1$ to $C_6$ alkylaminocarbonyl, a di($C_1$ to $C_6$ alkyl) aminocarbonyl, -C(S)$NH_2$, a $C_1$ to $C_6$ alkylaminosulfonyl, a di($C_1$ to $C_6$ alkyl)aminosulfonyl or a tri($C_1$ to $C_6$ alkyl)

silyl, when p1, p2, p3 or p4 is an integer of 2 or more, each Z may be the same or different from each other, further, when two Zs are adjacent to each other, the adjacent two Zs may form a 5-membered ring or 6-membered ring with the carbon atoms to which two Zs are bonded by forming $-CH_2CH_2CH_2-$, $-CH_2CH_2O-$, $-CH_2OCH_2-$, $-OCH_2O-$, $-CH_2CH_2S-$, $-CH_2SCH_2-$, $-CH_2CH_2CH_2CH_2-$, $-CH_2CH_2CH_2O-$, $-CH_2CH_2OCH_2-$, $-CH_2OCH_2O-$, $-OCH_2CH_2O-$, $-OCH_2CH_2S-$ or -CH=CHCH=CH-, and at this time, each hydrogen atom bonded to the respective carbon atoms which form the ring may be optionally substituted by a halogen atom or a $C_1$ to $C_6$ alkyl group,

$R^7$ represents a halogen atom, cyano, a $C_3$ to $C_8$ cycloalkyl, a $C_3$ to $C_8$ halocycloalkyl, -OH, $-OR^8$, -SH, $-S(O)_rR^8$, $-N(R^{10})R^9$, $-N(R^{10})CHO$, $-N(R^{10})C(O)R^9$, $-N(R^{10})C(O)OR^9$, $-N(R^{10})C(O)SR^9$, $-N(R^{10})C(S)OR^9$, $-N(R^{10})C(S)SR^9$, $-N(R^{10})S(O)_2R^9$, $-C(O)OR^9$, $-C(O)N(R^{10})R^9$, $-Si(R^{13})(R^{14})R^{12}$, phenyl, a phenyl substituted by $(Z)_{p1}$, L or M,

$R^8$ represents a $C_1$ to $C_6$ alkyl, a ($C_1$ to $C_6$) alkyl optionally substituted by $R^{25}$, a $C_3$ to $C_8$ cycloalkyl, a ($C_3$ to $C_8$) cycloalkyl optionally substituted by $R^{25}$, a $C_2$ to $C_6$ alkenyl, a ($C_2$ to $C_6$) alkenyl optionally substituted by $R^{25}$, a $C_3$ to $C_8$ cycloalkenyl, a $C_3$ to $C_8$ halocycloalkenyl, a $C_3$ to $C_6$ alkynyl, a ($C_3$ to $C_6$) alkynyl optionally substituted by $R^{25}$, phenyl, a phenyl substituted by $(Z)_{p1}$, L or M,

$R^9$ represents a $C_1$ to $C_6$ alkyl, a $C_1$ to $C_6$ haloalkyl, a $C_3$ to $C_6$ cycloalkyl ($C_1$ to $C_4$) alkyl, a $C_1$ to $C_6$ alkoxy($C_1$ to $C_4$) alkyl, a $C_1$ to $C_6$ alkylthio ($C_1$ to $C_4$) alkyl, a cyano($C_1$ to $C_6$) alkyl, a phenyl($C_1$ to $C_4$) alkyl, a phenyl($C_1$ to $C_4$) alkyl substituted by $(Z)_{p1}$, an L-($C_1$ to $C_4$) alkyl, an M-($C_1$ to $C_4$) alkyl, a $C_3$ to $C_8$ cycloalkyl, a $C_3$ to $C_8$ halocycloalkyl, a $C_3$ to $C_6$ alkenyl, a $C_3$ to $C_6$ haloalkenyl, a $C_3$ to $C_6$ alkynyl, phenyl or a phenyl substituted by $(Z)_{p1}$,

$R^{10}$ represents a hydrogen atom or a $C_1$ to $C_6$ alkyl, or $R^9$ and $R^{10}$ are combined in combination to form a $C_2$ to $C_6$ alkylene chain whereby they may form a 3 to 7-membered ring with an atom(s) to which they are bonded, and at this time, the alkylene chain may contain one oxygen atom, sulfur atom or nitrogen atom, and may be optionally substituted by a halogen atom, a $C_1$ to $C_6$ alkyl group, a $C_1$ to $C_6$ alkoxy group, a formyl group, a $C_1$ to $C_6$ alkylcarbonyl group or a $C_1$ to $C_6$ alkoxycarbonyl group,

$R^{11}$ represents a hydrogen atom, a $C_1$ to $C_6$ alkyl, a $C_1$ to $C_6$ haloalkyl, a phenyl-($C_1$ to $C_4$) alkyl, a phenyl($C_1$ to $C_4$) alkyl substituted by $(Z)_{p1}$, a $C_3$ to $C_6$ alkenyl, a $C_3$ to $C_6$ haloalkenyl, a $C_3$ to $C_6$ alkynyl or a $C_3$ to $C_6$ haloalkynyl, or $R^{11}$ is combined with $R^9$ to form a $C_2$ to $C_4$ alkylene chain whereby it may form a 5 to 7-membered ring with an atom(s) to which they are bonded, and at this time, the alkylene chain may contain one oxygen atom, sulfur atom or nitrogen atom, and may be optionally substituted by a halogen atom or a $C_1$ to $C_6$ alkyl group,

$R^{12}$ represents a $C_1$ to $C_6$ alkyl, a $C_1$ to $C_6$ haloalkyl, a $C_3$ to $C_6$ alkenyl, phenyl or a phenyl substituted by $(Z)_{p1}$,

$R^{13}$ and $R^{14}$ each independently represent a $C_1$ to $C_6$ alkyl or a $C_1$ to $C_6$ haloalkyl,

$R^{15}$ represents a hydrogen atom, a $C_1$ to $C_6$ alkyl, a $C_1$ to $C_6$ haloalkyl, a $C_1$ to $C_6$ alkoxycarbonyl($C_1$ to $C_4$) alkyl, a $C_1$ to $C_6$ haloalkoxycarbonyl($C_1$ to $C_4$) alkyl, a phenyl($C_1$ to $C_4$) alkyl, a phenyl($C_1$ to $C_4$) alkyl substituted by $(Z)_{p1}$, a $C_3$ to $C_6$ alkenyl, a $C_3$ to $C_6$ haloalkenyl, a $C_3$ to $C_6$ alkynyl, a $C_3$ to $C_6$ haloalkynyl, a $C_1$ to $C_6$ alkoxy, a $C_1$ to $C_6$ alkoxycarbonyl, a $C_1$ to $C_6$ haloalkoxycarbonyl, phenyl or a phenyl substituted by $(Z)_{P1}$,

$R^{16}$ represents a halogen atom, cyano, nitro, a $C_3$ to $C_8$ cycloalkyl, a $C_3$ to $C_8$ halocycloalkyl, $-OR^{26}$, $-N(R^{27})R^{26}$, -SH, $-S(O)_rR^{28}$, $-SO_2NHR^{30}$, $-SO_2N(R^{30})R^{29}$, -CHO, $C(O)R^{29}$, -C(O)OH, $-C(O)OR^{29}$, $-C(O)SR^{29}$, $-C(O)NHR^{30}$, $-C(O)N(R^{30})R^{29}$, $-C(O)C(O)OR^{29}$, $-C(R^{32})-NOH$, $-C(R^{32})=NOR^{31}$, $-Si(R^{13})(R^{14})R^{12}$, $-P(O)(OR^{22})_2$, $-P(S)(OR^{22})_2$, $-P(phenyl)_2$, $-P(O)(phenyl)_2$, phenyl, a phenyl substituted by $(Z)_{p1}$, L or M,

$R^{17}$ represents a $C_1$ to $C_{12}$ alkyl, a $C_1$ to $C_{12}$ haloalkyl, a $C_1$ to $C_{12}$ alkoxy($C_1$ to $C_{12}$) alkyl, a cyano($C_1$ to $C_{12}$) alkyl, a $C_1$ to $C_{12}$ alkoxycarbonyl($C_1$ to $C_{12}$) alkyl, a phenyl($C_1$ to $C_4$) alkyl, a phenyl($C_1$ to $C_4$) alkyl substituted by $(Z)_{p1}$, a $C_3$ to $C_{12}$ alkenyl, a $C_3$ to $C_{12}$ haloalkenyl, a $C_3$ to $C_{12}$ alkynyl, a $C_3$ to $C_{12}$ haloalkynyl, a $C_1$ to $C_{12}$ alkylcarbonyl, a $C_1$ to $C_{12}$ alkoxycarbonyl, phenyl or a,phenyl substituted by $(Z)_{p1}$,

$R^{18}$ represents a $C_1$ to $C_{12}$ alkyl, a $C_1$ to $C_{12}$ haloalkyl, a $C_1$ to $C_{12}$ alkoxy($C_1$ to $C_{12}$) alkyl, a cyano($C_1$ to $C_{12}$) alkyl, a $C_1$ to $C_{12}$ alkoxycarbonyl($C_1$ to $C_{12}$) alkyl, a phenyl($C_1$ to $C_4$) alkyl, a phenyl($C_1$ to $C_4$) alkyl substituted by $(Z)_{p1}$, a $C_3$ to $C_{12}$ alkenyl, a $C_3$ to $C_{12}$ haloalkenyl, a $C_3$ to $C_{12}$ alkynyl, a $C_3$ to $C_{12}$ haloalkynyl, phenyl or a phenyl substituted by $(Z)_{p1}$, or $R^{17}$ and $R^{18}$ are combined in combination to form a $C_4$ to $C_7$ alkylene chain whereby it may form a 5 to 8-membered ring with the nitrogen atom to which they are bonded, and at this time, the alkylene chain may contain one oxygen atom or sulfur atom, and may be optionally substituted by a $C_1$ to $C_4$ alkyl group or a $C_1$ to $C_4$ alkoxy group,

$R^{19}$ represents a hydrogen atom, a $C_1$ to $C_6$ alkyl, a $C_1$ to $C_6$ haloalkyl, a phenyl-($C_1$ to $C_4$) alkyl, a phenyl($C_1$ to $C_4$) alkyl substituted by $(Z)_{p1}$, a $C_3$ to $C_6$ alkenyl, a $C_3$ to $C_6$ haloalkenyl, a $C_3$ to $C_6$ alkynyl, -CHO, a $C_1$ to $C_6$ alkylcarbonyl, a $C_1$ to $C_6$ haloalkylcarbonyl, a $C_1$ to $C_6$ alkoxycarbonyl, a $C_1$ to $C_6$ haloalkoxycarbonyl, a phenyl($C_1$ to $C_4$) alkoxycarbonyl, a phenyl($C_1$ to $C_4$) alkoxycarbonyl substituted by $(Z)_{p1}$, phenoxycarbonyl, a phenoxycarbonyl substituted by $(Z)_{p1}$, phenylcarbonyl, a phenylcarbonyl substituted by $(Z)_{p1}$, phenyl or a phenyl substituted by $(Z)_{p1}$,

$R^{20}$ represents a hydrogen atom, a $C_1$ to $C_6$ alkyl, -CHO, a $C_1$ to $C_6$ alkylcarbonyl, a $C_1$ to $C_6$ haloalkylcarbonyl or a $C_1$ to $C_6$ alkoxycarbonyl,

$R^{21}$ represents cyano, a $C_3$ to $C_8$ cycloalkyl, a $C_3$ to $C_8$ halocycloalkyl, -OH, $-OR^8$, -SH, $-S(O)_rR^8$, $-N(R^{10})R^9$, $-N(R^{10})CHO$, $-N(R^{10})C(O)R^9$, $-N(R^{10})C(O)OR^9$, $-N(R^{10})C(O)SR^9$, $-N(R^{10})C(S)OR^9$, $-N(R^{10})C(S)SR^9$, $-N(R^{10})S(O)_2R^9$, $-C(O)OR^9$, $-C(O)N(R^{10})R^9$, $-Si(R^{13})(R^{14})R^{12}$, phenyl, a phenyl substituted by $(Z)_{p1}$, L or M,

$R^{22}$ represents a $C_1$ to $C_6$ alkyl or a $C_1$ to $C_6$ haloalkyl,

$R^{23}$ represents a halogen atom, cyano, a $C_1$ to $C_6$ alkyl, a $C_1$ to $C_6$ haloalkyl, a hydroxy($C_1$ to $C_6$) alkyl, a $C_1$ to $C_4$ alkoxy($C_1$ to $C_4$) alkyl, a $C_1$ to $C_4$ alkoxycarbonyl($C_1$ to $C_4$) alkyl, a $C_1$ to $C_6$ alkoxy, a $C_1$ to $C_6$ alkoxycarbonyl, phenyl or a phenyl substituted by $(Z)_{p1}$, when q1, q2, q3 or q4 is an integer of 2 or more, each $R^{23}$ may be the same or different from each other,

$R^{24}$ represents a hydrogen atom, a $C_1$ to $C_6$ alkyl, a $C_1$ to $C_6$ haloalkyl, -CHO, a $C_1$ to $C_6$ alkylcarbonyl, a $C_1$ to $C_6$ haloalkylcarbonyl, a phenyl($C_1$ to $C_4$) alkylcarbonyl, a phenyl($C_1$ to $C_4$) alkylcarbonyl substituted by $(Z)_{p1}$, a $C_1$ to $C_6$ alkoxycarbonyl, a $C_1$ to $C_6$ haloalkoxycarbonyl, a phenyl($C_1$ to $C_4$) alkoxycarbonyl, a phenyl($C_1$ to $C_4$) alkoxycarbonyl substituted by $(Z)_{p1}$, a $C_1$ to $C_6$ alkylthio carbonyl, a $C_1$ to $C_6$ alkoxythiocarbonyl, a $C_1$ to $C_6$ alkylaminocarbonyl, a di($C_1$ to $C_6$ alkyl)aminocarbonyl, a $C_1$ to $C_6$ alkylaminothiocarbonyl, a di($C_1$ to $C_6$ alkyl)aminothiocarbonyl, phenylcarbonyl, a phenylcarbonyl substituted by $(Z)_{p1}$, a $C_1$ to $C_6$ alkylsulfonyl, a $C_1$ to $C_6$ haloalkylsulfonyl, phenylsulfonyl, a phenylsulfonyl substituted by $(Z)_{p1}$, $-P(O)(OR^{22})_2$ or $-P(S)(OR^{22})_2$,

$R^{25}$ represents a halogen atom, cyano, a $C_3$ to $C_8$ cycloalkyl, a $C_3$ to $C_8$ halocycloalkyl, a $C_1$ to $C_6$ alkoxy, a $C_1$ to $C_6$ haloalkoxy, a $C_1$ to $C_6$ alkylthio, a $C_1$ to $C_6$ haloalkylthio, a $C_1$ to $C_6$ alkylsulfonyl, a $C_1$ to $C_6$ haloalkylsulfonyl, a $C_1$ to $C_6$ alkylamino, a di($C_1$ to $C_6$ alkyl)amino, -CHO, a $C_1$ to $C_6$ alkylcarbonyl, a $C_1$ to $C_6$ haloalkylcarbonyl, a $C_1$ to $C_6$ alkoxycarbonyl, a $C_1$ to $C_6$ haloalkoxycarbonyl, $-CH=NOR^{11}$, $-C(R^9)=NOR^{11}$, phenyl, a phenyl substituted by $(Z)_{p1}$, L or M,

$R^{26}$ represents a hydrogen atom, a $C_1$ to $C_8$ alkyl, a ($C_1$ to $C_8$) alkyl optionally substituted by $R^{33}$, a $C_3$ to $C_8$ cycloalkyl, a ($C_3$ to $C_8$) cycloalkyl optionally substituted by $R^{33}$, a $C_3$ to $C_8$ alkenyl, a ($C_3$ to $C_8$) alkenyl optionally substituted by $R^{33}$, a $C_3$ to $C_8$ alkynyl, a ($C_3$ to $C_8$) alkynyl optionally substituted by $R^{33}$, -CHO, $-C(O)R^{29}$, $-C(O)OR^{29}$, $-C(O)SR^{29}$, $-C(O)NHR^{30}$, $-C(O)N(R^{30})R^{29}$, $-C(O)C(O)R^{29}$, $-C(O)C(O)OR^{29}$, $-C(S)R^{29}$, $-C(S)OR^{29}$, $-C(S)SR^{29}$, $-C(S)NHR^{30}$, $-C(S)N(R^{30})R^{29}$, $-3(O)_2R^{29}$, $S(O)_2N(R^{30})R^{29}$, $-Si(R^{13})(R^{14})R^{12}$,- $P(O)(OR^{22})_2$, $-P(S)(OR^{22})_2$, phenyl, a phenyl substituted by $(Z)_{p1}$, L or M,

$R^{27}$ represents a hydrogen atom, a $C_1$ to $C_6$ alkyl, a $C_1$ to $C_6$ haloalkyl, a $C_3$ to $C_6$ cycloalkyl or a $C_1$ to $C_6$ alkoxy, or $R^{26}$ and $R^{27}$ are combined in combination to form a $C_2$ to $C_5$ alkylene chain whereby it forms a 3 to 6-membered ring with the nitrogen atom to which they are bonded, and at this time, the alkylene chain may contain one oxygen atom or sulfur atom, and may be substituted by a halogen atom, a $C_1$ to $C_6$ alkyl group, a $C_1$ to $C_6$ alkoxy group, a phenyl group or a phenyl group substituted by $(Z)p_1$,

$R^{2a}$ represents a $C_1$ to $C_8$ alkyl, a ($C_1$ to $C_8$) alkyl optionally substituted by $R^{33}$, a $C_3$ to $C_8$ cycloalkyl, a ($C_3$ to $C_8$) cycloalkyl optionally substituted by $R^{33}$, a $C_3$ to $C_8$ alkenyl, a ($C_3$ to $C_8$) alkenyl optionally substituted by $R^{33}$, a $C_3$ to $C_8$ alkynyl, a ($C_3$ to $C_8$) alkynyl optionally substituted by $R^{33}$, -SH, a $C_1$ to $C_6$ alkylthio, a $C_1$ to $C_6$ haloalkylthio, phenylthio, a phenylthio substituted by $(Z)p_1$, -CHO, $-C(O)R^{29}$, $-C(O)OR^{29}$, $-C(O)SR^{29}$, $-C(O)NHR^{30}$, $-C(O)N(R^{30})R^{29}$, $-C(O)C(O)R^{29}$, $-C(O)C(O)OR^{29}$, $-C(S)R^{29}$, $-C(S)OR^{29}$, $-C(S)SR^{29}$, $-C(S)NHR^{30}$, $-C(S)N(R^{30})R^{29}$, $-P(O)(OR^{22})_2$, $-P(S)(OR^{22})_2$, phenyl, a phenyl substituted by $(Z)_{p1}$, L-18, L-21, L-25, L-30 to L-35, L-45, L-48, L-49 or M,

$R^{29}$ represents a $C_1$ to $C_6$ alkyl, a $C_1$ to $C_6$ haloalkyl, a $C_3$ to $C_8$ cycloalkyl ($C_1$ to $C_4$) alkyl, a $C_1$ to $C_6$ alkoxy($C_1$ to $C_4$) alkyl, a $C_1$ to $C_6$ haloalkoxy($C_1$ to $C_4$) alkyl, a $C_1$ to $C_6$ alkylthio ($C_1$ to $C_4$) alkyl, a $C_1$ to $C_6$ haloalkylthio ($C_1$ to $C_4$) alkyl, a $C_1$ to $C_6$ alkylsulfonyl($C_1$ to $C_4$) alkyl, a $C_1$ to $C_6$ haloalkylsulfonyl($C_1$ to $C_4$) alkyl, a cyano($C_1$ to $C_6$) alkyl, a $C_1$ to $C_6$ alkylcarbonyl($C_1$ to $C_4$) alkyl, a $C_1$ to $C_6$ haloalkylcarbonyl($C_1$ to $C_4$) alkyl, a $C_1$ to $C_6$ alkoxycarbonyl ($C_1$ to $C_4$) alkyl, a di($C_1$ to $C_6$ alkyl)aminocarbonyl($C_1$ to $C_4$) alkyl, a tri($C_1$ to $C_4$ alkyl)silyl ($C_1$ to $C_4$) alkyl, a phenyl ($C_1$ to $C_4$) alkyl, a phenyl($C_1$ to $C_4$) alkyl substituted by $(Z)_{p1}$, an L-($C_1$ to $C_4$) alkyl, an M-($C_1$ to $C_4$) alkyl, a $C_3$ to $C_8$ cycloalkyl, a $C_3$ to $C_8$ halocycloalkyl, a $C_2$ to $C_6$ alkenyl, a $C_2$ to $C_6$ haloalkenyl, a $C_2$ to $C_6$ alkynyl, a $C_2$ to $C_6$ haloalkynyl, phenyl, a phenyl substituted by $(Z)_{p1}$, L or M,

$R^{30}$ represents a hydrogen atom, a $C_1$ to $C_6$ alkyl, a $C_1$ to $C_6$ haloalkyl, phenyl or a phenyl substituted by $(Z)_{P1}$, or $R^{29}$ and $R^{30}$ are combined to form a $C_2$ to $C_5$ alkylene chain whereby it may form a 3 to 6-membered ring with the nitrogen atom to which they are bonded, and at this time, the alkylene chain may contain one oxygen atom, sulfur atom or nitrogen atom, and may be optionally substituted by a halogen atom, a $C_1$ to $C_6$ alkyl group, a $C_1$ to $C_6$ alkoxy group, a formyl group, a $C_1$ to $C_6$ alkylcarbonyl group, a $C_1$ to $C_6$ alkoxycarbonyl group, a phenyl group or a phenyl group substituted by $(Z)_{p1}$,

$R^{31}$ represents a hydrogen atom, a $C_1$ to $C_8$ alkyl, a ($C_1$ to $C_8$) alkyl optionally substituted by $R^{33}$, a $C_3$ to $C_8$ cycloalkyl, a $C_3$ to $C_8$ alkenyl, a ($C_3$ to $C_8$) alkenyl optionally substituted by $R^{33}$, a $C_3$ to $C_8$ alkynyl or a ($C_3$ to $C_8$) alkynyl optionally substituted by $R^{33}$,

$R^{32}$ represents a hydrogen atom, a $C_1$ to $C_6$ alkyl, a $C_1$ to $C_6$ haloalkyl, a $C_3$ to $C_8$ cycloalkyl ($C_1$ to $C_4$) alkyl, a $C_1$ to $C_6$ alkoxy($C_1$ to $C_4$) alkyl, a $C_1$ to $C_6$ haloalkoxy($C_1$ to $C_4$) alkyl, a $C_1$ to $C_6$ alkylthio ($C_1$ to $C_4$) alkyl, a $C_1$ to $C_6$ haloalkylthio ($C_1$ to $C_4$) alkyl, a $C_1$ to $C_6$ alkylsulfonyl($C_1$ to $C_4$) alkyl, a $C_1$ to $C_6$ haloalkylsulfonyl($C_1$ to $C_4$) alkyl, a phenyl($C_1$ to $C_4$) alkyl, a phenyl($C_1$ to $C_4$) alkyl substituted by $(Z)_{P1}$, phenyl or a phenyl substituted by $(Z)_{p1}$,

$R^{33}$ represents a halogen atom, cyano, nitro, a $C_3$ to $C_8$ cycloalkyl, a $C_3$ to $C_8$ halocycloalkyl, -OH, $-OR^{34}$, -SH, $-S(O)_rR^{34}$, $-NHR^{35}$, $-N(R^{35})R^{34}$, -CHO, $-C(O)R^{29}$,- $C(O)OR^{29}$, $-C(O)SR^{29}$, $-C(O)NHR^{30}$, $-C(O)N(R^{30})R^{29}$, $-C(O)C(O)OR^{29}$, $-CH=NOR^{11}$, $-C(R^9)=NOR^{11}$, $-Si(R^{13})(R^{14})R^{12}$, $-P(O)(OR^{22})_2$, $-P(S)(OR^{22})_2$, $-P(phenyl)_2$, $-P(O)(phenyl)_2$, phenyl, a phenyl substituted by $(Z)_{p1}$, L or M,

$R^{34}$ represents a $C_1$ to $C_6$ alkyl, a $C_1$ to $C_6$ haloalkyl, a $C_1$ to $C_6$ alkoxy($C_1$ to $C_4$) alkyl, a $C_1$ to $C_6$ alkylthio($C_1$ to $C_4$) alkyl, a phenyl($C_1$ to $C_4$) alkyl, a phenyl($C_1$ to $C_4$) alkyl substituted by $(Z)_{p1}$, a $C_3$ to $C_8$ cycloalkyl, a $C_3$ to $C_8$ halocycloalkyl, a $C_3$ to $C_6$ alkenyl, a $C_3$ to $C_6$ haloalkenyl, a $C_3$ to $C_8$ cycloalkenyl, a $C_3$ to $C_8$ halocycloalkenyl, a $C_3$ to $C_6$ alkynyl, a $C_3$ to $C_6$ haloalkynyl, -CHO, a $C_1$ to $C_6$ alkylcarbonyl, a $C_1$ to $C_6$ haloalkylcarbonyl, a $C_1$ to $C_6$ alkoxycarbonyl, a $C_1$ to $C_6$ haloalkoxycarbonyl, a $C_1$ to $C_6$ alkylaminocarbonyl, a di($C_1$ to $C_6$ alkyl) aminocarbonyl, phenylcarbonyl, a phenylcarbonyl substituted by $(Z)_{p1}$, a $C_1$ to $C_6$ alkylaminothiocarbonyl, a di($C_1$ to $C_6$ alkyl) aminothiocarbonyl, phenyl, a phenyl substituted by $(Z)_{p1}$, L or M,

$R^{35}$ represents a hydrogen atom, a $C_1$ to $C_6$ alkyl, a $C_1$ to $C_6$ haloalkyl, a $C_3$ to $C_8$ cycloalkyl, a $C_3$ to $C_6$ alkenyl, a $C_3$ to $C_6$ alkynyl, a $C_1$ to $C_6$ alkylcarbonyl, a $C_1$ to $C_6$ haloalkylcarbonyl, a $C_1$ to $C_6$ alkoxycarbonyl, a $C_1$ to $C_6$ haloalkoxycarbonyl, phenoxycarbonyl, a phenoxycarbonyl substituted by $(Z)_{p1}$, phenylcarbonyl, a phenylcarbonyl substituted by $(Z)_{p1}$, a $C_1$ to $C_6$ alkylsulfonyl, a $C_1$ to $C_6$ haloalkylsulfonyl, phenyl, a phenyl substituted by $(Z)_{p1}$, L or M, or $R^{34}$ and $R^{35}$ are combined to form a $C_2$ to $C_5$ alkylene chain, whereby it may form a 3 to 6-membered ring with the nitrogen atom to which they are bonded, and at this time, the alkylene chain may contain one oxygen atom or sulfur atom, and may be substituted by a halogen atom or a methyl group,

m is an integer of 0 to 4,
n is an integer of 0 to 4,
p1 is an integer of 1 to 5,
p2 is an integer of 0 to 4,
p3 is an integer of 0 to 3,
p4 is an integer of 0 to 2,
p5 is an integer of 0 or 1,
q1 is an integer of 0 to 3,
q2 is an integer of 0 to 5,
q3 is an integer of 0 to 7,
q4 is an integer of 0 to 9,
r is an integer of 0 to 2,
t is an integer of 0 or 1,
or a salt thereof.

**2.** The substituted benzanilide compound according to Claim 1, wherein $W^1$ and $W^2$ each respresent an oxygen atom, X represents a halogen atom, cyano, nitro, a $C_1$ to $C_6$ alkyl, a $C_1$ to $C_6$ haloalkyl, a $C_1$ to $C_6$ alkoxy, a $C_1$ to $C_6$ haloalkoxy, a $C_1$ to $C_6$ alkylsulfonyloxy, a $C_1$ to $C_6$ haloalkylsulfonyloxy, a $C_1$ to $C_6$ alkylthio, a $C_1$ to $C_6$ haloalkylthio, a $C_1$ to $C_6$ alkylsulfinyl, a $C_1$ to $C_6$ haloalkylsulfinyl, a $C_1$ to $C_6$ alkylsulfonyl or a $C_1$ to $C_6$ haloalkylsulfonyl, and when m is 2 or 3, each X may be the same or different from each other, and when two Xs are adjacent to each other, the adjacent two Xs may form a 5-membered ring or 6-membered ring with the carbon atoms to which two Xs are bonded by forming $-OCH_2O-$ or $-OCH_2CH_2O-$ and at this time, the hydrogen atom(s) bonded to the respective carbon atoms which form a ring may be optionally replaced with a halogen atom, a $C_1$ to $C_4$ alkyl group or a $C_1$ to $C_4$ haloalkyl group, Y represents a halogen atom, a $C_1$ to $C_6$ alkyl, a $C_1$ to $C_6$ haloalkyl, a hydroxy($C_1$ to $C_6$) alkyl, a $C_1$ to $C_3$ alkoxy($C_1$ to $C_3$) alkyl, a $C_1$ to $C_6$ alkoxy, a $C_1$ to $C_6$ haloalkoxy, a $C_1$ to $C_6$ alkylthio, a $C_1$ to $C_6$ haloalkylthio, a $C_1$ to $C_6$ alkylamino or a di($C_1$ to $C_6$ alkyl)amino, when n is 2 or 3, each Y may be the same or different from each other,

$R^1$ represents a $C_1$ to $C_8$ alkyl, a ($C_1$ to $C_8$) alkyl optionally substituted by $R^{16}$, a $C_3$ to $C_8$ cycloalkyl, a $C_3$ to $C_8$ alkenyl, a $C_3$ to $C_8$ alkynyl, a $C_1$ to $C_8$ alkoxy, M-4, M-5, M-8, M-9, M-13 to M-19, M-21 or M-22,

$R^2$ and $R^3$ each independently represent a hydrogen atom, a $C_1$ to $C_6$ alkyl, a $C_1$ to $C_4$ alkoxy($C_1$ to $C_4$) alkyl, a $C_1$ to $C_4$ alkylthio ($C_1$ to $C_4$) alkyl, a $C_1$ to $C_4$ alkylsulfonyl($C_1$ to $C_4$) alkyl, a $C_3$ to $C_6$ alkenyl, a $C_3$ to $C_6$ alkynyl, a $C_1$ to $C_6$ alkylthio, a $C_1$ to $C_6$ haloalkylthio, phenylthio, a phenylthio substituted by $(Z)_{p1}$ or $-SN(R^{18})R^{17}$, or $R^2$ and $R^1$ may be combined to form a $C_2$ to $C_6$ alkylene chain whereby they may form a 3 to 7-membered ring with the nitrogen atom to which they are bonded, and at this time, the alkylene chain may contain one oxygen atom or sulfur atom,

$R^4$ represents a $C_1$ to $C_6$ alkyl, a $C_1$ to $C_6$ haloalkyl, a ($C_1$ to $C_6$) alkyl optionally substituted by $R^{21}$, a ($C_1$ to $C_6$) haloalkyl optionally substituted by $R^{21}$, a $C_3$ to $C_8$ cycloalkyl, a $C_3$ to $C_8$ halocycloalkyl, a $C_3$ to $C_6$ haloalkenyl, a $C_3$ to $C_6$ haloalkynyl, phenyl or a phenyl substituted by $(Z)_{p1}$,

$R^5$ represents cyano, a ($C_1$ to $C_6$) alkyl optionally substituted by $R^{21}$, a ($C_1$ to $C_6$) haloalkyl optionally substituted by $R^{21}$, a $C_3$ to $C_8$ cycloalkyl, a $C_3$ to $C_8$ halocycloalkyl, a ($C_2$ to $C_6$) alkenyl optionally substituted by $R^{21}$, a ($C_2$ to $C_6$) alkynyl optionally substituted by $R^{21}$, $-C(O)OR^9$, $-C(O)SR^9$, $-C(O)NHR^{10}$, $-C(O)N(R^{10})R^9$, $-C(S)OR^9$, $-C(S)SR^9$, $-C(S)NHR^{10}$, $-C(S)N(R^{10})R^9$, phenyl, a phenyl substituted by $(Z)_{p1}$, a phenoxyphenyl substituted by $(Z)_{p1}$, a pyridyloxyphenyl substituted by $(Z)_{p1}$, L-1 to L-4, L-8 to L-13, L-15 to L-23, L-25 to L-35, L-37, L-38, L-40, L-43 to L-58, M-4, M-5, M-8, M-9, M-14 to M-18 or M-19, or may be combined with Y existing at the adjacent position to form a $C_2$ to $C_3$ alkylene chain, whereby it may form a 5 to 6-membered ring which fuses with a benzene ring, and at this time, the alkylene chain may contain one oxygen atom, sulfur atom or nitrogen atom, and may be optionally substituted

by a halogen atom or a $C_1$ to $C_6$ haloalkyl group,

$R^6$ represents a hydrogen atom, a $C_1$ to $C_6$ alkyl, a $C_1$ to $C_6$ haloalkyl, a $C_1$ to $C_4$ alkoxy($C_1$ to $C_4$) alkyl, a $C_1$ to $C_4$ alkylthio($C_1$ to $C_4$) alkyl, a cyano($C_1$ to $C_6$) alkyl, a phenyl-($C_1$ to $C_4$) alkyl, a phenyl($C_1$ to $C_4$) alkyl substituted by $(Z)_{p1}$, a $C_3$ to $C_6$ alkenyl, a $C_3$ to $C_6$ haloalkenyl, a phenyl($C_3$ to $C_6$) alkenyl, a phenyl($C_3$ to $C_6$) alkenyl substituted by $(Z)_{p1}$, a $C_3$ to $C_6$ alkynyl, a $C_3$ to $C_6$ haloalkynyl, a phenyl($C_3$ to $C_6$) alkynyl, a phenyl($C_3$ to $C_6$) alkynyl substituted by $(Z)_{p1}$, $-S(O)_2R^9$, $-C(O)R^9$, $-C(O)NHR^{10}$, $-C(O)N(R^{10})R^9$, $-C(S)NHR^{10}$, $-C(S)N(R^{10})R^9$, $-Si(R^{13})(R^{14})R^{12}$, $-P(O)(OR^{22})_2$ or $-P(S)(OR^{22})_2$,

Z represents a halogen atom, cyano, nitro, a $C_1$ to $C_6$ alkyl, a $C_1$ to $C_6$ haloalkyl, a $C_1$ to $C_3$ alkylthio($C_1$ to $C_3$) alkyl, a $C_1$ to $C_3$ haloalkylthio($C_1$ to $C_3$) alkyl, a $C_1$ to $C_3$ alkylsulfinyl($C_1$ to $C_3$) alkyl, a $C_1$ to $C_3$ haloalkylsulfinyl($C_1$ to $C_3$) alkyl, a $C_1$ to $C_3$ alkylsulfonyl-($C_1$ to $C_3$) alkyl, a $C_1$ to $C_3$ haloalkylsulfonyl($C_1$ to $C_3$) alkyl, a $C_1$ to $C_6$ alkoxy, a $C_1$ to $C_6$ haloalkoxy, a $C_1$ to $C_3$ haloalkoxy($C_1$ to $C_3$) haloalkoxy, a $C_1$ to $C_6$ alkylsulfonyloxy, a $C_1$ to $C_6$ haloalkylsulfonyloxy, a $C_1$ to $C_6$ alkylthio, a $C_1$ to $C_6$ haloalkylthio, a $C_1$ to $C_6$ alkylsulfinyl, a $C_1$ to $C_6$ haloalkylsulfinyl, a $C_1$ to $C_6$ alkylsulfonyl, a $C_1$ to $C_6$ haloalkylsulfonyl, $-C(O)NH_2$ or $-C(S)NH_2$, and when p1, p2, p3 or p4 is an integer of 2 or more, each Z may be the same or different from each other,

further, when two Zs are adjacent to each other, the adjacent two Zs may form a 5-membered ring or 6-membered ring with the carbon atoms to which two Zs are bonded by forming $-CF_2CF_2O-$, $-CF_2OCF_2-$, $-OCF_2O-$, $-OCF_2CHFO-$, $-OCF_2CF_2O-$ or $-CH=CHCH=CH-$,

$R^9$ represents a $C_1$ to $C_6$ alkyl, a $C_1$ to $C_6$ haloalkyl, a $C_3$ to $C_6$ cycloalkyl($C_1$ to $C_4$) alkyl, a phenyl($C_1$ to $C_4$) alkyl, a phenyl($C_1$ to $C_4$) alkyl substituted by $(Z)_{p1}$, a $C_3$ to $C_8$ cycloalkyl, a $C_3$ to $C_8$ halocycloalkyl, phenyl or a phenyl substituted by $(Z)_{p1}$,

$R^{10}$ represents a hydrogen atom or a $C_1$ to $C_6$ alkyl, or $R^9$ and $R^{10}$ are combined to form a $C_4$ to $C_5$ alkylene chain, whereby it may form a 5-membered ring or 6-membered ring with the nitrogen atom to which they are bonded, and at this time, the alkylene chain may contain one oxygen atom or sulfur atom,

$R^{12}$ represents a $C_1$ to $C_6$ alkyl, phenyl or a phenyl substituted by $(Z)_{p1}$,

$R^{13}$ and $R^{14}$ each independently represent a $C_1$ to $C_6$ alkyl,

$R^{15}$ represents a $C_1$ to $C_6$ alkyl, a $C_1$ to $C_6$ haloalkyl, a phenyl($C_1$ to $C_4$) alkyl, a phenyl($C_1$ to $C_4$) alkyl substituted by $(Z)p_1$, phenyl or a phenyl substituted by $(Z)_{p1}$,

$R^{16}$ represents a halogen atom, cyano, a $C_3$ to $C_6$ cycloalkyl, $-OR^{26}$, $-N(R^{27})R^{26}$, $-S(O)rR^{28}$, $-SO_2N(R^{30})R^{29}$, a $C_1$ to $C_6$ alkoxycarbonyl, $-C(O)N(R^{30})R^{29}$, $-C(R^{32})=NOH$, $-C(R^{32})=NOR^{31}$, $-Si(R^{13})(R^{14})R^{12}$, phenyl, a phenyl substituted by $(Z)_{p1}$, L-1, L-2, L-3, L-4, L-45, L-46, L-47 or M,

$R^{17}$ represents a $C_1$ to $C_6$ alkyl, a $C_1$ to $C_6$ alkoxycarbonyl($C_1$ to $C_4$) alkyl or a $C_1$ to $C_6$ alkoxycarbonyl,

$R^{18}$ represents a $C_1$ to $C_6$ alkyl, or $R^{17}$ and $R^{18}$ are combined to form a $C_4$ to $C_5$ alkylene chain whereby it may form a 5-membered ring or 6-membered ring with the nitrogen atom to which they are bonded, and at this time, the alkylene chain may contain one oxygen atom or sulfur atom, and may be optionally substituted by a methyl group or a methoxy group,

$R^{21}$ represents cyano, a $C_3$ to $C_6$ cycloalkyl, a $C_3$ to $C_6$ halocycloalkyl, a $C_1$ to $C_6$ alkoxy, a $C_1$ to $C_6$ haloalkoxy, phenoxy, a phenoxy substituted by $(Z)_{p1}$, a $C_1$ to $C_6$ alkylthio, a $C_1$ to $C_6$ haloalkylthio, phenylthio, a phenylthio substituted by $(Z)_{p1}$, a $C_1$ to $C_6$ alkylsulfinyl, a $C_1$ to $C_6$ haloalkylsulfinyl, a $C_1$ to $C_6$ alkylsulfonyl, a $C_1$ to $C_6$ haloalkylsulfonyl, phenylsulfonyl, a phenylsulfonyl substituted by $(Z)_{p1}$, a $C_1$ to $C_6$ alkylamino, a di($C_1$ to $C_6$ alkyl)amino, phenylamino, a phenylamino substituted by $(Z)_{p1}$. a $C_1$ to $C_6$ alkoxycarbonyl, phenyl, a phenyl substituted by $(Z)_{p1}$, L-1 to L-5, L-8 to L-24, L-36, L-39, L-45 to L-52 or L-53,

$R^{22}$ represents a $C_1$ to $C_6$ alkyl,

$R^{23}$ represents a $C_1$ to $C_4$ alkyl, when q1, q2, q3 or q4 is an integer of 2 or more, each $R^{23}$ may be the same or different from each other,

$R^{24}$ represents -CHO, a $C_1$ to $C_6$ alkylcarbonyl, a $C_1$ to $C_6$ alkoxycarbonyl or a $C_1$ to $C_6$ alkylsulfonyl,

$R^{26}$ represents a hydrogen atom, a $C_1$ to $C_6$ alkyl, a $C_1$ to $C_6$ haloalkyl, a $C_1$ to $C_4$ alkoxy($C_1$ to $C_4$) alkyl, a $C_1$ to $C_4$ alkylthio($C_1$ to $C_4$) alkyl, a phenyl($C_1$ to $C_4$) alkyl, a phenyl($C_1$ to $C_4$) alkyl substituted by $(Z)_{p1}$, a $C_1$ to $C_6$ alkylcarbonyl, a $C_1$ to $C_6$ haloalkylcarbonyl, a $C_3$ to $C_6$ cycloalkylcarbonyl, a $C_1$ to $C_6$ alkoxycarbonyl, $-C(O)N(R^{30})R^{29}$, a $C_1$ to $C_6$alkylsulfonyl, a di($C_1$ to $C_6$ alkyl)aminosulfonyl, phenylsulfonyl, a phenylsulfonyl substituted by $(Z)_{p1}$, a di($C_1$ to $C_6$ alkyl)phosphoryl, a di($C_1$ to $C_6$ alkyl)thiophosphoryl, a tri($C_1$ to $C_4$ alkyl)silyl, phenyl or a phenyl substituted by $(Z)_{p1}$,

$R^{27}$ represents a hydrogen atom or a $C_1$ to $C_6$ alkyl,

$R^{28}$ represents a $C_1$ to $C_6$ alkyl, a $C_1$ to $C_6$ haloalkyl, a hydroxy($C_1$ to $C_4$) alkyl, a $C_1$ to $C_4$ alkoxy($C_1$ to $C_4$) alkyl, a $C_1$ to $C_4$ alkylthio($C_1$ to $C_4$) alkyl, a $C_1$ to $C_4$ alkylcarbonyl-($C_1$ to $C_4$) alkyl, a $C_1$ to $C_4$ alkoxycarbonyl($C_1$ to $C_4$) alkyl, a $C_1$ to $C_4$ alkylaminocarbonyl($C_1$ to $C_4$) alkyl, a di($C_1$ to $C_4$ alkyl)aminocarbonyl($C_1$ to $C_4$) alkyl, a tri($C_1$ to $C_4$ alkyl) silyl ($C_1$ to $C_4$) alkyl, a phenyl($C_1$ to $C_4$) alkyl, a phenyl($C_1$ to $C_4$) alkyl substituted by $(Z)_{p1}$, a $C_3$ to $C_6$ alkenyl, a $C_3$ to $C_6$ alkynyl, a $C_1$ to $C_6$ alkylthio, phenyl, a phenyl substituted by $(Z)_{p1}$, L-21, L-35, L-45 or L-48,

$R^{29}$ represents a $C_1$ to $C_6$ alkyl, a $C_1$ to $C_4$ alkoxy($C_1$ to $C_4$) alkyl, a $C_1$ to $C_4$ alkylthio($C_1$ to $C_4$) alkyl, a phenyl($C_1$

to $C_4$) alkyl, a phenyl($C_1$ to $C_4$) alkyl substituted by $(Z)_{p1}$, a $C_3$ to $C_6$ cycloalkyl, a $C_3$ to $C_6$ alkenyl, a $C_2$ to $C_6$ alkynyl, phenyl or a phenyl substituted by $(Z)_{p1}$,
$R^{30}$ represents a hydrogen atom or a $C_1$ to $C_6$ alkyl, or $R^{29}$ and $R^{30}$ are combined to form a $C_2$ to $C_5$ alkylene chain, whereby it may form a 3 to 6-membered ring with the nitrogen atom to which they are bonded, and at this time, the alkylene chain may contain one oxygen atom or sulfur atom,
$R^{31}$ represents a $C_1$ to $C_6$ alkyl, a phenyl($C_1$ to $C_4$) alkyl or a phenyl($C_1$ to $C_4$) alkyl substituted by $(Z)_{p1}$,
$R^{32}$ represents a hydrogen atom or a $C_1$ to $C_6$ alkyl,
m is an integer of 0 to 3,
n is an integer of 0 to 3,
q2, q3 and q4 are each independently an integer of 0 to 2
or a salt thereof.

**3.** The substituted benzanilide compound according to Claim 2, wherein X represents a halogen atom, nitro, a $C_1$ to $C_6$ alkyl, a $C_1$ to $C_6$ haloalkyl, a $C_1$ to $C_6$ alkoxy, a $C_1$ to $C_6$ haloalkoxy, a $C_1$ to $C_6$ alkylsulfonyloxy, a $C_1$ to $C_6$ alkylthio, a $C_1$ to $C_6$ haloalkylthio, a $C_1$ to $C_6$ alkylsulfinyl, a $C_1$ to $C_6$ haloalkylsulfinyl, a $C_1$ to $C_6$ alkylsulfonyl or a $C_1$ to $C_6$ haloalkylsulfonyl, and when m is 2, each × may be the same or different from each other,
Y represents a halogen atom, a $C_1$ to $C_6$ alkyl, a $C_1$ to $C_6$ haloalkyl, a $C_1$ to $C_6$ alkoxy or a $C_1$ to $C_6$ alkylthio, and when n is 2, each Y may be the same or different from each other,
$R^1$ represents a $C_1$ to $C_8$ alkyl, a ($C_1$ to $C_8$) alkyl optionally substituted by $R^{16}$, a $C_3$ to $C_8$ alkenyl or a $C_3$ to $C_8$ alkynyl,
$R^2$ represents a hydrogen atom or a $C_1$ to $C_6$ alkyl,
$R^3$ represents a hydrogen atom,
$R^4$ represents a $C_1$ to $C_6$ alkyl, a $C_1$ to $C_6$ haloalkyl, a $C_1$ to $C_3$ alkoxy($C_1$ to $C_3$) haloalkyl, a $C_1$ to $C_3$ alkylthio($C_1$ to $C_3$) haloalkyl, a $C_3$ to $C_6$ cycloalkyl or a $C_3$ to $C_6$ halocycloalkyl,
$R^5$ represents a ($C_1$ to $C_6$) alkyl optionally substituted by $R^{21}$, a ($C_1$ to $C_6$) haloalkyl optionally substituted by $R^{21}$, a ($C_2$ to $C_6$) alkenyl optionally substituted by $R^{21}$, a ($C_2$ to $C_6$) alkynyl optionally substituted by $R^{21}$, a $C_1$ to $C_6$ alkoxycarbonyl, phenyl, a phenyl substituted by $(Z)_{p1}$, a phenoxyphenyl substituted by $(Z)_{p1}$, a pyridyloxyphenyl substituted by $(Z)_{p1}$, L-1 to L-4, L-8 to L-13, L-15 to L-23, L-45 to L-52 or L-53, or may be combined with Y existing at the adjacent position to form a $C_2$ to $C_3$ alkylene chain, whereby it may form a 5 to 6-membered ring which fuses with a benzene ring, and at this time, the alkylene chain may contain one oxygen atom, sulfur atom or nitrogen atom, and may be optionally substituted by a halogen atom,
$R^6$ represents a hydrogen atom, a $C_1$ to $C_6$ alkyl, a $C_1$ to $C_4$ alkoxy($C_1$ to $C_4$) alkyl, a $C_1$ to $C_6$ alkylcarbonyl or a tri ($C_1$ to $C_4$ alkyl)silyl,
$R^{16}$ represents $-OR^{26}$, $-N(R^{27})R^{26}$, $-S(O)_rR^{28}$, $-SO_2N(R^{30})R^{29}$, $-C(R^{32})=NOH$ or $-C(R^{32})=NOR^{31}$,
$R^{21}$ represents a $C_1$ to $C_6$ alkoxy, a $C_1$ to $C_6$ haloalkoxy, phenoxy, a phenoxy substituted by $(Z)_{p1}$, phenylthio, a phenylthio substituted by $(Z)_{p1}$, phenylsulfonyl, a phenylsulfonyl substituted by $(Z)_{p1}$, a $C_1$ to $C_6$ alkylamino, a di($C_1$ to $C_6$ alkyl)amino, phenylamino, a phenylamino substituted by $(Z)_{p1}$, a $C_1$ to $C_6$ alkoxycarbonyl, phenyl, a phenyl substituted by $(Z)_{p1}$, L-1 to L-5, L-8 to L-24, L-36, L-39, L-45 to L-52 or L-53,
$R^{26}$ represents a hydrogen atom, a $C_1$ to $C_6$ alkyl, a $C_1$ to $C_6$ alkylcarbonyl, a $C_1$ to $C_6$ alkoxycarbonyl, a $C_1$ to $C_6$ alkylaminocarbonyl, a di($C_1$ to $C_6$ alkyl)aminocarbonyl or a $C_1$ to $C_6$ alkylsulfonyl,
$R^{28}$ represents a $C_1$ to $C_6$ alkyl,
$R^{29}$ represents a $C_1$ to $C_6$ alkyl,
$R^{30}$ represents a hydrogen atom or a $C_1$ to $C_6$ alkyl,
$R^{31}$ represents a $C_1$ to $C_6$ alkyl,
$R^{32}$ represents a hydrogen atom,
m is an integer of 0 to 2,
n is an integer of 0 to 2
or a salt thereof.

**4.** The substituted benzanilide compound according to Claim 3, wherein X represents a halogen atom, nitro, a $C_1$ to $C_4$ alkyl, a $C_1$ to $C_4$ haloalkyl, a $C_1$ to $C_4$ alkylthio, a $C_1$ to $C_4$ alkylsulfinyl or a $C_1$ to $C_4$ alkylsulfonyl, and when m is 2, each X may be the same or different from each other,
Y represents a halogen atom or a $C_1$ to $C_4$ alkyl, when n is 2, each Y may be the same or different from each other,
$R^1$ represents a $C_1$ to $C_8$ alkyl, a $C_1$ to $C_4$ alkylthio($C_1$ to $C_4$) alkyl, a $C_1$ to $C_4$ alkylsulfinyl($C_1$ to $C_4$) alkyl or a $C_1$ to $C_4$ alkylsulfonyl($C_1$ to $C_4$) alkyl,
$R^2$ represents a hydrogen atom,
$R^4$ represents a $C_1$ to $C_6$ alkyl or a $C_1$ to $C_6$ haloalkyl,
$R^5$ represents phenyl, a phenyl substituted by $(Z)_{p1}$, a phenoxyphenyl substituted by $(Z)_{p1}$, a pyridyloxyphenyl substituted by $(Z)_{p1}$, L-1 to L-4, L-8 to L-13, L-15 to L-23, L-45 to L-52 or L-53,

$R^6$ represents a hydrogen atom or a salt thereof.

**5.** An N-substituted phenyl-3-nitrophthalimide or substituted aniline represented by the formula (2) or the formula (3):

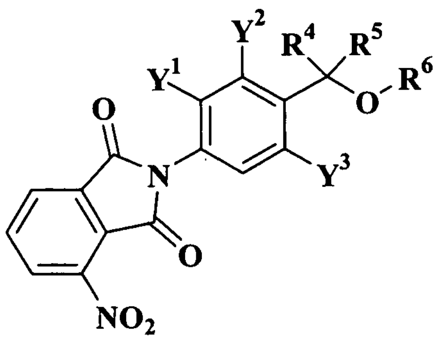

(2)

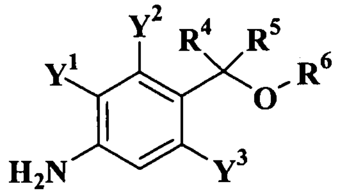

(3)

wherein $Y^1$ represents a hydrogen atom, a halogen atom, a $C_1$ to $C_6$ alkyl, a $C_1$ to $C_6$ haloalkyl, a $C_1$ to $C_6$ alkoxy or a $C_1$ to $C_6$ alkylthio,
$Y^2$ and $Y^3$ each independently represent a hydrogen atom, or may form a $C_2$ to $C_3$ alkylene chain in combination with $R^5$, whereby it may form a 5 to 6-membered ring which fuses with a benzene ring, at this time, the alkylene chain may contain one oxygen atom, sulfur atom or nitrogen atom, and may be optionally substituted by a halogen atom,
$R^4$ represents a $C_1$ to $C_6$ alkyl, a $C_1$ to $C_6$ haloalkyl, a $C_1$ to $C_3$ alkoxy($C_1$ to $C_3$) haloalkyl, a $C_1$ to $C_3$ alkylthio($C_1$ to $C_3$) haloalkyl, a $C_3$ to $C_6$ cycloalkyl or a $C_3$ to $C_6$ halocycloalkyl,
$R^5$ represents a ($C_1$ to $C_6$) alkyl optionally substituted by $R^{21}$, a ($C_1$ to $C_6$) haloalkyl optionally substituted by $R^{21}$, a ($C_2$ to $C_6$) alkenyl optionally substituted by $R^{2'}$, a ($C_2$ to $C_6$) alkynyl optionally substituted by $R^{21}$, a $C_1$ to $C_6$ alkoxycarbonyl, phenyl, a phenyl substituted by $(Z)_{p1}$, a phenoxyphenyl substituted by $(Z)_{p1}$, a pyridyloxyphenyl substituted by $(Z)_{p1}$, L-1 to L-4, L-8 to L-13, L-15 to L-23, L-45 to L-52 or L-53,
$R^6$ represents a hydrogen atom, a $C_1$ to $C_6$ alkyl, a $C_1$ to $C_4$ alkoxy($C_1$ to $C_4$) alkyl, a $C_1$ to $C_6$ alkylcarbonyl or a tri ($C_1$ to $C_4$ alkyl)silyl,
$R^{21}$ represents a $C_1$ to $C_6$ alkoxy, a $C_1$ to $C_6$ haloalkoxy, phenoxy, a phenoxy substituted by $(Z)_{p1}$, phenylthio, a phenylthio substituted by $(Z)_{p1}$, phenylsulfonyl, a phenylsulfonyl substituted by $(Z)_{p1}$, a $C_1$ to $C_6$ alkylamino, a di($C_1$ to $C_6$ alkyl)amino, phenylamino, a phenylamino substituted by $(Z)_{p1}$, a $C_1$ to $C_6$ alkoxycarbonyl, phenyl, a phenyl substituted by $(Z)_{p1}$, L-1 to L-5, L-8 to L-24, L-36, L-39, L-45 to L-52 or L-53,
L-1 to L-5, L-8 to L-24, L-36, L-39, L-45 to L-52 or L-53 each represent the following aromatic heterocyclic ring,

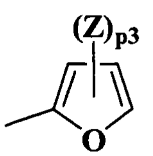

L-1

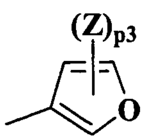

L-2

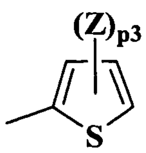

L-3

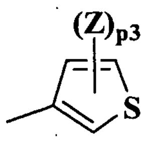

L-4

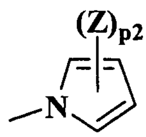

L-5

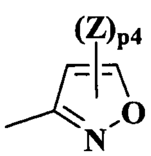

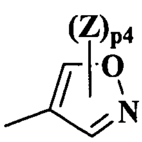

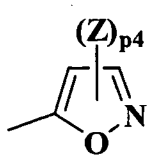

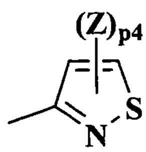

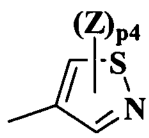

L-8 L-9 L-10 L-11 L-12

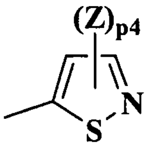
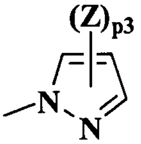
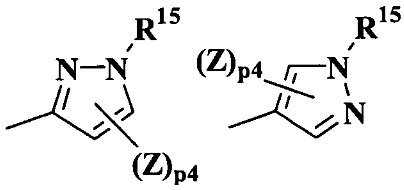
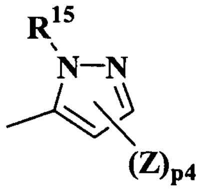

L-13 L-14 L-15 L-16 L-17

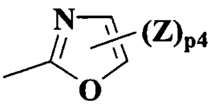
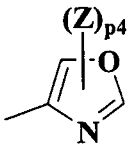
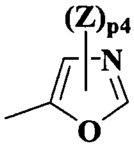
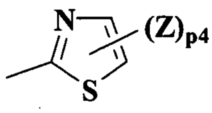
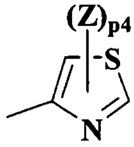

L-18 L-19 L-20 L-21 L-22

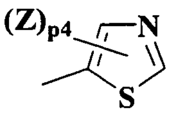
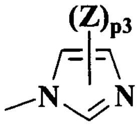
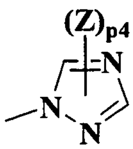
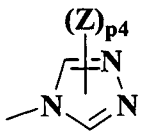

L-23 L-24 L-36 L-39

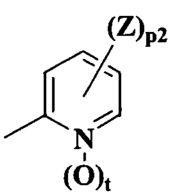
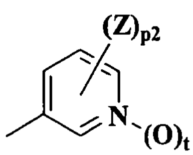
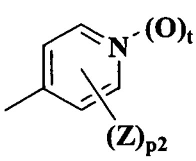
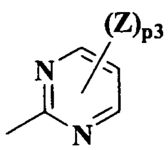
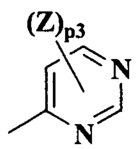

L-45 L-46 L-47 L-48 L-49

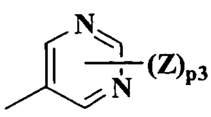
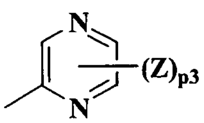
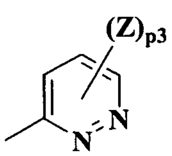
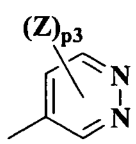

L-50 L-51 L-52 L-53

Z represents a halogen atom, cyano, nitro, a $C_1$ to $C_6$ alkyl, a $C_1$ to $C_6$ haloalkyl, a $C_1$ to $C_3$ alkylthio($C_1$ to $C_3$) alkyl, a $C_1$ to $C_3$ haloalkylthio($C_1$ to $C_3$) alkyl, a $C_1$ to $C_3$ alkylsulfinyl($C_1$ to $C_3$) alkyl, a $C_1$ to $C_3$ haloalkylsulfinyl($C_1$ to $C_3$) alkyl, a $C_1$ to $C_3$ alkylsulfonyl-($C_1$ to $C_3$) alkyl, a $C_1$ to $C_3$ haloalkylsulfonyl($C_1$ to $C_3$) alkyl, a $C_1$ to $C_6$ alkoxy, a $C_1$ to $C_6$ haloalkoxy, a $C_1$ to $C_3$ haloalkoxy($C_1$ to $C_3$) haloalkoxy, a $C_1$ to $C_6$ alkylsulfonyloxy, a $C_1$ to $C_6$ haloalkylsulfonyloxy, a $C_1$ to $C_6$ alkylthio, a $C_1$ to $C_6$ haloalkylthio, a $C_1$ to $C_6$ alkylsulfinyl, a $C_1$ to $C_6$ haloalkylsulfinyl, a $C_1$ to

$C_6$ alkylsulfonyl, a $C_1$ to $C_6$ haloalkylsulfonyl, $-C(O)NH_2$ or $-C(S)NH_2$, when p1, p2, p3 or p4 is an integer of 2 or more, each Z may be the same or different from each other,
further, when two Zs are adjacent to each other, the adjacent two Zs may form a 5-membered ring or 6-membered ring with the carbon atoms to which two Zs are bonded by forming $-CF_2CF_2O-$, $-CF_2OCF_2-$, $-OCF_2O-$, $-OCF_2CHFO-$, $-OCF_2CF_2O-$ or
$-CH=CHCH=CH-$,
$R^{15}$ represents a $C_1$ to $C_6$ alkyl, phenyl or a phenyl substituted by $(Z)_{p1}$,
p1 is an integer of 1 to 5,
p2 is an integer of 0 to 4,
p3 is an integer of 0 to 3,
p4 is an integer of 0 to 2,
p5 is an integer of 0 or 1,
r is an integer of 0 to 2,
t is an integer of 0 or 1.]
or a salt thereof.

**6.** A noxious organism controlling agent which comprises one or more kinds selected from the substituted benzanilide compound and a salt thereof according to any one of Claims 1 to 4 as an effective ingredient.

**7.** An agricultural chemical which comprises one or more kinds selected from the substituted benzanilide compound and a salt thereof according to any one of Claims 1 to 4 as an effective ingredient.

**8.** An insecticide or araricide which comprises one or more kinds selected from the substituted benzanilide compound and a salt thereof according to any one of Claims 1 to 4 as an effective ingredient.

## INTERNATIONAL SEARCH REPORT

International application No.
PCT/JP2004/014810

**A. CLASSIFICATION OF SUBJECT MATTER**

Int.Cl[7] C07C215/46, 233/75, 309/66, 317/28, 323/42, C07D213/38, 213/40, 213/64, 231/14, 249/08, 307/83, C07F7/18, A01N37/22, 43/12

According to International Patent Classification (IPC) or to both national classification and IPC

**B. FIELDS SEARCHED**

Minimum documentation searched (classification system followed by classification symbols)

Int.Cl[7] C07C215/46, 233/75, 309/66, 317/28, 323/42, C07D213/38, 213/40, 213/64, 231/14, 249/08, 307/83, C07F7/18, A01N37/22, 43/12

Documentation searched other than minimum documentation to the extent that such documents are included in the fields searched

Electronic data base consulted during the international search (name of data base and, where practicable, search terms used)

CA(STN), REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| X | JP 2003-40864 A (Nissan Chemical Industries, Ltd.), 13 February, 2003 (13.02.03), Full text (Family: none) | 1,6-8 |
| A | JP 11-240857 A (Nihon Nohyaku Co., Ltd.), 07 September, 1999 (07.09.99), Full text & EP 919542 A2 | 1-4,6-8 |
| A | JP 2001-240580 A (Nihon Nohyaku Co., Ltd.), 04 September, 2001 (04.09.01), Full text & WO 01/46124 A1 & EP 1241159 A1 & US 2004/0009982 A | 1-4,6-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 December, 2004 (22.12.04) | 25 January, 2005 (25.01.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No.
PCT/JP2004/014810

C (Continuation). DOCUMENTS CONSIDERED TO BE RELEVANT

| Category* | Citation of document, with indication, where appropriate, of the relevant passages | Relevant to claim No. |
|---|---|---|
| A | JP 2001-131141 A (Nihon Nohyaku Co., Ltd.), 15 May, 2001 (15.05.01), Full text & EP 1006107 A2 & US 6603044 B1 | 1-4,6-8 |

Form PCT/ISA/210 (continuation of second sheet) (January 2004)

**INTERNATIONAL SEARCH REPORT**

International application No.
PCT/JP2004/014810

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

Compounds showing insecticidal activity and having the partial structure "N-C-(1,2-phenylene)-C-N-(1,4-phenylene)-C-O" are known to persons skilled in the art (JP 11-240857 A, JP 2001-131141 A, JP 2001-240580 A, and WO 03/011028 A1).

In view of this, the technical feature "N-C-(1,2-phenylene)-C-N-(1,4-phenylene)-C-O" of all the compounds of claim 1 cannot be regarded as a special technical feature (a technical feature which as a whole clearly shows a contribution to the prior art). There is no technical relationship involving a special technical feature among (continued to extra sheet)

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
Part of claims 1-3 and 6-8 and claim 4
($R^5$ is phenyl or the like optionally substituted by $(Z)_{p1}$ or is L or M)

**Remark on Protest** ☐ The additional search fees were accompanied by the applicant's protest.
☐ No protest accompanied the payment of additional search fees.

Form PCT/ISA/210 (continuation of first sheet (2)) (January 2004)

**INTERNATIONAL SEARCH REPORT**

International application No.
PCT/JP2004/014810

Continuation of Box No.III of continuation of first sheet(2)

the compounds (the choices) of claim 1.
Therefore, this international application does not comply with the requirement of unity of invention.

Form PCT/ISA/210 (extra sheet) (January 2004)